# EUROPÄISCHE PATENTSCHRIFT

(11) **EP 1 558 600 B1**
(45) Veröffentlichungstag und Bekanntmachung des Hinweises auf die Patenterteilung: **07.05.2008**
(21) Anmeldenummer: 03809317.5
(22) Anmeldetag: 23.10.2003
(51) Int. Cl.: C07D 401/14, C07D 471/10, C07D 513/04, C07D 453/04, C07D 401/04, C07D 451/04, C07D 471/04, C07D 495/04, C07D 487/08, A61K 31/4545, A61P 25/06

(54) **AUSGEWÄHLTE CGRP-ANTAGONISTEN, VERFAHREN ZU DEREN HERSTELLUNG SOWIE DEREN VERWENDUNG ALS ARZNEIMITTEL**
SELECTED CGRP ANTAGONISTS, METHOD FOR PRODUCTION AND USE THEREOF AS MEDICAMENT
ANTAGONISTES DU CGRP SELECTIONNES, PROCEDES DE PRODUCTION ET D'UTILISATION COMME MEDICAMENTS DESDITS ANTAGONISTES

(30) Priorität: 25.10.2002 DE 10250080
(43) Veröffentlichungstag der Anmeldung: 03.08.2005
(73) Patentinhaber: Boehringer Ingelheim Pharma GmbH & Co. KG, 55216 Ingelheim am Rhein (DE)
(72) Erfinder: RUDOLF, Klaus, 88447 Warthausen (DE); MÜLLER, Stephan, Georg, 88447 Warthausen (DE); STENKAMP, Dirk, 88400 Biberach (DE); LUSTENBERGER, Philipp, 88447 Warthausen (DE); DREYER, Alexander, 88484 Gutenzell-Hürbel (DE); BAUER, Eckhart, 88400 Biberach (DE); SCHINDLER, Marcus, 88400 Biberach (DE); KIRSTEN, Arndt, 88400 Biberach (DE); DOODS, Henri, 88447 Warthausen (DE)
(86) Internationale Anmeldenummer: PCT/EP2003/011762
(87) Internationale Veröffentlichungsnummer: WO 2004/037810

(56) Entgegenhaltungen:
- WO-A-00/18764
- WO-A-01/10425
- JOHN J. MALLEE ET AL.: "Receptor Activity-modifying Protein 1 Determines the Species Selectivity of Non-peptide CGRP Receptor Antagonists" J. BIOL. CHEM., Bd. 277, Nr. 16, 19. April 2002 (2002-04-19), Seiten 14294-14298, XP002271313

## Beschreibung

Gegenstand der vorliegenden Erfindung sind die CGRP-Antagonisten der allgemeinen Formel 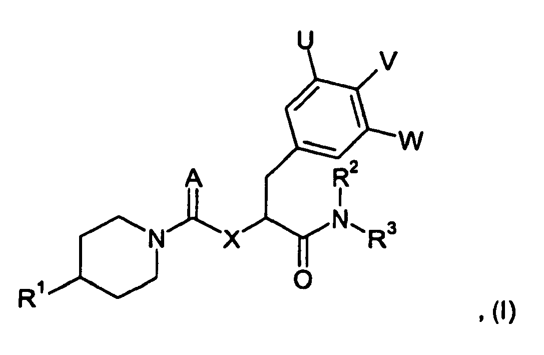 deren Tautomere, deren Diastereomere, deren Enantiomere, deren Hydrate, deren Gemische und deren Salze sowie die Hydrate der Salze, insbesondere deren physiologisch verträgliche Salze mit anorganischen oder organischen Säuren, diese Verbindungen enthaltende Arzneimittel, deren Verwendung und Verfahren zu ihrer Herstellung.

In der WO 01/10425 werden bereits CGRP-Antagonisten beschrieben, die zur Bekämpfung von menopausalen Hitzewallungen geeignet sind. Die dort genannten Verbindungen sind strukturell unterschiedlich zu den erfindungsgemäßen Verbindungen; beispielsweise unterscheiden sie sich in der Definition der Gruppe -X der allgemeinen Formel (I).

In der obigen allgemeinen Formel (I) bedeuten in einer ersten Ausführungsform
A ein Sauerstoff- oder Schwefelatom, eine Phenylsulfonylimino- oder Cyaniminogruppe.
X ein Sauerstoff- oder Schwefelatom, eine gegebenenfalls durch eine C₁₋₆-Alkylgruppe substituierte Iminogruppe oder eine gegebenenfalls durch eine C₁₋₆-Alkylgruppe substituierte Methylengruppe,
U eine C₁₋₆-Alkyl-, C₂₋₆-Alkenyl- oder C₂₋₆-Alkinylgruppe, in der jede Methylengruppe mit bis zu 2 Fluoratomen und jede Methylgruppe mit bis zu 3 Fluoratomen substituiert sein kann,
V ein Chlor- oder Bromatom, eine Amino-, Methylamino- oder Hydroxygruppe,
W ein Wasserstoff-, Fluor-, Chlor-, Brom- oder lodatom, eine Difluor- oder Trifluormethylgruppe,
R¹ einen gesättigten, einfach oder zweifach ungesättigten 5- bis 7-gliedrigen Aza-, Diaza-, Triaza-, Oxaza-, Thiaza-, Thiadiaza- oder *S,S*-Dioxido-thiadiaza-Heterocyclus,
   wobei die vorstehend erwähnten Heterocyclen über ein Kohlenstoff- oder Stickstoffatom verknüpft sind,
   eine oder zwei Carbonyl- oder Thiocarbonylgruppen benachbart zu einem Stickstoffatom enthalten,
   an einem der Stickstoffatome durch eine Alkylgruppe substituiert sein können,
   an einem oder an zwei Kohlenstoffatomen durch eine Alkylgruppe, durch eine Phenyl-, Phenylmethyl-, Naphthyl-, Biphenylyl-, Pyridinyl-, Diazinyl-, Furyl-, Thienyl-, Pyrrolyl-, 1,3-Oxazolyl-, 1,3-Thiazolyl-, Isoxazolyl-, Pyrazolyl-, 1-Methylpyrazolyl-, Imidazolyl- oder 1-Methylimidazolylgruppe substituiert sein können, wobei die Substituenten gleich oder verschieden sein können, und
   wobei eine olefinische Doppelbindung eines der vorstehend erwähnten ungesättigten Heterocyclen mit einem Phenyl-, Naphthyl-, Pyridin-, Diazin-, 1,3-Oxazol-, Thienyl-, Furan-, Thiazol-, Pyrrol-, *N*-Methylpyrrol- oder Chinolin-Ring, mit einem gegebenenfalls am Stickstoffatom durch eine Alkylgruppe substituierten 1*H*-Chinolin-2-on-Ring oder mit einem Imidazol- oder *N*-Methylimidazol-Ring kondensiert sein kann oder auch zwei olefinische Doppelbindungen eines der vorstehend erwähnten ungesättigten Heterocyclen jeweils mit einem Phenylring kondensiert sein können,
      wobei die in R¹ enthaltenen Phenyl-, Pyridinyl-, Diazinyl-, Furyl-, Thienyl-, Pyrrolyl-, 1,3-Oxazolyl-, 1,3-Thiazolyl-, Isoxazolyl-, Pyrazolyl-, 1-Methylpyrazolyl-, Imidazolyl- oder 1-Methylimidazolylgruppen sowie benzo-, thieno-, pyrido- und diazinokondensierten Heterocyclen im Kohlenstoffgerüst zusätzlich durch Fluor-, Chlor-, Brom- oder lodatome, durch Alkyl-, Alkoxy-, Nitro-, Alkylthio-, Alkylsulfinyl-, Alkylsulfonyl-, Alkylsulfonylamino-, Phenyl-, Difluormethyl-, Trifluormethyl-, Alkoxycarbonyl-, Carboxy-, Hydroxy-, Amino-, Alkylamino-, Dialkylamino-, Acetyl-, Acetylamino-, Propionylamino-, Aminocarbonyl-, Alkylaminocarbonyl-, Dialkylaminocarbonyl-, (4-Morpholinyl)-carbonyl-, (1-Pyrrolidinyl)carbonyl-, (1-Piperidinyl)carbonyl-, (Hexahydro-1-azepinyl)carbonyl-, (4-Methyl-1-piperazinyl)carbonyl-, Methylendioxy-, Aminocarbonylamino-, Alkanoyl-, Cyano-, Difluormethoxy-, Trifluormethoxy-, Trifluormethylthio-, Trifluormethylsulfinyl- oder Trifluormethylsulfonylgruppen mono-, di- oder trisubstituiert sein können, wobei die Substituenten gleich oder verschieden sein können,
R² das Wasserstoffatom,
eine Phenylmethylgruppe oder eine C₂₋₇-Alkylgruppe, die in ω-Stellung durch eine Cyclohexyl-, Phenyl-, Pyridinyl-, Diazingl-, Hydroxy-, Amino-, Alkylamino-, Dialkylamino-, Carboxy-, Alkoxycarbonyl-, Aminocarbonyl-, Aminocarbonylamino-, Acetylamino-, 1-Pyrrolidinyl-, 1-Piperidinyl-, 4-(1-Piperidinyl)-1-piperidinyl-, 4-Morpholinyl-, Hexahydro-1*H*-1-azepinyl-, [Bis-(2-hydroxyethyl)]amino-, 4-Alkyl-1-piperazinyl- oder 4-(ω-Hydroxy-C₂₋₇-alkyl)-1-piperazinylgruppe substituiert sein kann,
eine Phenyl- oder Pyridinylgruppe,
   wobei die vorstehend erwähnten heterocyclischen Reste und Phenylgruppen zusätzlich im Kohlenstoffgerüst durch Fluor-, Chlor-, Brom- oder lodatome, durch Methyl-, Alkoxy-, Difluormethyl-, Trifluormethyl-, Hydroxy-, Amino-, C₁₋₃-Alkylamino-, Di-(C₁₋₃-alkyl)-amino-, Acetylamino-, Aminocarbonyl-, Cyano-, Methylsulfonyloxy-, Difluormethoxy-, Trifluormethoxy-, Trifluormethylthio-, Trifluormethylsulfinyl-, Trifluormethylsulfonyl-, Amino-C₁₋₃-alkyl-, C₁₋₃-Alkylamino-C₁₋₃-alkyl- oder Di-(C₁₋₃-alkyl)-amino-C₁₋₃-alkylgruppen mono-, di- oder trisubstituiert und die Substituenten gleich oder verschieden sein können,
R³ das Wasserstoffatom oder eine gegebenenfalls durch eine Phenyl- oder Pyridinylgruppe substituierte C₁₋₃-Alkylgruppe,
   wobei die C₁₋₃-Alkylgruppe mit einer in R² vorhandenen Alkylgruppe oder einem in R² vorhandenen Phenyl- oder Pyridylring und dem Stickstoffatom, an dem sie gebunden sind, unter Ausbildung eines Ringes verbunden sein kann oder
R² und R³ zusammen mit dem eingeschlossenen Stickstoffatom einen Rest der allgemeinen Formel 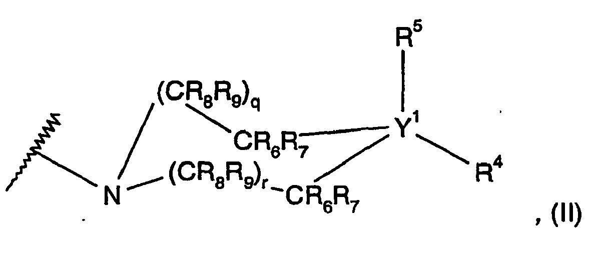
in der
   Y¹ das Kohlenstoffatom oder, wenn R⁵ ein freies Elektronenpaar darstellt, auch das Stickstoffatom,
   q und r, wenn Y¹ das Kohlenstoffatom darstellt, die Zahlen 0, 1 oder 2, oder
   q und r, wenn Y¹ das Stickstoffatom darstellt, die Zahlen 1 oder 2,
   R⁴ das Wasserstoffatom, eine Amino-, Alkylamino-, Cycloalkylamino-, Dialkylamino-, *N*-(Cycloalkyl)-alkylamino-, Dicycloalkylamino-, Hydroxy-, Alkyl-, Cycloalkyl-, Amino-C₂₋₇-alkyl-, Alkylamino-C₂₋₇-alkyl-, Dialkylamino-C₂₋₇-alkyl-, Aminoiminomethyl-, Alkylcarbonyl-, Alkylsulfonyl-, Alkylcarbonylamino-, Alkylsulfonylamino-, *N*-Alkylcarbonyl-*N*-alkylamino-, *N*-Alkylsulfonyl-*N*-alkylamino-, Aminocarbonylamino-, Alkylaminocarbonylamino-, Dialkylaminocarbonylamino-, Cycloalkylaminocarbonylamino-, Dicycloalkylaminocarbonylamino-, Phenylaminocarbonylamino-, Aminocarbonylalkyl-, Alkylaminocarbonylalkyl-, Dialkylaminocarbonylalkyl-, Aminocarbonylaminoalkyl-, Alkoxycarbonyl-, Alkoxycarbonylalkyl- oder Carboxyalkylgruppe,
   oder auch, wenn Y¹ nicht das Stickstoffatom darstellt, die Carboxy-, Aminomethyl-, Alkylaminomethyl- oder Dialkylaminomethylgruppe,
   eine Phenyl-, Phenyl-C₁₋₃-alkyl-, Pyridinyl-, Diazinyl-, 1-Naphthyl-, 2-Naphthyl-, Pyridinylcarbonyl- oder Phenylcarbonylgruppe, die jeweils im Kohlenstoffgerüst durch Fluor-, Chlor-, Brom- oder lodatome, durch Alkyl-, Alkoxy-, Methylsulfonyloxy-, Difluormethyl-, Trifluormethyl-, Hydroxy-, Amino-, Acetylamino-, Aminocarbonyl-, Aminocarbonylamino-, Aminocarbonylaminomethyl-, Cyano-, Carboxy-, Alkoxycarbonyl-, Carboxyalkyl-, Alkoxycarbonylalkyl-, Alkanoyl-, ω-(Dialkylamino)alkanoyl-, ω-(Dialkylamino)alkyl-, ω-(Dialkylamino)hydroxyalkyl-, ω-(Carboxy)alkanoyl-, Difluormethoxy-, Trifluormethoxy-, Trifluormethylthio-, Trifluormethylsulfinyl- oder Trifluormethylsulfonylgruppen mono-, di- oder trisubstituiert sein können, wobei die Substituenten gleich oder verschieden sein können,
   eine gesättigte oder ein- oder mehrfach ungesättigte 4- bis 10-gliedrige Azacycloalkylgruppe, eine 5- bis 10-gliedrige Oxaza-, Thiaza-, Diaza- oder Triazacycloalkylgruppe, eine 6- bis 10-gliedrige Azabicyclo- oder Diazabicycloalkylgruppe, eine 1-Alkyl-4-piperidinylcarbonyl- oder 4-Alkyl-1-piperazinylcarbonyl-, eine 1-Alkyl-4-piperidinylamino-, 1-Alkyl-4-piperidinylaminocarbonyl- oder 1-Alkyl-4-piperidinylaminosulfonylgruppe,
      wobei die vorstehend genannten mono- und bicyclischen Heterocyclen über ein Stickstoff- oder ein Kohlenstoffatom gebunden sind,
      eine Methylengruppe in den vorstehend genannten mono- und bicyclischen Heterocyclen durch eine Carbonyl- oder Sulfonylgruppe ersetzt sein kann,
      in den vorstehend genannten mono- und bicyclischen Heterocyclen eine nicht direkt an ein Stickstoff-, Sauerstoff- oder Schwefelatom gebundene Methylengruppe durch ein oder zwei Fluoratome substituiert sein kann, die vorstehend genannten mono- und bicyclischen Heterocyclen sowie die 1-Alkyl-4-piperidinylcarbonyl- und 4-Alkyl-1-piperazinylcarbonylgruppe im Ring ein- oder mehrfach durch eine C₁₋₇-Alkylgruppe oder/und
      einfach durch eine Benzyl-, Alkanoyl-, Dialkylamino-, Phenylcarbonyl-, Pyridinylcarbonyl-, Carboxy-, Carboxyalkanoyl-, Carboxyalkyl-, Alkoxycarbonylalkyl-, Alkoxycarbonyl-, Aminocarbonyl-, Alkylaminocarbonyl-, Alkylsulfonyl-, Cycloalkyl- oder Cycloalkylalkylgruppe, durch eine im Ring gegebenenfalls alkylsubstituierte Cycloalkylcarbonyl-, Azacycloalkylcarbonyl-, Diazacycloalkylcarbonyl- oder Oxazacycloalkylcarbonylgruppe substituiert sein können,
         wobei die in diesen Substituenten enthaltenen alicyclischen Teile 3 bis 10 Ringglieder und die heteroalicyclischen Teile jeweils 4 bis 10 Ringglieder umfassen und
         die in den vorstehend genannten Resten enthaltenen Phenyl- und Pyridinyl-Reste ihrerseits durch Fluor-, Chlor-, Brom- oder lodatome, durch Alkyl-, Alkoxy-, Methylsulfonyloxy-, Difluormethyl-, Trifluormethyl-, Hydroxy-, Amino-, Acetylamino-, Aminocarbonyl-, Aminocarbonylamino-, Aminocarbonylaminomethyl-, Cyano-, Carboxy-, Alkoxycarbonyl-, Carboxyalkyl-, Alkoxycarbonylalkyl-, Alkanoyl-, ω-(Dialkylamino)alkanoyl-, ω-(Carboxy)alkanoyl-, Difluormethoxy-, Trifluormethoxy-, Trifluormethylthio-, Trifluormethylsulfinyl- oder Trifluormethylsulfonylgruppen mono-, di- oder trisubstituiert sein können, wobei die Substituenten gleich oder verschieden sein können,
   R⁵ ein Wasserstoffatom,
   einen C₁₋₄-Alkylrest, wobei ein unverzweigter Alkylrest in ω-Stellung durch eine Phenyl-, Pyridinyl-, Diazinyl-, Amino-, Alkylamino-, Dialkylamino-, 1-Pyrrolidinyl-, 1-Piperidinyl-, 4-Methyl-1-piperazinyl-, 4-Morpholinyl- oder Hexahydro-1*H*-1-azepinyl-Gruppe substituiert sein kann,
   eine Alkoxycarbonyl-, die Cyano- oder Aminocarbonylgruppe oder auch, wenn Y¹ ein Stickstoffatom darstellt, ein freies Elektronenpaar,
   oder, wenn Y¹ kein Stickstoffatom darstellt, auch das Fluoratom, oder
   R⁴ und R⁵ zusammen, wenn Y¹ das Kohlenstoffatom darstellt, einen 4- bis 7-gliedrigen cycloaliphatischen Ring, in dem eine oder zwei Methylengruppen durch eine -NH- oder -N(Alkyl)-Gruppe und eine oder zwei weitere Methylengruppen durch Carbonylgruppen ersetzt sein können,
   wobei ein an ein Stickstoffatom innerhalb der voranstehend erwähnten Gruppe R⁴ gebundenes Wasserstoffatom durch einen Schutzrest ersetzt sein kann,
   R⁶ und R⁷, die gleich oder verschieden sein können, jeweils ein Wasserstoffatom, eine C₁₋₃-Alkyl- oder Dialkylaminogruppe oder auch, wenn Y¹ kein Stickstoffatom darstellt, das Fluoratom und
   R⁸ und R⁹, die gleich oder verschieden sein können, jeweils ein Wasserstoffatom oder eine C₁₋₃-Alkyl-, Carboxy- oder C₁₋₃-Alkoxycarbonylgruppe,
wobei alle vorstehend genannten Alkyl- und Alkoxygruppen sowie die innerhalb der anderen genannten Reste vorhandenen Alkylgruppen, sofern nichts anderes angegeben ist, 1 bis 7 Kohlenstoffatome umfassen und geradkettig oder verzweigt sein können, wobei jede Methylengruppe mit bis zu 2 Fluoratomen und jede Methylgruppe mit bis zu 3 Fluoratomen substituiert sein kann,
alle vorstehend genannten Cycloalkylgruppen sowie die innerhalb der anderen genannten Reste vorhandenen Cycloalkylgruppen, sofern nichts anderes angegeben ist, 3 bis 10 Kohlenstoffatome umfassen können, wobei jede Methylengruppe mit bis zu 2 Fluoratomen substituiert sein kann,
alle vorstehend erwähnten aromatischen und heteroaromatischen Reste zusätzlich durch Fluor-, Chlor- oder Bromatome, durch Cyano- oder Hydroxygruppen mono-, di- oder trisubstituiert und die Substituenten gleich oder verschieden sein können und
unter den in den vor- und nachstehenden Definitionen genannten Schutzresten die aus der Peptidchemie geläufigen Schutzgruppen zur verstehen sind, insbesondere
eine im Phenylkern gegebenenfalls durch ein Halogenatom, durch eine Nitro- oder Phenylgruppe, durch eine oder zwei Methoxygruppen substituierte Phenylalkoxycarbonylgruppe mit 1 bis 3 Kohlenstoffatomen im Alkoxyteil,
   beispielsweise die Benzyloxycarbonyl-, 2-Nitro-benzyloxycarbonyl-, 4-Nitro-benzyloxycarbonyl-, 4-Methoxy-benzyloxycarbonyl-, 2-Chlor-benzyloxycarbonyl-, 3-Chlor-benzyloxycarbonyl-, 4-Chlor-benzyloxycarbonyl-, 4-Biphenylyl-a,a-di-' methyl-benzyloxycarbonyl- oder 3,5-Dimethoxy-α,α-dimethyl-benzyloxycarbonylgruppe,
eine Alkoxycarbonylgruppe mit insgesamt 1 bis 5 Kohlenstoffatomen im Alkylteil,
   beispielsweise die Methoxycarbonyl-, Ethoxycarbonyl-, *n*-Propoxycarbonyl-, Isopropoxycarbonyl-, *n*-Butoxycarbonyl-, 1-Methylpropoxycarbonyl-, 2-Methylpropoxy-carbonyl- oder *tert*.Butyloxycarbonylgruppe,
die Allyloxycarbonyl-, 2,2,2-Trichlor-(1,1-dimethylethoxy)carbonyl- oder 9-Fluorenylmethoxycarbonyl-Gruppe oder
die Formyl-, Acetyl- oder Trifluoracetylgruppe.

Eine zweite Ausführungsform der vorliegenden Erfindung besteht in den Verbindungen der obigen allgemeinen Formel (I), in denen
A, U, V, W, X, R² und R³ wie voranstehend unter der ersten Ausführungsform erwähnt definiert sind und
R¹ einen einfach oder zweifach ungesättigten 5- bis 7-gliedrigen Aza-, Diaza-, Triaza- oder Thiaza-Heterocyclus bedeutet,
   wobei die vorstehend erwähnten Heterocyclen über ein Kohlenstoff- oder Stickstoffatom verknüpft sind,
   eine oder zwei Carbonylgruppen benachbart zu einem Stickstoffatom enthalten,
   an einem Kohlenstoffatom durch eine Phenyl-, Pyridinyl-, Diazinyl-, Thienyl-, Pyrrolyl-, 1,3-Thiazolyl-, Isoxazolyl-, Pyrazolyl- oder 1-Methylpyrazolyl-Gruppe substituiert sein können und
   eine olefinische Doppelbindung eines der vorstehend erwähnten ungesättigten Heterocyclen mit einem Phenyl-, Naphthyl-, Pyridin-, Diazin-, Thienyl- oder Chinolin-Ring oder mit einem gegebenenfalls am Stickstoffatom durch eine Methylgruppe substituierten 1*H*-Chinolin-2-on-Ring kondensiert sein kann,
      wobei die in R¹ enthaltenen Phenyl-, Pyridinyl-, Diazinyl-, Thienyl-, Pyrrolyl-, 1,3-Thiazolyl-, Isoxazolyl-, Pyrazolyl- oder 1-Methylpyrazolylgruppen sowie die benzo-, pyrido- und diazinokondensierten Heterocyclen im Kohlenstoffgerüst zusätzlich durch Fluor-, Chlor-, Brom- oder lodatome, durch Alkyl-, Alkoxy-, Nitro-, Difluormethyl-, Trifluormethyl-, Hydroxy-, Amino-, Alkylamino-, Dialkylamino-, Acetylamino-, Acetyl-, Cyano-, Difluormethoxy- oder Trifluormethoxygruppen mono-, di- oder trisubstituiert sein können, wobei die Substituenten gleich oder verschieden sein können,
wobei die vorstehend genannten Alkylgruppen oder die in den vorstehend genannten Resten enthaltenen Alkylgruppen, sofern nichts anderes angegeben wurde, 1 bis 7 Kohlenstoffatome enthalten und verzweigt oder unverzweigt sein können, wobei jede Methylengruppe mit bis zu 2 Fluoratomen und jede Methylgruppe mit bis zu 3 Fluoratomen substituiert sein kann, und
die vorstehend erwähnten aromatischen und heteroaromatischen Reste zusätzlich durch Fluor-, Chlor- oder Bromatome, durch Cyano- oder Hydroxygruppen mono-, di- oder trisubstituiert und die Substituenten gleich oder verschieden sein können.

Eine dritte Ausführungsform der vorliegenden Erfindung besteht in den Verbindungen der obigen allgemeinen Formel (I), in denen
A, U, V, W, X, R² und R³ wie unter der ersten Ausführungsform erwähnt definiert sind und
R¹ einen einfach ungesättigten 5- bis 7-gliedrigen Diaza- oder Triaza-Heterocyclus bedeutet,
   wobei die vorstehend erwähnten Heterocyclen über ein Stickstoffatom verknüpft sind,
   eine Carbonylgruppe benachbart zu einem Stickstoffatom enthalten und
   zusätzlich an einem Kohlenstoffatom durch eine Phenylgruppe substituiert sein können,
   und wobei eine olefinische Doppelbindung eines der vorstehend erwähnten ungesättigten Heterocyclen mit einem Phenyl-, Thienyl- oder Chinolin-Ring kondensiert sein kann,
      wobei die in R¹ enthaltenen Phenylgruppen sowie benzokondensierten Heterocyclen im Kohlenstoffgerüst zusätzlich durch Fluor-, Chlor-, Brom- oder lodatome, durch Methyl-, Methoxy-, Nitro-, Difluormethyl-, Trifluormethyl-, Hydroxy-, Amino-, Alkylamino-, Dialkylamino-, Acetylamino-, Acetyl-, Cyano- Difluormethoxy- oder Trifluormethoxygruppen mono-, di- oder trisubstituiert sein können, wobei die Substituenten gleich oder verschieden sein können, vorzugsweise jedoch unsubstituiert oder durch ein Fluor-, Chlor- oder Bromatom, durch eine Methyl- oder Methoxygruppe monosubstituiert sind,
wobei alle vorstehend genannten Alkylgruppen sowie die innerhalb der anderen Reste vorhandenen Alkylgruppen, sofern nichts anderes angegeben ist, 1 bis 7 Kohlenstoffatome umfassen und geradkettig oder verzweigt sein können und die vorstehend erwähnten aromatischen und heteroaromatischen Reste zusätzlich durch Fluor-, Chlor- oder Bromatome, durch Cyano- oder Hydroxygruppen mono-, di- oder trisubstituiert und die Substituenten gleich oder verschieden sein können,

Eine vierte Ausführungsform der vorliegenden Erfindung besteht in den Verbindungen der obigen allgemeinen Formel (I), in denen
A, U, V, W, X, R² und R³ wie unter der ersten Ausführungsform erwähnt definiert sind und
R¹ eine 4-(2-Oxo-1,2,4,5-tetrahydro-1,3-benzdiazepin-3-yl)-, 4-(2-Oxo-1,4-dihydro-2*H*-chinazolin-3-yl)-, 4-(5-Oxo-3-phenyl-4,5-dihydro-1,2,4-triazol-1-yl)-, 4-(2-Oxo-1,2-dihydro-imidazo[4,5-c]chinolin-3-yl)-, 4-(2-Oxo-1,2-dihydro-4*H*-thieno[3,4-d]pyrimidin-3-yl)-, 4-(2-Oxo-1,4-dihydro-2*H*-thieno[3,2-d]pyrimidin-3-yl)-, 4-(5-Oxo-4,5,7,8-tetrahydro-2-thia-4,6-diaza-azulen-6-yl)-, 4-(2-Oxo-1,2,4,5-tetrahydro-thieno[3,2-d]-1,3-diazepin-3-yl)-, 4-(2-Oxo-1,2,4,5-tetrahydro-thieno-[2,3-d]-1,3-diazepin-3-yl)- oder 4-(2-Oxo-1,4-dihydro-2*H*-thieno[2,3-d]-pyrimidin-3-yl)-gruppe bedeutet,
   wobei die vorstehend erwähnten mono- und bicyclischen Heterocyclen im Kohlenstoffgerüst zusätzlich durch eine Methoxygruppe monosubstituiert sein können,
wobei die vorstehend erwähnten aromatischen und heteroaromatischen Reste zusätzlich durch Fluor-, Chlor- oder Bromatome, durch Cyano- oder Hydroxygruppen mono-, di- oder trisubstituiert und die Substituenten gleich oder verschieden sein können.

Eine fünfte Ausführungsform der vorliegenden Erfindung besteht in den Verbindungen der obigen allgemeinen Formel (I), in denen
A, U, V, W, X und R¹ wie eingangs unter der ersten Ausführungsform erwähnt definiert sind und
R² das Wasserstoffatom oder
eine Phenylmethylgruppe oder eine C₂₋₇-Alkylgruppe, die in ω-Stellung durch eine Phenyl-, Pyridinyl-, Hydroxy-, Amino-, Alkylamino-, Dialkylamino-, Carboxy-, Alkoxycarbonyl-, Aminocarbonyl-, Aminocarbonylamino-, Acetylamino-, 1-Pyrrolidinyl-, 1-Piperidinyl-, 4-Morpholinyl-, [Bis-(2-hydroxyethyl)]aminogruppe substituiert sein kann,
   wobei die vorstehend erwähnten heterocyclischen Reste und Phenylgruppen zusätzlich im Kohlenstoffgerüst durch Fluor-, Chlor-, Brom- oder lodatome, durch Methyl-, Alkoxy-, Difluormethyl-, Trifluormethyl-, Hydroxy-, Amino-, C₁₋₃-Alkylamino-, Di-(C₁₋₃-alkyl)-amino-, Acetylamino-, Aminocarbonyl-, Cyano-, Difluormethoxy-, Trifluormethoxy-, Amino-C₁₋₃-alkyl-, C₁₋₃-Alkylamino-C₁₋₃-alkyl- oder Di-(C₁₋₃-Alkyl)-amino-C₁₋₃-alkylgruppen mono-, di- oder trisubstituiert und die Substituenten gleich oder verschieden sein können,
R³ das Wasserstoffatom oder eine C₁₋₃-Alkylgruppe,
   wobei die C₁₋₃-Alkylgruppe mit einer in R² vorhandenen Alkylgruppe oder einem in R² vorhandenen Phenyl- oder Pyridylring und dem Stickstoffatom, an dem sie gebunden sind, unter Ausbildung eines 5- bis 7-gliedrigen Ringes verbunden sein kann oder
R² und R³ zusammen mit dem eingeschlossenen Stickstoffatom einen Rest der allgemeinen Formel 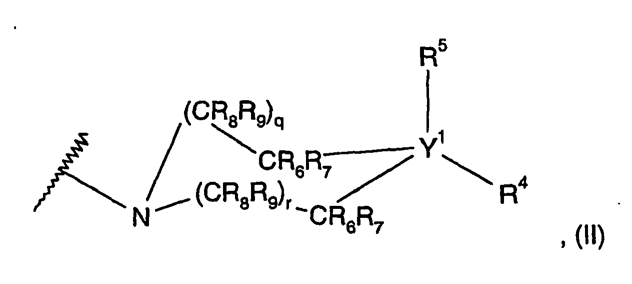 in der
   Y¹ das Kohlenstoffatom oder, wenn R⁵ ein freies Elektronenpaar darstellt, auch das Stickstoffatom,
   q und r, wenn Y¹ das Kohlenstoffatom darstellt, die Zahlen 0 oder 1 oder
   q und r, wenn Y¹ das Stickstoffatom darstellt, die Zahlen 1 oder 2,
   R⁴ das Wasserstoffatom, eine Hydroxy-, Amino-, Alkylamino-, C₃₋₆-Cycloalkylamino-, *N*-(C₃₋₆-Cycloalkyl)-alkylamino- oder Dialkylamino-, eine Alkyl-, Trifluormethyl-, C₃₋₆-Cycloalkyl-, Dialkylamino-C₂₋₇-alkyl-, Carboxyalkyl-, Alkoxycarbonylalkyl-, Alkylsulfonyl-, Alkylsulfonylamino- oder *N*-(Alkylsulfonyl)-alkylaminogruppe,
   oder auch, wenn Y¹ nicht das Stickstoffatom darstellt, die Carboxy- oder Dialkylaminomethylgruppe,
   eine Phenyl-, Phenyl-C₁₋₃-alkyl-, Pyridinyl- oder Diazinylgruppe, die jeweils durch ein Fluor-, Chlor- oder Bromatom, durch eine Trifluormethylcarbonyl-, Methyl- oder Methoxygruppe substituiert sein können,
   eine gesättigte oder ein- oder mehrfach ungesättigte 4- bis 7-gliedrige Azacycloalkylgruppe, eine 5- bis 7-gliedrige Oxaza-, Diaza- oder Triazacycloalkylgruppe,eine 7- bis 9-gliedrige Azabicyclo- oder Diazabicycloalkylgruppe, eine 1-Alkyl-4-piperidinylamino- oder 1-Alkyl-4-piperidinylaminosulfonylgruppe
      wobei die vorstehend genannten mono- und bicyclischen Heterocyclen über ein Stickstoff- oder ein Kohlenstoffatom gebunden sind,
      eine Methylengruppe der vorstehend genannten mono- und bicyclischen Heterocyclen durch eine Carbonyl- oder Sulfonylgruppe ersetzt sein kann,
      in den vorstehend genannten mono- und bicyclischen Heterocyclen eine nicht direkt an ein Stickstoff-, Sauerstoff- oder Schwefelatom gebundene Methylengruppe durch ein oder zwei Fluoratome substituiert sein kann, die vorstehend genannten mono- und bicyclischen Heterocyclen durch eine oder zwei C₁₋₃-Alkylgruppen, in der jede Methylengruppe mit bis zu 2 und jede Methylgruppe mit bis zu 3 Fluoratomen substituierte sein kann, oder/und
      durch eine C₃₋₆-Cycloalkyl-C₁₋₃-alkyl-, Benzyl-, C₁₋₄-Alkanoyl-, Di-(C₁₋₃-alkyl)-amino- oder C₁₋₃-Alkylsulfonyl-, durch eine Alkoxycarbonyl-, Benzyloxycarbonyl-, Alkoxycarbonylalkyl-, Carboxy- oder Carboxyalkylgruppe substituiert sein können,
   R⁵ ein Wasserstoffatom, eine C₁₋₃-Alkyl- oder Alkoxycarbonylgruppe oder,
   wenn Y¹ ein Stickstoffatom darstellt, auch ein freies Elektronenpaar, oder
   R⁴ und R⁵ zusammen, wenn Y¹ das Kohlenstoffatom darstellt, einen 5- bis 6-gliedrigen cycloaliphatischen Ring; in dem eine oder zwei Methylengruppen durch eine -NH- oder -N(Methyl)-Gruppe und eine oder zwei weitere Methylengruppen durch Carbonylgruppen ersetzt sein können,
   R⁶ und R⁷, die gleich oder verschieden sein können, jeweils ein Wasserstoffatom oder eine C₁₋₃-Alkyl- oder Di-(C₁₋₃-alkyl)-aminogruppe und
   R⁸ und R⁹, die gleich oder verschieden sein können, jeweils ein Wasserstoffatom oder eine C₁₋₃-Alkyl-, Carboxy- oder C₁₋₃-Alkoxycarbonylgruppe darstellen,
wobei alle vorstehend genannten Alkylgruppen sowie die innerhalb der anderen Reste vorhandenen Alkylgruppen, sofern nichts anderes angegeben ist, 1 bis 7 Kohlenstoffatome umfassen und geradkettig oder verzweigt sein können und die vorstehend erwähnten aromatischen und heteroaromatischen Reste zusätzlich durch Fluor-, Chlor- oder Bromatome, durch Cyano- oder Hydroxygruppen mono-, di- oder trisubstituiert und die Substituenten gleich oder verschieden sein können,
bedeuten.

Eine sechste Ausführungsform der vorliegenden Erfindung besteht in den Verbindungen der obigen allgemeinen Formel (I), in denen
A, U, V, W, X und R¹ wie unter der ersten Ausführungsform erwähnt definiert sind und
R² eine Phenylmethylgruppe oder eine C₂₋₇-Alkylgruppe, die in ω-Stellung durch eine Phenyl-, Amino-, Alkylamino- oder Dialkylaminogruppe substituiert sein kann,
   wobei die vorstehend genannte Phenylgruppe durch eine Amino-C₁₋₃-alkyl-, C₁₋₃-Alkylamino-C₁₋₃alkyl- oder Di-(C₁₋₃-alkyl)-amino-C₁₋₃-alkylgruppe substituiert sein kann,
R³ das Wasserstoffatom oder eine C₁₋₃-Alkylgruppe,
R² und R³ zusammen mit dem Stickstoffatom, an dem sie gebunden sind, eine 7-Dimethylaminomethyl-1,2,4,5-tetrahydro-3-benzazepin-3-yl- oder 2-Amino-4,5,7,8-tetrahydro-thiazolo[4,5-d]azepin-6-yl-gruppe oder
R² und R³ zusammen mit dem eingeschlossenen Stickstoffatom einen Rest der allgemeinen Formel 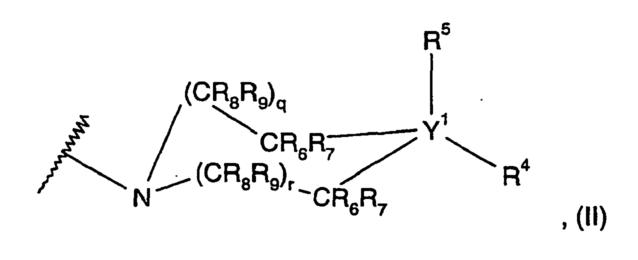 in der
   Y¹ das Kohlenstoffatom oder, wenn R⁵ ein freies Elektronenpaar darstellt, auch das Stickstoffatom,
   q und r, wenn Y¹ das Kohlenstoffatom darstellt, die Zahlen 0 oder 1 oder
   q und r, wenn Y¹ das Stickstoffatom darstellt, die Zahlen 1 oder 2,
   R⁴ das Wasserstoffatom,
   eine Phenyl-, Benzyl- oder Pyridinylgruppe, die jeweils durch ein Fluor-, Chlor- oder Bromatom, durch eine Trifluormethylcarbonyl-, Methyl- oder Methoxygruppe substituiert sein können,
   eine Hydroxy-, Carboxy-, Methyl-, Trifluormethyl-, *n*-Propyl-, Phenyl-, *p*-Tolyl-, *p*-Trifluormethylcarbonyl-phenyl-, *p*-(3-Dimethylaminopropyl)-phenyl-, Amino-, Benzyl-, *tert*-Butylamino-, Dimethylamino-, Diethylamino-, Diethylaminomethyl-, 2-Dimethylaminoethyl-, 2-Diethylaminoethyl-, 5-Aminopentyl-, Methoxycarbonyl-, Methoxycarbonylmethyl-, Perhydro-azepin-1-yl-, 4-Methyl-perhydro-1,4-diazepin-1-yl-, 1-Methyl-1-piperidinyl-4-yl-, 4-Piperazin-1-yl-, 4-Acetyl-piperazin-1-yl-, 4-Cyclopropylmethyl-piperazin-1-yl-, Pyrrolidin-1-yl-, 4-Ethyl-piperazin-1-yl-, 4-Isopropyl-piperazin-1-yl-, Piperidin-1-yl-, Piperidin-4-yl-, Morpholin-4-yl-, 4,4-Difluor-1-piperidin-1-yl-, 1-Methyl-1-aza-bicyclo[3.2.1]oct-4-yl- oder 4-Methylpiperazin-1-yl-, 4-Ethylpiperazin-1-yl-, 1-Methyl-piperidin-1-yl-, 4-Carboxymethyl-piperazin-1-yl-, 1-Carboxymethyl-piperidin-4-yl-, 4-Benzyloxycarbonyl-piperazin-1-yl-, 1-Ethoxycarbonylmethyl-piperidin-4-yl-, Azetidin-1-yl-, 5-Methyl-2,5-diaza-bicyclo[2.2.1]hept-2-yl-, 1-Benzyl-piperidin-4-yl-, 4-Benzyl-piperazin-1-yl-, 4-Dimethylaminomethyl-1-phenyl-, 2,2,2-Trifluorethyl-piperazin-1-yl-, 1-Methylsulfonyl-piperidin-4-yl-, Piperidin-1-yl-methyl-, 1-Methyl-piperidin-4-yl-amino-, Methylsulfonylamino-, *N*-Methylsulfonyl-*N*-methylamino-, *N*-(Cyclopentyl)-methylamino-, 1,1-Dioxo-λ⁶-isothiazolidin-2-yl-, 2-Oxo-perhydro-1,3-oxazin-3-yl-, Cyclohexyl-, 2-Oxo-imidazolidin-1-yl-, 2-Methyl-imidazol-1-yl-, 4-Methyl-imidazol-1-yl-, 4-Thiazol-2-yl-, 2,4-Dimethyl-imidazol-1-yl-, 4-Imidazol-1-yl-, 1,2,4-Triazol-1-yl-, 1-Aza-bicyclo[2.2.2]oct-3-yl-, 1-Methyl-piperidin-4-yl-methylsulfonyl-, 1*H*-Imidazol-4-yl-, 4-Ethoxycarbonylmethyl-piperazin-1-yl-, 1-Ethoxycarbonyl-piperidin-4-yl-, 4-*tert*-Butoxycarbonylmethyl-piperazin-1-yl-, 1-(2,2,2-Trifluorethyl)-piperidin-4-yl-, 4-Methylsulfonyl-piperazin-1-yl-, 2-Carboxy-4-methyl-piperazin-1-yl-, 3-Carboxy-4-methyl-piperazin-1-yl-, 2-Ethoxycarbonyl-4-methyl-piperazin-1-yl-, 3-Ethoxycarbonyl-4-methyl-piperazin-1-yl- oder 4-(2,2,2-Trifluorethyl)-piperazin-1-yl-gruppe,
   R⁵ ein Wasserstoffatom, eine Methylgruppe oder, wenn Y¹ ein Stickstoffatom bedeutet, auch ein freies Elektronenpaar, oder
   R⁴ und R⁵ zusammen, wenn Y¹ das Kohlenstoffatom darstellt, eine 1-Methylpiperidin-4-yliden-, Cyclohexyliden- oder Imidazolidin-2,4-dion-5-yliden-gruppe darstellen,
   R⁶ und R⁷ jeweils ein Wasserstoffatom oder eine Dimethylaminogruppe und
   R⁸ und R⁹ jeweils das Wasserstoffatom, eine Carboxy- oder Ethoxycarbonylgruppe darstellen,
wobei alle vorstehend genannten Alkylgruppen sowie die innerhalb der anderen Reste vorhandenen Alkylgruppen, sofern nichts anderes angegeben ist, 1 bis 7 Kohlenstoffatome umfassen und geradkettig oder verzweigt sein können und die vorstehend erwähnten aromatischen und heteroaromatischen Reste zusätzlich durch Fluor-, Chlor- oder Bromatome, durch Cyano- oder Hydroxygruppen mono-, di- oder trisubstituiert und die Substituenten gleich oder verschieden sein können,
bedeuten,
wobei bei allen voranstehend genannten Ausführungsformen jeweils denjenigen Verbindungen, in denen
(i) A ein Sauerstoffatom, eine Cyanimino- oder Phenylsulfonyliminogruppe,
   X ein Sauerstoffatom, eine Imino- oder Methylengruppe,
   U eine unverzweigte C₁₋₆-Alkyl-, C₂₋₄-Alkenyl- oder C₂₋₄-Alkinylgruppe, in der jede Methylengruppe mit bis zu 2 Fluoratomen und die Methylgruppe mit bis zu 3 Fluoratomen substituiert sein kann,
   V eine Amino- oder Hydroxygruppe und
   W ein Wasserstoff-, Chlor- oder Bromatom oder eine Trifluormethylgruppe bedeuten,
   eine herausragende Bedeutung zukommt,
   den Verbindungen, in denen
(ii) A ein Sauerstoffatom,
   X ein Sauerstoffatom, eine Imino- oder Methylengruppe,
   U eine Methyl-, Ethyl-, C₂₋₄-Alkenyl- oder C₂₋₄-Alkinylgruppe, in der die Methylengruppe mit bis zu 2 Fluoratomen und die Methylgruppe mit bis zu 3 Fluoratomen substituiert sein kann,
   V eine Amino- oder Hydroxygruppe und
   W ein Wasserstoff-, Chlor- oder Bromatom oder eine Trifluormethylgruppe bedeuten,
   eine besonders herausragende Bedeutung zukommt und
   den Verbindungen, in denen
(iii) A ein Sauerstoffatom,
   X ein Sauerstoffatom, eine Imino- oder Methylengruppe,
   U eine Trifluormethyl- oder Pentafluorethylgruppe,
   V eine Amino- oder Hydroxygruppe und
   W ein Wasserstoff-, Chlor- oder Bromatom oder eine Trifluormethylgruppe bedeuten,
eine ganz besonders herausragende Bedeutung zukommt.

Eine siebte Ausführungsform der vorliegenden Erfindung besteht in den Verbindungen der obigen allgemeinen Formel (I), in denen
A ein Sauerstoffatom, eine Cyanimino- oder Phenylsulfonyliminogruppe,
X ein Sauerstoffatom, eine Imino- oder Methylengruppe,
U eine unverzweigte C₁₋₆-Alkyl-, C₂₋₆-Alkenyl- oder C₂₋₆-Alkinylgruppe, in der jede Methylengruppe mit bis zu 2 Fluoratomen und die Methylgruppe mit bis zu 3 Fluoratomen substituiert sein kann,
V eine Amino- oder Hydroxygruppe,
W ein Wasserstoff-, Chlor- oder Bromatom oder eine Trifluormethylgruppe,
R¹ einen einfach ungesättigten 5- bis 7-gliedrigen Diaza- oder Triaza-Heterocyclus,
   wobei die vorstehend erwähnten Heterocyclen über ein Stickstoffatom verknüpft sind,
   eine Carbonylgruppe benachbart zu einem Stickstoffatom enthalten,
   zusätzlich an einem Kohlenstoffatom durch eine Phenylgruppe substituiert sein können und
   wobei eine olefinische Doppelbindung eines der vorstehend erwähnten ungesättigten Heterocyclen mit einem Phenyl-, Thienyl- oder Chinolin-Ring kondensiert sein kann,
   wobei die in R¹ enthaltenen Phenylgruppen sowie benzokondensierten Heterocyclen im Kohlenstoffgerüst zusätzlich durch Fluor-, Chlor-, Brom- oder lodatome, durch Methyl-, Methoxy-, Nitro-, Difluormethyl-, Trifluormethyl-, Hydroxy-, Amino-, Alkylamino-, Dialkylamino-, Acetylamino-, Acetyl-, Cyano- Difluormethoxy- oder Trifluormethoxygruppen mono-, di- oder trisubstituiert sein können, wobei die Substituenten gleich oder verschieden sein können, vorzugsweise jedoch unsubstituiert oder durch ein Fluor-, Chlor- oder Bromatom, durch eine Methyl- oder Methoxygruppe monosubstituiert sind,
R² das Wasserstoffatom oder
eine Phenylmethylgruppe oder eine C₂₋₇-Alkylgruppe, die in ω-Stellung durch eine Phenyl-, Pyridinyl-, Hydroxy-, Amino-, Alkylamino-, Dialkylamino-, Alkoxycarbonyl-, Carboxy-, Aminocarbonyl-, Aminocarbonylamino-, Acetylamino-, 1-Pyrrolidinyl-, 1-Piperidinyl-, 4-Morpholinyl-, [Bis-(2-hydroxyethyl)]aminogruppe substituiert sein kann,
   wobei die vorstehend erwähnten heterocyclischen Reste und Phenylgruppen zusätzlich im Kohlenstoffgerüst durch Fluor-, Chlor-, Brom- oder lodatome, durch Methyl-, Alkoxy-, Difluormethyl-, Trifluormethyl-, Hydroxy-, Amino-, C₁₋₃-Alkylamino-, Di-(C₁₋₃-alkyl)-amino-, Acetylamino-, Aminocarbonyl-, Cyano-, Difluormethoxy-, Trifluormethoxy-, Amino-C₁₋₃-alkyl-, C₁₋₃-Alkylamino-C₁₋₃-alkyl- oder Di-(C₁₋₃-Alkyl)-amino-C₁₋₃-alkylgruppen mono-, di- oder trisubstituiert und die Substituenten gleich oder verschieden sein können,
R³ das Wasserstoffatom oder eine C₁₋₃-Alkylgruppe,
   wobei die C₁₋₃-Alkylgruppe mit einer in R² vorhandenen Alkylgruppe oder einem in R² vorhandenen Phenyl- oder Pyridylring und dem Stickstoffatom, an dem sie gebunden sind, unter Ausbildung eines 5- bis 7-gliedrigen Ringes verbunden sein kann oder
R² und R³ zusammen mit dem eingeschlossenen Stickstoffatom einen Rest der allgemeinen Formel 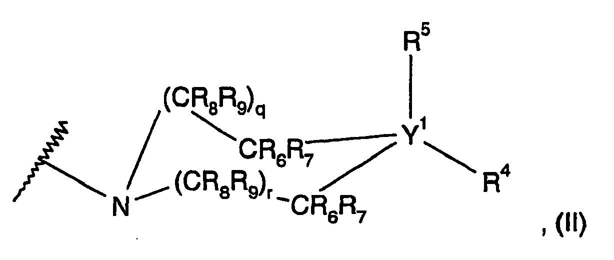 in der
   Y¹ das Kohlenstoffatom oder, wenn R⁵ ein freies Elektronenpaar darstellt, auch das Stickstoffatom,
   q und r, wenn Y¹ das Kohlenstoffatom darstellt, die Zahlen 0 oder 1 oder
   q und r, wenn Y¹ das Stickstoffatom darstellt, die Zahlen 1 oder 2,
   R⁴ das Wasserstoffatom, eine Hydroxy-, Amino-, Alkylamino-, C₃₋₆-Cycloalkylamino-, *N*-(C₃₋₆-Cycloalkyl)-alkylamino- oder Dialkylamino-, eine Alkyl-, Trifluormethyl-, C₃₋₆-Cycloalkyl-, Dialkylamino-C₂₋₇-alkyl-, Carboxyalkyl-, Alkoxycarbonylalkyl-, Alkylsulfonyl-, Alkylsulfonylamino- oder *N*-(Alkylsulfonyl)-alkylaminogruppe,
   oder auch, wenn Y¹ nicht das Stickstoffatom darstellt, die Carboxy- oder Dialkylaminomethylgruppe,
   eine Phenyl-, Phenyl-C₁₋₃-alkyl-, Pyridinyl- oder Diazinylgruppe, die jeweils durch ein Fluor-, Chlor- oder Bromatom, durch eine Trifluormethylcarbonyl-, Methyl- oder Methoxygruppe substituiert sein kann,
   eine gesättigte oder ein- oder mehrfach ungesättigte 4- bis 7-gliedrige Azacycloalkylgruppe, eine 5- bis 7-gliedrige Oxaza-, Diaza- oder Triazacycloalkylgruppe, eine 7- bis 9-gliedrige Azabicyclo- oder Diazabicycloalkylgruppe, eine 1-Alkyl-4-piperidinylamino- oder 1-Alkyl-4-piperidinylaminosulfonylgruppe,
   wobei die vorstehend genannten mono- und bicyclischen Heterocyclen über ein Stickstoff- oder ein Kohlenstoffatom gebunden sind,
   eine Methylengruppe der vorstehend genannten mono- und bicyclischen Heterocyclen durch eine Carbonyl- oder Sulfonylgruppe ersetzt sein kann,
   in den vorstehend genannten mono- und bicyclischen Heterocyclen eine nicht direkt an ein Stickstoff-, Sauerstoff- oder Schwefelatom gebundene Methylengruppe durch ein oder zwei Fluoratome substituiert sein kann,
   die vorstehend genannten mono- und bicyclischen Heterocyclen durch eine oder zwei C₁₋₃-Alkylgruppen, in der jede Methylengruppe mit bis zu 2 und jede Methylgruppe mit bis zu 3 Fluoratomen substituiert sein kann, oder/und
   durch eine C₃₋₆-Cycloalkyl-C₁₋₃-alkyl-, Benzyl-, C₁₋₄Alkanoyl-, Di-(C₁₋₃-alkyl)-amino- oder C₁₋₃-Alkylsulfonyl-, durch eine Alkoxycarbonyl-, Benzyloxycarbonyl-, Alkoxycarbonylalkyl-, Carboxy- oder Carboxyalkylgruppe substituiert sein können,
R⁵ ein Wasserstoffatom, eine C₁₋₃-Alkyl- oder Alkoxycarbonylgruppe oder,
wenn Y¹ ein Stickstoffatom darstellt, auch ein freies Elektronenpaar, oder
R⁴ und R⁵ zusammen, wenn Y¹ das Kohlenstoffatom darstellt, einen 5- bis 6-gliedrigen cycloaliphatischen Ring, in dem eine oder zwei Methylengruppen durch eine -NH- oder -N(Methyl)-Gruppe und eine oder zwei weitere Methylengruppen durch eine oder zwei Carbonylgruppen ersetzt sein können,
R⁶ und R⁷, die gleich oder verschieden sein können, jeweils das Wasserstoffatom oder eine C₁₋₃-Alkyl- oder Di-(C₁₋₃-alkyl)-aminogruppe und
R⁸ und R⁹, die gleich oder verschieden sein können, jeweils das Wasserstoffatom oder eine C₁₋₃-Alkyl-, Carboxy- oder C₁₋₃-Alkoxycarbonylgruppe darstellen, bedeuten,
wobei die vorstehend genannten Alkylgruppen oder die in den vorstehend genannten Resten enthaltenen Alkylgruppen, sofern nichts anderes angegeben wurde, 1 bis 7 Kohlenstoffatome enthalten und verzweigt oder unverzweigt sein können und die vorstehend erwähnten aromatischen und heteroaromatischen Reste zusätzlich durch Fluor-, Chlor- oder Bromatome, durch Cyano- oder Hydroxygruppen mono-, di- oder trisubstituiert und die Substituenten gleich oder verschieden sein können.

Eine achte Ausführungsform der vorliegenden Erfindung besteht in den Verbindungen der obigen allgemeinen Formel (1), in denen
A ein Sauerstoffatom,
X ein Sauerstoffatom, eine Imino- oder Methylengruppe,
U eine Methyl-, Ethyl-, C₂₋₄-Alkenyl- oder C₂₋₄-Alkinylgruppe, in der die Methylengruppe mit bis zu 2 Fluoratomen und die Methylgruppe mit bis zu 3 Fluoratomen substituiert sein können,
V eine Amino- oder Hydroxygruppe,
W ein Wasserstoff-, Chlor- oder Bromatom oder eine Trifluormethylgruppe,
R¹ eine 4-(2-Oxo-1,2,4,5-tetrahydro-1,3-benzdiazepin-3-yl)-, 4-(2-Oxo-1,4-dihydro-2*H*-chinazolin-3-yl)-, 4-(5-Oxo-3-phenyl-4,5-dihydro-1,2,4-triazol-1-yl), 4-(2-Oxo-1,2-dihydro-imidazo[4,5-c]chinolin-3-yl)-, 4-(2-Oxo-1,2-dihydro-4*H*-thieno[3,4-d]pyrimidin-3-yl)-, 4-(2-Oxo-1,4-dihydro-2*H*-thieno[3,2-d]pyrimidin-3-yl)-, 4-(5-Oxo-4,5,7,8-tetrahydro-2-thia-4,6-diaza-azulen-6-yl)-, 4-(2-Oxo-1,2,4,5-tetrahydro-thieno[3,2-d]-1,3-diazepin-3-yl)-, 4-(2-Oxo-1,2,4,5-tetrahydro-thieno-[2,3-d]-1,3-diazepin-3-yl)- oder 4-(2-Oxo-1,4-dihydro-2*H*-thieno[2,3-d]-pyrimidin-3-yl)-gruppe,
   wobei die vorstehend erwähnten mono- und bicyclischen Heterocyclen im Kohlenstoffgerüst zusätzlich durch eine Methoxygruppe monosubstituiert sein können,
R² eine Phenylmethylgruppe oder eine C₂₋₇-Alkylgruppe, die in ω-Stellung durch eine Phenyl-, Amino-, Alkylamino- oder Dialkylaminogruppe substituiert sein kann,
wobei die vorstehend genannte Phenylgruppe durch eine Amino-C₁₋₃-alkyl-, C₁₋₃-Alkylamino-C₁₋₃-alkyl- oder Di-(C₁₋₃-alkyl)-amino-C₁₋₃-alkylgruppe substituiert sein kann,
R³ das Wasserstoffatom oder eine C₁₋₃-Alkylgruppe,
R² und R³ zusammen mit dem Stickstoffatom, an dem sie gebunden sind, eine 7-Dimethylaminomethyl-1,2,4,5-tetrahydro-3-benzazepin-3-yl- oder 2-Amino-4,5,7,8-tetrahydro-thiazolo[4,5-d]azepin-6-yl-gruppe oder
R² und R³ zusammen mit dem eingeschlossenen Stickstoffatom einen Rest der allgemeinen Formel 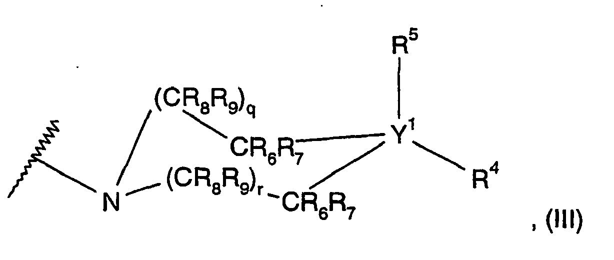 in der
   Y¹ das Kohlenstoffatom oder, wenn R⁵ ein freies Elektronenpaar darstellt, auch das Stickstoffatom,
   q und r, wenn Y¹ das Kohlenstoffatom darstellt, die Zahlen 0 oder 1 oder
   q und r, wenn Y¹ das Stickstoffatom darstellt, die Zahlen 1 oder 2,
   R⁴ das Wasserstoffatom,
   eine Phenyl-, Benzyl- oder Pyridinylgruppe, die jeweils durch ein Fluor-, Chlor- oder Bromatom, durch eine Trifluormethylcarbonyl-, Methyl- oder Methoxygruppe substituiert sein können,
   eine Hydroxy-, Carboxy-, Methyl-, Trifluormethyl-, *n*-Propyl-, Phenyl-, *p*-Tolyl-, *p*-Trifluormethylcarbonyl-phenyl-, *p*-(3-Dimethylaminopropyl)-phenyl-, Amino-, Benzyl-, *tert*-Butylamino-, Dimethylamino-, Diethylamino-, Diethylaminomethyl-, 2-Dimethylaminoethyl-, 2-Diethylaminoethyl-, 5-Aminopentyl-, Methoxycarbonyl-, Methoxycarbonylmethyl-, Perhydro-azepin-1-yl-, 4-Methyl-perhydro-l,4-diazepin-1-yl-, 1-Methyl-1-piperidinyl-4-yl-, 4-Piperazin-1-yl-, 4-Acetyl-piperazin-1-yl-, 4-Cyclopropylmethyl-piperazin-1-yl-, Pyrrolidin-1-yl-, 4-Ethyl-piperazin-1-yl-, 4-Isopropyl-piperazin-1-yl-, Piperidin-1-yl-, Piperidin-4-yl-, Morpholin-4-yl-, 4,4-Difluor-1-piperidin-1-yl-, 1-Methyl-1-aza-bicyclo[3.2.1]oct-4-yl-, 4-Methylpiperazin-1-yl-, 4-Ethylpiperazin-1-yl-, 1-Methyl-piperidin-1-yl-, 4-Carboxymethyl-piperazin-1-yl-, 1-Carboxymethyl-piperidin-4-yl-, 4-Benzyloxycarbonyl-piperazin-1-yl-, 1-Ethoxycarbonylmethyl-piperidin-4-yl-, Azetidin-1-yl-, 5-Methyl-2,5-diaza-bicyclo[2.2.1]hept-2-yl-, 1-Benzyl-piperidin-4-yl-, 4-Benzyl-piperazin-1-yl-, 4-Dimethylaminomethyl-1-phenyl-, 2,2,2-Trifluorethyl-piperazin-1-yl-, 1-Methylsulfonyl-piperidin-4-yl-, Piperidin-1-yl-methyl-, 1-Methyl-piperidin-4-yl-amino-, Methylsulfonylamino-, *N*-Methylsulfonyl-*N*-methylamino-, *N*-(Cyclopentyl)-methylamino-, 1,1-Dioxo-λ⁶-isothiazolidin-2-yl-, 2-Oxo-perhydro-1,3-oxazin-3-yl-, Cyclohexyl-, 2-Oxo-imidazolidin-1-yl-, 2-Methyl-imidazol-1-yl-, 4-Methyl-imidazol-1-yl-, 4-Thiazol-2-yl-, 2,4-Dimethyl-imidazol-1-yl-, 4-lmidazol-1-yl-, 1,2,4-Triazol-1-yl-, 1-Aza-bicyclo[2.2.2]oct-3-yl-, 1-Methyl-piperidin-4-yl-methylsulfonyl-, 1*H*-Imidazol-4-yl-, 4-Ethoxycarbonylmethyl-piperazin-1-yl-, 1-Ethoxycarbonyl-piperidin-4-yl-, 4-*tert*-Butoxycarbonylmethyl-piperazin-1-yl-, 1-(2,2,2-Trifluorethyl)-piperidin-4-yl-, 4-Methylsulfonyl-piperazin-1-yl-, 2-Carboxy-4-methyl-piperazin-1-yl-, 3-Carboxy-4-methyl-piperazin-1-yl-, 2-Ethoxycarbonyl-4-methyl-piperazin-1-yl-, 3-Ethoxycarbonyl-4-methyl-piperazin-1-yl- oder 4-(2,2,2-Trifluorethyl)-piperazin-1-yl-gruppe,
   R⁵ ein Wasserstoffatom, eine Methylgruppe oder, wenn Y¹ ein Stickstoffatom bedeutet, auch ein freies Elektronenpaar, oder
   R⁴ und R⁵ zusammen, wenn Y¹ das Kohlenstoffatom darstellt, eine 1-Methylpiperidin-4-yliden-, Cyclohexyliden- oder imidazolidin-2,4-dion-5-yliden-gruppe darstellen,
   R⁶ und R⁷ jeweils ein Wasserstoffatom oder eine Dimethylaminogruppe und
   R⁸ und R⁹ jeweils das Wasserstoffatom, eine Carboxy- oder Ethoxycarbonylgruppe darstellen, bedeuten
wobei alle vorstehend genannten Alkylgruppen sowie die innerhalb der anderen Reste vorhandenen Alkylgruppen, sofern nichts anderes angegeben ist, 1 bis 7 Kohlenstoffatome umfassen und geradkettig oder verzweigt sein können und die vorstehend erwähnten aromatischen und heteroaromatischen Reste zusätzlich durch Fluor-, Chlor- oder Bromatome, durch Cyano- oder Hydroxygruppen mono-, di- oder trisubstituiert und die Substituenten gleich oder verschieden sein können.

Als ganz besonders bevorzugte Verbindungen der obigen allgemeinen Formel (I) seien beispielsweise folgende genannt:

| | Struktur | Name |
|---|---|---|
| (1) | 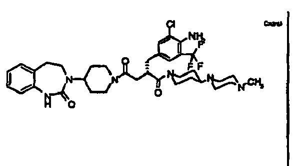 | (*S*)-2-(4-Amino-3-chlor-5-trifluor-methyl-benzyl)-1-[4-(4-methyl-piperazin-1-yl)-piperidin-1-yl]-4-[4-(2-oxo-1,2,4,5-tetrahydro-1,3-benzdiazepin-3-yl)-piperidin-1-yl]-butan-1,4-dion |
| (2) | 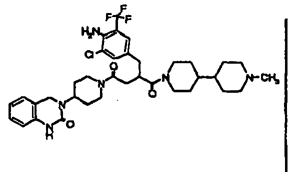 | 2-(4-Amino-3-chlor-5-trifluormethyl-benzyl)-1-(1'-methyl-[4,4']bipiperi-dinyl-1-yl)-4-[4-(2-oxo-1,4-dihydro-2*H*-chinazolin-3-yl)-piperidin-1-yl]-butan-1,4-dion |
| (3) | 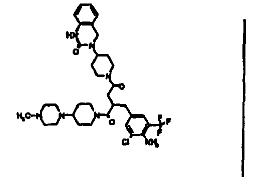 | 2-(4-Amino-3-chlor-5-trifluormethyl-benzyl)-1-[4-(4-methyl-piperazin-1-yl)-piperidin-1-yl]-4-[4-(2-oxo-1,4-dihydro-2*H*-chinazolin-3-yl)-piperidin-1-yl]-butan-1,4-dion |
| (4) | 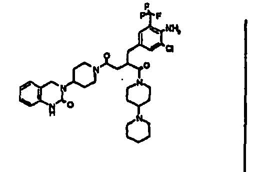 | 2-(4-Amino-3-chior-5-trifluormethyl-benzyi)-1-[1,4']bipiperidinyi-1'-yl-4-[4-(2-oxo-1,4-dihydro-2*H*-chinazolin-3-yl)-piperidin-1-yl]-butan-1,4-dion |
| (5) | 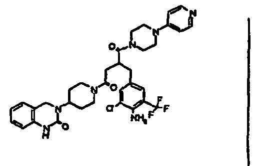 | 2-(4-Amino-3-chlor-5-trifluormethyl-benzyl)-4-[4-(2-oxo-1,4-dihydro-2*H*-chinazolin-3-yl)-piperidin-1-yl]-1-(4-pyridin-4-yl-piperazin-1-yl)-butan-1,4-dion |
| (6) | 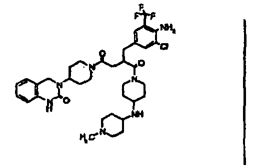 | 2-(4-Amino-3-chlor-5-trifluormethyl-benzyl)-1-[4-(1-methyl-piperidin-4-ylamino)-piperidin-1-yl]-4-[4-(2-oxo-1,4-dihydro-2*H*-chinazolin-3-yl)-piperidin-1-yl]-butan-1,4-dion |
| (7) | 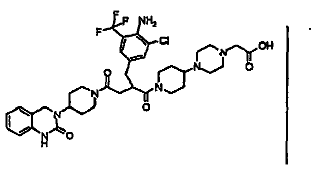 | [4-(1-{2-(4-Amino-3-chlor-5-trifluormethyl-benzyl)-4-oxo-4-[4-(2-oxo-1,4-dihydro-2*H*-chinazolin-3-yl)-piperidin-1-yl]-butyryl}-piperidin-4-yl)-piperazin-1-yl]-essigsäure |
| (8) | 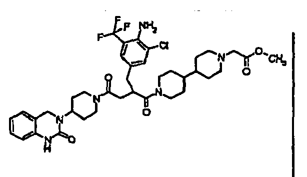 | (1'-{2-(4-Amino-3-chlor-5-trifluormethyl-benzyl)-4-oxo-4-[4-(2-oxo-1,4-dihydro-2*H*-chinazolin-3-yl)-pipeddin-1-yl]-butyryl}-[4,4']bipiperidinyl-1-yl)-essigsäuremethylester |
| (9) | 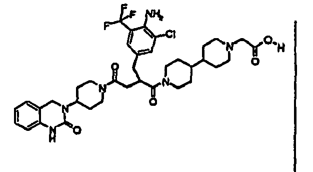 | (1'-{2-(4-Amino-3-chlor-5-trifluormethyl-benzyl)-4-oxo-4-[4-(2-oxo-1,4-dihydro-2*H*-chinazolin-3-yl)-piperidin-1-yl]-butyryl}-[4,4']bipiperidinyl-1-yl)-essigsäure |
| (10) | 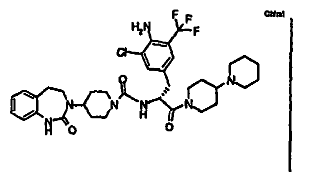 | 4-(2-Oxo-1,2,4,5-tetrahydro-1,3-benzdiazepin-3-yl)-piperidin-1-carbonsäure-[(*R*)-1-(4-amino-3-chlor-5-triftuormethyl-benzyl)-2-1,4'-bipiperidinyl-1'-yl-2-oxo-ethyl]-amid |
| (11) | 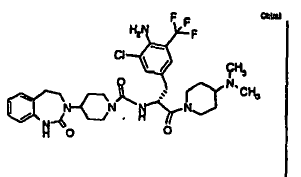 | 4-(2-Oxo-1,2,4,5-tetrahydro-1,3-benzdiazepin-3-yl)-piperidin-1-carbonsäure-[(*R*)-1-(4-amino-3-chlor-5-trifluormethyl-benzyl)-2-(4-dimethylamino-piperidin-1-yl)-2-oxo-ethyl]-amid |
| (12) | 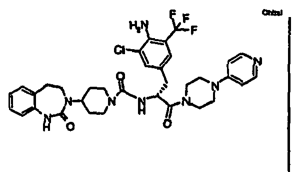 | 4-(2-Oxo-1,2,4,5-tetrahydro-1,3-benzdiazepin-3-yl)-piperidin-1-carbonsäure-[(*R*)-1-(4-amino-3-chlor-5-trifluormethyl-benzyl)-2-oxo-2-(4-pyridin-4-yl-piperazin-1-yl)-ethyl]-amid |
| (13) | 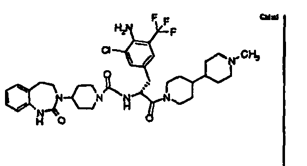 | 4-(2-Oxo-1,2,4,5-tetrahydro-1,3-benzdiazepin-3-yl)-piperidin-1-carbonsäure-[(*R*)-1-(4-amino-3-chlor-5-trifluormethyl-benzyl)-2-(1'-methyl-4,4'-bipiperidinyl-1-yl)-2-oxo-ethyl]-amid |
| (14) | 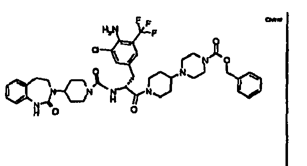 | 4-[1-((*R*)-3-(4-Amino-3-chlor-5-trifluormethyl-phenyl)-2-{[4-(2-oxo-1,2,4,5-tetrahydro-1,3-benzdiazepin-3-yl)-piperidin-1-carbonyl]-amino}-propionyl)-piperidin-4-yl]-piperazin-1-carbonsäurebenzylester |
| (15) | 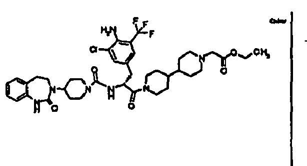 | [1'-((*R*)-3-(4-Amino-3-chlor-5-tri-fluormethyl-phenyl)-2-{[4-(2-oxo-1,2,4,5-tetrahydro-1,3-benzdiazepin-3-yl)-piperidin-1-carbonyl]-amino}-propionyl)-4,4'-bipiperidinyl-1-yl]-essigsäureethylester |
| (16) | 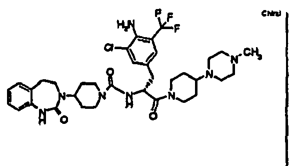 | 4-(2-Oxo-1,2,4,5-tetrahydro-1,3-benzdiazepin-3-yl)-piperidin-1-carbonsäure-{(*R*)-1-(4-amino-3-chlor-5-trifluormethyl-benzyl)-2-[4-(4-methyl-piperazin-1-yl)-piperidin-1-yl]-2-oxo-ethyl}-amid |
| (17) | 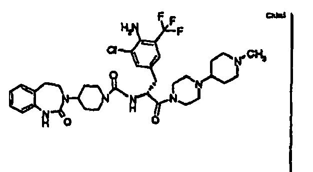 | 4-(2-Oxo-1,2,4,5-tetrahydro-1,3-benzdiazepin-3-yl)-piperidin-1-carbonsäure-{(*R*)-1-(4-amino-3-chlor-5-trifluormethyl-benzyl)-2-[4-(1-methyl-piperidin-4-yl)-piperazin-1-yl]-2-oxo-ethyl}-amid |
| (18) | 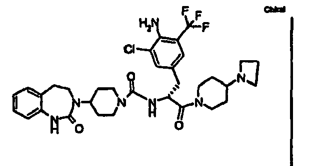 | 4-(2-Oxo-1,2,4,5-tetrahydro-1,3-benzdiazepin-3-yl)-piperidin-1-carbonsäure-[(*R*)-1-(4-amino-3-chlor-5-trifluormethyl-benzyl)-2-(4-azetidin-1-yl-piperidin-1-yl)-2-oxo-ethyl]-amid |
| (19) | 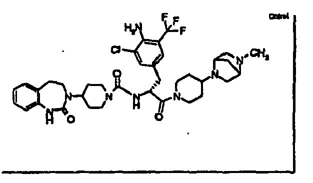 | 4-(2-Oxo-1,2,4,5-tetrahydro-1,3-benzdiazepin-3-yl)-piperidin-1-carbonsäure-{(*R*)-1-(4-amino-3-chlor-5-trifluormethyl-benzyl)-2-[4-(5-methyl-2,5-diaza bicyclo[2.2.1]-hept-2-yl)-piperidin-1-yl]-2-oxo-ethyl}-amid |
| (20) | 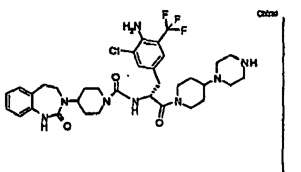 | 4-(2-Oxo-1,2,4,5-tetrahydro-1,3-benzdiazepin-3-yl)-piperidin-1-carbonsäure-[(*R*)-1-(4-amino-3-chlor-5-trifluormethyl-benzyl)-2-oxo-2-(4-piperazin-1-yl-piperidin-1-yl)-ethyl]-amid |
| (21) | 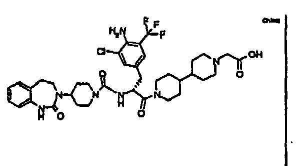 | [1'-((*R*)-3-(4-Amino-3-chlor-5-trifluormethyl-phenyl)-2-{[4-(2-oxo-1,2,4,5-tetrahydro-1,3-benzdiazepin-3-yl)-piperidin-1-carbonyl]-amino}-propionyl)-4,4'-bipiperidinyl-1-yl]-essigsäure |
| (22) | 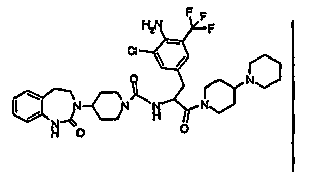 | 4-(2-Oxo-1,2,4,5-tetrahydro-1,3-benzdiazepin-3-yl)-piperidin-1-carbonsäure-[1-(4-amino-3-chlor-5-trifluormethyl-benzyl)-2-1,4'-bipiperidinyl-1'-yl-2-oxo-ethyl]-amid |
| (23) | 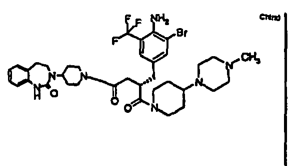 | (*S*)-2-(4-Amino-3-brom-5-trifluormethyl-benzyl)-1-[4-(4-methyl-piperazin-1-yl)-piperidin-1-yl]-4-[4-(2-oxo-1,2,4,5-tetrahydro-1,3-benzdiazepin-3-yl)-piperidin-1-yl]-butan-1,4-dion |
| (24) | 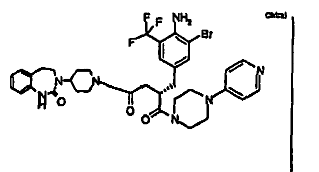 | (*S*)-2-(4-Amino-3-brom-5-trifluormethyl-benzyl)-4-[4-(2-oxo-1,2,4,5-tetrahydro-1,3-benzdiazepin-3-yl)-piperidin-1-yl]-1-(4-pyridin-4-yl-piperazin-1-yl)-butan-1,4-dion |
| (25) | 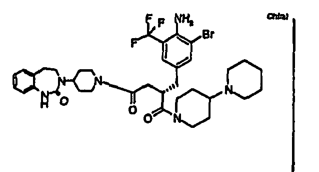 | (*S*)-2-(4-Amino-3-brom-5-trifluormethyl-benzyl)-1-1,4'-bipiperidinyl-1'-yl-4-[4-(2-oxo-1,2,4,5-tetrahydro-1,3-benzdiazepin-3-yl)-piperidin-1-yl]-butan-1,4-dion |
| (26) | 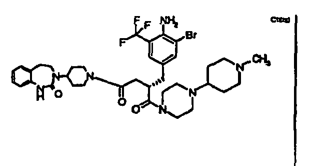 | (*S*)-2-(4-Amino-3-brom-5-trifluormethyl-berizyl)-1-[4-(1-methyl-piperidin-4-yl)-piperazin-1-yl]-4-[4-(2-oxo-1,2,4,5-tetrahydro-1,3-benzdiazepin-3-yl)-piperidin-1-yl]-butan-1,4-dion |
| (27) | 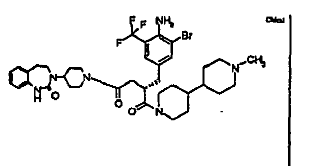 | (*S*)-2-(4-Amino-3-brom-5-trifluormethyl-benzyl)-1-(1'-methyl-4,4'-bipiperidinyl-1-yl)-4-[4-(2-oxo-1,2,4,5-tetrahydro-1,3-benzdiazepin-3-yl)-piperidin-1-yl]-butan-1,4-dion |
| (28) | 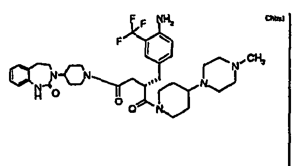 | (*S*)-2-(4-Amino-3-trifluormethyl-benzyl)-1-[4-(4-methyl-piperazin-1-yl)-piperidin-1-yl]-4-[4-(2-oxo-1,2,4,5-tetrahydro-1,3-benzdiazepin-3-yl)-piperidin-1-yl]-butan-1,4-dion |
| (29) | 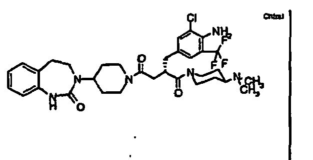 | (*S*)-2-(4-Amino-3-chlor-5-trifluormethyl-benzyl)-1-(4-dimethylamino-piperidin-1-yl)-4-[4-(2-oxo-1,2,4,5-tetrahydro-1,3-benzdiazepin-3-yl)-piperidin-1-yl]-butan-1,4-dion |
| (30) | 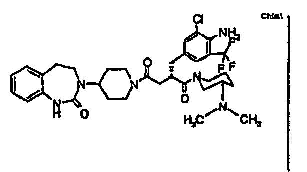 | (*S*)-2-(4-Amino-3-chlor-5-trifluormethyl-benzyl)-1-(3-dimethylamino-piperidin-1-yl)-4-[4-(2-oxo-1,2,4,5-tetrahydro-1,3-benzdiazepin-3-yl)-piperidin-1-yl]-butan-1,4-dion |
| (31) | 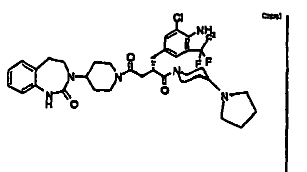 | (*S*)-2-(4-Amino-3-chlor-5-trifluormethyl-benzyl)-4-[4-(2-oxo-1,2,4,5-tetrahydro-1,3-benzdiazepin-3-yl)-piperidin-1-yl]-1-(4-pyrrolidin-1-yl-piperidin-1-yl)-butan-1,4-dion |
| (32) | 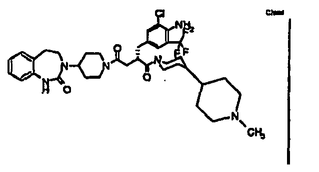 | (*S*)-2-(4-Amino-3-chlor-5-trifluormethyl-benzyl)-1-(1'-methyl-4,4'-bipiperidinyl-1-yl)-4-[4-(2-oxo-1,2,4,5-tetrahydro-1,3-benzdiazepin-3-yl)-piperidin-1-yl]-butan-1,4-dion |
| (33) | 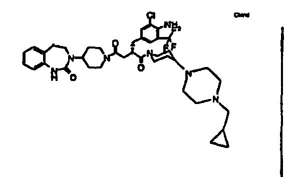 | (*S*)-2-(4-Amino-3-chlor-5-trifluormethyl-benzyl)-1-[4-(4-cyclopropyl-methyl-piperazin-1-yl)-piperidin-1-yl]-4-[4-(2-oxo-1,2,4,5-tetrahydro-1,3-benzdiazepin-3-yl)-piperidin-1-yl]-butan-1,4-dion |
| (34) | 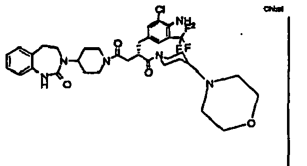 | (*S*)-2-(4-Amino-3-chlor-5-trifluormethyl-benzyl)-1-(4-morpholin-4-yl-piperidin-1-yl)-4-[4-(2-oxo-1,2,4,5-tetrahydro-1,3-benzdiazepin-3-yl)-piperidin-1-yl]-butan-1,4-dion |
| (35) | 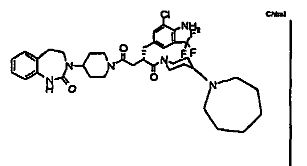 | (*S*)-2-(4-Amino-3-chlor-5-trifluormethyl-benzyl)-4-[4-(2-oxo-1,2,4,5-tetrahydro-1,3-benzdiazepin-3-yl)-piperidin-1-yl]-1-(4-perhydroazepin-1-yl-piperidin-1-yl)-butan-1,4-dion |
| (36) | 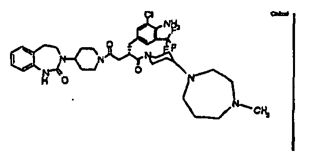 | (*S*)-2-(4-Amino-3-chlor-5-trifluormethyl-benzyl)-1-[4-(4-methyl-perhydro-1,4-diazepin-1-yl)-piperidin-1-yl]-4-[4-(2-oxo-1,2,4,5-tetrahydro-1,3-benzdiazepin-3-yl)-piperidin-1-yl]-butan-1,4-dion |
| (37) | 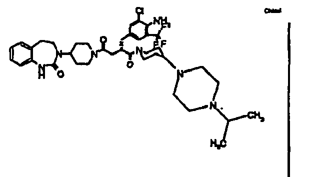 | (*S*)-2-(4-Amino-3-chlor-5-trifluormethyl-benzyl)-1-[4-(4-isopropyl-piperazin-1-yl)-piperidin-1-yl]-4-[4-(2-oxo-1,2,4,5-tetrahydro-1,3-benzdiazepin-3-yl)-piperidin-1-yl]-butan-1,4-dion |
| (38) | 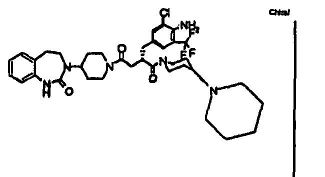 | (*S*)-2-(4-Amino-3-chlor-5-trifluormethyl-benzyl)-1-1,4'-bipiperidinyl-1'-yl-4-[4-(2-oxo-1,2,4,5-tetrahydro-1,3-benzdiäzepin-3-yl)-piperidin-1-yl]-butan-1,4-dion |
| (39) | 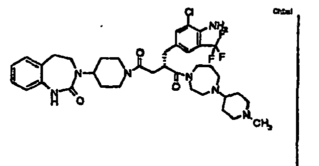 | (*S*)-2-(4-Amino-3-chlor-5-trifluormethyl-benzyl)-1-[4-(1-methyl-piperidin-4-yl)-perhydro-1,4-diazepin-1-yl]-4-[4-(2-oxo-1,2,4,5-tetrahydro-1 ,3-benzdiazepin-3-yl)-piperidin-1-yl]-butan-1,4-dion |
| (40) | 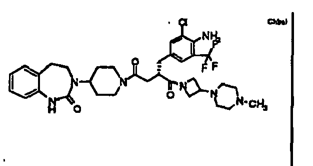 | (*S*)-2-(4-Amino-3-chlor-5-trifluormethyl-benzyl)-1-[3-(4-methyl-piperazin-1-yl)-azetidin-1-yl]-4-[4-(2-oxo-1,2,4,5-tetrahydro-1,3-benzdiazepin-3-yl)-piperidin-1-yl]-butan-1,4-dion |
| (41) | 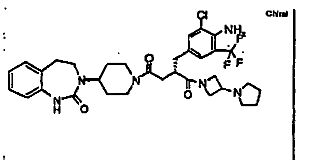 | (*S*)-2-(4-Amino-3-chlor-5-trifluormethyl-benzyl)-4-[4-(2-oxo-1,2,4,5-tetrahydro-1,3-benzdiazepin-3-yl)-piperidin-1-yl]-1-(3-pyrrolidin-1-yl-azetidin-1-yl)-butan-1,4-dion |
| (42) | 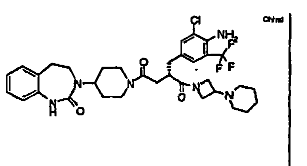 | (*S*)-2-(4-Amino-3-chlor-5-trifluormethyl-benzyl)-4-[4-(2-oxo-1,2,4,5-tetrahydro-1,3-benzdiazepin-3-yl)-piperidin-1-yl]-1-(3-piperidin-1-yl-azetidin-1-yl)-butan-1,4-dion |
| (43) | 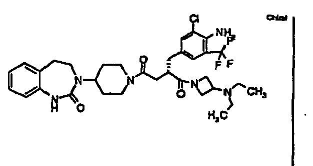 | (*S*)-2-(4-Amino-3-chlor-5-trifluormethyl-benzyl)-1-(3-diethylamino-azetidin-1-yl)-4-[4-(2-oxo-1,2,4,5-tetrahydro-1,3-benzdiazepin-3-yl)-piperidin-1-yl]-butan-1,4-dion |
| (44) | 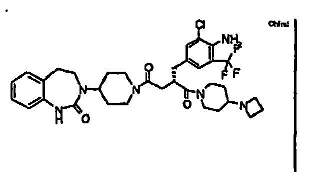 | (*S*)-2-(4-Amino-3-chlor-5-trifluormethyl-benzyl)-1-(4-azetidin-1-yl-piperidin-1-yl)-4-[4-(2-oxo-1,2,4,5-tetrahydro-1,3-benzdiazepin-3-yl)-piperidin-1-yl]-butan-1,4-dion |
| (45) | 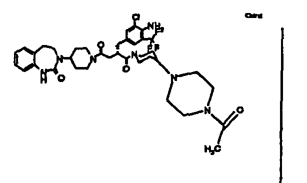 | (*S*)-1 -[4-(4-Acetyl-piperazin-1-yl)-piperidin-1-yl]-2-(4-amino-3-chlor-5-trifluormethyl-benzyl)-4-[4-(2-oxo-1,2,4,5-tetrahydro-1,3-benzdiazepin-3-yl)-piperidin-1-yl]-butan-1,4-dion |
| (46) | 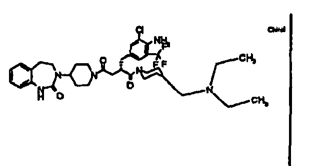 | (*S*)-2-(4-Amino-3-chlor-5-trifluormethyl-benzyl)-1-(4-diethylamino-methyl-piperidin-1-yl)-4-[4-(2-oxo-1,2,4,5-tetrahydro-1,3-benzdiazepin-3-yl)-piperidin-1-yl]-butan-1,4-dion |
| (47) | 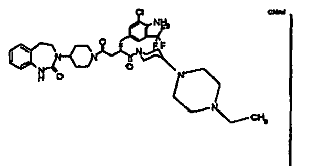 | (*S*)-2-(4-Amino-3-chlor-5-trifluormethyl-benzyl)-1-[4-(4-ethyl-piperazin-1-yl)-piperidin-1-yl]-4-[4-(2-oxo-1,2,4,5-tetrahydro-1,3-benzdiazepin-3-yl)-piperidin-1-yl]-butan-1,4-dion |
| (48) | 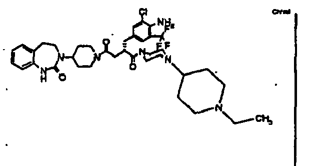 | (*S*)-2-(4-Amino-3-chlor-5-trifluormethyl-benzyl)-1-[4-(1-ethyl-piperidin-4-yl)-piperazin-1-yl]-4-[4-(2-oxo-1,2,4,5-tetrahydro-1,3-benzdiazepin-3-yl)-piperidin-1-yl]-butan-1,4-dion |
| (49) | 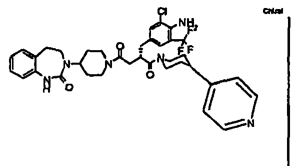 | (*S*)-2-(4-Amino-3-chlor-5-trifluormethyl-benzyl)-4-[4-(2-oxo-1,2,4,5-tetrahydro-1,3-benzdiazepin-3-yl)-piperidin-1-yl]-1-(3,4,5,6-tetrahydro-2*H*-4,4'-bipyridinyl-1-yl)-butan-1,4-dion |
| (50) | 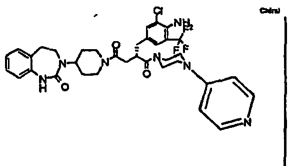 | (*S*)-2-(4-Amino-3-chlor-5-trifluormethyl-benzyl)-4-[4-(2-oxo-1,2,4,5-tetrahydro-1,3-benzdiazepin-3-yl)-piperidin-1-yl]-1-(4-pyridin-4-yl-piperazin-1-yl)-butan-1,4-dion |
| (51) | 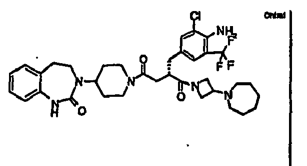 | (*S*)-2-(4-Amino-3-chlor-5-trifluormethyl-benzyl)-4-[4-(2-oxo-1,2,4,5-tetrahydro-1,3-benzdiazepin-3-yl)-piperidin-1-yl]-1-(3-perhydroazepin-1-yl-azetidin-1-yl)-butan-1,4-dion |
| (52) | 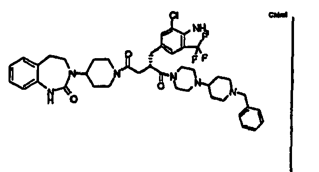 | (*S*)-2-(4-Amino-3-chlor-5-trifluormethyl-benzyl)-1-[4-(1-benzyl-piperidin-4-yl)-piperazin-1-yl]-4-[4-(2-oxo-1,2,4,5-tetrahydro-1,3-benzdiazepin-3-yl)-piperidin-1-yl]-butan-1,4-dion |
| (53) | 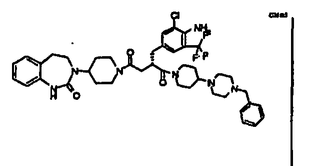 | (*S*)-2-(4-Amino-3-chlor-5-trifluormethyl-benzyl)-1-[4-(4-benzyl-piperazin-1-yl)-piperidin-1-yl]-4-[4-(2-oxo-1,2,4,5-tetrahydro-1,3-benzdiazepin-3-yl)-piperidin-1-yl]-butan-1,4-dion |
| (54) | 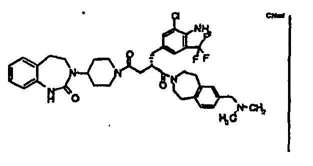 | (*S*)-2-(4-Amino-3-chlor-5-trifluormethyl-benzyl)-1-(7-dimethylamino-methyl-1,2,4,5-tetrahydro-3-benzazepin-3-yl)-4-[4-(2-oxo-1,2,4,5-tetrahydro-1,3-benzdiazepin-3-yl)-piperidin-1-yl]-butan-1,4-dion |
| (55) | 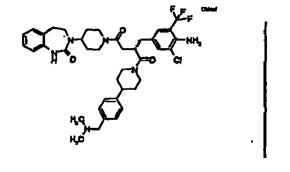 | (*S*)-2-(4-Amino-3-chlor-5-trifluormethyl-benzyl)-1-[4-(4-dimethyl-aminomethyl-phenyl)-piperidin-1-yl]-4-[4-(2-oxo-1,2,4,5-teträhydro-1,3-benzdiazepin-3-yl)-piperidin-1-yl]-butan-1,4-dion |
| (56) | 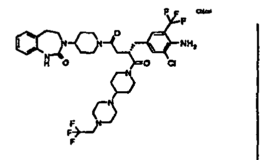 | (*S*)-2-(4-Amino-3-chlor-5-trifluormethyl-benzyl)-4-[4-(2-oxo-1,2,4,5-tetrahydro-1,3-benzdiazepin-3-yl)-piperidin-1-yl]-1-{4-[4-(2,2,2-trifluorethyl)-piperazin-1-yl]-piperidin-1-yl}-butan-1,4-dion |
| (57) | 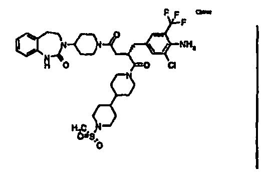 | (*S*)-2-(4-Amino-3-chlor-5-trifluormethyl-benzyl)-1-(1'-methansulfonyl-4,4'-bipiperidinyl-1-yl)-4-[4-(2-oxo-1,2,4,5-tetrahydro-1,3-benzdiazepin-3-yl)-piperidin-1-yl]-butan-1,4-dion |
| (58) | 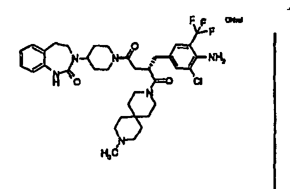 | (*S*)-2-(4-Amino-3-chlor-5-trifluormethyl-benzyl)-1-(9-methyl-3,9-diaza-spiro[5.5]undec-3-yl)-4-[4-(2-oxo-1,2,4,5-tetrahydro-1,3-benzdiazepin-3-yl)-piperidi n-1-yl]-butan-1,4-dion |
| (59) | 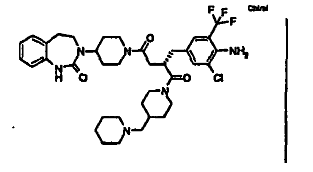 | (*S*)-2-(4-Amino-3-chlor-5-trifluormethyl-benzyl)-4-[4-(2-oxo-1,2,4,5-tetrahydro-1,3-benzdiazepin-3-yl)-piperidin-1-yl]-1-(4-piperidin-1-ylmethyl-piperidin-1-yl)-butan-1,4-dion |
| (60) | 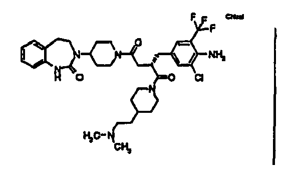 | (*S*)-2-(4-Amino-3-chlor-5-trifluormethyl-benzyl)-1-[4-(2-dimethylamino-ethyl)-piperidin-1-yl]-4-[4-(2-oxo-1,2,4,5-tetrahydro-1,3-benzdiazepin-3-yl)-piperidin-1-yl]-butan-1,4-dion |
| (61) | 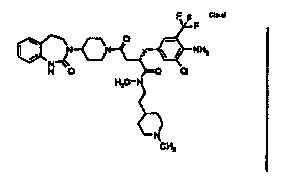 | (*S*)-2-(4-Amino-3-chlor-5-trifluormethyl-benzyl)-Mmethyl-N-[2-(1-methyl-piperidin-4-yl)-ethyl]-4-oxo-4-[4-(2-oxo-1,2,4,5-tetrahydro-1,3-benzdiazepin-3-yl)-piperidin-1-yl]-butyramid |
| (62) | 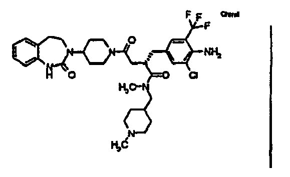 | (*S*)-2-(4-Amino-3-chlor-5-trifluormethyl-benzyl)-*N*-methyl-(1-methyl-piperidin-4-ylmethyl)-4-oxo-4-[4-(2-oxo-1,2,4,5-tetrahydro-1,3-benzdiazepin-3-yl)-piperidin-1-yl]-butyramid |
| (63) | 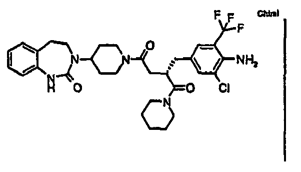 | (*S*)-2-(4-Amino-3-chlor-5-trifluormethyl-benzyl)-4-[4-(2-oxo-1,2,4,5-tetrahydro-1,3-benzdiazepin-3-yl)-piperidin-1-yl]-1-piperidin-1-yl-butan-1,4-dion |
| (64) | 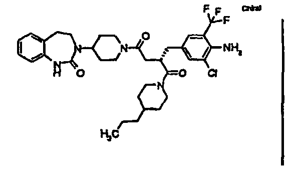 | (*S*)-2-(4-Amino-3-chlor-S-trifluormethyl-benzyl)-4-[4-(2-oxo-1,2,4,5-tetrahydro-1,3-benzdiazepin-3-yl)-piperidin-1-yl]-1-(4-propyl-piperidin-1-yl)-butan-1,4-dion |
| (65) | 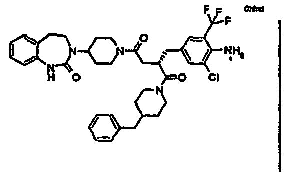 | (*S*)-2-(4-Amino-S-chlor-5-trifluormethyl-benzyl)-1-(4-benzyl-piperidin-1-yl)-4-[4-(2-oxo-1,2,4,5-tetrahydro-1,3-benzdiazepin-3-yl)-piperidin-1-yl]-butan-1,4-dion |
| (66) | 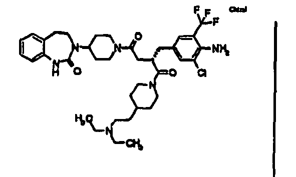 | (*S*)-2-(4-Amino-3-chlor-5-trifluormethyl-benzyl)-1-[4-(2-diethylamino-ethyl)-piperidin-1-yl]-4-[4-(2-oxo-1,2,4,5-tetrahydro-1,3-benzdiazepin-3-yl)-piperidin-1-yl]-butan-1,4-dion |
| (67) | 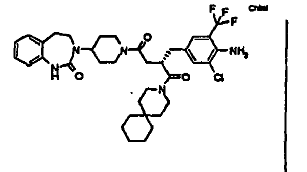 | (*S*)-2-(4-Amino-3-chlor-5-trifluormethyl-benzyl)-1-(3-aza-spiro[5.5]-undec-3-yl)-4-[4-(2-oxo-1,2,4,5-tetrahydro-1,3-benzdiazepin-3-yl)-piperidin-1-yl]-butan-1,4-dion |
| (68) | 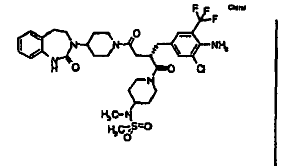 | *N*-(1-{(*S*)-2-(4-Amino-3-chlor-5-trifluormethyl-benzyl)-4-oxo-4-[4-(2-oxo-1,2,4,5-tetrahydro-1,3-benzdiazepin-3-yl)-piperidin-1-yl]-butyryl}-piperidin-4-yl)-N-methyl-methan-sulfonamid |
| (69) | 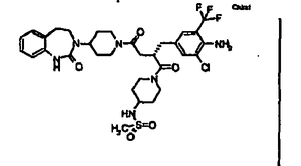 | *N*-(1-{(*S*)-2-(4-Amino-3-chlor-5-trifluormethyl-benzyl)-4-oxo-4-[4-(2-oxo-1,2,4,5-tetrahydro-1,3-benzdiazepin-3-yl)-piperidin-1-yl]-butyryl}-piperidin-4-yl)-methansulfonamid |
| (70) | 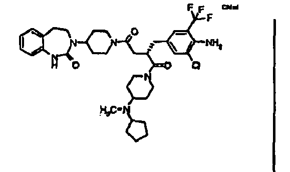 | (*S*)-2-(4-Amino-3-chlor-5-trifluormethyl-benzyl)-1-[4-(cyclopentyl-methyl-amino)-piperidin-1-yl]-4-[4-(2-oxo-1,2,4,5-tetrahydro-1,3-benzdiazepin-3-yl)-piperidin-1-yl]-butan-1,4-dion |
| (71) | 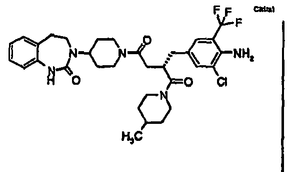 | (*S*)-2-(4-Amino-3-chlor-5-trifluormethyl-benzyl)-1-(4-methyl-piperidin-1-yl)-4-[4-(2-oxo-1,2,4,5-tetrahydro-1,3-benzdiazepin-3-yl)-piperidin-1-yl]-butan-1,4-dion |
| (72) | 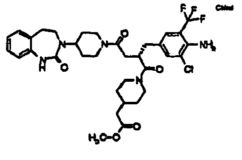 | (1-{(*S*)-2-(4-Amino-3-chlor-5-trifluormethyl-benzyl)-4-oxo-4-[4-(2-oxo-1,2,4,5-tetrahydro-1,3-benzdiazepin-3-yl)-piperidin-1-yl]-butyryl}-piperidin-4-yl)-essigsäuremethylester |
| (73) | 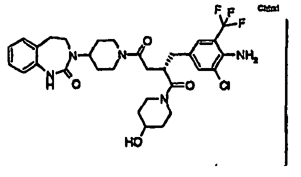 | (*S*)-2-(4-Amino-3-chlor-5-trifluormethyl-benzyl)-1-(4-hydroxy-piperidin-1-yl)-4-[4-(2-oxo-1,2,4,5-tetrahydro-1,3-benzdiazepin-3-yl)-piperidin-1-yl]-butan-1,4-dion |
| (74) | 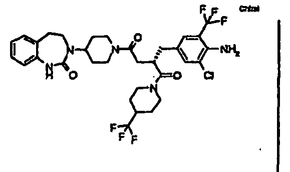 | (*S*)-2-(4-Amino-3-chlor-5-trifluormethyl-benzyl)-4-[4-(2-oxo-1,2,4,5-tetrahydro-1,3-benzdiazepin-3-yl)-piperidin-1-yl]-1-(4-trifluormethyl-piperidin-1-yl)-butan-1,4-dion |
| (75) | 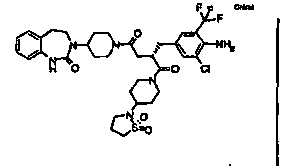 | (*S*)-2-(4-Amino-3-chlor-5-trifluormethyl-benzyl)-1-[4-(1,1-dioxo-λ⁶-isothiazolidin-2-yl)-piperidin-1-yl]-4-[4-(2-oxo-1,2,4,5-tetrahydro-1,3-benzdiazepin-3-yl)-piperidin-1-yl]-butan-1,4-dion |
| (76) | 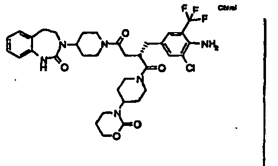 | (*S*)-2-(4-Amino-3-chlor-5-trifluormethyl-benzyl)-1-[4-(2-oxo-perhydro-1,3-oxazin-3-yl)-piperidin-1-yl]-4-[4-(2-oxo-1,2,4,5-tetrahydro-1,3-benzdiazepin-3-yl)-piperidin-1-yl]-butan-1,4-dion |
| (77) | 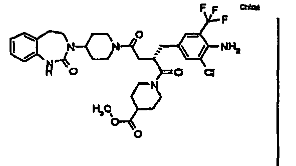 | 1-{(*S*)-2-(4-Amino-3-chlor-5-trifluormethyl-benzyl)-4-oxo-4-[4-(2-oxo-1,2,4,5-tetrahydro-1,3-benzdiazepin-3-yl)-piperidin-1-yl]-butyryl}-piperidin-4-carbonsäuremethylester |
| (78) | 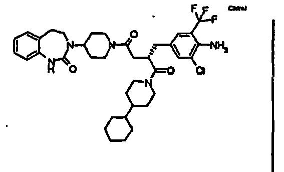 | (*S*)-2-(4-Amino-3-chlor-5-trifluormethyl-benzyl)-1-(4-cyclohexyl-piperidin-1-yl)-4-[4-(2-oxo-1,2,4,5-tetrahydro-1,3-benzdiazepin-3-yl)-piperidin-1-yl]-butan-1,4-dion |
| (79) | 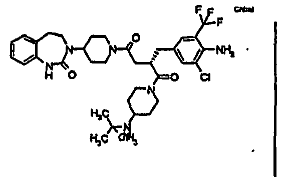 | (*S*)-2-(4-Amino-3-chlor-5-trifluormethyl-benzyl)-1-(4-tert-butylamino-piperidin-1-yl)-4-[4-(2-oxo-1,2,4,5-tetrahydro-1,3-benzdiazepin-3-yl)-piperidin-1-yl]-butan-1,4-dion |
| (80) | 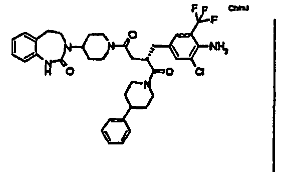 | (*S*)-2-(4-Amino-3-chlor-5-trifluormethyl-benzyl)-4-[4-(2-oxo-1,2,4,5-tetrahydro-1,3-benzdiazepin-3-yl)-piperidin-1-yl]-1-(4-phenyl-piperidin-1-yl)-butan-1,4-dion |
| (81) | 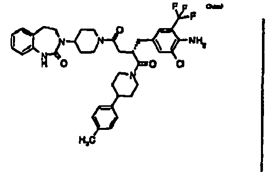 | (*S*)-2-(4-Amino-3-chlor-5-trifluormethyl-benzyl)-4-[4-(2-oxo-1,2,4,5-tetrahydro-1,3-benzdiazepin-3-yl)-piperidin-1-yl]-1-(4-*p*-tolyl-piperidin-1-yl)-butan-1,4-dion |
| (82) | 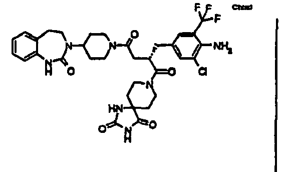 | 8-{(*S*)-2-(4-Amino-3-chlor-5-trifluormethyl-benzyl)-4-oxo-4-[4-(2-oxo-1,2,4,5-tetrahydro-1,3-benzdiazepin-3-yl)-piperidin-1-yl]-butyryl}-1,3,8-triaza-spiro[4.5]decan-2,4-dion |
| (83) | 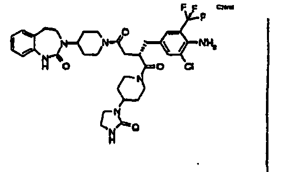 | (*S*)-2-(4-Amino-3-chlor-5-trifluormethyl-benzyl)-1-[4-(2-oxo-imidazolidin-1-yl)-piperidin-1-yl]-4-[4-(2-oxo-1,2,4,5-tetrahydro-1,3-benzdiazepin-3-yl)-piperidin-1-yl]-butan-1,4-dion |
| (84) | 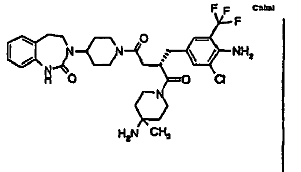 | (*S*)-2-(4-Amino-3-chlor-5-trifluormethyl-benzyl)-1-(4-amino-4-methyl-piperidin-1-yl)-4-[4-(2-oxo-1,2,4,5-tetrahydro-1,3-benzdiazepin-3-yl)-piperidin-1-yl]-butan-1,4-dion |
| (85) | 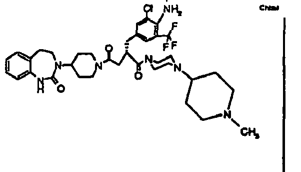 | (*S*)-2-(4-Amino-3-chlor-5-trifluormethyl-benzyl)-1-[4-(1-methyl-piperidin-4-yl)-piperazin-1-yl]-4-[4-(2-oxo-1,2,4,5-tetrahydro-1,3-benzdiazepin-3-yl)-piperidin-1-yl]-butan-1,4-dion |
| (86) | 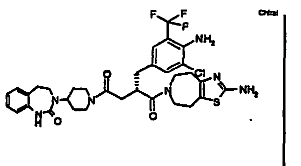 | (*S*)-2-(4-Amino-3-chlor-5-trifluormethyl-benzyl)-1-(2-amino-4,5,7,8-tetrahydro-thiazolo[4,5-d]azepin-6-yl)-4-[4-(2-oxo-1,2,4,5-tetrahydro-1,3-benzdiazepin-3-yl)-piperidin-1-yl]-butan-1,4-dion |
| (87) | 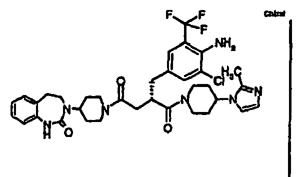 | (*S*)-2-(4-Amino-3-chlor-5-trifluormethyl-benzyl)-1-[4-(2-methyl-imidazol-1-yl)-piperidin-1-yl]-4-[4-(2-oxo-1,2,4,5-tetrahydro-1,3-benzdiazepin-3-yl)-piperidin-1-yl]-butan-1,4-dion |
| (88) | 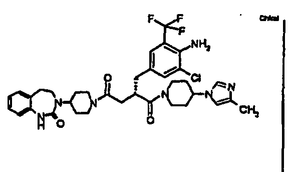 | (*S*)-2-(4-Amino-3-chlor-5-trifluormethyl-benzyl)-1-[4-(4-methyl-imidazol-1-yl)-piperidin-1-yl]-4-[4-(2-oxo-1,2,4,5-tetrahydro-1,3-benzdiazepin-3-yl)-piperidin-1-yl]-butan-1,4-dion |
| (89) | 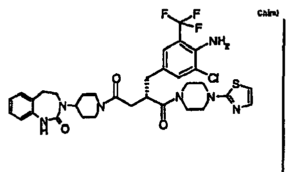 | (*S*)-2-(4-Amino-3-chlor-5-trifluormethyl-benzyl)-4-[4-(2-oxo-1,2,4,5-tetrahydro-1,3-benzdiazepin-3-yl)-piperidin-1-yl]-1-(4-thiazol-2-yl-piperazin-1-yl)-butan-1,4-dion |
| (90) | 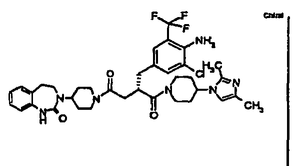 | (*S*)-2-(4-Amino-3-chlor-5-trifluormethyl-benzyl)-1-[4-(2,4-dimethyl-imidazol-1-yl)-piperidin-1-yl]-4-[4-(2-oxo-1,2,4,5-tetrahydro-1,3-benzdiazepin-3-yl)-piperidin-1-yl]-butan-1,4-dion |
| (91) | 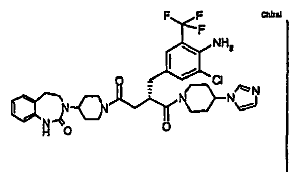 | (*S*)-2-(4-Amino-3-chlor-5-trifluormethyl-benzyl)-1-(4-imidazol-1-yl-piperidin-1-yl)-4-[4-(2-oxo-1,2,4,5-tetrahydro-1,3-benzdiazepin-3-yl)-piperidin-1-yl]-butan-1,4-dion |
| (92) | 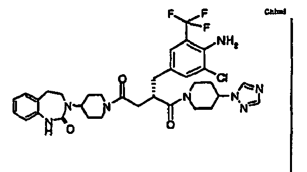 | (*S*)-2-(4-Amino-3-chlor-5-trifluormethyl-benzyl)-4-[4-(2-oxo-1,2,4,5-tetrahydro-1,3-benzdiazepin-3-yl)-piperidin-1-yl]-1-(4-1,2,4-triazol-1-yl-piperidin-1-yl)-butan-1,4-dion |
| (93) | 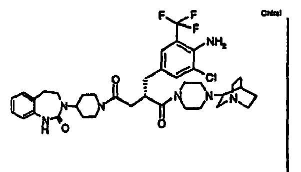 | (*S*)-2-(4-Amino-3-chlor-5-trifluormethyl-benzyl)-1-[4-(1-aza-bicyclo-[2.2.2]oct-3-yl)-piperazin-1-yl]-4-[4-(2-oxo-1,2,4,5-tetrahydro-1,3-benzdiazepin-3-yl)-piperidin-1-yl]-butan-1,4-dion |
| (94) | 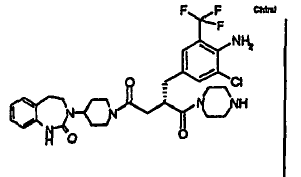 | (*S*)-2-(4-Amino-3-chlor-5-trifluormethyl-benzyl)-4-[4-(2-oxo-1,2,4,5-tetrahydro-1,3-benzdiazepin-3-yl)-piperidin-1-yl]-1-piperazin-1-yl-butan-1,4-dion |
| (95) | 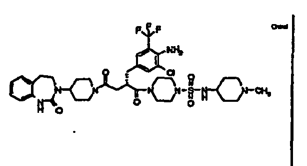 | 4-{(*S*)-2-(4-Amino-3-chlor-5-trifluormethyl-benzyl)-4-oxo-4-[4-(2-oxo-1,2,4,5-tetrahydro-l ,3-benzdiazepin-3-yl)-piperidin-1-yl]-butyryl}-piperazin-1-sulfonsäure-(1-methyl-piperidin-4-yl)-amid |
| (96) | 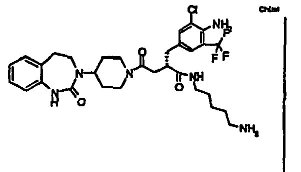 | (*S*)-2-(4-Amino-3-chlor-5-trifluormethyl-benzyl)-*N*-(5-amino-pentyl)-4-oxo-4-[4-(2-oxo-1,2,4,5-tetrahydro-1,3-benzdiazepin-3-yl)-piperidin-1-yl]-butyramid |
| (97) | 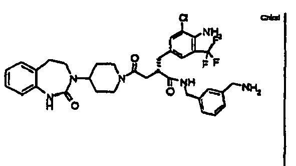 | (*S*)-2-(4-Amino-3-chlor-5-trifluormethyl-berizyl)-*N*-(3-aminomethyl-benzyl)-4-oxo-4-[4=(2-oxo-1,2,4,5-tetrahydro-1,3-benzdiazepin-3-yl)-piperidin-1-yl]-butyramid |
| (98) | 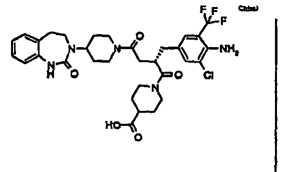 | 1-{(*S*)-2-(4-Amino-3-chlor-5-trifluormethyl-benzyl)-4-oxo-4-[4-(2-oxo-1,2,4,5-tetrahydro-1,3-benzdiazepin-3-yl)-piperidin-1-yl]-butyryl}-piperidin-4-carbonsäure |
| (99) | 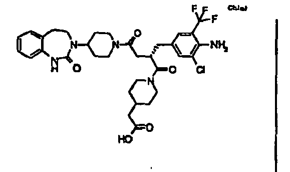 | (1-{(*S*)-2-(4-Amino-3-chlor-5-trifluormethyl-benzyl)-4-oxo-4-[4-(2-oxo-1,2,4,5-tetrahydro-1,3-benzdiazepin-3-yl)-piperidin-1-yl]-butyryl}-piperidin-4-yl)-essigsäure |
| (100) | 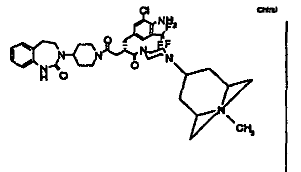 | (*S*)-2-(4-Amino-3-chlor-5-trifluormethyl-benzyl)-1-[4-(8-methyl-8-aza-bicyclo[3.2.1]oct-3-yl)-piperazin-1-yl]-4-[4-(2-oxo-1,2,4,5-tetrahydro-1,3-benzdiazepin-3-yl)-piperidin-1-yl]-butan-1,4-dion |
| (101) | 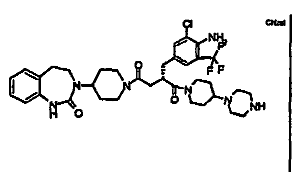 | (*S*)-2-(4-Amino-3-chlor-5-trifluormethyl-benzyl)-4-[4-(2-oxo-1,2,4,5-tetrahydro-1,3-benzdiazepin-3-yl)-piperidin-1-yl]-1-(4-piperazin-1-yl-piperidin-1-yl)-butan-1,4-dion |
| (102) | 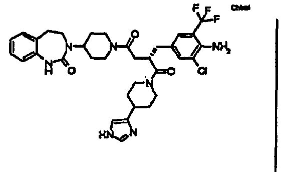 | (*S*)-2-(4-Amino-3-chlor-5-trifluormethyl-benzyl)-1-[4-(1*H*-imidazol-4-yl)-piperidin-1-yl]-4-[4-(2-oxo-1,2,4,5-tetrahydro- 1,3-benzdiazepin-3-yl)-piperidin-1-yl]-butan-1,4-dion |
| (103) | 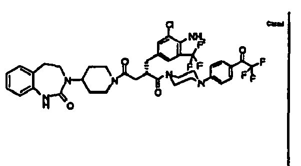 | (*S*)-2-(4-Amino-3-chlor-5-trifluormethyl-benzyl)-4-[4-(2-oxo-1,2,4,5-tetrahydro-1,3-benzdiazepin-3-yl)-piperidin-1-yl]-1-{4-[4-(2,2,2-trifluoracetyl)-phenyl]-piperazin-1-yl}-butan-1,4-dion |
| (104) | 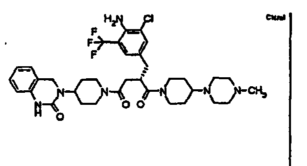 | (*S*)-2-(4-Amino-3-chlor-5-trifluormethyl-benzyl)-1-[4-(4-methyl-piperazin-1-yl)-piperidin-1-yl]-4-[4-(2-oxo-1,4-dihydro-2*H*-chinazolin-3-yl)-piperidin-1-yl]-butan-1,4-dion |
| (105) | 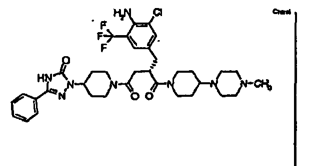 | (*S*)-2-(4-Amino-3-chlor-5-trifluormethyl-benzyl)-1-[4-(4-methyl-piperazin-1-yl)-piperidin-1-yl]-4-[4-(5-oxo-3-phenyl-4,5-dihydro-1,2,4-triazol-1-yl)-piperidin-1-yl]-butan-1,4-dion |
| (106) | 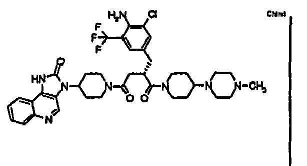 | (*S*)-2-(4-Amino-3-chlor-5-trifluormethyl-benzyl)-1-[4-(4-methyl-piperazin-1-yl)-piperidin-1-yl]-4-[4-(2-oxo-1,2-dihydro-imidazo[4,5-c]chinolin-3-yl)-piperidin-1-yl]-butan-1,4-dion |
| (107) | 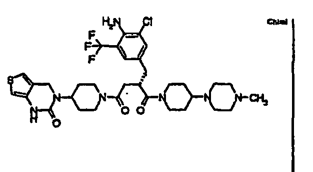 | (*S*)-2-(4-Amino-3-chlor-5-trifluormethyl-benzyl)-1-[4-(4-methyl-piperazin-1-yl)-piperidin-1-yl]-4-[4-(2-oxo-1,2-dihydro-4*H*-thieno[3,4-d]pyrimidin-3-yl)-piperidin-1-yl]-butan-1,4-dion |
| (108) | 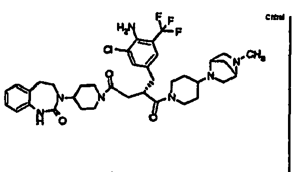 | (*S*)-2-(4-Amino-3-chlor-5-trifluormethyl-benzyl)-1-[4-(5-methyl-2,5-diaza-bicyclo[2.2.1]hept-2-yl)-piperidin-1 -yl]-4-[4-(2-oxo-1,2,4,5-tetrahydro-1,3-benzdiazepin-3-yl)-piperidin-1-yl]-butan-1,4-dion |
| (109) | 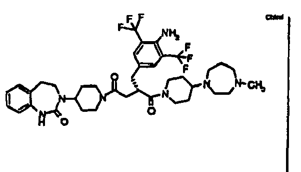 | (*S*)-2-(4-Amino-3,5-bis-trifluormethyl-benzyl)-1-[4-(4-methyl-perhydro-1,4-diazepin-1-yl)-piperidin-1-yl]-4-[4-(2-oxo-1,2,4,5-tetrahydro-1,3-benzdiazepin-3-yl)-piperidin-1-yl]-butan-1,4-dion |
| (110) | 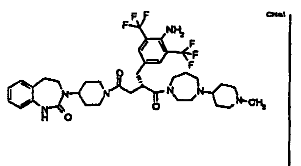 | (*S*)-2-(4-Amino-3,5-bis-trifluor methyl-benzyl)-1-[4-(1-methyl-piperidin-4-yl)-perhydro-1,4-diazepin-1-yl]-4-[4-(2-oxo-1,2,4,5-tetrahydro-1,3-benzdiazepin-3-yl)-piperidin-1-yl]-butan-1,4-dion |
| (111) | 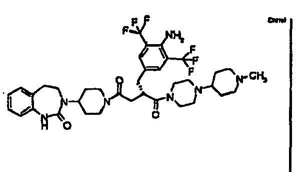 | (*S*)-2-(4-Amino-3,5-bis-trifluormethyl-benzyl)-1-[4-(1-methyl-piperidin-4-yl)-piperazin-1-yl]-4-[4-(2-oxo-1,2,4,5-tetrahydro-1,3-benzdiazepin-3-yl)-piperidin-1-yl]-butan-1,4-dion |
| (112) | 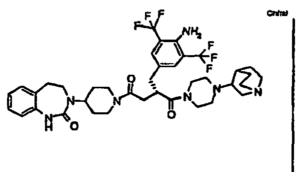 | (*S*)-2-(4-Amino-3,5-bis-trifluormethyl-benzyl)-1-[4-(1-aza-bicyclo-[2.2.2]oct-3-yl)-piperazin-1-yl]-4-[4-(2-oxo-1,2,4,5-tetrahydro-1,3-benz-diazepin-3-yl)-piperidin-1-yl]-butan-1,4-dion |
| (113) | 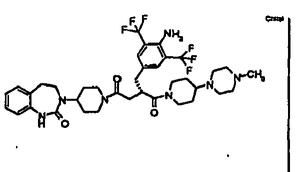 | (*S*)-2-(4-Amino-3,5-bis-trifluormethyl-benzyl)-1-[4-(4-methyl-piperazin-1-yl)-piperidin-1-yl]-4-[4-(2-oxo-1,2,4,5-tetrahydro-1,3-benz-diazepin-3-yl)-piperidin-1-yl]-butan-1,4-dion |
| (114) | 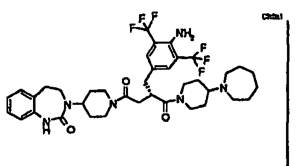 | (*S*)-2-(4-Amino-3,5-bis-trifluormethyl-benzyl)-4-[4-(2-oxo-1,2,4,5-tetrahydro-1,3-benzdiazepin-3-yl)-piperidin-1-yl]-1-(4-perhydro-azepin-1-yl-piperidin-1-yl)-butan-1,4-dion |
| (115) | 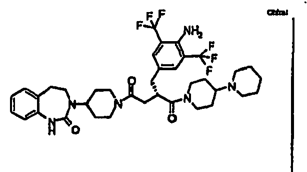 | (*S*)-2-(4-Amino-3,5-bis-trifluormethyl-benzyl)-1-1,4'-bipiperidinyl-1'-yl-4-[4-(2-oxo-1,2,4,5-tetrahydro-1,3-benzdiazepin-3-yl)-piperidin-1-yl]-butan-1,4-dion |
| (116) | 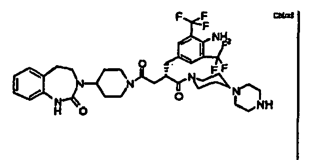 | (*S*)-2-(4-Amino-3,5-bis-trifluormethyl-benzyl)-4-[4-(2-oxo-1,2,4,5-tetrahydro-1,3-benzdiazepin-3-yl)-piperidin-1-yl]-1-(4-piperazin-1-yl-piperidin-1-yl)-butan-1,4-dion |
| (117) | 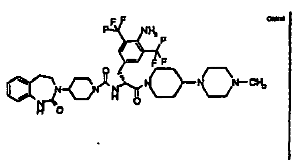 | 4-(2-Oxo-1,2,4,5-tetrahydro-1,3-benzdiazepin-3-yl)-piperidin-1-carbonsäure-{(*R*)-1-(4-amino-3,5-bis-trifluormethyl-benzyl)-2-[4-(4-methyl-piperazin-1-yl)-piperidin-1-yl]-2-oxo-ethyl}-amid |
| (118) | 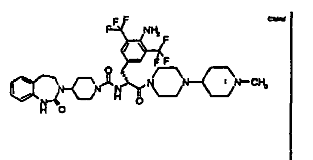 | 4-(2-Oxo-1,2,4,5-tetrahydro-1,3-benzdiazepin-3-yl)-piperidin-1-carbonsäure-{(*R*)-1-(4-amino-3,5-bis-trifluormethyl-benzyl)-2-[4-(1-methyl-piperidin-4-yl)-piperazin-1-yl]-2-oxo-ethyl}-amid |
| (119) | 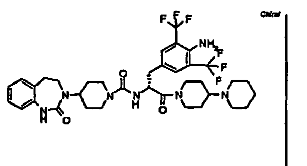 | 4-(2-Oxo-1,2,4,5-tetrahydro-1,3-benzdiazepin-3-yl)-piperidin-1-carbonsäure-[(*R*)-1-(4-amino-3,5-bis-trifluormethyl-benzyl)-2-1,4'-bipiperidinyl-1'-yl-2-oxo-ethyl]-amid |
| (120) | 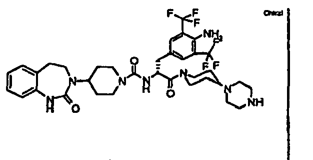 | 4-(2-Oxo-1,2,4,5-tetrahydro-1,3-benzdiazepin-3-yl)-piperidin-1-carbonsäure-[(*R*)-1-(4-amino-3,5-bis-trifluormethyl-benzyl)-2-oxo-2-(4-piperazin-1-yl-piperidin-1-yl)-ethyl]-amid |
| (121) | 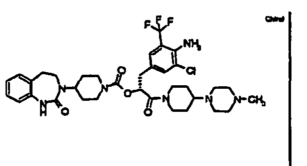 | 4-(2-Oxo-1,2,4,5-tetrahydro-1,3-benzdiazepin-3-yl)-piperidin-1-carbonsäure-(*R*)-1-(4-amino-3-chlor-5-trifluormethyl-benzyl)-2-[4-(4-methyl-piperazin-1-yl)-piperidin-1-yl]-2-oxo-ethylester |
| (122) | 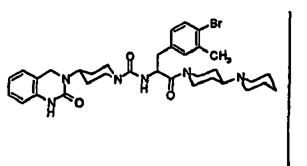 | 4-(2-Oxo-1,4-dihydro-2*H*-chinazolin-3-yl)-piperidin-1-carbonsäure-[2-[1,4']bipiperidinyl-1'-yl-1-(4-brom-3-methyl-benzyl)-2-oxo-ethyl]-amid |
| (123) | 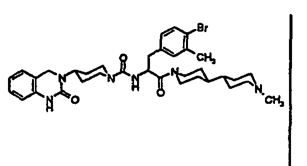 | 4-(2-Oxo-1,4-dihydro-2*H*-chinazolin-3-yl)-piperidin-1-carbonsäure-[1-(4-brom-3-methyl-benzyl)-2-(1'-methyl-[4,4']bipiperidinyl-1-yl)-2-oxo-ethyl]-amid |
| (124) | 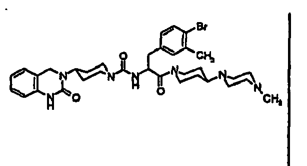 | 4-(2-Oxo-1,4-dihydro-2*H*-chinazolin-3-yl)-piperidin-1-carbonsäure-{1-(4-brom-3-methyl-benzyl)-2-[4-(4-methyl-piperazin-1-yl)-piperidin-1-yl]-2-oxo-ethyl}-amid |
| (125) | 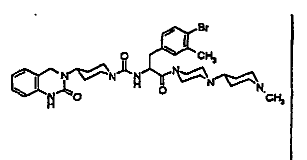 | 4-(2-Oxo-1,4-dihydro-2*H*-chinazolin-3-yl)-piperidin-1-carbonsäure-{1-(4-brom-3-methyl-benzyl)-2-[4-(1-methyl-piperidin-4-yl)-piperazin-1-yl]-2-oxo-ethyl}-amid |
| (126) | 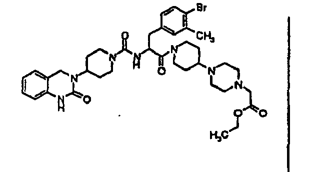 | {4-[1-(3-(4-Brom-3-methyl-phenyl)-2-{[4-(2-oxo-1,4-dihydro-2*H*-chinazolin-3-yl)-piperidin-1-carbonyl]-amino}-propionyl)-piperidin-4-yl]-piperazin-1-yl}-essigsäureethylester |
| (127) | 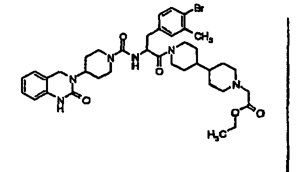 | [1'-(3-(4-Brom-3-methyl-phenyl)-2-{[4-(2-oxo-1,4-dihydro-2*H*-chinazolin-3-yl)-piperidin-1-carbonyl]-amino}-propionyl)-[4,4']bipiperidinyl-1-yl]-essigsäureethylester |
| (128) | 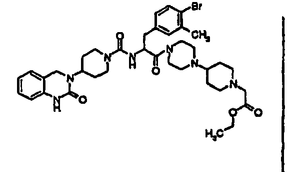 | {4-[4-(3-(4-Brom-3-methyl-phenyl)-2-{[4-(2-oxo-1,4-dihydro-2*H*-chinazolin-3-yl)-piperidin-1-carbonyl]-amino}-propionyl)-piperazin-1-yl]-piperidin-1-yl}-essigsäureethylester |
| (129) | 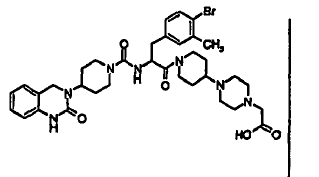 | {4-[1-(3-(4-Brom-3-methyl-phenyl)-2-{[4-(2-oxo-1,4-dihydro-2*H*-chinazolin-3-yl)-piperidin-1-carbonyl]-amino}-propionyl)-piperidin-4-yl]-piperazin-1-yl}-essigsäure |
| (130) | 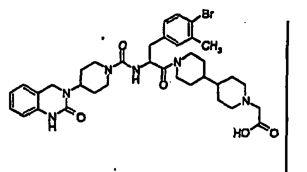 | [1'-(3-(4-Brom-3-methyl-phenyl)-2-{[4-(2-oxo-1,4-dihydro-2*H*-chinazolin-3-yl)-piperidin-1-carbonyl]-amino}-propionyl)-[4,4']bipiperidinyl-1-yl]-essigsäure |
| (131) | 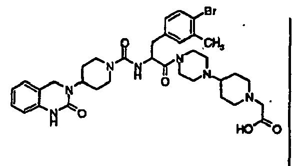 | {4-[4-(3-(4-Brom-3-methyl-phenyl)-2-{[4-(2-oxo-1,4-dihydro-2*H*-chinazolin-3-yl)-piperidin-1-carbonyl]-amino}-propionyl)-piperazin-1-yl]-piperidin-1-yl}-essigsäure |
| (132) | 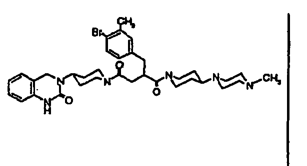 | 2-(4-Brom-3-methyl-benzyl)-1-[4-(4-methyl-piperazin-1-yl)-piperidin-1-yl]-4-[4-(2-oxo-1,4-dihydro-2*H*-chinazolin-3-yl)-piperidin-1-yl]-butan-1,4-dion |
| (133) | 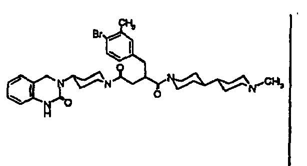 | 2-(4-Brom-3-methyl-benzyl)-1-(1'-methyl-[4,4']bipiperidinyl-1-yl)-4-[4-(2-oxo-1,4-dihydro-2*H*-chinazolin-3-yl)-piperidin-1-yl]-butan-1,4-dion |
| (134) | 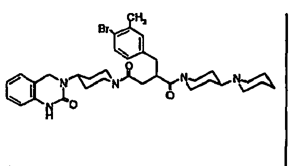 | 1-[1,4']Bipiperidinyl-1'-yl-2-(4-brom-3-methyl-benzyl)-4-[4-(2-oxo-1,4-dihydro-2*H*-chinazolin-3-yl)-piperidin-1-yl]-butan-1,4-dion |
| (135) | 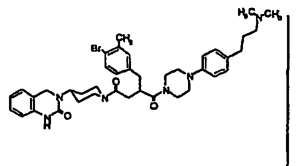 | 2-(4-Brom-3-methyl-benzyl)-1-{4-[4-(3-dimethylamino-propyl)-phenyl]-piperazin-1-yl}-4-[4-(2-oxo-1,4-dihydro-2*H*-chinazolin-3-yl)-piperidin-1-yl]-butan-1,4-dion |
| (136) | 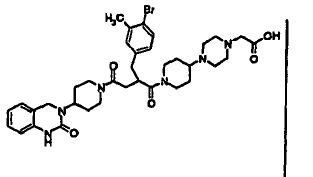 | [4-(1-{2-(4-Brom-3-methyl-benzyl)-4-oxo-4-[4-(2-oxo-1,4-dihydro-2*H*-chinazolin-3-yl)-piperidin-1-yl]-butyryl}-piperidin-4-yl)-piperazin-1-yl]-essigsäure |
| (137) | 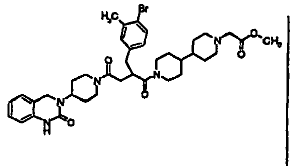 | (1'-{2-(4-Brom-3-methyl-benzyl)-4-oxo-4-[4-(2-oxo-1,4-dihydro-2*H*-chinazolin-3-yl)-piperidin-1-yl]-butyryl}-[4,4']bipiperidinyl-1-yl)-essigsäuremethylester |
| (138) | 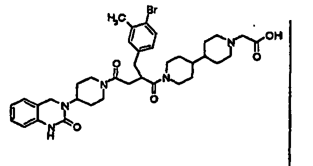 | (1'-{2-(4-Brom-3-methyl-benzyl)-4-oxo-4-[4-(2-oxo-1,4-dihydro-2*H*-chinazolin-3-yl)-piperidin-1-yl]-butyryl}-[4,4']bipiperidinyl-1-yl)-essigsäure |
| (139) | 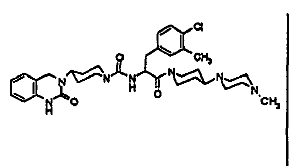 | 4-(2-Oxo-1,4-dihydro-2*H*-chinazolin-3-yl)-piperidin-1-carbonsäure{1-(4-chlor-3-methyl-benzyl)-2-[4-(4-methyl-piperazin-1-yl)-piperidin-1-yl]-2-öxo-ethyl}-amid |
| (140) | 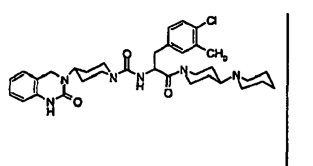 | 4-(2-Oxo-1,4-dihydro-2*H*-chinazolin-3-yl)-piperidin-1-carbonsäure-[2-[1,4']bipipendinyl-1'-yl-1-(4-chlor-3-methyl-benzyl)-2-oxo-ethyl]-amid |
| (141) | 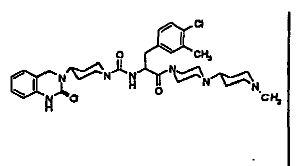 | 4-(2-Oxo-1,4-dihydro-2*H*-chinazolin-3-yl)-piperidin-1-carbonsäure-{1-(4-chlor-3-methyl-benzyl)-2-[4-(1-methyl-piperidin-4-yl)-piperazin-1-yl]-2-oxo-ethyl}-amid |
| (142) | 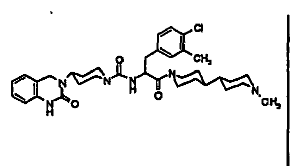 | 4-(2-Oxo-1,4-dihydro-2*H*-chinazolin-3-yl)-piperidin-1-carbonsäure-[1-(4-chlor-3-methyl-benzyl)-2-(1'-methyl-[4,4']bipiperidinyl-1-yl)-2-oxo-ethyl]-amid |
| (143) | 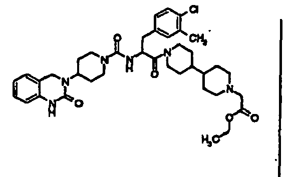 | [1'-(3-(4-Chlor-3-methyl-phenyl)-2-{[4-(2-oxo-1,4-dihydro-2*H*-chinazolin-3-yl)-piperidin-1-carbonyl]-amino}-propionyl)-[4,4']bipiperidinyl-1-yl]-essigsäureethylester |
| (144) | 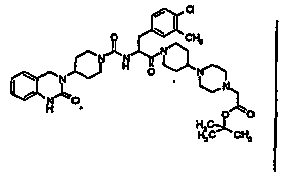 | {4-[1-(3-(4-Chlor-3-methyl-phenyl)-2-{[4-(2-oxo-1,4-dihydro-2*H*-chinazolin-3-yl)-piperidin-1-carbonyl]-amino}-propionyl)-pipeddin-4-yl]-piperazin-1-yl}-essigsäure-tert-butylester |
| (145) | 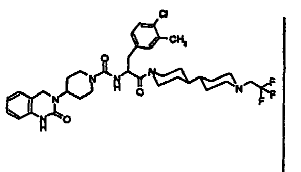 | 4-(2-Oxo-1,4-dihydro-2*H*-chinazolin-3-yl)-piperidin-1-carbonsäure-{1-(4-chlor-3-methyl-benzyl)-2-oxo-2-[1'-(2,2,2-trifluor-ethyl)-[4,4']bipiperidinyl-1-yl]-ethyl}-amid |
| (146) | 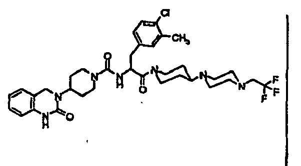 | 4-(2-Oxo-1,4-dihydro-2*H*-chinazolin-3-yl)-piperidin-1-carbonsäure-(1-(4-chlor-3-methyl-benzyl)-2-oxo-2-{4-[4-(2,2,2-trifluor-ethyl)-piperazin-1-yl]-piperidin-1-yl}-ethyl)-amid |
| (147) | 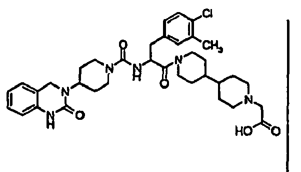 | [1'-(3-(4-Chlor-3-methyl-phenyl)-2-{[4-(2-oxo-1,4-dihydro-2*H*-chinazolin-3-yl)-piperidin-1-carbonyl]-amino}-propionyl)-[4,4']bipiperidinyl-1-yl]-essigsäure |
| (148) | 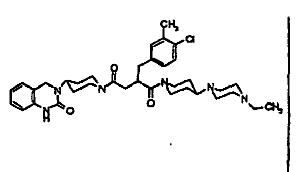 | 2-(4-Chlor-3-methyl-benzyl)-1-[4-(4-ethyl-piperazin-1-yl)-piperidin-1-yl]-4-[4-(2-oxo-1,4-dihydro-2*H*-chinazolin-3-yl)-piperidin-1-yl]-butan-1,4-dion |
| (149) | 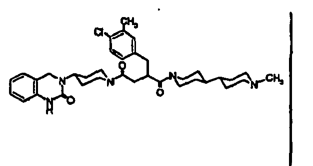 | 2-(4-Chlor-3-methyl-benzyl)-1-(1'-methyl-[4,4']bipiperidinyl-1-yl)-4-[4-(2-oxo-1,4-dihydro-2*H*-chinazolin-3-yl)-piperidin-1-yl]-butan-1,4-dion |
| (150) | 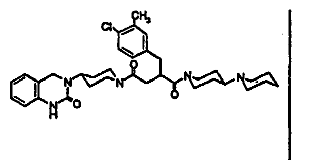 | 1-[1,4']Bipiperidinyl-1 '-yl-2-(4-chlor-3-methyl-benzyl)-4-[4-(2-oxo-1,4-dihydro-2*H*-chinazolin-3-yl)-piperidin-1-yl]-butan-1,4-dion |
| (151) | 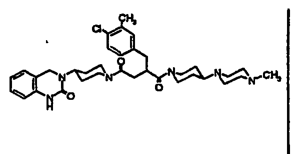 | 2-(4-Chlor-3-methyl-benzyl)-1-[4-(4-methyl-piperazin-1-yl)-piperidin-1-yl]-4-[4-(2-oxo-1,4-dihydro-2*H*-chinazolin-3-yl)-piperidin-1-yl]-butan-1,4-dion |
| (152) | 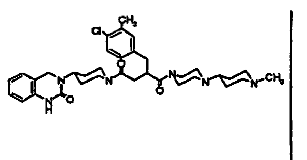 | 2-(4-Chlor-3-methyl-benzyl}-1-[4-(1-methyl-piperidin-4-yl)-piperazin-1-yl]-4-[4-(2-oxo-1,4-dihydro-2*H*-chinazolin-3-yl)-piperidin-1-yl]-butan-1,4-dion |
| (153) | 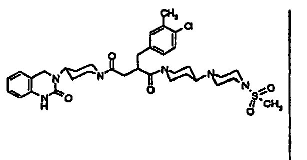 | 2-(4-Chlor-3-methyl-benzyl)-1-[4-(4-methansulfonyl-piperazin-1-yl)-piperidin-1-yl]-4-[4-(2-oxo-1,4-dihydro-2*H*-chinazolin-3-yl)-piperidin-1-yl]-butan-1,4-dion |
| (154) | 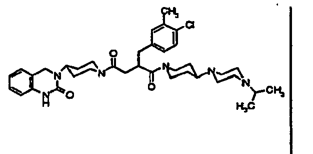 | 2-(4-Chlor-3-methyl-benzyl)-1-[4-(4-isopropyl-piperazin-1-yl)-piperidin-1-yl]-4-[4-(2-oxo-1,4-dihydro-2*H* chinazolin-3-yl)-piperidin-1-yl]-butan-1,4-dion |
| (155) | 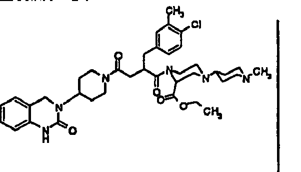 | 1-{2-(4-Chlor-3-methyl-benzyl)-4-oxo-4-[4-(2-oxo-1,4-dihydro-2*H*-chinazolin-3-yl)-piperidin-1-yl]-butyryl}-4-(1-methyl-piperidin-4-yl)-piperazin-2-carbonsäureethylester |
| (156) | 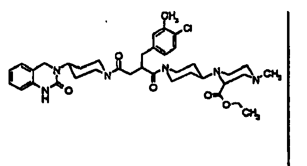 | 1-(1-{2-(4-Chlor-3-methyl-benzyl)-4-oxo-4-[4-(2-oxo-1,4-dihydro-2*H* chinazolin-3-yl)-piperidin-1-yl]-butyryl}-piperidin-4-yl)-4-methyl-piperazin-2-carbonsäureethylester |
| (157) | 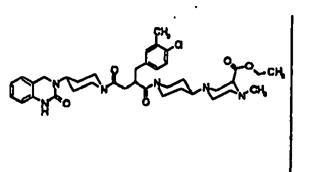 | 4-(1-{2-(4-Chlor-3-methyl-benzyl)-4-oxo-4-[4-(2-oxo-1,4-dihydro-2*H*-chinazolin-3-yl)-piperidin-1-yl]-butyryl}-piperidin-4-yl)-1-methyl-piperazin-2-carbonsäureethylester |
| (158) | 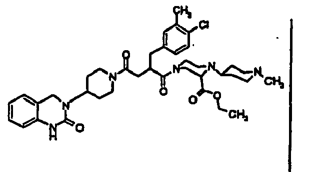 | 4-{2-(4-Chlor-3-methyl-benzyl)-4-oxo-4-[4-(2-oxo-1,4-dihydro-2*H*-chinazolin-3-yl)-piperidin-1-yl]-butyryl}-1-(1-methyl-piperidin-4-yl)-piperazin-2-carbonsäureethylester |
| (159) | 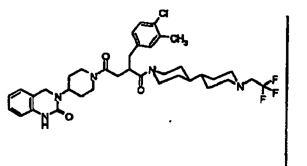 | 2-(4-Chlor-3-methyl-benzyl)-4-[4-(2-oxo-1,4-dihydro-2*H*-chinazolin-3-yl)-piperidin-1-yl]-1-[1'-(2,2,2-trifluorethyl)-[4,4']bipiperidinyl-1-yl]-butan-1,4-dion |
| (160) | 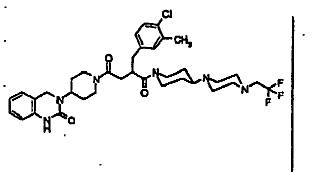 | 2-(4-Chlor-3-methyl-benzyl)-4-[4-(2-oxo-1,4-dihydro-2*H*-chinazolin-3-yl)-piperidin-1-yl]-1-{4-[4-(2,2,2-trifluorethyl)-piperazin-1-yl]-piperidin-1-yl}-butan-1,4-dion |
| (161) | 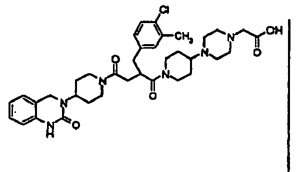 | [4-(1-{2-(4-Chlor-3-methyl-benzyl)-4-oxo-4-[4-(2-oxo-1,4-dihydro-2*H*-chinazolin-3-yl)-piperidin-1-yl]-butyryl}-piperidin-4-yl)-piperazin-1-yl]-essigsäure |
| (162) | 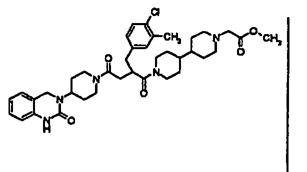 | (1'-{2-(4-Chlor-3-methyl-benzyl)-4-oxo-4-[4-(2-oxo-1,4-dihydro-2*H*-chinazolin-3-yl)-piperidin-1-yl]-butyryl}-[4,4']bipiperidinyl-1-yl)-essigsäuremethylester |
| (163) | 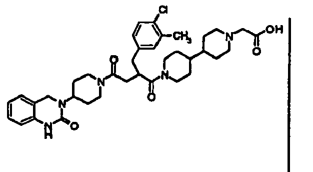 | (1'-{2-(4-Chlor-3-methyl-benzyl)-4-oxo-4-[4-(2-oxo-1,4-dihydro-2*H* chinazolin-3-yl)-piperidin-1-yl]-butyryl}-[4,4']bipiperidinyl-1-yl)-essigsäure |
| (164) | 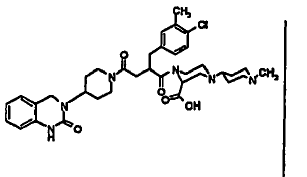 | 1-{2-(4-Chlor-3-methyl-benzyl)-4-oxo-4-[4-(2-oxo-1,4-dihydro-2*H*-chinazolin-3-yl)-piperidin-1-yl]-butyryl}-4-(1-methyl-piperidin-4-yl)-piperazin-2-carbonsäure |
| (165) | 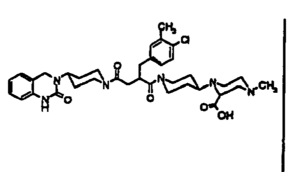 | 1-(1-{2-(4-Chlor-3-methyl-benzyl)-4-oxo-4-[4-(2-oxo-1,4-dihydro-2*H*-chinazolin-3-yl)-piperidin-1-yl]-butyryl}-piperidin-4-yl)-4-methyl-piperazin-2-carbonsäure |
| (166) | 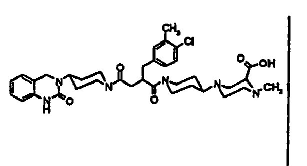 | 4-(1-{2-(4-Chlor-3-methyl-benzyl)-4-oxo-4-[4-(2-oxo-1,4-dihydro-2*H*-chinazolin-3-yl)-piperidin-1-yl]-butyryl}-piperidin-4-yl)-1-methyl-piperazin-2-carbonsäure |
| (167) | 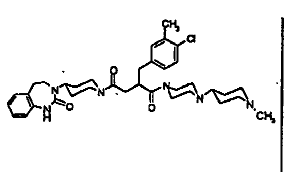 | 2-(4-Chlor-3-methyl-benzyl)-1-[4-(1-methyl-piperidin-4-yl)-piperazin-1-yl]-4-[4-(2-oxo-1,2,4,5-tetrahydro-1,3-benzdiazepin-3-yl)-piperidin-1-yl]-butan-1,4-dion |
| (168) | 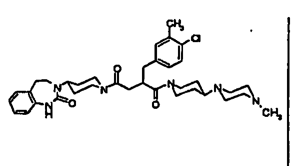 | 2-(4-Chlor-3-methyl-benzyl)-1-[4-(4-methyl-piperazin-1-yl)-piperidin-1-yl]-4-[4-(2-oxo-1,2,4,5-tetrahydro-1,3-benzdiazepin-3-yl)-piperidin-1-yl]-butan-1,4-dion |
| (169) | 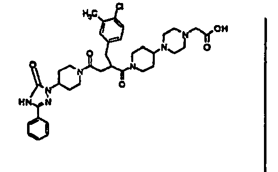 | [4-(1-{2-(4-Chlor-3-methyl-benzyl)-4-oxo-4-[4-(5-oxo-3-phenyl-4,5-dihydro-1,2,4-triazol-1-yl)-piperidin-1-yl]-butyryl}-piperidin-4-yl)-piperazin-1-yl]-essigsäure |
| (170) | 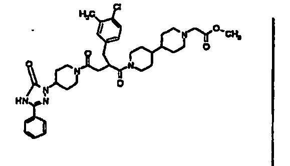 | (1'-{2-(4-Chlor-3-methyl-benzyl)-4-oxo-4-[4-(5-oxo-3-phenyl-4,5-dihydro-1,2,4-triazol-1-yl)-piperidin-1-yl]-butyryl}-4,4'-bipiperidinyl-1-yl)-essigsäuremethylester |
| (171) | 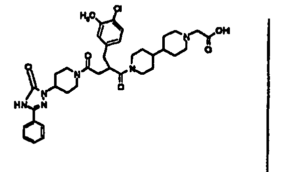 | (1'-{2-(4-Chlor-3-methyl-benzyl)-4-oxo-4-[4-(5-oxo-3-phenyl-4,5-dihydro-1,2,4-triazol-1-yl)-piperidin-1 -yl]-butyryl}-4,4'-bipiperidinyl-1-yl)-essigsäure |
| (172) | 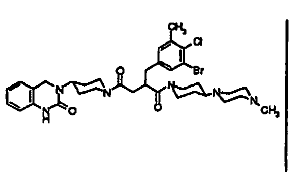 | 2-(3-Brom-4-chlor-5-methyl-benzyl)-1-[4-(4-methyl-piperazin-1-yl)-piperidin-1-yl]-4-[4-(2-oxo-1,4-dihydro-2*H*-chinazolin-3-yl)-piperidin-1-yl]-butan-1,4-dion |
| (173) | 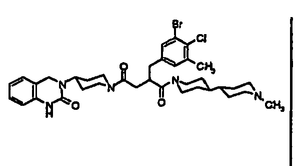 | 2-(3-Brom-4-chlor-5-methyl-benzyl)-1-(1'-methyl-[4,4']bipiperidinyl-1-yl)-4-[4-(2-oxo-1,4-dihydro-2*H*-chinazolin-3-yl)-piperidin-1-yl]-butan-1,4-dion |
| (174) | 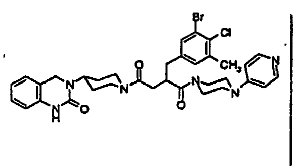 | 2-(3-Brom-4-chlor-5-methyl-benzyl)-4-[4-(2-oxo-1,4-dihydro-2*H*-chinazolin-3-yl)-piperidin-1-yl]-1-(4-pyridin-4-yl-piperazin-1-yl)-butan-1,4-dion |
| (175) | 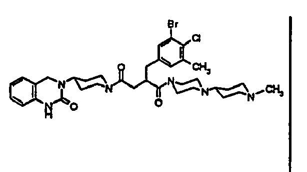 | 2-(3-Brom-4-chlor-5-methyl-benzyl)-1-[4-(1-methyl-piperidin-4-yl)-piperazin-1-yl]-4-[4-(2-oxo-1,4-dihydro-2*H*-chinazolin-3-yl)-piperidin-1-yl]-butan-1,4-dion |
| (176) | 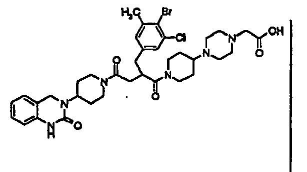 | [4-(1-{2-(3-Brom-4-chlor-5-methyl-benzyl)-4-oxo-4-[4-(2-oxo-1,4-dihydro-2*H*-chinazolin-3-yl)-piperidin-1-yl]-butyryl}-piperidin-4-yl)-piperazin-1-yl]-essigsäure |
| (177) | 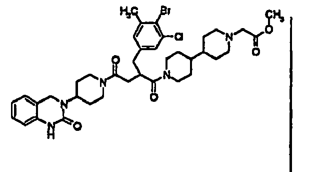 | (1'-{2-(3-Brom-4-Chlor-5-methyl-benzyl)-4-oxo-4-[4-(2-oxo-1,4-dihydro-2*H*-chinazolin-3-yl)-piperidin-1-yl]-butyryl}-[4,4']bipiperidinyl-1-yl)-essigsäuremethylester |
| (178) | 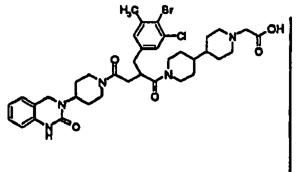 | (1'-{2-(3-Brom-4-Chlor-5-methyl-benzyl)-4-oxo-4-[4-(2-oxo-1,4-dihydro-2*H*-chinazolin-3-yl)-piperidin-1-yl]-butyryl}-[4,4']bipiperidinyl-1-yl)-essigsäure |
| (179) | 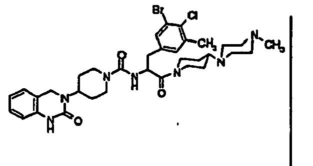 | 4-(2-Oxo-1,4-dihydro-2*H*-chinazolin-3-yl)-piperidin-1-carbonsäure{1-(3-brom-4-chlor-5-methyl-benzyl)-2-[4-(4-methyl-piperazin-1-yl)-piperidin-1-yl]-2-oxo-ethyl}-amid |
| (180) | 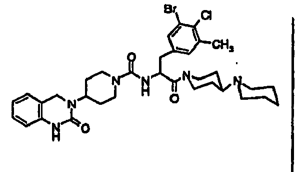 | 4-(2-Oxo-1,4-dihydro-2*H-*chinazolin-3-yl)-piperidin-1-carbonsäure-[2-[1,4']bipiperidinyl-1'-yl-1-(3-brorn-4-chlor-5-methyl-benzyl)-2-oxo-ethyl]-amid |
| (181) | 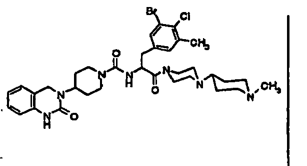 | 4-(2-Oxo-1,4-dihydro-2*H*-chinazolin-3-yl)-piperidin-1-carbonsäure-{1-(3-brom-4-chlor-5-methyl-benzyl)-2-[4-(1-methyl-piperidin-4-yl)-piperazin-1-yl]-2-oxo-ethyl}-amid |
| (182) | 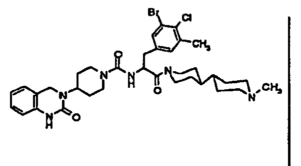 | 4-(2-Oxo-1,4-dihydro-2*H*-chinazolin-3-yl)-piperidin-1-carbonsäure-[1-(3-brom-4-chlor-5-methyl-benzyl)-2-(1'-methyl-[4,4']bipiperidinyl-1-yl)-2-oxo-ethyl]-amid |
| (183) | 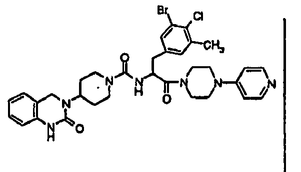 | 4-(2-Oxo-1,4-dihydro-2*H*-chinazolin-3-yl)-piperidin-1-carbonsäure-[1-(3-brom-4-chlor-5-methyl-benzyl)-2-oxo-2-(4-pyridin-4-yl-piperazin-1-yl)-ethyl]-amid |
| (184) | 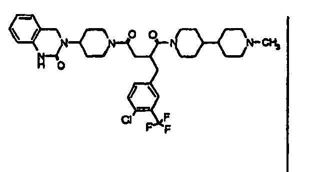 | 2-(4-Chlor-3-trifluormethyl-benzyl)-1-(1'-methyl-[4,4']bipipehdinyl-1-yl)-4-[4-(2-oxo-1,4-dihydro-2*H*-chinazolin-3-yl)-piperidin-1-yl]-butan-1,4-dion |
| (185) | 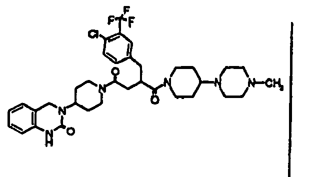 | 2-(4-Chlor-3-trifluormethyl-benzyl)-1-[4-(4-methyl-piperazin-1-yl)-piperidin-1-yl]-4-[4-(2-oxo-1,4-dihydro-2*H*-chinazolin-3-yl)-piperidin-1-yl]-butan-1,4-dion |
| (186) | 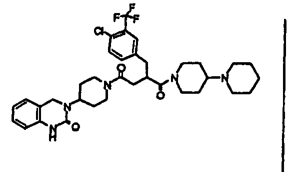 | 1-[1,4']Bipiperdinyl-1'-yl-2-(4-chlor-3-trifluormethyl-benzyl)-4-[4-(2-oxo-1,4-dihydro-2*H*-chinazolin-3-yl)-piperidin-1-yl]-butan-1,4-dion |
| (187) | 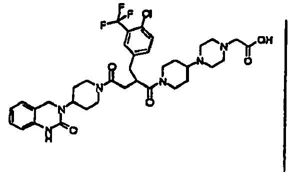 | [4-(1-{2-(4-Chlor-3-trifluormethyl-benzyl)-4-oxo-4-[4-(2-oxo-1,4-dihydro-2*H*-chinazolin-3-yl)-piperidin-1-yl]-butyryl}-piperidin-4-yl)-piperazin-1-yl]-essigsäure |
| (188) | 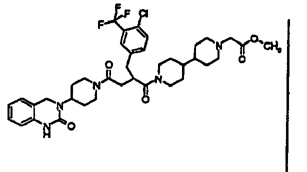 | (1'-{2-(4-Chlor-3-trifluormethyl-benzyl)-4-oxo-4-[4-(2-oxo-1,4-dihydro-2*H*-chinazolin-3-yl)-piperidin-1-yl]-butyryl}-[4,4']bipiperidinyl-1-yl)-essigsäuremethylester |
| (189) | 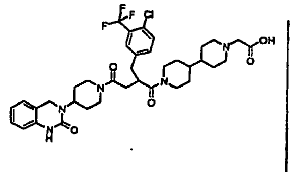 | (1'-{2-(4-Chlor-3-trifluormethyl-benzyl)-4-oxo-4-[4-(2-oxo-1,4-dihydro-2*H*-chinazolin-3-yl)-piperidin-1-yl]-butyryl}-[4,4']bipiperidinyl-1-yl)-essigsäure |
| (190) | 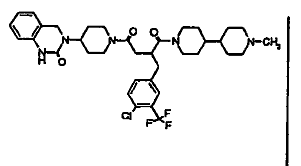 | 2-(4-Chlor-3-trifluormethyl-benzyl)-1-(1'-methyl-[4,4']bipiperidinyl-1-yl)-4-[4-(2-oxo-1,4-dihydro-2*H*-chinazolin-3-yl)-piperidin-1-yl]-butan-1,4-dion |

deren Enantiomere, deren Diastereomere und deren Salze, wobei die Verbindungen
(1) (*S*)-2-(4-Amino-3-chlor-5-trifluormethyl-benzyl)-1-[4-(4-methyl-Piperazin-1-yl)-piperidin-1-yl]-4-[4-(2-oxo-1,2,4,5-tetrahydro-1,3-benzdiazepin-3-yl)-piperidin-1-yl]-butan-1,4-dion,
(2) 2-(4-Amino-3-chlor-5-trifluormethyl-benzyl)-1-(1'-methyl-[4,4']bipiperidmyl-1-yl)-4-[4-(2-oxo-1,4-dihydro-2*H*-quinazolin-3-yl)-piperidin-1-yl]-butan-1,4-dion,
(3) 2-(4-Amino-3-chlor-5-trifluormethyl-benzyl)-1-[4-(4-methyl-piperazin-1-yl)-piperidin-1-yl]-4-[4-(2-oxo-1,4-dihydro-2*H*-quinazolin-3-yl)-piperidin-1-yl]-butan-1,4-dion,
(4) 2-(4-Amino-3-chlor-5-trifluormethyl-benzyl)-1-[1,4']bipiperidinyl-1'-yl-4-[4-(2-oxo-1,4-dihydro-2*H*-quinazolin-3-yl)-piperidin-1-yl]-butan-1,4-dion,
(5) 2-(4-Amino-3-chlor-5-trifluormethyl-benzyl)-4-[4-(2-oxo-1,4-dihydro-2*H* quinazolin-3-yl)-piperidin-1-yl]-1-(4-pyridin-4-yl-piperazin-1-yl)-butan-1,4-dion,
(6) (1'-{2-(4-Amino-3-chlor-5-trifluormethyl-benzyl)-4-oxo-4-[4-(2-oxo-1,4-dihydro-2*H*-chinazolin-3-yl)-piperidin-1-yl]-butyryl}-[4,4']bipiperidinyl-1-yl)-essigsäuremethylester,
(7) 4-(2-Oxo-1,2,4,5-tetrahydro-1,3-benzdiazepin-3-yl)-piperidin-1-carbonsäure-[(*R*)-1-(4-amino-3-chlor-5-trifluormethyl-benzyl)-2-1,4'-bipiperidinyl-1'-yl-2-oxo-ethyl]-amid,
(8) 4-(2-Oxo-1,2,4,5-tetrahydro-1,3-benzdiazepin-3-yl)-piperidin-1-carbonsäure-[(*R*)-1-(4-amino-3-chlor-5-trifluormethyl-benzyl)-2-(4-dimethylamino-piperidin-1-yl)-2-oxo-ethyl]-amid,
(9) 4-(2-Oxo-1,2,4,5-tetrahydro-1,3-benzdiazepin-3-yl)-piperidin-1-carbonsäure-[(*R*)-1-(4-amino-3-chlor-5-trifluormethyl-benzyl)-2-oxo-2-(4-pyridin-4-yl-piperazin-1-yl)-ethyl]-amid,
(10) 4-(2-Oxo-1,2,4,5-tetrahydro-1,3-benzdiazepin-3-yl)-piperidin-1-carbonsäure-[(*R*)-1-(4-amino-3-chlor-5-trifluormethyl-benzyl)-2-(1'-methyl-4,4'-bipiperidinyl-1-yl)-2-oxo-ethyl]-amid,
(11) 4-[1-((*R*)-3-(4-Amino-3-chlor-5-trifluormethyl-phenyl)-2-{[4-(2-oxo-1,2,4,5-tetrahydro-1,3-benzdiazepin-3-yl)-piperidin-1-carbonyl]-amino}-proplonyl)-piperidin-4-yl]-piperazin-1-carbonsäurebenzylester,
(12) [1'-((*R*)-3-(4-Amino-3-chlor-5-trifluormethyl-phenyl)-2-{[4-(2-oxo-1,2,4,5-tetrahydro-1,3-benzdiazepin-3-yl)-piperidin-1-carbonyl]-amino}-propionyl)-4,4'-bipiperidinyl-1-yl]-essigsäureethylester,
(13) 4-(2-Oxo-1,2,4,5-tetrahydro-1,3-benzdiazepin-3-yl)-piperidin-1-carbonsäure-{(*R*)-1-(4-amino-3-chlor-5-trifluormethyl-benzyl)-2-[4-(4-methyl-piperazin-1-yl)-piperidin-1-yl]-2-oxo-ethyl}-amid,
(14) 4-(2-Oxo-1,2,4,5-tetrahydro-1,3-benzdiazepin-3-yl)-piperidin-1-carbonsäure-{(*R*)-1-(4-amino-3-chlor-5-trifluormethyl-benzyl)-2-[4-(1-methyl-piperidin-4-yl)-piperazin-1-yl]-2-oxo-ethyl}-amid,
(15) 4-(2-Oxo-1,2,4,5-tetrahydro-1,3-benzdiazepin-3-yl)-piperidin-1-carbonsäure-[(*R*)-1-(4-amino-3-chlor-5-trifluormethyl-benzyl)-2-(4-azetidin-1-yl-piperidin-1-yl)-2-oxo-ethyl]-amid,
(16) 4-(2-Oxo-1,2,4,5-tetrahydro-1,3-benzdiazepin-3-yl)-piperidin-1-carbonsäure-{(*R*)-1-(4-amino-3-chlor-5-trifluormethyl-benzyl)-2-[4-(5-methyl-2,5-diaza-bicyclo[2.2.1]hept-2-yl)-piperidin-1-yl]-2-oxo-ethyl}-amid,
(17) 4-(2-Oxo-1,2,4,5-tetrahydro-1,3-benzdiazepin-3-yl)-piperidin-1-carbonsäure-[(*R*)-1-(4-amino-3-chlor-5-trifluormethyl-benzyl)-2-oxo-2-(4-piperazin-1-yl-piperidin-1-yl)-ethyl]-amid,
(18) [1'-((*R*)-3-(4-Amino-3-chlor-5-trifluormethyl-phenyl)-2-{[4-(2-oxo-1,2,4,5-tetrahydro-1,3-benzdiazepin-3-yl)-piperidin-1-carbonyl]-amino}-propionyl)-4,4'-bipiperidinyl-1-yl]-essigsäure,
(19) 4-(2-Oxo-1,2,4,5-tetrahydro-1,3-benzdiazepin-3-yl)-piperidin-1-carbonsäure-[1-(4-amino-3-chlor-5-trifluormethyl-benzyl)-2-1,4'-bipiperidinyl-1'-yl-2-oxo-ethyl]-amid,
(20) (*S*)-2-(4-Amino-3-brom-5-trifluormethyl-benzyl)-1-[4-(4-methyl-piperazin-1-yl)-piperidin-1-yl]-4-[4-(2-oxo-1,2,4,5-tetrahydro-1,3-benzdiazepin-3-yl)-piperidin-1-yl]-butan-1,4-dion,
(21) (*S*)-2-(4-Amino-3-brom-5-trifluormethyl-benzyl)-4-[4-(2-oxo-1,2,4,5-tetrahydro-1,3-benzdiazepin-3-yl)-piperidin-1-yl]-1-(4-pyridin-4-yl-piperazin-1-yl)-butan-1,4-dion,
(22) (*S*)-2-(4-Amino-3-brom-5-trifluormethyl-benzyl)-1-1,4'-bipiperidinyl-1'-yl-4-[4-(2-oxo-1,2,4,5-tetrahydro-1,3-benzdiazepin-3-yl)-piperidin-1-yl]-butan-1,4-dion,
(23) (*S*)-2-(4-Amino-3-brom-5-trifluormethyl-benzyl)-1-[4-(1-methyl-piperidin-4-yl)-piperazin-1-yl]-4-[4-(2-oxo-1,2,4,5-tetrahydro-1,3-benzdiazepin-3-yl)-piperidin-1-yl]-butan-1,4-dion,
(24) (*S*)-2-(4-Amino-3-brom-5-trifluormethyl-benzyl)-1-(1'-methyl-4,4'-bipiperidinyl-1-yl)-4-[4-(2-oxo-1,2,4,5-tetrahydro-1,3-benzdiazepin-3-yl)-piperidin-1-yl]-butan-1,4-dion,
(25) (*S*)-2-(4-Amino-3-trifluormethyl-benzyl)-1-[4-(4-methyl-piperazin-1-yl)-piperidin-1-yl]-4-[4-(2-oxo-1,2,4,5-tetrahydro-1,3-benzdiazepin-3-yl)-piperidin-1-yl]-butan-1,4-dion,
(26) (*S*)-2-(4-Amino-3-chlor-5-trifluormethyl-benzyl)-1-(4-dimethylamino-piperidin-1-yl)-4-[4-(2-oxo-1,2,4,5-tetrahydro-1,3-benzdiazepin-3-yl)-piperidin-1-yl]-butan-1,4-dion,
(27) (*S*)-2-(4-Amino-3-chlor-5-trifluormethyl-benzyl)-4-[4-(2-oxo-1,2,4,5-tetrahydro-1,3-benzdiazepin-3-yl)-piperidin-1-yl]-1-(4-pyrrolidin-1-yl-piperidin-1-yl)-butan-1,4-dion,
(28) (*S*)-2-(4-Amino-3-chlor-5-trifluormethyl-benzyl)-1-(1'-methyl-4,4'-bipiperidinyl-1-yl)-4-[4-(2-oxo-1,2,4,5-tetrahydro-1,3-benzediazepin-3-yl)-piperidin-1-yl]-butan-1,4-dion,
(29) (*S*)-2-(4-Amino-3-chlor-5-trifluormathyl-benzyl)-1-[4-(4-cyclopropylmethyl-piperazin-1-yl)-piperidin-1-yl]-4-[4-(2-oxo-1,2,4,5-tetrahydro-1,3-benzdiazepin-3-yl)-piperidin-1-yl]-butan-1,4-dion,
(30) (*S*)-2-(4-Amino-3-chlor-5-trifluormethyl-benzyl)-1-(4-morpholin-4-yl-piperidin-1-yl)-4-[4-(2-oxo-1,2,4,5-tetrahydro-1,3-benzdiazepin-3-yl)-piperidin-1-yl]-butan-1,4-dion,
(31) (*S*)-2-(4-Amino-3-chlor-5-trifluormethyl-benzyl)-4-[4-(2-oxo-1,2,4,5-tetrahydro-1,3-benzdiazepin-3-yl)-piperidin-1-yl]-1-(4-perhydro-azepin-1-yl-pipeddin-1-yl)-butan-1,4-dion,
(32) (*S*)-2-(4-Amino-3-chlor-5-trifluormethyl-benzyl)-1-[4-(4-methyl-perhydro-1,4-di-azepin-1-yl)-piperidin-1-yl]-4-[4-(2-oxo-1,2,4,5-tetrahydro-1,3-benzdiazepin-3-yl)-piperidin-1-yl]-butan-1,4-dion,
(33) (*S*)-2-(4-Amino-3-chlor-5-trifluormethyl-benzyl)-1-[4-(4-isopropyl-piperazin-1-yl)-piperidin-1-yl]-4-[4-(2-oxo-1,2,4,5-tetrahydro-1,3-benzdiazepin-3-yl)-piperidin-1-yl]-butan-1,4-dion,
(34) (*S*)-2-(4-Amino-3-chlor-5-trifluormethyl-benzyl)-1-1,4'-bipiperidinyl-1'-yl-4-[4-(2-oxo-1,2,4,5-tetrahydro-1,3-benzdiazepin-3-yl)-piperidin-1-yl]-butan-1,4-dion,
(35) (*S*)-2-(4-Amino-3-chlor-5-trifluormethyl-benzyl)-1-[4-(1-methyl-piperidin-4-yl)-perhydro-1,4-diazepin-1-yl]-4-[4-(2-oxo-1,2,4,5-tetrahydro-1,3-benzdiazepin-3-yl)-piperidin-1-yl]-butan-1,4-dion,
(36) (*S*)-2-(4-Amino-3-chlor-5-trifluormethyl-benzyl)-1-[3-(4-methyl-piperazin-1-yl)-azetidin-1-yl]-4-[4-(2-oxo-1,2,4,5-tetrahydro-1,3-benzdiazepin-3-yl)-piperidin-1-yl]-butan-1,4-dion,
(37) (*S*)-2-(4-Amino-3-chlor-5-trifluormethyl-benzyl)-4-[4-(2-oxo-1,2,4,5-tetrahydro-1,3-benzdiazepin-3-yl)-piperidin-1-yl]-1-(3-pyrrolidin-1-yl-azetidin-1-yl)-butan-1,4-dion,
(38) (*S*)-2-(4-Amino-3-chlor-5-trifluormethyl-benzyl)-4-[4-(2-oxo-1,2,4,5-tetrahydro-1,3-benzdiazepin-3-yl)-piperidin-1-yl]-1-(3-piperidin-1-yl-azetidin-1-yl)-butan-1,4-dion,
(39) (*S*)-2-(4-Amino-3-chlor-5-trifluormethyl-benzyl)-1-(4-azetidin-1-yl-piperidin-1-yl)-4-[4-(2-oxo-1,2,4,5-tetrahydro-1,3-benzdiazepin-3-yl)-piperidin-1-yl]-butan-1,4-dion,
(40) (*S*)-1-[4-(4-Acetyl-piperazin-1-yl)-piperidin-1-yl]-2-(4-amino-3-chlor-5-trifluormethyl-benzyl)-4-[4-(2-oxo-1,2,4,5-tetrahydro-1,3-benzdiazepin-3-yl)-piperidin-1-yl]-butan-1,4-dion,
(41) (*S*)-2-(4-Amino-3-chlor-5-trifluormethyl-benzyl)-1-(4-diethylaminomethyl-piperidin-1-yl)-4-[4-(2-oxo-1,2,4,5-tetrahydro-1,3-benzdiazepin-3-yl)-piperidin-1-yl]-butan-1,4-dion,
(42) (*S*)-2-(4-Amino-3-chlor-5-trifluormethyl-benzyl)-1-[4-(4-ethyl-piperazin-1-yl)-piperidin-1-yl]-4-[4-(2-oxo-1,2,4,5-tetrahydro-1,3-benzdiazepin-3-yl)-piperidin-1-yl]-butan-1,4-dion,
(43) (*S*)-2-(4-Amino-3-chlor-5-trifluormethyl-benzyl)-1-[4-(1-ethyl-piperidin-4-yl)-piperazin-1-yl]-4-[4-(2-oxo-1,2,4,5-tetrahydro-1,3-benzdiazepin-3-yl)-pipendin-1-yl]-butan-1,4-dion,
(44) (*S*)-2-(4-Amino-3-chlor-5-trifluormethyl-benzyl)-4-[4-(2-oxo-1,2,4,5-tetrahydro-1,3-benzdiazepin-3-yl)-piperidin-1-yl]-1-(3,4,5,6-tetrahydro-2*H*-4,4'-bipyridinyl-1-yl)-butan-1,4-dion,
(45) (*S*)-2-(4-Amino-3-chlor-5-trifluormethyl-benzyl)-4-[4-(2-oxo-1,2,4,5-tetrahydro-1,3-benzdiazepin-3-yl)-piperidin-1-yl]-1-(4-pyridin-4-yl-piperazin-1-yl)-butan-1,4-dion,
(46) (*S*)-2-(4-Amino-3-chlor-5-trifluormethyl-benzyl)-4-[4-(2-oxo-1,2,4,5-tetrahydro-1,3-benzdiazepin-3-yl)-piperidin-1-yl]-1-(3-perhydro-azepin-1-yl-azetidin-1-yl)-butan-1,4-dion,
(47) (*S*)-2-(4-Amino-3-chlor-5-trifluormethyl-benzyl)-1-[4-(1-benzyl-piperidin-4-yl)-piperazin-1-yl]-4-[4-(2-oxo-1,2,4,5-tetrahydro-1,3-benzdiazepin-3-yl)-piperidin-1-yl]-butan-1,4-dion,
(48) (*S*)-2-(4-Amino-3-chlor-5-trifluormethyl-benzyl)-1-[4-(4-benzyl-piperazin-1-yl)-piperidin-1-yl]-4-[4-(2-oxo-1,2,4,5-tetrahydro-1,3-benzdiazepin-3-yl)-piperidin-1-yl]-butan-1,4-dion,
(49) (*S*)-2-(4-Amino-3-chlor-5-trifluormethyl-benzyl)-1-(7-dimethylaminomethyl-1,2,4,5-tetrahydro-3-benzazepin-3-yl)-4-[4-(2-oxo-1,2,4,5-tetrahydro-1,3-benzdiazepin-3-yl)-piperidin-1-yl]-butan-1,4-dion,
(50) (*S*)-2-(4-Amino-3-chlor-5-trifluormethyl-benzyl)-1-[4-(4-dimethylaminomethyl-phenyl)-piperidin-1-yl]-4-[4-(2-oxo-1,2,4,5-tetrahydro-1,3-benzdiazepin-3-yl)-piperidin-1-yl]-butan-1,4-dion,
(51) (*S*)-2-(4-Amino-3-chlor-5-trifluormethyl-benzyl)-4-[4-(2-oxo-1,2,4,5-tetrahydro-1,3-benzdiazepin-3-yl)-piperidin-1-yl]-1-{4-[4-(2,2,2-trifluor-ethyl)-piperazin-1-yl]-piperidin-1-yl}-butan-1,4-dion,
(52) (*S*)-2-(4-Amino-3-chlor-5-trifluormethyl-benzyl)-1-(1'-methansulfonyl-4,4'-bipiperidinyl-1-yl)-4-[4-(2-oxo-1,2,4,5-tetrahydro-1,3-benzdiazepin-3-yl)-piperidin-1-yl]-butan-1,4-dion,
(53) (*S*)-2-(4-Amino-3-chlor-5-trifluormethyl-benzyl)-1-(9-methyl-3,9-diaza-spiro[5.5]undec-3-yl)-4-[4-(2-oxo-1,2,4,5-tetrahydro-1,3-benzdiazepin-3-yl)-piperidin-1-yl]-butan-1,4-dion,
(54) (*S*)-2-(4-Amino-3-chlor-5-trifluormethyl-benzyl)-4-[4-(2-oxo-1,2,4,5-tetrahydro-1,3-benzdiazepin-3-yl)-piperidin-1-yl]-1-(4-piperidin-1-yl-methyl-piperidin-1-yl)-butan-1,4-dion,
(55) (*S*)-2-(4-Amino-3-chlor-5-trifluormethyl-benzyl)-1-[4-(2-dimethylamino-ethyl)-piperidin-1-yl]-4-[4-(2-oxo-1,2,4,5-tetrahydro-1,3-benzdiazepin-3-yl)-piperidin-1-yl]-butan-1,4-dion,
(56) (*S*)-2-(4-Amino-3-chlor-5-trifluormethyl-benzyl)-*N*-methyl-*N*-[2-(1-methylpiperidin-4-yl)-ethyl]-4-oxo-4-[4-(2-oxo-1,2,4,5-tetrahydro-1,3-benzdiazepin-3-yl)-piperidin-1-yl]-butyramid,
(57) (*S*)-2-(4-Amino-3-chtor-5-trifluormethyl-benzyl)-*N*-methyl-*N*-(1-methyl-piperidin-4-ylmethyl)-4-oxo-4-[4-(2-oxo-1,2,4,5-tetrahydro-1,3-benzdiazepin-3-yl)-piperidin-1-yl]-butyramid,
(58) (*S*)-2-(4-Amino-3-chlor-5-trifluormethyl-benzyl)-1-[4-(2-diethylamino-ethyl)-piperidin-1-yl]-4-[4-(2-oxo-1,2,4,5-tetrahydro-1,3-benzdiazepin-3-yl)-piperidin-1-yl]-butan-1,4-dion,
(59) (*S*)-2-(4-Amino-3-chlor-5-trifluormethyl-benzyl)-1-[4-(cyclopentyl-methylamino)-piperidin-1-yl]-4-[4-(2-oxo-1,2,4,5-tetrahydro-1,3-benzdiazepin-3-yl)-piperidin-1-yl]-butan-1,4-dion,
(60) (*S*)-2-(4-Amino-3-chlor-5-trifluormethyl-benzyl)-1-[4-(1,1-dioxo-1,6-isothiazolidin-2-yl)-piperidin-1-yl]-4-[4-(2-oxo-1,2,4,5-tetrahydro-1,3-benzdiazepin-3-yl)-piperidin-1-yl]-butan-1,4-dion,
(61) (*S*)-2-(4-Amino-3-chlor-5-trifluormethyl-benzyl)-1-[4-(2-oxo-perhydro-1,3-oxazin-3-yl)-pipendin-1-yl]-4-[4-(2-oxo-1,2,4,5-tetrahydro-1,3-benzdiazepin-3-yl)-piperidin-1-yl]-butan-1,4-dion,
(62) 1-{(*S*)-2-(4-Amino-3-chlor-5-trifluormethyl-benzyl)-4-oxo-4-[4-(2-oxo-1,2,4,5-tetrahydro-1,3-benzdiazepin-3-yl)-piperidin-1-yl]-butyryl}-piperidin-4-carbonsäuremethylester,
(63) 8-{(*S*)-2-(4-Amino-3-chlor-5-trifluormethyl-benzyl)-4-oxo-4-[4-(2-oxo-1,2,4,5-tetrahydro-1,3-benzdiazepin-3-yl)-piperidin-1-yl]-butyryl}-1,3,8-triaza-spiro[4.5]decan-2,4-dion,
(64) (*S*)-2-(4-Amino-3-chlor-5-trifluormethyl-benzyl)-1-[4-(2-oxo-imidazolidin-1-yl)-piperidin-1-yl]-4-[4-(2-oxo-1,2,4,5-tetrahydro-1,3-benzdiazepin-3-yl)-pipendin-1-yl]-butan-1,4-dion,
(65) (*S*)-2-(4-Amino-3-chlor-5-trifluormethyl-benzyl)-1-[4-(1-methyl-piperidin-4-yl)-piperazin-1-yl]-4-[4-(2-oxo-1,2,4,5-tetrahydro-1,3-benzdiazepin-3-yl)-piperidin-1-yl]-butan-1,4-dion,
(66) (*S*)-2-(4-Amino-3-chlor-5-trifluormethyl-benzyl)-1-(2-amino-4,5,7,8-tetrahydro-thiazolo[4,5-d]azepin-6-yl)-4-[4-(2-oxo-1,2,4,5-tetrahydro-1,3-benzdiazepin-3-yl)-piperidin-1-yl]-butan-1,4-dion,
(67) (*S*)-2-(4-Amino-3-chlor-5-trifluormethyl-benzyl)-1-[4-(2-methyl-imidazol-1-yl)-piperidin-1-yl]-4-[4-(2-oxo-1,2,4,5-tetrahydro-1,3-benzdiazepin-3-yl)-piperidin-1-yl]-butan-1,4-dion,
(68) (*S*)-2-(4-Amino-3-chlor-5-trifluormethyl-benzyl)-1-[4-(4-methyl-imidazol-1-yl)-piperidin-1-yl]-4-[4-(2-oxo-1,2,4,5-tetrahydro-1,3-benzdiazepin-3-yl)-piperidin-1-yl]-butan-1,4-dion,
(69) (*S*)-2-(4-Amino-3-chlor-5-trifluormethyl-benzyl)-4-[4-(2-oxo-1,2,4,5-tetrahydro-1,3-benzdiazepin-3-yl)-piperidin-1-yl]-1-(4-thiazol-2-yl-piperazin-1-yl)-butan-1,4-dion,
(70) (*S*)-2-(4-Amino-3-chlor-5-trifluormethyl-benzyl)-1-[4-(2,4-dimethyl-imidazol-1-yl)-piperidin-1-yl]-4-[4-(2-oxo-1,2,4,5-tetrahydro-1,3-benzdiazepin-3-yl)-piperidin-1-yl]-butan-1,4-dion,
(71) (*S*)-2-(4-Amino-3-chlor-5-trifluormethyl-benzyl)-1-(4-imidazol-1-yl-piperidin-1-yl)-4-[4-(2-oxo-1,2,4,5-tetrahydro-1,3-benzdiazepin-3-yl)-pipeddin-1-yl]-butan-1,4-dion,
(72) (*S*)-2-(4-Amino-3-chlor-5-trifluormethyl-benzyl)-4-[4-(2-oxo-1,2,4,5-tetrahydro-1,3-benzdiazepin-3-yl)-piperidin-1-yl]-1-(4-1,2,4-triazol-1-yl-piperidin-1-yl)-butan-1,4-dion,
(73) (*S*)-2-(4-Amino-3-chlor-5-trifluormethyl-benzyl)-1-[4-(1-aza-bicyclo[2.2.2]oct-3-yl)-piperazin-1-yl]-4-[4-(2-oxo-1,2,4,5-tetrahydro-1,3-benzdiazepin-3-yl)-piperidin-1-yl]-butan-1,4-dion,
(74) (*S*)-2-(4-Amino-3-chlor-5-trifluormethyl-benzyl)-*N*-(5-amino-pentyl)-4-oxo-4-[4-(2-oxo-1,2,4,5-tetrahydro-1,3-benzdiazepin-3-yl)-piperidin-1-yl]-butyramid,
(75) (*S*)-2-(4-Amino-3-chlor-5-trifluormethyl-benzyl)-*N*-(3-aminomethyl-benzyl)-4-oxo-4-[4-(2-oxo-1,2,4,5-tetrahydro-1,3-benzdiazepin-3-yl)-piperidin-1-yl]-butyramid,
(76) (*S*)-2-(4-Amino-3-chlor-5-trifluormethyl-benzyl)-1-[4-(8-methyl-8-aza-bicyclo[3.2.1 ]oct-3-yl)-piperazin-1-yl]-4-[4-(2-oxo-1,2,4,5-tetrahydro-1,3-benzdiazepin-3-yl)-piperidin-1-yl]-butan-1,4-dion,
(77) (*S*)-2-(4-Amino-3-chlor-5-trifluormethyl-benzyl)-4-[4-(2-oxo-1,2,4,5-tetrahydro-1,3-benzdiazepin-3-yl)-piperidin-1-yl]-1-(4-piperazin-1-yl-piperidin-1-yl)-butan-1,4-dion,
(78) (*S*)-2-(4-Amino-3-chlor-5-trifluormethyl-benzyl)-1-[4-(1*H*-imidazol-4-yl)-piperidin-1-yl]-4-[4-(2-oxo-1,2,4,5-tetrahydro-1,3-benzdiazepin-3-yl)-piperidin-1-yl]-butan-1,4-dion,
(79) (*S*)-2-(4-Amino-3-chlor-5-trifluormethyl-benzyl)-1-[4-(4-methyl-piperazin-1-yl)-piperidin-1-yl]-4-[4-(2-oxo-1,4-dihydro-2*H*-chinazolin-3-yl)-piperidin-1-yl]-butan-1,4-dion,
(80) (*S*)-2-(4-Amino-3-chlor-5-trifluormethyl-benzyl)-1-[4-(4-methyl-piperazin-1-yl)-piperidin-1-yl]-4-[4-(5-oxo-3-phenyl-4,5-dihydro-1,2,4-triazol-1-yl)-piperidin-1-yl]-butan-1,4-dion,
(81) (*S*)-2-(4-Amino-3-chlor-5-trifluormethyl-benzyl)-1-[4-(4-methyl-piperazin-1-yl)-piperidin-1-yl]-4-[4-(2-oxo-1,2-dihydro-imidazo[4,5-c]chinolin-3-yl)-piperidin-1-yl]-butan-1,4-dion,
(82) (*S*)-2-(4-Amino-3-chlor-5-trifluormethyl-benzyl)-1-[4-(4-methyl-piperazin-1-yl)-piperidin-1-yl]-4-[4-(2-oxo-1,2-dihydro-4*H*-thieno[3,4-d]pyrimidin-3-yl)-piperidin-1-yl]-butan-1,4-dion,
(83) (*S*)-2-(4-Amino-3-chlor-5-trifluormethyl-benzyl)-1-[4-(5-mothyl-2,5-diaza-bicyclo[2.2.1]hept-2-yl)-piperidin-1-yl]-4-[4-(2-oxo-1,2,4,5-tetrahydro-1,3-benzdiazepin-3-yl)-piperidin-1-yl]-butan-1,4-dion,
(84) (*S*)-2-(4-Amino-3,5-bis-trifluormethyl-benzyl)-1-[4-(4-methyl-perhydro-1,4-diazepin-1-yl)-piperidin-1-yl]-4-[4-(2-oxo-1,2,4,5-tetrahydro-1,3-benzdiazepin-3-yl)-piperidin-1-yl]-butan-1,4-dion,
(85) (*S*)-2-(4-Amino-3,5-bis-trifluormethyl-benzyl)-1-[4-(1-methyl-plperidin-4-yl)-perhydro-1,4-diazepin-1-yl]-4-[4-(2-oxo-1,2,4,5-tetrahydro-1,3-benzdiazepin-3-yl)-piperidin-1-yl]-butan-1,4-dion,
(86) (*S*)-2-(4-Amino-3,5-bis-trifluormethyl-benzyl)-1-[4-(1-methyl-piperidin-4-yl)-piperazin-1-yl]-4-[4-(2-oxo-1,2,4,5-tetrahydro-1,3-benzdiazepin-3-yl)-piperidin-1-yl]-butan-1,4-dion,
(87) (*S*)-2-(4-Amino-3,5-bis-trifluormethyl-benzyl)-1-[4-(1-aza-bicyclo[2.2.2]oct-3-yl)-piperazin-1-yl]-4-[4-(2-oxo-1,2,4,5-tetrahydro-1,3-benzdiazepin-3-yl)-piperidin-1-yl]-butan-1,4-dion,
(88) (*S*)-2-(4-Amino-3,5-bis-trifluormethyl-benzyl)-1-[4-(4-methyl-piperazin-1-yl)-piperidin-1-yl]-4-[4-(2-oxo-1,2,4,5-tetrahydro-1,3-benzdiazepin-3-yl)-piperidin-1-yl]-butan-1,4-dion,
(89) (*S*)-2-(4-Amino-3,5-bis-trifluormethyl-benzyl)-4-[4-(2-oxo-1,2,4,5-tetrahydro-1,3-benzdiazepin-3-yl)-piperidin-1-yl]-1-(4-perhydro-azepin-1-yl-piperidin-1-yl)-butan-1,4-dion,
(90) (*S*)-2-(4-Amino-3,5-bis-trifluormethyl-benzyl)-1-1,4'-bipiperidinyl-1'-yl-4-[4-(2-oxo-1,2,4,5-tetrahydro-1,3-benzdiazepin-3-yl)-piperidin-1-yl]-butan-1,4-dion,
(91) (*S*)-2-(4-Amino-3,5-bis-trifluormethyl-benzyl)-4-[4-(2-oxo-1,2,4,5-tetrahydro-1,3-benzdiazepin-3-yl)-piperidin-1-yl]-1-(4-piperazin-1-yl-piperidin-1-yl)-butan-1,4-dion,
(92) 4-(2-Oxo-1,2,4,5-tetrahydro-1,3-benzdiazepin-3-yl)-piperidin-1-carbonsäure-{(*R*)-1-(4-amino-3,5-bis-trifluormethyl-benzyl)-2-[4-(4-methyl-piperazin-1-yl)-piperidin-1-yl]-2-oxo-ethyl}-amid,
(93) 4-(2-Oxo-1,2,4,5-tetrahydro-1,3-benzdiazepin-3-yl)-piperidin-1-carbonsäure-{(*R*)-1-(4-amino-3,5-bis-trifluormethyl-benzyl)-2-[4-(1-methyl-piperidin-4-yl)-piperazin-1-yl]-2-oxo-ethyl}-amid,
(94) 4-(2-Oxo-1,2,4,5-tetrahydro-1,3-benzdiazepin-3-yl)-piperidin-1-carbonsäure-[(*R*)-1-(4-amino-3,5-bis-trifluormethyl-benzyl)-2-1,4'-bipiparidinyl-1'-yl-2-oxo-ethyl]-amid,
(95) 4-(2-Oxo-1,2,4,5-tetrahydro-1,3-benzdiazepin-3-yl)-piperidin-1-carbonsäure-[(*R*)-1-(4-amino-3,5-bis-trifluormethyl-benzyl)-2-oxo-2-(4-piperazin-1-yl-piperidin-1-yl)-ethyl]-amid,
(96) 4-(2-Oxo-1,2,4,5-tetrahydro-1,3-benzdiazepin-3-yl)-piperidin-1-carbonsäure-(R)-1-(4-amino-3-chlor-5-trifluormethyl-benzyl)-2-[4-(4-methyl-piperazin-1-yl)-piperidin-1-yl]-2-oxo-ethylester,
(97) 4-(2-Oxo-1,4-dihydro-2*H*-chinazolin-3-yl)-piperidin-1-carbonsäure-[1-(4-brom-3-methyl-benzyl)-2-(1'-methyl-[4,4']bipiperidinyl-1-yl)-2-oxo-ethyl]-amid,
(98) 4-(2-Oxo-1,4-dihydro-2*H*-chinazolin-3-yl)-piperidin-l-carbonsäure-{1-(4-brom-3-methyl-benzyl)-2-[4-(4-methyl-piperazin-1-yl)-piperidin-1-yl]-2-oxo-ethyl}-amid,
(99) 4-(2-Oxo-1,4-dihydro-2*H*-chinazolin-3-yl)-piperidin-1-carbonsäure-{1-(4-brom-3-methyl-benzyl)-2-[4-(1-methyl-piperidin-4-yl)-piperazin-1-yl]-2-oxo-ethyl}-amid,
(100) 2-(4-Brom-3-methyl-benzyl)-1-(1'-methyl-[4,4']bipiperidinyl-1-yl)-4-[4-(2-oxo-1,4-dihydro-2*H*-chinazolin-3-yl)-piperidin-1-yl]-butan-1,4-dion,
(101) 1-[1,4']Bipiperidinyl-1'-yl-2-(4-brom-3-methyl-benzyl)-4-[4-(2-oxo-1,4-dihydro-2*H*-chinazolin-3-yl)-piperidin-1-yl]-butan-1,4-dion,
(102) 4-(2-Oxo-1,4-dihydro-2H-chinazolin-3-yl)-piperidin-1-carbonsäure{1-(4-chlor-3-methyl-benzyl)-2-[4-(4-methyl-piperazin-1-yl)-piperidin-1-yl]-2-oxo-ethyl}-amid,
(103) 4-(2-Oxo-1,4-dihydro-2*H*-chinazolin-3-yl)-piperidin-1-carbonsäure-{1-(4-chlor-3-methyl-benzyl)-2-[4-(1-methyl-piperidin-4-yl)-piperazin-1-yl]-2-oxo-ethyl}-amid,
(104) 4-(2-Oxo-1,4-dihydro-2*H*-chinazolin-3-yl)-piperidin-1-carbonsäure-[1-(4-chlor-3-methyl-benzyl)-2-(1'-methyl-[4,4']bipiperidinyl-1-yl)-2-oxo-ethyl]-amid,
(105) 2-(4-Chlor-3-methyl-benzyl)-1-[4-(4-methyl-piperazin-1-yl)-piperidin-1-yl]-4-[4-(2-oxo-1,4-dihydro-2*H*-chinazolin-3-yl)-piperidin-1-yl]-butan-1,4-dion,
(106) 2-(4-Chlor-3-methyl-benzyl)-1-[4-(1-methyl-piperidin-4-yl)-piperazin-1-yl]-4-[4-(2-oxo-1,2,4,5-tetrahydro-1,3-benzdiazepin-3-yl)-piperidin-1-yl]-butan-1,4-dion,
(107) 2-(4-Chlor-3-methyl-benzyl)-1-[4-(4-methyl-piperazin-1-yl)-piperidin-1-yl]-4-[4-(2-oxo-1,2,4,5-tetrahydro-1,3-benzdiazepin-3-yl)-piperidin-1-yl]-butan-1,4-dion,
(108) 2-(3-Brom-4-chlor-5-methyl-benzyl)-1-[4-(4-methyl-piperazin-1-yl)-piperidin-1-yl]-4-[4-(2-oxo-1,4-dihydro-2*H*-chinazolin-3-yl)-piperidin-1-yl]-butan-1,4-dion,
(109) 2-(3-Brom-4-chlor-5-methyl-benzyl)-1-(1'-methyl-[4,4']bipiperidinyl-1-yl)-4-[4-(2-oxo-1,4-dihydro-2*H*-chinazolin-3-yl)-piperidin-1-yl]-butan-1,4-dion,
(110) 2-(3-Brom-4-chlor-5-methyl-benzyl)-1-[4-(1-methyl-piperidin-4-yl)-piperazin-1-yl]-4-[4-(2-oxo-1,4-dihydro-2*H*-chinazolin-3-yl)-piperidin-1-yl]-butan-1,4-dion,
(111)- 4-(2-Oxo-1,4-dihydro-2*H*-chinazolin-3-yl)-piperidin-1-carbonsäure{1-(3-brom-4-chlor-5-methyl-benzyl)-2-[4-(4-methyl-piperazin-1-yl)-piperidin-1-yl]-2-oxo-ethyl}-amid,
(112) 4-(2-Oxo-1,4-dihydro-2*H*-chinazolin-3-yl)-piperidin-1-carbonsäure-{1-(3-brom-4-chlor-5-methyl-benzyl)-2-[4-(1-methyl-piperidin-4-yl)-piperazin-1-yl]-2-oxo-ethyl}-amid,
(113) 4-(2-Oxo-1,4-dihydro-2*H*-chinazolin-3-yl)-piperidin-1-carbonsäure-[1-(3-brom-4-chlor-5-methyl-benzyl)-2-(1'-methyl-[4,4']bipiperidinyl-1-yl)-2-oxo-ethyl]-amid,
(114) 2-(4-Chlor-3-trifluormethyl-benzyl)-1-[4-(4-methyl-piperazin-1-yl)-piperidin-1-yl]-4-[4-(2-oxo-1,4-dihydro-2*H*-chinazolin-3-yl)-piperidin-1-yl]-butan-1,4-dion,
deren Enantiomere, deren Diastereomere und deren Salze eine herausragende und die Verbindungen
(1) (*S*)-2-(4-Amino-3-chlor-5-trifluormethyl-benzyl)-1-[4-(4-methyl-piperazin-1-yl)-piperidin-1-yl]-4-[4-(2-oxo-1,2,4,5-tetrahydro-1,3-benzdiazepin-3-yl)-piperidin-1-yl]-butan-1,4-dion,
(2) 2-(4-Amino-3-chlor-5-trifluormethyl-benzyl)-1-(1'-methyl-[4,4']bipiperidinyl-1-yl)-4-[4-(2-oxo-1,4-dihydro-2*H*-quinazolin-3-yl)-piperidin-1-yl]-butan-1,4-dion,
(3) 2-(4-Amino-3-chlor-5-trifluormethyl-benzyl)-1-[4-(4-methyl-piperazin-1-yl)-piperidin-1-yl]-4-[4-(2-oxo-1,4-dihydro-2*H*-quinazolin-3-yl)-piperidin-1-yl]-butan-1,4-dion,
(4) 2-(4-Amino-3-chlor-5-trifluormethyl-benzyl)-1-[1,4']bipiperidinyl-1'-yl-4-[4-(2-oxo-1,4-dihydro-2*H*-quinazolin-3-yl)-piperidin-1-yl]-butan-1,4-dion,
(5) 2-(4-Amino-3-chlor-5-trifluormethyl-benzyl)-4-[4-(2-oxo-1,4-dihydro-2*H-*quinazolin-3-yl)-piperidin-1-yl]-1-(4-pyridin-4-yl-piperazin-1-yl)-butan-1,4-dion,
(6) 4-(2-Oxo-1,2,4,5-tetrahydro-1,3-benzdiazepin-3-yl)-piperidin-1-carbonsäure-[(*R*)-1-(4-amino-3-chlor-5-trifluormethyl-benzyl)-2-1,4'-bipiperidinyl-1'-yl-2-oxo-ethyl]-amid,
(7) 4-(2-Oxo-1,2,4,5-tetrahydro-1,3-benzdiazepin-3-yl)-piperidin-1-carbonsäure-[(*R*)-1-(4-amino-3-chlor-5-trifluormethyl-benzyl)-2-(4-dimethylamino-piperidin-1-yl)-2-oxo-ethyl]-amid,
(8) 4-(2-Oxo-1,2,4,5-tetrahydro-1,3-benzdiazepin-3-yl)-piperidin-1-carbonsäure-[(*R*)-1-(4-amino-3-chlor-5-trifluormethyl-benzyl)-2-oxo-2-(4-pyridin-4-yl-piperazin-1-yl)-ethyl]-amid,
(9) 4-(2-Oxo-1,2,4,5-tetrahydro-1,3-benzdiazepin-3-yl)-piperidin-1-carbonsäure-[(*R*)-1-(4-amino-3-chlor-5-trifluormethyl-benzyl)-2-(1'-methyl-4,4'-bipiperidinyl-1-yl)-2-oxo-ethyl]-amid,
(10) [1'-((*R*)-3-(4-Amino-3-chlor-5-trifluormethyl-phenyl)-2-{[4-(2-oxo-1,2,4,5-tetrahydro-1,3-benzdiazepin-3-yl)-piperidin-1-carbonyl]-amino}-propionyl)-4,4'-bipiperidinyl-1-yl]-essigsäureethylester,
(11) 4-(2-Oxo-1,2,4,5-tetrahydro-1,3-benzdiazepin-3-yl)-piperidin-1-carbonsäure-{(*R*)-1-(4-amino-3-chlor-5-trifluormethyl-benzyl)-2-[4-(4-methyl-piperazin-1-yl)-piperidin-1-yl]-2-oxo-ethyl}-amid,
(12) 4-(2-Oxo-1,2,4,5-tetrahydro-1,3-benzdiazepin-3-yl)-piperidin-1-carbonsäure-{(*R*)-1-(4-amino-3-chlor-5-trifluormethyl-benzyl)-2-[4-(1-methyl-piperidin-4-yl)-piperazin-1-yl]-2-oxo-ethyl}-amid,
(13) 4-(2-Oxo-1,2,4,5-tetrahydro-1,3-benzdiazepin-3-yl)-piperidin-1-carbonsäure-[(*R*)-1-(4-amino-3-chlor-5-trifluormethyl-benzyl)-2-(4-azetidin-1-yl-piperidin-1-yl)-2-oxo-ethyl]-amid,
(14) 4-(2-Oxo-1,2,4,5-tetrahydro-1,3-benzdiazepin-3-yl)-piperidin-1-carbonsäure-{(*R*)-1-(4-amino-3-chlor-5-trifluormethyl-benzyl)-2-[4-(5-methyl-2,5-diaza-bicyclo[2.2.1]hept-2-yl)-piperidin-1-yl]-2-oxo-ethyl}-amid,
(15) 4-(2-Oxo-1,2,4,5-tetrahydro-1,3-benzdiazepin-3-yl)-piperidin-1-carbonsäure-[(*R*)-1-(4-amino-3-chlor-5-trifluormethyl-benzyl)-2-oxo-2-(4-piperazin-1-yl-piperidin-1-yl)-ethyl]-amid,
(16) [1'-((*R*)-3-(4-Amino-3-chlor-5-trifluormethyl-phenyl)-2-{[4-(2-oxo-1,2,4,5-tetrahydro-1,3-benzdiazepin-3-yl)-piperidin-1-carbönyl]-amino}-propionyl)-4,4'-bipiperidinyl-1-yl]-essigsäure,
(17) 4-(2-Oxo-1,2,4,5-tetrahydro-1,3-benzdiazepin-3-yl)-piperidin-1-carbonsäure-[1-(4-ämino-3-chlor-5-trifluormethyl-benzyl)-2-1,4'-bipiperidinyl-1'-yl-2-oxo-ethyl]-amid,
(18) (*S*)-2-(4-Amino-3-brom-5-trifluormethyl-benzyl)-1-[4-(4-methyl-piperazin-1-yl)-piperidin-1-yl]-4-[4-(2-oxo-1,2,4,5-tetrahydro-1,3-benzdiazepin-3-yl)-piperidin-1-yl]-butan-1,4-dion,
(19) (*S*)-2-(4-Amino-3-brom-5-trifluormethyl-benzyl)-4-[4-(2-oxo-1,2,4,5-tetrahydro-1,3-benzdiazepin-3-yl)-piperidin-1-yl]-1-(4-pyridin-4-yl-piperazin-1-yl)-butan-1,4-dion,
(20) (*S*)-2-(4-Amino-3-brom-5-trifluormethyl-benzyl)-1-1,4'-bipiperidinyl-1'-yl-4-[4-(2-oxo-1,2,4,5-tetrahydro-1,3-benzdlazepin-3-yl)-piperidin-1-yl]-butan-1,4-dion,
(21) (*S*)-2-(4-Amino-3-brom-5-trifluormethyl-benzyl)-1-[4-(1-methyl-piperidin-4-yl)-piperazin-1-yl]-4-[4-(2-oxo-1,2,4,5-tetrahydro-1,3-benzdiazepin-3-yl)-piperidin-1-yl]-butan-1,4-dion,
(22) (*S*)-2-(4-Amino-3-brom-5-trifluormethyl-benzyl)-1-(1'-methyl-4,4'-bipiperidinyl-1-yl)-4-[4-(2-oxo-1,2,4,5-tetrahydro-1,3-benzdiazepin-3-yl)-pipendin-1-yl]-butan-1,4-dion,
(23) (*S*)-2-(4-Amino-3-chlor-5-trifluormethyl-benzyl)-4-[4-(2-oxo-1,2,4,5-tetrahydro-1,3-benzdiazepin-3-yl)-piperidin-1-yl]-1-(4-pyrrolidin-1-yl-piperidin-1-yl)-butan-1,4-dion,
(24) (*S*)-2-(4-Aminp-3-chlor-5-trifluormethyl-benzyl)-1-(1'-methyl-4,4'-bipiperidinyl-1-yl)-4-[4-(2-oxo-1,2,4,5-tetrahydro-1,3-benzdiazepin-3-yl)-piperidin-1-yl]-butan-1,4-dion,
(25) (*S*)-2-(4-Amino-3-chlor-5-trifluormethyl-benzyl)-1-[4-(4-cyclopropylmethyl-piperazin-1-yl)-piperidin-1-yl]-4-[4-(2-oxo-1,2,4,5-tetrahydro-1,3-benzdiazepin-3-yl)-piperidin-1,4-yl]-butan-1,4-dion,
(26) (*S*)-2-(4-Amino-3-chlor-5-trifluormethyl-benzyl)-4-[4-(2-oxo-1,2,4,5-tetrahydro-1,3-benzdiazepin-3-yl)-piperidin-1-yl]-1-(4-perhydro-azepin-1-yl-piperidin-1-yl)-butan-1,4-dion,
(27) (*S*)-2-(4-Amino-3-chlor-5-triftuormethyl-benzyl)-1-[4-(4-methyt-perhydro-1,4-diazepin-1-yl)-piperidin-1-yl]-4-[4-(2-oxo-1,2,4,5-tetrahydro-1,3-benzdiazepin-3-yl)-piperidin-1-yl]-butan-1,4-dion,
(28) (*S*)-2-(4-Amino-3-chlor-5-trifluormethyl-benzyl)-1-1,4'-bipiperidinyl-1'-yl-4-[4-(2-oxo-1,2,4,5-tetrahydro-1,3-benzdiazepin-3-yl)-pipeddin-1-yl]-butan-1,4-dion,
(29) (*S*)-2-(4-Amino-3-chlor-5-trifluormethyl-benzyl)-1-[4-(1-methyl-piperidin-4-yl)-perhydro-1,4-diazepin-1-yl]-4-[4-(2-oxo-1,2,4,5-tetrahydro-1,3-benzdiazepin-3-yl)-piperidin-1-yl]-butan-1,4-dion,
(30) (*S*)-2-(4-Amino-3-chlor-5-trifluormethyl-benzyl)-1-[3-(4-methyl-piperazin-1-yl)-azetidin-1-yl]-4-[4-(2-oxo-1,2,4,5-tetrahydro-1,3-benzdiazepin-3-yl)-piparidin-1-yl]-butan-1,4-dion,
(31) (*S*)-2-(4-Amino-3-chlor-5-trifluormethyl-benzyl)-1-(4-azetidin-1-yl-piperidin-1-yl)-4-[4-(2-oxo-1,2,4,5-tetrahydro-1,3-benzdiazepin-3-yl)-piperidin-1-yl]-butan-1,4-dion,
(32) (*S*)-1-[4-(4-Acetyl-piperazin-1-yl)-piperidin-1-yl]-2-(4-amino-3-chlor-5-trifluormethyl-benzyl)-4-[4-(2-oxo-1,2,4,5-tetrahydro-1,3-benzdiazepin-3-yl)-piperidin-1-yl]-butan-1,4-dion,
(33) (*S*)-2-(4-Amino-3-chlor-5-trifluormethyl-benzyl)-1-(4-diethylaminomethyl-piperidin-1-yl)-4-[4-(2-oxo-1,2,4,5-tetrahydro-1,3-benzdiazepin-3-yl)-piperidin-1-yl]-butan-1,4-dion,
(34) (*S*)-2-(4-Amino-3-chlor-5-trifluormethyl-benzyl)-1-[4-(4-ethyl-piperazin-1-yl)-piperidin-1-yl]-4-[4-(2-oxo-1,2,4,5-tetrahydro-1,3-benzdiazepin-3-yl)-pipendin-1-yl]-butan-1,4-dion,
(35) (*S*)-2-(4-Amino-3-chlor-5-trifluormethyl-benzyl)-1-[4-(1-ethyl-piperidin-4-yl)-piperazin-1-yl]-4-[4-(2-oxo-1,2,4,5-tetrahydro-1,3-benzdiazepin-3-yl)-piperidin-1-yl]-butan-1,4-dion,
(36) (*S*)-2-(4-Amino-3-chlor-5-trifluormethyl-benzyl)-4-[4-(2-oxo-1,2,4,5-tetrahydro-1,3-benzdiazepin-3-yl)-piperidin-1-yl]-1-(3,4,5,6-tetrahydro-2*H*-4,4'-bipyridinyl-1-yl)-butan-1,4-dion,
(37) (*S*)-2-(4-Amino-3-chlor-5-trifluormethyl-benzyl)-4-[4-(2-oxo-1,2,4,5-tetrahydro-1,3-benzdiazepin-3-yl)-piperidin-1-yl]-1-(4-pyridin-4-yl-piperazin-1-yl)-butan-1,4-dion,
(38) (*S*)-2-(4-Amino-3-chlor-5-trifluormethyl-benzyl)-1-[4-(1-benzyl-piperidin-4-yl)-piperazin-1-yl]-4-[4-(2-oxo-1,2,4,5-tetrahydro-1,3-benzdiazepin-3-yl)-piperidin-1-yl]-butan-1,4-dion,
(39) (*S*)-2-(4-Amino-3-chlor-5-trifluormethyl-benzyl)-1-(1'-methansulfonyl-4,4'-bipiperidinyl-1-yl)-4-[4-(2-oxo-1,2,4,5-tetrahydro-1,3-benzdiazepin-3-yl)-piperidin-1-yl]-butan-1,4-dion,
(40) (*S*)-2-(4-Amino-3-chlor-5-trifluormethyl-benzyl)-1-(9-methyl-3,9-diaza-spiro[5.5]undec-3-yl)-4-[4-(2-oxo-1,2,4,5-tetrahydro-1,3-benzdiazepin-3-yl)-piperidin-1-yl]-butan-1,4-dion,
(41) (*S*)-2-(4-Amino-3-chlor-5-trifluormethyl-benzyl)-4-[4-(2-oxo-1,2,4,5-tetrahydro-1,3-benzdiazepin-3-yl)-piperidin-1-yl]-1-(4-piperidin-1-yl-methyl-piperidin-1-yl)-butan-1,4-dion,
(42) (*S*)-2-(4-Amino-3-chlor-5-trifluormethyl-benzyl)-1-[4-(2-dimethylamino-ethyl)-piperidin-1-yl]-4-[4-(2-oxo-1,2,4,5-tetrahydro-1,3-benzdiazepin-3-yl)-piperidin-1-yl]-butan-1,4-dion,
(43) (*S*)-2-(4-Amino-3-chlor-5-trifluormethyl-benzyl)-N-methyl-N-[2-(1-methylpiperidin-4-yl)-ethyl]-4-oxo-4-[4-(2-oxo-1,2,4,5-tetrahydro-1,3-benzdiazepin-3-yl)-piperidin-1-yl]-butyramid,
(44) (*S*)-2-(4-Amino-3-chlor-5-trifluormethyl-benzyl)-*N*-methyl-*N*-(1-methyl-piperidin-4-ylmethyl)-4-oxo-4-[4-(2-oxo-1,2,4,5-tetrahydro-1,3-benzdiazepin-3-yl)-piperidin-1-yl]-butyramid,
(45) (*S*)-2-(4-Amino-3-chlor-5-trifluormethyl-benzyl)-1-[4-(2-diethylamino-ethyl)-piperidin-1-yl]-4-[4-(2-oxo-1,2,4,5-tetrahydro-1,3-benzdtazepin-3-yl)-piperidin-1-yl]-butan-1,4-dion,
(46) (*S*)-2-(4-Amino-3-chlor-5-trifluormethyl-benzyl)-1-[4-(2-oxo-perhydro-1,3-oxazin-3-yl)-piperidin-1-yl]-4-[4-(2-oxo-1,2,4,5-tetrahydro-1,3-benzdiazepin-3-yl)-piperidin-1-yl]-butan-1,4-dion,
(47) (*S*)-2-(4-Amino-3-chlor-5-trifluormethyl-benzyl)-1-[4-(2-oxo-imidazolidin-1-yl)-pipendin-1-yl]-4-[4-(2-oxo-1,2,4,5-tetrahydro-1,3-benzdiazepin-3-yl)-piperidin-1-yl]-butan-1,4-dion,
(48) (*S*)-2-(4-Amino-3-chlor-5-trifluormethyl-benzyl)-1-[4-(1-methyl-piperidin-4-yl)-piperazin-1-yl]-4-[4-(2-oxo-1,2,4,5-tetrahydro-1,3-benzdiazepin-3-yl)-piperidin-1-yl]-butan-1,4-dion,
(49) (*S*)-2-(4-Amino-3-chlor-5-trifluormethyl-benzyl)-1-[4-(2-methyl-irnidazol-1-yl)-piperidin-1-yl]-4-[4-(2-oxo-1,2,4,5-tetrahydro-1,3-benzdiazepin-3-yl)-piperidin-1-yl]-butan-1,4-dion,
(50) (*S*)-2-(4-Amino-3-chlor-5-trifluormethyl-benzyl)-1-[4-(4-methyl-imidazol-1-yl)-piperidin-1-yl]-4-[4-(2-oxo-1,2,4,5-tetrahydro-1,3-benzdiazepin-3-yl)-piperidin-1-yl]-butan-1,4-dion,
(51) (*S*)-2-(4-Amino-3-chlor-5-trifluormethyl-benzyl)-1-[4-(2,4-dimethyl-imidazol-1-yl)-piperidin-1-yl]-4-[4-(2-oxo-1,2,4,5-tetrahydro-1,3-benzdiazepin-3-yl)-piperidin-1-yl]-butan-1,4-dion,
(52) (*S*)-2-(4-Amino-3-chlor-5-trifluormethyl-benzyl)-1-(4-imidazol-1-yl-piperidin-1-yl)-4-[4-(2-oxo-1,2,4,5-tetrahydro-1,3-benzdiazepin-3-yl)-piperidin-1-yl]-butan-1,4-dion,
(53) (*S*)-2-(4-Amino-3-chlor-5-trifluormethyl-benzyl)-4-[4-(2-oxo-1,2,4,5-tetrahydro-1,3-benzdiazepin-3-yl)-piperidin-1-yl]-1-(4-1,2,4-triazol-1-yl-piperidin-1-yl)-butan-1,4-dion,
(54) (*S*)-2-(4-Amino-3-chlor-5-trifluormethyl-benzyl)-1-[4-(1-aza-bicyclo[2.2.2]oct-3-yl)-piperazin-1-yl]-4-[4-(2-oxo-1,2,4,5-tetrahydro-1,3-benzdiazepin-3-yl)-piperidin-1-yl]-butan-1,4-dion,
(55) (*S*)-2-(4-Amino-3-chlor-5-trifluormethyl-benzyl)-1-[4-(8-methyl-8-aza-bicyclo[3.2.1]oct-3-yl)-piperazin-1-yl]-4-[4-(2-oxo-1,2,4,5-tetrahydro-1,3-benzdiazepin-3-yl)-piperidin-1-yl]-butan-1,4-dion,
(56) (S)-2-(4-Amino-3-chlor-5-trifluormethyl-benzyl)-4-[4-(2-oxo-1,2,4,5-tetrahydro-1,3-benzdiazepin-3-yl)-piperidin-1-yl]-1-(4-piperazin-1-yl-piperidin-1-yl)-butan-1,4-dion,
(57) (*S*)-2-(4-Amino-3-chlor-5-trifluormethyl-benzyl)-1-[4-(1*H*-imidazol-4-yl)-piperidin-1-yl]-4-[4-(2-oxo-1,2,4,5-tetrahydro-1,3-benzdiazepi n-3-yl)-piperidin-1-yl]-butan-1,4-dion,
(58) (*S*)-2-(4-Amino-3-chlor-5-trifluormethyl-benzyl)-1-[4-(4-methyl-piperazin-1-yl)-piperidin-1-yl]-4-[4-(2-oxo-1,4-dihydro-2H-chinazolin-3-yl)-piperidin-1-yl]-butan-1,4-dion,
(59) (*S*)-2-(4-Amino-3-chlor-5-trifluormethyl-benzyl)-1-[4-(4-methyl-piperazin-1-yl)-piperidin-1-yl]-4-[4-(5-oxo-3-phenyl-4,5-dihydro-1,2,4-triazol-1-yl)-piperidin-1-yl]-butan-1,4-dion,
(60) (*S*)-2-(4-Amino-3-chfor-5-trifluormethyl-benzyl)-1-[4-(4-methyl-piperazin-1-yl)-piperidin-1-yl]-4-[4-(2-oxo-1,2-dihydro-imidazo[4,5-c]chinolin-3-yl)-piperidin-1-yl]-butan-1,4-dion,
(61) (*S*)-2-(4-Ammo-3-chlor-5-triftuormethyl)-1-[4-(4-methyl-piperazin-1-yl)-piperidin-1-yl]-4-[4-(2-oxo-1,2-dihydro-4*H*-thieno[3,4-d]pyrimidin-3-yl)-piperidin-1-yl]-butan-1,4-dion,
(62) (*S*)-2-(4-Amino-3-chlor-5-trifiuormethyl-benzyl)-1-[4-(5-methyl-2,5-diaza-bicyclo[2.2.1]hept-2-yl)-piperidin-1-yl]-4-[4-(2-oxo-1,2,4,5-tetrahydro-1,3-benzdiazepin-3-yl)-piperidin-1-yl]-butan-1,4-dion,
(63) (*S*)-2-(4-Amino-3,5-bis-triftuormethyl-benzyl)-1-[4-(4-methyl-perhydro-1,4-diazepin-1-yl)-piperidin-1-yl]-4-[4-(2-oxo-1,2,4,5-tetrahydro-1,3-benzdiazepin-3-yl)-piperidin-1-yl]-butan-1,4-dion,
(64) (*S*)-2-(4-Amino-3,5-bis-trifluormethyl-benzyl)-1-[4-(1-methyl-piperidin-4-yl)-perhydro-1,4-diazepin-1-yl]-4-[4-(2-oxo-1,2,4,5-tetrahydro-1,3-benzdiazepin-3-yl)-piparidin-1-yl]-butan-1,4-dion,
(65) (*S*)-2-(4-Amino-3,5-bis-trifluormethyl-benzyl)-1-[4-(1-methyl-pipertdin-4-yl)-piperazin-1-yl]-4-[4-(2-oxo-1,2,4,5-tetrahydro-1,3-benzdiazepin-3-yl)-piperidin-1-yl]-butan-1,4-dion,
(66) (*S*)-2-(4-Amino-3,5-bis-trifluormethyl-benzyl)-1-[4-(1-aza-bicyclo[2.2.2]oct-3-yl)-piperazin-1-yl]-4-[4-(2-oxo-1,2,4,5-tetrahydro-1,3-benzdiazepin-3-yl)-piperidin-1-yl]-butan-1,4-dion,
(67) (*S*)-2-(4-Amino-3,5-bis-trifluormethyl-benzyl)-1-[4-(4-methyl-piperazin-1-yl)-piperidin-1-yl]-4-[4-(2-oxo-1,2,4,5-tetrahydro-1,3-benzdiazepin-3-yl)-piperidin-1-yl]-butan-1,4-dion,
(68) (*S*)-2-(4-Amino-3,5-bis-trifluormethyl-benzyl)-4-[4-(2-oxo-1,2,4,5-tetrahydro-1,3-benzdiazepin-3-yl)-piperidin-1-yl]-1-(4-perhydro-azepin-1-yl-piperidin-1-yl)-butan-1,4-dion,
(69) (*S*)-2-(4-Amino-3,5-bis-trifluormethyl-benzyl)-1-1,4'-bipiperidinyl-1'-yl-4-[4-(2-oxo-1,2,4,5-tetrahydro-1,3-benzdiazepin-3-yl)-piperidin-1-yl]-butan-1,4-dion,
(70) (*S*)-2-(4-Amino-3,5-bis-trifluormethyl-benzyl)-4-[4-(2-oxo-1,2,4,5-tetrahydro-1,3-benzdiazepin-3-yl)-piperidin-1-yl]-1-(4-piperazin-1-yl-piperidin-1-yl)-butan-1,4-dion,
(71) 4-(2-Oxo-1,2,4,5-tetrahydro-1,3-benzdiazepin-3-yl)-piperidin-1-carbonsäure-{(*R*)-1-(4-amino-3,5-bis-trifluormethyl-benzyl)-2-[4-(4-methyl-piperazin-1-yl)-piperidin-1-yl]-2-oxo-ethyl}-amid,
(72) 4-(2-Oxo-1,2,4,5-tetrahydro-1,3-benzdiazepin-3-yl)-piperidin-1-carbonsäure-{(*R*)-1-(4-amino-3,5-bis-trifluormethyl-benzyl)-2-[4-(1-methyl-pipeddin-4-yl)-piperazin-1-yl]-2-oxo-ethyl}-amid,
(73) 4-(2-Oxo-1,2,4,5-tetrahydro-1,3-benzdiazepin-3-yl)-piperidin-1-carbonsäure-[(*R*)-1-(4-amino-3,5-bis-trifluormethyl-benzyl)-2-1,4'-bipiperidinyl-1'-yl-2-oxo-ethyl]-amid,
(74) 4-(2-Oxo-1,2,4,5-tetrahydro-1,3-benzdiazepin-3-yl)-piperidin-1-carbonsäure-[(*R*)-1-(4-amino-3,5-bis-trifluormethyl-benzyl)-2-oxo-2-(4-piperazin-1-yl-piperidin-1-yl)-ethyl]-amid,
(75) 4-(2-Oxo-1,2,4,5-tetrahydro-1,3-benzdiazepin-3-yl)-piperidm-1-carbonsäure-(R)-1-(4-amino-3-chlor-5-trifluormethyl-benzyl)-2-[4-(4-methyl-piperazin-1-yl)-piperidin-1-yl]-2-oxo-ethylester,
(76) 4-(2-Oxo-1,4-dihydro-2*H*-chinazolin-3-yl)-piperidin-1-carbonsäure-[1-(4-brom-3-methyl-benzyl)-2-(1'-methyl-[4,4']bipiperidinyl-1-yl)-2-oxo-ethyl]-amid,
(77) 4-(2-Oxo-1,4-dihydro-2*H*-chinazolin-3-yl)-piperidin-1-carbonsäure-{1-(4-brom-3-methyl-benzyl)-2-[4-(4-methyl-piperazin-1-yl)-piperidin-1-yl]-2-oxo-ethyl}-amid,
(78) 4-(2-Oxo-1,4-dihydro-2*H*-chinazolin-3-yl)-piperidin-1-carbonsäure-{1-(4-brom-3-methyl-benzyl)-2-[4-(1-methyl-piperidin-4-yl)-piperazin-1-yl]-2-oxo-ethyl}-amid,
(79) 4-(2-Oxo-1,4-dihydro-2H-chinazolin-3-yl)-piperidin-1-carbonsäure{1-(4-chlor-3-methyl-benzyl)-2-[4-(4-methyl-piperazin-1-yl)-piperidin-1-yl]-2-oxo-ethyl}-amid,
(80) 4-(2-Oxo-1,4-dihydro-2*H*-chinazolin-3-yl)-piperidin-1-carbonsäure-{1-(4-chlor-3-methyl-benzyl)-2-[4-(1-methyl-piperidin-4-yl)-piperazin-1-yl]-2-oxo-ethyl}-amid,
(81) 4-(2-Oxo-1,4-dihydro-2*H*-chinazolin-3-yl)-piperidin-1-carbonsäure{1-(3-brom-4-chlor-5-methyl-benzyl)-2-[4-(4-methyl-piperazin-1-yl)-piperidin-1-yl]-2-oxo-ethyl}-amid,
(82) 4-(2-Oxo-1,4-dihydro-2*H*-chinazolin-3-yl)-piperidin-1-carbonsäure-{1-(3-brom-4-chlor-5-methyl-benzyl)-2-[4-(1-methyl-piperidin-4-yl)-piperazin-1-yl]-2-oxo-ethyl}-amid,
deren Enantiomere, deren Diastereomere und deren Salze eine besonders herausragende Bedeutung besitzen.

Die Verbindungen der allgemeinen Formel (I) werden nach prinzipiell bekannten Methoden hergestellt. Die folgenden Verfahren haben sich zur Herstellung der erfindungsgemäßen Verbindungen der allgemeinen Formel (I) besonders bewährt:
(a) Zur Herstellung von Verbindungen der allgemeinen Formel (I), in der X das Sauerstoffatom oder die NH-Gruppe bedeutet und R¹ bis R³ wie eingangs erwähnt definiert sind, mit der Maßgabe, dass R² und R³ keine freie Carbonsäurefunktion enthalten:
   Umsetzung von Piperidinen der allgemeinen Formel 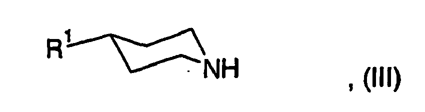 in der R¹ wie eingangs erwähnt definiert ist,
   (i) mit Kohlensäurederivaten der allgemeinen Formel 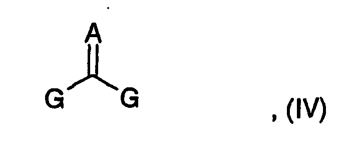 in der A wie eingangs erwähnt definiert ist und G eine nucleofuge Gruppe, bevorzugt die Phenoxy-, 1*H*-Imidazol-1-yl-, 1*H*-1,2,4-Triazol-1-yl-, Trichlormethoxy- oder die 2,5-Dioxopyrrolidin-1-yloxy-Gruppe, bedeutet, mit der Maßgabe, dass X die NH-Gruppe darstellt, oder
   (ii) mit Kohlensäurederivaten der allgemeinen Formel 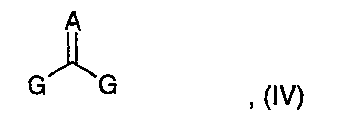 in der A das Sauerstoffatom darstellt und G eine nucleofuge Gruppe, die gleich oder verschieden sein kann, bevorzugt das Chloratom, die p-Nitrophenoxy- oder Trichlormethoxy-Gruppe, bedeutet, mit der Maßgabe, dass X das Sauerstoffatom bedeutet,
   und mit Verbindungen der allgemeinen Formel 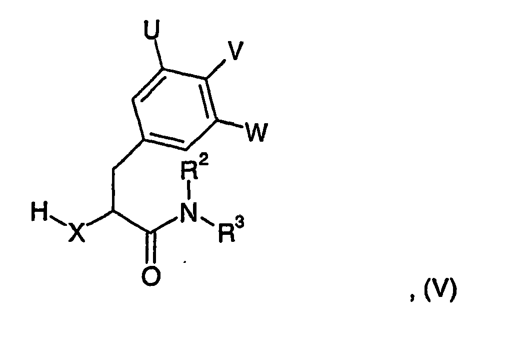 in der X das Sauerstoffatom oder eine -NH-Gruppe bedeutet und U, V, W, R² und R³ wie eingangs erwähnt definiert sind, mit der Maßgabe, dass R² und R³ keine freie Carbonsäure- und/oder keine weitere freie primäre oder sekundäre aliphatische Aminofunktion oder weitere freie Hydroxyfunktionen enthalten.
   Die im Prinzip zweistufigen Reaktionen werden in der Regel als Eintopfverfahren durchgeführt, und zwar bevorzugt in der Weise, dass man in der ersten Stufe eine der beiden Komponenten (III) oder (V) mit äquimolaren Mengen des Kohlensäurederivates der allgemeinen Formel (IV) in einem geeigneten Lösemittel bei tieferer Temperatur zur Reaktion bringt, anschließend wenigstens äquimolare Mengen der anderen Komponente (III) oder (V) zugibt und die Umsetzung bei höherer Temperatur beendet. Die Umsetzungen mit Bis-(trichlormethyl)-carbonat werden bevorzugt in Gegenwart von wenigstens 2 Äquivalenten (bezogen auf Bis-(trichlormethyl)-carbonat) einer tertiären Base, beispielsweise von Triethylamin, *N*-Ethyldiisopropylamin, Pyridin, 1,5-Diaza-bicyclo-[4,3,0]-non-5-en, 1,4-Diazabicyclo-[2,2,2]octan oder 1,8-Diazabicyclo-[5,4,0]-un-dec-7-en, durchgeführt. Als Lösungsmittel, die wasserfrei sein sollten, kommen beispielsweise Tetrahydrofuran, Dioxan, Dimethylformamid, Dimethylacetamid, *N*-Methyl-2-pyrroildon, 1,3-Dimethyl-2-imidazolidinon oder Acetonitril in Betracht, bei Verwendung von Bis-(trichlormethyl)-carbonat als Carbonylkomponente werden wasserfreie Chlorkohlenwasserstoffe, beispielsweise Dichlormethan, 1,2-Dichlorethan oder Trichlorethylen, bevorzugt. Die Reaktionstemperaturen liegen für die erste Reaktionsstufe zwischen -30°C und +25°C, bevorzugt -5°C und +10°C, für die zweite Reaktionsstufe zwischen +15°C und der Siedetemperatur des verwendeten Lösemittels, bevorzugt zwischen +20°C und +70°C (Siehe auch: H. A. Staab und W. Rohr, "Synthesen mit heterocyclischen Amiden (Azoliden)", Neuere Methoden der Präparativen Organischen Chemie, Band V, S. 53-93, Verlag Chemie, Weinheim/Bergstr., 1967; P. Majer und R.S. Randad, J. Org. Chem. 59, S. 1937-1938 (1994); K. Takeda, Y. Akagi, A. Saiki, T. Sukahara und H. Ogura, Tetrahedron Letters 24 (42), 4569-4572 (1983); S.R. Sandler und W. Karo in "Organic Functional Group Preparations", Vol. II, S. 223-245, Academis Press, New York 1971).
(b) Zur Herstellung von Verbindungen der allgemeinen Formel (I), in der X die Methylengruppe bedeutet und R¹ bis R³ wie eingangs erwähnt definiert sind, mit der Maßgabe, dass diese Gruppen keine freie Carbonsäure- und/oder keine weitere freie primäre oder sekundäre aliphatische Aminofunktion enthalten:
   Kupplung einer Carbonsäure der allgemeinen Formel 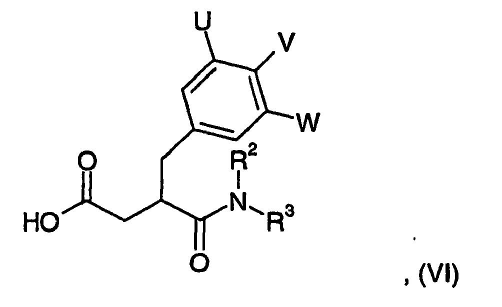 in der U, V, W, R² und R³ wie eingangs erwähnt definiert sind, mit einem Piperidin der allgemeinen Formel 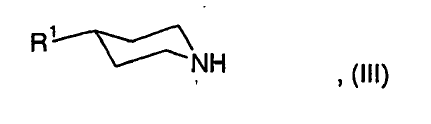 in der R¹ die eingangs erwähnten Bedeutungen besitzt.

   Die Kupplung wird bevorzugt unter Verwendung von aus der Peptidchemie bekannten Verfahren (siehe z. B. Houben-Weyl, Methoden der Organischen Chemie, Bd. 15/2) durchgeführt, wobei zum Beispiel Carbodiimide, wie z.B. Dicyclohexylcarbodiimid (DCC), Diisopropylcarbodiimid (DIC) oder Ethyl-(3-dimethylaminopropyl)-carbodümid, O-(1*H*-Benzotriazol-1-yl)-*N,N-N',N'*-tetramethyluronium-hexafluorphosphat (HBTU) oder -tetrafluorborat (TBTU) oder 1*H*-Benzotriazol-1-yl-oxy-tris-(dimethylamino)-phosphoniumhexafluorphosphat (BOP) eingesetzt werden. Durch Zugabe von 1-Hydroxybenzotriazol (HOBt) oder von 3-Hydroxy-4-oxo-3,4-dihydro-1,2,3-benzotriazin (HOObt) kann die Reaktionsgeschwindigkeit gesteigert werden. Die Kupplungen werden normalerweise mit äquimolaren Anteilen der Kupplungskomponenten sowie des Kupplungsreagenz in Lösemitteln wie Dichlormethan, Tetrahydrofuran, Acetonitril, Dimethylformamid (DMF), Dimethylacetamid (DMA), *N*-Methylpyrrolidon (NMP) oder Gemischen aus diesen und bei Temperaturen zwischen -30°C und +30°C, bevorzugt -20°C und +25°C, durchgeführt. Sofern erforderlich, wird als zusätzliche Hilfsbase *N*-Ethyldiisopropylamin (DIEA) (Hünig-Base) bevorzugt.
   Als weiteres Kupplungsverfahren zur Synthese von Verbindungen der allgemeinen Formel (I) wird das sogenannte "Anhydridverfahren" (siehe auch: M. Bodanszky, "Peptide Chemistry", Springer-Verlag 1988, S. 58-59; M. Bodanszky, "Principles of Peptide Synthesis", Springer-Verlag 1984, S. 21-27) eingesetzt. Bevorzugt wird das "gemischte Anhydridverfahren" in der Variante nach Vaughan (J.R. Vaughan Jr., J. Amer. Chem.Soc. 73, 3547 (1951)), bei der unter Verwendung von Chlorkohlensäureisobutylester in Gegenwart von Basen, wie 4-Methylmorpholin oder 4-Ethylmorpholin, das gemischte Anhydrid aus der zu kuppelnden Carbonsäure der allgemeinen Formel (VI) und dem Kohlensäure-monoisobutylester erhalten wird. Die Herstellung dieses gemischten Anhydrids und die Kupplung mit Aminen erfolgt im Eintopfverfahren, unter Verwendung der vorstehend genannten Lösemittel und bei Temperaturen zwischen -20 und +25°C, bevorzugt 0°C und +25°C.
(c) Zur Herstellung von Verbindungen der allgemeinen Formel (1), in der X die Methylengruppe bedeutet und R² und R³ wie eingangs erwähnt definiert sind, mit der Maßgabe, dass diese Gruppen kein freies primäres oder sekundäres Amin enthalten:
   Kupplung einer Verbindung der allgemeinen Formel 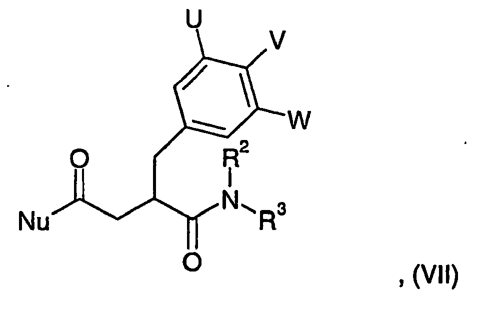 in der U, V, W, R² und R³ wie eingangs erwähnt definiert sind, mit der Maßgabe, dass R² und R³ kein freies primäres oder sekundäres Amin enthalten, und Nu eine Austrittsgruppe, beispielsweise ein Halogenatom, wie das Chlor-, Brom- oder lodatom, eine Alkylsulfonyloxygruppe mit 1 bis 10 Kohlenstoffatomen im Alkylteil, eine gegebenenfalls durch Chlor- oder Bromatome, durch Methyl- oder Nitrogruppen mono-, di- oder trisubstituierte Phenylsulfonyloxy- oder Naphthylsulfonyloxygruppe, wobei die Substituenten gleich oder verschieden sein können, eine 1*H*-Imidazol-1-yl-, eine gegebenenfalls durch eine oder zwei Methylgruppen im Kohlenstoffgerüst substituierte 1*H*-Pyrazol-1-yl-, eine 1*H*-1,2,4-Triazol-1-yl-, 1*H*-1,2,3-Triazol-1-yl-, 1*H-*1,2,3,4-Tetrazol-1-yl-, eine Vinyl-, Propargyl-, *p*-Nitrophenyl-, 2,4-Dinitrophenyl-, Trichlorphenyl-, Pentachlorphenyl-, Pentafluorphenyl-, Pyranyl- oder Pyridinyl-, eine Dimethylaminyloxy-, 2(1*H*)-Oxopyridin-1-yl-oxy-, 2,5-Dioxopyrrolidin-1-yloxy-, Phthalimidyloxy-, 1*H*-Benzo-triazol-1-yloxy- oder Azidgruppe bedeutet,
   mit einem Piperidin der allgemeinen Formel 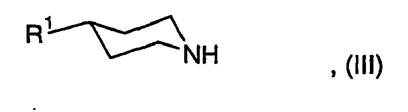 in der R¹ wie eingangs erwähnt definiert ist.
   Die Umsetzung wird unter Schotten-Baumann- oder Einhorn-Bedingungen durchgeführt, das heißt die Komponenten werden in Gegenwart von wenigstens einem Äquivalent einer Hilfsbase bei Temperaturen zwischen -50°C und +120°C, bevorzugt -10°C und +30°C, und gegebenenfalls in Gegenwart von Lösemitteln zur Reaktion gebracht. Als Hilfsbasen kommen bevorzugt Alkali- und Erdalkalihydroxide, beispielsweise Natriumhydroxid, Kaliumhydroxid oder Bariumhydroxid, Alkalicarbonate, z. B. Natriumcarbonat, Kaliumcarbonat oder Cäsiumcarbonat, Alkaliacetate, z.B. Natrium- oder Kaliumacetat, sowie tertiäre Amine, beispielsweise Pyridin, 2,4,6-Tri-methylpyridin, Chinolin, Triethylamin, *N*-Ethyl-diisopropylamin, *N*-Ethyl-dicyclohexyl-amin, 1,4-Di-azabicyclo[2,2,2]octan oder 1,8-Diazabicyclo-[5,4,0]-undec-7-en, als Lösemittel beispielsweise Dichlormethan, Tetrahydrofuran, 1,4-Dioxan, Acetonitril, Dimethylformamid, Dimethylacetamid, *N*-Methylpyrrolidon oder Gemische davon in Betracht; werden als Hilfsbasen Alkali- oder Erdalkalihydroxide, Alkalicarbonate oder -acetate verwendet, kann dem Reaktionsgemisch auch Wasser als Cosolvens zugesetzt werden.
(d) Zur Herstellung von Verbindungen der allgemeinen Formel (I) in der alle Reste wie eingangs erwähnt definiert sind:
   Kupplung einer Carbonsäure der allgemeinen Formel 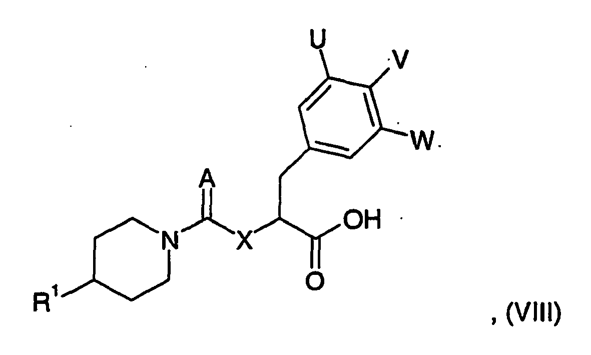
   in der alle Reste wie eingangs erwähnt definiert sind, mit einem Amin der allgemeinen Formel HNR²R³, in der R² und R³ wie eingangs definiert sind, mit der Maßgabe, dass sie keine freie Carbonsäure- und/oder keine weitere freie primäre oder sekundäre aliphatische Aminofunktion enthalten.
   Die Kupplung wird bevorzugt unter Verwendung von aus der Peptidchemie bekannten Verfahren (siehe z. B. Houben-Weyl, Methoden der Organischen Chemie, Bd. 15/2) durchgeführt, wobei zum Beispiel Carbodiimide, wie z.B. Dicyclohexylcarbodiimid (DCC), Diisopropylcarbodiimid (DIC) oder Ethyl-(3-dimethylaminopropyl)-carbodiimid, O-(1*H*-Benzotriazol-1-yl)-*N,N*-*N,N'*-tetramethyluronium-hexafluorphosphat (HBTU) oder -tetrafluorborat (TBTU) oder 1*H*-Benzotriazol-1-yl-oxy-tris-(dimethylamino)-phosphoniumhexafluorphosphat (BOP) eingesetzt werden. Durch Zugabe von 1-Hydroxybenzotriazol (HOBt) oder von 3-Hydroxy-4-oxo-3,4-dihydro-1,2,3-benzotriazin (HOObt) kann die Reaktionsgeschwindigkeit gesteigert werden. Die Kupplungen werden normalerweise mit äquimolaren Anteilen der Kupplungskomponenten sowie des Kupplungsreagenz in Lösungsmitteln wie Dichlormethan, Tetrahydrofuran, Acetonitril, Dimethylformamid (DMF), Dimethylacetamid (DMA), *N*-Methylpyrrolidon (NMP) oder Gemischen aus diesen und bei Temperaturen zwischen -30 und +30°C, bevorzugt -20 und +25°C, durchgeführt. Sofern erforderlich wird als zusätzliche Hilfsbase *N*-Ethyldiisopropylamin (DIEA) (Hünig-Base) bevorzugt.
   Als weiteres Kupplungsverfahren zur Synthese von Verbindungen der allgemeinen Formel (I) wird das sogenannte "Anhydridverfahren" (siehe auch: M. Bodanszky, "Peptide Chemistry", Springer-Verlag 1988, S. 58-59; M. Bodanszky, "Principles of Peptide Synthesis", Springer-Verlag 1984, S. 21-27) eingesetzt. Bevorzugt wird das "gemischte Anhydridverfahren" in der Variante nach Vaughan (J.R. Vaughan Jr., J. Amer. Chem.Soc. 73, 3547 (1951)), bei der unter Verwendung von Chlorkohlensäureisobutylester in Gegenwart von Basen, wie 4-Methylmorpholin oder 4-Ethylmorpholin, das gemischte Anhydrid aus der zu kuppelnden Carbonsäure der allgemeinen Formel (VIII) und dem Kohlensäuremonoisobutylester erhalten wird. Die Herstellung dieses gemischten Anhydrids und die Kupplung mit den Aminen der allgemeinen Formel HNR²R³ erfolgt im Eintopfverfahren, unter Verwendung der vorstehend genannten Lösemittel und bei Temperaturen zwischen -20°C und +25°C, bevorzugt zwischen 0°C und +25°C.
(e) Zur Herstellung von Verbindungen der allgemeinen Formel (I), in der R¹ wie eingangs erwähnt definiert ist, mit der Maßgabe, dass kein freies primäres oder sekundäres Amin enthalten ist:
   Kupplung einer Verbindung der allgemeinen Formel 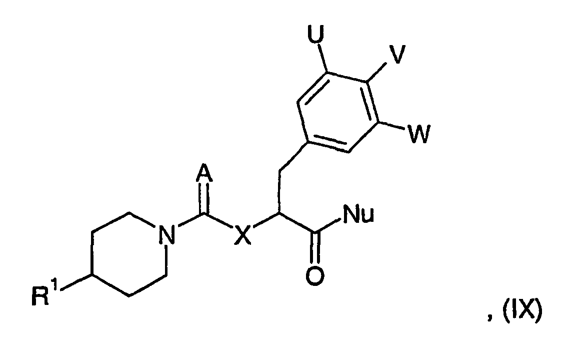 in der alle Reste wie eingangs erwähnt definiert sind und Nu eine Austrittsgruppe, beispielsweise ein Halogenatom, wie das Chlor-, Brom- oder Iodatom, eine Alkylsulfonyloxygruppe mit 1 bis 10 Kohlenstoffatomen im Alkylteil, eine gegebenenfalls durch Chlor- oder Bromatome, durch Methyl- oder Nitrogruppen mono-, di- oder trisubstituierte Phenylsulfonyloxy- oder Naphthylsulfonyloxygruppe, wobei die Substituenten gleich oder verschieden sein können, eine 1*H*-Imidazol-1-yl-, eine gegebenenfalls durch eine oder zwei Methylgruppen im Kohlenstoffgerüst substituierte 1*H*-Pyrazol-1-yl-, eine 1*H*-1,2,4-Triazol-1-yl-, 1*H*-1,2,3-Triazol-1-yl-, 1*H-*1,2,3,4-Tetrazol-1-yl-, eine Vinyl-, Propargyl-, *p*-Nitrophenyl-, 2,4-Dinitrophenyl-, Trichlorphenyl-, Pentachlorphenyl-, Pentafluorphenyl-, Pyranyl- oder Pyridinyl-, eine Dimethylaminyloxy-, 2(1*H*)-Oxopyridin-1-yl-oxy-, 2,5-Dioxopyrrolidin-1-yloxy-, Phthalimidyloxy-, 1*H*-Benzo-triazol-1-yloxy- oder Azidgruppe bedeutet,
mit einem Amin der allgemeinen Formel HNR²R³, in der R² und R³ wie eingangs definiert sind, mit der Maßgabe, dass keine freie Carbonsäure- und/oder keine weitere freie primäre oder sekundäre aliphatische Aminofunktion enthalten ist.

Die Umsetzung wird unter Schotten-Baumann- oder Einhorn-Bedingungen durchgeführt, das heißt, die Komponenten werden in Gegenwart von wenigstens einem Äquivalent einer Hilfsbase bei Temperaturen zwischen -50°C und +120°C, bevorzugt -10°C und +30°C, und gegebenenfalls in Gegenwart von Lösungsmitteln zur Reaktion gebracht. Als Hilfsbasen kommen bevorzugt Alkali- und Erdalkalihydroxide, beispielsweise Natriumhydroxid, Kaliumhydroxid oder Bariumhydroxid, Alkalicarbonate, z. B. Natriumcarbonat, Kaliumcarbonat oder Cäsiumcarbonat, Alkaliacetate, z.B. Natrium- oder Kaliumacetat, sowie tertiäre Amine, beispielsweise Pyridin, 2,4,6-Trimethylpyridin, Chinolin, Triethylamin, *N*-Ethyldiisopropylamin, *N*-Ethyldicyclohexylamin, 1,4-Di-azabicyclo[2,2,2]octan oder 1,8-Diaza-bicyclo[5,4,0]-undec-7-en, als Lösungsmittel beispielsweise Dichlormethan, Tetrahydrofuran, 1,4-Dioxan, Acetonitril, Dimethylformamid, Dimethylacetamid, *N*-Methylpyrrolidon oder Gemische davon in Betracht; werden als Hilfsbasen Alkali- oder Erdalkalihydroxide, Alkalicarbonate oder -acetate verwendet, kann dem Reaktionsgemisch auch Wasser als Cosolvens zugesetzt werden.

Die erfindungsgemäßen neuen Verbindungen der allgemeinen Formel (I) enthalten ein oder mehrere Chiralitätszentren. Sind beispielsweise zwei Chiralitätszentren vorhanden, dann können die Verbindungen in Form zweier diastereomerer Antipodenpaare auftreten. Die Erfindung umfaßt die einzelnen Isomeren ebenso wie ihre Gemische.

Die Trennung der jeweiligen Diastereomeren gelingt auf Grund ihrer unterschiedlichen physikochemischen Eigenschaften, z.B. durch fraktionierte Kristallisation aus geeigneten Lösemitteln, durch Hochdruckflüssigkeits- oder Säulenchromatographie unter Verwendung chiraler oder bevorzugt achiraler stationärer Phasen.

Die Trennung von unter die allgemeine Formel (I) fallenden Racematen gelingt beispielsweise durch HPLC an geeigneten chiralen stationären Phasen (z. B. Chiral AGP, Chiralpak AD). Racemate, die eine basische oder saure Funktion enthalten, lassen sich auch über die diastereomeren, optisch aktiven Salze trennen, die bei Umsetzung mit einer optisch aktiven Säure, beispielsweise (+)- oder (-)-Weinsäure, (+)- oder (-)-Diacetylweinsäure, (+)- oder (-)-Monomethyltartrat oder (+)-Camphersulfonsäure, bzw. einer optisch aktiven Base, beispielsweise mit (R)-(+)-1-Phenylethylamin, (S)-(-)-1-Phenylethylamin oder (S)-Brucin, entstehen.

Nach einem üblichen Verfahren zur Isomerentrennung wird das Racemat einer Verbindung der allgemeinen Formel (I) mit einer der vorstehend angegebenen optisch aktiven Säuren bzw. Basen in äquimolarer Menge in einem Lösemittel umgesetzt und die erhaltenen kristallinen, diastereomeren, optisch aktiven Salze unter Ausnutzung ihrer verschiedenen Löslichkeit getrennt. Diese Umsetzung kann in jeder Art von Lösemitteln durchgeführt werden, solange sie einen ausreichenden Unterschied hinsichtlich der Löslichkeit der Salze aufweisen. Vorzugsweise werden Methanol, Ethanol oder deren Gemische, beispielsweise im Volumenverhältnis 50:50, verwendet. Sodann wird jedes der optisch aktiven Salze in Wasser gelöst, mit einer Base, wie Natriumcarbonat oder Kaliumcarbonat, oder mit einer geeigneten Säure, beispielsweise mit verdünnter Salzsäure oder wässeriger Methansulfonsäure, vorsichtig neutralisiert und dadurch die entsprechende freie Verbindung in der (+)- oder (-)-Form erhalten.

Jeweils nur das (*R*)- oder (*S*)-Enantiomer bzw. ein Gemisch zweier optisch aktiver, unter die allgemeine Formel (I) fallender diastereomerer Verbindungen wird auch dadurch erhalten, dass man die oben beschriebenen Synthesen mit jeweils einer geeigneten (*R*)- bzw. (*S*)-konfigurierten Reaktionskomponente durchführt.

Die Ausgangsverbindungen der allgemeinen Formel (III) erhält man, soweit sie nicht literaturbekannt oder gar käuflich sind, entsprechend den in der internationalen Patentanmeldung WO 98/11128 und DE 199 52 146 angegebenen Verfahren. Die Ausgangsverbindungen der allgemeinen Formel (IV) sind käuflich. Verbindungen der allgemeinen Formel (V) lassen sich nach dem Peptidchemiker geläufigen Methoden aus geschützten Phenylalaninen und Aminen der allgemeinen Formel HNR²R³ herstellen.

Die zur Herstellung der optisch reinen Verbindungen der allgemeinen Formel (V) nötigen Phenyalaninderivate können aus den Verbindungen der allgemeinen Formel 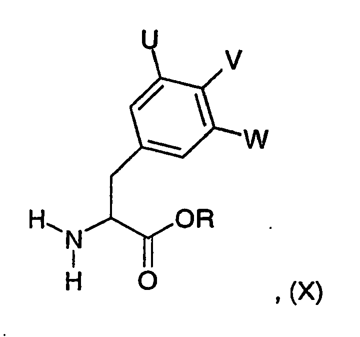 in der U, V und W wie eingangs erwähnt definiert sind und R eine unverzweigte Alkylgruppe, bevorzugt die Methyl- oder Ethylgruppe, darstellt, durch Racematspaltung hergestellt werden.

Diese Racematspaltung kann mit Hilfe enzymatischer Methoden durchgeführt werden, wobei nur ein Enantiomer des Racemates transformiert wird und das entstehende Gemisch dann mit Hilfe physikochemischer Methoden, bevorzugt mit Hilfe chromatographischer Methoden, getrennt wird. Ein geeignetes Enzymsystem für diesen Schritt stellt das Enzym Alcalase 2.4 L FG (Novozymes A/S; DK 2880 Bagsvaerd) dar. Die Verbindungen der allgemeinen Formel (X) können dann mit für Peptidchemiker geläufigen Methoden in die enantiomerenreinen Verbindungen der allgemeinen Formel (V) überführt werden.

Falls die Gruppe X in Verbindungen der allgemeinen Formel (V) das Sauerstoffatom darstellt, können die für die Synthese benötigten Hydroxycarbonsäuren der allgemeinen Formel 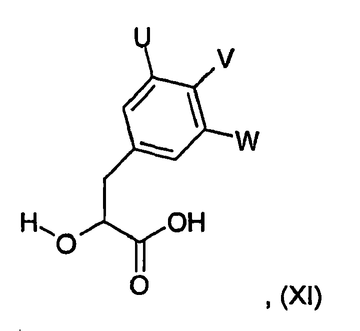 in der U, V und W wie eingangs erwähnt definiert sind, aus Verbindungen der allgemeinen Formel (X) gewonnen werden, mit der Maßgabe, dass R das Wasserstoffatom darstellt.

Unter der Maßgabe, dass V nicht die Amino- oder Methylaminogruppe darstellt, können durch Diazotierung von Verbindungen der allgemeinen Formel (X) mit einem geeigneten Diazotierungsreagenz, bevorzugt Natriumnitrit in sauren Milieu, die Verbindungen der allgemeinen Formel (XI) erhalten werden. Bei Einsatz enantiomerenreiner Verbindungen werden die entsprechenden enantiomerenreinen Hydroxycarbonsäureverbindungen erhalten, wobei die Reaktion unter Retention der Konfiguration abläuft.

Ein weiterer Zugang zu Verbindungen der allgemeinen Formel (XI), in der U, V und W wie eingangs erwähnt definiert sind, besteht in der Alkylierung der Verbindung 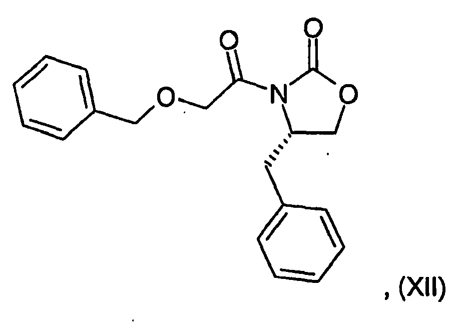 mit entsprechend substituierten Benzylchloriden, Benzylbromiden oder Benzyliodiden der allgemeinen Formel (XIII) 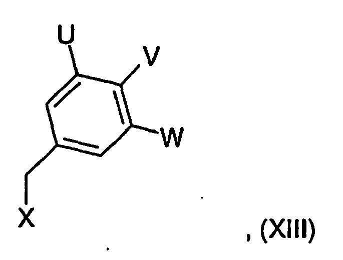 in der U, V und W wie eingangs erwähnt definiert sind und X ein Chlor-, Brom- oder Jodatom bedeutet, in Analogie zu literaturbekannten Methoden (Michael T. Crimmins, Kyle A. Emmitte und Jason D. Katz, Org. Lett. 2, 2165-2167 [2000]).

Die entstehenden diastereomeren Produkte können dann mit Hilfe physikochemischer Methoden, bevorzugt mit Hilfe chromatographischer Methoden, getrennt werden. Die hydrolytische Abspaltung des chiralen Auxiliars, Kupplung mit Aminen der allgemeinen Formel HNR²R³ und Abspaltung der Benzylschutzgruppe eröffnet ebenfalls einen Zugang zu enantiomerenreinen Hydroxycarbonsäureverbindungen des allgemeinen Formel (V).

Die Ausgangsverbindungen der allgemeinen Formel (VI) erhält man beispielsweise durch Umsetzung von Aminen der allgemeinen Formel HNR²R³ mit 2-(Alkoxycarbonylmethyl)-3-aryl-propansäuren und anschließende hydrolytische Abspaltung der Alkylgruppe. Die erforderlichen 2-(Alkoxycarbonylmethyl)-3-aryl-propansäuren können in Analogie zu literaturbekannten Methoden (David A. Evans, Leester D. Wu, John J. M. Wiener, Jeffrey S. Johnson, David H. B. Ripin und Jason S. Tedrow, J. Org.Chem 64, 6411-6417 [1999]; Saul G. Cohen und Aleksander Milovanovic, J. Am. Chem. Soc. 90, 3495-3502 [1968]; Hiroyuki Kawano, Youichi Ishii, Takao Ikariya, Masahiko Saburi, Sadao Yoshikawa, Yasuzo Uchida und Hidenori Kumobayashi, Tetrahedron Letters 28, 1905-1908 [1987]) hergestellt werden. Carbonsäuren der allgemeinen Formel (VIII) können nach den in der WO 98/11128 angegebenen Verfahren aus allgemein zugänglichen Ausgangsmaterialien hergestellt werden.

Die erhaltenen Verbindungen der allgemeinen Formel (I) können, sofern sie geeignete basische Funktionen enthalten, insbesondere für pharmazeutische Anwendungen in ihre physiologisch verträglichen Salze mit anorganischen oder organischen Säuren übergeführt werden. Als Säuren kommen hierfür beispielsweise Salzsäure, Bromwasserstoffsäure, Phosphorsäure, Salpetersäure, Schwefelsäure, Methansulfonsäure, Ethansulfonsäure, Benzolsulfonsäure, *p*-Toluolsulfonsäure, Essigsäure, Fumarsäure, Bernsteinsäure, Milchsäure, Mandelsäure, Äpfelsäure, Zitronensäure, Weinsäure oder Maleinsäure in Betracht.

Außerdem lassen sich die neuen Verbindungen der Formel (I), falls sie Carbonsäurefunktion enthalten, gewünschtenfalls in ihre Additionssalze mit anorganischen oder organischen Basen, insbesondere für die pharmazeutische Anwendung in ihre physiologisch verträglichen Additionssalze überführen. Als Basen kommen hierfür beispielsweise Natriumhydroxid, Kaliumhydroxid, Ammoniak, Cyclohexylamin, Dicyclohexylamin, Äthanolamin, Diethanolamin und Triethanolamin in Betracht.

Die vorliegende Erfindung betrifft Racemate, sofern die Verbindungen der allgemeinen Formel (I) nur ein Chiralitätselement besitzen. Die Anmeldung umfasst jedoch auch die einzelnen diastereomeren Antipodenpaare oder deren Gemische, die dann vorliegen, wenn mehr als ein Chiralitätselement in den Verbindungen der allgemeinen Formel (I) vorhanden ist, sowie die einzelnen optisch aktiven Enantiomeren, aus denen sich die erwähnten Racemate zusammensetzen.

Ebenfalls mit vom Gegenstand dieser Erfindung umfasst sind die erfindungsgemäßen Verbindungen, einschließlich deren Salze, in denen ein oder mehrere Wasserstoffatome durch Deuterium ausgetauscht sind.

Die neuen Verbindungen der allgemeinen Formel (I) und deren physiologisch verträgliche Salze weisen wertvolle pharmakologische Eigenschaften auf, die auf ihre selektiven CGRP-antagonistischen Eigenschaften zurückgehen. Ein weiterer Gegenstand der Erfindung sind diese Verbindungen enthaltende Arzneimittel, deren Verwendung und deren Herstellung.

Die voranstehend genannten neuen Verbindungen und deren physiologisch verträgliche Salze besitzen CGRP-antagonistische Eigenschaften und zeigen gute Affinitäten in CGRP-Rezeptorbindungsstudien. Die Verbindungen weisen in den nachstehend beschriebenen pharmakologischen Testsystemen CGRP-antagonistische Eigenschaften auf.

Zum Nachweis der Affinität der voranstehend genannten Verbindungen zu humanen CGRP-Rezeptoren und ihrer antagonistischen Eigenschaften wurden die folgenden Versuche durchgeführt:

### A. Bindungsstudien mit (den humanen CGRP-Rezeptor exprimierenden) SK-N-MC-Zellen

SK-N-MC-Zellen werden in "Dulbecco's modified Eagle Medium" kultiviert. Das Medium konfluenter Kulturen wird entfernt. Die Zellen werden zweimal mit PBS-Puffer (Gibco 041-04190 M) gewaschen, durch Zugabe von PBS-Puffer, versetzt mit 0.02% EDTA, abgelöst und durch Zentrifugation isoliert. Nach Resuspension in 20 ml "Balanced Salts Solution" [BSS (in mM): NaCl 120, KCl 5.4, NaHCO₃ 16.2, MgSO₄. 0.8, NaHPO₄ 1.0, CaCl₂ 1.8, D-Glucose 5.5, HEPES 30, pH 7.40] werden die Zellen zweimal bei 100 x g zentrifugiert und in BSS resuspendiert. Nach Bestimmung der Zellzahl werden die Zellen mit Hilfe eines Ultra-Turrax homogenisiert und für 10 Minuten bei 3000 x g zentrifugiert. Der Überstand wird verworfen und das Pellet in Tris-Puffer (10 mM Tris, 50 mM NaCl, 5 mM MgCl₂, 1 mM EDTA, pH 7.40), angereichert mit 1 % Rinderserum-Albumin und 0.1% Bacitracin, rezentrifugiert und resuspendiert (1 ml / 1000000 Zellen). Das Homogenat wird bei -80°C eingefroren. Die Membranpräparationen sind bei diesen Bedingungen für mehr als 6 Wochen stabil.

Nach Auftauen wird das Homogenat 1:10 mit Assay-Puffer (50 mM Tris, 150 mM NaCl, 5 mM MgCl₂, 1 mM EDTA, pH 7.40) verdünnt und 30 Sekunden lang mit einem Ultra-Turrax homogenisiert. 230 µl des Homogenats werden für 180 Minuten bei Raumtemperatur mit 50, pM ¹²⁵I-Iodotyrosyl-Calcitonin-Gene-Related Peptide (Amersham) und ansteigenden Konzentrationen der Testsubstanzen in einem Gesamtvolumen von 250 µl inkubiert. Die Inkubation wird durch rasche Filtration durch mit Polyethylenimin (0.1%) behandelte GF/B-Glasfasefilter mittels eines Zellharvesters beendet. Die an Protein gebundene Radioaktivität wird mit Hilfe eines Gammacounters bestimmt. Als nichtspezifische Bindung wird die gebundene Radioaktivität nach Gegenwart von 1 µM humanem CGRP-alpha während der Inkubation definiert.

Die Analyse der Konzentrations-Bindungskurven erfolgt mit Hilfe einer computergestützten nichtlinearen Kurvenanpassung.

Die eingangs erwähnten Verbindungen zeigen in dem beschriebenen Test IC₅₀-Werte ≤ 10000 nM.

### B. CGRP-Antagonismus in SK-N-MC-Zellen

SK-N-MC-Zellen (1 Mio. Zellen) werden zweimal mit 250 µl Inkubationspuffer (Hanks' HEPES, 1 mM 3-Isobutyl-1-methylxanthin, 1% BSA, pH 7.4) gewaschen und bei 37°C für 15 Minuten vorinkubiert. Nach Zugabe von CGRP (10 µl) als Agonist in steigenden Konzentrationen (10⁻¹¹ bis 10⁻⁶ M) bzw. zusätzlich von Substanz in 3 bis 4 verschiedenen Konzentrationen wird nochmals 15 Minuten inkubiert.

Intrazelluläres cAMP wird anschließend durch Zugabe von 20 µl 1 M HCl und Zentrifugation (2000 x g, 4°C für 15 Minuten) extrahiert. Die Überstände werden in flüssigem Stickstoff eingefroren und bei -20°C gelagert.

Die cAMP-Gehalte der Proben werden mittels Radioimmunassay (Fa. Amersham) bestimmt und die pA₂-Werte antagonistisch wirkender Substanzen graphisch ermittelt.

Die erfindungsgemäßen Verbindungen zeigen in dem beschriebenen *in vitro-*Testmodell CGRP-antagonistische Eigenschaften in einem Dosisbereich zwischen 10⁻¹² bis 10⁻⁵ M.

Aufgrund ihrer pharmakologischen Eigenschaften eignen sich die erfindungsgemäßen Verbindungen und deren Salze mit physiologisch verträglichen Säuren somit zur akuten und prophylaktischen Behandlung von Kopfschmerzen, insbesondere Migräne- bzw. Cluster-Kopfschmerz, Spannungskopfschmerz und chronischer Kopfschmerz. Die erfindungsgemäßen Verbindungen eigenen sich ebenfalls zur Vorbeugung von Migräne-Kopfschmerz während der Prodromerscheinung oder zur Vorbeugung und Behandlung von Migräne-Kopfschmerz, der vor oder während der Menstruation auftritt.
Weiterhin beeinflussen die erfindungsgemäßen Verbindungen auch die folgenden Erkrankungen positiv:
Nicht-insulinabhängigen Diabetes mellitus ("NIDDM"), cardiovaskuläre Erkrankungen, Morphintoleranz, Clostritiumtoxin-bedingte Durchfallerkrankungen, Erkrankungen der Haut, insbesondere thermische und strahlenbedingte Hautschäden inklusive Sonnenbrand, entzündliche Erkrankungen, z.B. entzündliche Gelenkerkrankungen (Arthritis), neurogene Entzündungen der oralen Mucosa, entzündliche Lungenerkrankungen, allergische Rhinitis, Asthma, Erkrankungen, die mit einer überschießenden Gefäßerweiterung und dadurch bedingter verringerter Gewebedurchblutung einhergehen, z.B. Schock und Sepsis oder Hautrötungen. Darüber hinaus zeigen die erfindungsgemäßen Verbindungen eine lindernde Wirkung auf Schmerzzustände im allgemeinen, insbesondere bei neuropathischen Schmerzen, wie beispielsweise dem komplexen regionalen Schmerzsyndrom (CRPS), bei neuropathischen Schmerzzuständen im Rahmen systemischer neurotoxischer Erkrankungen, wie beispielsweise Diabetes Mellitus, sowie bei Schmerzzuständen, die auf entzündliche Prozesse zurückzuführen sind.
Die Symptomatik menopausaler, durch Gefäßerweiterung und erhöhten Blutfluß verursachter Hitzewallungen östrogendefizienter Frauen sowie hormonbehandelter Prostatakarzinompatienten wird durch die CGRP-Antagonisten der vorliegenden Anwendung präventiv und akut-therapeutisch günstig beeinflußt, wobei sich dieser Therapieansatz vor der Hormonsubstitution durch Nebenwirkungsarmut auszeichnet.

Die zur Erzielung einer entsprechenden Wirkung erforderliche Dosierung beträgt zweckmäßigerweise bei intravenöser oder subkutaner Gabe 0.0001 bis 3 mg/kg Körpergewicht, vorzugsweise 0.01 bis 1 mg/kg Körpergewicht, und bei oraler, nasaler oder inhalativer Gabe 0.01 bis 10 mg/kg Körpergewicht, vorzugsweise 0.1 bis 10 mg/kg Körpergewicht, jeweils 1 bis 3 x täglich.

Sofern die Behandlung mit CGRP-Antagonisten oder/und CGRP-Release-Hemmern in Ergänzung zu einer üblichen Hormonsubstitution erfolgt, empfiehlt sich eine Verringerung der vorstehend angegebenen Dosierungen, wobei die Dosierung dann 1/5 der vorstehend angegebenen Untergrenzen bis zu 1/1 der vorstehend angegebenen Obergrenzen betragen kann.

Die erfindungsgemäß hergestellten Verbindungen können entweder alleine oder gegebenenfalls in Kombination mit anderen Wirksubstanzen zur Behandlung von Migräne intravenös, subkutan, intramuskulär, intrarektal, intranasal, durch Inhalation, transdermal oder oral erfolgen, wobei zur Inhalation insbesondere Aerosolformulierungen geeignet sind. Die Kombinationen können entweder simultan oder sequentiell verabreicht werden.

Als Kombinationspartner denkbare Wirkstoffklassen sind z.B. Antiemetica, Prokinetica, Neuroleptica, Antidepressiva, Neurokinin-Antagonisten, Anticonvulsiva, Histamin-H1-Rezeptorantagonisten, Antimuscarinika, β-Blocker, α-Agonisten und α-Antagonisten, Ergotalkaloiden, schwachen Analgetica, nichtsteroidalen Antiphlogistica, Corticosteroiden, Calcium-Antagonisten, 5-HT_{1B/1D}-Agonisten oder andere Antimigränemitteln, die zusammen mit einem oder mehreren inerten üblichen Trägerstoffen und/oder Verdünnungsmitteln, z.B. mit Maisstärke, Milchzucker, Rohrzucker, mikrokristalliner Zellulose, Magnesiumstearat, Polyvinylpyrrolidon, Zitronensäure, Weinsäure, Wasser, Wasser/Ethanol, Wasser/Glycerin, Wasser/-Sorbit, Wasser/Polyethylenglykol, Propylenglykol, Cetylstearylalkohol, Carboxymethylcellulose oder fetthaltigen Substanzen wie Hartfett oder deren geeigneten Gemischen, in übliche galenische Zubereitungen wie Tabletten, Dragees, Kapseln, Pulver, Suspensionen, Lösungen, Dosieraerosole oder Zäpfchen eingearbeitet werden können.

Für die oben erwähnten Kombinationen kommen somit als weitere Wirksubstanzen beispielsweise die nicht-steroidalen Antiphlogistika Aceclofenac, Acemetacin, Acetylsalicylsäure, Azathioprin, Diclofenac, Diflunisal, Fenbufen, Fenoprofen, Flurbiprofen, Ibuprofen, Indometacin, Ketoprofen, Leflunomid, Lornoxicam, Mefenaminsäure, Naproxen, Phenylbutazon, Piroxicam, Sulfasalazin, Zomepirac oder deren pharmazeutisch verträgliche Salze sowie Meloxicam und andere selektive COX2-Inhibitoren, wie beispielsweise Rofecoxib und Celecoxib, in Betracht.

Weiterhin können Ergotamin, Dihydroergotamin, Metoclopramid, Domperidon, Diphenhydramin, Cyclizin, Promethazin, Chlorpromazin, Vigabatrin, Timolol, Isomethepten, Pizotifen, Botox, Gabapentin, Topiramat, Riboflavin, Montelukast, Lisinopril, Prochlorperazin, Dexamethason, Flunarizin, Dextropropoxyphen, Meperidin, Metoprolol, Propranolol, Nadolol, Atenolol, Clonidin, Indoramin, Carbamazepin, Phenytoin, Valproat, Amitryptilin, Lidocain oder Diltiazem und andere 5-HT_{1B/1D}-Agonisten wie z.B. Almotriptan, Avitriptan, Eletriptan, Frovatriptan, Naratriptan, Rizatriptan, Sumatriptan und Zolmitriptan verwendet werden.

Die Dosis für diese Wirksubstanzen beträgt hierbei zweckmäßigerweise 1/5 der üblicherweise empfohlenen niedrigsten Dosierung bis zu 1/1 der normalerweise empfohlenen Dosierung, also beispielsweise 20 bis 100 mg Sumatriptan.

Ein weiterer Gegenstand der Erfindung ist die Verwendung der erfindungsgemäßen Verbindungen als wertvolle Hilfsmittel zur Erzeugung und Reinigung (Affinitätschromatographie) von Antikörpern sowie, nach geeigneter radioaktiver Markierung, beispielsweise durch Tritiierung geeigneter Vorstufen, beispielsweise durch katalytische Hydrierung mit Trithium oder Ersatz von Halogenatomen durch Tritium, in RIA- und ELISA-Assays und als diagnostische bzw. analytische Hilfsmittel in der Neurotransmitter-Forschung.

### Experimenteller Teil

Für hergestellte Verbindungen liegen in der Regel IR-, ¹H-NMR und/oder Massenspektren vor. Wenn nicht anders angegeben, werden R_{f}-Werte unter Verwendung von DC-Fertigplatten Kieselgel 60 F254 (E. Merck, Darmstadt, Artikel-Nr. 1.05714) ohne Kammersättigung bestimmt. Die unter der Bezeichnung Alox ermittelten R_{f}-Werte werden unter Verwendung von DC-Fertigplatten Aluminiumoxid 60 F254 (E. Merck, Darmstadt, Artikel-Nr. 1.05713) ohne Kammersättigung bestimmt. Die bei den Fliessmitteln angegebenen Verhältnisse beziehen sich auf Volumeneinheiten der jeweiligen Lösungsmittel. Die angegebenen Volumeneinheiten bei NH₃ beziehen sich auf eine konzentrierte Lösung von NH₃ in Wasser.
Soweit nicht anders vermerkt sind die bei den Aufarbeitungen der Reaktionslösungen verwendeten Säure-, Basen- und Salzlösungen wässrige Systeme der angebenen Konzentrationen.
Zu chromatographischen Reinigungen wird Kieselgel der Firma Millipore (MATREX™, 35-70 µm) verwendet. Zu chromatographischen Reinigungen wird Alox (E. Merck, Darmstadt, Aluminiumoxid 90 standardisiert, 63-200 µm, Artikel-Nr: 1.01097.9050) verwendet.
Die angegebenen HPLC-Daten werden unter nachstehend angeführten Parametern gemessen:
Analytische Säule: Zorbax-Säule (Agilent Technologies), SB (Stable Bond) - C18; 3.5 µm; 4.6 x 75 mm; Säulentemperatur: 30°C; Fluss: 0.8 mL / min; Injektionsvolumen: 5 µL; Detektion bei 254 nm

### Methode A:

| Zeit (min) | Volumenprozent Wasser (mit 0.1 % Ameisensäure) | Volumenprozent Acetonitril (mit 0.1 % Ameisensäure) |
|---|---|---|
| 0 | 90 | 10 |
| 9 | 10 | 90 |
| 10 | 10 | 90 |
| 11 | 90 | 10 |

Bei präparativen HPLC-Reinigungen werden in der Regel die gleichen Gradienten verwendet, die bei der Erhebung der analytischen HPLC-Daten benutzt wurden.
Die Sammlung der Produkte erfolgt massengesteuert, die Produkt enthaltenden Fraktionen werden vereinigt und gefriergetrocknet.
Falls nähere Angaben zur Konfiguration fehlen, bleibt offen, ob es sich um reine Enantiomere handelt oder ob partielle oder gar völlige Racemisierung eingetreten ist.

In den Versuchsbeschreibungen werden die folgenden Abkürzungen verwendet:
- abs.: absolutiert
- Boc: *tert.*-Butoxycarbonyl
- CDI: *N,N'*-Carbonyldiimidazol
- CDT: 1,1'-Carbonyldi-(1,2,4-triazol)
- Cyc: Cyclohexan
- DCM: Dichlormethan
- DMF: *N,N*-Dimethylformarnid
- EtOAc: Essigsäureethylester
- EtOH: Ethanol
- halbkonz.: halbkonzentriert
- HCl: Salzsäure
- HOAc: Essigsäure
- HOBt: 1-Hydroxybenzotriazol-hydrat
- i. vac.: in vacuo (im Vakuum)
- KOH: Kaliumhydroxid
- konz.: konzentriert
- MeOH: Methanol
- NaCl: Natriumchlorid
- NaOH: Natriumhydroxid
- org.: organisch
- PE: Petrolether
- RT: Raumtemperatur
- TBTU: 2-(1*H*-Benzotriazol-1-yl)-1,1,3,3-tetramethyluronium-Tetrafluorborat
- TFA: Trifluoressigsäure
- THF: Tetrahydrofuran

### Vorschriften zur Herstellung der verwendeten Amine (Ausgangsverbindungen)

### Beispiel A1

### 2-Methyl-5-piperidin-4-yl-2,5-diaza-bicyclo[2.2.1]heptan (Beispiele 7.9 und 10.79)

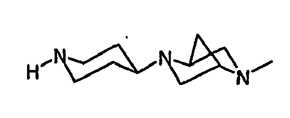

### A1a 2-(1-Benzyl-piperidin-4-yl)-5-methyl-2,5-diaza-bicyclo[2.2.1]heptan

Zu einer Lösung von 2.38 mL (12.7 mmol) 1-Benzyl-piperidin-4-on und 3.5 g (12.77 mmol) 2-Methyl-2,5-diaza-bicyclo[2.2.1]heptan in 100 mL MeOH wurden 0.73 mL (12.7 mmol) Essigsäure zugegeben und das Reaktionsgemisch 3 h bei RT gerührt. Unter einem Stickstoffstrom wurden 0.99 g (15.0 mmol) NaBH₃CN zugegeben und die Reaktionslösung über Nacht bei RT nachgerührt. Man säuerte mit 7 mL konz. HCl an, rührte 1 h bei RT nach und engte i.vac. ein. Der Rückstand wurde mit 200 mL 15% K₂CO₃-Lösung versetzt, zweimal mit jeweils 200 mL DCM extrahiert und die vereinigten organischen Phasen über Na₂SO₄ getrocknet. Nach Entfernen des Trocken- und Lösungsmittels wurde der Rückstand chromatographisch (Kieselgel, Gradient: DCM nach DCM/MeOH/NH₃ 10:85:5) gereinigt.

| | |
|---|---|
| Ausbeute: | 2.1 g (58% der Theorie) |
| ESI-MS: | (M+H)⁺ = 286 |
| R_{f} = | 0.15 (Kieselgel, DCM/MeOH/NH₃ 80:20:2) |

### A1b 2-Methyl-5-piperidin-4-yl-2,5-diaza-bicyclo[2.2.1]heptan

Eine Lösung von 2.1 g (7.36 mmol) 2-(1-Benzyl-piperidin-4-yl)-5-methyl-2,5-diaza-bicyclo[2.2.1]heptan in 100 mL MeOH wurde mit 500 mg 10% Pd/C versetzt und bei RT und 50 psi H₂ 3 h hydriert. Der Katalysator wurde abgesaugt und das Lösungsmittel i.vac. entfernt. Das Produkt wurde ohne Reinigung für weitere Umsetzungen verwendet.

| | |
|---|---|
| Ausbeute: | 1.4 g (97% der Theorie) |
| ESI-MS: | (M+H)⁺ = 196 |
| R_{f} = | 0.10 (Kieselgel, DCM/MeOH/NH₃ 80:20:2) |

### Beispiel A2

### 1-Cyclopropylmethyl-4-piperidin-4-yl-piperazin (Beispiel 10.4)

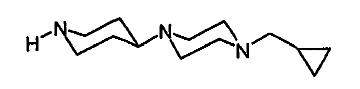

### A2a 4-(4-Cyclopropylmethyl-piperazin-1-yl)-piperidin-1-carbonsäure-tert-butylester

Zu einer Lösung von 1.71 g (5.0 mmol) 4-Piperazin-1-yl-piperidin-1-carbonsäure-tert-butylester und 0.75 mL (10.0 mmol) Cyclopropancarbaldehyd in 100 mL EtOH wurden bei RT in 4 Portionen 1.26 g (20.0 mmol) NaBH₃CN zugegeben und das Reaktionsgemisch über Nacht bei RT gerührt. Man engte i.vac. ein, nahm den Rückstand in gesättigter NaHCO₃-Lösung auf, extrahierte erschöpfend mit EtOAc und trocknete die organische Phase über Na₂SO₄. Nach Entfernen des Trocken- und Lösungsmittels wurde der Rückstand chromatographisch (Kieselgel, EtOAc/MeOH/NH₃ 90:10:0.5) gereinigt.

| | |
|---|---|
| Ausbeute: | 1.36 g (84% der Theorie) |
| EI: | (M)⁺ = 323 |

### A2b 1-Cyclopropylmethyl-4-piperidin-4-yl-piperazin

Zu einer Lösung von 1.36 g (4.2 mmol) 4-(4-Cyclopropylmethyl-piperazin-1-yl)-piperidin-1-carbonsäure-*tert*-butylester in 30 mL DCM wurden 5 mL TFA gegeben und das Reaktionsgemisch 4 h bei RT gerührt. Die Reaktionslösung wurde i.vac. eingeengt, der Rückstand mit Diethylether versetzt, der Niederschlag abgesaugt und getrocknet. Das Produkt fiel als Tris-Trifluoracetat-Salz an.

| | |
|---|---|
| Ausbeute: | 1.86 g (78% der Theorie) |
| EI: | (M)⁺ = 223 |

### Beispiel A3

### 4-Azetidin-1-yl-piperidin (Beispiel 10.15)

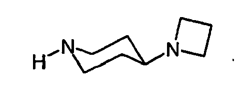

### A3a 4-Azetidin-1-yl-1-benzyl-piperidin

Zu einer Lösung von 3.0 mL (16.45 mmol) 1-Benzyl-piperidin-4-on und 1.0 g (17.51 mmol) Azetidin in 100 mL DCM wurde 1.0 mL (17.49 mmol) Essigsäure zugegeben und das Reaktionsgemisch 1 h bei RT gerührt. Unter Eiskühlung wurden in 4 Portionen innerhalb 1 h 6.0 g (39.55 mmol) NaBH(OAc)₃ zugegeben und die Reaktionslösung über Nacht bei RT nachgerührt. Man versetzte mit 15% K₂CO₃-Lösung und rührte eine weitere Stunde nach. 200 mL EtOAc wurden zugegeben, die organische Phase abgetrennt und über MgSO₄ getrocknet. Nach Entfernen des Trocken- und Lösungsmittels wird der Rückstand chromatographisch (Kieselgel, Gradient: DCM nach MeOH/NH₃ 9:1) gereinigt.

| | |
|---|---|
| Ausbeute: | 3.2 g (84% der Theorie) |
| EI: | (M)⁺ = 230 |
| R_{f} = | 0.57 (Kieselgel, DCM/MeOH/Cyc/NH₃ 70:15:15:2) |

### A3b 4-Azetidin-1-yl-piperidin

Eine Lösung von 3.2 g (13.89 mmol) 4-Azetidin-1-yl-1-benzyl-piperidin in 50 mL MeOH wurde mit 500 mg 10% Pd/C versetzt und bei RT und 3 bar H₂ 7.5 h hydriert. Der Katalysator wurde abgesaugt und das Lösungsmittel i.vac. entfernt. Das Produkt wurde ohne Reinigung für weitere Umsetzungen verwendet.

| | |
|---|---|
| Ausbeute: | 1.9 g (98% der Theorie) |
| ESI-MS: | (M+H)⁺ = 141 |
| R_{f} = | 0.19 (Kieselgel, DCM/MeOH/NH₃ 70:25:5) |

### Beispiel A4

### 1-Azetidin-3-yl-perhydro-azepin (Beispiel 10.22)

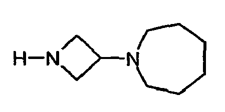

### A4a 1-(1-Benzhydryl-azetidin-3-yl)-perhydro-azepin

Unter Stickstoffatmosphäre wurden zu einer Lösung von 31.7 g (100 mmol) Methansulfonsäure-1,1dibenzyl-azetidin-3-yl-ester in 200 mL DMF 31.7 g (320 mmol) Perhydro-azepin gegeben und das Gemisch 7 Tage bei 50°C gerührt. Die Reaktionslösung wurde mit 1 L Wasser versetzt, die wässrige Phase zweimal mit EtOAc extrahiert, die vereinigten organischen Phasen zweimal mit Wasser gewaschen und über Na₂SO₄ getrocknet. Nach Entfernen des Trocken- und Lösungsmittels wurde der Rückstand zweimal chromatographisch (Kieselgel, 1.Säule: DCM/MeOH/NH₃ 19:1:0.025 und 2. Säule: *tert*-Butylmethylether) gereinigt.

| | |
|---|---|
| Ausbeute: | 22.2 g (69% der Theorie) |
| R_{f} = | 0.82 (Kieselgel, DCM/MeOH/NH₃ 19:1:0.025) |

### A4b 1-Azetidin-3-yl-perhydro-azepin

Zu einer Lösung von 22.0 g (68.6 mmol) 1-(1-Benzhydryl-azetidin-3-yl)-perhydro-azepin in 400 mL MeOH und 69 mL 2 N HCl wurden 5 g 10% Pd/C gegeben und das Reaktionsgemisch bei 45°C bis zur theoretischen Aufnahme an H₂ hydriert (3 h). Nach Filtration wurde das Lösungsmittel i.vac. entfernt und das Produkt, das als Bis-Hydrochlorid anfiel, ohne Reingung weiter umgesetzt.

| | |
|---|---|
| Ausbeute: | 15.5 g (100% der Theorie) |
| Schmelzpunkt: | 205-220°C |
| R_{f} = | 0.08 (Kieselgel, DCM/MeOH/NH₃ 9:1:0.1) |

### Beispiel A5

### 1-Benzyl-4-piperidin-4-yl-piperazin (Beispiel 10.24)

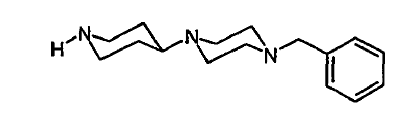

### A5a 4-(4-Benzyl-piperazin-1-yl)-piperidin-1-carbonsäure-tert-butylester

Eine Lösung von 66.7 mL (381 mmol) 1-Benzyl-piperazin und 75.8 g (380 mmol) 4-Oxo-piperidin-1-carbonsäure-*tert*-butylester in 1 L THF wurde mit Eisessig auf einen pH-Wert von 5 eingestellt und dann wurde unter Eiskühlung innerhalb von 2 h portionenweise 100 g (448 mmol) NaBH(OAc)₃ zugegeben und das Reaktionsgemisch über Nacht bei RT gerührt. Die Reaktionslösung wurde vorsichtig mit 2.2 M K₂CO₃-Lösung alkalisch gestellt, 1 h gerührt, erschöpfend mit EtOAc extrahiert und die vereinigten organischen Phasen über Na₂SO₄ getrocknet. Nach Entfernen des Trocken- und Lösungsmittels wurde der Rückstand ohne Reinigung weiter umgesetzt.

| | |
|---|---|
| Ausbeute: | 114 g (83% der Theorie) |
| ESI-MS: | (M+Na)⁺ = 382 |
| R_{f} = | 0.74 (Kieselgel, DCM/MeOH/Cyc/NH₃ 70:15:15:2) |

### A5b 1-Benzyl-4-piperidin-4-yl-piperazin

Zu einer Lösung von 5 g (13.91 mmol) 4-(4-Benzyl-piperazin-1-yl)-piperidin-1-carbonsäure-tert butylester in 200 mL DCM wurden 20 mL TFA gegeben und das Reaktionsgemisch über Nacht bei RT gerührt. Man engte i.vac. ein, versetzte den Rückstand mit 200 mL 15% K₂CO₃-Lösung, extrahierte dreimal mit jeweils 100 mL DCM und trocknete die vereinigten organischen Phasen über MgSO₄. Nach Entfernen des Trocken- und Lösungsmittels erhielt man das gewünschte Produkt, welches ohne Reinigung weiter umgesetzt wurde.

| | |
|---|---|
| Ausbeute: | 2.9 g (80% der Theorie) |
| ESI-MS: | (M+H)⁺ = 260 |
| R_{f} = | 0.58 (Kieselgel, DCM/MeOH/NH₃ 70:25:5) |

### Beispiel A6

### Dimethyl-(2,3,4,5-tetrahydro-1H-3-benzazepin-7-ylmethyl)-amin (Beispiel 10.25)

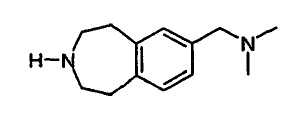

### A6a 1-(7-Dimethylaminomethyl-1,2,4,5-tetrahydro-3-benzazepin-3-yl)-2,2,2-trifluor-ethanon

Zu einer Lösung von 4.5 g (16.59 mmol) 3-(2,2,2-Trifluor-acetyl)-2,3,4,5-tetrahydro-1*H*-3-benzazepin-7-carbaldehyd in 150 mL THF wurden 11.7 mL (23.4 mmol) einer 2 M Dimethylamin-Lösung in THF zugegeben und die Lösung mit 1 mL Eisessig auf pH 5 gebracht. Nach 30 min wurden 4.62 g (21.79 mmol) NaBH(OAc)₃ zugegeben und das Reaktionsgemisch über Nacht bei RT gerührt. Die Reaktionslösung wurde vorsichtig mit gesättigter NaHCO₃-Lösung versetzt, 30 min nachgerührt, erschöpfend mit EtOAc extrahiert, die organische Phase abgetrennt und über Na₂SO₄ getrocknet. Nach Entfernen des Trocken- und Lösungsmittels erhielt man das gewünschte Produkt, welches ohne Reinigung weiter umgesetzt wurde.

| | |
|---|---|
| Ausbeute: | 4.5 g (90% der Theorie) |
| ESI-MS: | (M+H)⁺ = 301 |
| R_{f} = | 0.76 (Kieselgel, DCM/MeOH/Cyc/NH₃ 70:15:15:2) |

### A6b Dimethyl-(2,3,4,5-tetrahydro-1H-3-benzazepin-7-ylmethyl)-amin

Zu einer Lösung von 4.5 g (14.98 mmol) 1-(7-Dimethylaminomethyl-1,2,4,5-tetrahydro-3-benzazepin-3-yl)-2,2,2-trifluor-ethanon in 50 mL MeOH wurden 50 mL Wasser und 8.5 g (61.51 mmol) K₂CO₃ gegeben und das Reaktionsgemisch 72 h bei RT gerührt. Die Reaktionslösung wurde i.vac. eingeengt, der Rückstand mit DCM versetzt, von unlöslichen Bestandteilen filtriert und i.vac. eingeengt. Man erhielt das gewünschte Produkt in Form eines hellbraunen Öles.

| | |
|---|---|
| Ausbeute: | 2.9 g (95% der Theorie) |
| ESI-MS: | (M+H)⁺ = 205 |
| R_{f} = | 0.39 (Kieselgel, DCM/Me0H/Cyc/NH₃ 70:15:15:2) |

### Beispiel A7

### 1-Piperidin-4-yl-4-(2,2,2-trifluor-ethyl)-piperazin (Beispiel 10.27)

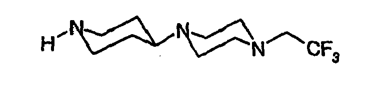

### A7a 1-[4-(1-Benzyl-piperidin-4-yl)-piperazin-1-yl]-2,2,2-trifiuor-ethanon

Zu einer auf 0°C gekühlten Lösung von 15.0 g (57.83 mmol) 1-(1-Benzyl-piperidin-4-yl)-piperazin und 20.1 mL (145 mmol) Triethylamin in 200 DCM wurde eine Lösung von 8.21 mL (57.83 mmol) Trifluoressigsäureanhydrid in 40 mL DCM zugetropft und das Reaktionsgemisch 5 h bei RT gerührt. Man versetzte mit Wasser, trennte die organische Phase ab und trocknete diese über Na₂SO₄. Nach Entfernen des Trocken- und Lösungsmittels wurde das Rohprodukt ohne Reinigung weiter umgesetzt.

| | |
|---|---|
| Ausbeute: | 8.3 g (40% der Theorie) |
| EI: | (M)⁺ = 205 |
| R_{f} = | 0.48 (Kieselgel, DCM/MeOH/NH₃ 90:10:1) |

### A7b 1-(1-Benzyl-piperidin-4-yl)-4-(2,2,2-trifluor-ethyl)-piperazin

Zu einer Lösung von 1.0 g (2.81 mmol) 1-[4-(1-Benzyl-piperidin-4-yl)-piperazin-1-yl]-2,2,2-trifluor-ethanon in 9 mL 1,4-Dioxan und 1 mL THF wurden 0.53 g (14.07 mmol) NaBH₄ zugegeben und dann zur resultierenden Suspension innerhalb von 10 min eine Lösung von 1.08 mL (14.07 mmol) TFA in 10 mL 1,4-Dioxan zugetropft. Die Reaktionslösung wurde 5 h unter Rückfluss erhitzt, nach dem Abkühlen mit Wasser zersetzt und i.vac. eingeengt. Der erhaltene Rückstand wurde chromatographisch (Kieselgel, EtOAc/MeOH/NH₃ 95:5:0.5) gereinigt.

| | |
|---|---|
| Ausbeute: | 0.41 g (43% der Theorie) |
| ESI-MS: | (M+H)⁺ = 342 |
| R_{f} = | 0.45 (Kieselgel, EtOAc/MeOH/NH₃ 80:20:2) |

### A7c 1-Piperidin-4-yl-4-(2,2,2-trifluor-ethyl)-piperazin

Zu einer Lösung von 0.41 g (1.20 mmol) 1-(1-Benzyl-piperidin-4-yl)-4-(2,2,2-trifluorethyl)-piperazin in 30 mL MeOH wurden 50 mg 10% Pd/C gegeben und das Reaktionsgemisch bei RT und 3 bar H₂ 2.5 h hydriert. Zur Vervollständigung der Reaktion wurde eine Spatelspitze Pd(OH)₂ zugegeben und weitere 1.5 h hydriert. Nach Absaugen des Katalysators und Entfernen des Lösungsmittels erhielt man das gewünschte Produkt.

| | |
|---|---|
| Ausbeute: | 0.28 g (94% der Theorie) |
| ESI-MS: | (M+H)⁺ = 252 |
| R_{f} = | 0.05 (Kieselgel, EtOAc/MeOH/NH₃ 70:30:3) |

### Beispiel A8

### Methyl-[2-(1-methyl-piperidin-4-yl)-ethyl]-amin (Beispiel 10.32)

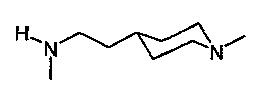

### A8a (N,N-Dimethyl-2-(1-methyl-piperidin-4-yl)-acetamid

Eine Lösung von 9.3 g (50 mmol) N-Methylpiperidinyl-4-essigsäureethylester in 50 mL 40% wässriger Methylamin-Lösung wurde 15 h bei 80°C im Bombenrohr gerührt. Das Lösungsmittel wurde i.vac. entfernt und der Rückstand in EtOAc gelöst. Die organische Phase wurde über MgSO₄ getrocknet und das Lösungsmittel i.vac. entfernt.

| | |
|---|---|
| Ausbeute: | 4.7 g (55% der Theorie) |
| R_{f} = | 0.53 (Kieselgel, DCM/MeOH 21:1) |

### A8b Methyl-[2-(1-methyl-pipeddin-4-yl)-ethyl]-amin

Zu einer Lösung von 1.1 g (30 mmol) Lithiumaluminiumhydrid in 50 mL Diethylether wurden 3.1 g (20 mmol) *N,N*-Dimethyl-2-(1-methyl-piperidin-4-yl)-acetamid in 40 mL THF zugetropft. Das Gemisch wurde 2 h am Rückfluß erhitzt und dann über Nacht bei RT gerührt. Nach Zugabe von Wasser, 6 N NaOH und wieder von Wasser wurde die Lösung 30 min gerührt. Nach Filtration wurde das Lösungsmittel i.vac. entfernt. Der Rückstand wurde in Diethylether gelöst, über MgSO₄ getrocknet und das Lösungsmittel i.vac. entfernt.

| | |
|---|---|
| Ausbeute: | 2.5 g (80% der Theorie) |
| R_{f} = | 0.29 (Alox, DCM/MeOH 21:1) |

### Beispiel A9

### Cyclopentyl-methyl-piperidin-4-yl-amin (Beispiel 10.41)

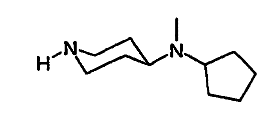

### A9a (1-Benzyl-piperidin-4-yl)-cyclopentyl-methyl-amin

Zu einer Lösung von 6.5 g (31.8 mmol) (1-Benzyl-piperidin-4-yl)-methyl-amin und 2.7 g (32.0 mmol) Cyclopentanon in 200 mL THF wurden 6.0 mL (105 mmol) Eisessig zugegeben und das Reaktionsgemisch 10 min auf 55°C erhitzt. Nach Abkühlen auf 15°C wurden portionenweise 10.6 g (50.0 mmol) NaBH(OAc)₃ zugegeben und die Reaktionslösung über Nacht bei RT gerührt. Zur Vervollständigung der Reaktion wurden nochmals 3.0 g (14.2 mmol) NaBH(OAc)₃ zugegeben und weitere 5 h bei RT gerührt. Man gab vorsichtig 100 mL Wasser zu, stellte mit Na₂CO₃ einen alkalischen pH-Wert ein, extrahierte erschöpfend mit Diethylether, wusch die vereinigten organischen Phasen mit Wasser und engte i.vac. ein. Nach Entfernen des Lösungsmittels wurde der Rückstand chromatographisch (Kieselgel, EtOAc/MeOH/NH₃ 9:1:0.3) gereinigt.

| | |
|---|---|
| Ausbeute: | 5.0 g (58% der Theorie) |
| EI: | (M)⁺ = 272 |
| R_{f} = | 0.6 (Kieselgel, EtOAc/MeOH/NH₃ 9:1:0.3) |

### A9b Cyclopentyl-methyl-piperidin-4-yl-amin

Zu einer Lösung von 5.0 g (18.35 mmol) (1-Benzyl-piperidin-4-yl)-cyciopentyl-methylamin in 100 mL MeOH wurden 1.0 g 10% Pd/C gegeben und das Reaktionsgemisch bei RT und 5 bar H₂ 3 h hydriert. Nach Absaugen des Katalysators und Entfernen des Lösungsmittels erhielt man das gewünschte Produkt, welches ohne Reinigung weiter umgesetzt wurde.

| | |
|---|---|
| Ausbeute: | 3.0 g (90% der Theorie) |
| R_{f} = | 0.05 (Kieselgel, EtOAc/MeOH/NH₃ 9:1:0.4) |

### Beispiel A10

### 4-(1,1-Dioxo-1λ⁶-isothiazolidin-2-yl)-piperidin (Beispiel 10.46)

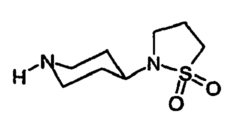

### A10a 1-Benzyl-4-(1,1-dioxo-1λ⁶-isothiazolidin-2-yl)-piperidin

Zu einer Lösung von 19.0 g (100 mmol) 1-Benzyl-piperidin-4-ylamin und 27.2 g (197 mmol) K₂CO₃ in 200 mL Acetonitril wurde bei RT 3-Chlor-propan-1-sulfonylchlorid zugetropft. Die Reaktionslösung wurde über Nacht stehengelassen. Nach Filtration wurde das Lösungsmittel durch Destillation entfernt. Der Rückstand wurde in 120 mL EtOH aufgenommen und mit 6.2 g (111 mmol) KOH versetzt. Das Gemisch wurde 1 h unter Rückfluss erhitzt und nach Abkühlung mit HCl sauer gestellt. Der Niederschlag wurde abgesaugt und getrocknet.

| | |
|---|---|
| Ausbeute: | 15.4 g (46% der Theorie) |
| Schmelzpunkt: | 255-257°C |

### A10b 4-(1,1-Dioxo-1λ⁶-isothiazolidin-2-yl)-piperidin

Analog Beispiel 17d wurde aus 16.5 g (56.1 mmol) 1-Benzyl-4-(1,1-dioxo-1λ⁶-isothiazolidin-2-yl)-piperidin das Produkt hergestellt.

| | |
|---|---|
| Ausbeute: | 8.5 g (74% der Theorie) |
| Schmelzpunkt: | 89-92°C |
| R_{f} = | 0.13 (Kieselgel, DCM/MeOH 8:2) |

### Beispiel A11

### tert-Butyl-piperidin-4-yl-amin (Beispiel 10.50)

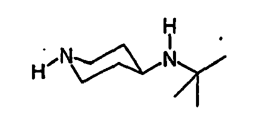

### A11a (1-Benzyl-piperidin-4-yl)-tert-butyl-amin

Unter Argonatmosphäre wurde zu einer auf 0°C gekühlten Lösung von 24.1 mL (130 mmol) 1-Benzyl-piperidin-4-on und 55 mL (519 mmol) *tert*-Butylamin in 200 mL Toluol eine Lösung von 8.6 mL (78 mmol) TiCl₄ in 100 mL Toluol so zugetropft, dass die Innentemperatur 15°C nicht überstieg. Das Reaktionsgemisch wurde über Nacht bei RT gerührt, der ausgefallene Niederschlag abgesaugt und die verbleibende Lösung nach Zugabe von 65 mg Platinoxid bis zur theoretischen Aufnahme an H₂ hydriert. Nach beendeter Hydrierung wurden zur Suspension 160 mL 2 N NaOH-Lösung gegeben, filtriert, die organische Phase abgetrennt, die wässrige Lösung dreimal mit Toluol extrahiert und die vereinigten organischen Phasen über Na₂SO₄ getrocknet. Nach Entfernen des Trocken- und Lösungsmittels wurde das Produkt ohne Reinigung weiter umgesetzt.

| | |
|---|---|
| Ausbeute: | 13.9 g (43% der Theorie) |

### A11b tert-Butyl-piperidin-4-yl-amin

Zu einer Lösung von 13.9 g (56.0 mmol) (1-Benzyl-piperidin-4-yl)-*tert*-butyl-amin in 140 mL MeOH wurden 1.5 g 10% Pd/C gegeben und das Reaktionsgemisch bei RT bis zur theoretischen Aufnahme an H₂ hydriert (2 h). Nach Absaugen des Katalysators und Entfernen des Lösungsmittels erhielt man das gewünschte Produkt, welches ohne Reinigung weiter umgesetzt wurde.

| | |
|---|---|
| Ausbeute: | 8.3 g (95% der Theorie) |

### Beispiel A12

### 4-(2-Methyl-imidazol-1-yl)-piperidin (Beispiel 10.58)

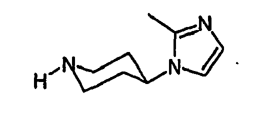

### A12a 4-(2-Methyl-imidazol-1-yl)-piperidin-1-carbonsäure-tert-butylester

Unter Stickstoffatmosphäre wurden zu einer Lösung von 2.0 g (21.92 mmol) 2-Methyl-1*H*-imidazol in 20 mL DMF bei RT portionenweise 1.0 g (22.92 mmol) NaH (55% in Mineralöl) innerhalb von 20 min zugegeben und das Reaktionsgemisch 30 min bei dieser Temperatur nachgerührt. Dann wurde eine Lösung von 4.0 g (14.32 mmol) 4-Methansulfonyloxy-piperidin-1-carbonsäure-*tert*-butylester in 50 mL DMF langsam zugetropft und die Reaktionslösung anschliessend 2.5 h bei 100°C gerührt. Man engte i.vac. ein, nahm den Rückstand in 150 mL DCM auf, wusch die organische Phase zweimal mit jeweils 50 mL Wasser und trocknete über MgSO₄. Nach Entfernen des Trocken- und Lösungsmittels wurde der Rückstand ohne Reinigung weiter umgesetzt.

| | |
|---|---|
| Ausbeute: | 0.65 g (17% der Theorie) |
| ESI-MS: | (M+H)⁺ = 266 |
| R_{f} = | 0.56 (Kieselgel, DCM/MeOH/Cyc/NH₃ 70:15:15:2) |

### A12b 4-(2-Methyl-imidazol-1-yl)-piperidin

Eine Lösung von 650 mg (2.45 mmol) 4-(2-Methyl-imidazol-1-yl)-piperidin-1-carbonsäure-*tert*-butylester wurde in 10 mL 4 M HCl in 1,4-Dioxan gelöst und das Reaktionsgemisch 2 h bei RT gerührt. Man engte i.vac. ein, versetzte den Rückstand mit Diisopropylether und wenig Isopropanol, saugte den Niederschlag ab und trocknete diesen im Umlufttrockenschrank. Das gewünschte Produkt fiel als Dihydrochlorid an.

| | |
|---|---|
| Ausbeute: | 430 mg (74% der Theorie) |
| ESI-MS: | (M+H)⁺ =166 |
| R_{f} = | 0.54 (Kieselgel, DCM/MeOH/NH₃ 75:25:5) |

### Beispiel A13

### 4-(2,4-Dimethyl-imidazol-1-yl)-piperidin (Beispiel 10.61)

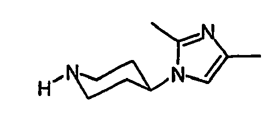

### A13a 4-(2,4-Dimethyl-imidazol-1-yl)-piperidin-1-carbonsäure-tert-butylester

Hergestellt analog Beispiel A12a aus 2.2 g (22.20 mmol) 2,4-Dimethyl-1*H*-imidazol und 4.0 g (14.32 mmol) 4-Methansulfonyloxy-piperidin-1-carbonsäure-*tert*-butylester.

| | |
|---|---|
| Ausbeute: | 0.45 g (11 % der Theorie) |
| ESI-MS: | (M+H)⁺ = 280 |
| R_{f} = | 0.51 (Kieselgel, DCM/MeOH/Cyc/NH₃ 70:15:15:2) |

### A13b 4-(2,4-Dimethyl-imidazol-1-yl)-piperidin

Hergestellt analog Beispiel A12b aus 450 mg (1.61 mmol) 4-(2,4-Dimethyl-imidazol-1-yl)-piperidin-1-carbonsäure-*tert*-butylester. Das gewünschte Produkt fiel als Dihydrochlorid an.

| | |
|---|---|
| Ausbeute: | 300 mg (74% der Theorie) |
| ESI-MS: | (M+H)⁺ = 180 |
| R_{f} = | 0.63 (Kieselgel, DCM/MeOH/NH₃ 75:25:5) |

### Beispiel A14

### Piperazin-1-sulfonsäure-(1-methyl-piperidin-4-yl)-amid (Beispiel 10.66)

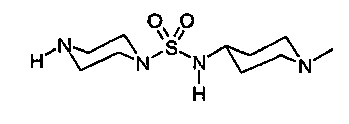

### A14a 4-Benzyl-piperazin-1-sulfonsäure-(1-methyl-piperidin-4-yl)-amid

Zu einer auf 0°C gekühlten Lösung von 1.0 g (8.76 mmol) 1-Methyl-piperidin-4-ylamin und 1.25 mL (9.0 mmol) Triethylamin in 50 mL DCM wurden 1.89 g (11.0 mmol) 1,3,2-Benzdioxathiol-2,2-dioxid zugegeben, das Kältebad entfernt und das Reaktionsgemisch über Nacht bei RT gerührt. Man engte i.vac. ein, verrührte den Rückstand mit Diethylether/Diisopropylether, filtrierte und trocknete das Rohprodukt. Unter Stickstoffatmosphäre wurde dieses Rohprodukt (2.5 g) und 3.16 g (17.4 mmol) 1-Benryl-piperazin in 100 mL 1,4-Dioxan gelöst und das Reaktionsgemisch 2 h unter Rückfluss erhitzt. Man engte i.vac. ein und reinigte den Rückstand chromatographisch (Kieselgel, DCM/MeOH/NH₃ 90:10:1).

| | |
|---|---|
| Ausbeute: | 1.1g (36% der Theorie) |
| ESI-MS: | (M+H)⁺ = 353 |
| R_{f} = | 0.50 (Kieselgel, DCM/MeOH/NH₃ 80:20:1) |

### A14b Piperazin-1-sulfonsäure-(1-methyl-piperidin-4-yl)-amid

Zu einer Lösung von 1.1 g (3.12 mmol) 4-Benzyl-piperazin-1-sulfonsäure-(1-methylpiperidin-4-yl)-amid in 100 mL MeOH wurden 500 mg 10% Pd/C gegeben und das Reaktionsgemisch bei RT bis zur theoretischen Aufnahme an H₂ hydriert (2.5 h). Nach Absaugen des Katalysators und Entfernen des Lösungsmittels erhielt man das gewünschte Produkt, welches ohne Reinigung weiter umgesetzt wurde.

| | |
|---|---|
| Ausbeute: | 0.82 g (100% der Theorie) |
| ESI-MS: | (M+H)⁺ = 263 |
| R_{f} = | 0.05 (Kieselgel, EtOAc/MeOH/NH₃ 90:10:1) |

### Beispiel A15

### 1-(1-Methyl-piperidin-4-yl)-piperazin-2-carbonsäureethylester

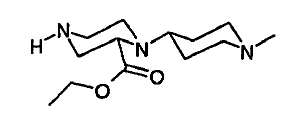

### A15a 4-(1-Methyl-piperidin-4-yl)-piperazin-1,3-dicarbonsäure-1-tert-butylester-3-ethylester

Eine Lösung von 1.0 g (3.87 mmol) Piperazin-1,3-dicarbonsäure-1-*tert*-butylester 3-ethylester und 0.45 mL (3.87 mmol) 1-Methyl-piperidin-4-on in 25 mL THF wurde mit Eisessig auf einen pH-Wert zwischen 5 und 6 gebracht und dann wurden portionenweise 1.0 g (4.48 mmol) NaBH(OAc)₃ zugegeben und das Reaktionsgemisch über Nacht bei RT gerührt. Zur Vervollständigung der Reaktion wurden erneut 1 mL (8.6 mmol) 1-Methyl-piperidin-4-on und 0.2 g (0.9 mmol) NaBH(OAC)₃ zugegeben und weitere 2 h bei RT gerührt. Das überschüssige NaBH(OAc)₃ wurde durch Zugabe von wenig Wasser zersetzt, das Gemisch mit K₂CO₃ gesättigt und durchgerührt. Das K₂CO₃ wurde filtriert, die organische Phase eingeengt und chromatographisch (Kieselgel, EtOAc/MeOH/NH₃ 90:10:1) gereinigt.

| | |
|---|---|
| Ausbeute: | 0.78 g (57% der Theorie) |
| ESI-MS: | (M+H)⁺ = 356 |
| R_{f} = | 0.46 (Kieselgel, EtOAc/MeOH/NH₃ 80:20:2) |

### A15b 1-(1-Methyl-piperidin-4-yl)-piperazin-2-carbonsäureethylester

Zu einer Lösung von 0.78 g (2.20 mmol) 4-(1-Methyl-piperidin-4-yl)-piperazin-1,3-dicarbonsäure-1-*tert*-butyl-ester-3-ethylester in 30 mL DCM wurden unter Eiskühlung 2.0 mL TFA zugegeben und das Reaktionsgemisch 3 h bei RT gerührt. Man engte i.vac. ein und setzte das Produkt, das als Tris-trifluoracetat-Salz anfiel ohne Reinigung weiter um.

| | |
|---|---|
| Ausbeute: | quantitativ |
| EI: | (M)⁺ = 255 |
| R_{f} = | 0.11 (Kieselgel, EtOAc/MeOH/NH₃ 70:30:3) |

### Beispiel A16

### 4-(1-Methyl-piperidin-4-yl)-piperazin-2-carbonsäureethylester

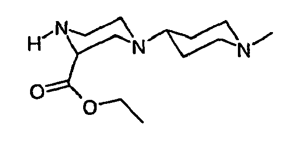

### A16a 4-Benzyl-piperazin-1,3-dicarbonsäure-1-tert-butytester-3-ethylester

Zu einer Lösung von 10.69 g (41.39 mmol) Piperazin-1,3-dicarbonsäure-1-*tert-*butylester-3-ethylester und 7.32 mL (42 mmol) Ethyldiisopropylamin in 150 mL THF wurde eine Lösung von 5.0 mL (42.10 mmol) Benzylbromid in 50 mL THF zugetropft, 2 h bei RT gerührt und dann 3 h unter Rückfluss erhitzt. Zur Vervollständigung der Reaktion wurden erneut 0.5 mL (4.21 mmol) Benzylbromid zugegeben und weitere 3 h unter Rückfluss erhitzt. Die Reaktionslösung wurde nach dem Abkühlen filriert, das Filtrat i.vac. eingeengt und der Rückstand chromatographisch (Kieselgel. Cyc/EtOAc 8:2) gereinigt.

| | |
|---|---|
| Ausbeute: | 13.04 g (90% der Theorie) |
| ESI-MS: | (M+H)⁺ = 349 |
| R_{f} = | 0.51 (Kieselgel, Cyc/EtOAc 8:2) |

### A16b 1-Benzyl-piperazin-2-carbonsäureethylester

Zu einer Lösung von 13.04 g (37.42 mmol) 4-Benzyl-piperazin-1,3-dicarbonsäure-1-*tert*-butylester-3-ethylester in 200 mL DCM wurden unter Eiskühlung 35 mL TFA zugegeben und das Reaktionsgemisch 5 h bei RT gerührt. Man engte i.vac. ein und setzte das Produkt, das als Bis-trifluoracetat-Salz anfiel ohne Reinigung weiter um.

| | |
|---|---|
| Ausbeute: | quantitativ |
| ESI-MS: | (M+H)⁺ = 249 |

### A16c 1-Benzyl-4-(1-methyl-piperidin-4-yl)-piperazin-2-carbonsäureethylester

Zu einer Lösung von 8.15 g (17.11 mmol) 1-Benzyl-piperazin-2-carbonsäureethylester (eingesetzt als Bis-trifluoracetat-Salz) und 2.05 mL (17.22 mmol) 1-Methyl-piperidin-4-on in 200 mL THF wurden 4.0 g (17.93 mmol) NaBH(OAc)₃ zugegeben und das Reaktionsgemisch über Nacht bei RT gerührt. Man engte i.vac. ein und reinigte den Rückstand chromatographisch (Kieselgel, Gradient: EtOAc nach EtOAc/MeOH/NH₃ 50:50:2).

| | |
|---|---|
| Ausbeute: | 5.91 g (100% der Theorie) |
| ESI-MS: | (M+H)⁺ = 346 |
| R_{f} = | 0.53 (Kieselgel, EtOAc/MeOH/NH₃ 80:20:2) |

### A16d 4-(1-Methyl-piperidin-4-yl)-piperazin-2-carbonsäureethylester

Zu einer Lösung von 5.91 g (17.11 mmol) 1-Benzyl-4-(1-methyl-piperidin-4-yl)-piperazin-2-carbonsäureethylester in 150 mL EtOH wurden 0.5 g Pd(OH)₂ gegeben und das Reaktionsgemisch bei RT und 3 bar H₂ 3.5 h hydriert. Nach Absaugen des Katalysators und Entfernen des Lösungsmittels erhielt man das gewünschte Produkt, welches ohne Reinigung weiter umgesetzt wurde.

| | |
|---|---|
| Ausbeute: | 4.44 g (100% der Theorie) |
| ESI-MS: | (M+H)⁺ = 256 |
| R_{f} = | 0.24 (Kieselgel, EtOAc/MeOH/NH₃ 70:30:3) |

### Beispiel A17

### 1-Methyl-4-piperidin-4-yl-piperazin-2-carbonsäureethylester

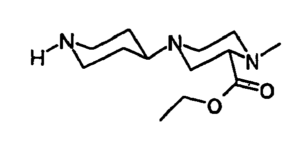

### A17a 4-Methyl-piperazin-1,3-dicarbonsäure-1-tert-butylester-3-ethylester

Zu einer Lösung von 5.04 g (19.51 mmol) Piperazin-1,3-dicarbonsäure-1-tert-butylester-3-ethylester und 3.4 mL (19.52 mmol) Ethyldiisopropylamin in 100 mL THF wurde bei RT eine Lösung von 1.25 mL (19.90 mmol) lodmethan in 20 mL THF langsam zugetropft, das Reaktionsgemisch 20 min nachgerührt und anschliessend 3 h auf 60°C erwärmt. Zur Vervollständigung der Reaktion wurden erneut 0.2 mL (3.18 mmol) lodmethan zugegeben und weitere. 3 h bei 75°C erhitzt. Nach dem Abkühlen wurde von unlöslichen Bestandteilen filtriert, das Filtrat eingeengt und der Rückstand chromatographisch (Kieselgel, EtOAc) gereinigt.

| | |
|---|---|
| Ausbeute: | 4.2 g (79% der Theorie) |
| ESI-MS: | (M+H)⁺ = 273 |
| R_{f} = | 0.58 (Kieselgel, EtOAc) |

### A17b 1-Methyl-piperazin-2-carbonsäureethylester

Zu einer Lösung von 4.20 g (15.42 mmol) 4-Methyl-piperazin-1,3-dicarbonsäure-1-*tert*-butylester-3-ethylester in 80 mL DCM wurden unter Eiskühlung 20 mL TFA zugegeben und das Reaktionsgemisch 1 h bei RT gerührt. Man engte i.vac. ein und setzte das Produkt, das als Bis-trifluoracetat-Salz anfiel ohne Reinigung weiter um.

| | |
|---|---|
| Ausbeute: | quantitativ |
| ESI-MS: | (M+H)⁺ = 173 |
| R_{f} = | 0.16 (Kieselgel, EtOAc/MeOH/NH₃ 70:30:3) |

### A17c 4-(1-Benzyl-piperidin-4-yl)-1-methyl-piperazin-2-carbonsäureethylester

Zu einer Lösung von 6.17 g (15.41 mmol) 1-Methyl-piperazin-2-carbonsäureethylester (eingesetzt als Bis-trifluoracetat-Salz) und 3.77 mL (19.93 mmol) 1-Benzylpiperidin-4-on in 80 mL THF wurden portionenweise 4.5 g (20.17 mmol) NaBH(OAc)₃ zugegeben und das Reaktionsgemisch 2 h bei RT gerührt. Überschüssiges NaBH(OAc)₃ wurde durch Zugabe von wenig Wasser zersetzt, die Reaktionslösung i.vac. eingeengt und der Rückstand chromatographisch (Kieselgel, Gradient: EtOAc/MeOH/NH₃ 90:10:1 nach EtOAc/MeOH/NH₃ 80:20:2) gereinigt.

| | |
|---|---|
| Ausbeute: | quantitativ |
| ESI-MS: | (M+H)⁺ = 345 |
| R_{f} = | 0.41 (Kieselgel, EtOAc/MeOH/NH₃ 80:20:2) |

### A17d 1-Methyl-4-piperidin-4-yl-piperazin-2-carbonsäureethylester

Zu einer Lösung des Rohproduktes aus A17c in 200 mL EtOH wurden 1 g 10% Pd/C gegeben und das Reaktionsgemisch bei RT und 3 bar H₂ 17.5 h hydriert. Nach Absaugen des Katalysators und Entfernen des Lösungsmittels erhielt man das gewünschte Produkt, welches ohne Reinigung weiter umgesetzt wurde.

| | |
|---|---|
| Ausbeute: | 5.32 g (100% der Theorie, bezogen auf A17a) |
| ESI-MS: | (M+H)⁺ = 256 |
| R_{f} = | 0.1 (Kieselgel, EtOAc/MeOH/NH₃ 50:50:5) |

### Beispiel A18

### 4-Methyl-1-piperidin-4-yl-piperazin-2-carbonsäureethylester

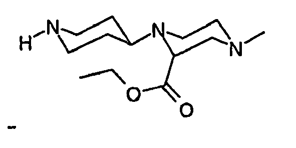

### A18a 1-Benzyl-4-methyl-piperazin-2-carbonsäureethylester

Zu einer Lösung von 10.3 g (21.64 mmol) 1-Benzyl-piperazin-2-carbonsäureethylester (siehe Beispiel A16b, eingesetzt als Bis-trifluoracetat-Salz) und 12 mL (68.89 mmol) Ethyldiisopropylamin in 200 mL THF wurde eine Lösung von 1.4 mL (22.29 mmol) lodmethan in 50 mL THF langsam zugetropft und das Reaktionsgemisch über Nacht bei RT gerührt. Zur Vervollständigung der Reaktion wurden erneut 0.2 mL (3.18 mmol) lodmethan zugegeben und weitere 2 h bei RT gerührt. Der entstandene Niederschlag wurde filtriert, das Filtrat i.vac. eingeengt und der Rückstand chromatographisch (Kieselgel, EtOAc/MeOH/NH₃ 90:10:1) gereinigt.

| | |
|---|---|
| Ausbeute: | 4.1 g (72% der Theorie) |
| ESI-MS: | (M+Na)⁺ = 285 |
| R_{f} = | 0.83 (Kieselgel, EtOAc/MeOH/NH₃ 90:10:1) |

### A18b 4-Methyl-piperazin-2-carbonsäureethylester

Zu einer Lösung von 4.1 g (15.63 mmol) 1-Benzyl-4-methyl-piperazin-2-carbonsäureethylester in 100 mL EtOH wurden 450 mg 10% Pd/C zugegeben und das Reaktionsgemisch bei RT und 5 bar H₂ 11 h hydriert. Zur Vervollständigung der Reaktion wurden 450 mg Pd(OH)₂ zugegeben und das Reaktionsgemisch weitere 2.5 h bei 50°C und 3 bar H₂ hydriert. Der Katalysator wurde abgesaugt, das Filtrat eingeengt und der Rückstand chromatographisch (Kieselgel, EtOAc/MeOH/NH₃ 80:20:2) gereinigt.

| | |
|---|---|
| Ausbeute: | 2.18 g (81 % der Theorie) |
| ESI-MS: | (M+H)⁺ = 173 |
| R_{f} = | 0.56 (Kieselgel, EtOAc/MeOH/NH₃ 80:20:2) |

### A18c 1-(1-Benzyl-piperidin-4-yl)-4-methyl-piperazin-2-carbonsäureethylester

Eine Lösung von 1.58 g (9.17 mmol) 4-Methyl-piperazin-2-carbonsäureethylester und 1.68 mL (9.2 mmol) 1-Benzyl-piperidin-4-on in 30 mL THF wurde mit Eisessig auf einen pH-Wert von 5 gebracht und dann wurden portionenweise 2.2 g (9.86 mmol) NaBH(OAc)₃ zugegeben und das Reaktionsgemisch über Nacht bei RT gerührt. Überschüssiges NaBH(OAc)₃ wurde durch Zugabe von wenig Wasser zersetzt und die Reaktionslösung über K₂CO₃ getrocknet. Die überstehende Lösung wurde abdekantiert, i.vac. eingeengt und der Rückstand chromatographisch (Kieselgel, Gradient: EtOAc nach EtOAc/MeOH/NH₃ 90:9:1) gereinigt.

| | |
|---|---|
| Ausbeute: | 0.71 g (22% der Theorie) |
| ESI-MS: | (M+H)⁺ = 346 |
| R_{f} = | 0.84 (Kieselgel, EtOAc/MeOH/NH₃ 70:30:3) |

### A18d 4-Methyl-1-piperidin-4-yl-piperazin-2-carbonsäureethylester

Zu einer Lösung von 2.28 g (6.6 mmol) 1-(1-Benzyl-piperidin-4-yl)-4-methylpiperazin-2-carbonsäureethylester in 100 mL EtOH wurden 200 mg Pd(OH)₂ zugegeben und das Reaktionsgemisch bei RT und 3 bar H₂ 9.5 h hydriert. Zur Vervollständigung der Reaktion wurden erneut 100 mg Pd(OH)₂ zugegeben und das Reaktionsgemisch weitere 6 h hydriert. Der Katalysator wurde abgesaugt, das Filtrat eingeengt und der Rückstand ohne Reinigung weiter umgesetzt.

| | |
|---|---|
| Ausbeute: | 1.7 g (100% der Theorie) |
| ESI-MS: | (M+H)⁺ = 256 |
| R_{f} = | 0.21 (Kieselgel, EtOAc/MeOH/NH₃ 60:40:4) |

### Beispiel A19

### (4-Piperidin-4-yl-piperazin-1-yl)-essigsäure-fert-butylester

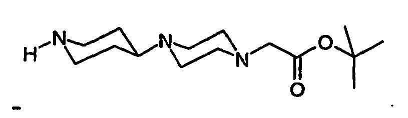

### A19a [4-(1-Benzyl-piperidin-4-yl)-piperazin-1-yl]-essigsäure-tert-butylester

Zu einer Lösung von 74 g (123 mmol) 1-(1-Benzyl-piperidin-4-yl)-piperazin (eingesetzt als Tris-trifluoracetat-Salz) in 1 L Acetonitril wurden 100 g (709 mmol) K₂CO₃ zugegeben und die Suspension 10 min bei RT gerührt. Dann erfolgte die Zugabe von 20 mL (133 mmol) Bromessigsäure-*tert*-butylester. Das Reaktionsgemisch wurde 3 h bei RT gerührt und zum Trocknen mit MgSO₄ versetzt. Die unlöslichen Bestandteile wurden abfiltriert, das Lösungsmittel i.vac. eingeengt, der Rückstand mit Wasser versetzt, der entstandene Niederschlag abgesaugt und im Umlufttrockenschrank bei 50°C getrocknet.

| | |
|---|---|
| Ausbeute: | 30 g (65% der Theorie) |
| ESI-MS: | (M+H)⁺ = 374 |
| R_{f} = | 0.51 (Kieselgel, DCM/MeOH/Cyc/NH₃ 70:15:15:2) |

### A19b (4-Piperidin-4-yl-piperazin-1-yl)-essigsäure-tert-butylester

Zu einer Lösung von 30 g (80.3 mmol) [4-(1-Benzyl-piperidin-4-yl)-piperazin-1-yl]-essigsäure-*tert*-butylester in 300 mL THF wurden 6 g 10% Pd/C gegeben und das Reaktionsgemisch bei 50°C und 3 bar H₂ bis zur theoretischen Aufnahme an H₂ hydriert. Nach Absaugen des Katalysators und Entfernen des Lösungsmittels erhielt man das gewünschte Produkt, welches ohne Reinigung weiter umgesetzt wurde.

| | |
|---|---|
| Ausbeute: | 22.3 g (98% der Theorie) |
| ESI-MS: | (M+H)⁺ = 284 |
| R_{f} = | 0.17 (Kieselgel, DCM/MeOH/Cyc/NH₃ 70:15:15:2) |

### Beispiel A20

### (4-Piperidin-4-yl-piperazin-1-yl)-essigsäureethylester

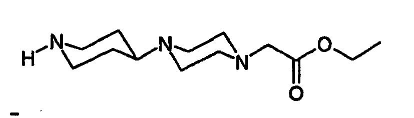

### A20a 4-(4-Ethoxycarbonylmethyl-piperazin-1-yl)-piperidin-1-carbonsäure-tert-butylester

Zu einer auf 0°C gekühlten Lösung von 4.0 g (13.08 mmol) 4-Piperazin-1-yl-piperidin-1-carbonsäure-tert-butylester (eingesetzt als Hydrochlorid) und 6.54 mL (39.23 mmol) Ethyldiisopropylamin in 50 mL Acetonitril wurde eine Lösung von 1.42 mL (13.3 mmol) Chloressigsäureethylester in 10 mL Acetonitril zugegeben. Nach Entfernen des Kältebades wurde und eine Spatelspitze Nal zugegeben und das Reaktionsgemisch über Nacht bei RT gerührt. Man versetzte mit gesättigter NaHCO₃-Lösung, extrahierte erschöpfend mit DCM und trocknete die organische Phase über Na₂SO₄. Nach Entfernen des Trocken- und Lösungsmittels wurde der Rückstand ohne Reinigung weiter umgesetzt.

| | |
|---|---|
| Ausbeute: | 4.25 g (91% der Theorie) |
| ESI-MS: | (M+H)⁺ = 356 |
| R_{f} = | 0.67 (Kieselgel, DCM/MeOH 9:1) |

### A20b (4-Piperidin-4-yl-piperazin-1-yl)-essigsäureethylester

Zu einer auf 0°C gekühlten Lösung von 4.25 g (11.96 mmol) 4-(4-Ethoxycarbonylmethyl-piperazin-1-yl)-piperidin-1-carbonsäure-tert-butylester in 80 mL DCM wurden 6 mL TFA gegeben und das Reaktionsgemisch über Nacht bei RT gerührt. Man engte i.vac. ein, verrührte den Rückstand mit *tert*-Butylmethylether, saugte den Niederschlag ab und trocknete das Produkt, das als Tris-tifluoracetat-Salz anfiel.

| | |
|---|---|
| Ausbeute: | 6.7 g (94% der Theorie) |
| ESI-MS: | (M+H)⁺ = 256 |
| R_{f} = | 0.38 (Kieselgel, DCM/MeOH/NH₃ 75:25:5) |

### Beispiel A21

### [4,4']Bipiperidinyl-1-yl-essigsäureethylester

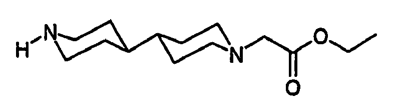

### A21a 1'-Ethoxycarbonylmethyl-[4,4']bipiperidinyl-1-carbonsäure-tert-butylester

Analog Beispiel A19a wurde aus 8.40 g (31.1 mmol) [4,4']Bipiperidinyl-1-carbonsäure-*tert*-butylester und 3.53 mL (31.1 mmol) Bromessigsäure-ethylester das Produkt erhalten.

| | |
|---|---|
| Ausbeute: | 9.4 g (85% der Theorie) |
| EI-MS: | (M)⁺ = 355 |
| R_{f} = | 0.64 (Kieselgel, EtOAc/MeOH 9:1) |

### A21b [4,4']Bipiperidinyl-1-yl-essigsäureethylester

Analog Beispiel 20b wurde aus 7.50 g (21.2 mmol) 1'-Ethoxycarbonylmethyl-[4,4']bipiperidinyl-1-carbonsäure-*tert*-butylester das Produkt als Bis-trifluoracetat-Salz erhalten.

| | |
|---|---|
| Ausbeute: | 10.1 g (99% der Theorie) |
| ESI-MS: | (M+H)⁺ = 255 |
| R_{f} = | 0.15 (Kieselgel, DCM) |

### Beispiel A22

### (4-Piperazin-1-yl-piperidin-1-yl)-essigsäureethylester

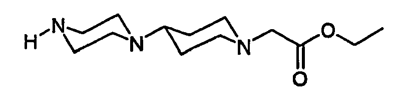

### A22a 4-(4-Benzyl-piperazin-1-yl)-piperidin-1-carbonsäure-tert-butylester

Eine Lösung von 66.7 mL (381 mmol) 1-Benzylpiperazin und 75.6 g (380 mmol) 4-Oxo-piperidin-1-carbonsäure-tert-butylester wurde mit Eisessig auf pH 5 eingestellt. Unter Eiskühlung wurden 100 g (380 mmol) NaBH(OAc)₃ über 2 h zugegeben und das Gemisch wird über Nacht bei RT gerührt. Das Reaktionsgemisch wurde mit K₂CO₃-Lösung (300 g/L) alkalisch gestellt, eine Stunde bei RT gerührt und dreimal mit EtOAc extrahiert. Die organische Phase wurde über MgSO₄ getrocknet und das Lösungsmittel i.vac. entfernt.

| | |
|---|---|
| Ausbeute: | 114 g (83% der Theorie) |
| ESI-MS: | (M+H)⁺ = 369 |
| R_{f} = | 0.74 (Kieselgel, DCM/Cyc/MeOH/NH₃ 70:15:15:2) |

### A22b 1-Benzyl-4-piperidin-4-yl-piperazin

Analog Beispiel 20b wurde aus 40.0 g (111 mmol) 4-(4-Benzyl-piperazin-1-yl)-piperidin-1-carbonsäure-tert-butylester das Produkt als Tris-trifluoracetat-Salz erhalten.

| | |
|---|---|
| Ausbeute: | 54.8 g (82% der Theorie) |
| ESI-MS: | (M+H)⁺ = 260 |
| R_{f} = | 0.18 (Kieselgel, DCM/Cyc/MeOH/NH₃ 70:15:15:2) |

### A22c [4-(4-Benzyl-piperazin-1-yl)-piperidin-1-yl]-essigsäureethylester

Analog Beispiel A19a wurde aus 51.8 g (86 mmol) 1-Benzyl-4-piperidin-4-yl-piperazin (eingesetzt als Tris-trifluoracetat) und 10.3 mL (91 mmol) Bromessigsäure-ethylester das Produkt erhalten.

| | |
|---|---|
| Ausbeute: | 25.3 g (85% der Theorie) |
| EI-MS: | (M)⁺ = 346 |
| R_{f} = | 0.58 (Kieselgel, DCM/Cyc/MeOH/NH₃ 70:15:15:2) |

### A22d (4-Piperazin-1-yl-piperidin-1-yl)-essigsäureethylester

Analog Beispiel A19b wurde aus 25.3 g (73.3 mmol) [4-(4-Benzyl-piperazin-1-yl)-piperidin-1-yl]-essigsäureethylester das Produkt erhalten. Das Produkt enthält 59% (4-Piperazin-1-yl-piperidin-1-yl)-essigsäuremethylester.

| | |
|---|---|
| Ausbeute: | 17.4 g (93% der Theorie) |
| ESI-MS: | (M+H)⁺ = 256 und für Methylester: (M+H)⁺ = 242 |
| R_{f} = | 0.15 (Kieselgel, DCM/Cyc/MeOH/NH₃ 70:15:15:2) |

### Beispiel A23

### 1-(2,2,2-Trifluor-ethyl)-[4,4']bipiperidinyl

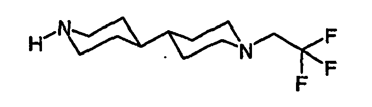

### A23a 1'-(2,2,2-Trifluor-acetyl)-[4,4']bipiperidinyl-1-carbonsäure-tert-butylester

Analog Beispiel A7a wurde aus 15.0 g (55.9 mmol) [4,4']Bipiperidinyl-1-carbonsäure-*tert*-butylester das Produkt hergestellt.

| | |
|---|---|
| Ausbeute: | 19.0 g (93% der Theorie) |
| ESI-MS: | (M+Na)⁺ = 387 |

### A23b 1'-(2,2,2-Trifluor-ethyl)-[4,4']bipiperidinyl-1-carbonsäure-tert-butylester

Analog Beispiel A7b wurde aus 20.7 g (56.7 mmol) 1'-(2,2,2-Trifluor-acetyl)-[4,4']bipiperidinyl-1-carbonsäure-*tert*-butylester das Produkt hergestellt.

| | |
|---|---|
| Ausbeute: | 19.9 g (100% der Theorie) |
| ESI-MS: | (M+H)⁺ = 351 |
| R_{f} = | 0.78 (Kieselgel, PE/EtOAc 1:1) |

### A23c 1-(2,2,2-Trifluor-ethyl)-[4,4']bipiperidinyl

Analog Beispiel A20b wurde aus 21.4 g (61.1 mmol) 1'-(2,2,2-Trifluor-ethyl)-[4,4']bipiperidinyl-1-carbonsäure-*tert*-butylester das Produkt als Bis-Trifluoracetat erhalten.

| | |
|---|---|
| Ausbeute: | 26.8 g (92% der Theorie) |
| ESI-MS: | (M+H)⁺ = 251 |
| R_{f} = | 0.17 (Kieselgel, DCM/MeOH/NH₃ 9:1:0.1) |

### Beispiel A24

### Dimethyl-[3-(4-piperazin-1-yl-phenyl)-propyl]-amin

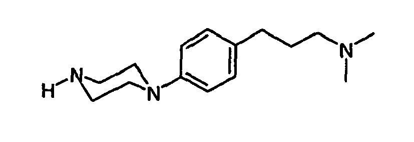

### A24a 1-[4-(4-Benzyl-piperazin-1-yl)-phenyl]-ethanon

Eine Lösung von 35.3 g (200 mmol) Benzylpiperazin und 13.8 g (100 mmol) 4-Fluoracetophenon in 34 mL (200 mmol) Ethyldiisopropylamin wurde 2 Tage unter Rückfluss erhitzt. Nach Abkühlung wurde der Rückstand mit *tert*-Butylmethylether verrührt, abgesaugt und an der Luft getrocknet. Das Produkt wurde ohne Reinigung weiter umgesetzt.

| | |
|---|---|
| Ausbeute: | 12.2 g (42% der Theorie) |
| EI-MS: | (M)⁺ = 294 |
| R_{f} = | 0.53 (Kieselgel, PE/EtOAc 3:2) |

### A24b 1-[4-(4-Benzyl-piperazin-1-yl)-phenyl]-3-dimethylamino-propan-1-on

Zu einer Lösung von 6.9 g (23.4 mmol) 1-[4-(4-Benzyl-piperazin-1-yl)-phenyl]-ethanon und 2.90 g (35.1 mmol) Dimethylamin Hydrochlorid in 100 mL EtOH und 10 mL konz. HCl wurden 1.40 g Paraformaldehyd gegeben. Die Reaktionsmischung wurde 20 h unter Rückfluss erhitzt. Das Lösungsmittel wurde i.vac. entfernt und der Rückstand mit Acetonitril versetzt. Der Niederschlag wurde abgesaugt und im Umlufttrockenschrank bei 30°C getrocknet.

| | |
|---|---|
| Ausbeute: | 4.4 g (48% der Theorie als Hydrochlorid) |
| EI-MS: | (M)⁺ = 351 |
| R_{f} = | 0.35 (Kieselgel, DCM/EtOAc/Cyc/MeOH/NH₃ 60:16:5:5:0.6) |

### A24c Dimethyl-[3-(4-piperazin-1-yl-phenyl)-propyl]-amin

Zu einer Lösung von 8.00 g (20.6 mmol) 1-[4-(4-Benzyl-piperazin-1-yl)-phenyl]-3-dimethylamino-propan-1-on in 6.7 mL konz. HCl und 300 mL MeOH wurden 2 g 10% Pd/C gegeben. Die Reaktionsmischung wurde bei 3 bar H₂ und 50°C 3 h gerührt. Nach Filtration wurde das Filtrat zur Trockene eingedampft. Der Rückstand wurde mit EtOH und EtOAc versetzt und über Nacht gerührt. Der Niederschlag wurde unter Stickstoff abgesaugt und im Umlufttrockenschrank bei 20°C getrocknet.

| | |
|---|---|
| Ausbeute: | 5.7 g (86% der Theorie als Bis-Hydrochlorid) |
| EI-MS: | (M)⁺ = 247 |
| R_{f} = | 0.35 DCM/CycIMeOH/NH₃ 70:15:15:2) |

Die anderen bei der Herstellung der Endverbindungen eingesetzten Amine sind entweder kommerziell erhältlich oder wurden nach literaturbekannten Verfahren hergestellt.

### Herstellung der Endverbindungen

### Beispiel 1

### (S)-2-(4-Amino-3-chlor-5-trifluormethyl-benzyl)-1-[4-(4-methyl-piperazin-1-yl)-piperidin-1-yl]-4-[4-(2-oxo-1,2,4,5-tetrahydro-1,3-benzdiazepin-3-yl)-piperidin-1-yl]-butan-1,4-dion

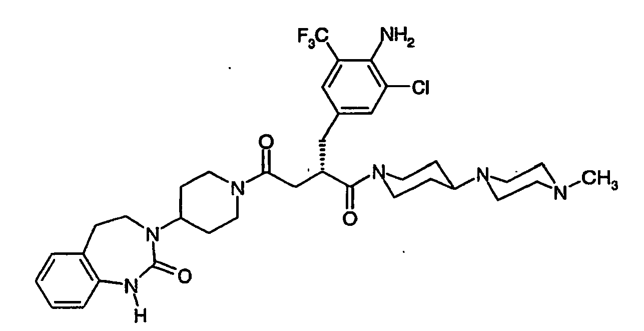

### 1a (4-Amino-3-chlor-5-trifluormethyl-phenyl)-methanol

Zu einer Lösung von 93.4 g (0,39 mol) 4-Amino-3-chlor-5-trifluormethyl-benzoesäure (beschrieben in Arzneim.-Forsch. 1984, 34(11A), 1612-1624) in 1 L THF wurden 69.56 g (0.43 mol) CDI zugegeben und die Mischung 1 h bei 40°C gerührt. Das Reaktionsgemisch wurde anschließend bei RT unter Stickstoffatmosphäre vorsichtig und unter Kühlung zu einer Lösung von 51.4 g (1.36 mol) NaBH₄ in 450 mL Wasser gegeben. Die Mischung wurde 2 h bei RT gerührt, mit 500 mL Wasser und 300 mL halbkonz. HCI versetzt, eine weitere Stunde gerührt und dann mit EtOAc erschöpfend extrahiert. Die vereinigten org. Phasen wurden über Na₂SO₄ getrocknet und i. vac. eingeengt. Das verbleibende Öl wurde mit 500 mL PE versetzt und unter Eiskühlung gerührt. Der Niederschlag wurde abgesaugt, mit PE gewaschen und getrocknet. Man erhielt 29.7 g des gewünschten Produktes.
Die Mutterlauge wurde nochmals eingeengt, mit PE versetzt und gekühlt. Der erhaltene Niederschlag wurde wiederum mit PE gewaschen und getrocknet. Man erhielt weitere 21.8 g des gewünschten Produktes.

| | |
|---|---|
| Ausbeute: | 51.5 g (59% der Theorie) eines weißen Feststoffs |
| R_{f} = | 0.73 (Kieselgel, PE/EtOAc 1:1) |

### 1b 4-Amino-3-chlor-5-trifluormethyl-benzaldehyd

Eine Mischung aus 17.0 g (75.4 mmol) (4-Amino-3-chlor-5-trifluormethyl-phenyl)-methanol, 100 g (1.15 mol) Mangandioxid und 300 mL DCM wurde über Nacht bei RT gerührt. Der Niederschlag wurde abgesaugt und die Lösung i. vac. eingeengt. Man erhielt das gewünschte Produkt als weißen Feststoff.

| | |
|---|---|
| Ausbeute: | 16.0 g (95% der Theorie) |
| ESI-MS: | (M+H)⁺ = 224/226 (Cl) |

### 1c [2-((R)-4-Benzyl-2-oxo-oxazolidin-3-yl)-2-oxo-ethyl]-phosphonsäurediethylester

Eine Lösung von 168.0 g (0.56 mol) (*R*)-4-Benzyl-3-(2-brom-acetyl)-oxazolidin-2-on und 188.6 mL (1.1 mol) Triethylphosphit wurde 1.5 Stunden bei 60°C gerührt, wobei das entstehende Ethylbromid abdestilliert wurde. Das Reaktionsgemisch wurde i. vac. eingedampft und der verbleibende Rückstand über Kieselgel chromatographisch gereinigt. Man erhielt das gewünschte Produkt in Form eines gelb-braunen Öles.

| | |
|---|---|
| Ausbeute: | 130 g (65 % der Theorie) |
| ESI-MS: | (M+H)⁺ = 356 |

### 1d (R)-3-[(E)-3-(4-Amino-3-chlor-5-trifluormethyl-phenyl)-acryloyl]-4-benzyl-oxazolidin-2-on

Unter Stickstoffatmosphäre wurden zu einer Lösung von 31.98 g (90.0 mmol) [2-((*R*)-4-Benzyl-2-oxo-oxazolidin-3-yl)-2-oxo-ethyl]-phosphonsäurediethylester in 400 mL THF 3.93 g (90.0 mmol) NaH (55% in Mineralöl) portionenweise zugegeben. Das Reaktionsgemisch wurde 30 min bei RT und weitere 35 min bei 35°C gerührt. Nach Beendigung der Gasentwicklung wurden 16.0 g (71.5 mmol) 4-Amino-3-chlor-5-trifluormethyl-benzaldehyd, gelöst in 50 mL THF, zugetropft und weitere 12 h bei RT gerührt. Die Reaktionslösung wurde mit gesättigter NH₄Cl-Lösung versetzt, das Gemisch erschöpfend mit EtOAc extrahiert und die vereinigten Extrakte getrocknet und i. vac. eingeengt. Der verbleibende Rückstand wurde über Kieselgel chromatographisch gereinigt. Man erhielt das gewünschte Produkt in Form eines gelben Öles.

| | |
|---|---|
| Ausbeute: | 38.2 g (62% der Theorie) |
| ESI-MS: | (M+H)⁺ = 425/427 (Cl) |
| R_{f} = | 0.55 (Kieselgel, PE/EtOAc 2:1) |

### 1e (R)-3-[3-(4-Amino-3-chlor-5-trifluormethyl-phenyl)-propionyl]-4-benzyl-oxazolidin-2-on

Eine Mischung aus 23.7 g (55.8 mmol) (*R*)-3-[(*E*)-3-(4-Amino-3-chlor-5-trifluormethylphenyl)-acryloyl]-4-benzyl-oxazolidin-2-on, 400 mL MeOH und 5.0 g Raney-Nickel wurde 2 h bei RT und 3 bar H₂ in einem Parr-Autoklaven geschüttelt. Der Katalysator wurde abgesaugt und das Lösungsmittel i. vac. entfernt. Man erhielt das gewünschte Produkt in Form eines gelben Öles.

| | |
|---|---|
| Ausbeute: | 22.5 g (95% der Theorie) |
| ESI-MS: | (M+H)⁺ = 427/429 (Cl) |

### 1f (S)-3-(4-Amino-3-chlor-5-trifluormethyl-benzyl)-4-((R)-4-benzyl-2-oxo-oxazolidin-3-yl)-4-oxo-butansäure-tert-butylester

Unter Argonatmosphäre wurde zu einer auf -78°C gekühlten Lösung von. 22.5 g (52.71 mmol) (*R*)-3-[3-(4-Amino-3-chlor-5-trifluormethyl-phenyl)-propionyl]-4-benzyl-oxazolidin-2-on in 105 mL THF 63.24 mL (63.24 mmol) einer Natrium-bis(trimethylsilyl)-amid-Lösung (1 M in THF) zugetropft und das Gemisch bei -78°C 2 h gerührt. Zu der Reaktionsmischung wurden bei -78°C tropfenweise 38.9 mL (263.5 mmol) Bromessigsäure-*tert*-butylester zugegeben, diese weitere 24 h bei -78°C gerührt und anschließend auf RT gebracht. Nach Zugabe von 200 mL gesättigter NH₄Cl-Lösung wurde zweimal mit je 300 mL EtOAc extrahiert, die vereinigten org. Phasen über Na₂SO₄ getrocknet und i. vac. eingeengt. Der verbleibende Rückstand wurde über Kieselgel chromatographisch gereinigt. Man erhielt das gewünschte Produkt in Form eines gelben Öles.

| | |
|---|---|
| Ausbeute: | 15.6 g (55% der Theorie) |
| ESI-MS: | (M+H)⁺ = 541/543 (Cl) |
| R_{f} = | 0.35 (Kieselgel, PE/EtOAc 8:2) |

### 1g (S)-2-(4-Amino-3-chior-5-trifluormethyl-benzyl)-bernsteinsäure-4-tert-butylester

Zu einer Lösung von 2.51 g (57.6 mmol) Lithiumhydroxid Hydrat in 150 mL Wasser wurden 11.75 ml (115.1 mmol) H₂O₂ (35% in Wasser) zugegeben. Dieses Gemisch wurde anschließend zu einer eisgekühlten Lösung von 15.6 g (28.8 mmol) (*S*)-3-(4-Amino-3-chlor-5-trifluormethyl-benzyl)-4-((*R*)-4-benzyl-2-oxo-oxazolidin-3-yl)-4-oxo-butansäure-*tert*-butylester in 600 mL THF zugetropft und das Reaktionsgemisch für weitere 2 h unter Eiskühlung gerührt. Anschließend wurden zu der Reaktionsmischung 150 mL gesättigte Natriumsulfit-Lösung gegeben und mit Zitronensäure-Lösung angesäuert. Die org. Phase wurde abgetrennt, getrocknet und i. vac. eingeengt. Man erhielt 15.6 g eines zähen gelben Öles.
Die wässrige Phase wurde erschöpfend mit EtOAc extrahiert, die vereinigten org. Phasen mit Wasser gewaschen, getrocknet und i. vac. eingeengt. Man erhielt weitere 5.5 g eines gelben Öles.
Das Rohprodukt, das noch (*R*)-4-Benzyl-oxazolidin-2-on enthielt, wurde ohne Reinigung weiter umgesetzt.

| | |
|---|---|
| Ausbeute: | 21.1 g Rohprodukt |
| ESI-MS: | (M+H)⁺ = 380/382 (Cl) |

### 1 g (S)-3-(4-Amino-3-chlor-5-trifluormethyl-benzyl)-4-[4-(4-methyl-piperazin-1-yl)-piperidin-1-yl]-4-oxo-butansäure-tert-butylester

Eine Mischung aus 15.4 g (40.3 mmol) (*S*)-2-(4-Amino-3-chlor-5-trifluormethyl-benzyl)-bernsteinsäure-4-*tert*-butylester, 7.4 g (40.3 mmol) (1-Methyl-4-piperidin-4-yl)-piperazin, 5.45 g (40.3 mmol) HOBt, 12.94 g (40.3 mmol) TBTU, 11.77 mL (85.0 mmol) Triethylamin und 400 mL THF wurde 12 h bei RT gerührt. Anschließend wurde i. vac. eingeengt und der verbleibende Rückstand zwischen EtOAc und NaHCO₃-Lösung verteilt. Die org. Phase wurde abgetrennt, getrocknet und i. vac. eingeengt. Der erhaltene Rückstand wurde über Aluminiumoxid chromatographisch aufgereinigt. Man erhielt das gewünschte Produkt in Form eines gelben Öles.

| | |
|---|---|
| Ausbeute: | 11.0 g (50% der Theorie) |
| ESI-MS: | (M+H)⁺ = 547/549 (CI) |
| R_{f} = | 0.35 (Alox; EtOAc/DCM 6:4) |

### 1h (S)-3-(4-Amino-3-chlor-5-trifluormethyl-benzyl)-4-[4-(4-methyl-piperazin-1-yl)-piperidin-1-yl]-4-oxo-butansäure

Zu der Lösung von 7.0 g (12.8 mmol) (*S*)-3-(4-Amino-3-chlor-5-trifluormethyl-benzyl)-4-[4-(4-methyl-piperazin-1-yl)-piperidin-1-yl]-4-oxo-butansäure-*tert*-butylester in 375 mL Acetonitril wurden 5.75 g (38.4 mmol) Nal, 3 mL Anisol und 4.92 mL (38.4 mmol) Trimethylsilylchlorid zugegeben. Das Reaktionsgemisch wurde 90 min bei 40°C gerührt, mit weiteren 5.75 g (38.4 mmol) Nal und 4.92 mL (38.4 mmol) Trimethylsilylchlorid versetzt und weitere 2 h bei 40°C gerührt. Die Mischung wurde i. vac. eingeengt und als Rohprodukt weiter umgesetzt.

### 1i (S)-2-(4-Amino-3-chlor-5-trifluormethyl-benzyl)-1-[4-(4-methyl-piperazin-1-yl)-piperidin-1-yl]-4-[4-(2-oxo-1,2,4,5-tetrahydro-1,3-benzdiazepin-3-yl)-piperidin-1-yl]-butan-1,4-dion

Eine Mischung aus 6.3 g (12.8 mmol) (*S*)-3-(4-Amino-3-chlor-5-trifluormethyl-benzyl)-4-[4-(4-methyl-piperazin-1-yl)-piperidin-1-yl]-4-oxo-butansäure, 3.16 g (12.9 mmol) 3-Piperidin-4-yl-1,3,4,5-tetrahydro-1,3-benzdiazepin-2-on, 4.66 g (14.5 mmol) TBTU, 1.96 g (14.5 mmol) HOBt, 9.45 mL (68 mmol) Triethylamin und 300 mL DMF wurde 12 h bei RT gerührt. Das Reaktionsgemisch wurde i. vac. eingeengt, der Rückstand zwischen EtOAc und gesättigter NaHCO₃-Lösung verteilt. Die org. Phase wurde abgetrennt, getrocknet und i. vac. eingeengt. Der Rückstand wurde über Kieselgel chromatographisch aufgereinigt. Das erhaltene gelbe Öl wurde mit Ether verrieben und abgesaugt. Man erhielt das gewünschte Produkt in Form eines weißen Feststoffs.

| | |
|---|---|
| Ausbeute: | 3.8 g (39% der Theorie) |
| ESI-MS: | (M+H)⁺ = 718/20 (CI) |
| R_{f} = | 0.22 (Kieselgel, EtOAc/MeOH/NH₃ 70:30:3) |

### Beispiel 2

### 2-(4-Amino-3-chlor-5-trifluormethyl-benzyl)-1-(1'-methyl-[4,4']bipiperidinyl-1-yl)-4-[4-(2-oxo-1,4-dihydro-2H-chinazolin-3-yl)-piperidin-1-yl]-butan-1,4-dion

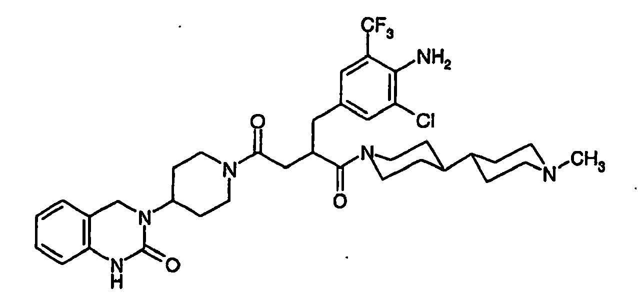

### 2a 2-Chlor-4-chlormethyl-6-trifluormethyl-phenylamin

Zu einer Lösung von 1.00 g (4.43 mmol) (4-Amino-3-chlor-5-trifluormethyl-phenyl)-methanol in 50 mL DCM wurde bei RT 0.94 mL (13.00 mmol) SOCl₂ zugegeben und das Gemisch 3 h bei RT gerührt. Das Reaktionsgemisch wurde auf Eis gegossen und die wässrige Phase mit DCM erschöpfend extrahiert. Die vereinigten org. Phasen wurden mit eiskalter NaHCO₃-Lösung gewaschen, über Na₂SO₄ getrocknet, über Aktivkohle filtriert und i. vac. eingeengt. Das Rohprodukt wurde ohne weitere Reinigung im nachfolgenden Reaktionsschritt eingesetzt.

| | |
|---|---|
| Ausbeute: | 1.08 g (quantitative Ausbeute) |
| EI-MS: | M⁺ = 243/245/247 (Cl₂) |
| R_{f} = | 0.81 (Kieselgel, PE/EtOAc 2:1) |

### 2b 4-(4-Amino-3-chlor-5-trifluormethyl-phenyl)-3,3-bis-ethoxycarbonyl-butansäure-tert-butylester

Zu einer Lösung von 1.20 g (4.43 mmol) 3-Ethoxycarbonyl-bernsteinsäure-1-*tert-*butylester-4-ethylester in 50 mL abs. THF wurden unter Stickstoffatmosphäre und Eiskühlung portionenweise 193 mg (4.43 mmol) NaH (55% in Mineralöl) zugegeben und die Mischung 1 h bei RT gerührt. 1.1 g (4.43 mmol) 2-Chlor-4-chlormethyl-6-trifluormethyl-phenylamin, gelöst in 10 mL abs. THF, wurde zugetropft und das Gemisch 16 h bei RT gerührt. Das Reaktionsgemisch wurde mit Wasser verdünnt und die wässrige Phase mit EtOAc extrahiert. Die org. Phase wurde über MgSO₄ getrocknet und i. vac. eingeengt. Das Rohprodukt wurde ohne weitere Reinigung im nachfolgenden Reaktionsschritt eingesetzt.

| | |
|---|---|
| Ausbeute: | 2.1 g (98% der Theorie) |
| ESI-MS: | (M+H)⁺ = 482/484 (Cl) |
| R_{f} = | 0.48 (Kieselgel, PE/EtOAc 4:1) |

### 2c 4-(4-Amino-3-chlor-5-trifluormethyl-phenyl)-3,3-bis-ethoxycarbonyl-butansäure

Zu einer Lösung von 30.0 g (62.25 mmol) 4-(4-Amino-3-chlor-5-trifluormethylphenyl)-3,3-bis-ethoxycarbonyl-butansäure-*tert*-butylester in 200 mL DCM wurde unter Eiskühlung 20 mL TFA zugegeben und die Mischung 16 h bei RT gerührt. Das Reaktionsgemisch wurde i. vac. eingeengt und der Rückstand aus PE umkristallisiert. Der Niederschlag wurde abfiltriert, mit PE gewaschen und getrocknet.

| | |
|---|---|
| Ausbeute: | 23.6 g (89% der Ausbeute) |
| ESI-MS: | (M-H)⁻ = 424/426 (Cl) |

### 2d 2-(4-Amino-3-chlor-5-trifluormethyl-benzyl)-2-{2-oxo-2-[4-(2-oxo-1,4-dihydro-2H-chinazolin-3-yl)-piperidin-1-yl]-ethyl}-malonsäure-diethylester

Zu einer Lösung von 8.00 g (19.0 mmol) 4-(4-Amino-3-chlor-5-trifluormethyl-phenyl)-3,3-bis-ethoxycarbonyl-butansäure, 4.39 g (19.0 mmol) 3-Piperidin-4-yl-3,4-dihydro-1*H*-chinazolin-2-on, 6.00 g (18.0 mmol) TBTU und 2.75 g (18.0 mmol) HOBT in 100 mL THF wurden 3.2 mL (23.0 mmol) Triethylamin zugetropft und die Mischung 16 h bei RT gerührt. Der gebildete Feststoff wurde abfiltriert, mit Diethylether gewaschen und i. vac. getrocknet.

| | |
|---|---|
| Ausbeute: | 10.45 g (87% der Theorie) |

### 2e 2-(4-Amino-3-chlor-5-trifluormethyl-benzyl)-4-oxo-4-[4-(2-oxo-1,4-dihydro-2H-chinazofin-3-yl)-piperidin-1-yl]-butansäure

Zu einer Lösung von 10.00 g (15.65 mmol) 2-(4-Amino-3-chlor-5-trifluormethyl-benzyl)-2-{2-oxo-2-[4-(2-oxo-1,4-dihydro-2*H*-chinazolin-3-yl)-piperidin-1-yl]-ethyl}-malonsäure-diethylester in 600 mL EtOH wurden 3.13 g (78.25 mmol) NaOH, gelöst in 300 mL Wasser, zugegeben und das Gemisch 4 h unter Rückfluss erhitzt. EtOH wurde i. vac. abgedampft, das Reaktionsgemisch mit konz. HCl auf pH 1 angesäuert und 1 h bei RT. Der gebildete Niederschlag wurde abfiltriert, mit Wasser gewaschen und i. vac. getrocknet.

| | |
|---|---|
| Ausbeute: | 8.01 g (95% der Theorie) |

### 2f 2-(4-Amino-3-chlor-5-trifluormethyl-benzyl)-1-(1'-methyl-[4,4']bipiperidinyl-1-yl)-4-[4-(2-oxo-1,4-dihydro-2H-chinazolin-3-yl)-piperidin-1-yl]-butan-1,4-dion

Zu einer Lösung von 0.80 g (1.48 mmol) 2-(4-Amino-3-chlor-5-trifluormethyl-benzyl)-4-oxo-4-[4-(2-oxo-1,4-dihydro-2*H*-chinazolin-3-yl)-piperidin-1-yl]-butansäure, 0.28 g (1.50 mmol) 1-Methyl-[4,4']bipiperidinyl, 0.49 g (1.50 mmol) TBTU und 0.23 g (1.50 mmol) HOBT in 100 mL THF wurden 2.0 mL Triethylamin zugegeben und die Mischung 16 h bei RT gerührt. Das Reaktionsgemisch wurde i. vac. eingeengt, der Rückstand mit gesättigter NaHCO₃-Lösung versetzt und das Gemisch mit EtOAc erschöpfend extrahiert. Die vereinigten org. Extrakte wurden über MgSO₄ getrocknet und i. vac. eingeengt. Der Rückstand wurde mittels Säulenchromatographie (Kieselgel, Gradient: EtOAc/MeOH/NH₃ 94:5:1 nach 70:25:5) gereinigt.

| | |
|---|---|
| Ausbeute: | 253 mg (24% der Theorie) |
| ESI-MS: | (M+H)⁺ = 703/705 (Cl) |
| R_{f} = | 0.66 (Kieselgel, DCM/Cyc/MeOH/NH₃ 70:15:15:2) |

### Beispiel 3

### 2-(4-Amino-3-chlor-5-trifluormethyl-berizyl)-1-[4-(4-methyl-piperazin-1-yl)-piperidin-1-yl]-4-[4-(2-oxo-1,4-dihydro-2H-chinazolin-3-yl)-pipeddin-1-yl]-butan-1,4-dion

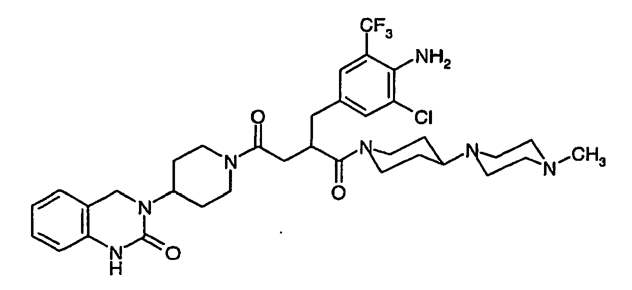

Das Produkt wurde analog zu Beispiel 2f ausgehend von 0.80 g (1.48 mmol) 2-(4-Amino-3-chlor-5-trifluormethyl-benzyl)-4-oxo-4-[4-(2-oxo-1,4-dihydro-2*H*-chinazolin-3-yl)-piperidin-1-yl]-butansäure und 0.30 g (1.50 mmol) 1-Methyl-4-piperidin-4-yl-piperazin erhalten.

| | |
|---|---|
| Ausbeute: | 600 mg (57% der Theorie) |
| EI-MS: | M⁺ = 703/705 (Cl) |
| R_{f}= | 0.56 (Kieselgel, DCM/Cyc/MeOH/NH₃ 70:15:15:2) |

### Beispiel 4

### 2-(4-Amino-3-chlor-5-trifluormethyl-benzyl)-1-[1,4']bipiperidinyl-1'-yl-4-[4-(2-oxo-1,4-dihydro-2H-chinazolin-3-yl)-piperidin-1-yl]-butan-1,4-dion

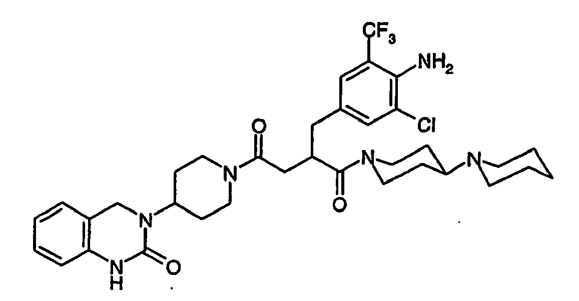

Das Produkt wurde analog zu Beispiel 2f ausgehend von 0.80 g (1.48 mmol) 2-(4-Amino-3-chlor-5-trifluormethyl-benzyl)-4-oxo-4-[4-(2-oxo-1,4-dihydro-2*H*-chinazolin-3-yl)-piperidin-1-yl]-butansäure und 0.27 g (1.50 mmol) [1,4']Bipiperidinyl erhalten.

| | |
|---|---|
| Ausbeute: | 240 mg (24% der Theorie) |
| ESI-MS: | (M+H)⁺ = 689/691 (Cl) |
| R_{f} = | 0.59 (Kieselgel, DCM/Cyc/MeOH/NH₃ 70:15:15:2) |

### Beispiel 5

### 2-(4-Amino-3-chlor-5-trifluormethyl-benzyl)-4-[4-(2-oxo-1,4-dihydro-2H-chinazolin-3-yl)-piperidin-1-yl]-1-(4-pyridin-4-yl-piperazin-1-yl)-butan-1,4-dion

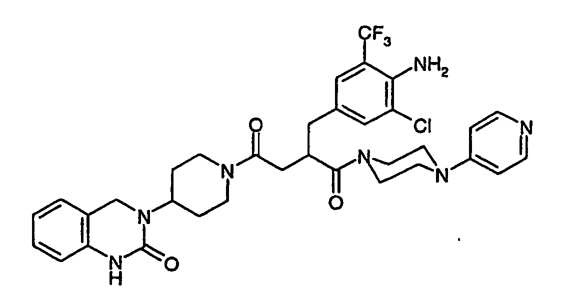

Das Produkt wurde analog zu Beispiel 2f ausgehend von 0.80 g (1.48 mmol) 2-(4-Amino-3-chlor-5-trifluormethyl-benzyl)-4-Oxo-4-[4-(2-oxo-1,4-dihydro-2*H*-chinazolin-3-yl)-piperidin-1-yl]-butansäure und 0.24 g (1.48 mmol) 1-Pyridin-4-yl-piperazin erhalten.

| | |
|---|---|
| Ausbeute: | 500 mg (50% der Theorie) |
| EI-MS: | M⁺ = 683/685 (Cl) |
| R_{f} = | 0.35 (Kieselgel, MeOH) |

### Beispiel 6

### 2-(4-Amino-3-chlor-5-trifluormethyl-benzyl)-1-[4-(1-methyl-piperidin-4-ylamino)-piperidin-1-yl]-4-[4-(2-oxo-1,4-dihydro-2H-chinazolin-3-yl)-piperidin-1-yl]-butan-1,4-dion

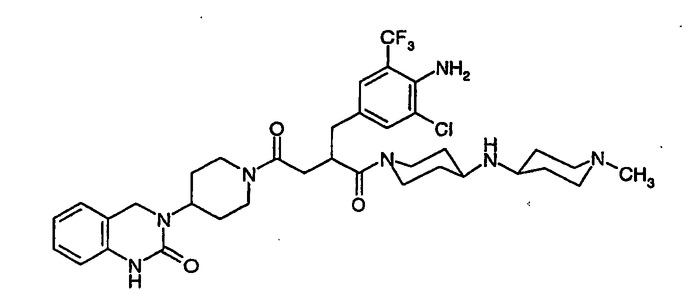

### 6a (1-Benzyl-piperidin-4-yl)-(1-methyl-piperidin-4-yl)-amin

Eine Lösung von 15.0 g (78.8 mmol) 1-Benzyl-piperidin-4-ylamin und 10 mL (78.8 mmol) 1-Methyl-piperidin-4-on in 300 mL THF wurde mit HOAc auf pH 5 angesäuert und 1 h bei RT gerührt. 19.0 g (90.0 mmol) NaBH(OAc)₃ wurden zugegeben und das Gemisch 16 h gerührt. Das Gemisch wurde i. vac. eingeengt, der Rückstand in MeOH gelöst und durch Zugabe von HCl in MeOH ausgefällt. Der gebildete Niederschlag wurde abfiltriert, mit MeOH gewaschen und i. vac. getrocknet.

| | |
|---|---|
| Ausbeute: | 21.8 g (70% der Theorie) |
| R_{f} = | 0.30 (Kieselgel, DCM/Cyc/MeOH/NH₃ 70:15:15:2) |

### 6b (1-Methyl-piperidin-4-yl)-piperidin-4-yl-amin-trihydrochlorid

Zu einer Suspension von 5 g 10% Pd/C in 80 mL Wasser wurde eine Lösung von 10.0 g (25.3 mmol) (1-Benzyl-piperidin-4-yl)-(1-methyl-piperidin-4-yl)-amin in 120 mL MeOH zugegeben und das Gemisch 2 h bei 50 °C und 3 bar H₂ hydriert. Das Reaktionsgemisch wurde filtriert und das Filtrat i. vac. eingeengt. Der Rückstand wurde mit EtOH versetzt, der gebildete Niederschlag abfiltriert, mit EtOH und Ether gewaschen und i, vac. getrocknet.

| | |
|---|---|
| Ausbeute: | 7.75 g (quantitative Ausbeute) |

### 6c 2-(4-Amino-3-chlor-5-trifluormethyl-benzyl)-1-[4-(1-methyl-piperidin-4-ylamino)-piperidin-1-yl]-4-[4-(2-oxo-1,4-dihydro-2H-chinazolin-3-yl)-piperidin-1-yl]-butan-1,4-dion

Das Produkt wurde analog zu Beispiel 2f ausgehend von 0.80 g (1.48 mmol) 2-(4-Amino-3-chlor-5-trifluormethyl-benzyl)-4-oxo-4-[4-(2-oxo-1,4-dihydro-2*H*-chinazolin-3-yl)-piperidin-1-yl]-butansäure und 0.24 g (1.48 mmol) (1-Methyl-piperidin-4-yl)-piperidin-4-yl-amin-trihydrochlorid erhalten.

| | |
|---|---|
| Ausbeute: | 300 mg (25% der Theorie) |
| EI-MS: | M⁺ = 717/719 (CI) |
| R_{f} = | 0.20 (Kieselgel, MeOH) |

### Beispiel 6.1

### [4-(1-{2-(4-Amino-3-chlor-5-trifluormethyl-benzyl)-4-oxo-4-[4-(2-oxo-1,4-dihydro-2H-chinazolin-3-yl)-piperidin-1-yl]-butyryl}-piperidin-4-yl)-piperazin-1-yl]-essigsäure

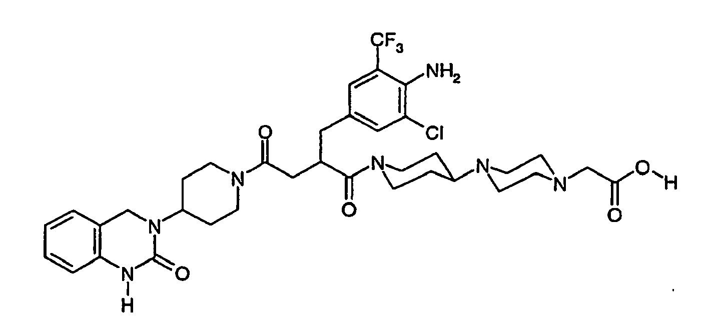

Hergestellt analog Beispiel 16.4 aus 108 mg (0.20 mmol) 2-(4-Amino-3-chlor-5-trifluormethyl-benzyl)-4-oxo-4-[4-(2-oxo-1,4-dihydro-2*H*-chinazolin-3-yl)-piperidin-1-yl]-butansäure und 51 mg (0.20 mmol) (4-Piperidin-4-yl-piperazin-1-yl)-essigsäureethylester.

| | |
|---|---|
| Ausbeute: | 16 mg (10% der Theorie) |
| ESI-MS: | (M+H)⁺ = 748/750(Cl) |

### Beispiel 6.2

### (1'-{2-(4-Amino-3-chlor-5-trifluormethyl-benzyl)-4-oxo-4-[4-(2-oxo-1,4-dihydro-2H-chinazolin-3-yl)-piperidin-1-yl]-butyryl}-[4,4']bipiperidinyl-1-yl)-essigsäuremethylester

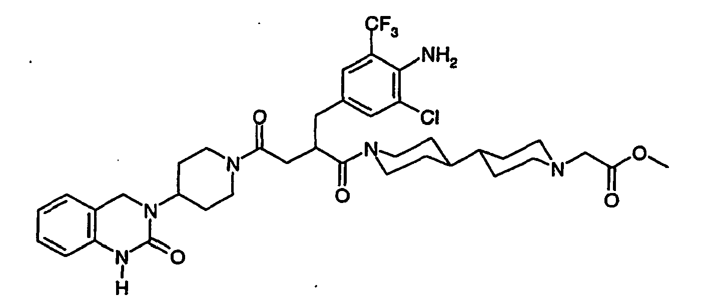

Hergestellt analog Beispiel 16.5 aus 216 mg (0.4 mmol) 2-(4-Amino-3-chlor-5-trifluormethyl-benzyl)-4-oxo-4-[4-(2-oxo-1,4-dihydro-2*H*-chinazolin-3-yl)-piperidin-1-yl]-butansäure und 102 mg (0.2 mmol) [4,4']Bipiperidinyl-1-yl-essigsäureethylester.

| | |
|---|---|
| Ausbeute: | 22 mg (22% der Theorie) |
| ESI-MS: | (M+H)⁺ = 761/763(Cl) |
| R_{f} = | 0.33 (Kieselgel, DCM/MeOH 9:1) |

### Beispiel 6.3

### (1'-{2-(4-Amino-3-chlor-5-trifluormethyl-benzyl)-4-oxo-4-[4-(2-oxo-1,4-dihydro-2H-chinazolin-3-yl)-piperidin-1-yl]-butyryl}-[4,4']bipiperidinyl-1-yl)-essigsäure

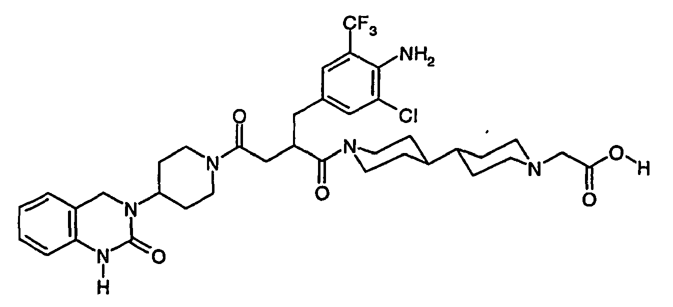

Hergestellt analog Beispiel 16.6 aus 201 mg (0.26 mmol) (1'-{2-(4-Amino-3-chlor-5-trifluormethyl-benzyl)-4-oxo-4-[4-(2-oxo-1,4-dihydro-2*H*-chinazolin-3-yl)-piperidin-1-yl]-butyryl}-[4,4']bipiperidinyl-1-yl)-essigsäuremethylester.

| | |
|---|---|
| Ausbeute: | 22 mg (22% der Theorie) |
| ESI-MS: | (M+H)⁺ = 761/763(Cl) |
| R_{f} = | 0.21 (Kieselgel, DCM/MeOH 9:1) |

### Beispiel 7

### 4-(2-Oxo-1,2,4,5-tetrahydro-1,3-benzdiazepin-3-yl)-piperidin-1-carbonsäure-[(R)-1-(4-amino-3-chlor-5-trifluormethyl-benzyl)-2-1,4'-bipiperidinyl-1'-yl-2-oxo-ethyl]-amid

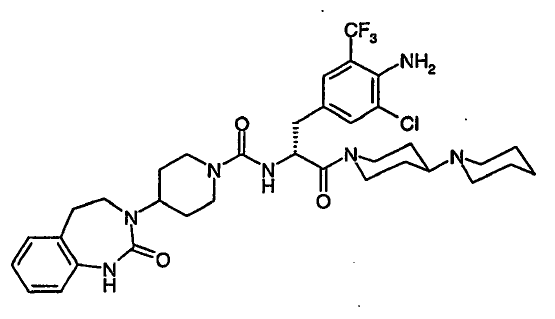

### 7a (R)-2-Amino-3-(4-amino-3-chlor-5-trifluormethyl-phenyl)-N-((1S,2S)-2-hydroxy-1-methyl-2-phenyl-ethyl)-N-methyl-propionamid

Die Alkylierung wurde nach der allgemeinen Arbeitsvorschrift von A. G. Myers et al. (J. Org. Chem. 1999, 64, 3322-3327.) ausgehend von 31.72 g (132 mmol) 2-Amino-*N*-((*1S,2S*)-2-hydroxy-1-methyl-2-phenyl-ethyl)-*N*-methyl-acetamid-monohydrat und 33.8 g (138 mmol) 2-Chlor-4-chlormethyl-6-trifluormethyl-phenylamin durchgeführt. Das Rohprodukt wurde mittels Säulenchromatographie (Kieselgel, DCM/Cyc/MeOH/NH₃ 70:15:15:2) gereinigt.

| | |
|---|---|
| Ausbeute: | 10.0 g (18% der Theorie) |
| ESI-MS: | (M+H)⁺ = 430/432 (Cl) |
| R_{f} = | 0.48 (Kieselgel, DCM/ Cyc/MeOH/NH₃ 70:15:15:2) |

### 7b (R)-2-Amino-3-(4-amino-3-chlor-5-trifluormethyl-phenyl)-propionsäure

Die Hydrolyse wurde nach der allgemeinen Arbeitsvorschrift von A. G. Myers et al. (J. Org. Chem. 1999, 64, 3322-3327.) ausgehend von 10.0 g (23.0 mmol) (*R*)-2-Amino-3-(4-amino-3-chlor-5-trifluormethyl-phenyl)-*N*-((1*S*,2*S*)-2-hydroxy-1-methyl-2-phenyl-ethyl)-*N*-methyl-propionamid durchgeführt. Das Rohprodukt wurde ohne weitere Reinigung im nachfolgenden Syntheseschritt eingesetzt.

### 7c (R)-3-(4-Amino-3-chlor-5-trifluormethyl-phenyl)-2-tert-butoxycarbonylamino-propionsäure

Zu einer Lösung von 6.5 g (23.0 mmol) (R)-2-Amino-3-(4-amino-3-chlor-5-trifluormethyl-phenyl)-propionsäure in 140 mL THF wurde eine Lösung von 3.71 g (35.0 mmol) NaHCO₃ in 100 mL Wasser zugegeben. 15.28 g (70.0 mmol) Boc-Anhydrid wurde zugegeben und das Gemisch 3 h bei RT gerührt. THF wurde i. vac. abgedampft, die wässrige Phase mit EtOAc gewaschen und mit 10% Zitronensäure-Lösung angesäuert. Die wässrige Phase wurde mit EtOAc erschöpfend extrahiert, die vereinigten org. Extrakte über Na₂SO₄ getrocknet und i. vac. eingeengt. Das Rohprodukt wurde ohne weitere Reinigung im nachfolgenden Reaktionsschritt eingesetzt.

| | |
|---|---|
| Ausbeute: | 2.00 g (15% der Theorie) |
| ESI-MS: | (M-H)⁻ = 381/383 (CI) |

### 7d [(R)-1-(4-Amino-3-chlor-5-trifluormethyl-benzyl)-2-[1,4']bipiperidinyl-1'-yl-2-oxo-ethyl]-carbaminsäure-tert-butylester

Zu einer Lösung von 2.00 g (5.22 mmol) (*R*)-3-(4-Amino-3-chlor-5-trifluormethylphenyl)-2-*tert*-butoxycarbonylamino-propionsäure, 0.99 g (5.30 mmol) [1,4']Bipiperidinyl, 1.77 g (5.50 mmol) TBTU und 0.74 g (5.50 mmol) HOBt in 150 mL THF wurden 1.53 mL (11.00 mmol) Triethylamin zugegeben und die Mischung 16 h bei RT gerührt. Das Reaktionsgemisch wurde i. vac. eingeengt, der Rückstand mit gesättigter NaHCO₃-Lösung versetzt und das Gemisch mit EtOAc erschöpfend extrahiert. Die vereinigten org. Extrakte wurden über MgSO₄ getrocknet und i. vac. eingeengt. Der Rückstand wurde mittels Säulenchromatographie (Aluminiumoxid (neutral, Aktivität III), DCM/MeOH 99:1) gereinigt.

| | |
|---|---|
| Ausbeute: | 500 mg (18% der Theorie) |

### 7e (R)-2-Amino-3-(4-amino-3-chlor-5-trifluormethyl-phenyl)-1-[1,4']bipiperidinyl-1'-yl-propan-1-on-dihydrochlorid

Zu einer Lösung von 500 mg (0.75 mmol) [(*R*)-1-(4-Amino-3-chlor-5-trifluormethyl-benzyl)-2-[1,4']bipiperidinyl-1'-yl-2-oxo-ethyl]-carbaminsäure-*tert*-butylester in 50 mL EtOH wurden bei RT 5 mL HCl (12 M in EtOH) zugegeben und das Gemisch 3 h gerührt und anschließend i. vac. eingeengt. Das Rohprodukt wurde ohne weitere Reinigung im nachfolgenden Reaktionsschritt eingesetzt.

| | |
|---|---|
| Ausbeute: | 380 mg (quantitative Ausbeute) |

### 7f 4-(2-Oxo-1,2,4,5-tetrahydro-1,3-benzdiazepin-3-yl)-piperidin-1-carbonsäure-[(R)-1-(4-amino-3-chlor-5-trifluormethyl-benzyl)-2-1,4'-bipiperidinyl-1'-yl-2-oxo-ethyl]-amid

Zu einer Lösung von 380 mg (0.75 mmol) (*R*)-2-Amino-3-(4-amino-3-chlor-5-trifluormethyl-phenyl)-1-[1,4']bipiperidinyl-1'-yl-propan-1-on-dihydrochlorid in 50 mL DMF und 0.56 mL (4.00 mmol) Triethylamin wurden bei 0 °C 180 mg (1.10 mmol) CDT zugegeben und das Gemisch 1.5 h bei 0°C gerührt. 242 mg (0.99 mmol) 3-Piperidin-4-yl-1,3,4,5-tetrahydro-1,3-benzdiazepin-2-on wurden zugegeben und das Reaktionsgemisch 1.5 h bei 100 °C gerührt. DMF wurde i. vac. abgedampft und der Rückstand mittels Säulenchromatographie (Kieselgel, DCM/Cyc/MeOH/NH₃ 70:15:15:2) gefolgt von HPLC gereinigt.

| | |
|---|---|
| Ausbeute: | 140 mg (20% der Theorie) |
| ESI-MS: | (M+H)⁺ = 704/706 (Cl) |
| R_{f} = | 0.58 (Kieselgel, DCM/Cyc/MeOH/NH₃ 70:15:15:2) |

### Beispiel 7.1

### 4-(2-Oxo-1,2,4,5-tetrahydro-1,3-benzdiazepin-3-yl)-piperidin-1-carbonsäure-[(R)-1-(4-amino-3-chlor-5-trifluormethyl-benzyl)-2-(4-dimethylamino-piperidin-1-yl)-2-oxo-ethyl]-amid

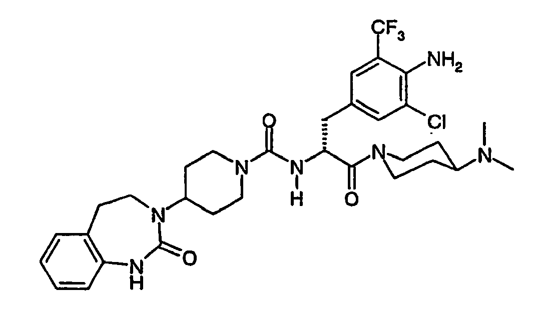

### 7.1a (R)-2-Amino-3-(4-amino-3-chlor-5-trifluormethyl-phenyl)-propionsäureethylester

Eine Lösung von 3.5 g (10.97 mmol) (*R*)-2-Amino-3-(4-amino-3-chlor-5-trifluormethylphenyl)-propionsäure in 100 mL EtOH und 70 mL ethanolischer HCl (11.5 M) wurde über Nacht bei RT gerührt. Man engte i.vac. ein, nahm den Rückstand in 150 mL Wasser auf, versetzte mit 30 mL 15% K₂CO₃-Lösung, extrahierte mit 150 mL EtOAc, trennte die organische Phase ab und trocknete diese über Na₂SO₄. Nach Entfernen des Trocken- und Lösungsmittels erhielt man das gewünschte Produkt.

| | |
|---|---|
| Ausbeute: | 3.5 g (92% der Theorie) |
| ESI-MS: | (M+H)⁺ = 311/313 (CI) |

### 7.1b (R)-3-(4-Amino-3-chlor-5-trifluormethyl-phenyl)-2-{[4-(2-oxo-1,2,4,5-tetrahydro-1,3-benzdiazepin-3-yl)-piperidin-1-carbonyl]-amino}-propionsäureethylester

Zu einer auf 0°C gekühlten Lösung von 3.2 g (10.2 mmol) (*R*)-2-Amino-3-(4-amino-3-chlor-5-trifluormethyl-phenyl)-propionsäureethylester und 1.8 mL (10.3 mmol) Ethyldiisopropylamin in 150 mL THF wurden 1.8 g (11.0 mmol) CDT zugegeben und das Reaktionsgemisch 45 min bei dieser Temperatur und nach Entfernen des Eisbades weitere 30 min gerührt. Dann erfolgte die Zugabe von 2.5 g (10.2 mmol) 3-Piperidin-4-yl-1,3,4,5-tetrahydro-1,3-benzdiazepin-2-on, suspendiert in 50 mL THF. Zur Reaktionslösung wurden 40 mL DMF zugegeben und diese 2 h bei 80°C gerührt. Man engt i.vac. ein, versetzte mit 200 mL EtOAc und 200 mL 10% Zitronensäure-Lösung, trennte die organische Phase ab, extrahierte diese mit 150 mL NaHCO₃-Lösung und trocknete über Na₂SO₄. Nach Entfernen des Trocken- und Lösungsmittels erhielt man das gewünschte Produkt.

| | |
|---|---|
| Ausbeute: | 5.9 g (100% der Theorie) |
| ESI-MS: | (M+H)⁺ = 582/584 (Cl) |
| R_{f} = | 0.4 (Kieselgel, EtOAc) |

### 7.1c (R)-3-(4-Amino-3-chlor-5-trifluormethyl-phenyl)-2-{[4-(2-oxo-1,2,4,5-tetrahydro-1,3-benzdiazepin-3-yl)-piperidin-1-carbonyl]-amino}-propionsäure

Zu einer Suspension von 6.0 g (10.31 mmol) (*R*)-3-(4-Amino-3-chlor-5-trifluormethyl-phenyl)-2-{[4-(2-oxo-1,2,4,5-tetrahydro-1,3-benzdiazepin-3-yl)-piperidin-1-carbonyl]-amino}-propionsäureethylester in 50 mL THF wurde eine Lösung von 0.64 g (15 mmol) Lithiumhydroxid Hydrat in 100 mL Wasser gegeben. Zu dieser Suspension wurden erneut jeweils 100 mL Wasser und THF gegeben, wobei sich nach 5 min eine Lösung bildete. Man rührte 1 h bei RT, zog das THF i.vac. ab, verdünnte mit 100 mL Wasser und tropfte unter Eiskühlung 1 M HCI bis zur sauren Reaktion zu. Die ausgefallene Substanz wurde filtriert, mit Wasser gewaschen und an der Luft getrocknet.

| | |
|---|---|
| Ausbeute: | 5.5 g (96% der Theorie) |
| ESI-MS: | (M+H)⁺ = 554/556 (Cl) |

### 7.1d 4-(2-Oxo-1,2,4,5-tetrahydro-1,3-benzdiazepin-3-yl)-piperidin-1-carbonsäure-[(R)-1-(4-amino-3-chlor-5-trifluormethyl-benzyl)-2-(4-dimethylamino-piperidin-1-yl)-2-oxo-ethyl]-amid

Zu einer Lösung von 400 mg (0.72 mmol) (*R*)-3-(4-Amino-3-chlor-5-trifluormethyl-phenyl)-2-{[4-(2-oxo-1,2,4,5-tetrahydro-1,3-benzdiazepin-3-yl)-piperidin-1-carbonyl]-amino}-propionsäure in 10 mL DMF wurden 241 mg (0.75 mmol) TBTU, 0.21 mL (1.5 mmol) Triethylamin und 103 mg (0.8 mmol) Dimethyl-piperidin-4-yl-amin gegeben und das Reaktionsgemisch über Nacht bei RT gerührt. Die Reaktionslösung wurde auf 150 mL 15% K₂CO₃-Lösung gegossen, 10 min bei RT nachgerührt, die ausgefallene Substanz abgesaugt, diese mit 30 mL Wasser nach gewaschen und über Nacht an der Luft getrocknet. Das Rohprodukt wurde in Isopropanol suspendiert, über Nacht bei RT gerührt, abgesaugt und bei 40°C getrocknet.

| | |
|---|---|
| Ausbeute: | 350 mg (73% der Theorie) |
| ESI-MS: | (M+H)⁺ = 664/666 (Cl) |
| Retentionszeit (HPLC): | 6.0 min (Methode A) |

Analog wurden folgende Verbindungen aus (*R*)-3-(4-Amino-3-chlor-5-trifluormethylphenyl)-2-{[4-(2-oxo-1,2,4,5-tetrahydro-1,3-benzdiazepin-3-yl)-piperidin-1-carbonyl]-amino}-propionsäure und der entsprechenden Menge an Amin hergestellt:

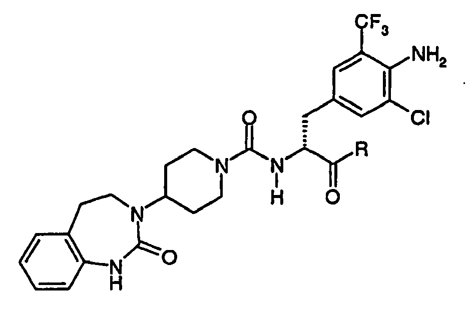

| **Beispiel** | **R** | **Ausbeute (%)** | **Massenspektrum** | **Retentionszeit HPLC (Methode)** |
|---|---|---|---|---|
| **7.2** | 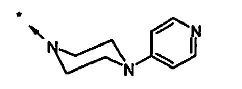 | 44 | 699/701 [M+H]⁺ | 6.1 min (A) |
| **7.3** | 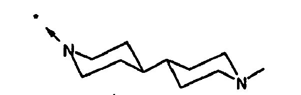 | 27 | 718/720 [M+H]⁺ | 6.2 min (A) |
| **7.4** | 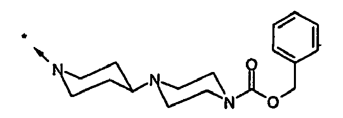 | 79 | 839/841 [M+H]⁺ | 7.1 min (A) |

Analog wurden folgende Verbindungen aus (*R*)-3-(4-Amino-3-chlor-5-trifluormethylphenyl)-2-{[4-(2-oxo-1,2,4,5-tetrahydro-1,3-benzdiazepin-3-yl)-piperidin-1-carbonyl]-amino}-propionsäure und der entsprechenden Menge an Amin hergestellt, wobei das Rohprodukt chromatographisch (Kieselgel, Gradient: DCM nach MeOH/NH₃ 9:1 innerhalb von 45 min) gereinigt wurde:

| **Beispiel** | **R** | **Ausbeute (%)** | **Massen-spektrum** | **Retentionszeit HPLC (Methode)** |
|---|---|---|---|---|
| **7.5** | 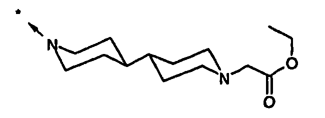 | 53 | 790/792 [M+H]⁺ | 6.6 min (A) |
| **7.6** | 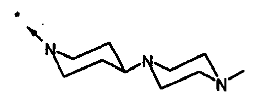 | 84 | 719/721 [M+H]⁺ | 5.7 min (A) |
| **7.7** | 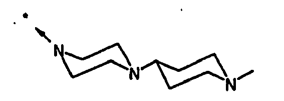 | 43 | 719/721 [M+H]⁺ | 5.0 min (A) |

Analog wurden folgende Verbindungen aus (*R*)-3-(4-Amino-3-chlor-5-trifluormethylphenyl)-2-{[4-(2-oxo-1,2,4,5-tetrahydro-1,3-benzdiazepin-3-yl)-piperidin-1-carbonyl]-amino}-propionsäure und der entsprechenden Menge an Amin hergestellt, wobei das Rohprodukt direkt via HPLC gereinigt wurde:

| **Beispiel** | **R** | **Ausbeute (%)** | **Massenspektrum** | **Retentionszeit HPLC (Methode)** |
|---|---|---|---|---|
| **7.8** | 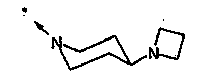 | 17 | 676/678 [M+H]⁺ | 6.2 min (A) |
| **7.9** | 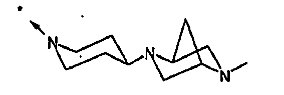 | 52 | 731/733 [M+H]⁺ | 5.5 min (A) |

### Beispiel 7.10

### 4-(2-Oxo-1,2,4,5-tetrahydro-1,3-benzdiazepin-3-yl)-piperidin-1-carbonsäure-[(R)-1-(4-amino-3-chlor-5-trifluormethyl-benzyl)-2-oxo-2-(4-piperazin-1-yl-piperidin-1-yl)-ethyl]-amid

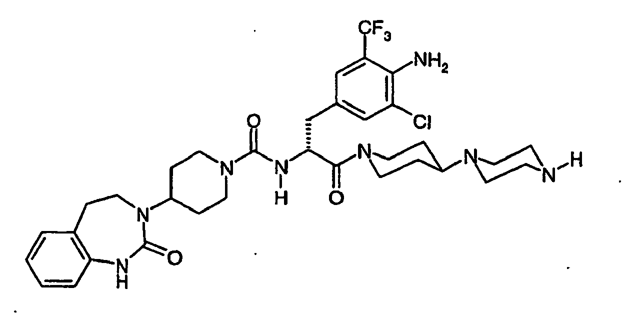

Eine Lösung von 600 mg (0.72 mmol) 4-[1-((*R*)-3-(4-Amino-3-chior-5-trifluormethylphenyl)-2-{[4-(2-oxo-1,2,4,5-tetrahydro-1,3-benzdiazepin-3-yl)-piperidin-1-carbonyl]-amino}-propionyl)-piperidin-4-yl]-piperazin-1-carbonsäurebenzylester (Beispiel 7.4) und 200 mg Raney-Nickel in 50 mL MeOH wurden bei RT und 50 psi H₂ 12 h hydriert. Der Katalysator wurde abfiltriert, das Lösungsmittel i.vac. eingeengt und der Rückstand mittels HPLC gereinigt.

| | |
|---|---|
| Ausbeute: | 160 mg (32% der Theorie) |
| ESI-MS: | (M+H)⁺ = 705/707 (Cl) |
| Retentionszeit (HPLC): | 5.5 min (Methode A) |

### Beispiel 7.11

### [1'-((R)-3-(4-Amino-3-chlor-5-trifluormethyl-phenyl)-2-{[4-(2-oxo-1,2,4,5-tetrahydro-1,3-benzdiazepin-3-yl)-piperidin-1-carbonyl]-amino}-propionyl)-4,4'-bipiperidinyl-1-yl]-essigsäure

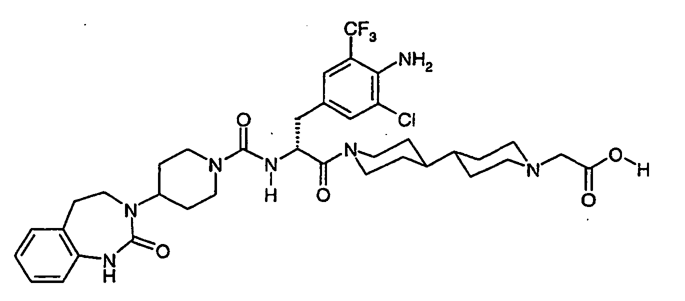

Zu einer Lösung von 250 mg (0.32 mmol) [1'-((R)-3-(4-Amino-3-chlor-5-trifluormethylphenyl)-2-{[4-(2-oxo-1,2,4,5-tetrahydro-1,3-benzdiazepin-3-yl)-piperidin-1-carbonyl]-amino}-propionyl)-4,4'-bipiperidinyl-1-yl]-essigsäureethylester (Beispiel 7.5) in 5 mL THF wurde eine Lösung von 20.1 mg (0.47 mmol) Lithiumhydroxid Hydrat in 10 mL Wasser gegeben und das Reaktionsgemisch 2 h bei RT gerührt. Man versetzte mit 0.5 mL 1 M HCl, saugte die ausgefallene Substanz ab und trocknete dieses bei 50°C. Das Rohprodukt wurde via HPLC gereinigt.

| | |
|---|---|
| Ausbeute: | 85 mg (35% der Theorie) |
| ESI-MS: | (M+H)⁺ = 762/764 (CI) |
| Retentionszeit (HPLC): | 6.2 min (Methode A) |

Analog können folgende Beispiele hergestellt werden:

| **Beispiel** | **Struktur** |
|---|---|
| **7.12** | 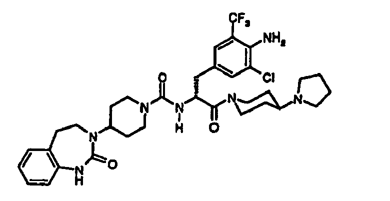 |
| **7.13** | 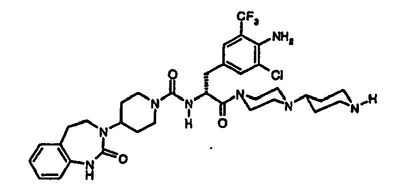 |
| **7.14** | 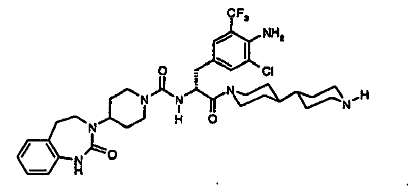 |

### Beispiel 8

### 4-(2-Oxo-1,2,4,5-tetrahydro-1,3-benzdiazepin-3-yl)-piperidin-1-carbonsäure-[1-(4-amino-3-chlor-5-trifluormethyl-benzyl)-2-1,4'-bipiperidinyl-1'-yl-2-oxo-ethyl]-amid

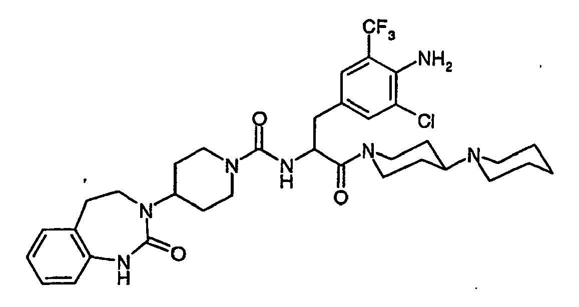

### 8a 2-Acetylamino-2-(4-amino-3-chlor-5-trifluormethyl-benzyl)-malonsäure-diethylester

Zu einer frisch zubereiteten Lösung von 2.55 g (0.11 mol) Natrium in 200 mL abs. EtOH wurde unter Stickstoffatmosphäre 24.11 g (0.11 mol) 2-Acetylaminomalonsäure-diethylester zugegeben und das Gemisch 15 min bei RT gerührt. Eine Lösung von 27.00 g (0.11 mol) 2-Chlor-4-chlormethyl-6-trifluormethyl-phenylamin (Beispiel 2a) in 100 mL 1,4-Dioxan wurde schnell zugetropft und das Gemisch 4 h bei RT gerührt. 500 mL Wasser wurden zugegeben und das Gemisch weitere 16 h gerührt. Der gebildete Niederschlag wurde abfiltriert, mit Wasser gewaschen und i. vac. getrocknet.

| | |
|---|---|
| Ausbeute: | 40.0 g (84% der Theorie) |
| R_{f} = | 0.14 (Kieselgel, PE/EtOAc 2:1) |

### 8b 2-Amino-3-(4-amino-3-chlor-5-trifluormethyl-phenyl)-propionsäure-hydrochlorid

Zu einer Lösung von 40.0 g (94.16 mmol) 2-Acetylamino-2-(4-amino-3-chlor-5-trifluormethyl-benzyl)-malonsäure-diethylester in 110 mL AcOH und 150 mL Wasser wurden 50 mL konz. HCl zugegeben und das Reaktionsgemisch 4 h auf 140 °C erhitzt. Der gebildete Niederschlag wurde abfiltriert und verworfen. Das Filtrat wurde i. vac. eingeengt, mit 100 mL EtOH versetzt und 15 min bei RT gerührt. Der gebildete Niederschlag wurde abfiltriert, mit EtOH gewaschen und i. vac. getrocknet. Das Rohprodukt wurde ohne weitere Reinigung im nachfolgenden Reaktionsschritt eingesetzt.

| | |
|---|---|
| Ausbeute: | 16 g (53% der Theorie) |
| ESI-MS: | (M-H)⁻ = 281/283 (Cl) |

### 8c 2-Amino-3-(4-amino-3-chlor-5-trifluormethyl-phenyl)-propionsäureethylester

16 g (50.14 mmol) 2-Amino-3-(4-amino-3-chlor-5-trifluormethyl-phenyl)-propionsäure-hydrochlorid wurden in 350 mL HCI (12 M in EtOH) gelöst und 5 h bei RT gerührt. Das Reaktionsgemisch wurde i. vac. auf 100 mL eingeengt und mit 200 mL Diethylether versetzt. Der gebildete Niederschlag wurde abfiltriert, mit Diethylether gewaschen und i. vac. getrocknet.

| | |
|---|---|
| Ausbeute: | 12.2 g (70% der Theorie) |
| ESI-MS: | (M+H)⁺ = 311/313 (Cl) |

### 8d 3-(4-Amino-3-chlor-5-trifluormethyl-phenyl)-2-{[4-(2-oxo-1,2,4,5-tetrahydro-1,3-benzdiazepin-3-yl)-piperidin-1-carbonyl]-amino}-propionsäureethylester

Zu einer Suspension von 8.00 g (23.04 mmol) 2-Amino-3-(4-amino-3-chlor-5-trifluormethyl-phenyl)-propionsäureethylester und 16.0 mL (115.00 mmol) Triethylamin in 100 mL DMF wurden bei 0 °C 4.15 g (23.04 mmol) CDT zugegeben und das Gemisch wurde 1.5 h bei 0 °C gerührt. Eine Lösung von 5.64 g (23.00 mmol) 3-Piperidin-4-yl-1,3,4,5-tetrahydro-1,3-benzdiazepin-2-on in 200 mL DMF wurde zugegeben und das Gemisch 2 h auf 100 °C erhitzt. Das Reaktionsgemisch wurde auf RT gekühlt, mit 1.5 L Wasser verdünnt und weitere 10 min gerührt. Der gebildete Niederschlag wurde abfiltriert, mit Wasser gewaschen und i. vac. getrocknet.

| | |
|---|---|
| Ausbeute: | 13.0 g (97% der Theorie) |
| ESI-MS: | (M+H)⁺ = 582/584 (Cl) |

### 8e 3-(4-Amino-3-chlor-5-trifluormethyl-phenyl)-2-{[4-(2-oxo-1,2,4,5-tetrahydro-1,3-benzdiazepin-3-yl)-piperidin-1-carbonyl]-amino}-propionsäure

Zu einer Lösung von 13.00 g (22.34 mmol) 3-(4-Amino-3-chlor-5-trifluormethylphenyl)-2-{[4-(2-oxo-1,2,4,5-tetrahydro-1,3-benzdiazepin-3-yl)-piperidin-1-carbonyl]-amino}-propionsäure-ethylester in 100 mL EtOH wurden 45 mL 1 M NaOH zugegeben und das Gemisch 16 h bei RT gerührt. EtOH wurde i. vac. abgedampft, 45 mL 1 M HCl zugegeben und 15 min gerührt. Der gebildete Niederschlag wurde abfiltriert, mit Wasser gewaschen und i. vac. bei 75 °C getrocknet.

| | |
|---|---|
| Ausbeute: | 10.5 g (85% der Theorie) |
| ESI-MS: | (M-H)⁻ = 552/554 (Cl) |

### 8f 4-(2-Oxo-1,2,4,5-tetrahydro-1,3-benzdiazepin-3-yl)-piperidin-1-carbonsäure-[1-(4-amino-3-chlor-5-trifluormethyl-benzyl)-2-1,4'-bipiperidinyl-1'-yl-2-oxo-ethyl]-amid

Zu einer Lösung von 1.00 g (1.81 mmol) 3-(4-Amino-3-chlor-5-trifluormethyl-phenyl)-2-{[4-(2-oxo-1,2,4,5-tetrahydro-1,3-benzdiazepin-3-yl)-piperidin-1-carbonyl]-amino}-propionsäure, 0.34 g (1.81 mmol) [1,4']Bipiperidinyl und 0.64 g (2.00 mmol) TBTU in 150 mL THF wurden 0.69 mL (5.00 mmol) Triethylamin zugegeben und die Mischung 16 h bei RT gerührt. Das Reaktionsgemisch wurde i. vac. eingeengt, der Rückstand mit gesättigter NaHCO₃-Lösung versetzt und das Gemisch mit EtOAc erschöpfend extrahiert. Die vereinigten org. Extrakte wurden über MgSO₄ getrocknet und i. vac. eingeengt. Der Rückstand wurde mittels Säulenchromatographie (Kieselgel, EtOAc/MeOH/NH₃ 75:25:2.5) gereinigt.

| | |
|---|---|
| Ausbeute: | 350 mg (28% der Theorie) |
| ESI-MS: | (M+H)⁺ = 704/706 (Cl) |
| R_{f} = | 0.58 (Kieselgel, DCM/Cyc/MeOH/NH₃ 70:15:15:2) |

### Beispiel 9

### (S)-2-(4-Amino-3-brom-5-trifluormethyl-benzyl)-1-[4-(4-methyl-piperazin-1-yl)-piperidin-1-yl]-4-[4-(2-oxo-1,2,4,5-tetrahydro-1,3-benzdiazepin-3-yl)-piperidin-1-yl]-butan-1,4-dion

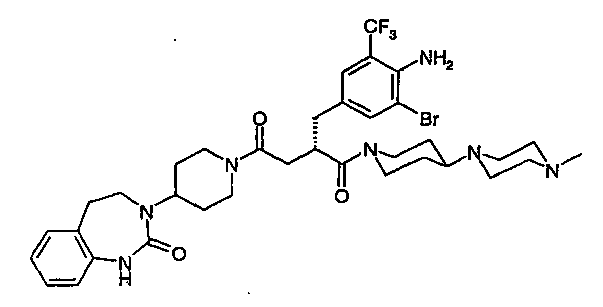

### 9a 4-Amino-3-trifluormethyl-benzoesäureethylester

Eine Lösung von 150 g (0.66 mol) *N*-(4-Cyan-2-trifluormethyl-phenyl)-acetamid in 360 mL trockenem EtOH und 540 mL 10 M ethanolischer HCL wurde in einer Druckapparatur 2 h 45 min auf 70°C erhitzt. Nach Abkühlen der Lösung wurde der entstandene Niederschlag abgesaugt, mit EtOH nach gewaschen und das Filtrat i.vac eingeengt. Der Rückstand wurde mit jeweils 300 mL Wasser und EtOH versetzt, kräftig gerührt, abgesaugt und mit Wasser/EtOH 1:1 nach gewaschen. Das Rohprodukt wurde ohne Reinigung weiter umgesetzt.

| | |
|---|---|
| Ausbeute: | 153 g (100% der Theorie) |
| R_{f} = | 0.4 (Kieselgel, PE/EtOAc 1:1) |

### 9b 4-Amino-3-trifluormethyl-benzoesäure

Zu einer Lösung von 100 g (2.5 mol) NaOH in 250 mL Wasser wurde bei RT eine Lösung von 153 g (0.66 mol) 4-Amino-3-trifluormethyl-benzoesäureethylester in 350 mL EtOH zugegeben und das Reaktionsgemisch 2 h bei 45°C gerührt. EtOH wurde i.vac entfernt, die verbleibende wässrige Lösung mit konz. HCl sauer gestellt, das ausgefallene Produkt abgesaugt, mit Wasser gewaschen und an der Luft getrocknet.

| | |
|---|---|
| Ausbeute: | 129 g (96% der Theorie) |
| ESI-MS: | (M-H)⁻ = 204 |

### 9c 4-Amino-3-brom-5-trifluormethyl-benzoesäure

Zu einer Lösung von 21.0 g (102 mmol) 4-Amino-3-trifluormethyl-benzoesäure in 250 mL Essigsäure wurde eine Lösung von 6 mL (117 mmol) Brom in 50 mL Essigsäure langsam zugetropft und anschliessend 2 h auf 60°C erhitzt. Nach dem Abkühlen wurde mit 1 L Wasser versetzt und der Niederschlag abgesaugt. Der Rückstand wurde in DCM gelöst, die organische Phase mit NaOH-Lösung alkalisch gestellt, die wässrige Phase abgetrennt und mit konz. HCl angesäuert. Der Niederschlag wurde abgesaugt und bei 60°C getrocknet.

| | |
|---|---|
| Ausbeute: | 18 g (62% der Theorie) |
| ESI-MS: | (M-H)⁻ = 282/284 (Br) |
| R_{f} = | 0.6 (Kieselgel, PE/EtOAc/AcOH 50:50:1) |

### 9d (4-Amino-3-brom-5-trifluormethyl-phenyl)-methanol

Zu einer Lösung von 18 g (63.4 mmol) 4-Amino-3-brom-5-trifluormethyl-benzoesäure in 400 mL THF wurden 12 g (74 mmol) CDI zugegeben, 1 h bei RT gerührt und 1 h auf 40°C erwärmt. Die aktivierte Säure wurde dann zu einer Lösung von 8.0 g (212 mmol) NaBH₄ in 200 mL Wasser zugetropft, wobei die Temperatur 40°C nicht überschreiten sollte. Das Reaktionsgemisch wurde 2.5 h bei RT gerührt, mit 300 mL halbkonz. HCl versetzt, 1 h nachgerührt und erschöpfend mit EtOAc extrahiert. Die organische Phase wurde mit 15% K₂CO₃-Lösung gewaschen und über Na₂SO₄ getrocknet. Nach Entfernen des Trocken- und Lösungsmittels wurde der Rückstand bei 50°C in Isopropanol gelöst; nach Abkühlen wurde der Niederschlag abgesaugt, in PE aufgenommen und erneut abgesaugt.

| | |
|---|---|
| Ausbeute: | 12.5 g (73% der Theorie) |
| EI: | (M)⁺ = 269/271 (Br) |
| R_{f} = | 0.9 (Kieselgel, MeOH) |

### 9e 4-Amino-3-brom-5-trifluormethyl-benzaldehyd

Zu einer Lösung von 12.5 g (46.3 mmol) (4-Amino-3-brom-5-trifluormethyl-phenyl)-methanol in 150 mL DCM wurden 53 g (0.61 mol) MnO₂ gegeben und das Reaktionsgemisch über Nacht bei RT gerührt. Das MnO₂ wurde abgesaugt, mit DCM nach gewaschen, das Lösungsmittel i.vac. entfernt und der Rückstand mit PE verrührt. Der Niederschlag wurde abgesaugt, mit wenig PE gewaschen und getrocknet.

| | |
|---|---|
| Ausbeute: | 9.5 g (77% der Theorie) |
| ESI-MS: | (M+H)⁺ = 268/270 (Br) |
| R_{f} = | 0.6 (Kieselgel, PE/EtOAc 2:1) |

### 9f 2-[1-(4-Amino-3-brom-5-trifluormethyl-phenyl)-meth-(E)-yliden]-bernsteinsäure-1-methylester

Zu einer Lösung von 9.5 g (35.4 mmol) 4-Amino-3-brom-5-trifluormethyl-benzaldehyd in 80 mL THF wurden 27.9 g (71.0 mmol) 2-(Triphenyl-λ⁵-phosphanyliden)-bernsteinsäure-1-methylester gegeben und das Reaktionsgemisch 120 h auf 40°C erwärmt. Der Niederschlag wurde abgesaugt, das Filtrat i.vac. eingeengt, der Rückstand mit Wasser und EtOAc versetzt, die organische Phase abgetrennt, diese dreimal mit Wasser gewaschen und dreimal mit 5% K₂CO₃-Lösung extrahiert. Die wässrige Phase wurde mit konz. HCl angesäuert, der entstandene Niederschlag abgetrennt, dieser mit Wasser gewaschen und bei 60°C getrocknet.

| | |
|---|---|
| Ausbeute: | 5.9 g (44%.der Theorie) |
| ESI-MS: | (M+H)⁺ = 382/384 (Br) |

### 9g (S)-2-(4-Amino-3-brom-5-trifluormethyl-benzyl)-bemsteinsäure-1-methylester

Unter Argon-Atmosphäre wurden zu einer Lösung von 5.9 g (15.44 mmol) 2-[1-(4-Amino-3-brom-5-trifl uormethyl-phenyl)-meth-(E)-yliden]-bernsteinsäure-1-methylester in 50 mL entgastem MeOH und 5.9 mL Triethylamin 130 mg (+)-1,2-Bis((*2R,5R*)-2,5-diethylphospholano)benzol(cyclooctadien)rhodium(I)tetrafluorborat gegeben und das Reaktionsgemisch bei 50 psi H₂ 4 h hydriert. Anschliessend wurde die Reaktionslösung i.vac. eingeengt, der Rückstand in 100 mL EtOAc gelöst, zweimal mit 2 M HCl gewaschen und erschöpfend mit 5% K₂CO₃-Lösung extrahiert. Die wässrige Phase wurde mit konz. HCl angesäuert, mit EtOAc erschöpfend extrahiert und die organische Phase über Na₂SO₄ getrocknet. Nach Entfernen des Trocken- und Lösungsmittels erhielt man das gewünschte Produkt.

| | |
|---|---|
| Ausbeute: | 5.8 g (98% der Theorie) |
| ESI-MS: | (M-H)⁻ = 382/384 (Br) |

### 9h (S)-2-(4-Amino-3-brom-5-trifluormethyl-benzyl)-4-oxo-4-[4-(2-oxo-1,2,4,5-tetrahydro-1,3-benzdiazepin-3-yl)-piperidin-1-yl]-butansäuremethylester

Zu einer Lösung von 5.80 g (15.1 mmol) (*S*)-2-(4-Amino-3-brom-5-trifluormethylbenzyl)-bernsteinsäure-1-methylester, 4.98 g (15.1 mmol) TBTU, 2.04 g (15.1 mmol) HOBt und 4.87 mL (35 mmol) Triethylamin in 200 mL THF wurden 3.70 g (15.1 mmol) 3-Piperidin-4-yl-1,3,4,5-tetrahydro-1,3-benzdiazepin-2-on gegeben und das Reaktionsgemisch über Nacht bei RT gerührt. Die Reaktionslösung wurde i.vac eingeengt, mit EtOAc und 20% Zitronensäure-Lösung versetzt, die organische Phase abgetrennt, mit gesättigter NaHCO₃-Lösung gewaschen und über Na₂SO₄ getrocknet. Nach Entfernen des Trocken- und Lösungsmittels erhielt man das gewünschte Produkt.

| | |
|---|---|
| Ausbeute: | 9.2 g (100% der Theorie) |
| ESI-MS: | (M+H)⁺ = 611/613 (Br) |

### 9i (S)-2-(4-Amino-3-brom-5-trifluormethyl-benzyl)-4-oxo-4-[4-(2-oxo-1,2,4,5-tetrahydro-1,3-benzdiazepin-3-yl)-piperidin-1-yl]-butansäure

Zu einer Lösung von 9.2 g (15.05 mmol) (*S*)-2-(4-Amino-3-brom-5-trifluormethylbenzyl)-4-oxo-4-[4-(2-oxo-1,2,4,5-tetrahydro-1,3-benzdiazepin-3-yl)-piperidin-1-yl]-butansäuremethylester in 70 mL THF wurde bei RT eine Lösung von 1.04 g (24.30 mmol) Lithiumhydroxid Hydrat in 30 mL Wasser gegeben und das Reaktionsgemisch 3 h bei RT gerührt. Das THF wurde i.vac. abgezogen, die wässrige Lösung mit konz. HCI angesäuert, mit EtOAc erschöpfend extrahiert, die organische Phase abgetrennt und über Na₂SO₄ getrocknet. Nach Entfernen des Trocken- und Lösungsmittels erhielt man des gewünschte Produkt.

| | |
|---|---|
| Ausbeute: | 7.8 g (87% der Theorie) |
| ESI-MS: | (M+H)⁺ = 597/599 (Br) |

Analog der in 9f bis 9i beschriebenen Sequenz konnte aus 4-Amino-3-chlor-5-trifluormethyl-benzaldehyd (siehe Beispiel 1b) (*S*)-2-(4-Amino-3-chlor-5-trifluormethylbenzyl)-bernsteinsäure-1-methylester und (*S*)-2-(4-Amino-3-chlor-5-trifluormethylbenzyl)-4-oxo-4-[4-(2-oxo-1,2,4,5-tetrahydro-1,3-benzdiazepin-3-yl)-piperidin-1-yl]-butansäure erhalten werden.

### 9k (S)-2-(4-Amino-3-brom-5-trifluormethyl-benzyl)-1-[4-(4-methyl-piperazin-1-yl)-piperidin-1-yl]-4-[4-(2-oxo-1,2,4,5-tetrahydro-1,3-benzdiazepin-3-yl)-piperidin-1-yl]-butan-1,4-dion

Zu einer Lösung von 500 mg (0.84 mmol) (*S*)-2-(4-Amino-3-brom-5-trifluormethylbenzyl)-4-oxo-4-[4-(2-oxo-1,2,4,5-tetrahydro-1,3-benzdiazepin-3-yl)-piperidin-1-yl]-butansäure, 289 mg (0.9 mmol) TBTU, 122 mg (0.9 mmol) HOBt und 0.35 mL (2.5 mmol) Triethylamin in 40 mL THF und 5 mL DMF wurden 154 mg (0.84 mmol) 1-Methyl-4-piperidin-4-yl-piperazin gegeben und das Reaktionsgemisch über Nacht bei RT gerührt. Die Reaktionslösung wurde i.vac. eingeengt, mit EtOAc und gesättigter NaHCO₃-Lösung versetzt, die organische Phase abgetrennt und über Na₂SO₄ getrocknet. Nach Entfernen des Trocken- und Lösungsmittels wurde der Rückstand chromatographisch (Kieselgel, Gradient: DCM nach MeOH/NH₃ 95:5) gereinigt.

| | |
|---|---|
| Ausbeute: | 423 mg (66% der Theorie) |
| ESI-MS: | (M+H)⁺ = 762/764 (Br) |
| Retentionszeit (HPLC): | 5.9 min (Methode A) |

Analog wurden folgende Verbindungen aus jeweils 500 mg (S)-2-(4-Amino-3-brom-5-trifluormethyl-benzyl)-4-oxo-4-[4-(2-oxo-1,2,4,5-tetrahydro-1,3-benzdiazepin-3-yl)-piperidin-1-yl]-butansäure und der entsprechenden Menge an Amin hergestellt:

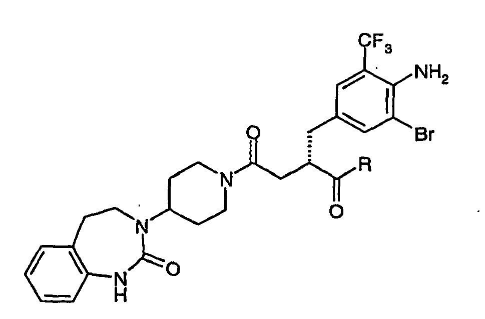

| **Beispiel** | **R** | **Ausbeute (%)** | **Massenspektrum** | **Retentionszeit HPLC (Methode)** |
|---|---|---|---|---|
| **9.1** | 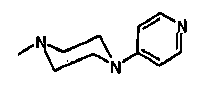 | 50 | 742/744 [M+H]⁺ | 6.4 min (A) |
| **9.2** | 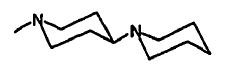 | 48 | 747/749 [M+H]⁺ | -6.5 min (A) |
| **9.3** | 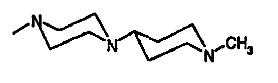 | 61 | 762/764 [M+H]⁺ | 5.4 min (A) |
| **9.4** | 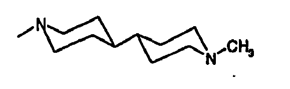 | 34 | 761/763 [M+H]⁺ | 6.3 min (A) |

Analog können folgende Beispiele hergestellt werden:

| **Beispiel** | **Struktur** |
|---|---|
| **9.5** | 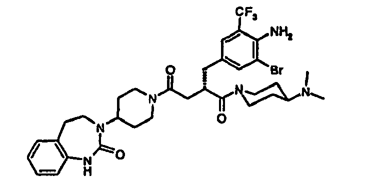 |
| **9.6** | 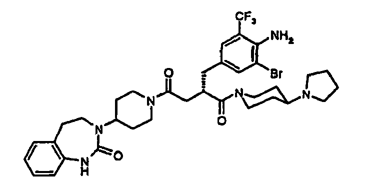 |
| **9.7** | 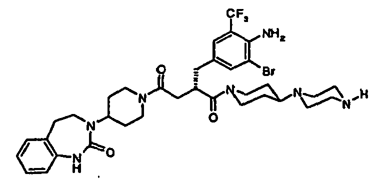 |
| **9.8** | 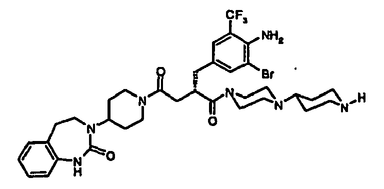 |
| **9.9** | 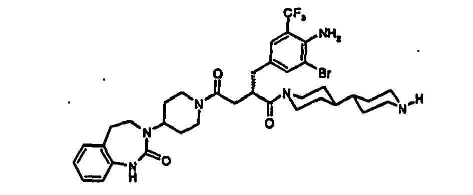 |

### Beispiel 9.10

### (S)-2-(4-Amino-3-trifluormethyl-benzyl)-1-[4-(4-methyl-piperazin-1-yl)-piperidin-1-yl]-4-[4-(2-oxo-1,2,4,5-tetrahydro-1,3-benzdiazepin-3-yl)-piperidin-1-yl]-butan-1,4-dion

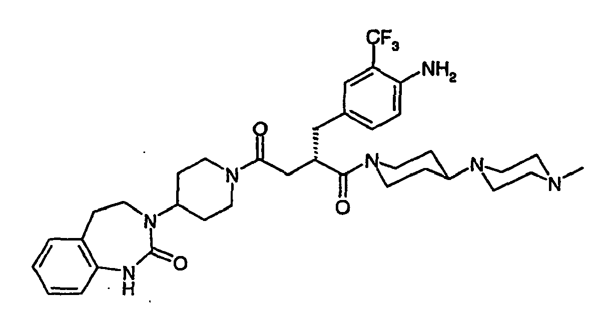

Zu einer Lösung von 150 mg (0.2 mmol) (*S*)-2-(4-Amino-3-brom-5-trifluormethylbenzyl)-1-[4-(4-methyl-piperazin-1-yl)-piperidin-1-yl]-4-[4-(2-oxo-1,2,4,5-tetrahydro-1,3-benzdiazepin-3-yl)-piperidin-1-yl]-butan-1,4-dion in 20 mL MeOH wurden 100 mg 10% Pd/C gegeben und das Reaktionsgemisch bei 50 psi H₂ 3 h bei RT hydriert. Der Katalysator wurde abgesaugt, das Lösungsmittel i.vac. eingeengt, der Rückstand mit 5% K₂CO₃-Lösung und EtOAc versetzt, die organische Phase abgetrennt und diese i.vac. eingeengt. Der Rückstand wurde mit Diisopropylether verrieben und abgesaugt.

| | |
|---|---|
| Ausbeute: | 134 mg (100% der Theorie) |
| ESI-MS: | (M+H)⁺ = 684 |
| Retentionszeit (HPLC): | 5.5 min (Methode A) |

### Beispiel 10

### (S)-2-(4-Amino-3-chlor-5-trifluormethyl-benzyl)-1-(4-dimethylamino-piperidin-1-yl)-4-[4-(2-oxo-1,2,4,5-tetrahydro-1,3-berizdiazepin-3-yl)-piperidin-1-yl]-butan-1,4-dion

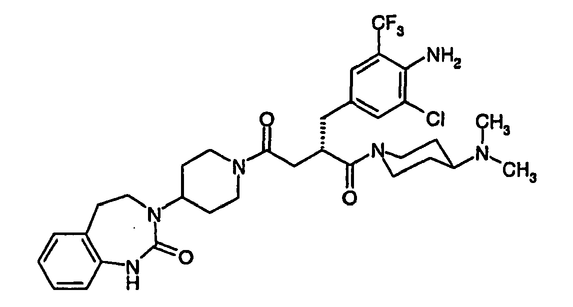

Zu einer Lösung von 130 mg (0.24 mmol) (*S*)-2-(4-Amino-3-chlor-5-trifluormethylbenzyl)-4-oxo-4-[4-(2-oxo-1,2,4,5-tetrahydro-1,3-benzdiazepin-3-yl)-piperidin-1-yl] butansäure in 10 mL DMF wurden 80.3 mg (0.25 mmol) TBTU, 0.21 mL (1.2 mmol) Ethyldiisopropylamin und 38.5 mg (0.3 mmol) Dimethyl-piperidin-4-yl-amin gegeben und das Reaktionsgemisch über Nacht bei RT gerührt. Die Reaktionslösung wurde auf 80 mL 15% K₂CO₃-Lösung gegossen, 10 min bei RT nachgerührt, die ausgefallene Substanz abgesaugt, diese mit 5 mL Wasser nach gewaschen und über das Wochenende an der Luft getrocknet. Anschliessend wurde das Produkt chromatographisch via HPLC gereinigt.

| | |
|---|---|
| Ausbeute: | 82 mg (53% der Theorie) |
| ESI-MS: | (M+H)⁺ = 663/665 (CI) |
| Retentionszeit (HPLC): | 6.1 min (Methode A) |

Analog wurden folgende Verbindungen hergestellt:

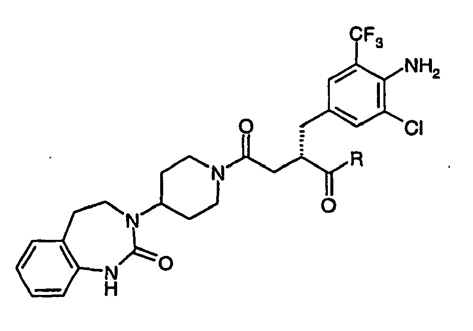

| **Beispiel** | **R** | **Ausbeute (%)** | **Massenspektrum** | **Retentionszeit HPLC (Methode)** |
|---|---|---|---|---|
| **10.1** | 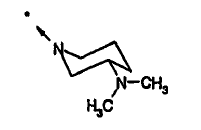 | 55 | 663/665 [M+H]⁺ | 6.3 min 6.5 min (A) |
| **10.2** | 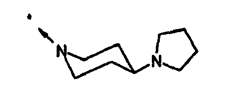 | 51 | 689/691 [M+H]⁺ | 6.4 min (A) |
| **10.3** | 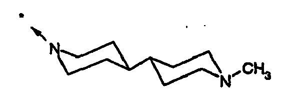 | 56 | 717/719 [M+H]⁺ | 6.2 min (A) |
| **10.4** | 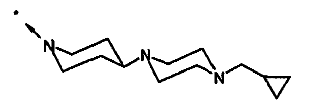 | 49 | 758/760 [M+H]⁺ | 6.0 min (A) |
| **10.5** | 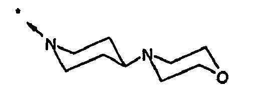 | 45 | 705/707 [M+H]⁺ | 6.2 min (A) |
| **10.6** | 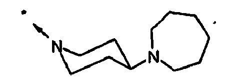 | 58 | 717/719 [M+H]⁺ | 6.6 min (A) |
| **10.7** | 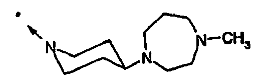 | 54 | 732/734 [M+H]⁺ | 5.4 min (A) |
| **10.8** | 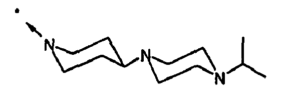 | 50 | 746/748 [M+H]⁺ | 5.9 min (A) |
| **10.9** | 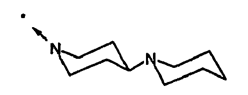 | 67 | 703/705 [M+H]⁺ | 6.4 min (A) |
| **10.10** | 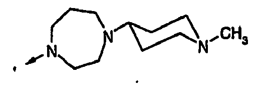 | 53 | 732/734 [M+H]⁺ | 5.5 min (A) |
| **10.11** | 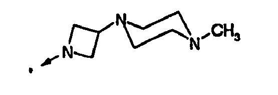 | 25 | 690/692 [M+H]⁺ | 6.0 min (A) |
| **10.12** | 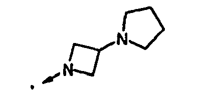 | 48 | 661/663 [M+H]⁺ | 6.3 min (A) |
| **10.13** | 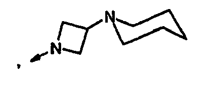 | 56 | 675/677 [M+H]⁺ | 6.5 min (A) |
| **10.14** | 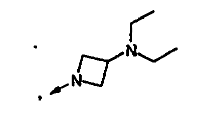 | 53 | 663/665 [M+H]⁺ | 6.4 min (A) |
| **10.15** | 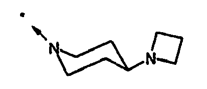 | 52 | 675/677 [M+H]⁺ | 6.3 min (A) |
| **10.16** | 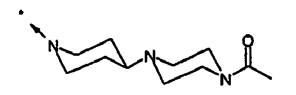 | 36 | 746/748 [M+H]⁺ | 6.1 min (A) |
| **10.17** | 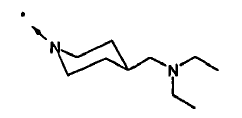 | 38 | 705/707 [M+H]⁺ | 6.4 min (A) |
| **10.18** | 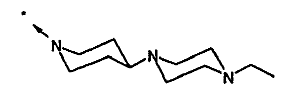 | 52 | 732/734 [M+H]⁺ | 5.8 min (A) |
| **10.19** | 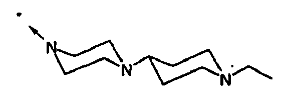 | 40 | 732/734 [M+H]⁺ | 5.4 min (A) |
| **10.20** | 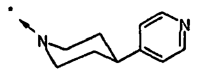 | 53 | 697/699 [M+H]⁺ | 6.3 min (A) |
| **10.21** | 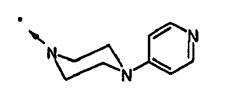 | 60 | 698/700 [M+H]⁺ | 6.3 min (A) |
| **10.22** | 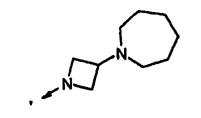 | 53 | 689/691 [M+H]⁺ | 6.7 min (A) |
| **10.23** | 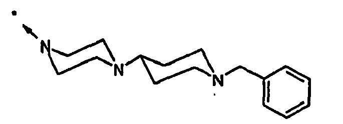 | 58 | 794/796 [M+H]⁺ | 5.7 min (A) |
| **10.24** | 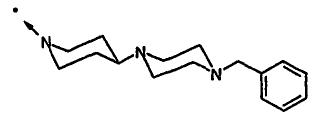 | 21 | 794/796 [M+H]⁺ | 6.8 min (A) |
| **10.25** | 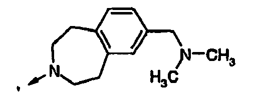 | 47 | 739/741 [M+H]⁺ | 6.6 min (A) |

### Beispiel 10.26

### (S)-2-(4-Amino-3-chlor-5-trifluormethyl-benzyl)-1-[4-(4-dimethylaminomethyl-phenyl)-piperidin-1-yl]-4-[4-(2-oxo-1,2,4,5-tetrahydro-1,3-benzdiazepin-3-yl)-piperidin-1-yl]-butan-1,4-dion

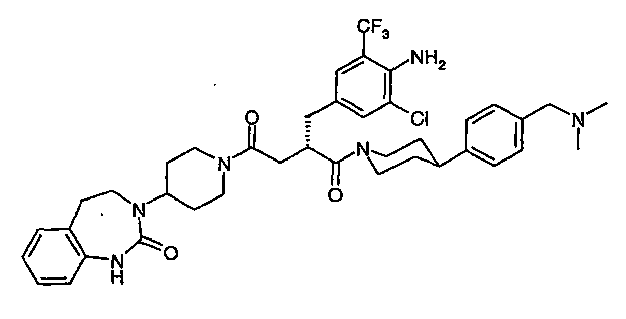

Zu einer Lösung von 130 mg (0.24 mmol) (*S*)-2-(4-Amino-3-chlor-5-trifluormethylbenzyl)-4-oxo-4-[4-(2-oxo-1,2,4,5-tetrahydro-1,3-benzdiazepin-3-yl)-piperidin-1-yl]-butansäure in 2 mL DMF wurden 80 mg (0.25 mmol) TBTU und 87 µl (0.5 mmol) Ethyldiisopropylamin gegeben und diese 30 min bei RT gerührt. Anschliessend wurden 80 mg (0.31 mmol) Dimethyl-(4-piperidin-4-yl-benzyl)-amin (eingesetzt als Hydrochlorid) zugegeben und das Reaktionsgemisch über Nacht bei RT gerührt. Die Reaktionslösung wurde über einen Spritzenfilter filtriert und direkt chromatographisch via HPLC gereinigt.

| | |
|---|---|
| Ausbeute: | 80 mg (45% der Theorie) |
| ESI-MS: | (M+H)⁺ = 753/755 (Cl) |
| Retentionszeit (HPLC): | 6.6 min (Methode A) |

Analog wurden folgende Verbindungen aus jeweils 130 mg (*S*)-2-(4-Amino-3-chlor-5-trifluormethyl-benzyl)-4-oxo-4-[4-(2-oxo-1,2,4,5-tetrahydro-1,3-benzdiazepin-3-yl)-piperidin-1-yl]-butansäure und der entsprechenden Menge an Amin (mit 1.07 eq. an Ethyldiisopropylamin bei den freien Aminen und der zusätzlich nötigen Menge an Base beim Einsatz von Aminsalzen) hergestellt:

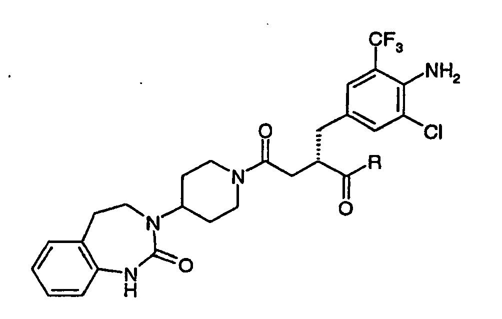

| **Beispiel** | **R** | **Ausbeute (%)** | **Massenspektrum** | **Retentionszeit HPLC (Methode)** |
|---|---|---|---|---|
| **10.27** | 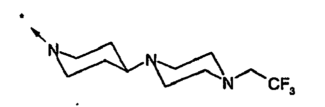 | 51 | 786/788 [M+H]⁺ | 6.9 min (A) |
| **10.28** | 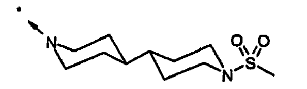 | 41 | 781/783 [M+H]⁺ | 8.1 min (A) |
| **10.29** | 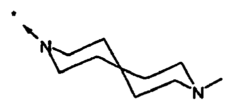 | 61 | 703/705 [M+H]⁺ | 6.2 min (A) |
| **10.30** | 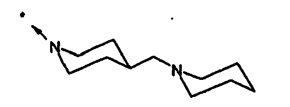 | 48 | 717/719 [M+H]⁺ | 6.4 min (A) |
| **10.31** | 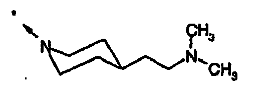 | 31 | 691/693 [M+H]⁺ | 6.2 min (A) |
| **10.32** | 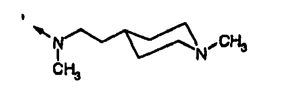 | 31 | 691/693 [M+H]⁺ | 6.3 min (A) |
| **10.33** | 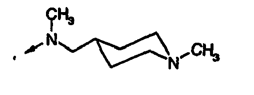 | 19 | 677/679 [M+H]⁺ | 6.3 min (A) |

### Beispiel 10.34

### (S)-2-(4-Amino-3-chlor-5-trifluormethyl-benzyl)-4-[4-(2-oxo-1,2,4,5-tetrahydro-1,3-benzdiazepin-3-yl)-piperidin-1-yl]-1-piperidin-1-yl-butan-1,4-dion

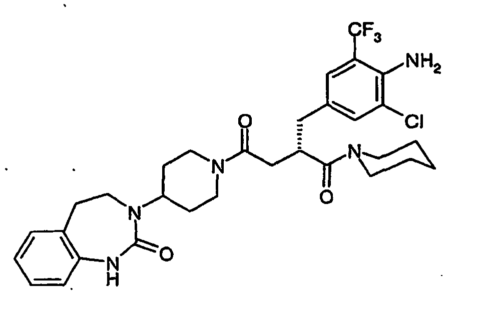

Zu einer Lösung von 100 mg (0.18 mmol) (*S*)-2-(4-Amino-3-chlor-5-trifluormethylbenzyl)-4-oxo-4-[4-(2-oxo-1,2,4,5-tetrahydro-1,3-benzdiazepin-3-yl)-piperidin-1-yl]-butansäure in 2 mL DMF wurden 62 mg (0.19 mmol) TBTU und 34 µL (0.2 mmol) Ethyldiisopropylamin gegeben und diese 30 min bei RT gerührt. Anschliessend wurden 24 µL (0.24 mmol) Piperidin zugegeben und das Reaktionsgemisch 64 h bei RT gerührt. Die Reaktionslösung wurde direkt chromatographisch via HPLC gereinigt.

| | |
|---|---|
| Ausbeute: | 54 mg (48% der Theorie) |
| ESI-MS: | (M+H)⁺ = 620/622 (Cl) |
| Retentionszeit (HPLC): | 8.4 min (Methode A) |

Analog wurden folgende Verbindungen aus jeweils 100 mg (*S*)-2-(4-Amino-3-chlor-5-trifluormethyl-benzyl)-4-oxo-4-[4-(2-oxo-1,2,4,5-tetrahydro-1,3-benzdiazepin-3-yl)-piperidin-1-yl]-butansäure und der entsprechenden Menge an Amin hergestellt:

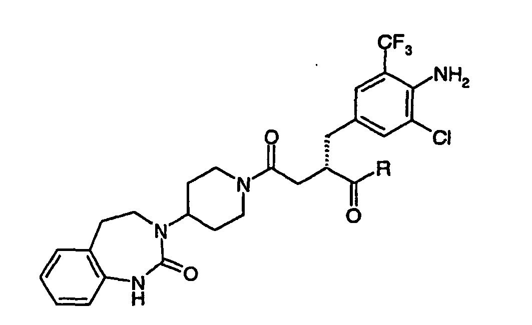

| **Beispiel** | **R** | **Ausbeute (%)** | **Massenspektrum** | **Retentionszeit HPLC (Methode)** |
|---|---|---|---|---|
| **10.35** | 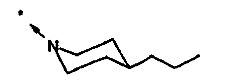 | 46 | 662/664 [M+H]⁺ | 9.6 min (A) |
| **10.36** | 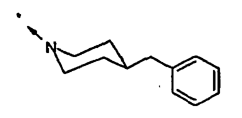 | 47 | 710/712 [M+H]⁺ | 9.6 min (A) |
| **10.37** | 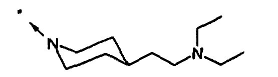 | 23 | 719/721 [M+H]⁺ | 6.4 min (A) |
| **10.38** | 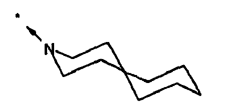 | 53 | 688/690 [M+H]⁺ | 9.9 min (A) |
| **10.39** | 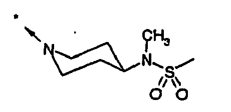 | 51 | 727/729 [M+H]⁺ | 7.7 min (A) |
| **10.40** | 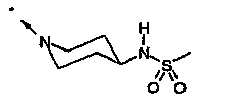 | 50 | 713/715 [M+H]⁺ | 7.4 min (A) |
| **10.41** | 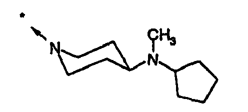 | 39 | 717/719 [M+H]⁺ | 6.6 min (A) |
| **10.42** | 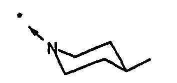 | 56 | 634/636 [M+H]⁺ | 8.7 min (A) |
| **10.43** | 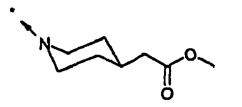 | 48 | 692/694 [M+H]⁺ | 8.2 min (A) |
| **10.44** | 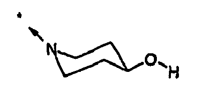 | 52 | 636/638 [M+H]⁺ | 7.0 min (A) |
| **10.45** | 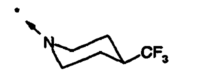 | 58 | 688/690 [M+H]⁺ | 8.7 min (A) |
| **10.46** | 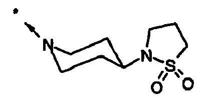 | 50 | 739/741 [M+H]⁺ | 7.6 min (A) |
| **10.47** | 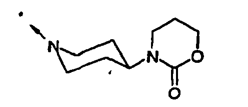 | 41 | 719/721 [M+H]⁺ | 7.2 min (A) |
| **10.48** | 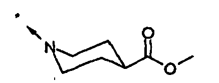 | 23 | 678/680 [M+H]⁺ | 8.0 min (A) |
| **10.49** | 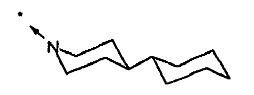 | 24 | 702/704 [M+H]⁺ | 10.5 min (A) |
| **10.50** | 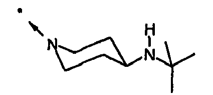 | 32 | 691/693 [M+H]⁺ | 6.6 min (A) |
| **10.51** | 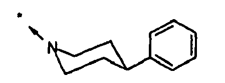 | 44 | 696/698 [M+H]⁺ | 9.2 min (A) |
| **10.52** | 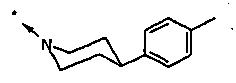 | 44 | 710/712 [M+H]⁺ | 9.6 min (A) |
| **10.53** | 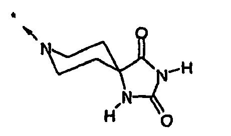 | 43 | 704/706 [M+H]⁺ | 7.0 min (A) |
| **10.54** | 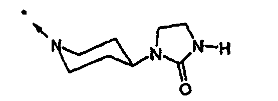 | 51 | 704/706 [M+H]⁺ | 7.0 min (A) |
| **10.55** | 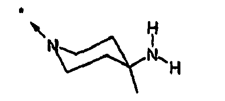 | 54 | 649/651 [M+H]⁺ | 6.2 min (A) |
| **10.56** | 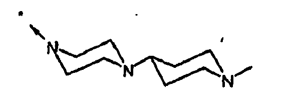 | 68 | 719/721 [M+H]⁺ | 5.4 min (A) |

### Beispiel 10.57

### (S)-2-(4-Amino-3-chlor-5-trifluormethyl-benzyl)-1-(2-amino-4,5,7,8-tetrahydrothiazolo[4,5-d]azepin-6-yl)-4-[4-(2-oxo-1,2,4,5-tetrahydro-1,3-benzdiazepin-3-yl)-piperidin-1-yl]-butan-1,4-dion

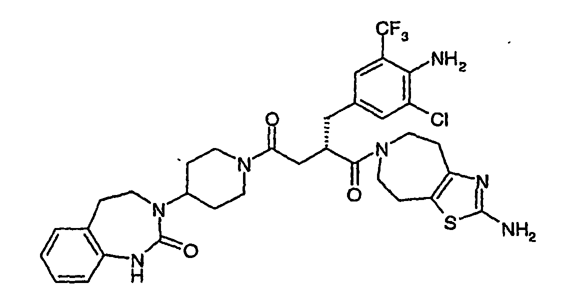

Eine Lösung von 300 mg (0.54 mmol) (*S*)-2-(4-Amino-3-chlor-5-trifluormethyl-benzyl)-4-oxo-4-[4-(2-oxo-1,2,4,5-tetrahydro-1,3-benzdiazepin-3-yl)-piperidin-1-yl]-butansäure und 190 mg (0.59 mmol) TBTU in 0.4 mL (2.27 mmol) Ethyldiisopropylamin und 15 mL DMF wurde 1 h bei RT gerührt. Dann erfolgte die Zugabe von 160 mg (0.64 mmol) 5,6,7,8-Tetrahydro-4*H*-thiazolo[4,5-*d*]azepin-2-ylamin (eingesetzt als Hydrobromid) und die Reaktionslösung wurde weitere 3 h bei RT gerührt. Das Reaktionsgemisch wurde auf 50 mL 15% K₂CO₃-Lösung gegossen, das ausgefallene Produkt abgesaugt und chromatographisch (Kieselgel, Gradient: DCM nach DCM/MeOH/NH₃ 10:9:1) gereinigt.

| | |
|---|---|
| Ausbeute: | 100 mg (26% der Theorie) |
| ESI-MS: | (M+H)⁺ = 704/706 (Cl) |
| Retentionszeit (HPLC): | 6.1 min (Methode A) |

Analog wurden folgende Verbindungen aus jeweils 300 mg (*S*)-2-(4-Amino-3-chlor-5-trifluormethyl-benzyl)-4-oxo-4-[4-(2-oxo-1,2,4,5-tetrahydro-1,3-benzdiazepin-3-yl)-piperidin-1-yl]-butansäure und der entsprechenden Menge an Amin (eingesetzt als freies Amin oder als Amin-Hydrochlorid) hergestellt:

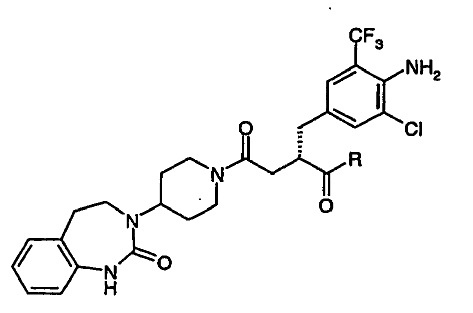

| **Beispiel** | **R** | **Ausbeute (%)** | **Massenspektrum** | **Retentionszeit HPLC (Methode)** |
|---|---|---|---|---|
| **10.58** | 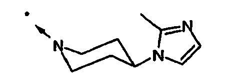 | 40 | 700/702 [M+H]⁺ | 6.4 min (A) |
| **10.59** | 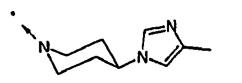 | 42 | 700/702 [M+H]⁺ | 6.4 min (A) |
| **10.60** | 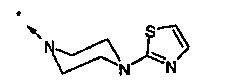 | 50 | 704/706 [M+H]⁺ | 7.5 min (A) |
| **10.61** | 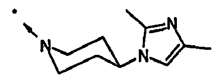 | 31 | 714/716 [M+H]⁺ | 6.5 min (A) |
| **10.62** | 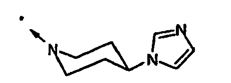 | 62 | 686/688 [M+H]⁺ | 6.3 min (A) |
| **10.63** | 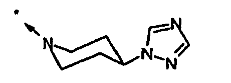 | 62 | 687/689 [M+H]⁺ | 7.2 min (A) |
| **10.64** | 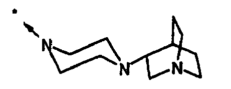 | 15 | 730/732 [M+H]⁺ | 6.0 min (A) |
| **10.65** | 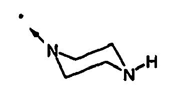 | 18 | 621/623 [M+H]⁺ | 6.1 min (A) |

Die Verbindungen der Beispiele 10.64 und 10.65 konnten beide aus einem Reaktionsansatz isoliert werden, da das eingesetzte 3-Piperazin-1-yl-1-azabicyclo[2.2.2]octan mit Piperazin verunreinigt war.

### Beispiel 10.66

### 4-{(S)-2-(4-Amino-3-chlor-5-trifluormethyl-benzyl)-4-oxo-4-[4-(2-oxo-1,2,4,5-tetrahydro-1,3-benzdiazepin-3-yl)-piperidin-1-yl]-butyryl}-piperazin-1-sulfonsäure (1-methyl-piperidin-4-yl)-amid

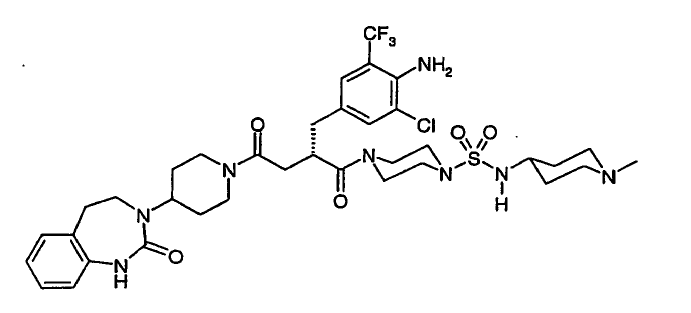

Eine Lösung von 500 mg (0.90 mmol) (*S*)-2-(4-Amino-3-chlor-5-trifluormethyl-benzyl)-4-oxo-4-[4-(2-oxo-1,2,4,5-tetrahydro-1,3-benzdiazepin-3-yl)-piperidin-1 -yl]-butansäure und 320 mg (1.00 mmol) TBTU in 0.2 mL (1.14 mmol) Ethyldiisopropylamin und 50 mL THF wurde 1 h bei RT gerührt. Dann erfolgte die Zugabe von 270 mg (1.03 mmol) Piperazin-1-sulfonsäure-(1-methyl-piperidin-4-yl)-amid und 5 mL DMF. Die Reaktionslösung wurde weiter über Nacht bei RT gerührt. Das Reaktionsgemisch wurde mit 50 mL EtOAc verdünnt, mit 30 mL 15% K₂CO₃-Lösung extrahiert und die organische Phase über Na₂SO₄ getrocknet. Nach Entfernen des Trocken- und Lösungsmittels wurde der Rückstand chromatographisch (Kieselgel, Gradient: DCM nach DCM/MeOH/NH₃ 10:9:1) gereinigt.

| | |
|---|---|
| Ausbeute: | 170 mg (24% der Theorie) |
| ESI-MS: | (M+H)⁺ = 797/799 (Cl) |
| Retentionszeit (HPLC): | 6.4 min (Methode A) |

### Beispiel 10.67

### (S)-2-(4-Amino-3-chlor-5-trifluormethyl-benzyl)-N-(5-amino-pentyl)-4-oxo-4-[4-(2-oxo-1,2,4,5-tetrahydro-1,3-benzdiazepin-3-yl)-piperidin-1=yl]-butyramid

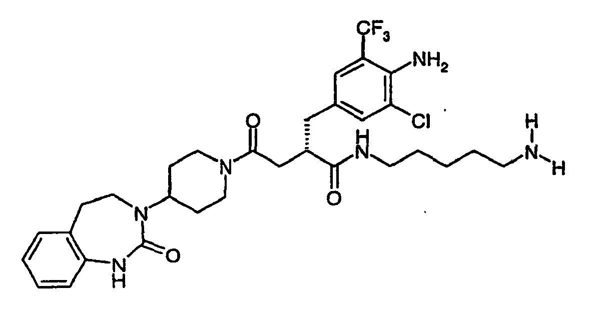

Zu einer Lösung von 260 mg (0.47 mmol) (*S*)-2-(4-Amino-3-chlor-5-trifluormethylbenzyl)-4-oxo-4-[4-(2-oxo-1,2,4,5-tetrahydro-1,3-benzdiazepin-3-yl)-piperidin-1-yl]-butansäure und 161 mg (0.50 mmol) TBTU in 0.43 mL (2.50 mmol) Ethyldiisopropylamin und 10 mL DMF wurden 61 mg (0.3 mmol) (5-Amino-pentyl)-carbaminsäure-*tert*-butylester gegeben und das Reaktionsgemisch über Nacht bei RT gerührt. Man versetzte mit 80 mL 15% K₂CO₃-Lösung, rührte 10 min nach, saugte die ausgefallene Substanz ab; diese wurde dann mit Wasser nach gewaschen und an der Luft getrocknet. Das Rohprodukt wurde in 20 mL DCM gelöst, mit 2 mL TFA versetzt und bei RT 2 h gerührt. Das Reaktionsgemisch wurde mit 15% K₂CO₃-Lösung neutralisiert, die organische Phase abgetrennt und eingeengt. Das so erhaltene Rohprodukt wurde direkt via HPLC gereinigt.

| | |
|---|---|
| Ausbeute: | 110 mg (37% der Theorie) |
| ESI-MS: | (M+H)⁺ = 637/639 (Cl) |
| Retentionszeit (HPLC): | 6.0 min (Methode A) |

Analog wurde folgende Verbindung aus 260 mg (0.47 mmol) (*S*)-2-(4-Amino-3-chlor-5-trifluormethyl-benzyl)-4-oxo-4-[4-(2-oxo-1,2,4,5-tetrahydro-1,3-benzdiazepin-3-yl)-piperidin-1-yl]-butansäure und 142 mg (0.6 mmol) (2-Aminomethyl-benzyl)-carbaminsäure-*tert*-butylester hergestellt:

| **Beispiel** | **R** | **Ausbeute (%)** | **Massenspektrum** | **Retentionszeit HPLC (Methode)** |
|---|---|---|---|---|
| **10.68** | 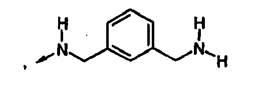 | 47 | 671/673 [M+H]⁺ | 6.4 min (A) |

### Beispiel 10.69

### 1-{(S)-2-(4-Amino-3-chlor-5-trifluormethyl-benzyl)-4-oxo-4-[4-(2-oxo-1,2,4,5-tetrahydro-1,3-benzdiazepin-3-yl)-piperidin-1-yl]-butyryl}-piperidin-4-carbonsäure

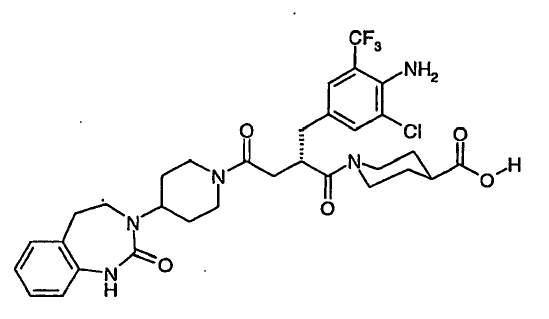

Zu einer Lösung von 15 mg (0.02 mmol) 1-{(*S*)-2-(4-Amino-3-chlor-5-trifluormethyl-benzyl)-4-oxo-4-[4-(2-oxo-1,2,4,5-tetrahydro-1,3-benzdiazepin-3-yl)-piperidin-1-yl]-butyryl}-piperidin-4-carbonsäuremethylester (Beispiel 10.48) in 5 mL THF wurden 2 mg (0.05 mmol) Lithiumhydroxid Hydrat, gelöst in wenig Wasser, zugegeben und das Reaktionsgemisch 3 h bei RT gerührt. Das Lösungsmittel wurde i.vac. abgezogen, der Rückstand in Wasser und Acetonitril aufgenommen und lyophilisiert.

| | |
|---|---|
| Ausbeute: | 14 mg (96% der Theorie) |
| ESI-MS: | (M+H)⁺ = 664/666 (Cl) |
| Retentionszeit (HPLC): | 7.2 min (Methode A) |

Analog wurde folgende Verbindung aus 20 mg (0.03 mmol) (1-{(*S*)-2-(4-Ammo-3-chlor-5-trifluormethyl-benzyl)-4-oxo-4-[4-(2-oxo-1,2,4,5-tetrahydro-1,3-benzdiazepin-3-yl)-piperidin-1-yl]-butyryl}-piperidin-4-yl)-essigsäuremethylester (Beispiel 10.43) hergestellt:

| **Beispiel** | **R** | **Ausbeute (%)** | **Massenspektrum** | **Retentionszeit HPLC (Methode)** |
|---|---|---|---|---|
| **10.70** | 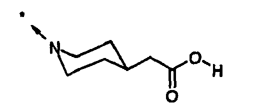 | 96 | 678/680 [M+H]⁺ | 7.3 min (A) |

### Beispiel 10.71

### (S)-2-(4-Amino-3-chlor-5-trifluormethyl-benzyl)-1-[4-(8-methyl-8-aza-bicyclo[3.2.1]oct-3-yl)-piperazin-1-yl]-4-[4-(2-oxo-1,2,4,5-tetrahydro-1,3-benzdiazepin-3-yl)-piperidin-1-yl]-butan-1,4-dion

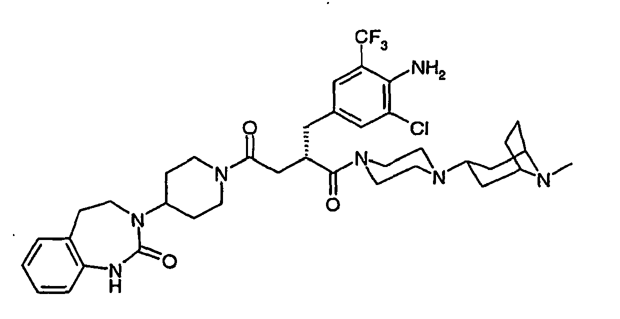

Zu einer Lösung von 1.04 g (1.88 mmol) (*S*)-2-(4-Amino-3-chlor-5-trifluormethyl-benzyl)-4-oxo-4-[4-(2-oxo-1,2,4,5-tetrahydro-1,3-benzdiazepin-3-yl)-piperidin-1-yl]-butansäure, 642 mg (2.0 mmol) TBTU und 1.64 mL (9.6 mmol) Ethyldiisopropylamin in 20 mL DMF wurden 850 mg (2.4 mmol) 8-Methyl-3-piperazin-1-yl-8-aza-bicyclo[3.2.1]octan gegeben und das Reaktionsgemisch über Nacht bei RT gerührt. Die Reaktionslösung wurde mit 15% K₂CO₃-Lösung versetzt, 10 min bei RT nachgerührt, die ausgefallene Substanz abgesaugt, diese mit 50 mL Wasser nach gewaschen und an der Luft getrocknet und chromatographisch (Kieselgel, Gradient: DCM nach DCM/MeOH/NH₃ 10:85:5) gereinigt.

| | |
|---|---|
| Ausbeute: | 1.07 g (77% der Theorie) |
| ESI-MS: . | (M+H)⁺ = 744/746 (CI) |
| Retentionszeit (HPLC): | 5.4 min (Methode A) |

### Beispiel 10.72

### (S)-2-(4-Amino-3-chlor-5-trifluormethyl-benzyl)-4-[4-(2-oxo-1,2,4,5-tetrahydro-1,3-benzdiazepin-3-yl)-piperidin-1-yl]-1-(4-piperazin-1-yl-piperidin-1-yl)-butan-1,4-dion

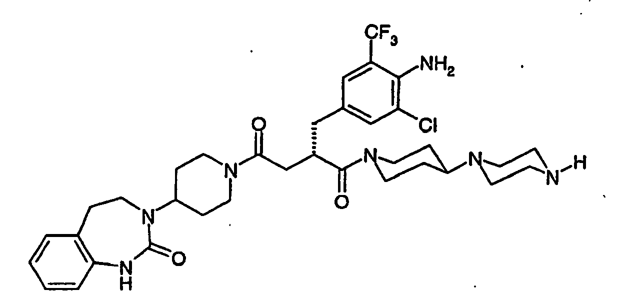

### 10.72a 4-(1 1-{(S)-2-(4-Amino-3-chlor-5-trifluormethyl-benzyl)-4-oxo-4-[4-(2-oxo-1,2,4,5-tetrahydro-1,3-benzdiazepin-3-yl)-piperidin-1-yl]-butyryl}-piperidin-4-yl)-piperazin-1-carbonsäurebenzylester

Zu einer Lösung von 390 mg (0.71 mmol) (*S*)-2-(4-Amino-3-chlor-5-trifluormethyl-benzyl)-4-oxo-4-[4-(2-oxo-1,2,4,5-tetrahydro-1,3-benzdiazepin-3-yl)-piperidin-1-yl]-butansäure in 10 mL DMF wurden 241 mg (0.75 mmol) TBTU, 0.62 mL (3.6 mmol) Ethyldiisopropylamin und 215 mg (0.71 mmol) 4-Piperidin-4-yl-piperazin-1-carbonsäurebenzylester gegeben und das Reaktionsgemisch über Nacht bei RT gerührt. Die Reaktionslösung wurde auf 80 mL 15% K₂CO₃-Lösung gegossen, 10 min bei RT nachgerührt, die ausgefallene Substanz abgesaugt, diese mit 5 mL Wasser nach gewaschen und über das Wochenende an der Luft getrocknet.

| | |
|---|---|
| Ausbeute: | 580 mg (98% der Theorie) |
| ESI-MS: | (M+H)⁺ = 838/840 (Cl) |

### 10.72b (S)-2-(4-Amino-3-chlor-5-trifluormethyl-benzyl)-4-[4-(2-oxo-1,2,4,5-tetrahydro-1,3-benzdiazepin-3-yl)-piperidin-1-yl]-1-(4-piperazin-1-yl-piperidin-1-yl)-butan-1,4-dion

Zu einer Lösung von 250 mg (0.30 mmol) 4-(1-{(*S*)-2-(4-Amino-3-chlor-5-trifluormethyl-benzyl)-4-oxo-4-[4-(2-oxo-1,2,4,5-tetrahydro-1,3-benzdiazepin-3-yl)-piperidin-1-yl]-butyryl}-piperidin-4-yl)-piperazin-1-carbonsäurebenzylester in 30 mL MeOH wurden 100 mg Raney-Nickel gegeben und das Reaktionsgemisch 5 h bei RT und 50 psi H₂ gerührt. Zur Vervollständigung der Reaktion wurden erneut 100 mg Raney-Nickel zugegeben und weitere 10 h bei RT gerührt. Der Katalysator wurde abgesaugt, das Lösungsmittel i.vac. entfernt und der Rückstand via HPLC gereinigt.

| | |
|---|---|
| Ausbeute: | 88 mg (42% der Theorie) |
| ESI-MS: | (M+H)⁺ = 704/706 (Cl) |
| Retentionszeit (HPLC): | 5.6 min (Methode A) |

### Beispiel 10.73

### (S)-2-(4-Amino-3-chlor-5-trifluormethyl-benzyl)-1-[4-(1H-imidazol-4-yl)-piperidin-1-yl]-4-[4-(2-oxo-1,2,4,5-tetrahydro-1,3-benzdiazepin-3-yl)-piperidin-1-yl]-butan-1,4-dion

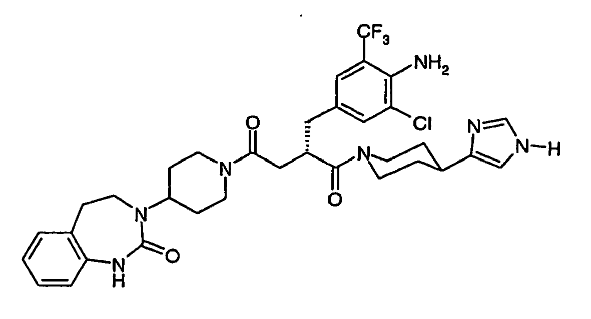

Zu einer Lösung von 650 mg (1.18 mmol) (*S*)-2-(4-Amino-3-chlor-5-trifluormethyl-benzyl)-4-oxo-4-[4-(2-oxo-1,2,4,5-tetrahydro-1,3-benzdiazepin-3-yl)-piperidin-1-yl]-butansäure in 20 mL DMF wurden 400 mg (1.25 mmol) TBTU und 0.65 mL (3.73 mmol) Ethyldiisopropylamin gegeben und diese 30 min bei RT gerührt. Anschliessend wurden 340 mg (1.52 mmol) 4-(1*H*-Imidazol-4-yl)-piperidin (eingesetzt als Bis-Hydrochlorid) zugegeben und das Reaktionsgemisch über Nacht bei RT gerührt. Man engte i.vac. ein, versetzte mit 30 mL 15% K₂CO₃-Lösung, extrahierte zweimal mit jeweils 15 mL DCM und trocknete die organische Phase mit MgSO₄. Nach Entfernen des Trocken- und Lösungsmittels wurde der Rückstand chromatographisch (Kieselgel, Gradient: DCM nach DCM/MeOH/NH₃ 20:75:5) gereinigt.

| | |
|---|---|
| Ausbeute: | 460 mg (57% der Theorie) |
| ESI-MS: | (M+H)⁺ = 686/688 (Cl) |
| R_{f} = | 0.35 (Kieselgel, DCM/Cyc/MeOH/NH₃ 70:15:15:2) |

### Beispiel 10.74

### (S)-2-(4-Amino-3-chlor-5-trifluormethyl-benzyl)-4-[4-(2-oxo-1,2,4,5-tetrahydro-1,3-benzdiazepin-3-yl)-piperidin-1-yl]-1-{4-[4-(2,2,2-trifluor-acetyl)-phenyl]-piperazin-1-yl}-butan-1,4-dion

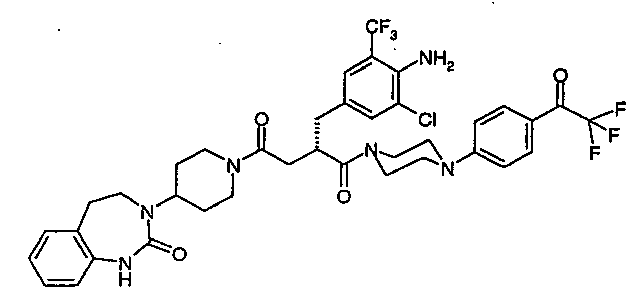

### 10.74a 4-(4-Brom-phenyl)-piperazin-1-carbonsäure-tert-butylester

Zu einer Suspension von 10.0 g (36 mmol) 4-(4-Brom-phenyl)-piperazin (eingesetzt als Hydrochlorid) und 15 mL (108 mmol) Triethylamin in 150 mL THF wurden portionenweise Boc-Anhydrid gegeben und das Reaktionsgemisch 3 h auf 60°C erhitzt. Nach dem Abkühlen wurde auf Wasser gegossen, der Niederschlag mit EtOAc extrahiert, die organische Phase mit Wasser gewaschen und über Na₂SO₄ getrocknet. Nach Entfernen des Trocken- und Lösungsmittels erhielt man das gewünschte Produkt.

| | |
|---|---|
| Ausbeute: | 12.0 g (98% der Theorie) |
| R_{f} = | 0.6 (Kieselgel, Cyc/EtOAc 2:1) |

### 10.74b 4-[4-(2,2,2-Trifluor-acetyl)-phenyl]-piperazin-1-carbonsäure-tert-butylester

Unter Stickstoffatmosphäre wurde zu einer auf -78°C gekühlten Lösung von 2.06 mL (3.3 mmol) *n*-Butyllithium (1.6 M in *n*-Hexan) in 40 mL trockenem THF eine Lösung von 1.02 g (3.0 mmol) 4-(4-Brom-phenyl)-piperazin-1-carbonsäure-tert-butylester in 20 mL THF langsam zugetropft und diese 15 min bei dieser Temperatur nachgerührt. Dann erfolgte langsam die tropfenweise Zugabe einer Lösung von 0.43 mL (3.0 mmol) N,N-Diethyl-2,2,2-trifluoracetamid in 10 mL THF. Nach beendeter Zugabe wurde 2 h bei -78°C gehalten, dann das Reaktionsgemisch auf 100 mL Wasser gegossen, zweimal mit jeweils 50 mL EtOAc extrahiert, die organische Phase über Na₂SO₄ abgesaugt, i.vac. eingeengt und chromatographisch (Kieselgel, Cyc/EtOAc 3:1) gereinigt.

| | |
|---|---|
| Ausbeute: | 267 mg (25% der Theorie) |
| EI: | (M)⁺ = 358 |
| R_{f} = | 0.37 (Kieselgel, Cyc/EtOAc 3:1) |

### 10.74c 2,2,2-Trifluor-1-(4-piperazin-1-yl-phenyl)-ethanon

Zu einer auf 0°C gekühlten Lösung von 267 mg (0.75 mmol) 4-[4-(2,2,2-Trifluoracetyl)-phenyl]-piperazin-1-carbonsäure-*tert*-butylester in 30 mL DCM wurden 2.0 mL TFA gegeben und das Reaktionsgemisch 24 h nachgerührt, wobei sich dieses auf RT erwärmte. Man engte i.vac. ein; das Rohprodukt wurde ohne Reinigung weiter umgesetzt.

| | |
|---|---|
| ESI-MS: | (M+H)⁺ = 259 |

### 10.74d (S)-2-(4-Amino-3-chlor-5-trifluormethyl-benzyl)-4-[4-(2-oxo-1,2,4,5-tetrahydro-1,3-benzdiazepin-3-yl)-piperidin-1-yl]-1-{4-[4-(2,2,2-trifluoracetyl)-phenyl]-piperazin-1-yl}-butan-1,4-dion

Zu einer Lösung von 234 mg (0.42 mmol) (*S*)-2-(4-Amino-3-chlor-5-trifluormethyl-benzyl)-4-oxo-4-[4-(2-oxo-1,2,4,5-tetrahydro-1,3-benzdiazepin-3-yl)-piperidin-1-yl]-butansäure, 136 mg (0.42 mmol) TBTU, 57 mg (0.42 mmol) HOBt und 0.14 mL (1.0 mmol) Triethylamin in 20 mL THF und 2 mL DMF wurde das in 10.74c erhaltene Rohprodukt gegeben und das Reaktionsgemisch 2 h bei RT gerührt. Die Reaktionslösung wurde mit halbgesättigter NaHCO₃-Lösung versetzt und mit 30 mL EtOAc extrahiert. Die organische Phase wurde über Na₂SO₄ abgesaugt, das Filtrat i.vac. eingeengt und der Rückstand chromatographisch (Kieselgel, EtOAc/MeOH 95:5) gereinigt.

| | |
|---|---|
| Ausbeute: | 246 mg (73% der Theorie) |
| ESI-MS: | (M+H)⁺ = 793/795 (Cl) |
| R_{f} = | 0.27 (Kieselgel, EtOAc) |

### Beispiel 10.75

### (S)-2-(4-Amino-3-chlor-5-trifluormethyl-benzyl)-1-[4-(4-methyl-piperazin-1-yl)-piperidin-1-yl]-4-[4-(2-oxo-1,4-dihydro-2H-chinazolin-3-yl)-piperidin-1-yl]-butan-1,4-dion

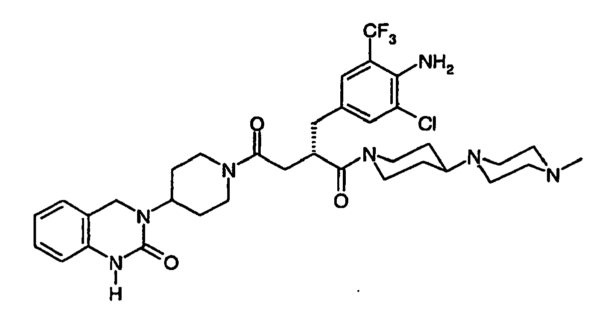

### 10.75a (S)-2-(4-Amino-3-chlor-5-trifluormethyl-benzyl)-4-oxo-4-[4-(2-oxo-1,4-dihydro-2H-chinazolin-3-yl)-piperidin-1-yl]-butansäuremethylester

Eine Lösung von 3.0 g (8.83 mmol) (*S*)-2-(4-Amino-3-chlor-5-trifluormethyl-benzyl)-bernsteinsäure-1-methylester und 3.05 g (9.5 mmol) TBTU, 1.28 g (9.47 mmol) HOBt in 1.7 mL (9.76 mmol) Ethyldiisopropylamin und 100 mL DMF wurde 1 h bei RT gerührt. Dann erfolgte die Zugabe von 2.2 g (9.51 mmol) 3-Piperidin-4-yl-3,4-dihydro-1*H*-chinazolin-2-on und die Reaktionslösung wurde über Nacht bei RT gerührt. Das Reaktionsgemisch wurde i.vac. eingeengt, der Rückstand in DCM aufgenommen, mit 10% Zitronensäure-Lösung und mit 15% K₂CO₃-Lösung gewaschen und über Na₂SO₄ getrocknet. Das Trockenmittel wurde durch Filtration über Aktivkohle entfernt; nach Entfernen des Lösungsmittels erhielt man das gewünschte Produkt.

| | |
|---|---|
| Ausbeute: | 4.8 g (98% der Theorie) |
| ESI-MS: | (M+H)⁺ = 553/555 (Cl) |
| R_{f} = | 0.71 (Kieselgel, DCM/CyclMeOH/NH₃ 70:15:15:2) |

### 10.75b (S)-2-(4-Amino-3-chlor-5-trifluormethyl-benzyl)-4-oxo-4-[4-(2-oxo-1,4-dihydro-2H-chinazolin-3-yl)-piperidin-1-yl]-butansäure

Zu einer Lösung von 4.8 g (8.68 mmol) (*S*)-2-(4-Amino-3-chlor-5-trifluormethyl-benzyl)-4-oxo-4-[4-(2-oxo-1,4-dihydro-2*H*-chinazolin-3-yl)-piperidin-1-yl]-butansäuremethylester in 28 mL THF wurden eine Lösung von 558 mg (13.02 mmol) Lithiumhydroxid Hydrat in 12 mL Wasser zugegeben und das Reaktionsgemisch 7 h bei RT gerührt. Man engte i.vac. ein, versetzte mit 100 mL Wasser, säuerte mit 1 M HCl an und saugte den entstandenen Niederschlag ab. Der Rückstand wurde in EtOAc gelöst, mit 15% K₂CO₃-Lösung extrahiert und die wässrige Phase erneut mit 1 M HCl angesäuert. Der entstandene Niederschlag wurde abgesaugt und getrocknet.

| | |
|---|---|
| Ausbeute: | 4.2 g (90% der Theorie) |
| ESI-MS: | (M+H)⁺ = 539/541 (CI) |
| R_{f} = | 0.09 (Kieselgel, DCM/Cyc/MeOH/NH₃ 70:15:15:2) |

### 10.75c (S)-2-(4-Amino-3-chlor-5-trifluormethyl-benzyl)-1-[4-(4-methyl-piperazin-1-yl)-piperidin-1-yl]-4-[4-(2-oxo-1,4-dihydro-2H-chinazolin-3-yl)-piperidin-1-yl]-butan-1,4-dion

Analog zu 10.75a wurde aus 500 mg (0.93 mmol) (*S*)-2-(4-Amino-3-chlor-5-trifluormethyl-benzyl)-4-oxo-4-[4-(2-oxo-1,4-dihydro-2*H*-chinazolin-3-yl)-piperidin-1-yl]-butansäure und 180 mg (0.98 mmol) 1-Methyl-4-piperidin-4-yl-piperazin das Rohprodukt erhalten. Nach der beschriebenen Aufarbeitung wurde dieses zuerst chromatographisch (Kieselgel, Gradient: DCM nach DCM/MeOH/NH₃ 70:27:3) und dann noch via HPLC gereinigt.

| | |
|---|---|
| Ausbeute: | 120 mg (18% der Theorie) |
| ESI-MS: | (M+H)⁺ = 704/706 (Cl) |
| R_{f} = | 0.43 (Kieselgel, DCM/Cyc/MeOH/NH₃ 70:15:15:2) |
| Retentionszeit (HPLC): | 5.6 min (Methode A) |

### Beispiel 10.76

### (S)-2-(4-Amino-3-chlor-5-trifluormethyl-benzyl)-1-[4-(4-methyl-piperazin-1-yl)-piperidin-1-yl]-4-[4-(5-oxo-3-phenyl-4,5-dihydro-1,2,4-triazol-1-yl)-piperidin-1-yl]-butan-1,4-dion

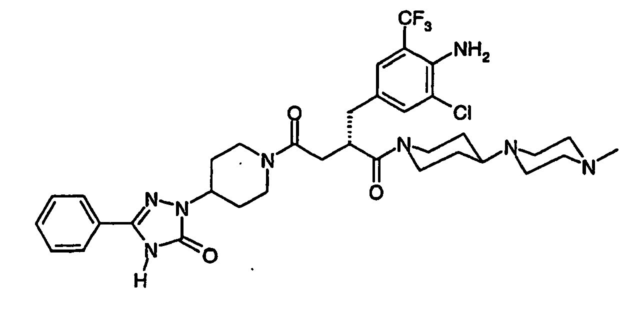

### 10.76a (S)-2-(4-Amino-3-chlor-5-trifluormethyl-benzyl)-4-oxo-4-[4-(5-oxo-3-phenyl-4,5-dihydro-1,2,4-triazol-1-yl)-piperidin-1-yl]-butansäuremethylester

Analog zu 10.75a wurde aus 3.0 g (8.31 mmol) (*S*)-2-(4-Amino-3-chfor-5-trifluormethyl-benzyl)-bernsteinsäure-1-methylester und 3.55 g (9.45 mmol) 5-Phenyl-2-piperidin-4-yl-2,4-dihydro-1,2,4-triazol-3-on das gewünschte Produkt erhalten.

| | |
|---|---|
| Ausbeute: | 2.5 g (50% der Theorie) |
| ESI-MS: | (M+H)⁺ = 566/568 (Cl) |
| R_{f} = | 0.67 (Kieselgel, DCM/Cyc/MeOH/NH₃ 70:15:15:2) |

### 10.76b (S)-2-(4-Amino-3-chlor-5-trifluormethyl-benzyl)-4-oxo-4-[4-(5-oxo-3-phenyl-4,5-dihydro-1,2,4-triazol-1-yl)-piperidin-1-yl]-butansäure

Analog zu 10.75b wurde aus 2.5 g (4.42 mmol) (*S*)-2-(4-Amino-3-chlor-5-trifluormethyl-benzyl)-4-oxo-4-[4-(5-oxo-3-phenyl-4,5-dihydro-1,2,4-triazol-1-yl)-piperidin-1-yl]-butansäuremethylester das gewünschte Produkt erhalten.

| | |
|---|---|
| Ausbeute: | 2.5 g (50% der Theorie) |
| ESI-MS: | (M+H)⁺ = 552/554 (Cl) |
| R_{f} = | 0.14 (Kieselgel, DCM/Cyc/MeOH/NH₃ 70:15:15:2) |

### 10.76c (S)-2-(4-Amino-3-chlor-5-trifluormethyl-benzyl)-1-[4-(4-methyl-piperazin-1-yl)-piperidin-1-yl]-4-[4-(5-oxo-3-phenyl-4,5-dihydro-1,2,4-triazol-1-yl)-piperidin-1-yl]-butan-1,4-dion

Analog zu 10.75a wurde aus 500 mg (0.91 mmol) (*S*)-2-(4-Amino-3-chlor-5-trifluormethyl-benzyl)-4-oxo-4-[4-(5-oxo-3-phenyl-4,5-dihydro-1,2,4-triazol-1-yl)-piperidin-1-yl]-butansäure und 180 mg (0.98 mmol) 1-Methyl-4-piperidin-4-yl-piperazin das Rohprodukt erhalten. Nach der beschriebenen Aufarbeitung wurde dieses chromatographisch (Kieselgel, Gradient: DCM nach DCM/MeOH/NH₃ 70:27:3) gereinigt.

| | |
|---|---|
| Ausbeute: | 350 mg (54% der Theorie) |
| ESI-MS: | (M+H)⁺ = 717/719 (Cl) |
| R_{f} = | 0.44 (Kieselgel, DCM/Cyc/MeOH/NH₃ 70:15:15:2) |
| Retentionszeit (HPLC): | 5.6 min (Methode A) |

### Beispiel 10.77

### (S)-2-(4-Amino-3-chlor-5-trifluormethyl-benzyl)-1-[4-(4-methyl-piperazin-1-yl)-pipendin-1-yl]-4-[4-(2-oxo-1,2-dihydro-imidazo[4,5-c]chinolin-3-yl)-piperidin-1-yl]-butan-1,4-dion

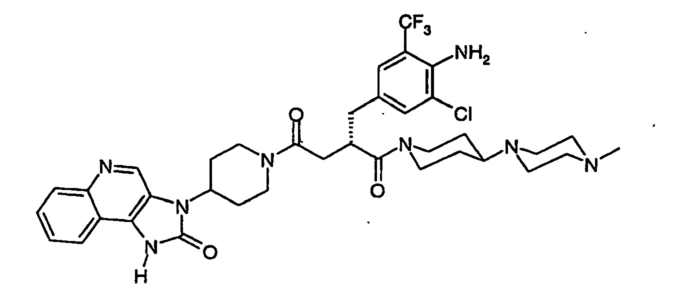

### 10.77a (S)-2-(4-Amino-3-chlor-5-trifluormethyl-benzyl)-4-oxo-4-[4-(2-oxo-1,2-dihydro-imidazo[4,5-c]chinolin-3-yl)-piperidin-1-yl]-butansäuremethylester

Analog zu 10.75a wurde aus 3.0 g (8.31 mmol) (*S*)-2-(4-Amino-3-chlor-5-trifluormethyl-benzyl)-bernsteinsäure-1-methylester und 2.55 g (9.40 mmol) 3-Piperidin-4-yl-1,3-dihydro-imidazo[4,5-c]chinoliri-2-on das gewünschte Produkt erhalten.

| | |
|---|---|
| Ausbeute: | 5.2 g (100% der Theorie) |
| ESI-MS: | (M+H)⁺ = 590/592 (Cl) |
| R_{f} = | 0.66 (Kieselgel, DCM/Cyc/MeOH/NH₃ 70:15:15:2) |

### 10.77b (S)-2-(4-Amino-3-chlor-5-trifluormethyl-benzyl)-4-oxo-4-[4-(2-oxo-1,2-dihydro-imidazo[4,5-c]chinolin-3-yl)-piperidin-1-yl]-butansäure

Analog zu 10.75b wurde aus 5.2 g (8.81 mmol) (*S*)-2-(4-Amino-3-chlor-5-trifluormethyl-benzyl)-4-oxo-4-[4-(2-oxo-1,2-dihydro-imidazo[4,5-*c*]chinolin-3-yl)-piperidin-1-yl]-butansäuremethylester das gewünschte Produkt erhalten.

| | |
|---|---|
| Ausbeute: | 2.75 g (54% der Theorie) |
| ESI-MS: | (M+H)⁺ = 576/578 (Cl) |
| R_{f} = | 0.09 (Kieselgel, DCM/Cyc/MeOH/NH₃ 70:15:15:2) |

### 10.77c (S)-2-(4-Amino-3-chlor-5-trifluormethyl-benzyl)-1-[4-(4-methyl-piperazin-1-yl)-piperidin-1-yl]-4-[4-(2-oxo-1,2-dihydro-imidazo[4,5-c]chinolin-3-yl)-piperidin-1-yl]-butan-1,4-dion

Analog zu 10.75a wurde aus 500 mg (0.87 mmol) (*S*)-2-(4-Amino-3-chlor-5-trifluormethyl-benzyl)-4-oxo-4-[4-(2-oxo-1,2-dihydro-imidazo[4,5-*c*]chinolin-3-yl)-piperidin-1-yl]-butansäure und 170 mg (0.93 mmol) 1-Methyl-4-piperidin-4-yl-piperazin das Rohprodukt erhalten. Nach der beschriebenen Aufarbeitung wurde der Rückstand mit Diisopropylether versetzt und im Ultraschallbad behandelt, das Produkt abgesaugt und getrocknet.

| | |
|---|---|
| Ausbeute: | 520 mg (81 % der Theorie) |
| ESI-MS: | (M+H)⁺ = 741/743 (Cl) |
| R_{f} = | 0.40 (Kieselgel, DCM/Cyc/MeOH/NH₃ 70:15:15:2) |
| Retentionszeit (HPLC): | 4.5 min (Methode A) |

### Beispiel 10.78

### (S)-2-(4-Amino-3-chlor-5-trifluormethyl-benzyl)-1-[4-(4-methyl-piperazin-1-yl)-piperidin-1-yl]-4-[4-(2-oxo-1,2-dihydro-4H-thieno[3,4-d]pyrimidin-3-yl)-piperidin-1-yl]-butan-1,4-dion

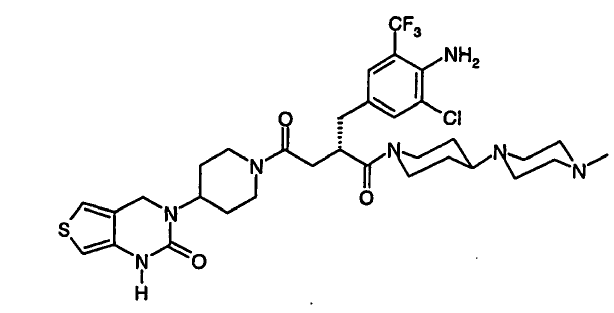

### 10.78a (S)-2-(4-Amino-3-chlor-5-trifluormethyl-benzyl)-4-oxo-4-[4-(2-oxo-1,2-dihydro-4H-thieno[3,4-d]pyrimidin-3-yl)-piperidin-1-yl]-butansäuremethylester

Analog zu 10.75a wurde aus 3.0 g (8.31 mmol) (*S*)-2-(4-Amino-3-chlor-5-trifluormethyl-benzyl)-bernsteinsäure-1-methylester und 3.34 g (9.51 mmol) 3-Piperidin-4-yl-3,4-dihydro-1*H*-thieno[3,4-*d*]pyrimidin-2-on das gewünschte Produkt erhalten.

| | |
|---|---|
| Ausbeute: | 2.2 g (45% der Theorie) |
| ESI-MS: | (M+H)⁺ = 559/561 (Cl) |
| R_{f} = | 0.56 (Kieselgel, DCM/Cyc/MeOH/NH₃ 70:15:15:2) |

### 10.78b (S)-2-(4-Amino-3-chlor-5-trifluormethyl-benzyl)-4-oxo-4-[4-(2-oxo-1,2-dihydro-4H-thieno[3,4-d]pyrimidin-3-yl)-piperidin-1-yl]-butansäure

Analog zu 10.75b wurde aus 2.2 g (3.94 mmol) (S)-2-(4-Amino-3-chlor-5-trifluormethyl-benzyl)-4-oxo-4-[4-(2-oxo-1,2-dihydro-4*H*-thieno[3,4-*d*]pyrimidin-3-yl)-piperidin-1-yl]-butansäuremethylester das gewünschte Produkt erhalten.

| | |
|---|---|
| Ausbeute: | 1.10 g (51 % der Theorie) |
| ESI-MS: | (M+H)⁺ = 545/547 (CI) |
| R_{f} = | 0.24 (Kieselgel, DCM/Cyc/MeOH/NH₃ 70:15:15:2) |

### 10.78c (S)-2-(4-Amino-3-chlor-5-trifluormethyl-benzyl)-1-[4-(4-methyl-piperazin-1-yl)-piperidin-1-yl]-4-[4-(2-oxo-1,2-dihydro-4H-thieno[3,4-d]pyrimidin-3-yl)-piperidin-1-yl]-butan-1,4-dion

Analog zu 10.75a wurde aus 500 mg (0.92 mmol) (*S*)-2-(4-Amino-3-chlor-5-trifluormethyl-benzyl)-4-oxo-4-[4-(2-oxo-1,2-dihydro-4*H*-thieno[3,4-*d*]pyrimidin-3-yl)-piperidin-1-yl]-butansäure und 180 mg (0.98 mmol) 1-Methyl-4-piperidin-4-yl-piperazin das Rohprodukt erhalten. Nach der beschriebenen Aufarbeitung wurde dieses chromatographisch (Kieselgel, Gradient: DCM nach DCNUMeOH/NH₃ 70:27:3) gereinigt.

| | |
|---|---|
| Ausbeute: | 100 mg (81 % der Theorie) |
| ESI-MS: | (M+H)⁺ =710/712 (CI) |
| R_{f} = | 0.43 (Kieselgel, DCM/Cyc/MeOH/NH₃ 70:15:15:2) |
| Retentionszeit (HPLC): | 5.5 min (Methode A) |

### Beispiel 10.79

### (S)-2-(4-Amino-3-chlor-5-trifluormethyl-benzyl)-1-[4-(5-methyl-2,5-diaza-bicyclo[2.2.1]hept-2-yl)-piperidin-1-yl]-4-[4-(2-oxo-1,2,4,5-tetrahydro-1,3-benzdiazepin-3-yl)-piperidin-1-yl]-butan-1,4-dion

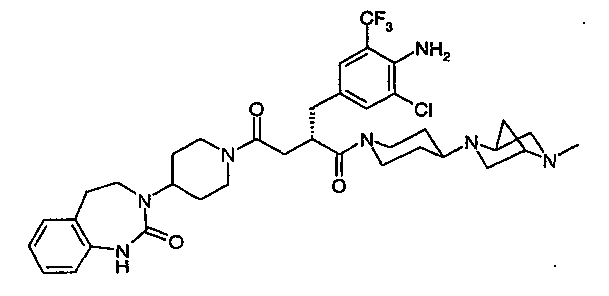

Hergestellt analog Beispiel 10.26 aus 100 mg (0.18 mmol) (*S*)-2-(4-Amino-3-chlor-5-trifluormethyl-benzyl)-4-oxo-4-[4-(2-oxo-1,2,4,5-tetrahydro-1,3-benzdiazepin-3-yl)-piperidin-1-yl]-butansäure und 39.1 mg (0.2 mmol) 2-Methyl-5-piperidin-4-yl-2,5-diaza-bicyclo[2.2.1]heptan unter Verwendung von Triethylamin als Base.

| | |
|---|---|
| Ausbeute: | 83 mg (63% der Theorie) |
| ESI-MS: | (M+H)⁺ = 730/732 (Cl) |
| Retentionszeit (HPLC): | 5.6 min (Methode A) |

Analog den beschriebenen Verfahren können folgende Verbindungen hergestellt werden:

| **Beispiel** | **Struktur** |
|---|---|
| **10.80** | 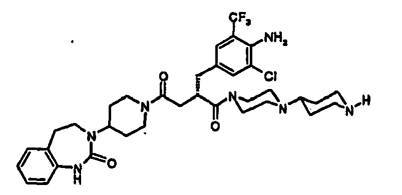 |
| **10.81** | 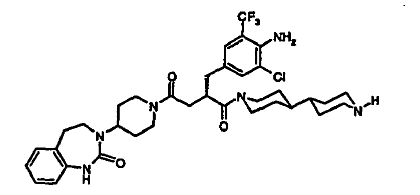 |

### Beispiel 11

### (S)-2-(4-Amino-3,5-bis-trifluormethyl-benzyl)-1-[4-(4-methyl-perhydro-1,4-diazepin-1-yl)-piperidin-1-yl]-4-[4-(2-oxo-1,2,4,5-tetrahydro-1,3-benzdiazepin-3-yl)-plperidin-1-yl]-butan-1,4-dion

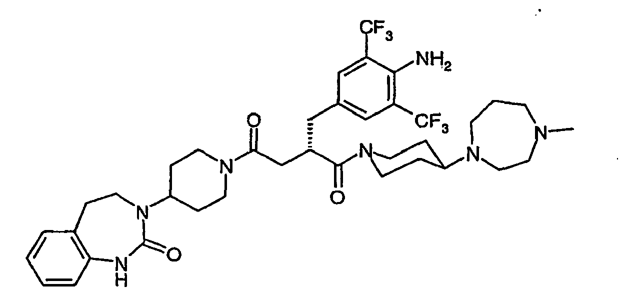

### 11a 4-Amino-3,5-bis-trifluormethyl-benzaldehyd

Eine Lösung von 5 g (19.67 mmol) 4-Amino-3,5-bis-trifluormethyl-benzonitril in 30 mL Ameisensäure wurde in 10 gleichen Portionen in Druckgefäßen 20 h bei 110°C geschüttelt. Die einzelnen Portionen wurden vereinigt, filtriert, mit Ameisensäure nach gewaschen und i.vac. eingeengt. Der Rückstand wurde chromatographisch (Kieselgel, PE/EtOAc 9:1) gereinigt.

| | |
|---|---|
| Ausbeute: | 3.8 g (75% der Theorie) |
| ESI-MS: | (M-H)⁻ = 256 |

### 11b 2-[1-(4-Amino-3,5-bis-trifluormethyl-phenyl)-meth-(E)-yliden]-bernsteinsäure-1-methylester

Zu einer Lösung von 4.2 g (16.33 mmol) 4-Amino-3,5-bis-trifluormethyl-benzaldehyd in 80 mL THF wurden 12.79 g (32.6 mmol) 2-(Triphenyl-λ⁵-phosphanyliden)-bernsteinsäure-1-methylester gegeben und das Reaktionsgemisch 120 h auf 40°C erwärmt. Man engte i.vac. ein, versetzte den Rückstand mit Wasser und EtOAc, trennte die organische Phase ab, wusch diese mit Wasser und extrahierte dreimal mit jeweils 80 mL 5% K₂CO₃-Lösung. Die vereinigten wässrigen Phasen wurden mit konz. HCI angesäuert, der ölige Niederschlag zweimal mit jeweils 100 mL EtOAc extrahiert und über Na₂SO₄ getrocknet. Nach Entfernen des Trocken- und Lösungsmittels erhielt man das gewünschte Produkt.

| | |
|---|---|
| Ausbeute: | 5.9 g (97% der Theorie) |
| EI: | (M)⁺ = 371 |

### 11c (S)-2-(4-Amino-3,5-bis-trifluormethyl-benzyl)-bernsteinsäure-1-methylester

Hergestellt analog Beispiel 9g aus 5.9 g 2-[1-(4-Amino-3,5-bis-trifluormethyl-phenyl)-meth-(E)-yliden]-bernsteinsäure-1-methylester.

| | |
|---|---|
| Ausbeute: | 5.9 g (97% der Theorie) |
| ESI-MS: | (M+H)⁺ = 374 |

### 11 d (S)-2-(4-Amino-3,5-bis-trifluormethyl-benzyl)-4-oxo-4-[4-(2-oxo-1,2,4,5-tetrahydro-1,3-benzdiazepin-3-yl)-piperidin-1-yl]-butansäuremethylester

Hergestellt analog Beispiel 9h aus 4.40 g (11.79 mmol) (*S*)-2-(4-Amino-3,5-bis-trifluormethyl-benzyl)-bernsteinsäure-1-methylester und 2.89 g (11.78 mmol) 3-Piperidin-4-yl-1,3,4,5-tetrahydro-1,3-benzdiazepin-2-on.

| | |
|---|---|
| Ausbeute: | 6.75 g (95% der Theorie) |
| ESI-MS: | (M+H)⁺ = 601 |
| R_{f} = | 0.13 (Kieselgel, PE/EtOAc 1:1) |

### 11e (S)-2-(4-Amino-3,5-bis-trifluormethyl-benzyl)-4-oxo-4-[4-(2-oxo-1,2,4,5-tetrahydro-1,3-benzdiazepin-3-yl)-piperidin-1-yl]-butansäure

Zu einer Lösung von 6.7 g (11.16 mmol) (*S*)-2-(4-Amino-3,5-bis-trifluormethyl-benzyl)-4-oxo-4-[4-(2-oxo-1,2,4,5-tetrahydro-1,3-benzdiazepin-3-yl)-piperidin-1-yl]-butansäuremethylester in 50 mL THF wurden bei RT eine Lösung von 0.72 g (16.75 mmol) Lithiumhydroxid Hydrat in 30 mL Wasser gegeben und das Reaktionsgemisch 5 h bei RT gerührt. Das THF wurde i.vac. abgezogen, die wässrige Lösung auf 10°C abgekühlt und mit konz. HCl auf pH 1 eingestellt, wobei das Produkt ausfiel. Dieses wurde abgesaugt und bei 65°C getrocknet. Die getrocknete Substanz wurde mit 300 mL Diisopropylether versetzt, über Nacht ausgerührt, abgesaugt, mit Diisopropylether nach gewaschen und getrocknet.

| | |
|---|---|
| Ausbeute: | 5.6 g (86% der Theorie) |
| ESI-MS: | (M+H)⁺ = 587 |

### 11f (S)-2-(4-Amino-3,5-bis-trifluormethyl-benzyl)-1-[4-(4-methyl-perhydro-1,4-diazepin-1-yl)-piperidin-1-yl]-4-[4-(2-oxo-1,2,4,5-tetrahydro-1,3-benzdiazepin-3-yl)-piperidin-1-yl]-butan-1,4-dion

Zu einer Lösung von 400 mg (0.68 mmol) (*S*)-2-(4-Amino-3,5-bis-trifluormethyl-benzyl)-4-oxo-4-[4-(2-oxo-1,2,4,5-tetrahydro-1,3-benzdiazepin-3-yl)-piperidin-1-yl]-butansäure, 241 mg (0.75 mmol) TBTU und 0.25 mL (1.8 mmol) Triethylamin in 5 mL DMF wurden 197 mg (1.0 mmol) 1-Methyl-4-piperidin-4-yl-perhydro-1,4-diazepin gegeben und das Reaktionsgemisch über Nacht bei RT gerührt. Die Reaktionslösung wurde langsam auf 150 mL 15% K₂CO₃-Lösung gegossen, das ausgefallene Produkt abgesaugt und an der Luft getrocknet. Das Rohprodukt wurde chromatographisch (Kieselgel, Gradient: DCM nach MeOH/NH₃ 95:5) gereinigt.

| | |
|---|---|
| Ausbeute: | 400 mg (77% der Theorie) |
| ESI-MS: | (M+H)⁺ = 767 |
| R_{f} = | 0.2 (Kieselgel, DCM/MeOH/NH₃ 85:15:1.5) |
| Retentionszeit (HPLC): | 5.6 min (Methode A) |

Analog wurden folgende Verbindungen aus jeweils 400 mg (Beispiel 11.7: 387 mg) (*S*)-2-(4-Amino-3,5-bis-trifluormethyl-benzyl)-4-oxo-4-[4-(2-oxo-1,2,4,5-tetrahydro-1,3-benzdiazepin-3-yl)-piperidin-1-yl]-butansäure und der entsprechenden Menge an Amin hergestellt:

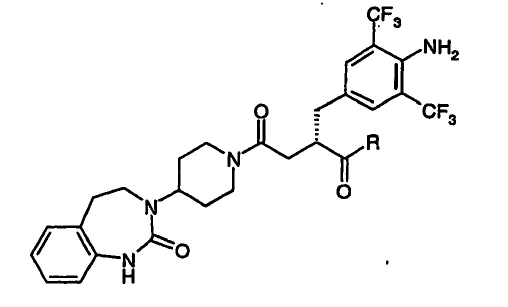

| **Beispiel** | **R** | **Ausbeute (%)** | **Massenspektrum** | **Retentionszeit HPLC (Methode)** |
|---|---|---|---|---|
| **11.1** | 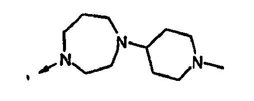 | 52 | 766 [M+H]⁺ | 5.7 min (A) |
| **11.2** | 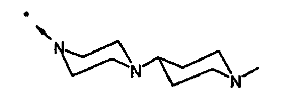 | 64 | 752 [M+H]⁺ | 5.6 min (A) |
| **11.3** | 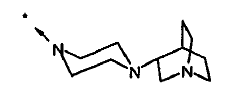 | 21 | 764 [M+H]⁺ | 6.2 min (A) |
| **11.4** | 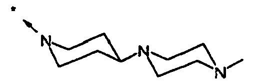 | 74 | 752 [M+H]⁺ | 6.0 min (A) |
| **11.5** | 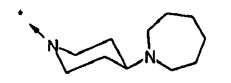 | 55 | 751 [M+H]⁺ | 6.8 min (A) |
| **11.6** | 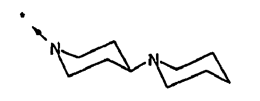 | 62 | 737 [M+H]⁺ | 6.7 min (A) |
| **11.7** | 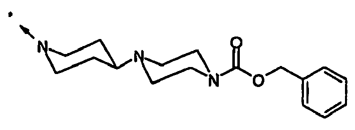 | 100 | 872 [M+H]⁺ | |

### Beispiel 11.7 wurde ohne Reinigung weiter umgesetzt.

### Beispiel 11.8

### (S)-2-(4-Amino-3,5-bis-trifluormethyl-benzyl)-4-[4-(2-oxo-1,2,4,5-tetrahydro-1,3-benzdiazepin-3-yl)-piperidin-1-yl]-1-(4-piperazin-1-yl-piperidin-1-yl)-butan-1,4-dion

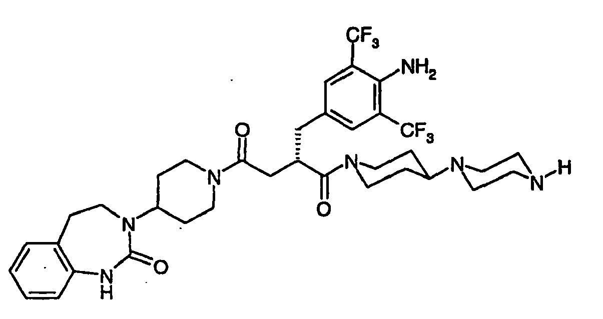

Eine Lösung von 560 mg (0.64 mmol) 4-(1-{(*S*)-2-(4-Amino-3,5-bis-trifluormethyl-benzyl)-4-oxo-4-[4-(2-oxo-1,2,4,5-tetrahydro-1,3-benzdiazepin-3-yl)-piperidin-1-yl]-butyryl}-piperidin-4-yl)-piperazin-1-carbonsäurebenzylester (Rohprodukt aus Beispiel 11.7) in 50 mL MeOH wurde mit 200 mg 10% Pd/C versetzt und das Reaktionsgemisch bei 3 h bei RT und 3 bar H₂ hydriert. Der Katalysator wurde abgesaugt, die Lösung i.vac. eingeengt und der Rückstand chromatographisch (Kieselgel, Gradient: DCM nach DCM/MeOH/NH₃ 10:85:5) gereinigt.

| | |
|---|---|
| Ausbeute: | 230 mg (49% der Theorie) |
| ESI-MS: | (M+H)⁺ = 738 |
| R_{f} = | 0.27 (Kieselgel, DCM/MeOH/NH₃ 50:50:5) |

Analog können folgende Verbindungen hergestellt werden:

| **Beispiel** | **Struktur** |
|---|---|
| **11.9** | 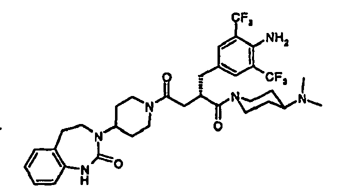 |
| **11.10** | 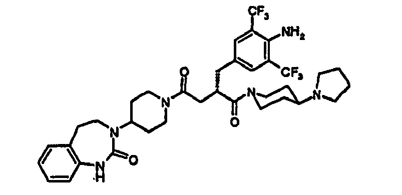 |
| **11.11** | 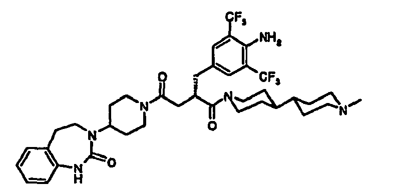 |
| **11.12** | 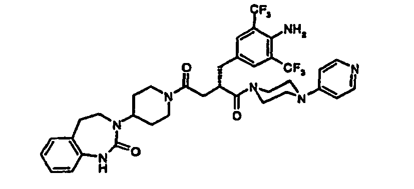 |
| **11.13** | 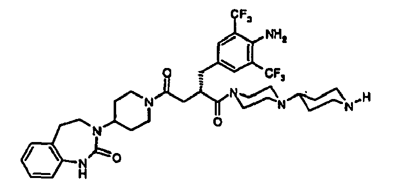 |
| **11.14** | 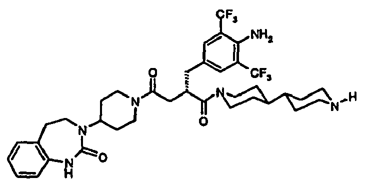 |

### Beispiel 12

### 4-(2-Oxo-1,2,4,5-tetrahydro-1,3-benzdiazepin-3-yl)-piperidin-1-carbonsäure-{(R)-1-(4-arnino-3,5-bis-tdfluormethyl-benzyl)-2-[4-(4-methyl-piperazin-1-yl)-pipeddin-1-yl]-2-oxo-ethyl}-amid

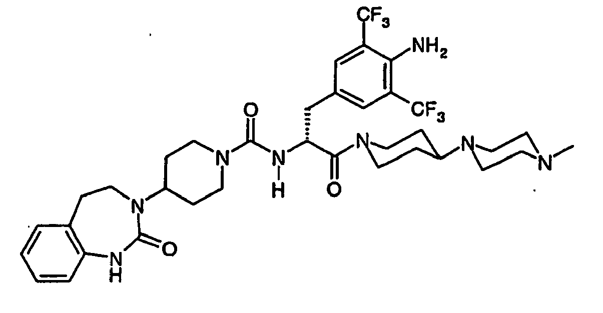

### 12a (4-Amino-3,5-bis-trifluormethyl-phenyl)-methanol

Unter Stickstoffatmosphäre wurden zu einer Lösung von 7.2 g (28.0 mmol) 4-Amino-3,5-bis-trifluormethyl-benzaldehyd (Beispiel 11a) in 100 mL MeOH 1.06 g (28 mmol) NaBH₄ portionenweise zugegeben und das Reaktionsgemisch 2 h bei RT gerührt. Die Reaktionslösung wurde mit 1 M HCl angesäuert, i.vac. eingeengt, der Rückstand mit 150 mL Wasser und 150 mL EtOAc versetzt, die organische Phase abgetrennt und über Na₂SO₄ getrocknet. Nach Entfernen des Trocken- und Lösungsmittels wurde der Rückstand chromatographisch (Kieselgel, PE/EtOAc 9:1) gereinigt.

| | |
|---|---|
| Ausbeute: | 5.1 g (70% der Theorie) |
| ESI-MS: | (M-H)⁻ = 258 |
| R_{f} = | 0.15 (Kieselgel, PE/EtOAc 9:1) |

### 12b 4-Chlormethyl-2,6-bis-trifluormethyl-phenylamin

Zu einer Lösung von 5.1 g (19.68 mmol) (4-Amino-3,5-bis-trifluormethyl-phenyl)-methanol in 80 mL DCM wurden bei RT 4.35 mL (60 mmol) Thionylchlorid zugegeben und das Reaktionsgemisch 3 h bei RT gerührt. Die Reaktionslösung wurde auf Eis und eiskalte NaHCO₃-Lösung gegossen, die organische Phase abgetrennt und über Na₂SO₄ getrocknet. Nach Entfernen des Trocken- und Lösungsmittels wurde der Rückstand ohne Reinigung weiter umgesetzt.

| | |
|---|---|
| Ausbeute: | 5.4 g (99% der Theorie) |
| R_{f} = | 0.55 (Kieselgel, PE/EtOAc 4:1) |

### 12c 2-Acetylamino-2-(4-amino-3,5-bis-trifluormethyl-benzyl)-malonsäurediethyl ester

Unter Stickstoffatmosphäre wurden zu einer Lösung von Natriumethanolat (hergestellt durch Reaktion von 0.46 g (20.0 mmol) Natrium mit EtOH) in 50 mL trockenem EtOH 4.34 g (20.0 mmol) 2-Acetylamino-malonsäurediethylester gegeben und das Reaktionsgemisch 15 min bei RT gerührt. Anschliessend wurde eine Lösung von 5.4 g (19.45 mmol) 4-Chlormethyl-2.,6-bis-trifluormethyl-phenylamin in 100 mL 1,4-Dioxan innerhalb von 5 min zugetropft, die Reaktionslösung weitere 4 h bei RT gerührt, mit 1 L Wasser versetzt und über Nacht gerührt. Der entstandene Niederschlag wurde abfiltriert, mit Wasser nach gewaschen und an der Luft getrocknet.

| | |
|---|---|
| Ausbeute: | 5.2 g (57% der Theorie) |
| ESI-MS: | (M+H)⁺ = 459 |
| R_{f} = | 0.65 (Kieselgel, PE/EtOAc 2:1) |

### 12d 2-Acetylamino-2-(4-amino-3,5-bis-trifluormethyl-benzyl)-malonsäure-mono-ethylester

Zu einer Lösung von 5.1 g (11.13 mmol) 2-Acetylamino-2-(4-amino-3,5-bis-trifluormethyl-benzyl)-malonsäurediethylester in 80 mL trockenem EtOH wurden 2.0 mL 6 M NaOH-Lösung zugegeben und das Reaktionsgemisch über Nacht bei RT gerührt. Man engte i.vac. ein, nahm den Rückstand in 150 mL Wasser auf, säuerte mit 1 M HCI an, extrahierte die wässrige Phase mit 150 mL EtOAc und trocknete die organische Phase über Na₂SO₄. Nach Entfernen des Trocken- und Lösungsmittels wurde der Rückstand ohne Reinigung weiter umgesetzt.

| | |
|---|---|
| Ausbeute: | 4.3 g (90% der Theorie) |
| ESI-MS: | (M+H)⁺ = 431 |
| R_{f} = | 0.1 (Kieselgel, PE/EtOAc 2:1) |

### 12e 2-Acetylamino-3-(4-amino-3,5-bis-trifluormethyl-phenyl)-propionsäureethyl ester

Eine Lösung von 4.3 g (10.0 mmol) 2-Acetylamino-2-(4-amino-3,5-bis-trifluormethyl-benzyl)-malonsäure-mono-ethylester in 200 mL Isopropanol und 80 mL Toluol wurden 15 h auf 100°C erhitzt. Man engt i.vac. ein und setzte den Rückstand ohne Reinigung weiter um.

| | |
|---|---|
| Ausbeute: | 3.8 g (98% der Theorie) |
| R_{f} = | 0.60 (Kieselgel, PE/EtOAc 1:1) |

### 12f (R)-2-Acetylamino-3-(4-amino-3,5-bis-trifluormethyl-phenyl)-propionsäureethylester

Zu einer 37 °C warmen Lösung von 3.65 g (20.5 mmol) Na₂HPO₄ Dihydrat in 130 mL Wasser wurde 4 mL Alcalase 2.4 L FG (Novozymes A/S; DK 2880 Bagsvaerd) gegeben und mittels Zugabe von NaH₂PO₄ Dihydrat ein pH-Wert von 7.5 eingestellt. Anschließend wurde bei 37 °C unter Rühren eine Lösung von 3.8 g (9.84 mmol) 2-Acetylamino-3-(4-amino-3,5-bis-trifluormethyl-phenyl)-propionsäureethylester in 40 mL Aceton zugetropft. Der pH-Wert des Reaktionsgemisches wurde dabei durch Zugabe von 1 M NaOH stets in einem Bereich von pH 7.4-7.6 gehalten. Nach erfolgter Zugabe wurde 4 h bei 37 °C nachgerührt. Nach Abkühlen auf RT wurde das Reaktionsgemisch mit 300 mL DCM, 300 mL 15% K₂CO₃-Lösung und 200 mL Wasser versetzt. Die organische Phase wurde abgetrennt, mit 7% K₂CO₃-Lösung gewaschen und über Na₂SO₄ getrocknet. Nach Entfernen des Trocken- und Lösungsmittels wurde das Rohprodukt (2.2 g) ohne Reinigung weiter umgesetzt.

| | |
|---|---|
| ESI-MS: | (M+H)⁺ 387 |
| R_{f} = | 0.60 (Kieselgel, PE/EtOAc 1:1) |

12g (*R*)-2-Amino-3-(4-amino-3,5-bis-trifluormethyl-phenyl)-propionsäureethylester Eine Lösung von 2.2 g des obigen Rohproduktes wurde in 4 M HCl 1.5 h unter Rückfluss erhitzt. Man engte i.vac. ein, nahm den Rückstand in 50 mL EtOH und 50 mL ethanolischer HCl (11.5 M) auf und rührte das Reaktionsgemisch über Nacht bei RT. Man engte erneut i.vac. ein, versetzte mit 50 mL 15% K₂CO₃-Lösung, extrahierte mit 200 mL EtOAc, trennte die organische Phase ab und engte diese i.vac. ein. Das Rohprodukt (1.8 g) wurde ohne Reinigung weiter umgesetzt.

| | |
|---|---|
| ESI-MS: | (M+H)⁺ 345 |
| R_{f} = | 0.50 (Kieselgel, DCM/MeOH/NH₃ 90:10:1) |

### 12h (R)-3-(4-Amino-3,5-bis-trifluormethyl-phenyl)-2-{[4-(2-oxo-1,2,4,5-tetrahydro-1,3-benzdiazepin-3-yl)-piperidin-1-carbonyl]-amino}-propionsäureethylester

Zu einer auf -5°C gekühlten Lösung von 1.8 g des obigen Rohproduktes in 50 mL THF wurden 0.94 g (5.7 mmol) CDT zugegeben und das Reaktionsgemisch 45 min bei dieser Temperatur und nach Entfernen des Eisbades weitere 30 min nachgerührt. Dann erfolgte die Zugabe einer Lösung von 1.28 g (5.2 mmol) 3-Piperidin-4-yl-1,3,4,5-tetrahydro-1,3-benzdiazepin-2-on in 50 mL DMF. Die Reaktionslösung wurde 2 h auf 80°C erhitzt, nach dem Abkühlen i.vac. eingeengt, der Rückstand mit 150 mL EtOAc und 150 mL 10% Zitronensäure-Lösung versetzt, die organische Phase abgetrennt, mit 150 mL gesättigter NaHCO₃-Lösung gewaschen und über Na₂SO₄ getrocknet. Nach Entfernen des Trocken- und Lösungsmittels wurde das Rohprodukt (3.7 g) ohne Reinigung weiter umgesetzt.

| | |
|---|---|
| ESI-MS: | (M+H)⁺ 616 |
| R_{f} = | 0.25 (Kieselgel, EtOAc) |

### 12i (R)-3-(4-Amino-3,5-bis-trifluormethyl-phenyl)-2-{[4-(2-oxo-1,2,4,5-tetrahydro-1,3-benzdiazepin-3-yl)-piperidin-1-carbonyl]-amino}-propionsäure

Zu einer Lösung von 3.7 g des obigen Rohproduktes in 50 mL THF wurde eine Lösung von 0.4 g (9.5 mmol) Lithiumhydroxid Hydrat in 50 mL Wasser gegeben und das Reaktionsgemisch bei RT über Nacht gerührt. Man zog i.vac. das THF ab, versetzte mit 100 mL Wasser und säuerte mit 1 M HCl an. Das ausgefallene Produkt wurde abgesaugt, mit 50 mL Wasser gewaschen und bei 60°C im Trockenschrank getrocknet.

| | |
|---|---|
| Ausbeute: | 2.6 g (90% der Theorie bezogen auf 12f) |
| ESI-MS: | (M-H)⁻ = 586 |
| Retentionszeit (HPLC): | 7.1 min (Methode A) |

### 12k 4-(2-Oxo-1,2,4,5-tetrahydro-1,3-benzdiazepin-3-yl)-piperidin-1-carbonsäure-{(R)-1-(4-amino-3,5-bis-trifluormethyl-benzyl)-2-[4-(4-methyl-piperazin-1-yl)-piperidin-1-yl]-2-oxo-ethyl}-amid

Zu einer Lösung von 500 mg (0.85 mmol) (*R*)-3-(4-Amino-3,5-bis-trifluormethylphenyl)-2-{[4-(2-oxo-1,2,4,5-tetrahydro-1,3-benzdiazepin-3-yl)-piperidi n-1-carbonyl]-amino}-propionsäure, 289 mg (0.9 mmol) TBTU und 0.28 mL (2.0 mmol) Triethylamin in 50 mL THF wurden 155 mg (0.85 mmol) 1-Methyl-4-piperidin-4-yl-piperazin zugegeben und die Reaktionsmischung über Nacht bei RT gerührt. Man engte i. vac. ein, nahm den Rückstand in 100 mL EtOAc und 100 mL 10% Zitronensäure-Lösung auf, separierte die organische Phase und zog das Lösungsmittel i.vac. ab. Anschliessend wurde der Rückstand chromatographisch (Kieselgel, Gradient: DCM nach DCM/MeOH/NH₃ 10:85:5) gereinigt.

| | |
|---|---|
| Ausbeute: | 570 mg (89% der Theorie) |
| ESI-MS: | (M+H)⁺ = 753 |
| R_{f} = | 0.5 (Kieselgel, DCM/MeOH/NH₃ 85:15:1.5) |

Analog wurden folgende Verbindungen aus (*R*)-3-(4-Amino-3,5-bis-trifluormethylphenyl)-2-{[4-(2-oxo-1,2,4,5-tetrahydro-1,3-benzdiazepin-3-yl)-piperidin-1-carbonyl]-amino}-propionsäure und der entsprechenden Menge an Amin hergestellt:

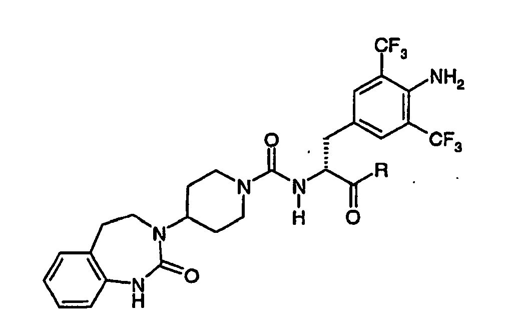

| **Beispiel** | **R** | **Ausbeute (%)** | **Massenspektrum** | **R_{f}-Wert auf Kieselgel (Fliessmittel)** |
|---|---|---|---|---|
| **12.1** | 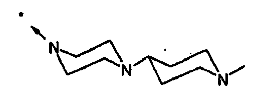 | 89 | 753 [M+H]⁺ | 0.4 (DCM/MeOH/NH ₃85:15:1.5) |
| **12.2** | 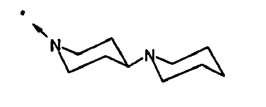 | 64 | 738 **[M+H]⁺** | 0.5 (DCM/MeOH/NH₃ 85:15:1.5) |
| **12.3** | 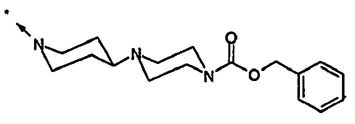 | 80 | 873 [M+H]⁺ | |

### Beispiel 12.3 wurde ohne Reinigung weiter umgesetzt.

### Beispiel 12.4

### 4-(2-Oxo-1,2,4,5-tetrahydro-1,3-benzdiazepin-3-yl)-piperidin-1-carbonsäure-[(R)-1-(4-amino-3,5-bis-trifluormethyl-benzyl)-2-oxo-2-(4-piperazin-1-yl-piperidin-1-yl)-ethyl]-amid

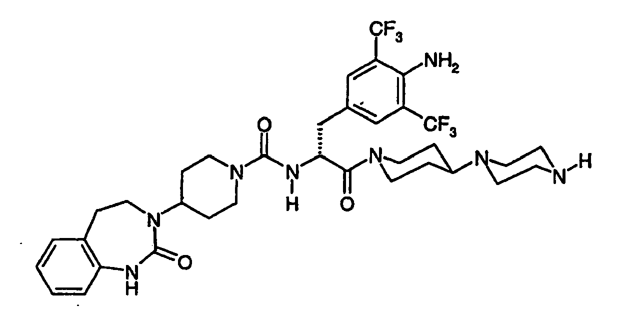

Hergestellt analog Beispiel 11.8 aus 450 mg (0.52 mmol) 4-[1-((*R*)-3-(4-Amino-3,5-bis-trifluormethyl-phenyl)-2-{[4-(2-oxo-1,2,4,5-tetrahydro-1,3-benzdiazepin-3-yl)-piperidin-1-carbonyl]-amino}-propionyl)-piperidin-4-yl]-piperazin-1-carbonsäure-benzylester (Rohprodukt aus Beispiel 12.3).

| | |
|---|---|
| Ausbeute: | 200 mg (53% der Theorie) |
| ESI-MS: | (M+H)⁺ = 739 |
| R_{f} = | 0.3 (Kieselgel, DCM/MeOH/NH₃ 50:50:5) |

Analog können folgende Verbindungen hergestellt werden:

| **Beispiel** | **Struktur** |
|---|---|
| **12.5** | 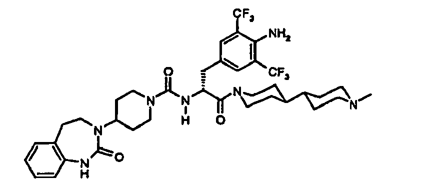 |
| **12.6** | 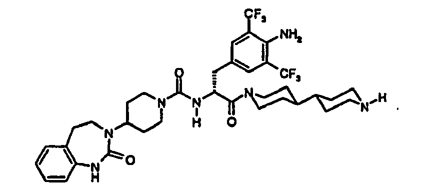 |
| **12.7** | 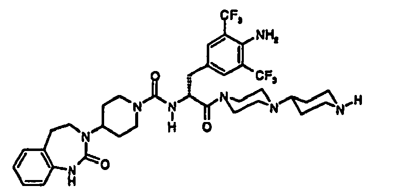 |
| **12.8** | 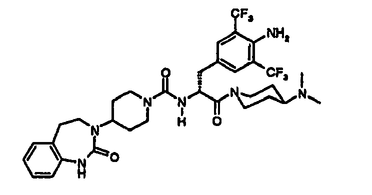 |
| **12.9** | 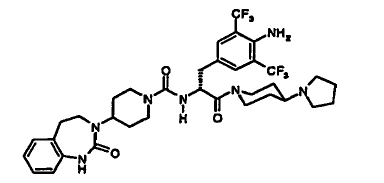 |
| **12.10** | 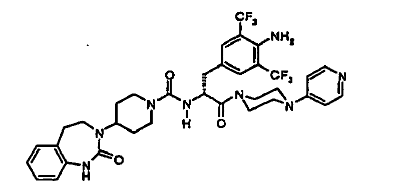 |

### Beispiel 13

### 4-(2-Oxo-1,2,4,5-tetrahydro-1,3-benzdiazepin-3-yl)-piperidin-1-carbonsäure-(R)-1-(4-amino-3-chlor-5-trifluormethyl-benzyl)-2-[4-(4-methyl-piperazin-1-yl)-piperidi n-1-yl]-2-oxo-ethylester

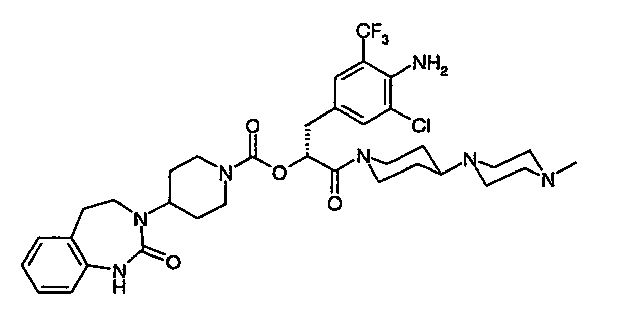

### 13a (S)-3-[(R)-3-(4-Amino-3-chlor-5-trifluormethyl-phenyl)-2-benzyloxy-propionyl]-4-benzyl-oxazolidin-2-on

Unter Stickstoffatmosphäre wurden zu einer auf -60°C gekühlten Lösung von 5.1 mL (5.1 mmol, 1 M in THF) Natrium-bis-trimethylsilylamid in 4 mL THF eine auf -60°C gekühlte Lösung von 1.33 g (4.1 mmol) (*S*)-4-Benzyl-3-(2-benzyloxy-acetyl)-oxazolidin-2-on in 15 mL THF innerhalb von 10 min zugetropft und das Reaktionsgemisch 1 h bei dieser Temperatur nachgerührt. Dann wurde auf -70°C gekühlt und eine Lösung von 2.0 g (8.2 mmol) 2-Chlor-4-chlormethyl-6-trifluormethyl-phenylamin (Beispiel 2a) in 15 mL THF langsam zugetropft. Man hielt die Reaktionslösung 1 h bei -70°C und liess dann innerhalb von 2 h auf RT erwärmen. Man versetzte mit 50 mL gesättigter NH₄Cl-Lösung, extrahierte mit 50 mL EtOAc, trennte die organische Phase ab, extrahierte die wässrige Phase nochmals mit 50 mL EtOAc, wusch die vereinigten organischen Phasen mit 100 mL gesättigter NaCl- und 1 M KHSO₄-Lösung und trocknete über Na₂SO₄. Nach Entfernen des Trocken- und Lösungsmittels wurde der Rückstand chromatographisch (Kieselgel, PE/EtOAc 4:1) gereinigt.

| | |
|---|---|
| Ausbeute: | 2.1 g (96% der Theorie) |
| ESI-MS: | (M+H)⁺ = 533/535 |
| R_{f} = | 0.15 (Kieselgel, PE/EtOAc 4:1) |

### 13b (R)-3-(4-Amino-3-chlor-5-trifluormethyl-phenyl)-2-benzyloxy-propionsäure

Zu einer auf 0°C gekühlten Lösung von 2.1 g (3.94 mmol) (*S*)-3-[(*R*)-3-(4-Amino-3-chlor-5-trifluormethyl-phenyl)-2-benzyloxy-propionyl]-4-benzyl-oxazolidin-2-on in 50 mL THF wurden eine Lösung von 0.34 g (8.0 mmol) Lithiumhydroxid Hydrat und 1.38 mL (16 mmol, 35% in Wasser) H₂O₂ in 25 mL Wasser gegeben und das Reaktionsgemisch 2 h bei 0°C gerührt. Man versetzte mit 5 mL gesättigter Na₂SO₃-Lösung und 5 mL gesättigter NaHCO₃-Lösung, rührte 30 min nach und entfernte dann i.vac. das THF. Der wässrige Rückstand wurde zweimal mit jeweils 50 mL EtOAc extrahiert und die vereinigten organischen Phasen über MgSO₄ getrocknet. Nach Entfernen des Trocken- und Lösungsmittels wurde das Rohprodukt ohne Reinigung weiter umgesetzt.

### 13c (R)-3-(4-Amino-3-chlor-5-trifluormethyl-phenyl)-2-benzyloxy-1-[4-(4-methylpiperazin-1-yl)-piperidin-1-yl]-propan-1-on

Zu einer Lösung von 1.5 g (4.01 mmol) (*R*)-3-(4-Amino-3-chlor-5-trifluormethylphenyl)-2-benzyloxy-propionsäure in 50 mL THF wurden 1.35 g (4.20 mmol) TBTU, 0.70 mL (5.0 mmol) Triethylamin und 0.75 g (4.01 mmol) 1-Methyl-4-piperidin-4-yl-piperazin gegeben und das Reaktionsgemisch über Nacht bei RT gerührt. Die Reaktionslösung wurde i.vac. eingeengt, der Rückstand mit 200 mL EtOAc und 200 mL gesättigter NaHCO₃-Lösung versetzt, die organische Phase abgetrennt und mit 100 mL 5% Zitronensäurelösung extrahiert. Der Zitronensäureextrakt wurde mit K₂CO₃ alkalisch gestellt und zweimal mit jeweils 100 mL EtOAc extrahiert. Die vereinigten organischen Phasen wurden i.vac. eingeengt und der Rückstand ohne Reinigung weiter umgesetzt.

| | |
|---|---|
| Ausbeute: | 1.75 g (81 % der Theorie) |
| ESI-MS: | (M+H)⁺ = 539/541 (Cl) |
| Retentionszeit (HPLC): | 5.9 min (Methode A) |

### 13d (R)-3-(4-Amino-3-chlor-5-trifluormethyl-phenyl)-2-hydroxy-1-[4-(4-methylpiperazin-1-yl)-piperidin-1-yl]-propan-1-on

Zu einer Suspension von 450 mg (0.84 mmol) (*R*)-3-(4-Amino-3-chlor-5-trifluormethyl-phenyl)-2-benzyloxy-1-[4-(4-methyl-piperazin-1-yl)-piperidin-1-yl]-propan-1-on und 380 mg (2.5 mmol) Nal in 30 mL Acetonitril wurden 0.32 mL (2.5 mmol) Chlor-trimethyl-silan zugegeben und das Reaktionsgemisch 7 h bei 80°C gerührt. Man versetzte mit 30 mL EtOH und 20 mL Isopropanol, rührte 30 min bei RT, fügte 15 mL NH₃-Lösung zu und rührte weitere 30 min. Man engte i.vac. ein, versetzte den Rückstand mit 100 mL 15% K₂CO₃-Lösung, extrahierte mit 100 mL EtOAc, trennte die organische Phase ab, wusch diese mit 3% Na₂SO₃-Lösung und trocknete über Na₂SO₄. Nach Entfernen des Trocken- und Lösungsmittels wurde das Rückstand ohne Reinigung weiter umgesetzt.

| | |
|---|---|
| Ausbeute: | 300 mg (80% der Theorie) |
| Retentionszeit (HPLC): | 3.7 min (Methode A) |

### 13e 4-(2-Oxo-1,2,4,5-tetrahydro-1,3-benzdiazepin-3-yl)-piperidin-1-carbonyl chlorid

Zu einer auf 0°C gekühlten Lösung von 2.5 g (10.2 mmol) 3-Piperidin-4-yl-1,3,4,5-tetrahydro-1,3-benzdiazepin-2-on und 2.6 mL (14.9 mmol) Ethyldiisopropylamin in 75 mL DCM wurden 6 g (12.1 mmol) Phosgen (20 Gewichtsprozent in Toluol) zugegeben und das Reaktionsgemisch 30 min bei dieser Temperatur nachgerührt. Man liess auf RT erwärmen, engte i.vac. auf ca. 50 mL ein und filtrierte über Kieselgel ab, wusch dieses mit 200 mL DCM/EtOAc (1:1) und engte die vereinigten Filtrate erneut i.vac. ein. Der Rückstand wurde mit Diisopropylether verrührt, abgesaugt und i.vac. getrocknet.

| | |
|---|---|
| Ausbeute: | 2.42 g (77% der Theorie) |
| R_{f} = | 0.43 (Kieselgel, DCM/EtOAc 1:1) |

### 13f 4-(2-Oxo-1,2,4,5-tetrahydro-1,3-benzdiazepin-3-yl)-piperidin-1-carbonsäure-(R)-1-(4-amino-3-chlor-5-trifluormethyl-benzyl)-2-[4-(4-methyl-piperazin-1-yl)-piperidin-1-yl]-2-oxo-ethylester

Unter Stickstoffatmosphäre wurden zu einer auf 0°C gekühlten Lösung von 300 mg (0.67 mmol) (*R*)-3-(4-Amino-3-chlor-5-trifluormethyl-phenyl)-2-hydroxy-1-[4-(4-methyl-piperazin-1-yl)-piperidin-1-yl]-propan-1-on in 30 mL THF 31 mg (0.7 mmol) NaH (55% in Mineralöl) zugegeben und das Reaktionsgemisch 30 min bei dieser Temperatur gerührt. Dann wurden portionenweise 246 mg (0.8 mmol) 4-(2-Oxo-1,2,4,5-tetrahydro-1,3-benzdiazepin-3-yl)-piperidin-1-carbonylchlorid zugegeben und die Reaktionslösung nach Entfernen des Kältebades 3 h bei RT gerührt. Man engt i.vac. ein, versetzte den Rückstand mit 4 mL Acetonitril und reinigte mittels HPLC-MS.

| | |
|---|---|
| Ausbeute: | 88 mg (15% der Theorie) |
| ESI-MS: | (M+H)⁺ = 720/722 (Cl) |
| Retentionszeit (HPLC): | 6.0 min (Methode A) |

Analog Beispiel 13c, 13d und 13f können folgende Verbindungen aus (*R*)-3-(4-Amino-3-chlor-5-trifluormethyl-phenyl)-2-benzyloxy-propionsäure und den jeweiligen Aminen hergestellt werden:

| **Beispiel** | **Struktur** |
|---|---|
| **13.1** | 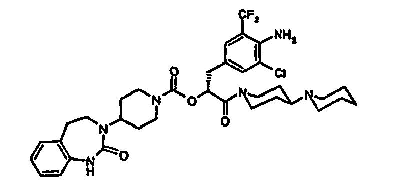 |
| **13.2** | 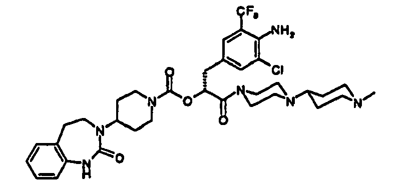 |
| **13.3** | 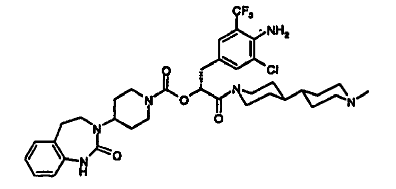 |
| **13.4** | 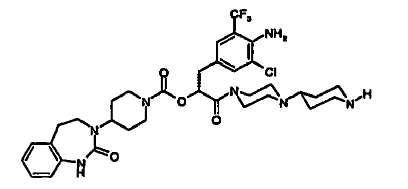 |
| **13.5** | 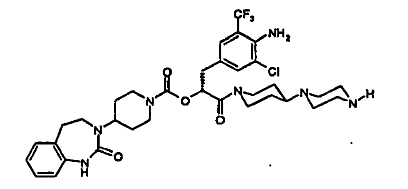 |
| **13.6** | 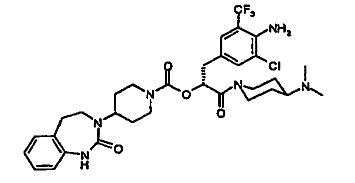 |
| **13.7** | 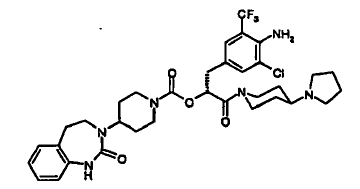 |
| **13.8** | 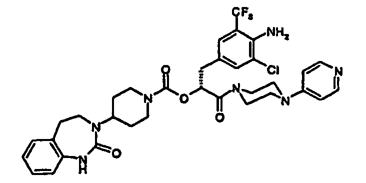 |
| **13.9** | 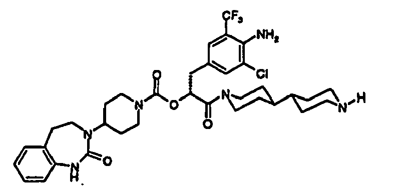 |

### Beispiel 14

### 4-(2-Oxo-1,4-dihydro-2H-chinazolin-3-yl)-piperidin-1-carbonsäure [2-[1,4']bipiperidinyl-1'-yl-1-(4-brom-3-methyl-benzyl)-2-oxo-ethyl]-amid

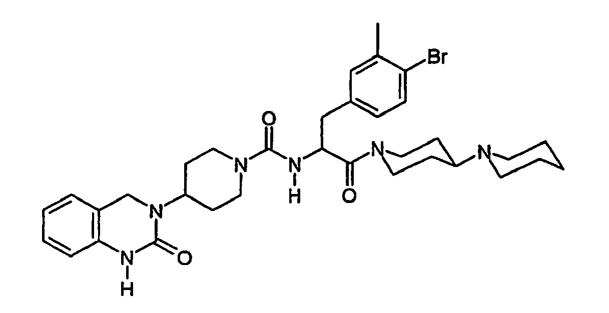

### 14a 2-Amino-3-(4-brom-3-methyl-phenyl)-propionsäureethylester Hydrochlorid

Die Mischung aus 31.4 g (115 mmol) N-(Diphenylmethylen)-glycinethylester, 28.5 g (108 mmol) (4-Brom-3-methylphenyl)-methylbromid, 3.55 g (11.0 mmol) Tetrabutylammoniumbromid, 116 g (550 mmol) K₂CO₃ und 400 mL Acetonitril wurde 4 h unter Rückfluss erhitzt. Der Feststoff wurde abfiltriert, die Mutterlauge im Vakuum eingedampft. Der Rückstand wurde in 500 mL *tert*-Butylmethylether aufgenommen und nach Zugabe von 200 mL 10% HCl über Nacht bei RT gerührt. Die organische Phase wurde abgetrennt, die wässrige noch zweimal mit je 50 mL *tert* -Butylmethylether gewaschen, dann unter äußerer Kühlung mit Eis mit 10% Na₂CO₃-Lösung neutralisiert und mit DCM erschöpfend extrahiert. Die vereinigten organischen Phasen wurden noch zweimal mit je 50 mL Wasser gewaschen, über MgSO₄ getrocknet, über Aktivkohle filtriert und im Vakuum eingeengt. Der verbliebene ölige Rückstand wurde in 50 mL wasserfreiem EtOH gelöst, mit etherischer HCl-Lösung versetzt und dann mit *tert*-Butylmethylether auf ein Gesamtvolumen von 500 mL verdünnt. Nach 20 minütigem Rühren fiel ein farbloser, kristalliner Niederschlag aus, der abgenutscht und an der Luft getrocknet wurde.

| | |
|---|---|
| Ausbeute: | 17.6 g (47% der Theorie) |
| R_{f} = | 0.45 (Kieselgel, DCM/MeOH/NH₃ 9:1:0.1) |

### 14b 3-(4-Brom-3-methyl-phenyl)-2-{[4-(2-oxo-1,4-dihydro-2H-chinazolin-3-yl)-piperidin-1-carbonyl]-amino}-propionsäureethylester

Zu einer eisgekühlten Suspension von 6.45 g (20.0 mmol) 2-Amino-3-(4-brom-3-methyl-phenyl)-propionsäureethylester Hydrochlorid in 50 mL DMF gab man 3.28 g (20.0 mmol) CDT und 2.77 mL (20.0 mmol) Triethylamin. Die Reaktionsmischung wurde anschließend 1 h bei 0 °C und 1 Stunde bei RT gerührt, dann mit der Suspension von 4.63 g (20.0 mmol) 3-Piperidin-4-yl-3,4-dihydro-1*H*-chinazolin-2-on in 50 mL DMF versetzt. Das Gemisch wurde 1.5 h auf 80°C erhitzt und dann in 500 mL Wasser eingerührt. Der nach einiger Zeit fest gewordene Niederschlag wurde mit Hilfe eines Ultra-Turrax-Rührers zerkleinert, gründlich mit Wasser gewaschen, abgenutscht und im Umlufttrockenschrank bei 50 °C getrocknet.

| | |
|---|---|
| Ausbeute: | 11.9 g (97% der Theorie; enthält 1.0 eq. DMF) |
| R_{f} = | 0.40 (Kieselgel, DCM/MeOH/NH₃ 9:1:0.1) |

### 14c 3-(4-Brom-3-methyl-phenyl)-2-{[4-(2-oxo-1,4-dihydro-2H-chinazolin-3-yl)-piperidin-1-carbonyl]-amino}-propionsäure

Zu der Lösung von 10.9 g (20 mmol) 3-(4-Brom-3-methyl-phenyl)-2-{[4-(2-oxo-1,4-dihydro-2*H*-chinazolin-3-yl)-piperidin-1-carbonyl]-amino}-propionsäureethylester in 60 mL EtOH wurden 60 mL 1 M NaOH gegeben und die Mischung anschließend 2 h unter Rückfluss erhitzt. Nach dem Abkühlen wurde mit 50 mL Wasser verdünnt und mit 20% Zitronensäure-Lösung sauer gestellt. Der ausgefallene Niederschlag wurde abgenutscht, gründlich mit Wasser gewaschen und im Umlufttrockenschrank bei 50 °C getrocknet.

| | |
|---|---|
| Ausbeute: | 9.6 g (93% der Theorie) |
| ESI-MS: | (M-H)⁻ = 513/515 (Br) |
| R_{f} = | 0.10 (Kieselgel, DCM/MeOH 9:1) |

### 14d 4-(2-Oxo-1,4-dihydro-2H-chinazolin-3-yl)-pipeddin-1-carbonsäure[2-[1,4']bipiperidinyl-1'-yl-1-(4-brom-3-methyl-benzyl)-2-oxo-ethyl]-amid

Analog Beispiel 2f wurden aus 515 mg (1.00 mmol) 3-(4-Brom-3-methyl-phenyl)-2-{[4-(2-oxo-1,4-dihydro-2*H*-chinazolin-3-yl)-piperidin-1-carbonyl]-amino}-propionsäure und 177 mg (1.00 mmol) [1,4']Bipiperidinyl das Produkt erhalten.

| | |
|---|---|
| Ausbeute: | 320 mg (48% der Theorie) |
| ESI-MS: | (M+H)⁺ = 665/667 (Br) |
| R_{f} = | 0.33 (Kieselgel, DCM/MeOH/NH₃ 9:1:0.1) |

Analog wurden folgende Verbindungen aus 3-(4-Brom-3-methyl-phenyl)-2-{[4-(2-oxo-1,4-dihydro-2*H*-chinazolin-3-yl)-piperidin-1-carbonyl]-amino}-propionsäure und der entsprechenden Menge an Amin hergestellt:

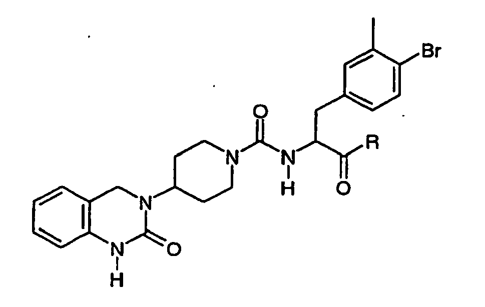

| **Beispiel** | **R** | **Ausbeute (%)** | **Massenspektrum** | **R_{f} (Kieselgel)** |
|---|---|---|---|---|
| **14.1** | 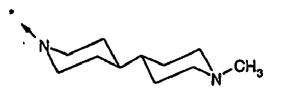 | 52 | 679/681 [M+H]⁺ | 0.28 (DCM/MeOH/NH₃ 9:1:0.1) |
| **14.2** | 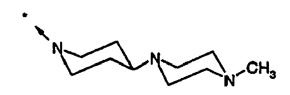 | 50 | | 0.24 (DCM/MeOH/NH₃ 9:1:0.1) |
| **14.3** | 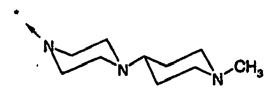 | 25 | 680/682 [M+H]⁺ | 0.19 (DCM/MeOH/NH₃ 9:1:0.1) |
| **14.4** | 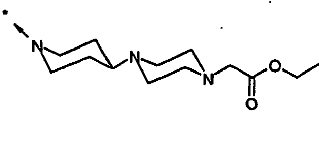 | 25 | 774/776 [M+Na]⁺ | 0.45 (DCM/MeOH 9:1) |
| **14.5** | 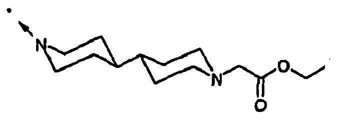 | 40 | 751/753 [M+H]⁺ | 0.48 (DCM/MeOH 9:1) |
| **14.6** | 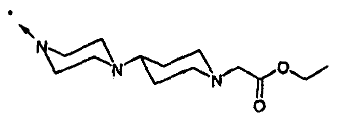 | 28 | 774/776 [M+Na]⁺ | 0.39 (DCM/MeOH 9:1) |

### Beispiel 15

### {4-[1-(3-(4-Brom-3-methyl-phenyl)-2-{[4-(2-oxo-1,4-dihydro-2H-chinazolin-3-yl)-piperidin-1-carbonyl]-amino}-propionyl)-piperidin-4-yl]-piperazin-1-yl}-essigsäure

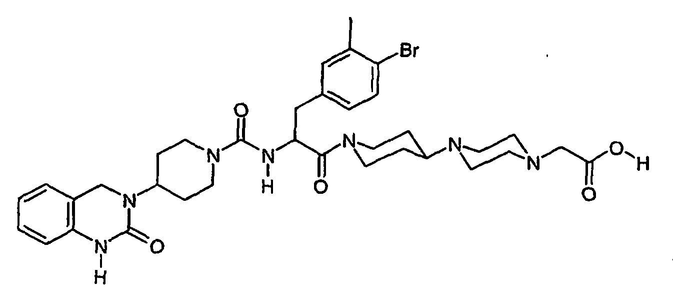

Zu einer Lösung von 80 mg (0.11 mmol) {4-[1-(3-(4-Brom-3-methyl-phenyl)-2-{[4-(2-oxo-1,4-dihydro-2*H*-chinazolin-3-yl)-piperidin-1-carbonyl]-amino}-propionyl)-plperidin-4-yl]-piperazin-1-yl}-essigsäureethylester (Beispiel 14.4) in 4 mL THF wurden 1.0 mL (1.00 mmol) 1 M NaOH gegeben. Das Reaktionsgemisch wurde über Nacht bei RT gerührt und das Lösungsmittel i.vac. entfernt. Zu dem Rückstand wird 1 mL 1 M HCl gegeben und erneut zur Trockene eingedampft. Der Rückstand wurde in EtOH aufgenommen und nach Filtration wurde die Mutterlauge i.vac. eingedampft. Der Rückstand wurde mit Diisopropylether verrieben und nach Filtration an der Luft getrocknet.

| | |
|---|---|
| Ausbeute: | 80 mg (100% der Theorie) |
| Retentionszeit (HPLC): | 5.9 min (Methode A) |

Analog wurden folgende Verbindungen aus den jeweiligen Ethylestern (Beispiele 14.5 und 14.6) hergestellt:

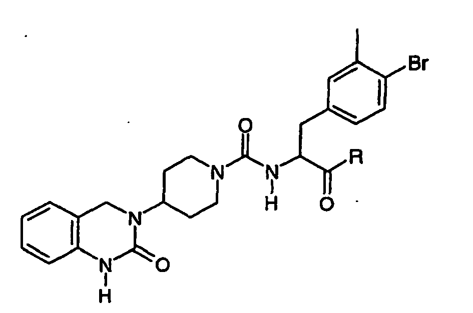

| **Beispiel** | **R** | **Ausbeute (%)** | **Massenspektrum** | **R_{f} (Kieselgel) oder Retentionszeit HPLC** |
|---|---|---|---|---|
| **15.1** | 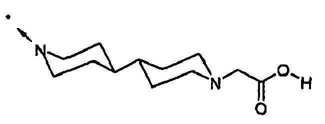 | 59 | 745/747 [M+Na]⁺ | 0.12 (DCM/MeOH 9:1) |
| **15.2** | 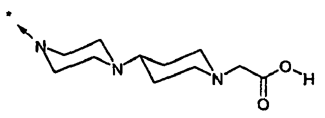 | 81 | 722/724 [M-H]⁻ | 5.5 min (A) |

### Beispiel 16

### 2-(4-Brom-3-methyl-benzyl)-1-[4-(4-methyl-piperazin-1-yl)-piperidin-1-yl]-4-[4-(2-oxo-1,4-dihydro-2H-chinazolin-3-yl)-piperidin-1-yl]-butan-1,4-dion

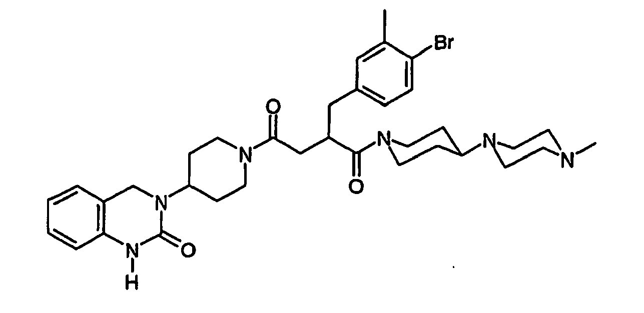

### 16a 2-(4-Brom-3-methyl-benzyl)-2-ethoxycarbonyl-bernsteinsäure-4-tert-butylester-1-ethylester

Analog Beispiel 2b wurde aus 11.4 g (41.7 mmol) 2-Ethoxycarbonyl-bemsteinsäure-4-*tert*-butylester-1-ethylester und 11.0 g (41.7 mmol) 1-Brom-4-brommethyl-2-methylbenzol das Produkt hergestellt.

| | |
|---|---|
| Ausbeute: | 21.3 g (100% der Theorie) |
| R_{f} = | 0.64 (Kieselgel, PE/EtOAc 8:2) |

### 16b 2-(4-Brom-3-methyl-benzyl)-2-ethoxycarbonyl-bernsteinsäureethylester

Analog Beispiel 2c wurde aus 21.3 g (41.7 mmol) 2-(4-Brom-3-methyl-benzyl)-2-ethoxycarbonyl-bernsteinsäure-4-*tert*-butylester-1-ethylester das Produkt hergestellt.

| | |
|---|---|
| Ausbeute: | 7.8 g (47% der Theorie) |
| R_{f} = | 0.26 (Kieselgel, PE/EtOAc 8:2) |

### 16c 2-(4-Brom-3-methyl-benzyl)-2-{2-oxo-2-[4-(2-oxo-1,4-dihydro-2H-chinazolin-3-yl)-piperidin-1-yl]-ethyl}-malonsäurediethylester

Analog Beispiel 2d wurde aus 7.80 g (19.4 mmol) 2-(4-Brom-3-methyl-benzyl)-2-ethoxycarbonyl-bernsteinsäureethylester und 4.50 g (19.4 mmol) 3-Piperidin-4-yl-3,4-dihydro-1*H*-chinazolin-2-on das Produkt hergestellt.

| | |
|---|---|
| Ausbeute: | 8.30 g (70% der Theorie) |
| EI-MS: | (M)⁺ = 613/615 (Br) |
| R_{f} = | 0.80 (Kieselgel, DCM/Cyc/MeOH/NH₃ 70:15:15:2) |

### 16d 2-(4-Brom-3-methyl-benzyl)-4-oxo-4-[4-(2-oxo-1,4-dihydro-21+chinazolin-3-yl)-piperidin-1-yl]-butansäure

Analog Beispiel 2e wurde aus 8.30 g (13.5 mmol) 2-(4-Brom-3-methyl-benzyl)-2-{2-oxo-2-[4-(2-oxo-1,4-dihydro-2H chinazolin-3-yl)-piperidin-1-yl]-ethyl}-malonsäure-diethylester das Produkt hergestellt.

| | |
|---|---|
| Ausbeute: | 5.10 g (74% der Theorie) |
| EI-MS: | (M)⁺ = 513/515 (Br) |
| R_{f} = | 0.20 (Kieselgel, DCM/Cyc/MeOH/NH₃ 70:15:15:2) |

### 16e 2-(4-Brom-3-methyl-benzyl)-1-[4-(4-methyl-piperazin-1-yl)-piperidin-1-yl]-4-[4-(2-oxo-1,4-dihydro-2H-chinazolin-3-yl)-piperidin-1-yl]-butan-1,4-dion

Analog Beispiel 2f wurde aus 0.51 g (1.00 mmol) 2-(4-Brom-3-methyl-benzyl)-4-oxo-4-[4-(2-oxo-1,4-dihydro-2*H*-chinazolin-3-yl)-piperidin-1-yl]-butansäure und 0.18 g (1.00 mmol) 1-Methyl-4-piperidin-4-yl-piperazin das Produkt hergestellt.

| | |
|---|---|
| Ausbeute: | 250 mg (37% der Theorie) |
| EI-MS: | (M)⁺ = 678/680 (Br) |
| R_{f} = | 0.50 (Kieselgel, DCM/Cyc/MeOH/NH₃ 70:15:15:2) |

Analog wurden folgende Verbindungen aus 2-(4-Btom-3-methyl-benzyl)-4-oxo-4-[4-(2-oxo-1,4-dihydro-2*H*-chinazolin-3-yl)-piperidin-1-yl]-butansäure und der entsprechenden Menge an Amin hergestellt:

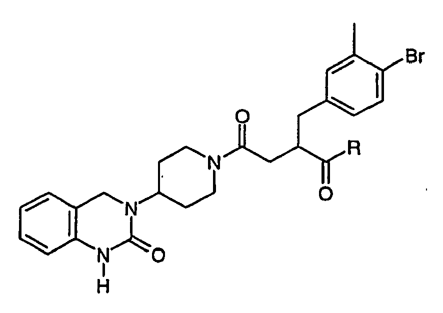

| **Beispiel** | **R** | **Ausbeute (%)** | **Massenspektrum** | **R_{f} (Kieselgel)** |
|---|---|---|---|---|
| **16.1** | 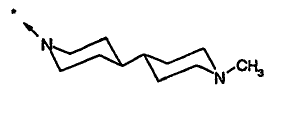 | 44 | 677/679 [M]⁺ | 0.50 (DCM/Cyc/MeOH/NH₃ 70:15:15:2) |
| **16.2** | 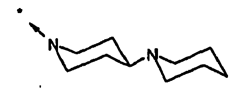 | 46 | 663/665 [M]⁺ | 0.52 (DCM/Cyc/MeOH/NH₃ 70:15:15:2) |
| **16.3** | 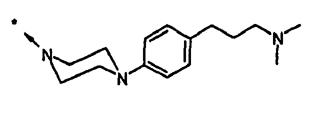 | 27 | 742/744 [M]⁺ | 0.56 (DCM/Cyc/MeOH/NH₃ 70:15:15:2) |

### Beispiel 16.4

### [4-(1-{2-(4-Brom-3-methyl-benzyl)-4-oxo-4-[4-(2-oxo-1,4-dihydro-2H-chinazolin-3-yl)-piperidin-1-yl]-butyryl}-piperidin-4-yl)-piperazin-1-yl]-essigsäure

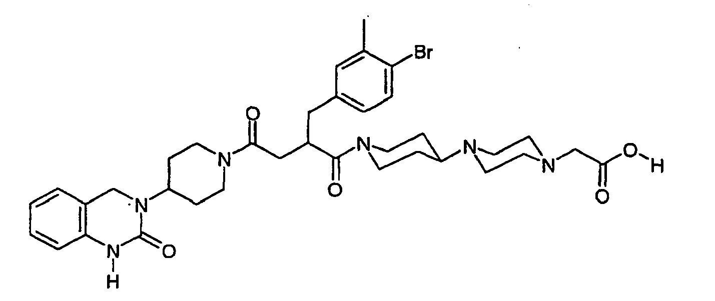

Diese Synthese wurde vom Synthese-Roboter Chemspeed ASW2000 (Chemspeed Ltd., Rheinstraße 32, CH-4302 Augst, Schweiz) durchgeführt.

### Ansatz:

| | |
|---|---|
| AGV 1: | 102 mg (0.20 mmol) 2-(4-Brom-3-methyl-benzyl)-4-oxo-4-[4-(2-oxo-1,4-dihydro-2*H*-chinazolin-3-yl)-piperidin-1-yl]-butansäure in 3 mL THF; |
| AGV 2: | 51 mg (0.20 mmol) (4-Piperidin-4-yl-piperazin-1-yl)-essigsäureethylester in 2 mL THF; |
| AGV 3: | 64 mg (0.20 mmol) TBTU in 2 mL DMF; |
| AGV 4: | 0.14 mL (1.00 mmol) Triethylamin; |
| AGV 5: | 1.00 mL 4 M NaOH; |
| AGV 6: | 1.00 mL 4 M HCl; |
| AGV 7: | 6 mL THF. |

Die AGV's 1 bis 4 wurden entsprechend positioniert, dann vom Roboter zusammenpipettiert und 8 h bei RT geschüttelt. Die Reaktionsgemische wurden eingedampft, mit je 7 mL EtOAc versetzt, die entstandenen Lösungen jeweils mit 10 mL 10% K₂CO₃-Lösung und mit 6 mL Wasser gewaschen und abermals vom Lösungsmittel befreit. Die Rückstände wurden jeweils in AGV 7 gelöst und nach Zugabe von AGV 5 bei RT 6 h gerührt. Die Reaktionsgemische wurden jeweils durch Zugabe von AGV 6 neutralisiert, danach eingedampft. Der erhaltene Rückstand wurde in je 1.9 mL DMF gelöst und auf eine Mikrotiterplatte gegeben. Die Probe wurden mittels HPLC-MS-Anlage (Agilent Technologies, Agilent 1100 Series Modules and Systems for HPLC and LC/MS) getrennt, das Produkt massengesteuert gesammelt. Das Endprodukt wurde gefriergetrocknet.

| | |
|---|---|
| Ausbeute: | 4 mg (3 % der Theorie). |
| ESI-MS: | (M-H)⁻ = 721/723 (Br) |
| | (M+H)⁺ = 723/725 (Br) |

### Beispiel 16.5

### (1'-{2-(4-Brom-3-methyl-benzyl)-4-oxo-4-[4-(2-oxo-1,4-dihydro-2H-chinazolin-3-yl)-piperidin-1-yl]-butyryl}-[4,4']bipiperidinyl-1-yl)-essigsäuremethylester

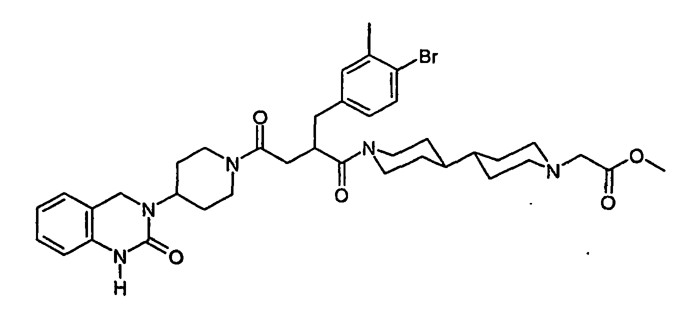

Diese Synthese wurde vom Synthese-Roboter Chemspeed ASW2000 (Chemspeed Ltd., Rheinstraße 32, CH-4302 Augst, Schweiz) durchgeführt.

### Ansatz:

| | |
|---|---|
| AGV 1: | 206 mg (0.40 mmol) 2-(4-Brom-3-methyl-benzyl)-4-oxo-4-[4-(2-oxo-1,4- |
| | dihydro-2*H*-chinazolin-3-yl)-piperidin-1-yl]-butansäure in 3 mL THF; |
| AGV 2: | 102 mg (0.40 mmol) [4,4']Bipiperidinyl-1-yl-essigsäureethylester in 4 mL THF; |
| AGV 3: | 128 mg (0.40 mmol) TBTU in 4 mL DMF; |
| AGV 4: | 0.14 mL (1.00 mmol) Triethylamin; |

Die AGV's 1 bis 4 wurden entsprechend positioniert, dann vom Roboter zusammenpipettiert und 8 h bei RT geschüttelt. Die Reaktionsgemische wurden eingedampft, mit je 7 mL EtOAc und 6 mL 10% K₂CO₃-Lösung versetzt, kräftig geschüttelt, die Wasserphase abgenommen und verworfen. Die organische Phase wurde eingedampft und in 6 mL MeOH gelöst. Ein Drittel dieser Lösung wurde entnommen auf eine Mikrotiterplatte gegeben. Die Probe wurden mittels HPLC-MS-Anlage (Agilent Technologies, Agilent 1100 Series Modules and Systems for HPLC and LC/MS) getrennt, das Produkt massengesteuert gesammelt. Das Endprodukt wurde gefriergetrocknet.

| | |
|---|---|
| Ausbeute: | 9 mg (9 % der Theorie). |
| EI-MS: | (M)⁺ = 735/737 (Br) |
| R_{f} = | 0.38 (Kieselgel, DCM/MeOH 9:1) |

Die restlichen 2/3 der MeOH-Lösung wurden eingedampft und das Rohprodukt (195 mg) in Beispiel 16.6 weiter umgesetzt.

### Beispiel 16.6

### (1'-{2-(4-Brom-3-methyl-benzyl)-4-oxo-4-[4-(2-oxo-1,4-dihydro-2H-chinazolin-3-yl)-piperidin-1-yl]-butyryl}-[4,4']bipiperidinyl-1-yl)-essigsäure

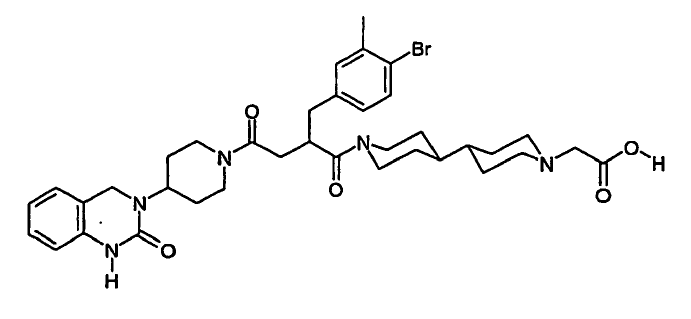

Diese Synthese wurde vom Synthese-Roboter Chemspeed ASW2000 (Chemspeed Ltd., Rheinstraße 32, CH-4302 Augst, Schweiz) durchgeführt.

### Ansatz:

| | |
|---|---|
| AGV 1: | 195 mg (0.26 mmol) (1'-{2-(4-Brom-3-methyl-benzyl)-4-oxo-4-[4-(2-oxo-1,4-dihydro-2*H*-chinazolin-3-yl)-piperidin-1-yl]-butyryl}-[4,4']bipiperidinyl-1-yl)-essigsäuremethylester; |
| AGV 2: | 5 mL MeOH |
| AGV 3: | 1.00 mL 4 M NaOH; |
| AGV 4: | 1.00 mL 4 M HCl; |

AGV 1 wurde in AGV 2 gelöst und anschließend wurde AGV 3 zugegeben. Der Ansatz wurde 5 h bei 20°C geschüttelt und anschließend mit AGV 4 neutralisiert. Das Reaktionsgemisch wurde eingedampft und in 2 mL DMF gelöst. Die Probe wurden mittels HPLC-MS-Anlage (Agilent Technologies, Agilent 1100 Series Modules and Systems for HPLC and LC/MS) getrennt, das Produkt massengesteuert gesammelt. Das Endprodukt wurde gefriergetrocknet.

| | |
|---|---|
| Ausbeute: | 22 mg (11 % der Theorie). |
| ESI-MS: | (M+H)⁺ = 722/724 (Br) |
| R_{f} = | 0.22 (Kieselgel, DCM/MeOH 9:1) |

### Beispiel 17

### 4-(2-Oxo-1,4-dihydro-2H-chinazolin-3-yl)-piperidin-1-carbonsäure{1-(4-chlor-3-methyl-benzyl)-2-[4-(4-methyl-piperazin-1-yl)-piperidin-1-yl]-2-oxo-ethyl}-amid

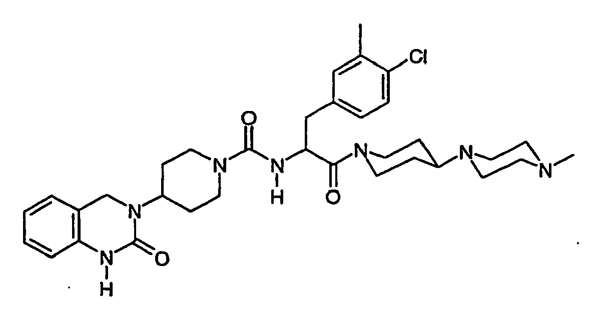

### 17a 2-Amino-3-(4-chlor-3-methyl-phenyl)-propionsäureethylester Hydrochlorid

Analog Beispiel 14a wurde aus 31.4 g (115 mmol) *N*-(Diphenylmethylen)-glycinethylester und 25.2 g (115 mmol) 4-Brommethyl-1-chlor-2-methyl-benzol das Produkt hergestellt.

| | |
|---|---|
| Ausbeute: | 20.4 g (64% der Theorie) |
| ESI-MS: | (M+H)⁺ = 241/243 (Cl) |
| R_{f} = | 0.35 (Kieselgel, DCM/MeOH/NH₃ 9:1:0.1) |

### 17b 3-(4-Chlor-3-methyl-phenyl)-2-{[4-(2-oxo-1,4-dihydro-2H-chinazolin-3-yl)-piperidin-1-carbonyl]-amino}-propionsäureethylester

Analog Beispiel 14b wurde aus 5.56 g (20.0 mmol) 2-Amino-3-(4-chlor-3-methylphenyl)-propionsäureethylester Hydrochlorid und 4.63 (20.0 mmol) 3-Piperidin-4-yl-3,4-dihydro-1*H*-chinazolin-2-on das Produkt erhalten.

| | |
|---|---|
| Ausbeute: | 9.50 g (95% der Theorie) |
| ESI-MS: | (M-H)⁻ = 497/499 (C1) |

### 17c 3-(4-Chlor-3-methyl-phenyl)-2-{[4-(2-oxo-1,4-dihydro-2H-chinazolin-3-yl)-piperidin-1-carbonyl]-amino}-propionsäure

Analog Beispiel 14c wurde aus 9.50 g (19.0 mmol) 3-(4-Chlor-3-methyl-phenyl)-2-{[4-(2-oxo-1,4-dihydro-2*H*-chinazolin-3-yl)-piperidin-1-carbonyl]-amino}-propionsäureethylester das Proukt erhalten.

| | |
|---|---|
| Ausbeute: | 8.90 g (99 % der Theorie) |
| ESI-MS: | (M-H)⁻ = 469/471 (Cl) |
| R_{f} = | 0.10 (Kieselgel, DCM/MeOH 9:1) |

### 17d 4-(2-Oxo-1,4-dihydro-2H-chinazolin-3-yl)-piperidin-1-carbonsäure{1-(4-chlor-3-methyl-benzyl)-2-[4-(4-methyl-piperazin-1-yl)-piperidin-1-yl]-2-oxo-ethyl}-amid

Analog Beispiel 2f wurde aus 706 mg (1.50 mmol) 3-(4-Chlor-3-methyl-phenyl)-2-{[4-(2-oxo-1,4-dihydro-2*H*-chinazolin-3-yl)-pipeddin-1-carbonyl]-amino}-propionsäure und 275 g (1.50 mmol) 1-Methyl-4-piperidin-4-yl-piperazin das Produkt hergestellt.

| | |
|---|---|
| Ausbeute: | 250 mg (26% der Theorie) |
| ESI-MS: | (M+H)⁺ = 636/638 (Cl) |
| R_{f} = | 0.17 (Kieselgel, DCM/MeOH/NH₃ 9:1:0.1) |

Analog wurden folgende Verbindungen aus 3-(4-Chlor-3-methyl-phenyl)-2-{[4-(2-oxo-1,4-dihydro-2*H*-chinazolin-3-yl)-piperidin-1-carbonyl]-amino}-propionsäure und der entsprechenden Menge an Amin hergestellt:

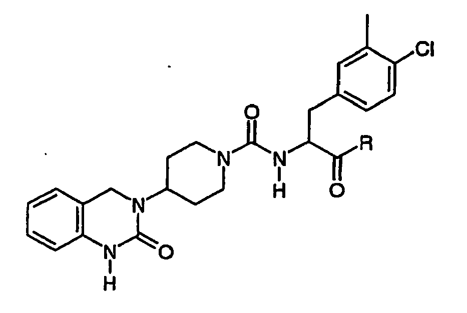

| **Beispiel** | **R** | **Ausbeute (%)** | **Massenspektrum** | **R_{f} (Kieselgel)** |
|---|---|---|---|---|
| **17.1** | 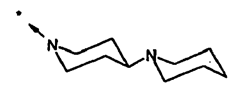 | 54 | 621/623 [M+H]⁺ | 0.33 (DCM/MeOH/NH₃ 9:1:0.1) |
| **17.2** | 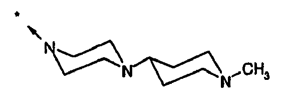 | 35 | 636/638 [M+H]⁺ | 0.18 (DCM/MeOH/NH₃ 9:1:0.1) |
| **17.3** | 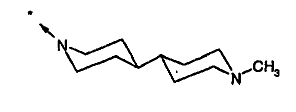 | 27 | 635/637 [M+H]⁺ | 0.26 (DCM/MeOH/NH₃ 9:1:0.1) |
| **17.4** | 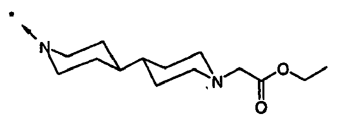 | 24 | 707/709 [M+Na]⁺ | 0.49 (DCM/MeOH 9:1) |
| **17.5** | 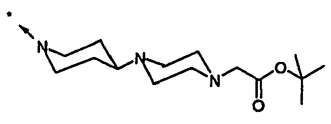 | 37 | 736/738 [M+Na]⁺ | 0.39 (DCM/MeOH 9:1) |
| **17.6** | 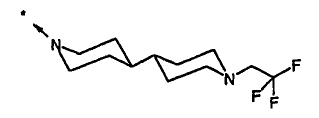 | 37 | 703/705 [M+Na]⁺ | 0.36 (DCM/MeOH/NH₃ |
| **17.7** | 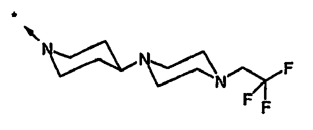 | 49 | 704/706 [M+Na]⁺ | 0.40 (DCNUMeOH/NH₃ 9:1:0.1) |

### Beispiel 18

### [1'-(3-(4-Chlor-3-methyl-phenyl)-2-{[4-(2-oxo-1,4-dihydro-2H-chinazolin-3-yl)-piperidin-1-carbonyl]-amino}-propionyl)-[4,4']bipiperidinyl-1-yl]-essigsäure

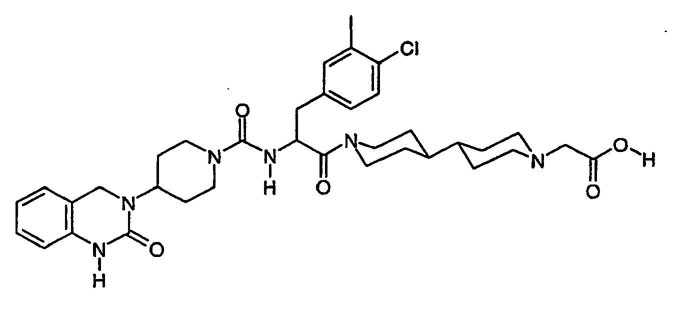

Analog Beispiel 15 wurde aus 220 mg (0.28 mmol) [1 '-(3-(4-Chlor-3-methyl-phenyl)-2-{[4-(2-oxo-1,4-dihydro-2*H*-chinazolin-3-yl)-piperidin-1-carbonyl]-amino}-propionyl)-[4,4']bipiperidinyl-1-yl]-essigsäureethylester das Produkt hergestellt.

| | |
|---|---|
| Ausbeute: | 190 mg (99 % der Theorie) |
| ESI-MS: | (M+H)⁺ = 679/681 (Cl) |
| R_{f} = | 0.13 (Kieselgel, DCM/MeOH 9:1) |

### Beispiel 19

### 2-(4-Chlor-3-methyl-benzyl)-1-[4-(4-ethyl-piperazin-1-yl)-piperidin-1-yl]-4-[4-(2-oxo-1,4-dihydro-2H-chinazolin-3-yl)-piperidin-1-yl]-butan-1,4-dion

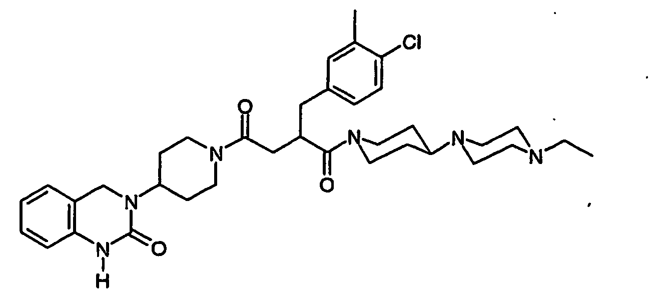

### 19a 2-(4-Chlor-3-methyl-benzyl)-2-ethoxycarbonyl-bernsteinsäure-4-tert-butylester-1-ethylester

Analog Beispiel 2b wurde aus 19.5 g (71.0 mmol) 2-Ethoxycarbonyl-bernsteinsäure-4-*tert*-butylester-1-ethylester und 15.5 g (71.0 mmol) 4-Brommethyl-1-chlor-2-methyl-benzol das Produkt hergestellt.

| | |
|---|---|
| Ausbeute: | 25.7 g (88% der Theorie) |
| R_{f} = | 0.74 (Kieselgel, DCM) |

### 19b 2-(4-Chlor-3-methyl-benzyl)-2-ethoxycarbonyl-bernsteinsäureethylester

Analog Beispiel 2c wurde aus 21.3 g (41.7 mmol) 2-(4-Chlor-3-methyl-benzyl)-2-ethoxycarbonyl-bernsteinsäure-4-*tert*-butylester-1-ethylester das Produkt hergestellt.

| | |
|---|---|
| Ausbeute: | 22.2 g (100% der Theorie) |
| R_{f} = | 0.18 (Kieselgel, DCM) |

### 19c 2-(4-Chlor-3-methyl-benzyl)-2-{2-oxo-2-[4-(2-oxo-1,4-dihydro-2H-chinazolin-3-yl)-piperidin-1-yl]-ethyl}-malonsäurediethylester

Analog Beispiel 2d wurde aus 8.00 g (22.4 mmol) 2-(4-Chlor-3-methyl-benzyl)-2-ethoxycarbonyl-bernsteinsäureethylester und 5.18 g (22.4 mmol) 3-Piperidin-4-yl-3,4-dihydro-1 *H*-chinazolin-2-on das Produkt hergestellt.

| | |
|---|---|
| Ausbeute: | 9.20 g (72% der Theorie) |
| EI-MS: | (M)⁺ = 569/570 (Cl) |
| R_{f} = | 0.64 (Kieselgel, DCM/MeOH/NH₃ 9:1:0.1) |

### 19d 2-(4-Chlor-3-methyl-benzyl)-4-oxo-4-[4-(2-oxo-1,4-dihydro-2H-chinazolin-3-yl)-piperidin-1-yl]-butansäure

Analog Beispiel 2e wurde aus 9.20 g (16.2 mmol) 2-(4-Chlor-3-methyl-benzyl)-2-{2-oxo-2-[4-(2-oxo-1,4-dihydro-2*H*-chinazolin-3-yl)-piperidin-1-yl]-ethyl}-malonsäure-diethylester das Produkt hergestellt.

| | |
|---|---|
| Ausbeute: | 7.20 g (95% der Theorie) |
| ESI-MS: | (M-H)⁻ = 468/470 (CI) |

### 19e 2-(4-Chlor-3-methyl-benzyl)-1-[4-(4-ethyl-piperazin-1-yl)-piperidin-1-yl]-4-[4-(2-oxo-1,4-dihydro-2H-chinazolin-3-yl)-piperidin-1-yl]-butan-1,4-dion

Analog Beispiel 2f wurde aus 470 mg (1.00 mmol) 2-(4-Chlor-3-methyl-benzyl)-4-oxo-4-[4-(2-oxo-1,4-dihydro-2*H*-chinazolin-3-yl)-piperidin-1-yl]-butansäure und 593 mg (1.10 mmol) 1-Ethyl-4-piperidin-4-yl-piperazin Tris-Trifluoracetat das Produkt hergestellt.

| | |
|---|---|
| Ausbeute: | 280 mg (43% der Theorie) |
| EI-MS: | (M)⁺ = 648/650 (CI) |
| Rf = | 0.47 (Kieselgel, DCM/MeOH/NH₃ 9:1:0.1) |

Analog wurden folgende Verbindungen aus 2-(4-Chlor-3-methyl-benzyl)-4-oxo-4-[4-(2-oxo-1,4-dihydro-2*H*-chinazolin-3-yl)-piperidin-1-yl]-butansäure und der entsprechenden Menge an Amin hergestellt:

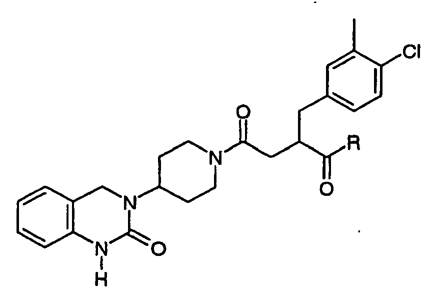

| **Beispiel** | **R** | **Ausbeute (%)** | **Massenspektrum** | **R_{f} (Kieselgel)** |
|---|---|---|---|---|
| **19.1** | 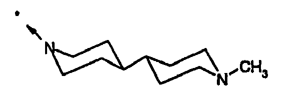 | 48 | 634/636 [M]⁺ | 0.49 (DCM/Cyc/MeOH/NH₃ 70:15:15:2) |
| **19.2** | 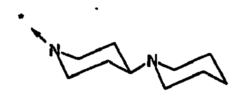 | 32 | 620/622 [M+H]⁺ | 0.54 (DCM/Cyc/MeOH/NH₃ 70:15:15:2) |
| **19.3** | 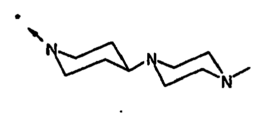 | 44 | 635/637 [M+H]⁺ | 0.50 (DCM/Cyc/MeOH/NH₃ 70:15:15:2) |
| **19.4** | 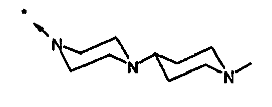 | 39 | 635/637 [M+H]⁺ | 0.49 (DCM/Cyc/MeOH/NH₃ 70:15:15:2) |
| **19.5** | 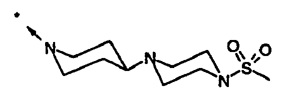 | 57 | 698/700 [M]⁺ | 0.54 (DCM/MeOH/NH₃ 9:1:0.1) |
| **19.6** | 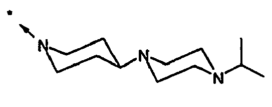 | 50 | 662/664 [M]⁺ | 0.48 (DCM/MeOH/NH₃ 9:1:0.1) |
| **19.7** | 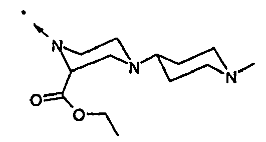 | 1 | 707/709 [M+H]⁺ | 0.76 (DCM/MeOH/NH₃ 8:2:0.2) |
| **19.8** | 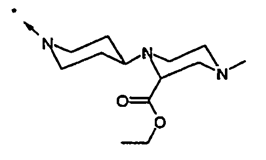 | 30 | 707/709 [M+H]⁺ | 0.77 (DCM/MeOH/NH₃ 8:2:0.2) |
| **19.9** | 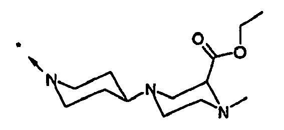 | 11 | 707/709 [M+H]+ | 0.56 (DCM/MeOH/NH₃ 8:2:0.2) |
| **19.10** | 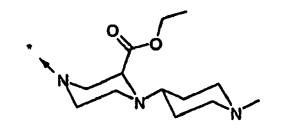 | 8 | 707/709 [M+H]⁺ | 0.56 (EtOAc/MeOH/NH₃ 8:2:0.2) |
| **19.11** | 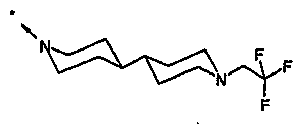 | 30 | 702/704 [M+H]⁺ | 0.36 (DCM/MeOH/NH₃ 9:1:0.1) |
| **19.12** | 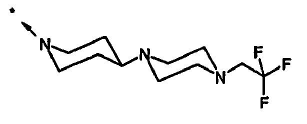 | 41 | 703/705 [M+H]⁺ | 0.72 (DCM/MeOH/NH₃ 8:2:0.1) |

### Beispiel 19.13

### [4-(1-{2-(4-Chlor-3-methyl-benzyl)-4-oxo-4-[4-(2-oxo-1,4-dihydro-2H-chinazolin-3-yl)-piperidin-1-yl]-butyryl}-piperidin-4-yl)-piperazin-1-yl]-essigsäure

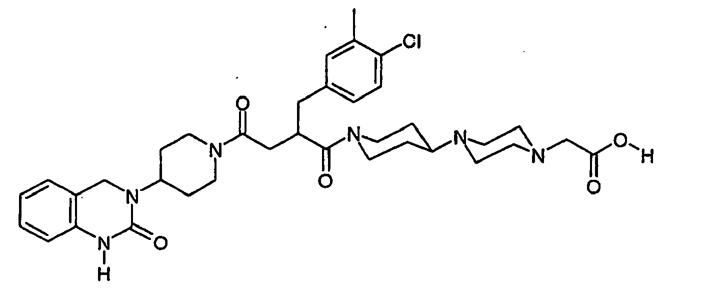

Analog Beispiel 16.4 wurde aus 94 mg (0.20 mmol) 2-(4-Chlor-3-methyl-benzyl)-4-oxo-4-[4-(2-oxo-1,4-dihydro-2*H*-chinazolin-3-yl)-piperidin-1-yl]-butansäure und 51 mg (0.20 mmol) (4-Piperidin-4-yl-piperazin-1-yl)-essigsäureethylester das Produkt erhalten.

| | |
|---|---|
| Ausbeute: | 27 mg (19 % d. Theorie) |
| EI-MS: | (M)⁺ = 679/681 (Cl) |
| Retentionszeit (HPLC): | 5.9 min (Methode A) |

### Beispiel 19.14

### (1'-{2-(4-Chlor-3-methyl-benzyl)-4-oxo-4-[4-(2-oxo-1,4-dihydro-2H chinazofin-3-yl)-piperidin-1-yl]-butyryl}-[4,4']bipiperidinyl-1-yl)-essigsäuremethylester

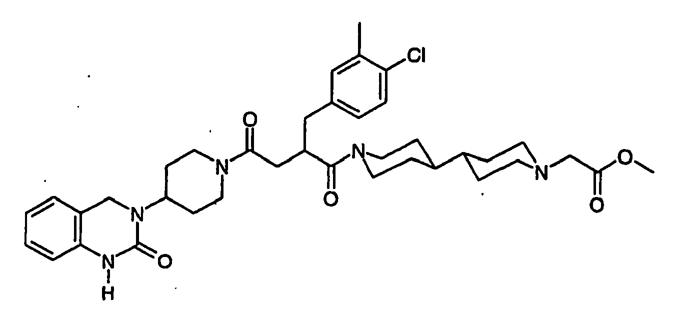

Analog Beispiel 16.5 wurde aus 188 mg (0.40 mmol) 2-(4-Chlor-3-methyl-benzyl)-4-oxo-4-[4-(2-oxo-1,4-dihydro-2*H*-chinazolin-3-yl)-piperidin-1-yl]-butansäure und 102 mg (0.40 mmol) [4,4']Bipiperidinyl-1-yl-essigsäureethylester das Produkt erhalten.

| | |
|---|---|
| Ausbeute: | 27 mg (30 % der Theorie) |
| ESI-MS: | (M+H)⁺ = 692/694 (CI) |
| R_{f} = | 0.36 (Kieselgel, DCM/MeOH 9:1) |

### Beispiel 19.15

### (1'-{2-(4-Chlor-3-methyl-benzyl)-4-oxo-4-[4-(2-oxo-1,4-dihydro-2H chinazolin-3-yl)-piperidin-1-yl]-butyryl}-[4,4']bipiperidinyl-1-yl)-essigsäure

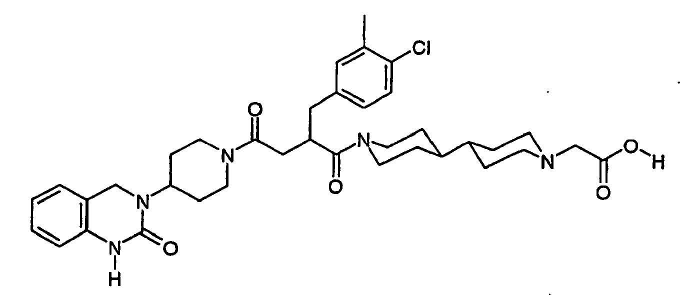

Analog Beispiel 16.6 würde aus 184 mg (0.26 mmol) (1'-{2-(4-Chlor-3-methylbenzyl)-4-oxo-4-[4-(2-oxo-1,4-dihydro-2*H*-chinazolin-3-yl)-piperidin-1-yl]-butyryl}-[4,4']bipiperidinyl-1-yl)-essigsäuremethylester das Produkt erhalten.

| | |
|---|---|
| Ausbeute: | 30 mg (16 % der Theorie) |
| ESI-MS: | (M+H)⁺ = 678/680 (Cl) |
| R_{f} = | 0.21 (Kieselgel, DCM/MeOH 9:1) |

Analog Beispiel 15 wurden folgende Verbindungen aus den jeweiligen Ethylestern (Beispiele 19.7 bis 19.9) hergestellt:

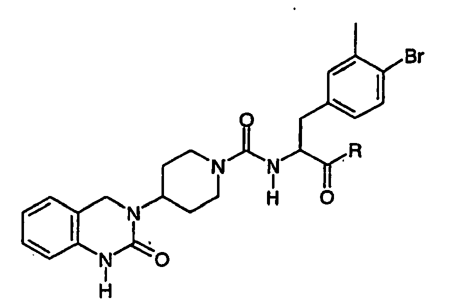

| **Beispiel** | **R** | **Ausbeute (%)** | **Massenspektrum** | **R_{f} (Kieselgel)** |
|---|---|---|---|---|
| **19.16** | 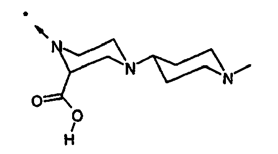 | 18 | 679/681 [M+H]⁺ | 0.05 (EtOAc/MeOH/NH₃ 6:4:0.4) |
| **19.17** | 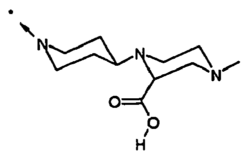 | 50 | 679/681 [M+H]⁺ | 0.21 (DCM/MeOH/NH₃ 8:2:0.2) |
| **19.18** | 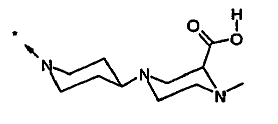 | 48 | 679/681 [M+H]⁺ | 0.14 (EtOAc/MeOH/NH₃ 6:4:0.4) |

### Beispiel 20

### 2-(4-Chlor-3-methyl-benzyl)-1-[4-(1-methyl-piperidin-4-yl)-piperazin-1-yl]-4-[4-(2-oxo-1,2,4,5-tetrahydro-1,3-benzdiazepin-3-yl)-piperidin-1-yl]-butan-1,4-dion

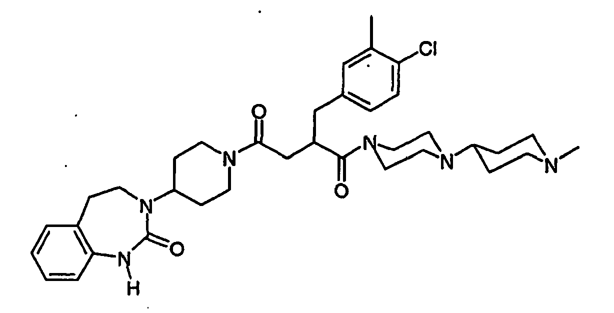

### 20a 2-(4-Chlor-3-methyl-benzyl)-2-{2-oxo-2-[4-(2-oxo-1,2,4,5-tetrahydro-1,3-benzdiazepin-3-yl)-piperidin-1-yl]-ethyl}-malonsäurediethylester

Analog Beispiel 2d wurde aus 1.43 g (4.00 mmol) 2-(4-Chlor-3-methyl-benzyl)-2-ethoxycarbonyl-bernsteinsäureethylester und 981 mg (4.00 mmol) 3-(1-Methylpiperidin-4-yl)-1,3,4,5-tetrahydro-1,3-benzdiazepin-2-on das Produkt hergestellt.

| | |
|---|---|
| Ausbeute: | 2.10 g (90% der Theorie) |
| R_{f} = | 0.69 (Kieselgel, DCM/Cyc/MeOH/NH₃ 70:15:15:2) |

### 20b 2-(4-Chlor-3-methyl-benzyl)-4-oxo-4-[4-(2-oxo-1,2,4,5-tetrahydro-1,3-benzdiazepin-3-yl)-piperidin-1-yl]-butansäure

Analog Beispiel 2e wurde aus 2.10 g (3.60 mmol) 2-(4-Chlor-3-methyl-benzyl)-2-{2-oxo-2-[4-(2-oxo-1,2,4,5-tetrahydro-1,3-benzdiazepin-3-yl)-piperidin-1-yl]-ethyl}-malonsäurediethylester das Produkt hergestellt. Das Produkt wurde ohne Reinigung weiter umgesetzt.

| | |
|---|---|
| Ausbeute: | 1.20 g (69 % der Theorie) |

### 20c 2-(4-Chlor-3-methyl-benzyl)-1-[4-(1-methyl-piperidin-4-yl)-piperazin-1-yl]-4-[4-(2-oxo-1,2,4,5-tetrahydro-1,3-benzdiazepin-3-yl)-piperidin-1-yl]-butan-1,4-dion

Analog Beispiel 2f wurde aus 800 mg (1.65 mmol) 2-(4-Chlor-3-methyl-berizyl)-4-oxo-4-[4-(2-oxo-1,2,4,5-tetrahydro-1,3-benzdiazepin-3-yl)-piperidin-1-yl]-butansäure und 302 mg (1.65 mmol) 1-(1-Methyl-piperidin-4-yl)-piperazin das Produkt hergestellt.

| | |
|---|---|
| Ausbeute: | 400 mg (37% der Theorie) |
| EI-MS: | (M)⁺ = 648/650 (Cl) |
| R_{f} = | 0.50 (Kieselgel, DCM/Cyc/MeOH/NH₃ 70:15:15:2) |

### Beispiel 20.1

### 2-(4-Chlor-3-methyl-benzyl)-1-[4-(4-methyl-piperazin-1-yl)-piperidin-1-yl]-4-[4-(2-oxo-1,2,4,5-tetrahydro-1,3-benzdiazepin-3-yl)-piperidin-1-yl]-butan-1,4-dion

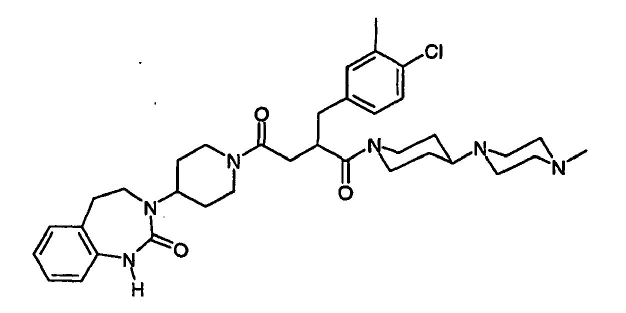

Analog Beispiel 2f wurde aus 400 mg (0.83 mmol) 2-(4-Chlor-3-methyl-benzyl)-4-oxo-4-[4-(2-oxo-1,2,4,5-tetrahydro-1,3-benzdiazepin-3-yl)-piperidin-1-yl]-butansäure und 152 mg (0.83 mmol) 1-Methyl-4-piperidin-4-yl-piperazin das Produkt hergestellt.

| | |
|---|---|
| Ausbeute: | 200 mg (37% der Theorie) |
| EI-MS: | (M)⁺ = 648/650 (CI) |
| R_{f} = | 0.51 (Kieselgel, DCM/Cyc/MeOH/NH₃ 70:15:15:2) |

### Beispiel 21

### [4-(1-{2-(4-Chlor-3-methyl-benzyl)-4-oxo-4-[4-(5-oxo-3-phemyl-4,5-dihydro-1,2,4-triazol-1-yl)-piperidin-1-yl]-butyryl}-piperidin-4-yl)-piperazin-1-yl]-essigsäure

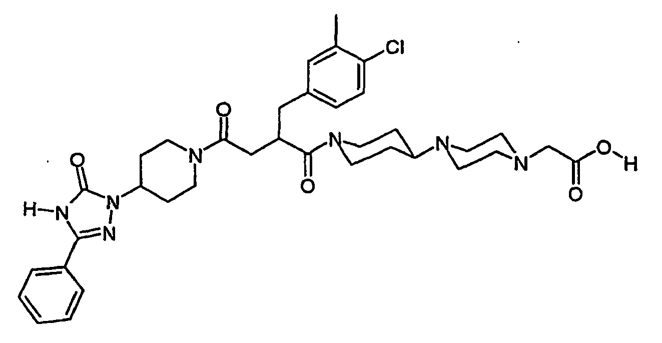

### 21a 2-(4-Chlor-3-methyl-benzyl)-2-{2-oxo-2-[4-(5-oxo-3-phenyl-4,5-dihydro-1,2,4-triazol-1-yl)-piperidin-1-yl]-ethyl}-malonsäurediethylester

Analog Beispiel 2d wurde aus 5.00 g (14.0 mmol) 2-(4-Chlor-3-methyl-benzyl)-2-ethoxycarbonyl-bernsteinsäureethylester und 3.42 g (14.0 mmol) 5-Phenyl-2-piperidin-4-yl-2,4-dihydro-1,2,4-triazol-3-on das Produkt hergestellt.

| | |
|---|---|
| Ausbeute: | 5.50 g (67 % der Theorie) |
| R_{f} = | 0.50 (Kieselgel, DCM/MeOH/NH₃ 9:1:0.1) |

### 21 b 2-(4-Chlor-3-methyl-benzyl)-4-oxo-4-[4-(5-oxo-3-phenyl-4,5-dihydro-1,2,4-triazol-1-yl)-piperidin-1-yl]-butansäure

Analog Beispiel 2e wurde aus 5.50 g (9.43 mmol) 2-(4-Chlor-3-methyl-benzyl)-2-{2-oxo-2-[4-(5-oxo-3-phenyl-4,5-dihydro-1,2,4-triazol-1-yl)-piperidin-1-yl]-ethyl}-malonsäurediethylester das Produkt hergestellt.

| | |
|---|---|
| Ausbeute: | 2.80 g (62 % der Theorie) |
| ESI-MS: | (M-H)⁻ = 481/483 (Cl) |

### 21c [4-(1-{2-(4-Chlor-3-methyl-benzyl)-4-oxo-4-[4-(5-oxo-3-phenyl-4,5-dihydro-1,2,4-triazol-1-yl)-piperidin-1-yl]-butyryl}-piperidin-4-yl)-piperazin-1-yl]-essigsäure

Analog Beispiel 16.4 wurde aus 96 mg (0.20 mmol) 2-(4-Chlor-3-methyl-benzyl)-4-oxo-4-[4-(5-oxo-3-phenyl-4,5-dihydro-1,2,4-triazol-1-yl)-piperidin-1-yl]-butansäure und 51 mg (0.20 mmol) (4-Piperidin-4-yl-piperazin-1-yl)-essigsäureethylester das Produkt erhalten.

| | |
|---|---|
| Ausbeute: | 3 mg (2 % der Theorie) |
| ESI-MS: | (M+H)⁺ = 692/694 (Cl) |

### Beispiel 21.1

### (1'-{2-(4-Chlor-3-methyl-benzyl)-4-oxo-4-[4-(5-oxo-3-phenyl-4,5-dihydro-1,2,4-triazol-1-yl)-piperidin-1-yl]-butyryl}-4,4'-bipiperidinyl-1-yl)-essigsäuremethylester

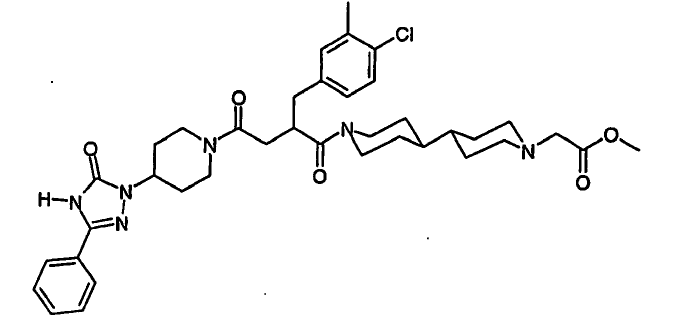

Analog Beispiel 16.5 wurde aus 193 mg (0.40 mmol) 2-(4-Chlor-3-methyl-benzyl)-4-oxo-4-[4-(5-oxo-3-phenyl-4,5-dihydro-1,2,4-triazol-1-yl)-piperidin-1-yl]-butansäure und 102 mg (0.40 mmol) [4,4']Bipiperidinyl-1-yl-essigsäureethylester das Produkt erhalten.

| | |
|---|---|
| Ausbeute: | 14 mg (15 % der Theorie) |
| ESI-MS: | (M+H)⁺ = 705/707 (Cl) |
| R_{f} = | 0.32 (Kieselgel, DCM/MeOH 9:1) |

### Beispiel 21.2

### (1'-{2-(4-Chlor-3-methyl-benzyl)-4-oxo-4-[4-(5-oxo-3-phenyl-4,5-dihydro-1,2,4-triazol-1-yl)-piperidin-1-yl]-butyryl}-4,4'-bipiperidinyl-1-yl)-essigsäure

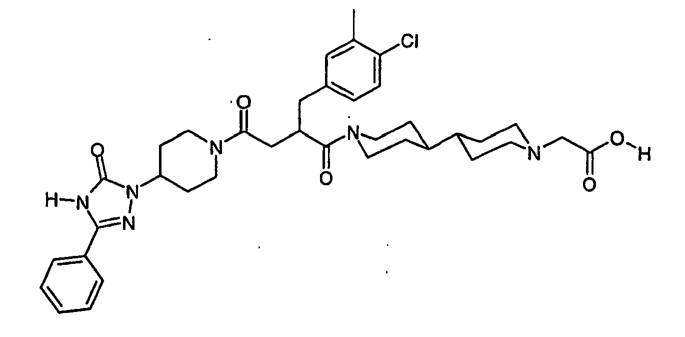

Analog Beispiel 16.6 wurde aus 187 mg (0.26 mmol) (1'-{2-(4-Chlor-3-methylbenzyl)-4-oxo-4-[4-(5-oxo-3-phenyl-4,5-dihydro-1,2,4-triazol-1-yl)-piperidin-1-yl]-butyryl}-4,4'-bipiperidinyl-1-yl)-essigsäuremethylester das Produkt erhalten.

| | |
|---|---|
| Ausbeute: | 11 mg (6 % der Theorie) |
| ESI-MS: | (M+H)⁺ = 691/693 (Cl) |
| R_{f} = | 0.21 (Kieselgel, DCM/MeOH 9:1) |

### Beispiel 22

### 2-(3-Brom-4-chlor-5-methyl-benzyl)-1-[4-(4-methyl-piperazin-1-yl)-piperidin-1-yl]-4-[4-(2-oxo-1,4-dihydro-2H-chinazolin-3-yl)-piperidin-1-yl]-butan-1,4-dion

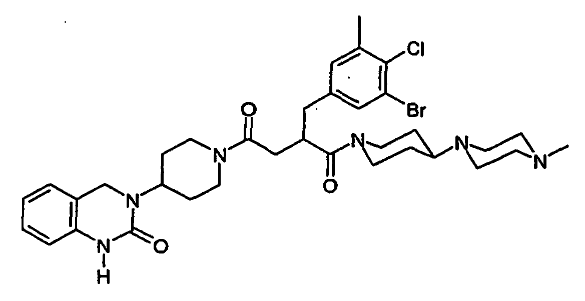

### 22a 1-(3-Brom-4-chlor-5-methyl-phenyl)-ethanon

Zu 59.2 g (444 mmol) Aluminiumtrichlorid wurden 25.0 g (148 mmol) 1-(4-Chlor-3-methyl-phenyl)-ethanon zugetropft. Es kam zu einem Temperaturanstieg auf 70°C. Das Gemisch wurde 30 min bei 80°C gerührt und dann wurden bei dieser Temperatur 10.7 mL (170 mmol) Brom zugetropft. Die Reaktionslösung wurde 1 h bei 80°C gerührt und dann auf Eis gegeben. Die wässrige Phase wurde mit Diethylether extrahiert und die vereinigten organischen Extrakte mit gesättigter NaHCO₃-Lösung gewaschen. Die organische Phase wurde über Na₂SO₄ getrocknet und das Lösungsmittel i.vac. entfernt. Eine Reinigung erfolgte durch Säulenchromatographie an Kieselgel (Toluol).

| | |
|---|---|
| Ausbeute: | 14.0 g (38% der Theorie) |
| EI-MS: | (M)⁺ = 246/248/250 (Br, Cl) |
| R_{f} = | 0.28 (Kieselgel, Toluol) |

### 22b 3-Brom-4-chlor-5-methyl-benzoesäure

Zu einer Lösung von 22.8 g (570 mmol) NaOH in 114 mL Wasser wurde bei 0°C 8.7 mL (171 mmol) Brom so zugetropft, dass die Temperatur 10°C nicht überstieg. 14.0 g (57.0 mmol) 1-(3-Brom-4-chlor-5-methyl-phenyl)-ethanon in 57 mL 1,4-Dioxan wurde bei 10°C zugetropft und das Gemisch 2 h bei RT gerührt. Das Reaktionsgemisch wurde mit Wasser verdünnt und das entstandene Bromoform abgetrennt. Die wässrige Phase wurde mit halbkonz. HCI sauer gestellt, der Niederschlag abgesaugt und dieser mit Wasser nach gewaschen.

| | |
|---|---|
| Ausbeute: | 11.0 g (78 % der Theorie) |
| EI-MS: | (M)⁺ = 248/250/252 (Br, Cl) |
| Schmelzpunkt: | 207-209°C |

### 22c (3-Brom-4-chlor-5-methyl-phenyl)-methanol

Zu einer Lösung von 11.0 g (44 mmol) 3-Brom-4-chlor-5-methyl-benzoesäure in 285 mL THF wurden bei RT 8.1 g (50 mmol) CDl gegeben. Das Reaktionsgemisch wurde 1 h bei 40°C gerührt. Diese Lösung wurde zu einer Lösung von 5.38 g (142 mmol) NaBH₄ in 47.5 mL Wasser gegeben. Das Gemisch wurde 3 h bei RT gerührt, dann mit 300 mL Wasser verdünnt und mit halbkonz. HCl sauer gestellt. Die wässrige Phase wurde mit EtOAc extrahiert und die organische Phase nacheinander mit Wasser und gesättigter NaHCO₃-Lösung gewaschen. Die organische Phase wurde über Na₂SO₄ getrocknet und das Lösungsmittel i.vac. entfernt.

| | |
|---|---|
| Ausbeute: | 9.00 g (87% der Theorie) |
| ESl-MS: | (M-H)⁻ = 233/235/237 (Br, CI) |
| R_{f} = | 0.62 (Kieselgel, PE/EtOAc 1:1) |

### 22d 1-Brom-5-brommethyl-2-chlor-3-methyl-benzol

Zu einer Lösung von 9.00 g (38 mmol) (3-Brom-4-chlor-5-methyl-phenyl)-methanol in 250 mL Diethylether wurde bei RT 5.2 mL (19 mmol) Phosphortribromid zugetropft und 1 h unter Rückfluß erhitzt. Das Reaktionsgemisch wurde auf gesättigte NaHCO₃-Lösung gegeben, die organische Phase abgetrennt, mit Wasser gewaschen und über Na₂SO₄ getrocknet. Nach Entfernen des Trocken- und Lösungsmittels erhielt man das gewünschte Produkt.

| | |
|---|---|
| Ausbeute: | 10.7 g (94% der Theorie) |
| EI-MS: | (M)⁺ = 296/298/300/302 (2Br, CI) |
| R_{f} = | 0.89 (Kieselgel, PE/EtOAc 1:1) |

### 22e 2-(3-Brom-4-chlor-5-methyl-benzyl)-2-ethoxycarbonyl-bernsteinsäure-4-tert-butylester-1-ethylester

Analog Beispiel 2b wurde aus 9.86 g (36 mmol) 2-Ethoxycarbonyl-bemsteinsäure-4-*tert*-butylester-1-ethylester und 10.7 g (36 mmol) 1-Brom-5-brommethyl-2-chlor-3-methyl-benzol das Produkt hergestellt.

| | |
|---|---|
| Ausbeute: | 17.5 g (99% der Theorie) |
| ESI-MS: | (M+H)⁺ = 513/515/517 (Br, Cl) |
| R_{f} = | 0.57 (Kieselgel, DCM) |

### 22f 2-(3-Brom-4-chlor-5-methyl-benzyl)-2-ethoxycarbonyl-bernsteinsäure-1-ethylester

Analog Beispiel 2c wurde aus 18.0 g (37 mmol) 2-(3-Brorn-4-chlor-5-methyl-benzyl)-2-ethoxycarbonyl-bemsteinsäure-4-*tert*-butylester-1-ethylester das Produkt hergestellt. Das Rohprodukt, das noch TFA enthielt wurde ohne Reinigung weiter umgesetzt.

| | |
|---|---|
| ESI-MS: | (M-H)⁻ = 433/435/437 (Br, Cl) |

### 22g 2-(3-Brom-4-chlor-5-methyl-benzyl)-2-{2-oxo-2-[4-(2-oxo-1,4-dihydro-2H-chinazolin-3-yl)-piperidin-1-yl]-ethyl}-malonsäuredlethylester

Analog Beispiel 2d wurde aus 18.2 g (42 mmol) 2-(3-Brom-4-chlor-5-methyl-benzyl)-2-ethoxycarbonyl-bernsteinsäure-1-ethylester und 9.60 g (42 mmol) 3-Piperidin-4-yl-3,4-dihydro-1*H*-chinazolin-2-on das Produkt hergestellt.

| | |
|---|---|
| Ausbeute: | 15.0 g (56% der Theorie) |
| EI-MS: | (M)⁺ = 647/649/651 (Br, Cl) |
| R_{f} = | 0.60 (Kieselgel, DCM/MeOH/NH₃ 9:1:0.1) |

### 22h 2-(3-Brom-4-chlor-5-methyl-benzyl)-4-oxo-4-[4-(2-oxo-1,4-dihydro-2H-chinazolin-3-yl)-piperidin-1-yl]-butansäure

Analog Beispiel 2e wurde aus 15.0 g (23 mmol) 2-(3-Brom-4-chlor-5-methyl-benzyl)-2-{2-oxo-2-[4-(2-oxo-1,4-dihydro-2*H*-chinazolin-3-yl)-piperidin-1-yl]-ethyl}-malonsäurediethylester das Produkt hergestellt.

| | |
|---|---|
| Ausbeute: | 11.8 g (93% der Theorie) |
| R_{f} = | 0.20 (Kieselgel, EtOAc/MeOH/Essigsäure 8:2:0.1) |

### 22i 2-(3-Brom-4-chlor-5-methyl-benzyl)-1-[4-(4-methyl-piperazin-1-yl)-piperidin-1-yl]-4-[4-(2-oxo-1,4-dihydro-2H-chinazolin-3-yl)-piperidin-1-yl]-butan-1,4-dion

Analog Beispiel 2f wurde aus 1.09 g (2.00 mmol) 2-(3-Brom-4-chlor-5-methylbenzyl)-4-oxo-4-[4-(2-oxo-1,4-dihydro-2*H*-chinazolin-3-yl)-piperidin-1-yl]-butansäure und 367 mg (2.00 mmol) 1-Methyl-4-piperidin-4-yl-piperazin das Produkt hergestellt.

| | |
|---|---|
| Ausbeute: | 773 mg (54% der Theorie) |
| EI-MS: | (M)⁺ = 712/714/716 (Br, Cl) |
| R_{f} = | 0.24 (Kieselgel, DCM/MeOH/NH₃ 9:1:0.1) |

Analog wurden folgende Verbindungen aus 2-(3-Brom-4-chlor-5-methyl-benzyl)-4-oxo-4-[4-(2-oxo-1,4-dihydro-2*H*-chinazolin-3-yl)-piperidin-1-yl]-butansäure und der entsprechenden Menge an Amin hergestellt:

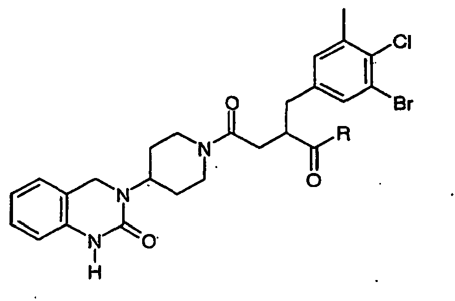

| **Beispiel** | **R** | **Ausbeute (%)** | **Massenspektrum** | **R_{f} (Kieselgel)** |
|---|---|---|---|---|
| **22.1** | 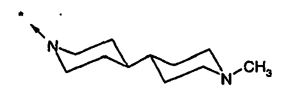 | 74 | 711/13/15 [M]⁺ | 0.63 (DCM/MeOH/NH₃ 8:2:0.1) |
| **22.2** | 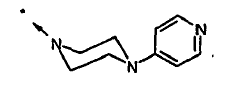 | 56 | 692/94/96 [M]⁺ | 0.26 (DCM/MeOH/NH₃ 9:1:0.1) |
| **22.3** | 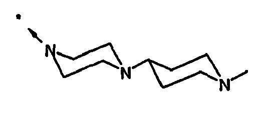 | 13 | 713/15/17 [M+H]⁺ | 0.43 (DCM/MeOH/NH₃ 8:2:0.1) |

### Beispiel 22.4

### [4-(1-{2-(3-Brom-4-chlor-5-methyl-benzyl)-4-oxo-4-[4-(2-oxo-1,4-dihydro-2H-chinazolin-3-yl)-piperidin-1-yl]-butyryl}-piperidin-4-yl)-piperazin-1-yl]-essigsäure

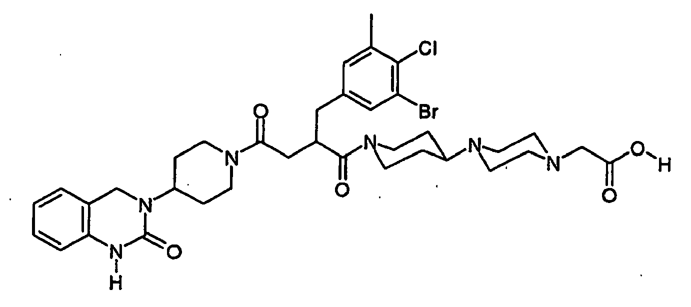

Analog Beispiel 16.4 wurde aus 109 mg (0.20 mmol) 2-(3-Brom-4-chlor-5-methylbenzyl)-4-oxo-4-[4-(2-oxo-1,4-dihydro-2*H*-chinazolin-3-yl)-piperidin-1-yl]-butansäure und 51 mg (0.20 mmol) (4-Piperidin-4-yl-piperazin-1-yl)-essigsäureethylester das Produkt erhalten.

| | |
|---|---|
| Ausbeute: | 10 mg (6% der Theorie) |
| ESI-MS: | (M-H)⁻ = 755/757/759 (Br, Cl) |
| R_{f} = | 0.21 (Kieselgel, DCM/MeOH 9:1) |

### Beispiel 22.5

### (1'-{2-(3-Brom-4-Chlor-5-methyl-benzyl)-4-oxo-4-[4-(2-oxo-1,4-dihydro-2H-chinazolin-3-yl)-piperidin-1-yl]-butyryl}-[4,4']bipiperidinyl-1-yl)-essigsäuremethylester

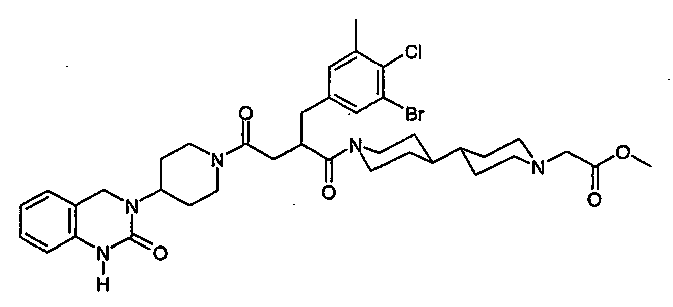

Analog Beispiel 16.5 wurde aus 220 mg (0.40 mmol) 2-(3-Brom-4-Chlor-5-methylbenzyl)-4-oxo-4-[4-(2-oxo-1,4-dihydro-2*H*-chinazolin-3-yl)-piperidin-1-yl]-butansäure und 102 mg (0.40 mmol) [4,4']Bipiperidinyl-1-yl-essigsäureethylester das Produkt erhalten.

| | |
|---|---|
| Ausbeute: | 19 mg (18 % der Theorie) |
| ESI-MS: | (M+H)⁺ = 770/772/774 (Br, Cl) |
| R_{f} = | 0.36 (Kieselgel, DCM/MeOH 9:1) |

### Beispiel 22.6

### (1'-{2-(3-Brom-4-Chlor-5-methyl-benzyl)-4-oxo-4-[4-(2-oxo-1,4-dihydro-2H-chinazolin-3-yl)-piperidin-1-yl]-butyryl}-[4,4']bipiperidinyl-1-yl)-essigsäure

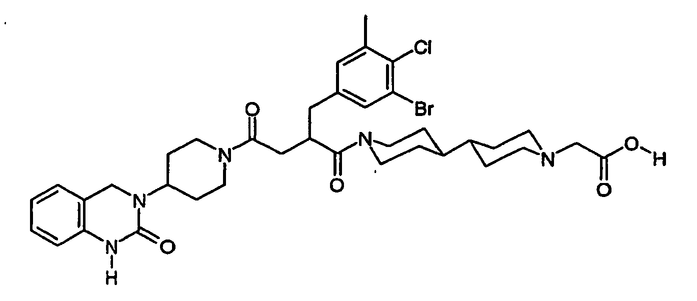

Analog Beispiel 16.6 wurde aus 204 mg (0.26 mmol) (1'-{2-(3-Brom-4-Chlor-5-methyl-benzyl)-4-oxo-4-[4-(2-oxo-1,4-dihydro-2*H*-chinazolin-3-yl)-piperidin-1-yl]-butyryl}-[4,4']bipiperidinyl-1-yl)-essigsäuremethylester das Produkt erhalten.

| | |
|---|---|
| Ausbeute: | 25 mg (12 % der Theorie) |
| ESI-MS: | (M+H)⁺ = 756/758/760 (Br, Cl) |
| R_{f} = | 0.23 (Kieselgel, DCM/MeOH 9:1) |

### Beispiel 23

### 4-(2-Oxo-1,4-dihydro-2H-chinazolin-3-yl)-piperidin-1-carbonsäure{1-(3-brom-4-chlor-5-methyl-benzyl)-2-[4-(4-methyl-piperazin-1-yl)-piperidin-1-yl]-2-oxo-ethyl}-amid

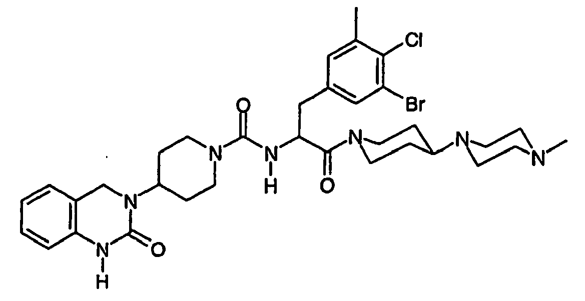

### 23a 2-Amino-3-(3-brom-4-chlor-5-methyl-phenyl)-propionsäureethylester Hydrochlorid

Analog Beispiel 14a wurde aus 6.06 g (22.2 mmol) *N*-(Diphenylmethylen)-glycinethylester und 6.30 g (115 mmol) 1-Brom-5-brommethyl-2-chlor-3-methylbenzol das Produkt hergestellt.

| | |
|---|---|
| Ausbeute: | 5.82 g (77% der Theorie) |
| ESI-MS: | (M+H)⁺ = 320/322/324 |
| R_{f} = | 0.70 (Kieselgel, DCM/MeOH/NH₃ 9:1:0.1) |

### 23b 3-(3-Brom-4-chlor-5-methyl-phenyl)-2-{[4-(2-oxo-1,4-dihydro-2H-chinazolin-3-yl)-piperidin-1-carbonyl]-amino}-propionsäureethylester

Analog Beispiel 14b wurde aus 5.82 g (16.3 mmol) 2-Amirio-3-(3-brom-4-chlor-5-methyl-phenyl)-propionsäureethylester Hydrochlorid und 3.77 (16.3 mmol) 3-Piperidin-4-yl-3,4-dihydro-1*H*-chinazolin-2-on das Produkt erhalten.

| | |
|---|---|
| Ausbeute: | 7.60 g (81 % der Theorie) |
| R_{f} = | 0.52 (Kieselgel, DCM/MeOH/NH₃ 9:1:0.1) |

### 23c 3-(3-Brom-4-chlor-5-methyl-phenyl)-2-{[4-(2-oxo-1,4-dihydro-2H-chinazolin-3-yl)-piperidin-1-carbonyl]-amino}-propionsäure

Analog Beispiel 14c wurde aus 7.60 g (13.1 mmol) 3-(3-Brom-4-chlor-5-methylphenyl)-2-{[4-(2-oxo-1,4-dihydro-2*H*-chinazolin-3-yl)-piperidin-1-carbonyl]-amino}-propionsäureethylester das Produkt erhalten.

| | |
|---|---|
| Ausbeute: | 6.70 g (93 % der Theorie) |
| ESI-MS: | (M-H)⁻ = 547/549/551 (Br, Cl) |
| R_{f} = | 0.05 (Kieselgel, DCM/MeOH/NH₃ 9:1:0.1) |

### 23d 4-(2-Oxo-1,4-dihydro-2Hchinazolin-3-yl)-piperidin-1-carbonsäure{1-(3-brom-4-chlor-5-methyl-benzyl)-2-[4-(4-methyl-piperazin-1-yl)-piperidin-1-yl]-2-oxo-ethyl}-amid

Analog Beispiel 2f wurde aus 1.35 g (2.45 mmol) 3-(3-Brom-4-chlor-5-methylphenyl)-2-{[4-(2-oxo-1,4-dihydro-2*H*-chinazolin-3-yl)-piperidin-1-carbonyl]-amino}-propionsäure und 449 g (2.45 mmol) 1-Methyl-4-piperidin-4-yl-piperazin das Produkt hergestellt.

| | |
|---|---|
| Ausbeute: | 1.10 g (63% der Theorie) |
| ESI-MS: | (M+H)⁺ = 714/716/718 (Br, CI) |
| R_{f} = | 0.41 (Kieselgel, DCM/MeOH/NH₃ 9:1:0.1) |

Analog wurden folgende Verbindungen aus 3-(3-Brom-4-chlor-5-methyl-phenyl)-2-{[4-(2-oxo-1,4-dihydro-2*H*-chinazolin-3-yl)-piperidin-1-carbonyl]-amino}-propionsäure und der entsprechenden Menge an Amin hergestellt:

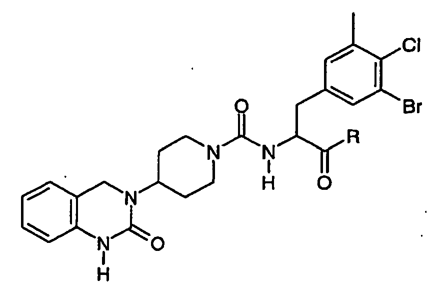

| **Beispiel** | **R** | **Ausbeute (%)** | **Massenspektrum** | **R_{f} (Kieselgel)** |
|---|---|---|---|---|
| **23.1** | 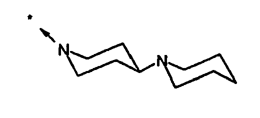 | 44 | 699/701/703 [M+H]⁺ | 0.40 (DCM/MeOH/NH₃ 9:1:0.1) |
| **23.2** | 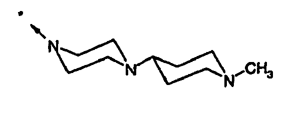 | 55 | 714/716/718 [M+H]⁺ | 0.24 (DCM/MeOH/NH₃ 9:1:0.1) |
| **23.3** | 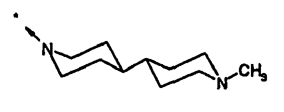 | 53 | 713/715/717 [M+H]⁺ | 0.37 (DCM/MeOH/NH₃ 9:1:0.1) |
| **23.4** | 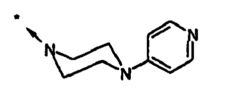 | 39 | 694/696/698 [M+H]⁺ | 0.49 (DCM/MeOH/NH₃ 9:1:0.1) |

### Beispiel 24

### 2-(4-Chlor-3-trifluormethyl-benzyl)-1-(1'-methyl-[4,4']bipiperidinyl-1-yl)-4-[4-(2-oxo-1,4-dihydro-2H-chinazolin-3-yl)-piperidin-1-yl]-butan-1,4-dion

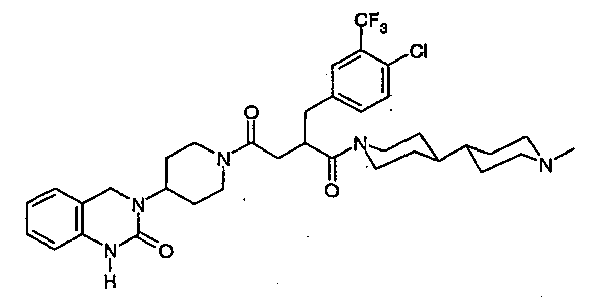

### 24a 2-[1-(4-Chlor-3-trifluormethyl-phenyl)-meth-(E)-ylidene]-bernsteinsäure-1-methylester

Zu einer frisch bereiteten Natriummethylat-Lösung (hergestellt durch Lösen von 3.64 g (158 mmol) Natrium im MeOH) in 300 mL MeOH wurden 20.7 mL (158 mmol) Bernsteinsäuredimethylester gegeben und das Reaktionsgemisch 1 h bei RT gerührt. Dann wurden 30 g (144 mmol) 4-Chlor-3-trifluormethyl-benzaldehyd zugegeben und die Reaktionslösung 6 h unter Rückfluss erhitzt. Man engte i.vac. ein, nahm den Rückstand in Wasser auf, säuerte mit 20% Zitronensäure-Lösung an und extrahierte erschöpfend mit EtOAc. Die organische Phase wurde fünfmal mit jeweils 200 mL 3% NH₃-Lösung extrahiert, die vereinigten wässrigen Phasen mit Zitronensäure-Lösung angesäuert, erschöpfend mit EtOAc extrahiert und über Na₂SO₄ getrocknet. Nach Entfernen des Trocken- und Lösungsmittels erhielt man das gewünschte Produkt in Form eines gelben Öles.

| | |
|---|---|
| Ausbeute: | 12 g (26% der Theorie) |
| R_{f} = | 0.33 (Kieselgel, PE/EtOAc/AcOH 75:25:5) |

### 24b 2-(4-Chlor-3-trifluormethyl-benzyl)-bernsteinsäure-1-methylester

Zu einer Lösung von 2.0 g (6.2 mmol) 2-[1-(4-Chlor-3-trifluormethyl-phenyl)-meth-(E)-yliden]-bernsteinsäure-1-methylester in 20 mL MeOH wurden 200 mg 10% Pt/C gegeben und das Reaktionsgemisch bei RT und 3 bar H₂ 3 h hydriert. Der Katalysator wurde abfiltriert und das Lösungsmittel i.vac. eingeengt. Das Rohprodukt wurde ohne Reinigung weiter umgesetzt.

| | |
|---|---|
| Ausbeute: | 1.85 g (92% der Theorie) |
| R_{f} = | 0.38 (Kieselgel, PE/EtOAc/AcOH 75:25:5) |

### 24c 2-(4-Chlor-3-trifluormethyl-benzyl)-4-oxo-4-[4-(2-oxo-1,4-dihydro-2H-chinazolin-3-yl)-piperidin-1-yl]-butansäuremethylester

Eine Lösung von 1.5 g (4.6 mmol) 2-(4-Chlor-3-trifluormethyl-benzyl)-bernsteinsäure-1-methylester, 1.64 g (5.1 mmol) TBTU, 0.69 g (5.0 mmol) HOBt und 1.32 mL (7.5 mmol) Ethyldiisopropylamin in 100 mL eines THF/Wasser-Gemisches (9:1) wurde 10 min bei RT gerührt und dann mit 1.2 g (5.0 mmol) 3-Piperidin-4-yl-3,4-dihydro-1*H-*chinazolin-2-on versetzt. Das Reaktionsgemisch wurde 2 h bei RT gerührt, i. vac. eingeengt, der Rückstand mit gesättigter NaHCO₃-Lösung versetzt, mit EtOAc erschöpfend extrahiert und die vereinigten organischen Extrakte über MgSO₄ getrocknet. Nach Entfernen des Trocken- und Lösungsmittels erhielt man das gewünschte Produkt, das ohne Reinigung weiter umgesetzt wurde.

| | |
|---|---|
| Ausbeute: | 2.2 g (89% der Theorie) |
| R_{f} = | 0.6 (Kieselgel, DCNUMeOH/Cyc/NH₃ 70:15:15:2) |

### 24d 2-(4-Chlor-3-trifluormethyl-benzyl)-4-oxo-4-[4-(2-oxo-1,4-dihydro-2H-chinazolin-3-yl)-piperidin-1-yl]-butansäure

Zu einer Lösung von 2.2 g (4.1 mmol) 2-(4-Chlor-3-trifluormethyl-benzyl)-4-oxo-4-[4-(2-oxo-1 ,4-dihydro-2*H*-chinazolin-3-yl)-piperidin-1-yl]-butansäuremethylester in 20 mL MeOH wurden 16 mL 1 M NaOH-Lösung gegeben und das Reaktionsgemisch 5 h bei 50°C gerührt. Man verdünnte mit 170 mL Wasser, extrahierte zweimal mit je 30 mL *tert*-Butylmethylether, versetzte die wässrige Phase mit 16 mL 1 M HCI, extrahierte dreimal mit je 70 mL EtOAc und trocknete die vereinigten organischen Phasen über Na₂SO₄. Nach Entfernen des Trocken- und Lösungsmittels wurde der Rückstand mit Diisopropylether verrieben, abgesaugt und an der Luft getrocknet.

| | |
|---|---|
| Ausbeute: | 1.1 g (51 % der Theorie) |
| R_{f} = | 0.25 (Kieselgel, DCM/MeOH/Cyc/NH₃ 70:15:15:2) |

### 24e 2-(4-Chlor-3-trifluormethyl-benzyl)-1-(1'-methyl-[4,4']bipiperidinyl-1-yl)-4-[4-(2-oxo-1,4-dihydro-2H-chinazolin-3-yl)-piperidin-1-yl]-butan-1,4-dion

Hergestellt analog zu 24c aus 790 mg (1.5 mmol) 2-(4-Chlor-3-trifluormethyl-benzyl)-4-oxo-4-[4-(2-oxo-1,4-dihydro-2*H*-chinazolin-3-yl)-piperidin-1-yl]-butansäure und 370 mg (1.6 mmol) 1-Methyl-[4,4']bipiperidinyl.

| | |
|---|---|
| Ausbeute: | 530 mg (51% der Theorie) |
| EI: | (M)⁺ = 687/689 (Cl) |
| R_{f} = | 0.6 (Kieselgel, DCM/MeOH/Cyc/NH₃ 70:15:15:2) |

### Beispiel 24.1

### 2-(4-Chlor-3-trifluormethyl-benzyl)-1-[4-(4-methyl-piperazin-1-yl)-piperidin-1-yl]-4-[4-(2-oxo-1,4-dihydro-2H-chinazolin-3-yl)-piperidin-1-yl]-butan-1,4-dion

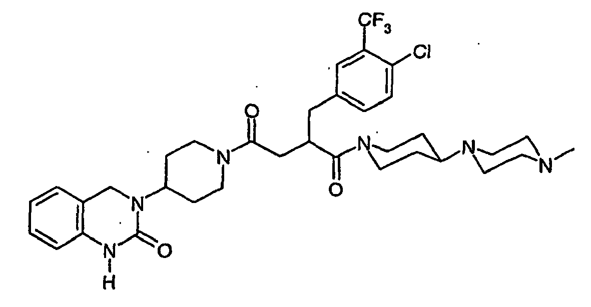

Zu einer Lösung von 800 mg (1.53 mmol) 2-(4-Chlor-3-trifluormethyl-benzyl)-4-oxo-4-[4-(2-oxo-1,4-dihydro-2*H*-chinazolin-3-yl)-piperidin-1-yl]-butansäure, 544 mg (1.7 mmol) TBTU, 206 mg (1.53 mmol) HOBt und 0.69 mL (4.94 mmol) Triethylamin in 100 mL THF wurden 280 mg (1.53 mmol) 1-Methyl-4-piperidin-4-yl-piperazin gegeben und das Reaktionsgemisch 2.5 h bei RT gerührt. Man engte i. vac. ein, nahm den Rückstand in DCM auf, wusch die organische Phase zweimal mit gesättigter NaHCO₃-Lösung und trocknete über MgSO₄. Nach Entfernen des Trocken- und Lösungsmittels wurde der Rückstand chromatographisch (Kieselgel, MeOH) gereinigt.

| | |
|---|---|
| Ausbeute: | 400 mg (38% der Theorie) |
| EI: | (M)⁺ = 688/690 (Cl) |
| R_{f} = | 0.25 (Kieselgel, MeOH) |

### Beispiel 24.2

### 1-[1,4']Bipiperidinyl-1'-yl-2-(4-chlor-3-trifluormethyl-benzyl)-4-[4-(2-oxo-1,4-dihydro-2H-chinazolin-3-yl)-piperidin-1-yl]-butan-1,4-dion

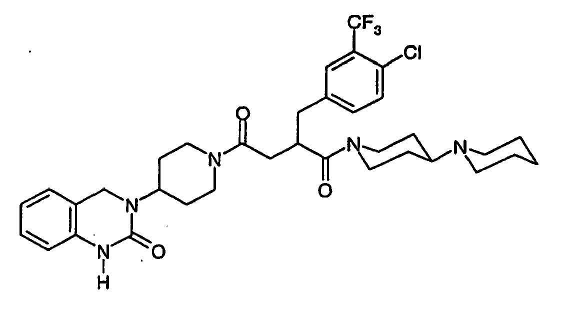

Hergestellt analog Beispiel 24.1 aus 800 mg (1.53 mmol) 2-(4-Chlor-3-trifluormethyl-benzyl)-4-oxo-4-[4-(2-oxo-1,4-dihydro-2*H*-chinazolin-3-yl)-piperidin-1-yl]-butansäure und 271 mg (1.61 mmol) [1,4']Bipiperidinyl.

| | |
|---|---|
| Ausbeute: | 470 mg (46% der Theorie) |
| EI: | (M)⁺ = 673/675 (Cl) |
| R_{f} = | 0.28 (Kieselgel, MeOH) |

### Beispiel 24.3

### [4-(1-{2-(4-Chlor-3-trifluormethyl-benzyl)-4-oxo-4-[4-(2-oxo-1,4-dihydro-2H-chinazolin-3-yl)-piperidin-1-yl]-butyryl}-piperidin-4-yl)-piperazin-1-yl]-essigsäure

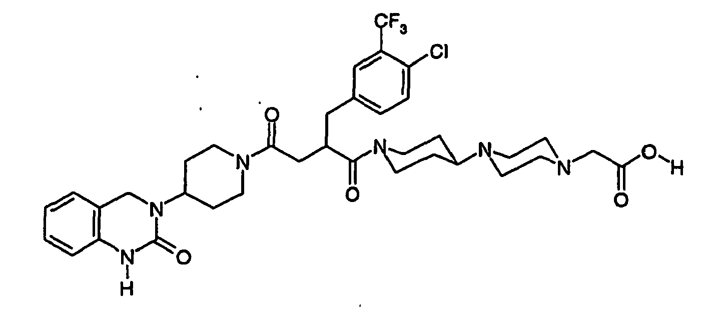

Hergestellt analog Beispiel 16.4 aus 105 mg (0.2 mmol) 2-(4-Chlor-3-trifluormethyl-benzyl)-4-oxo-4-[4-(2-oxo-1,4-dihydro-2*H*-chinazolin-3-yl)-piperidin-1-yl]-butansäure und 51 mg (0.2 mmol) (4-Piperidin-4-yl-piperazin-1-yl)-essigsäureethylester.

| | |
|---|---|
| Ausbeute: | 13 mg (8% der Theorie) |
| ESI-MS: | (M+H)⁺ = 733/735 (CI) |
| Retentionszeit (HPLC): | 6.3 min (Methode A) |

### Beispiel 24.4

### (1'-{2-(4-Chlor-3-trifluormethyl-benzyl)-4-oxo-4-[4-(2-oxo-1,4-dihydro-2H-chinazolin-3-yl)-piperidin-1-yl]-butyryl}-[4,4']bipiperidinyl-1-yl)-essigsäuremethylester

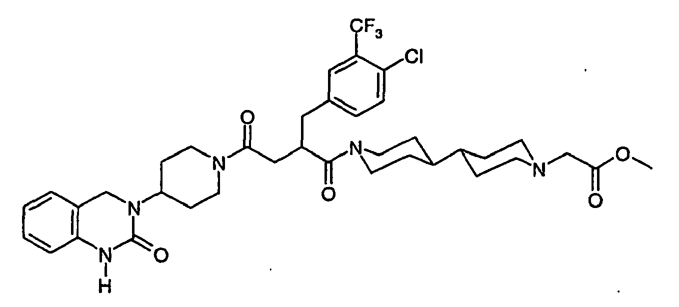

Hergestellt analog Beispiel 16.5 aus 209 mg (0.4 mmol) 2-(4-Chlor-3-trifluormethyl-benzyl)-4-oxo-4-[4-(2-oxo-1,4-dihydro-2*H*-chinazolin-3-yl)-piperidin-1-yl]-butansäure und 102 mg (0.4 mmol) [4,4']Bipiperidinyl-1-yl-essigsäureethylester.

| | |
|---|---|
| Ausbeute: | 17 mg (17% der Theorie) |
| ESI-MS: | (M+H)⁺ = 746/748 (CI) |
| R_{f} = | 0.44 (Kieselgel, DCM/MeOH 9:1) |

### Beispiel 24.5

### (1'-{2-(4-Chlor-3-trifluormethyl-benzyl)-4-oxo-4-[4-(2-oxo-1,4-dihydro-2H-chinazolin-3-yl)-piperidin-1-yl]-butyryl}-[4,4']bipiperidinyl-1-yl)-essigsäure

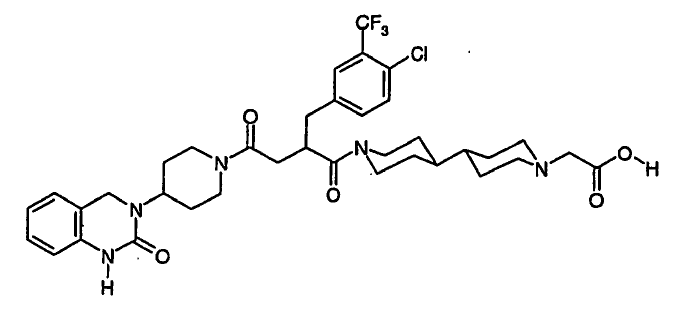

Hergestellt analog Beispiel 16.6 aus 198 mg (0.26 mmol) (1'-{2-(4-Chlor-3-trifluormethyl-benzyl)-4-oxo-4-[4-(2-oxo-1,4-dihydro-2*H*-chinazolin-3-yl)-piperidin-1-yl]-butyryl}-[4,4']bipiperidinyl-1-yl)-essigsäuremethylester.

| | |
|---|---|
| Ausbeute: | 19 mg (9% der Theorie) |
| ESI-MS: | (M+H)⁺ = 732/734(Cl) |
| R_{f} = | 0.22 (Kieselgel, DCM/MeOH 9:1) |

### Beispiel 24.6

### 2-(4-Chlor-3-trifluormethyl-benzyl)-1-(1'-rnethyl-[4,4']bipiperidinyl-1-yl)-4-[4-(2-oxo-1,4-dihydro-2H-chinazolin-3-yl)-piperidin-1-yl]-butan-1,4-dion

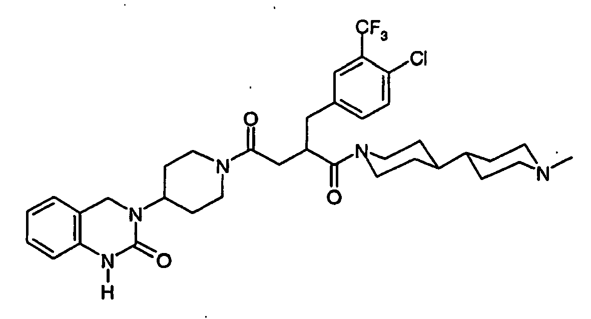

Hergestellt analog Beispiel 24.1 aus 800 mg (1.53 mmol) 2-(4-Chlor-3-trifluormethyl-benzyl)-4-oxo-4-[4-(2-oxo-1,4-dihydro-2*H*-chinazolin-3-yl)-piperidin-1-yl]-butansäure und 279 mg (1.53 mmol) 1-Methyl-[4,4']bipiperidinyl.

| | |
|---|---|
| Ausbeute: | 320 mg (30% der Theorie) |
| EI: | (M)⁺ = 687/689 (Cl) |
| R_{f} = | 0.20 (Kieselgel, MeOH) |

Mit den voranstehend beschriebenen Verfahren können auch die folgenden Verbindungen hergestellt werden:

| **Beispiel** | **Struktur** |
|---|---|
| **25.1** | 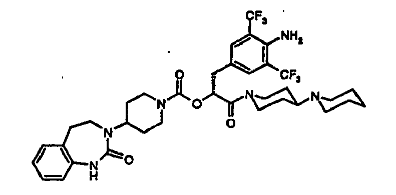 |
| **25.2** | 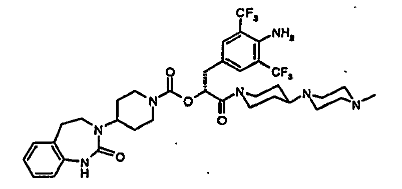 |
| **25.3** | 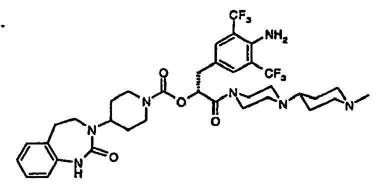 |
| **25.4** | 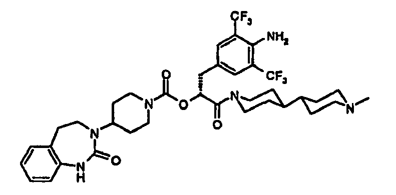 |
| **25.5** | 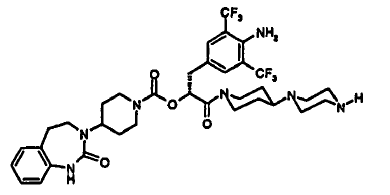 |
| **25.6** | 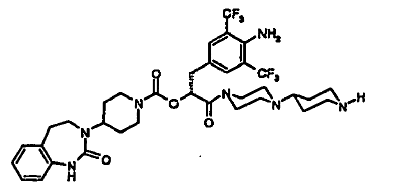 |
| **25.7** | 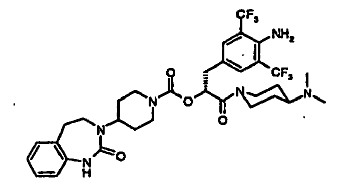 |
| **25.8** | 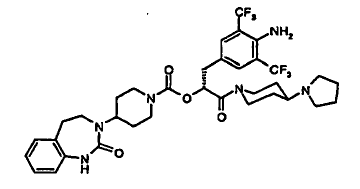 |
| **25.9** | 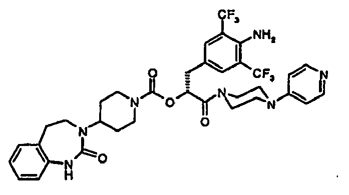 |
| **25.10** | 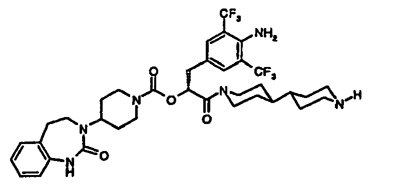 |

| **Beispiel** | **Struktur** |
|---|---|
| **26.1** | 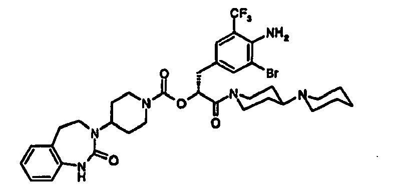 |
| **26.2** | 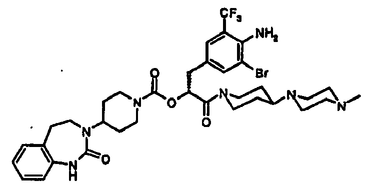 |
| **26.3** | 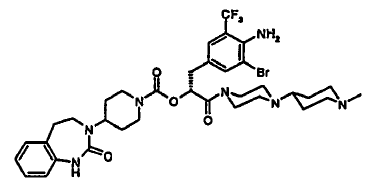 |
| **26.4** | 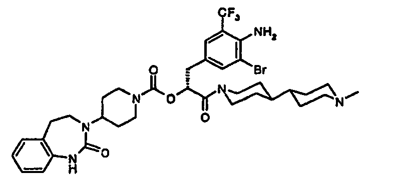 |
| **26.5** | 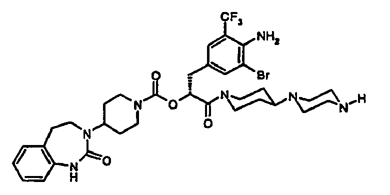 |
| **26.6** | 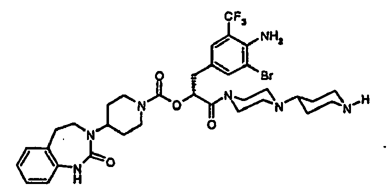 |
| **26.7** | 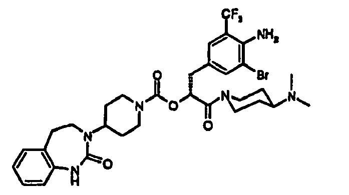 |
| **26.8** | 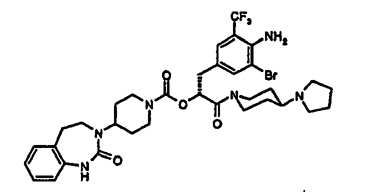 |
| **26.9** | 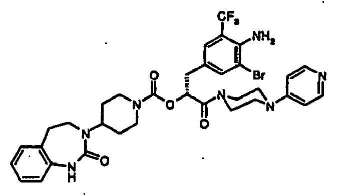 |
| **26.10** | 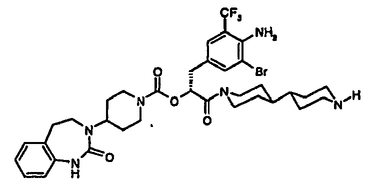 |

| **Beispiel** | **Struktur** |
|---|---|
| **27.1** | 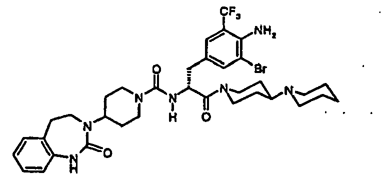 |
| **27.2** | 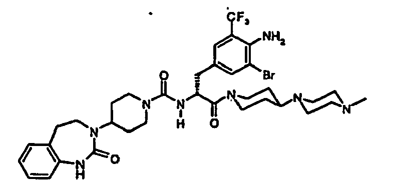 |
| **27.3** | 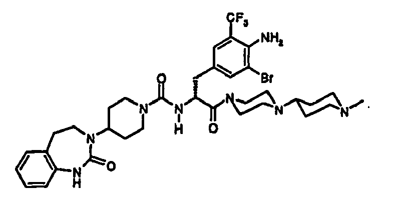 |
| **27.4** | 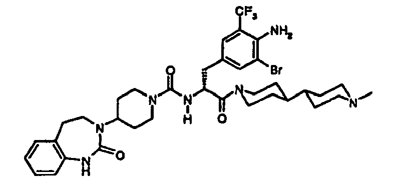 |
| **27.5** | 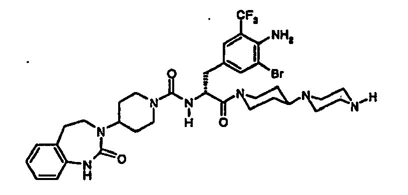 |
| **27.6** | 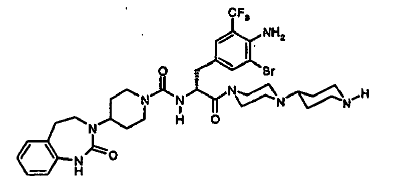 |
| **27.7** | 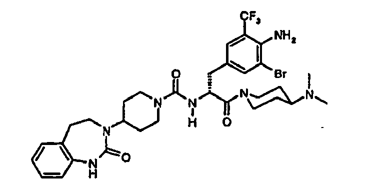 |
| **27.8** | 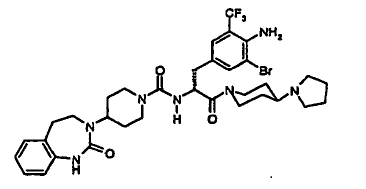 |
| **27.9** | 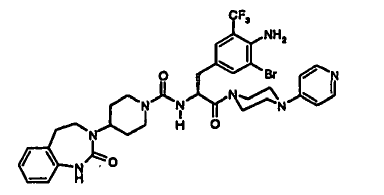 |
| **27.10** | 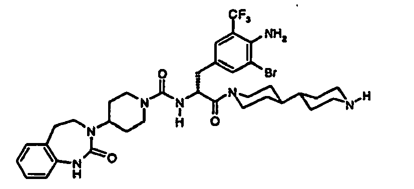 |

| **Beispiel** | **Struktur** |
|---|---|
| **28.1** | 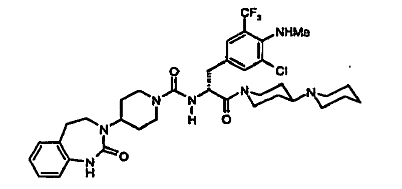 |
| **28.2** | 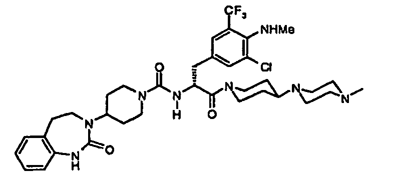 |
| **28.3** | 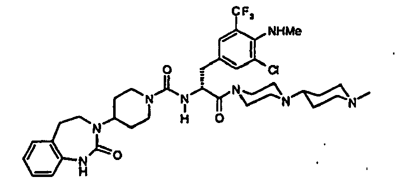 |
| **28.4** | 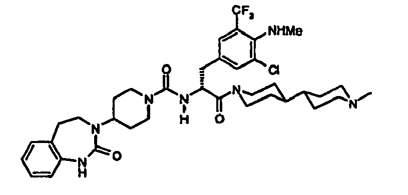 |
| **28.5** | 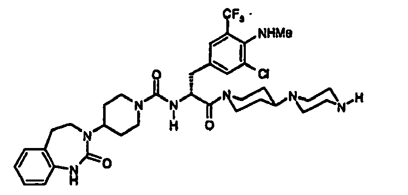 |
| **28.6** | 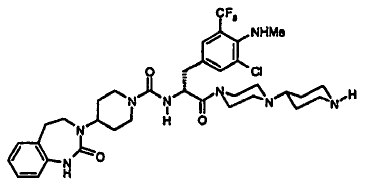 |
| **28.7** | 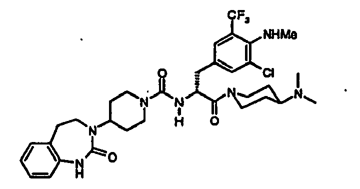 |
| **28.8** | 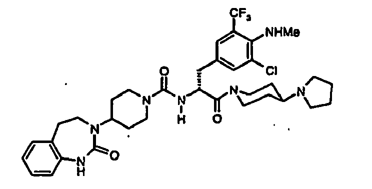 |
| **28.9** | 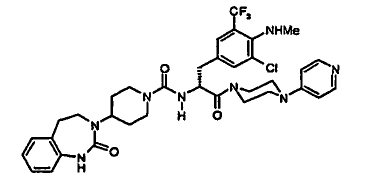 |
| **28.10** | 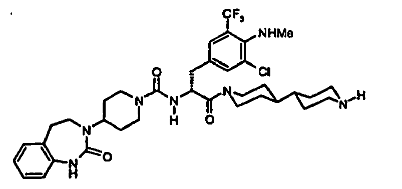 |

| **Beispiel** | **Struktur** |
|---|---|
| **29.1** | 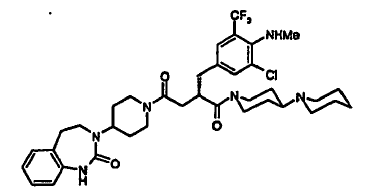 |
| **29.2** | 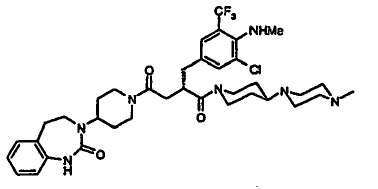 |
| **29.3** | 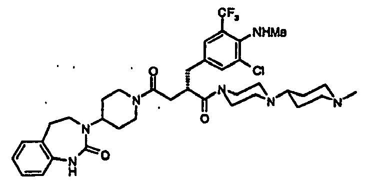 |
| **29.4** | 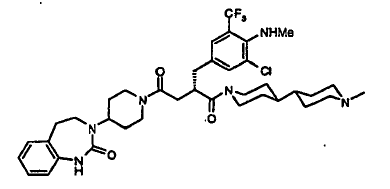 |
| **29.5** | 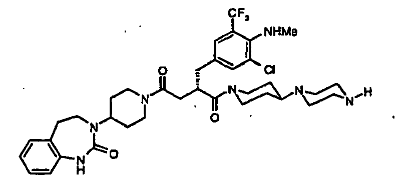 |
| **29.6** | 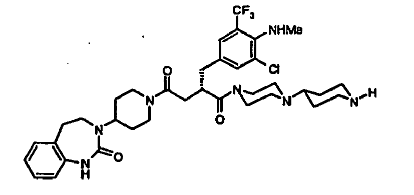 |
| **29.7** | 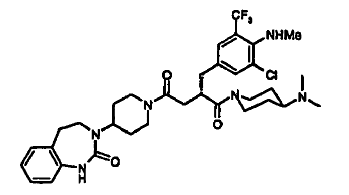 |
| **29.8** | 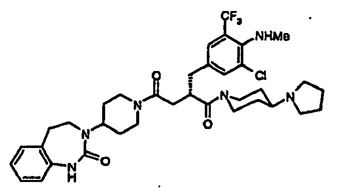 |
| **29.9** | 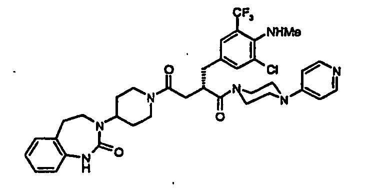 |
| **29.10** | 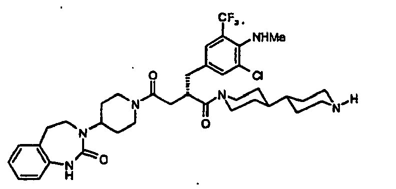 |

| **Beispiel** | **Struktur** |
|---|---|
| **30.1** | 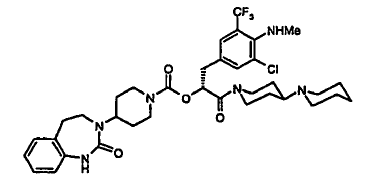 |
| **30.2** | 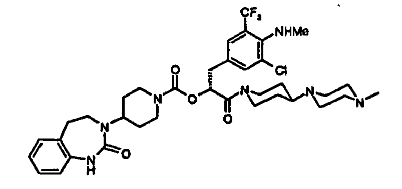 |
| **30.3** | 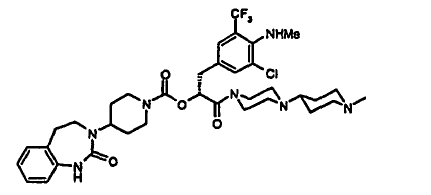 |
| **30.4** | 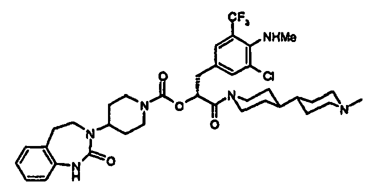 |
| **30.5** | 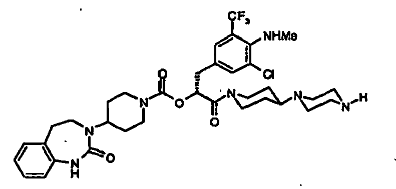 |
| **30.6** | 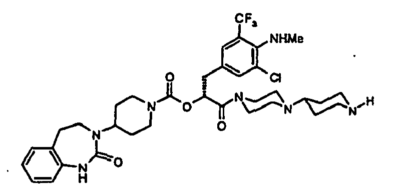 |
| **30.7** | 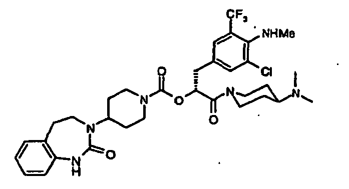 |
| **30.8** | 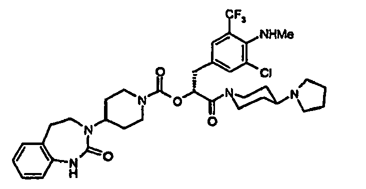 |
| **30.9** | 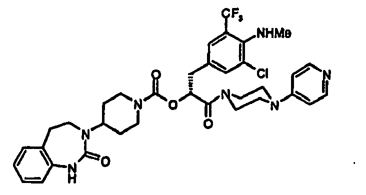 |
| **30.10** | 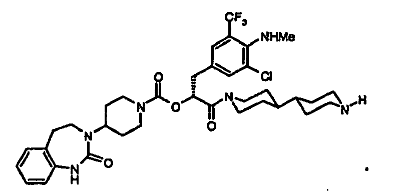 |

Mit 4-Amino-3-chlor-5-methyl-benzoesäure als Startmaterial können folgende Verbindungen erhalten werden:

| **Beispiel** | **Struktur** |
|---|---|
| **31.1** | 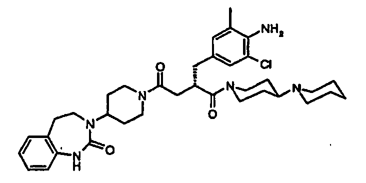 |
| **31.2** | 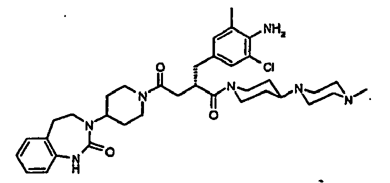 |
| **31.3** | 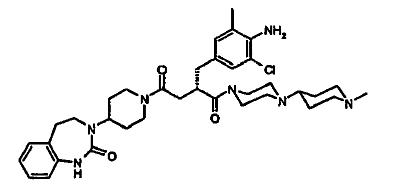 |
| **31.4** | 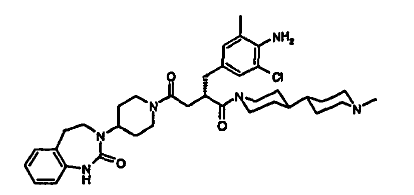 |
| **31.5** | 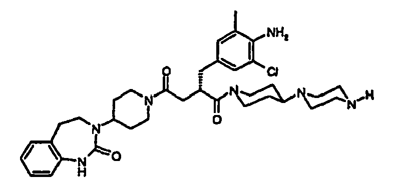 |
| **31.6** | 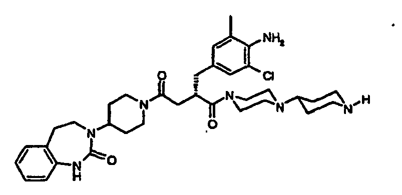 |
| **31.7** | 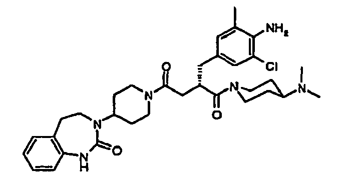 |
| **31.8** | 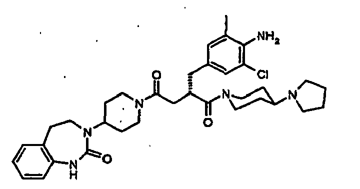 |
| **31.9** | 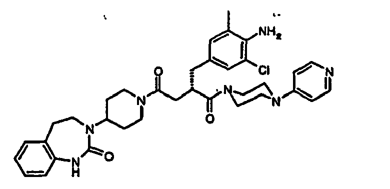 |
| **31.10** | 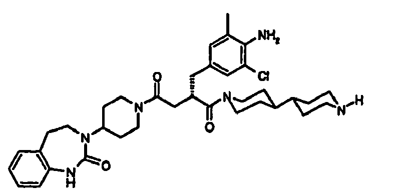 |

| **Beispiel** | **Struktur** |
|---|---|
| **32.1** | 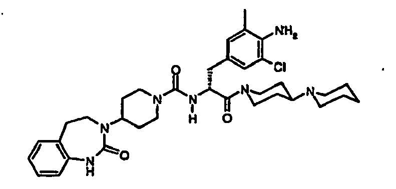 |
| **32.2** | 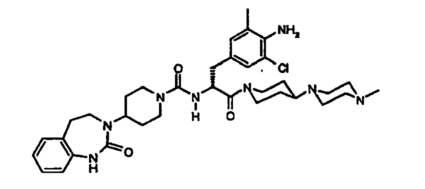 |
| **32.3** | 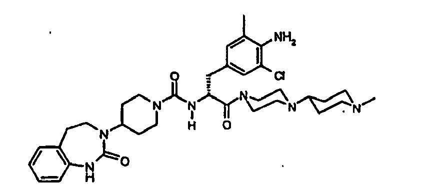 |
| **32.4** | 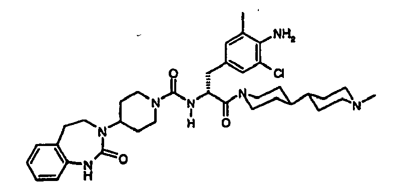 |
| **32.5** | 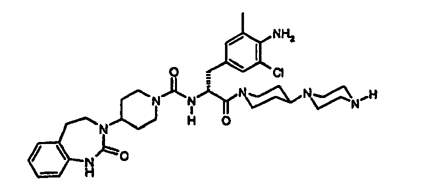 |
| **32.6** | 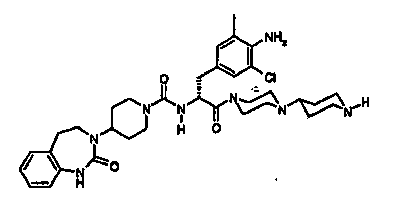 |
| **32.7** | 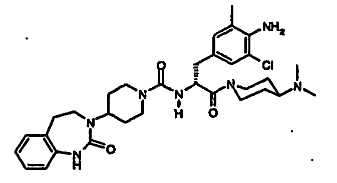 |
| **32.8** | 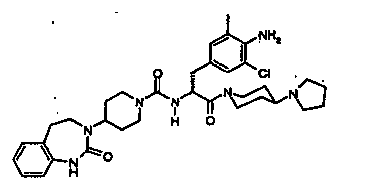 |
| **32.9** | 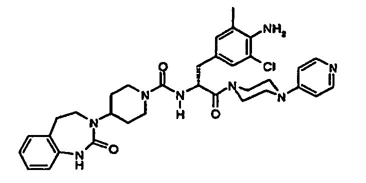 |
| **32.10** | 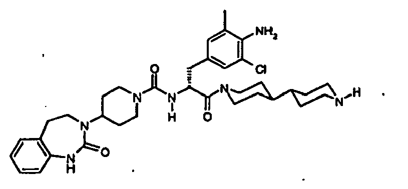 |

| **Beispiel** | **Struktur** |
|---|---|
| **33.1** | 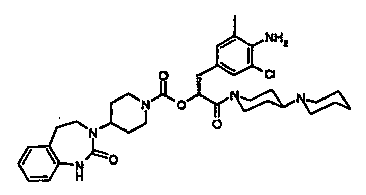 |
| **33.2** | 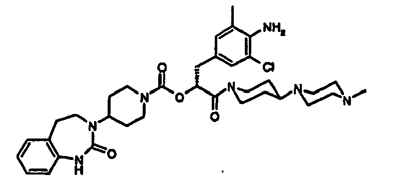 |
| **33.3** | 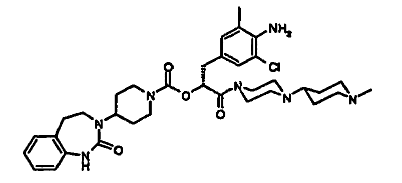 |
| **33.4** | 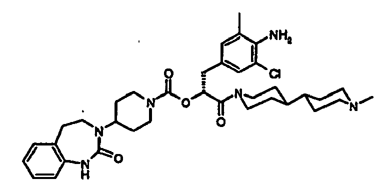 |
| **33.5** | 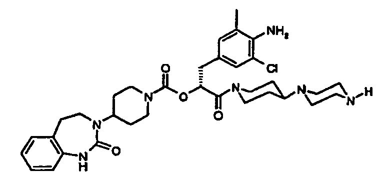 |
| **33.6** | 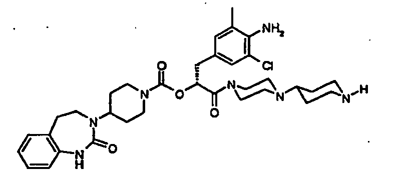 |
| **33.7** | 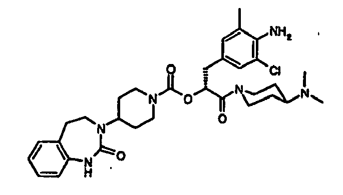 |
| **33.8** | 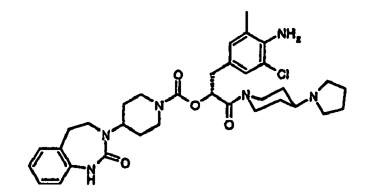 |
| **33.9** | 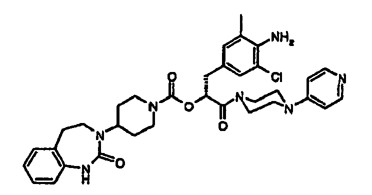 |
| **33.10** | 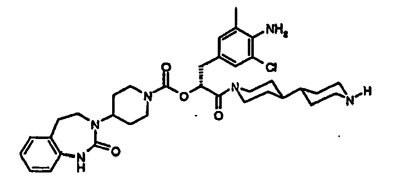 |

Mit 2-Chlor-6-trifluormethyl-phenol als Startmaterial können folgende Verbindungen erhalten werden, wobei gegebenenfalls die phenolische Hydroxyfunktion unter Verwendung einer geeigneten Schutzgruppe blockiert werden müsste:

| **Beispiel** | **Struktur** |
|---|---|
| **34.1** | 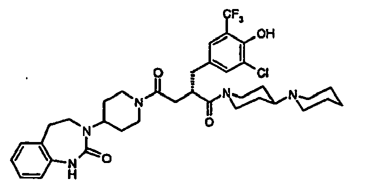 |
| **34.2** | 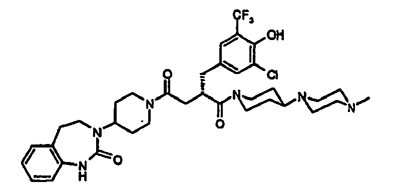 |
| **34.3** | 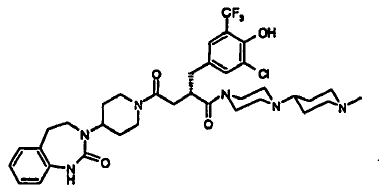 |
| **34.4** | 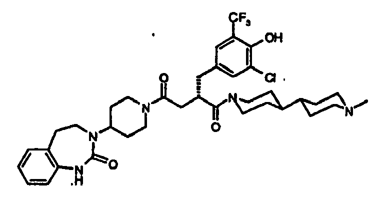 |
| **34.5** | 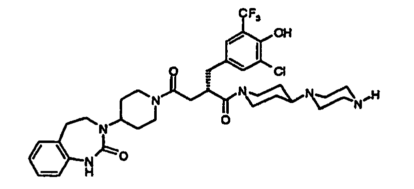 |
| **34.6** | 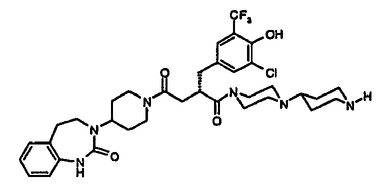 |
| **34.7** | 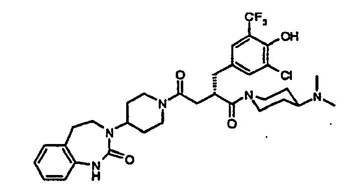 |
| **34.8** | 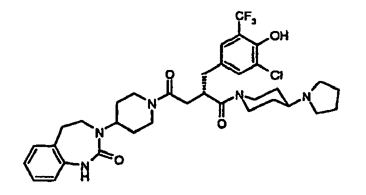 |
| **34.9** | 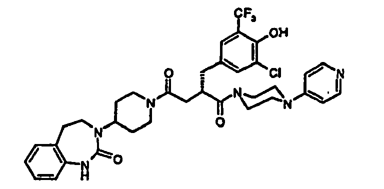 |
| **34.10** | 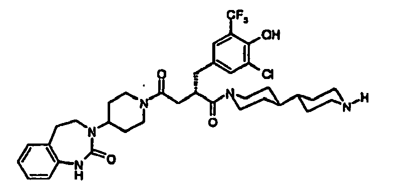 |

| **Beispiel** | **Struktur** |
|---|---|
| **35.1** | 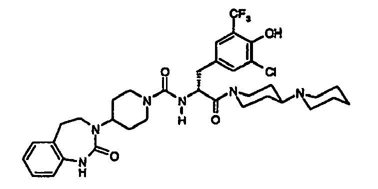 |
| **35.2** | 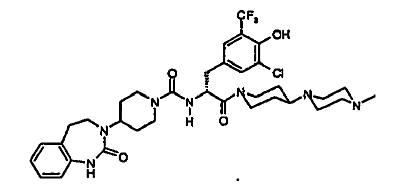 |
| **35.3** | 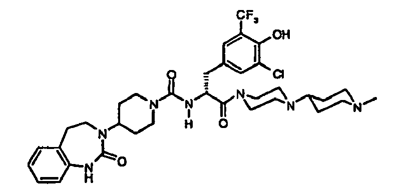 |
| **35.4** | 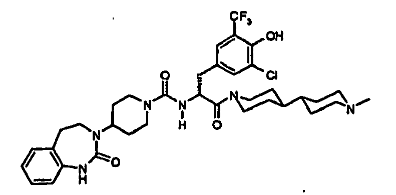 |
| **35.5** | 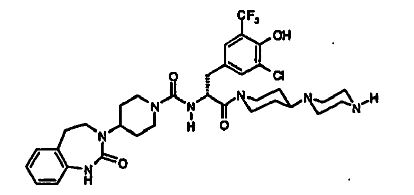 |
| **35.6** | 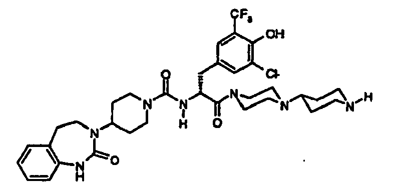 |
| **35.7** | 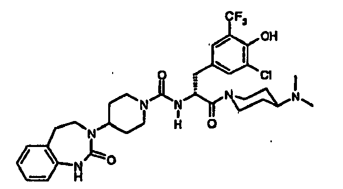 |
| **35.8** | 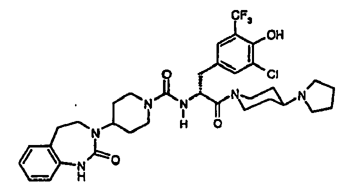 |
| **35.9** | 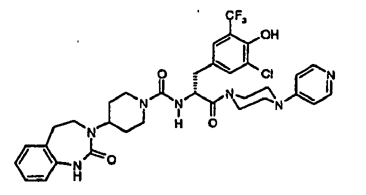 |
| **35.10** | 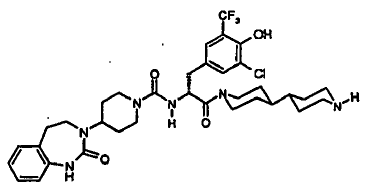 |

| **Beispiel** | **Struktur** |
|---|---|
| **36.1** | 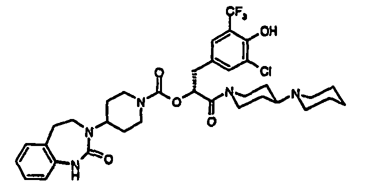 |
| **36.2** | 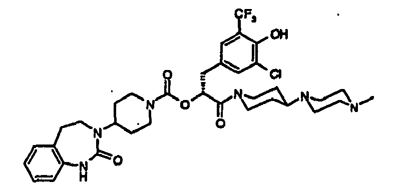 |
| **36.3** | 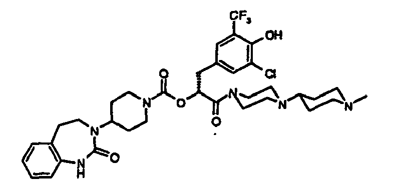 |
| **36.4** | 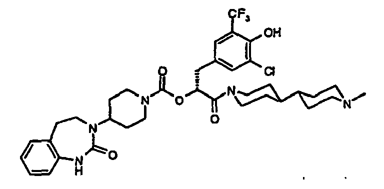 |
| **36.5** | 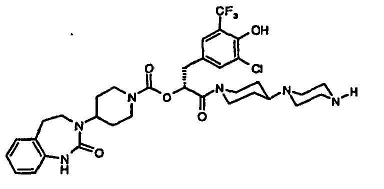 |
| **36.6** | 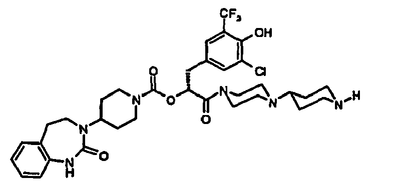 |
| **36.7** | 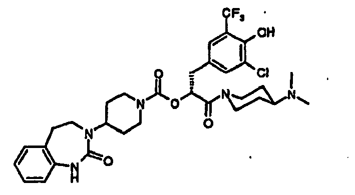 |
| **36.8** | 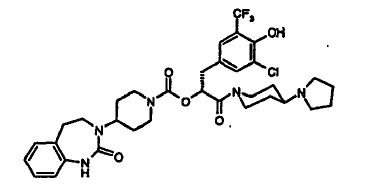 |
| **36.9** | 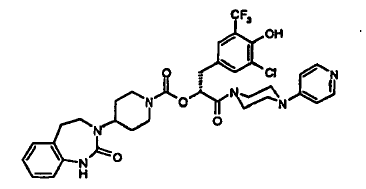 |
| **36.10** | 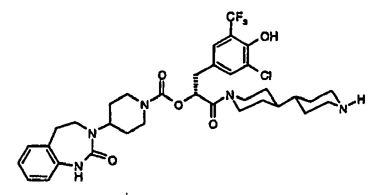 |

### Beispiel 37

### (S)-2-(4-Amino-3-chlor-5-trifluormethyl-benzyl)-1-[4-(4-methyl-piperazin-1-yl)-pipeddin-1-yl]-4-[4-(2-oxo-1,4-dihydro-2H-thieno[3,2-d]pyrimidin-3-yl)-piperidin-1-yl]-butan-1,4-dion

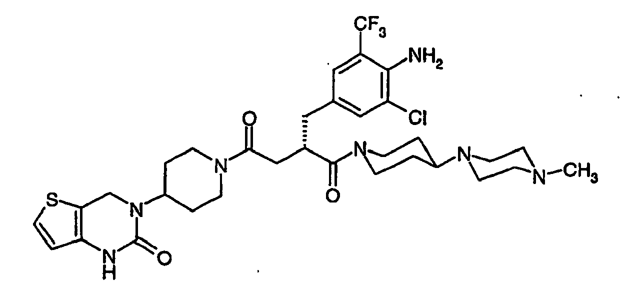

### Beispiel 37.1

### (S)-2-(4-Amino-3-chlor-5-trifluormethyl-benzyl)-1-[4-(4-methyl-piperazin-1-yl)-piperidin-1-yl]-4-[4-(5-oxo-4,5,7,8-tetrahydro-2-thia-4,6-diaza-azulen-6-yl)-piperidin-1-yl]-butan-1,4-dion

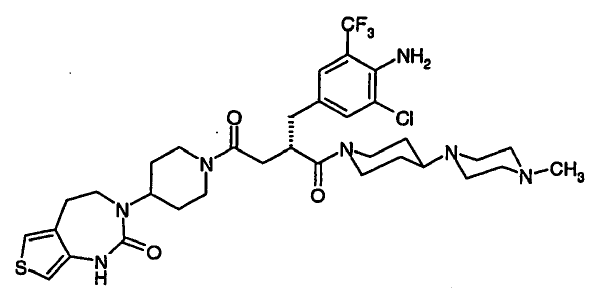

### Beispiel 37.2

### (S)-2-(4-Amino-3-chlor-5-trifluormethyl-benzyl)-1-[4-(4-methyl-piperazin-1-yl)-piperidin-1 -yl]-4-[4-(2-oxo-1,2,4,5-tetrahydro-thieno[3,2-d]-1,3-diazepin-3-yl)-piperidin-1-yl]-butan-1,4-dion

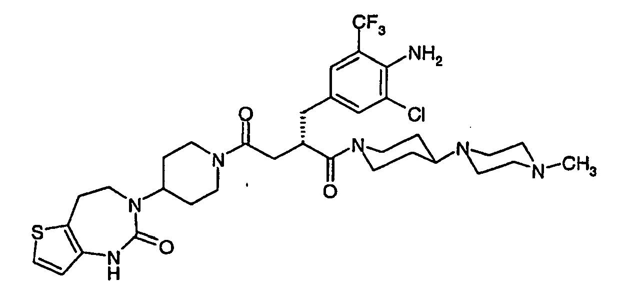

### Beispiel 37.3

### (S)-2-(4-Amino-3-chlor-5-trifluormethyl-benzyl)-1-[4-(4-methyl-piperazin-1-yl)-piperidin-1-yl]-4-[4-(2-oxo-1,2,4,5-tetrahydro-thieno[2,3-d]-1,3-diazepin-3-yl)-piperidin-1-yl]-butan-1,4-dion

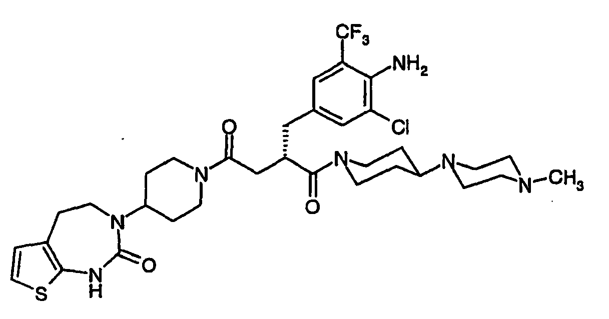

### Beispiel 37.4

### (S)-2-(4-Amino-3-chlor-5-trifluormethyl-benzyl)-1-[4-(4-methyl-piperazin-1-yl)-piperidin-1-yl]-4-[4-(2-oxo-1,4-dihydro-2H-thieno[2,3-d]pyrimidin-3-yl)-pipeddin-1-yl]-butan-1,4-dion

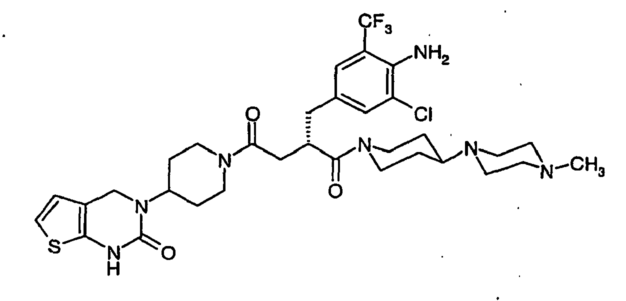

### Beispiel 37.5

### (S)-2-(4-Amino-3-chlor-5-trifluormethyl-benzyl)-1-(4,4-difluor-1,4'-bipiperidinyl-1'-yl)-4-[4-(2-oxo-1,2,4,5-tetrahydro-1,3-benzdiazepin-3-yl)-piperidin-1-yl]-butan-1,4-dion

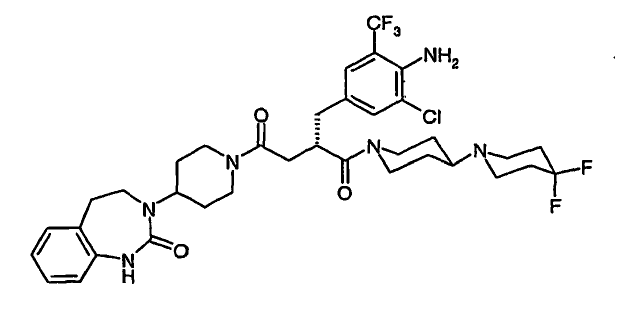

Die nachfolgenden Beispiele beschreiben die Herstellung pharmazeutischer Anwendungsformen, die als Wirkstoff eine beliebige Verbindung der allgemeinen Formel (I) enthalten:

### Beispiel I

### Kapseln zur Pulverinhalation mit 1 mg Wirkstoff

### Zusammensetzung:

| 1 Kapsel zur Pulverinhalation enthält: | |
|---|---|
| Wirkstoff | 1.0 mg |
| Milchzucker | 20.0 mg |
| Hartgelatinekapseln | 50.0 mg |
| | 71.0 mg |

### Herstellungsverfahren:

Der Wirkstoff wird auf die für Inhalativa erforderliche Korngröße gemahlen. Der gemahlene Wirkstoff wird mit dem Milchzucker homogen gemischt. Die Mischung wird in Hartgelatinekapseln abgefüllt.

### Beispiel II

### Inhalationslösung für Respimat^{®} mit 1 mg Wirkstoff

### Zusammensetzung:

| 1 Hub enthält: | |
|---|---|
| Wirkstoff | 1.0 mg |
| Benzalkoniumchlorid | 0.002 mg |
| Dinatriumedetat | 0.0075 mg |
| Wasser gereinigt ad | 15.0 µl |

### Herstellungsverfahren:

Der Wirkstoff und Benzalkoniumchlorid werden in Wasser gelöst und in Respimat^{®}-Kartuschen abgefüllt.

### Beispiel III

### Inhalationslösung für Vernebler mit 1 mg Wirkstoff

### Zusammensetzung:

| 1 Fläschchen enthält: | |
|---|---|
| Wirkstoff | 0.1 g |
| Natriumchlorid | 0.18 g |
| Benzalkoniumchlorid | 0.002 g |
| Wasser gereinigt ad | 20.0 ml |

### Herstellungsverfahren:

Wirkstoff, Natriumchlorid und Benzalkoniumchlorid werden in Wasser gelöst.

### Beispiel IV

### Treibgas-Dosieraerosol mit 1 mg Wirkstoff

### Zusammensetzung:

| 1 Hub enthält: | |
|---|---|
| Wirkstoff | 1.0 mg |
| Lecithin | 0.1 % |
| Treibgas ad | 50.0 µl |

### Herstellungsverfahren:

Der mikronisierte Wirkstoff wird in dem Gemisch aus Lecithin und Treibgas homogen suspendiert. Die Suspension wird in einen Druckbehälter mit Dosierventil abgefüllt.

### Beispiel V

### Nasalspray mit 1 mg Wirkstoff

### Zusammensetzung:

| | |
|---|---|
| Wirkstoff | 1.0 mg |
| Natriumchlorid | 0.9 mg |
| Benzalkoniumchlorid | 0.025 mg |
| Dinatriumedetat | 0.05 mg |
| Wasser gereinigt ad | 0.1 ml |

### Herstellungsverfahren:

Der Wirkstoff und die Hilfsstoffe werden in Wasser gelöst und in ein entsprechendes Behältnis abgefüllt.

### Beispiel VI

### Injektionslösung mit 5 mg Wirksubstanz pro 5 ml

### Zusammensetzung:

| | |
|---|---|
| Wirksubstanz | 5 mg |
| Glucose | 250 mg |
| Human-Serum-Albumin | 10 mg |
| Glykofurol | 250 mg |
| Wasser für Injektionszwecke ad | 5 ml |

### Herstellung:

Glykofurol und Glucose in Wasser für Injektionszwecke auflösen (Wfl); Human-Serum-Albumin zugeben; Wirkstoff unter Erwärmen auflösen; mit Wfl auf Ansatzvolumen auffüllen; unter Stickstoff-Begasung in Ampullen abfüllen.

### Beispiel VII

### Injektionslösung mit 100 mg Wirksubstanz pro 20 ml

### Zusammensetzung:

| | |
|---|---|
| Wirksubstanz | 100 mg |
| Monokaliumdihydrogenphosphat = KH₂PO₄ | 12 mg |
| Dinatriumhydrogenphosphat = Na₂HPO₄·2H₂O | 2 mg |
| Natriumchlorid | 180 mg |
| Human-Serum-Albumin | 50 mg |
| Polysorbat 80 | 20 mg |
| Wasser für Injektionszwecke ad | 10 ml |

### Herstellung:

Polysorbat 80, Natriumchlorid, Monokaliumdihydrogenphosphat und Dinatriumhydrogenphosphat in Wasser für Injektionszwecke (Wfl) auflösen; Human-Serum-Albumin zugeben; Wirkstoff unter Erwärmen auflösen; mit Wfl auf Ansatzvolumen auffüllen; in Ampullen abfüllen.

### Beispiel VIII

### Lyophilisat mit 10 mg Wirksubstanz

### Zusammensetzung:

| | |
|---|---|
| Wirksubstanz | 10 mg |
| Mannit | 300 mg |
| Human-Serum-Albumin | 20 mg |
| Wasser für Injektionszwecke ad | 2 ml |

### Herstellung:

Mannit in Wasser für Injektionszwecke (Wfl) auflösen; Human-Serum-Albumin zugeben; Wirkstoff unter Erwärmen auflösen; mit Wfl auf Ansatzvolumen auffüllen; in Vials abfüllen; gefriertrocknen.

### Lösungsmittel für Lyophilisat:

| | |
|---|---|
| Polysorbat 80 = Tween 80 | 20 mg |
| Mannit | 200 mg |
| Wasser für Injektionszwecke ad | 10 ml |

### Herstellung:

Polysorbat 80 und Mannit in Wasser für Injektionszwecke (Wfl) auflösen; in Ampullen abfüllen.

### Beispiel IX

### Tabletten mit 20 mg Wirksubstanz

### Zusammensetzung:

| | |
|---|---|
| Wirksubstanz | 20 mg |
| Lactose | 120 mg |
| Maisstärke | 40 mg |
| Magnesiumstearat | 2 mg |
| Povidon K 25 | 18 mg |

### Herstellung:

Wirksubstanz, Lactose und Maisstärke homogen mischen; mit einer wässerigen Lösung von Povidon granulieren; mit Magnesiumstearat mischen; auf einer Tablettenpresse abpressen; Tablettengewicht 200 mg.

### Beispiel X

### Kapseln mit 20 mg Wirksubstanz

### Zusammensetzung:

| | |
|---|---|
| Wirksubstanz | 20 mg |
| Maisstärke | 80 mg |
| Kieselsäure, hochdispers | 5 mg |
| Magnesiumstearat | 2.5 mg |

### Herstellung:

Wirksubstanz, Maisstärke und Kieselsäure homogen mischen; mit Magnesiumstearat mischen; Mischung auf einer Kapselfüllmaschine in Hartgelatine-Kapseln Größe 3 abfüllen.

### Beispiel XI

### Zäpfchen mit 50 mg Wirksubstanz

### Zusammensetzung:

| | |
|---|---|
| Wirksubstanz | 50 mg |
| Hartfett (Adeps solidus) q.s. ad | 1700 mg |

### Herstellung:

Hartfett bei ca. 38°C aufschmelzen; gemahlene Wirksubstanz im geschmolzenen Hartfett homogen dispergieren; nach Abkühlen auf ca. 35°C in vorgekühlte Formen ausgießen.

### Beispiel XII

### Injektionslösung mit 10 mg Wirksubstanz pro 1 mL

### Zusammensetzung:

| | |
|---|---|
| Wirksubstanz | 10 mg |
| Mannitol | 50 mg |
| Human-Serum-Albumin | 10 mg |
| Wasser für Injektionszwecke ad | 1 ml |

### Herstellung:

Mannitol in Wasser für Injektionszwecke auflösen (Wfl); Human-Serum-Albumin zugeben; Wirkstoff unter Erwärmen auflösen; mit Wfl auf Ansatzvolumen auffüllen; unter Stickstoff-Begasung in Ampullen abfüllen.

## Patentansprüche

1. CGRP-Antagonisten der allgemeinen Formel 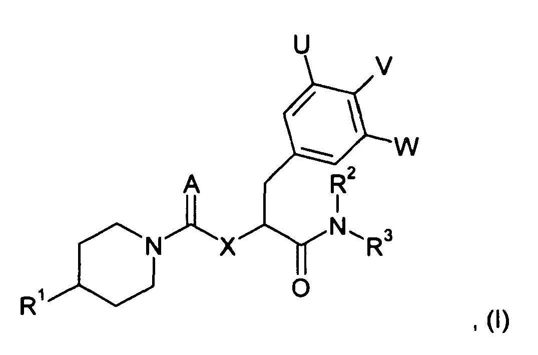 in der
A ein Sauerstoff- oder Schwefelatom, eine Phenylsulfonylimino- oder Cyaniminogruppe,
X ein Sauerstoff- oder Schwefelatom, eine gegebenenfalls durch eine C₁₋₆-Alkylgruppe substituierte Iminogruppe oder eine gegebenenfalls durch eine C₁₋₆-Alkylgruppe substituierte Methylengruppe,
U eine C₁₋₆-Alkyl-, C₂₋₆-Alkenyl- oder C₂₋₆-Alkinylgruppe, in der jede Methylengruppe mit bis zu 2 Fluoratomen und jede Methylgruppe mit bis zu 3 Fluoratomen substituiert sein kann,
V ein Chlor- oder Bromatom, eine Amino-, Methylamino- oder Hydroxygruppe,
W ein Wasserstoff-, Fluor-, Chlor-, Brom- oder Iodatom, eine Difluor- oder Trifluormethylgruppe,
R¹ einen gesättigten, einfach oder zweifach ungesättigten 5- bis 7-gliedrigen Aza-, Diaza-, Triaza-, Oxaza-, Thiaza-, Thiadiaza- oder S,S-Dioxido-thiadiaza-Heterocyclus,
wobei die vorstehend erwähnten Heterocyclen über ein Kohlenstoff- oder Stickstoffatom verknüpft sind,
eine oder zwei Carbonyl- oder Thiocarbonylgruppen benachbart zu einem Stickstoffatom enthalten,
an einem der Stickstoffatome durch eine Alkylgruppe substituiert sein können,
an einem oder an zwei Kohlenstoffatomen durch eine Alkylgruppe, durch eine Phenyl-, Phenylmethyl-, Naphthyl-, Biphenylyl-, Pyridinyl-, Diazinyl-, Furyl-, Thienyl-, Pyrrolyl-, 1,3-Oxazolyl-, 1,3-Thiazolyl-, Isoxazolyl-, Pyrazolyl-, 1-Methylpyrazolyl-, Imidazolyl- oder 1-Methylimidazolylgruppen substituiert sein können, wobei die Substituenten gleich oder verschieden sein können, und
wobei eine olefinische Doppelbindung eines der vorstehend erwähnten ungesättigten Heterocyclen mit einem Phenyl-, Naphthyl-, Pyridin-, Diazin-, 1,3-Oxazol-, Thienyl-, Furan-, Thiazol-, Pyrrol-, N-Methylpyrrol- oder Chinolin-Ring, mit einem gegebenenfalls am Stickstoffatom durch eine Alkylgruppe substituierten 1*H*-Chinolin-2-on-Ring oder mit einem Imidazol- oder N-Methylimidazol-Ring kondensiert sein kann oder auch zwei olefinische Doppelbindungen eines der vorstehend erwähnten ungesättigten Heterocyclen jeweils mit einem Phenylring kondensiert sein können,
wobei die in R¹ enthaltenen Phenyl-, Pyridinyl-, Diazinyl-, Furyl-, Thienyl-, Pyrrolyl-, 1,3-Oxazolyl-, 1,3-Thiazolyl-, Isoxazolyl-, Pyrazolyl-, 1-Methylpyrazolyl-, Imidazolyl- oder 1-Methylimidazolylgruppen sowie benzo-, thieno-, pyrido- und diazinokondensierten Heterocyclen im Kohlenstoffgerüst zusätzlich durch Fluor-, Chlor-, Brom- oder lodatome, durch Alkyl-, Alkoxy-, Nitro-, Alkylthio-, Alkylsulfinyl-, Alkylsulfonyl-, Alkylsulfonylamino-, Phenyl-, Difluormethyl-, Trifluormethyl-, Alkoxycarbonyl-, Carboxy-, Hydroxy-, Amino-, Alkylamino-, Dialkylamino-, Acetyl-, Acetylamino-, Propionylamino-, Aminocarbonyl-, Alkylaminocarbonyl-, Dialkylaminocarbonyl-, (4-Morpholinyl)-carbonyl-, (1-Pyrrolidinyl)carbonyl-, (1-Piperidinyl)carbonyl-, (Hexahydro-1-azepinyl)carbonyl-, (4-Methyl-1-piperazinyl)carbonyl-, Methylendioxy-, Aminocarbonylamino-, Alkanoyl-, Cyano-, Difluormethoxy-, Trifluormethoxy-, Trifluormethylthio-, Trifluormethylsulfinyl- oder Trifluormethylsulfonylgruppen mono-, di- oder trisubstituiert sein können, wobei die Substituenten gleich oder verschieden sein können,
R² das Wasserstoffatom,
eine Phenylmethylgruppe oder eine C₂₋₇-Alkylgruppe, die in ω-Stellung durch eine Cyclohexyl-, Phenyl-, Pyridinyl-, Diazinyl-, Hydroxy-, Amino-, Alkylamino-, Dialkylamino-, Carboxy-, Alkoxycarbonyl-, Aminocarbonyl-, Aminocarbonylamino-, Acetylamino-, 1-Pyrrolidinyl-, 1-Piperidinyl-, 4-(1-Piperidinyl)-1-piperidinyl-, 4-Morpholinyl-, Hexahydro-1*H*-1-azepinyl-, [Bis-(2-hydroxyethyl)]amino-, 4-Alkyl-1-piperazinyl- oder 4-(ω-Hydroxy-C₂₋₇-alkyl)-1-piperazinylgruppe substituiert sein kann,
eine Phenyl- oder Pyridinylgruppe,
wobei die vorstehend erwähnten heterocyclischen Reste und Phenylgruppen zusätzlich im Kohlenstoffgerüst durch Fluor-, Chlor-, Brom- oder lodatome, durch Methyl-, Alkoxy-, Difluormethyl-, Trifluormethyl-, Hydroxy-, Amino-, C₁₋₃-Alkylamino-, Di-(C₁₋₃-alkyl)-amino-, Acetylamino-, Aminocarbonyl-, Cyano-, Methylsulfonyloxy-, Difluormethoxy-, Trifluormethoxy-, Trifluormethylthio-, Trifluormethylsulfinyl-, Trifluormethylsulfonyl-, Amino-C₁₋₃-alkyl-, C₁₋₃-Alkylamino-C₁₋₃-alkyl- oder Di-(C₁₋₃-alkyl)-amino-C₁₋₃-alkylgruppen mono-, di- oder trisubstituiert und die Substituenten gleich oder verschieden sein können,
R³ das Wasserstoffatom oder eine gegebenenfalls durch eine Phenyl- oder Pyridinylgruppe substituierte C₁₋₃-Alkylgruppe,
wobei die C₁₋₃-Alkylgruppe mit einer in R² vorhandenen Alkylgruppe oder einem in R² vorhandenen Phenyl- oder Pyridylring und dem Stickstoffatom, an dem sie gebunden sind, unter Ausbildung eines Ringes verbunden sein kann oder
R² und R³ zusammen mit dem eingeschlossenen Stickstoffatom einen Rest der allgemeinen Formel 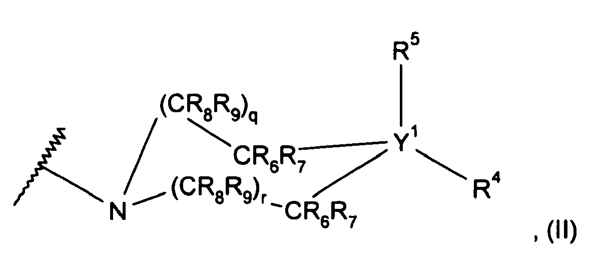 in der
Y¹ das Kohlenstoffatom oder, wenn R⁵ ein freies Elektronenpaar darstellt, auch das Stickstoffatom,
q und r, wenn Y¹ das Kohlenstoffatom darstellt, die Zahlen 0, 1 oder 2, oder
q und r, wenn Y¹ das Stickstoffatom darstellt, die Zahlen 1 oder 2,
R⁴ das Wasserstoffatom, eine Amino-, Alkylamino-, Cycloalkylamino-, Dialkylamino-, *N*-(Cycloalkyl)-aikylamino-, Dicycloalkylamino-, Hydroxy-, Alkyl-, Cycloalkyl-, Amino-C₂₋₇-alkyl-, Alkylamino-C₂₋₇-alkyl-, Dialkylamino-C₂₋₇-alkyl-, Aminoiminomethyl-, Alkylcarbonyl-, Alkylsulfonyl-, Alkylcarbonylamino-, Alkylsulfonylamino-, *N*-Alkylcarbonyl-*N*-alkylamino-, N-Alkylsulfonyl-N-alkylamino-, Aminocarbonylamino-, Alkylaminocarbonylamino-, Dialkylaminocarbonylamino-, Cycloalkylaminocarbonylamino-, Dicycloalkylaminocarbonylamino-, Phenylaminocarbonylamino-, Aminocarbonylalkyl-, Alkylaminocarbonylalkyl-, Dialkylaminocarbonylalkyl-, Aminocarbonylaminoalkyl-, Alkoxycarbonyl-, Alkoxycarbonylalkyl- oder Carboxyalkylgruppe,
oder auch, wenn Y¹ nicht das Stickstoffatom darstellt, die Carboxy-, Aminomethyl-, Alkylaminomethyl- oder Dialkylaminomethylgruppe,
eine Phenyl-, Phenyl-C₁₋₃-alkyl-, Pyridinyl-, Diazinyl-, 1-Naphthyl-, 2-Naphthyl-, Pyridinylcarbonyl- oder Phenylcarbonylgruppe, die jeweils im Kohlenstoffgerüst durch Fluor-, Chlor-, Brom- oder lodatome, durch Alkyl-, Alkoxy-, Methylsulfonyloxy-, Difluormethyl-, Trifluormethyl-, Hydroxy-, Amino-, Acetylamino-, Aminocarbonyl-, Aminocarbonylamino-, Aminocarbonylaminomethyl-, Cyano-, Carboxy-, Alkoxycarbonyl-, Carboxyalkyl-, Alkoxycarbonylalkyl-, Alkanoyl-, ω-(Dialkylamino)alkanoyl-, ω-(Dialkylamino)alkyl-, ω-(Dialkylamino)hydroxyalkyl-, ω-(Carboxy)alkanoyl-, Difluormethoxy-, Trifluormethoxy-, Trifluormethylthio-, Trifluormethylsulfinyl- oder Trifluormethylsulfonylgruppen mono-, di- oder trisubstituiert sein können, wobei die Substituenten gleich oder verschieden sein können,
eine gesättigte oder ein- oder mehrfach ungesättigte 4- bis 10-gliedrige Azacycloalkylgruppe, eine 5- bis 10-gliedrige Oxaza-, Thiaza-, Diaza- oder Triazacycloalkylgruppe, eine 6- bis 10-gliedrige Azabicyclo- oder Diazabicycloalkylgruppe, eine 1-Alkyl-4-piperidinylcarbonyl- oder 4-Alkyl-1-piperazinylcarbonyl-, eine 1-Alkyl-4-piperidinylamino-, 1-Alkyl-4-piperidinylaminocarbonyl- oder 1-Alkyl-4-piperidinylaminosulfonylgruppe,
wobei die vorstehend genannten mono- und bicyclischen Heterocyclen über ein Stickstoff- oder ein Kohlenstoffatom gebunden sind,
eine Methylengruppe in den vorstehend genannten mono- und bicyclischen Heterocyclen durch eine Carbonyl- oder Sulfonylgruppe ersetzt sein kann,
in den vorstehend genannten mono- und bicyclischen Heterocyclen eine nicht direkt an ein Stickstoff-, Sauerstoff- oder Schwefelatom gebundene Methylengruppe durch ein oder zwei Fluoratome substituiert sein kann,
die vorstehend genannten mono- und bicyclischen Heterocyclen sowie die 1-Alkyl-4-piperidinylcarbonyl- und 4-Alkyl-1-piperazinylcarbonylgruppe im Ring ein- oder mehrfach durch eine C₁₋₇-Alkylgruppe oder/und
einfach durch eine Benzyl-, Alkanoyl-, Dialkylamino-, Phenylcarbonyl-, Pyridinylcarbonyl-, Carboxy-, Carboxyalkanoyl-, Carboxyalkyl-, Alkoxycarbonylalkyl-, Alkoxycarbonyl-, Aminocarbonyl-, Alkylaminocarbonyl-, Alkylsulfonyl-, Cycloalkyl- oder Cycloalkylalkylgruppe, durch eine im Ring gegebenenfalls alkylsubstituierte Cycloalkylcarbonyl-, Azacycloalkylcarbonyl-, Diazacycloalkylcarbonyl- oder Oxazacycloalkylcarbonylgruppe substituiert sein können,
wobei die in diesen Substituenten enthaltenen alicyclischen Teile 3 bis 10 Ringglieder und die heteroalicyclischen Teile jeweils 4 bis 10 Ringglieder umfassen und
die in den vorstehend genannten Resten enthaltenen Phenyl- und Pyridinyl-Reste ihrerseits durch Fluor-, Chlor-, Brom- oder lodatome, durch Alkyl-, Alkoxy-, Methylsulfonyloxy-, Difluormethyl-, Trifluormethyl-, Hydroxy-, Amino-, Acetylamino-, Aminocarbonyl-, Aminocarbonylamino-, Aminocarbonylaminomethyl-, Cyano-, Carboxy-, Alkoxycarbonyl-, Carboxyalkyl-, Alkoxycarbonylalkyl-, Alkanoyl-, ω-(Dialkylamino)alkanoyl-, ω-(Carboxy)alkanoyl-, Difluormethoxy-, Trifluormethoxy-, Trifluormethylthio-, Trifluormethylsulfinyl- oder Trifluormethylsulfonylgruppen mono-, di- oder trisubstituiert sein können, wobei die Substituenten gleich oder verschieden sein können,
R⁵ ein Wasserstoffatom,
einen C₁₋₄-Alkylrest, wobei ein unverzweigter Alkylrest in ω-Stellung durch eine Phenyl-, Pyridinyl-, Diazinyl-, Amino-, Alkylamino-, Dialkylamino-, 1-Pyrrolidinyl-, 1-Piperidinyl-, 4-Methyl-1-piperazinyl-, 4-Morpholinyl- oder Hexahydro-1*H*-1-azepinyl-Gruppe substituiert sein kann,
eine Alkoxycarbonyl-, die Cyano- oder Aminocarbonylgruppe oder auch, wenn Y¹ ein Stickstoffatom darstellt, ein freies Elektronenpaar,
oder, wenn Y¹ kein Stickstoffatom darstellt, auch das Fluoratom, oder
R⁴ und R⁵ zusammen, wenn Y¹ das Kohlenstoffatom darstellt, einen 4- bis 7-gliedrigen cycloaliphatischen Ring, in dem eine eine oder zwei Methylengruppen durch eine -NH- oder -N(Alkyl)-Gruppe und eine oder zwei weitere Methylengruppen durch Carbonylgruppen ersetzt sein kann,
wobei ein an ein Stickstoffatom innerhalb der voranstehend genannten Gruppe R⁴ gebundenes Wasserstoffatom durch einen Schutzrest ersetzt sein kann,
R⁶ und R⁷, die gleich oder verschieden sein können, jeweils ein Wasserstoffatom, eine C₁₋₃-Alkyl- oder Dialkylaminogruppe oder auch, wenn Y¹ kein Stickstoffatom darstellt, das Fluoratom und
R⁸ und R⁹, die gleich oder verschieden sein können, jeweils ein Wasserstoffatom oder eine C₁₋₃-Alkyl-, Carboxy- oder C₁₋₃-Alkoxycarbonylgruppe darstellen,
wobei alle vorstehend genannten Alkyl- und Alkoxygruppen sowie die innerhalb der anderen genannten Reste vorhandenen Alkylgruppen, sofern nichts anderes angegeben ist, 1 bis 7 Kohlenstoffatome umfassen und geradkettig oder verzweigt sein können, wobei jede Methylengruppe mit bis zu 2 Fluoratomen und jede Methylgruppe mit bis zu 3 Fluoratomen substituiert sein kann,
alle vorstehend genannten Cycloalkylgruppen sowie die innerhalb der anderen genannten Reste vorhandenen Cycloalkylgruppen, sofern nichts anderes angegeben ist, 3 bis 10 Kohlenstoffatome umfassen können, wobei jede Methylengruppe mit bis zu 2 Fluoratomen substituiert sein kann, und
alle vorstehend erwähnten aromatischen und heteroaromatischen Reste zusätzlich durch Fluor-, Chlor- oder Bromatome, durch Cyano- oder Hydroxygruppen mono-, di- oder trisubstituiert und die Substituenten gleich oder verschieden sein können,
bedeuten, deren Tautomere, deren Diastereomere, deren Enantiomere, deren Hydrate, deren Gemische und deren Salzes sowie die Hydrate der Salze.

2. Verbindungen der allgemeinen Formel (I) gemäß Anspruch 1, in der
A, U, V, W, X, R² und R³ wie in Anspruch 1 definiert sind und
R¹ einen einfach oder zweifach ungesättigten 5- bis 7-gliedrigen Aza-, Diaza-, Triaza- oder Thiaza-Heterocyclus bedeutet,
wobei die vorstehend erwähnten Heterocyclen über ein Kohlenstoff- oder Stickstoffatom verknüpft sind,
eine oder zwei Carbonylgruppen benachbart zu einem Stickstoffatom enthalten,
an einem Kohlenstoffatom durch eine Phenyl-, Pyridinyl-, Diazinyl-, Thienyl-, Pyrrolyl-, 1,3-Thiazolyl-, Isoxazolyl-, Pyrazolyl- oder 1-Methylpyrazolyl-Gruppe substituiert sein können und
eine olefinische Doppelbindung eines der vorstehend erwähnten ungesättigten Heterocyclen mit einem Phenyl-, Naphthyl-, Pyridin-, Diazin-, Thienyl- oder Chinolin-Ring oder mit einem gegebenenfalls am Stickstoffatom durch eine Methylgruppe substituierten 1*H*-Chinolin-2-on-Ring kondensiert sein kann,
wobei die in R¹ enthaltenen Phenyl-, Pyridinyl-, Diazinyl-, Thienyl-, Pyrrolyl-, 1,3-Thiazolyl-, Isoxazolyl-, Pyrazolyl- oder 1-Methylpyrazolylgruppen sowie die benzo-, pyrido- und diazinokondensierten Heterocyclen im Kohlenstoffgerüst zusätzlich durch Fluor-, Chlor-, Brom- oder lodatome, durch Alkyl-, Alkoxy-, Nitro-, Difluormethyl-, Trifluormethyl-, Hydroxy-, Amino-, Alkylamino-, Dialkylamino-, Acetylamino-, Acetyl-, Cyano-, Difluormethoxy- oder Trifluormethoxygruppen mono-, di- oder trisubstituiert sein können, wobei die Substituenten gleich oder verschieden sein können,
wobei die vorstehend genannten Alkylgruppen oder die in den vorstehend genannten Resten enthaltenen Alkylgruppen, sofern nichts anderes angegeben wurde, 1 bis 7 Kohlenstoffatome enthalten und verzweigt oder unverzweigt sein können, wobei jede Methylengruppe mit bis zu 2 Fluoratomen und jede Methylgruppe mit bis zu 3 Fluoratomen substituiert sein kann, und
die vorstehend erwähnten aromatischen und heteroaromatischen Reste zusätzlich durch Fluor-, Chlor- oder Bromatome, durch Cyano- oder Hydroxygruppen mono-, di- oder trisubstituiert und die Substituenten gleich oder verschieden sein können,
deren Tautomere, deren Diastereomere, deren Enantiomere, deren Gemische und deren Salze.

3. Verbindungen der allgemeinen Formel (I) gemäß Anspruch 1, in der
A, U, V, W, X, R² und R³ wie in Anspruch 1 definiert sind und
R¹ einen einfach ungesättigten 5- bis 7-gliedrigen Diaza- oder Triaza-Heterocyclus bedeutet,
wobei die vorstehend erwähnten Heterocyclen über ein Stickstoffatom verknüpft sind,
eine Carbonylgruppe benachbart zu einem Stickstoffatom enthalten und
zusätzlich an einem Kohlenstoffatom durch eine Phenylgruppe substituiert sein können,
und wobei eine olefinische Doppelbindung eines der vorstehend erwähnten ungesättigten Heterocyclen mit einem Phenyl-, Thienyl- oder Chinolin-Ring kondensiert sein kann,
wobei die in R¹ enthaltenen Phenylgruppen sowie benzokondensierten Heterocyclen im Kohlenstoffgerüst zusätzlich durch Fluor-, Chlor-, Brom- oder lodatome, durch Methyl-, Methoxy-, Nitro-, Difluormethyl-, Trifluormethyl-, Hydroxy-, Amino-, Alkylamino-, Dialkylamino-, Acetylamino-, Acetyl-, Cyano- Difluormethoxy- oder Trifluormethoxygruppen mono-, di- oder trisubstituiert sein können, wobei die Substituenten gleich oder verschieden sein können, vorzugsweise jedoch unsubstituiert oder durch ein Fluor-, Chlor- oder Bromatom, durch eine Methyl- oder Methoxygruppe monosubstituiert sind,
wobei alle vorstehend genannten Alkylgruppen sowie die innerhalb der anderen Reste vorhandenen Alkylgruppen, sofern nichts anderes angegeben ist, 1 bis 7 Kohlenstoffatome umfassen und geradkettig oder verzweigt sein können und die vorstehend erwähnten aromatischen und heteroaromatischen Reste zusätzlich durch Fluor-, Chlor- oder Bromatome, durch Cyano- oder Hydroxygruppen mono-, di- oder trisubstituiert und die Substituenten gleich oder verschieden sein können,
deren Tautomere, deren Diastereomere, deren Enantiomere, deren Gemische und deren Salze.

4. Verbindungen der allgemeinen Formel (I) gemäß Anspruch 1, in der
A, U, V, W, X, R² und R³ wie in Anspruch 1 definiert sind und
R¹ eine 4-(2-Oxo-1,2,4,5-tetrahydro-1,3-benzdiazepin-3-yl)-, 4-(2-Oxo-1,4-dihydro-2H-chinazolin-3-yl)-, 4-(5-Oxo-3-phenyl-4,5-dihydro-1,2,4-triazol-1-yl)-, 4-(2-Oxo-1,2-dihydro-imidazo[4,5-c]chinolin-3-yl)-, 4-(2-Oxo-1,2-dihydro-4*H*-thieno[3,4-d]pyrimidin-3-yl)-, 4-(2-Oxo-1,4-dihydro-2*H*-thieno[3,2-d]pyrimidin-3-yl)-, 4-(5-Oxo-4,5,7,8-tetrahydro-2-thia-4,6-diaza-azulen-6-yl)-, 4-(2-Oxo-1,2,4,5-tetrahydro-thieno[3,2-d]-1,3-diazepin-3-yl)-, 4-(2-Oxo-1,2,4,5-tetrahydro-thieno[2,3-d]-1,3-diazepin-3-yl)- oder 4-(2-Oxo-1,4-dihydro-2*H*-thieno[2,3-d]pyrimidin-3-yl)-gruppe bedeutet,
wobei die vorstehend erwähnten mono- und bicyclischen Heterocyclen im Kohlenstoffgerüst zusätzlich durch eine Methoxygruppe monosubstituiert sein können,
wobei die vorstehend erwähnten aromatischen und heteroaromatischen Reste zusätzlich durch Fluor-, Chlor- oder Bromatome, durch Cyano- oder Hydroxygruppen mono-, di- oder trisubstituiert und die Substituenten gleich oder verschieden sein können,
deren Tautomere, deren Diastereomere, deren Enantiomere, deren Gemische und deren Salze.

5. Verbindungen der allgemeinen Formel (I) gemäß Anspruch 1, in der
A, U, V, W, X und R¹ wie in Anspruch 1 definiert sind und
R² das Wasserstoffatom oder
eine Phenylmethylgruppe oder eine C₂₋₇-Alkylgruppe, die in ω-Stellung durch eine Phenyl-, Pyridinyl-, Hydroxy-, Amino-, Alkylamino-, Dialkylamino-, Carboxy-, Alkoxycarbonyl-, Aminocarbonyl-, Aminocarbonylamino-, Acetylamino-, 1-Pyrrolidinyl-, 1-Piperidinyl-, 4-Morpholinyl-, [Bis-(2-hydroxyethyl)]aminogruppe substituiert sein kann,
wobei die vorstehend erwähnten heterocyclischen Reste und Phenylgruppen zusätzlich im Kohlenstoffgerüst durch Fluor-, Chlor-, Brom- oder lodatome, durch Methyl-, Alkoxy-, Difluormethyl-, Trifluormethyl-, Hydroxy-, Amino-, C₁₋₃-Alkylamino-, Di-(C₁₋₃-alkyl)-amino-, Acetylamino-, Aminocarbonyl-, Cyano-, Difluormethoxy-, Trifluormethoxy-, Amino-C₁₋₃-alkyl-, C₁₋₃-Alkylamino-C₁₋₃-alkyl- oder Di-(C₁₋₃-Alkyl)-amino-C₁₋₃-alkylgruppen mono-, di- oder trisubstituiert und die Substituenten gleich oder verschieden sein können,
R³ das Wasserstoffatom oder eine C₁₋₃-Alkylgruppe,
wobei die C₁₋₃-Alkylgruppe mit einer in R² vorhandenen Alkylgruppe oder einem in R² vorhandenen Phenyl- oder Pyridylring und dem Stickstoffatom, an dem sie gebunden sind, unter Ausbildung eines 5- bis 7-gliedrigen Ringes verbunden sein kann oder
R² und R³ zusammen mit dem eingeschlossenen Stickstoffatom einen Rest der allgemeinen Formel 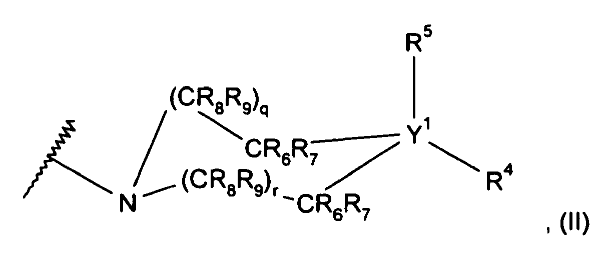 in der
Y¹ das Kohlenstoffatom oder, wenn R⁵ ein freies Elektronenpaar darstellt, auch das Stickstoffatom,
q und r, wenn Y¹ das Kohlenstoffatom darstellt, die Zahlen 0 oder 1 oder
q und r, wenn Y¹ das Stickstoffatom darstellt, die Zahlen 1 oder 2,
R⁴ das Wasserstoffatom, eine Hydroxy-, Amino-, Alkylamino-, C₃₋₆-Cycloalkylamino-, *N*-(C₃₋₆-Cycloalkyl)-alkylamino- oder Dialkylamino-, eine Alkyl-, Trifluormethyl-, C₃₋₆-Cycloalkyl-, Dialkylamino-C₂₋₇-alkyl-, Carboxyalkyl-, Alkoxycarbonylalkyl-, Alkylsulfonyl-, Alkylsulfonylamino- oder *N*-(Alkylsulfonyl)-alkylaminogruppe,
oder auch, wenn Y¹ nicht das Stickstoffatom darstellt, die Carboxy- oder Dialkylaminomethylgruppe,
eine Phenyl-, Phenyl-C₁₋₃-alkyl-, Pyridinyl- oder Diazinylgruppe, die jeweils durch ein Fluor-, Chlor- oder Bromatom, durch eine Trifluormethylcarbonyl-, Methyl- oder Methoxygruppe substituiert sein können,
eine gesättigte oder ein- oder mehrfach ungesättigte 4- bis 7-gliedrige Azacycloalkylgruppe, eine 5- bis 7-gliedrige Oxaza-, Diaza- oder Triazacycloalkylgruppe, eine 7- bis 9-gliedrige Azabicyclo- oder Diazabicycloalkylgruppe, eine 1-Alkyl-4-piperidinylamino- oder 1-Alkyl-4-piperidinylaminosulfonylgruppe,
wobei die vorstehend genannten mono- und bicyclischen Heterocyclen über ein Stickstoff- oder ein Kohlenstoffatom gebunden sind,
eine Methylengruppe der vorstehend genannten mono- und bicyclischen Heterocyclen durch eine Carbonyl- oder Sulfonylgruppe ersetzt sein kann,
in den vorstehend genannten mono- und bicyclischen Heterocyclen eine nicht direkt an ein Stickstoff-, Sauerstoff- oder Schwefelatom gebundene Methylengruppe durch ein oder zwei Fluoratome substituiert sein kann,
die vorstehend genannten mono- und bicyclischen Heterocyclen durch eine oder zwei C₁₋₃-Alkylgruppen, in der jede Methylengruppe mit bis zu 2 und jede Methylgruppe mit bis zu 3 Fluoratomen substituiert sein kann, oder/und
durch eine C₃₋₆-Cycloalkyl-C₁₋₃-alkyl-, Benzyl-, C₁₋₄-Alkanoyl-, Di-(C₁₋₃-alkyl)-amino- oder C₁₋₃-Alkylsulfonyl-, durch eine Alkoxycarbonyl-, Benzyloxycarbonyl-, Alkoxycarbonylalkyl-, Carboxy- oder Carboxyalkylgruppe substituiert sein können,
R⁵ ein Wasserstoffatom, eine C₁₋₃-Alkyl- oder Alkoxycarbonylgruppe oder,
wenn Y¹ ein Stickstoffatom darstellt, auch ein freies Elektronenpaar, oder
R⁴ und R⁵ zusammen, wenn Y¹ das Kohlenstoffatom darstellt, einen 5- bis 6-gliedrigen cycloaliphatischen Ring, in dem eine oder zwei Methylengruppen durch eine -NH- oder -N(Methyl)-Gruppe und eine oder zwei weitere Methylengruppen durch Carbonylgruppen ersetzt sein können,
R⁶ und R⁷, die gleich oder verschieden sein können, jeweils ein Wasserstoffatom oder eine C₁₋₃-Alkyl- oder Di-(C₁₋₃-alkyl)-aminogruppe und
R⁸ und R⁹, die gleich oder verschieden sein können, jeweils ein Wasserstoffatom oder eine C₁₋₃-Alkyl-, Carboxy- oder C₁₋₃-Alkoxycarbonylgruppe darstellen,
wobei alle vorstehend genannten Alkylgruppen sowie die innerhalb der anderen Reste vorhandenen Alkylgruppen, sofern nichts anderes angegeben ist, 1 bis 7 Kohlenstoffatome umfassen und geradkettig oder verzweigt sein können und die vorstehend erwähnten aromatischen und heteroaromatischen Reste zusätzlich durch Fluor-, Chlor- oder Bromatome, durch Cyano- oder Hydroxygruppen mono-, di- oder trisubstituiert und die Substituenten gleich oder verschieden sein können,
bedeuten, deren Tautomere, deren Diastereomere, deren Enantiomere, deren Gemische und deren Salze.

6. Verbindungen der allgemeinen Formel (I) gemäß Anspruch 1, in der
A, U, V, W, X und R¹ wie in Anspruch 1 definiert sind und
R² eine Phenylmethylgruppe oder eine C₂₋₇-Alkylgruppe, die in ω-Stellung durch eine Phenyl-, Amino-, Alkylamino- oder Dialkylaminogruppe substituiert sein kann,
wobei die vorstehend genannte Phenylgruppe durch eine Amino-C₁₋₃-alkyl-, C₁₋₃-Alkylamino-C₁₋₃alkyl- oder Di-(C₁₋₃-alkyl)-amino-C₁₋₃-alkylgruppe substituiert sein kann,
R³ das Wasserstoffatom oder eine C₁₋₃-Alkylgruppe,
R² und R³ zusammen mit dem Stickstoffatom, an dem sie gebunden sind, eine 7-Dimethylaminomethyl-1,2,4,5-tetrahydro-3-benzazepin-3-yl- oder 2-Amino-4,5,7,8-tetrahydro-thiazolo[4,5-d]azepin-6-yl-gruppe oder
R² und R³ zusammen mit dem eingeschlossenen Stickstoffatom einen Rest der allgemeinen Formel 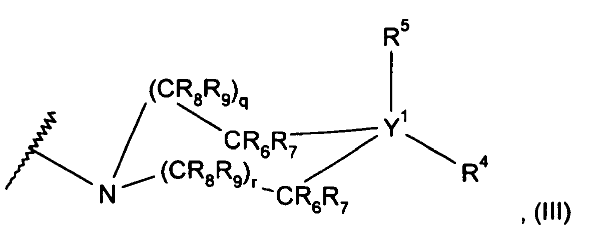 in der
Y¹ das Kohlenstoffatom oder, wenn R⁵ ein freies Elektronenpaar darstellt, auch das Stickstoffatom,
q und r, wenn Y¹ das Kohlenstoffatom darstellt, die Zahlen 0 oder 1 oder
q und r, wenn Y¹ das Stickstoffatom darstellt, die Zahlen 1 oder 2,
R⁴ das Wasserstoffatom,
eine Phenyl-, Benzyl- oder Pyridinylgruppe, die jeweils durch ein Fluor-, Chlor- oder Bromatom, durch eine Trifluormethylcarbonyl-, Methyl- oder Methoxygruppe substituiert sein können,
eine Hydroxy-, Carboxy-, Methyl-, Trifluormethyl-, *n*-Propyl-, Phenyl-, *p*-Tolyl-, *p*-Trifluormethylcarbonyl-phenyl-, *p*-(3-Dimethylaminopropyl)-phenyl-, Amino-, Benzyl-, *tert-*Butylamino-, Dimethylamino-, Diethylamino-, Diethylaminomethyl-, 2-Dimethylaminoethyl-, 2-Diethylaminoethyl-, 5-Aminopentyl-, Methoxycarbonyl-, Methoxycarbonylmethyl-, Perhydro-azepin-1-yl-, 4-Methyl-perhydro-1,4-diazepin-1-yl-, 1-Methyl-1-piperidinyl-4-yl-, 4-Piperazin-1-yl-, 4-Acetyl-piperazin-1-yl-, 4-Cyclopropylmethyl-piperazin-1-yl-, Pyrrolidin-1-yl-, 4-Ethyl-piperazin-1-yl-, 4-Isopropyl-piperazin-1-yl-, Piperidin-1-yl-, Piperidin-4-yl-, Morpholin-4-yl-, 4,4-Difluor-1-piperidin-1-yl-, 1-Methyl-1-aza-bicydo[3.2.1]oct-4-yl-, 4-Methylpiperazin-1-yl-, 4-Ethylpiperazin-1-yl-, 1-Methyl-piperidin-1-yl-, 4-Carboxymethyl-piperazin-1-yl-, 1-Carboxymethyl-piperidin-4-yl-, 4-Benzyloxycarbonyl-piperazin-1-yl-, 1-Ethoxycarbonylmethyl-piperidin-4-yl-, Azetidin-1-yl-, 5-Methyl-2,5-diaza-bicyclo[2.2.1]hept-2-yl-, 1-Benzyl-piperidin-4-yl-, 4-Benzyl-piperazin-1-yl-, 4-Dimethylaminomethyl-1-phenyl-, 2,2,2-Trifluorethyl-piperazin-1-yl-, 1-Methylsulfonyl-piperidin-4-yl-, Piperidin-1-yl-methyl-, 1-Methyl-piperidin-4-yl-amino-, Methylsulfonylamino-, *N*-Methylsulfonyl-*N*-methylamino-, *N*-(Cyclopentyl)-methylamino-, 1,1-Dioxo-λ⁶-isothiazolidin-2-yl-, 2-Oxo-perhydro-1,3-oxazin-3-yl-, Cyclohexyl-, 2-Oxo-imidazolidin-1-yl-, 2-Methyl-imidazol-1-yl-, 4-Methyl-imidazol-1-yl-, 4-Thiazol-2-yl-, 2,4-Dimethyl-imidazol-1-yl-, 4-Imidazol-1-yl-, 1,2,4-Triazol-1-yl-, 1-Aza-bicyclo[2.2.2]oct-3-yl-, 1-Methyl-piperidin-4-yl-methylsulfonyl-, 1*H*-Imidazol-4-yl-, 4-Ethoxycarbonylmethyl-piperazin-1-yl-, 1-Ethoxycarbonyl-piperidin-4-yl-, 4-*tert*-Butoxycarbonylmethyl-piperazin-1-yl-, 1-(2,2,2-Trifluorethyl)-piperidin-4-yl-, 4-Methylsulfonyl-piperazin-1-yl-, 2-Carboxy-4-methyl-piperazin-1-yl-, 3-Carboxy-4-methyl-piperazin-1-yl-, 2-Ethoxycarbonyl-4-methyl-piperazin-1-yl-, 3-Ethoxycarbonyl-4-methyl-piperazin-1-yl- oder 4-(2,2,2-Trifluorethyl)-piperazin-1-yl-gruppe,
R⁵ ein Wasserstoffatom, eine Methylgruppe oder, wenn Y¹ ein Stickstoffatom bedeutet, auch ein freies Elektronenpaar, oder
R⁴ und R⁵ zusammen, wenn Y¹ das Kohlenstoffatom darstellt, eine 1-Methylpiperidin-4-yliden-, Cyclohexyliden- oder Imidazolidin-2,4-dion-5-yliden-gruppe darstellen,
R⁶ und R⁷ jeweils ein Wasserstoffatom oder eine Dimethylaminogruppe und
R⁸ und R⁹ jeweils das Wasserstoffatom, eine Carboxy- oder Ethoxycarbonylgruppe darstellen,
wobei alle vorstehend genannten Alkylgruppen sowie die innerhalb der anderen Reste vorhandenen Alkylgruppen, sofern nichts anderes angegeben ist, 1 bis 7 Kohlenstoffatome umfassen und geradkettig oder verzweigt sein können und die vorstehend erwähnten aromatischen und heteroaromatischen Reste zusätzlich durch Fluor-, Chlor- oder Bromatome, durch Cyano- oder Hydroxygruppen mono-, di- oder trisubstituiert und die Substituenten gleich oder verschieden sein können,
bedeuten, deren Tautomere, deren Diastereomere, deren Enantiomere, deren Gemische und deren Salze.

7. Verbindungen der allgemeinen Formel (I) gemäß Anspruch 1, in der
A ein Sauerstoffatom, eine Cyanimino- oder Phenylsulfonyliminogruppe,
X ein Sauerstoffatom, eine Imino- oder Methylengruppe,
U eine unverzweigte C₁₋₆-Alkyl-, C₂₋₆-Alkenyl- oder C₂₋₆-Alkinylgruppe, in der jede Methylengruppe mit bis zu 2 Fluoratomen und die Methylgruppe mit bis zu 3 Fluoratomen substituiert sein kann,
V eine Amino- oder Hydroxygruppe,
W ein Wasserstoff-, Chlor- oder Bromatom oder eine Trifluormethylgruppe,
R¹ einen einfach ungesättigten 5- bis 7-gliedrigen Diaza- oder Triaza-Heterocyclus,
wobei die vorstehend erwähnten Heterocyclen über ein Stickstoffatom verknüpft sind,
eine Carbonylgruppe benachbart zu einem Stickstoffatom enthalten,
zusätzlich an einem Kohlenstoffatom durch eine Phenylgruppe substituiert sein können und
wobei eine olefinische Doppelbindung eines der vorstehend erwähnten ungesättigten Heterocyclen mit einem Phenyl-, Thienyl- oder Chinolin-Ring kondensiert sein kann,
wobei die in R¹ enthaltenen Phenylgruppen sowie benzokondensierten Heterocyclen im Kohlenstoffgerüst zusätzlich durch Fluor-, Chlor-, Brom- oder lodatome, durch Methyl-, Methoxy-, Nitro-, Difluormethyl-, Trifluormethyl-, Hydroxy-, Amino-, Alkylamino-, Dialkylamino-, Acetylamino-, Acetyl-, Cyano- Difluormethoxy- oder Trifluormethoxygruppen mono-, di- oder trisubstituiert sein können, wobei die Substituenten gleich oder verschieden sein können, vorzugsweise jedoch unsubstituiert oder durch ein Fluor-, Chlor- oder Bromatom, durch eine Methyl- oder Methoxygruppe monosubstituiert sind,
R² das Wasserstoffatom oder
eine Phenylmethylgruppe oder eine C₂₋₇-Alkylgruppe, die in ω-Stellung durch eine Phenyl-, Pyridinyl-, Hydroxy-, Amino-, Alkylamino-, Dialkylamino-, Alkoxycarbonyl-, Carboxy-, Aminocarbonyl-, Aminocarbonylamino-, Acetylamino-, 1-Pyrrolidinyl-, 1-Piperidinyl-, 4-Morpholinyl-, [Bis-(2-hydroxyethyl)]aminogruppe substituiert sein kann,
wobei die vorstehend erwähnten heterocyclischen Reste und Phenylgruppen zusätzlich im Kohlenstoffgerüst durch Fluor-, Chlor-, Brom- oder lodatome, durch Methyl-, Alkoxy-, Difluormethyl-, Trifluormethyl-, Hydroxy-, Amino-, C₁₋₃-Alkylamino-, Di-(C₁₋₃-alkyl)-amino-, Acetylamino-, Aminocarbonyl-, Cyano-, Difluormethoxy-, Trifluormethoxy-, Amino-C₁₋₃-alkyl-, C₁₋₃-Alkylamino-C₁₋₃-alkyl- oder Di-(C₁₋₃-Alkyl)-amino-C₁₋₃-alkylgruppen mono-, di- oder trisubstituiert und die Substituenten gleich oder verschieden sein können,
R³ das Wasserstoffatom oder eine C₁₋₃-Alkylgruppe,
wobei die C₁₋₃-Alkylgruppe mit einer in R² vorhandenen Alkylgruppe oder einem in R² vorhandenen Phenyl- oder Pyridylring und dem Stickstoffatom, an dem sie gebunden sind, unter Ausbildung eines 5- bis 7-gliedrigen Ringes verbunden sein kann oder
R² und R³ zusammen mit dem eingeschlossenen Stickstoffatom einen Rest der allgemeinen Formel 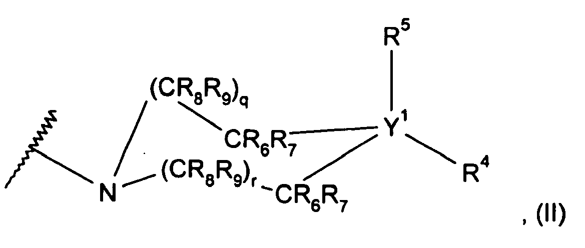 in der
Y¹ das Kohlenstoffatom oder, wenn R⁵ ein freies Elektronenpaar darstellt, auch das Stickstoffatom,
q und r, wenn Y¹ das Kohlenstoffatom darstellt, die Zahlen 0 oder 1 oder
q und r, wenn Y¹ das Stickstoffatom darstellt, die Zahlen 1 oder 2,
R⁴ das Wasserstoffatom, eine Hydroxy-, Amino-, Alkylamino-, C₃₋₆-Cycloalkylamino-, *N*-(C₃₋₆-Cycloalkyl)-alkylamino- oder Dialkylamino-, eine Alkyl-, Trifluormethyl-, C₃₋₆-Cycloalkyl-, Dialkylamino-C₂₋₇-alkyl-, Carboxyalkyl-, Alkoxycarbonylalkyl-, Alkylsulfonyl-, Alkylsulfonylamino- oder *N*-(Alkylsulfonyl)-alkylaminogruppe,
oder auch, wenn Y¹ nicht das Stickstoffatom darstellt, die Carboxy- oder Dialkylaminomethylgruppe,
eine Phenyl-, Phenyl-C₁₋₃-alkyl-, Pyridinyl- oder Diazinylgruppe, die jeweils durch ein Fluor-, Chlor- oder Bromatom, durch eine Trifluormethylcarbonyl-, Methyl- oder Methoxygruppe substituiert sein kann,
eine gesättigte oder ein- oder mehrfach ungesättigte 4- bis 7-gliedrige Azacycloalkylgruppe, eine 5- bis 7-gliedrige Oxaza-, Diaza- oder Triazacycloalkylgruppe, eine 7- bis 9-gliedrige Azabicyclo- oder Diazabicycloalkylgruppe, eine 1-Alkyl-4-piperidinylamino- oder 1-Alkyl-4-piperidinylaminosulfonylgruppe,
wobei die vorstehend genannten mono- und bicyclischen Heterocyclen über ein Stickstoff- oder ein Kohlenstoffatom gebunden sind,
eine Methylengruppe der vorstehend genannten mono- und bicyclischen Heterocyclen durch eine Carbonyl- oder Sulfonylgruppe ersetzt sein kann,
in den vorstehend genannten mono- und bicyclischen Heterocyclen eine nicht direkt an ein Stickstoff-, Sauerstoff- oder Schwefelatom gebundene Methylengruppe durch ein oder zwei Fluoratome substituiert sein kann,
die vorstehend genannten mono- und bicyclischen Heterocyclen durch eine oder zwei C₁₋₃-Alkylgruppen, in der jede Methylengruppe mit bis zu 2 und jede Methylgruppe mit bis zu 3 Fluoratomen substituiert sein kann, oder/und
durch eine C₃₋₆-Cycloalkyl-C₁₋₃-alkyl-, Benzyl-, C₁₋₄-Alkanoyl-, Di-(C₁₋₃-alkyl)-amino- oder C₁₋₃-Alkylsulfonyl-, durch eine Alkoxycarbonyl-, Benzyloxycarbonyl-, Alkoxycarbonylalkyl-, Carboxy- oder Carboxyalkylgruppe substituiert sein können,
R⁵ ein Wasserstoffatom, eine C₁₋₃-Alkyl- oder Alkoxycarbonylgruppe oder,
wenn Y¹ ein Stickstoffatom darstellt, auch ein freies Elektronenpaar, oder
R⁴ und R⁵ zusammen, wenn Y¹ das Kohlenstoffatom darstellt, einen 5- bis 6-gliedrigen cycloaliphatischen Ring, in dem eine oder zwei Methylengruppen durch eine -NH- oder -N(Methyl)-Gruppe und eine oder zwei weitere Methylengruppen durch Carbonylgruppen ersetzt sein können,
R⁶ und R⁷, die gleich oder verschieden sein können, jeweils ein Wasserstoffatom oder eine C₁₋₃-Alkyl- oder Di-(C₁₋₃-alkyl)-aminogruppe und
R⁸ und R⁹, die gleich oder verschieden sein können, jeweils ein Wasserstoffatom oder eine C₁₋₃-Alkyl-, Carboxy- oder C₁₋₃-Alkoxycarbonylgruppe darstellen, bedeuten,
wobei die vorstehend genannten Alkylgruppen oder die in den vorstehend genannten Resten enthaltenen Alkylgruppen, sofern nichts anderes angegeben wurde, 1 bis 7 Kohlenstoffatome enthalten und verzweigt oder unverzweigt sein können und die vorstehend erwähnten aromatischen und heteroaromatischen Reste zusätzlich durch Fluor-, Chlor- oder Bromatome, durch Cyano- oder Hydroxygruppen mono-, di- oder trisubstituiert und die Substituenten gleich oder verschieden sein können,
deren Tautomere, deren Enantiomere, deren Diastereomere, deren Gemische und deren Salze.

8. Verbindungen der allgemeinen Formel (I) gemäß Anspruch 1, in der
A ein Sauerstoffatom,
X ein Sauerstoffatom, eine Imino- oder Methylengruppe,
U eine Methyl-, Ethyl-, C₂₋₄-Alkenyl- oder C₂₋₄-Alkinylgruppe, in der die Methylengruppe mit bis zu 2 Fluoratomen und die Methylgruppe mit bis zu 3 Fluoratomen substituiert sein können,
V eine Amino- oder Hydroxygruppe,
W ein Wasserstoff-, Chlor- oder Bromatom oder eine Trifluormethylgruppe,
R¹ eine 4-(2-Oxo-1,2,4,5-tetrahydro-1,3-benzdiazepin-3-yl)-, 4-(2-Oxo-1,4-dihydro-2H-chinazolin-3-yl)-, 4-(5-Oxo-3-phenyl-4,5-dihydro-1,2,4-triazol-1-yl)-, 4-(2-Oxo-1,2-dihydro-imidazo[4,5-c]chinolin-3-yl)-, 4-(2-Oxo-1,2-dihydro-4*H*-thieno[3,4-d]pyrimidin-3-yl)-, 4-(2-Oxo-1,4-dihydro-2*H*-thieno[3,2-d]pyrimidin-3-yl)-, 4-(5-Oxo-4,5,7,8-tetrahydro-2-thia-4,6-diaza-azulen-6-yl)-, 4-(2-Oxo-1,2,4,5-tetrahydro-thieno[3,2-d]-1,3-diazepin-3-yl)-, 4-(2-Oxo-1,2,4,5-tetrahydro-thieno[2,3-d]-1,3-diazepin-3-yl)- oder 4-(2-Oxo-1,4-dihydro-2*H*-thieno[2,3-d]pyrimidin-3-yl)-gruppe,
wobei die vorstehend erwähnten mono- und bicyclischen Heterocyclen im Kohlenstoffgerüst zusätzlich durch eine Methoxygruppe monosubstituiert sein können,
R² eine Phenylmethylgruppe oder eine C₂₋₇-Alkylgruppe, die in ω-Stellung durch eine Phenyl-, Amino-, Alkylamino- oder Dialkylaminogruppe substituiert sein kann,
wobei die vorstehend genannte Phenylgruppe durch eine Amino-C₁₋₃-alkyl-, C₁₋₃-Alkylamino-C₁₋₃-alkyl- oder Di-(C₁₋₃-alkyl)-amino-C₁₋₃-alkylgruppe substituiert sein kann,
R³ das Wasserstoffatom oder eine C₁₋₃-Alkylgruppe,
R² und R³ zusammen mit dem Stickstoffatom, an dem sie gebunden sind, eine 7-Dimethylaminomethyl-1,2,4,5-tetrahydro-3-benzazepin-3-yl- oder 2-Amino-4,5,7,8-tetrahydro-thiazolo[4,5-d]azepin-6-yl-gruppe oder
R² und R³ zusammen mit dem eingeschlossenen Stickstoffatom einen Rest der allgemeinen Formel 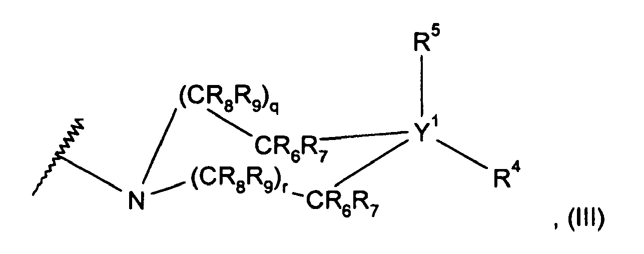 in der
Y¹ das Kohlenstoffatom oder, wenn R⁵ ein freies Elektronenpaar darstellt, auch das Stickstoffatom,
q und r, wenn Y¹ das Kohlenstoffatom darstellt, die Zahlen 0 oder 1 oder
q und r, wenn Y¹ das Stickstoffatom darstellt, die Zahlen 1 oder 2,
R⁴ das Wasserstoffatom,
eine Phenyl-, Benzyl- oder Pyridinylgruppe, die jeweils durch ein Fluor-, Chlor- oder Bromatom, durch eine Trifluormethylcarbonyl-, Methyl- oder Methoxygruppe substituiert sein können,
eine Hydroxy-, Carboxy-, Methyl-, Trifluormethyl-, *n*-Propyl-, Phenyl-, *p*-Tolyl-, *p*-Trifluormethylcarbonyl-phenyl-, *p*-(3-Dimethylaminopropyl)-phenyl-, Amino-, Benzyl-, *tert*-Butylamino-, Dimethylamino-, Diethylamino-, Diethylaminomethyl-, 2-Dimethylaminoethyl-, 2-Diethylaminoethyl-, 5-Aminopentyl-, Methoxycarbonyl-, Methoxycarbonylmethyl-, Perhydro-azepin-1-yl-, 4-Methyl-perhydro-1,4-diazepin-1-yl-, 1-Methyl-1-piperidinyl-4-yl-, 4-Piperazin-1-yl-, 4-Acetyl-piperazin-1-yl-, 4-Cyclopropylmethyl-piperazin-1-yl-, Pyrrolidin-1-yl-, 4-Ethyl-piperazin-1-yl-, 4-Isopropyl-piperazin-1-yl-, Piperidin-1-yl-, Piperidin-4-yl-, Morpholin-4-yl-, 4,4-Difluor-1-piperidin-1-yl-, 1-Methyl-1-aza-bicyclo[3.2.1]oct-4-yl-, 4-Methylpiperazin-1-yl-, 4-Ethylpiperazin-1-yl-, 1-Methyl-piperidin-1-yl-, 4-Carboxymethyl-piperazin-1-yl-, 1-Carboxymethyl-piperidin-4-yl-, 4-Benzyloxycarbonyl-piperazin-1-yl-, 1-Ethoxycarbonylmethyl-piperidin-4-yl-, Azetidin-1-yl-, 5-Methyl-2,5-diaza-bicyclo[2.2.1]hept-2-yl-, 1-Benzyl-piperidin-4-yl-, 4-Benzyl-piperazin-1-yl-, 4-Dimethylaminomethyl-1-phenyl-, 2,2,2-Trifluorethyl-piperazin-1-yl-, 1-Methylsulfonyl-piperidin-4-yl-, Piperidin-1-yl-methyl-, 1-Methyl-piperidin-4-yl-amino-, Methylsulfonylamino-, *N*-Methylsulfonyl-*N*-methylamino-, *N*-(Cyclopentyl)-methylamino-, 1,1-Dioxo-λ⁶-isothiazolidin-2-yl-, 2-Oxo-perhydro-1,3-oxazin-3-yl-, Cyclohexyl-, 2-Oxo-imidazolidin-1-yl-, 2-Methyl-imidazol-1-yl-, 4-Methyl-imidazol-1-yl-, 4-Thiazol-2-yl-, 2,4-Dimethyl-imidazol-1-yl-, 4-lmidazol-1-yl-, 1,2,4-Triazol-1-yl-, 1-Aza-bicyclo[2.2.2]oct-3-yl-, 1-Methyl-piperidin-4-yl-methylsulfonyl-, 1*H*-Imidazol-4-yl-, 4-Ethoxycarbonylmethyl-piperazin-1-yl-, 1-Ethoxycarbonyl-piperidin-4-yl-, 4-*tert*-Butoxycarbonylmethyl-piperazin-1-yl-, 1-(2,2,2-Trifluorethyl)-piperidin-4-yl-, 4-Methylsulfonyl-piperazin-1-yl-, 2-Carboxy-4-methyl-piperazin-1-yl-, 3-Carboxy-4-methyl-piperazin-1-yl-, 2-Ethoxycarbonyl-4-methyl-piperazin-1-yl-, 3-Ethoxycarbonyl-4-methyl-piperazin-1-yl- oder 4-(2,2,2-Trifluorethyl)-piperazin-1-yl-gruppe,
R⁵ ein Wasserstoffatom, eine Methylgruppe oder, wenn Y¹ ein Stickstoffatom bedeutet, auch ein freies Elektronenpaar, oder
R⁴ und R⁵ zusammen, wenn Y¹ das Kohlenstoffatom darstellt, eine 1-Methylpiperidin-4-yliden, Cyclohexyliden- oder Imidazolidin-2,4-dion-5-yliden-gruppe darstellen,
R⁶ und R⁷ jeweils ein Wasserstoffatom oder eine Dimethylaminogruppe und
R⁸ und R⁹ jeweils das Wasserstoffatom, eine Carboxy- oder Ethoxycarbonylgruppe darstellen, bedeuten
wobei alle vorstehend genannten Alkylgruppen sowie die innerhalb der anderen Reste vorhandenen Alkylgruppen, sofern nichts anderes angegeben ist, 1 bis 7 Kohlenstoffatome umfassen und geradkettig oder verzweigt sein können und die vorstehend erwähnten aromatischen und heteroaromatischen Reste zusätzlich durch Fluor-, Chlor- oder Bromatome, durch Cyano- oder Hydroxygruppen mono-, di- oder trisubstituiert und die Substituenten gleich oder verschieden sein können,
deren Enantiomere, deren Diastereomere und deren Salze.

9. Folgende Verbindungen der allgemeinen Formel (I) gemäß Anspruch 1:
(1) (*S*)-2-(4-Amino-3-chlor-5-trifluormethyl-benzyl)-1-[4-(4-methyl-piperazin-1-yl)-piperidin-1-yl]-4-[4-(2-oxo-1,2,4,5-tetrahydro-1,3-benzdiazepin-3-yl)-piperidin-1-yl]-butan-1,4-dion,
(2) 2-(4-Amino-3-chlor-5-trifluormethyl-benzyl)-1-(1'-methyl-[4.4']bipiperidinyl-1-yl)-4-[4-(2-oxo-1,4-dihydro-2*H*-quinazolin-3-yl)-piperidin-1-yl]-butan-1,4-dion,
(3) 2-(4-Amino-3-chlor-5-trifluormethyl-benzyl)-1-[4-(4-methyl-piperazin-1-yl)-piperidin-1-yl]-4-[4-(2-oxo-1,4-dihydro-2*H*-quinazolin-3-yl)-piperidin-1-yl]-butan-1,4-dion,
(4) 2-(4-Amino-3-chlor-5-trifluormethyl-benzyl)-1-[1,4']bipiperidinyl-1'-yl-4-[4-(2-oxo-1,4-dihydro-2*H*-quinazolin-3-yl)-piperidin-1-yl]-butan-1,4-dion,
(5) 2-(4-Amino-3-chlor-5-trifluormethyl-benzyl)-4-[4-(2-oxo-1,4-dihydro-2*H-*quinazolin-3-yl)-piperidin-1-yl]-1-(4-pyridin-4-yl-piperazin-1-yl)-butan-1,4-dion,
(6) 2-(4-Amino-3-chlor-5-trifluormethyl-benzyl)-1-[4-(1-methyl-piperidin-4-ylamino)-piperidin-1-yl]-4-[4-(2-oxo-1,4-dihydro-2*H*-quinazolin-3-yl)-piperidin-1-yl]-butan-1,4-dion,
(7) [4-(1-{2-(4-Amino-3-chlor-5-trifluormethyl-benzyl)-4-oxo-4-[4-(2-oxo-1,4-dihydro-2*H*-chinazolin-3-yl)-piperidin-1-yl]-butyryl}-piperidin-4-yl)-piperazin-1-yl]-essigsäure,
(8) (1'-{2-(4-Amino-3-chlor-5-trifluormethyl-benzyl)-4-oxo-4-[4-(2-oxo-1,4-dihydro-2*H*-chinazolin-3-yl)-piperidin-1-yl]-butyryl}-[4,4']bipiperidinyl-1-yl)-essigsäuremethylester,
(9) (1'-{2-(4-Amino-3-chlor-5-trifluormethyl-benzyl)-4-oxo-4-[4-(2-oxo-1,4-dihydro-2*H*-chinazolin-3-yl)-piperidin-1-yl]-butyryl}-[4,4']bipiperidinyl-1-yl)-essigsäure,
(10) 4-(2-Oxo-1,2,4,5-tetrahydro-1,3-benzdiazepin-3-yl)-piperidin-1-carbonsaure-[(*R*)-1-(4-amino-3-chlor-5-trifluormethyl-benzyl)-2-1,4'-bipiperidinyl-1'-yl-2-oxo-ethyl]-amid,
(11) 4-(2-Oxo-1,2,4,5-tetrahydro-1,3-benzdiazepin-3-yl)-piperidin-1-carbonsäure-[(*R*)-1-(4-amino-3-chlor-5-trifluormethyl-benzyl)-2-(4-dimethylamino-piperidin-1-yl)-2-oxo-ethyl]-amid,
(12) 4-(2-Oxo-1,2,4,5-tetrahydro-1,3-benzdiazepin-3-yl)-piperidin-1-carbonsäure-[(*R*)-1-(4-amino-3-chlor-5-trifluormethyl-benzyl)-2-oxo-2-(4-pyridin-4-yl-piperazin-1-yl)-ethyl]-amid,
(13) 4-(2-Oxo-1,2,4,5-tetrahydro-1,3-benzdiazepin-3-yl)-piperidin-1-carbonsäure-[(*R*)-1-(4-amino-3-chlor-5-trifluormethyl-benzyl)-2-(1'-methyl-4,4'-bipiperidinyl-1-yl)-2-oxo-ethyl]-amid,
(14) 4-[1-((*R*)-3-(4-Amino-3-chlor-5-trifluormethyl-phenyl)-2-{[4-(2-oxo-1,2,4,5-tetrahydro-1,3-benzdiazepin-3-yl)-piperidin-1-carbonyl]-amino}-propionyl)-piperidin-4-yl]-piperazin-1-carbonsäurebenzylester,
(15) [1'-((*R*)-3-(4-Amino-3-chlor-5-trifluormethyl-phenyl)-2-{[4-(2-oxo-1,2,4,5-tetrahydro-1,3-benzdiazepin-3-yl)-piperidin-1-carbonyl]-amino}-propionyl)-4,4'-bipiperidinyl-1-yl]-essigsäureethylester,
(16) 4-(2-Oxo-1,2,4.5-tetrahydro-1,3-benzdiazepin-3-yl)-piperidin-1-carbonsäure-{(*R*)-1-(4-amino-3-chlor-5-trifluormethyl-benzyl)-2-[4-(4-methyl-piperazin-1-yl)-piperidin-1-yl]-2-oxo-ethyl}-amid,
(17) 4-(2-Oxo-1,2,4.5-tetrahydro-1,3-benzdiazepin-3-yl)-piperidin-1-carbonsäure-{(*R*)-1-(4-amino-3-chlor-5-trifluormethyl-benzyl)-2-[4-(1-methyl-piperidin-4-yl)-piperazin-1-yl]-2-oxo-ethyl}-amid,
(18) 4-(2-Oxo-1,2,4,5-tetrahydro-1,3-benzdiazepin-3-yl)-piperidin-1-carbonsäure-[(*R*)-1-(4-amino-3-chlor-5-trifluormethyl-benzyl)-2-(4-azetidin-1-yl-piperidin-1-yl)-2-oxo-ethyl]-amid,
(19) 4-(2-Oxo-1,2,4,5-tetrahydro-1,3-benzdiazepin-3-yl)-piperidin-1-carbonsäure-{(*R*)-1-(4-amino-3-chlor-5-trifluormethyl-benzyl)-2-[4-(5-methyl-2,5-diaza-bicyclo[2.2.1]hept-2-yl)-piperidin-1-yl]-2-oxo-ethyl}-amid,
(20) 4-(2-Oxo-1,2,4,5-tetrahydro-1,3-benzdiazepin-3-yl)-piperidin-1-carbonsäure-[(*R*)-1-(4-amino-3-chlor-5-trifluormethyl-benzyl)-2-oxo-2-(4-piperazin-1-yl-piperidin-1-yl)-ethyl]-amid,
(21) [1'-((*R*)-3-(4-Amino-3-chlor-5-trifluormethyl-phenyl)-2-{[4-(2-oxo-1,2,4,5-tetrahydro-1,3-benzdiazepin-3-yl)-piperidin-1-carbonyl]-amino}-propionyl)-4,4'-bipiperidinyl-1-yl]-essigsäure,
(22) 4-(2-Oxo-1,2,4,5-tetrahydro-1,3-benzdiazepin-3-yl)-piperidin-1-carbonsäure-[1-(4-amino-3-chlor-5-trifluormethyl-benzyl)-2-1,4'-bipiperidinyl-1'-yl-2-oxo-ethyl]-amid,
(23) (*S*)-2-(4-Amino-3-brom-5-trifluormethyl-benzyl)-1-[4-(4-methyl-piperazin-1-yl)-piperidin-1-yl]-4-[4-(2-oxo-1,2,4,5-tetrahydro-1,3-benzdiazepin-3-yl)-piperidin-1-yl]-butan-1,4-dion,
(24) (*S*)-2-(4-Amino-3-brom-5-trifluormethyl-benzyl)-4-[4-(2-oxo-1,2,4,5-tetrahydro-1,3-benzdiazepin-3-yl)-piperidin-1-yl]-1-(4-pyridin-4-yl-piperazin-1-yl)-butan-1,4-dion,
(25) (*S*)-2-(4-Amino-3-brom-5-trifluormethyl-benzyl)-1-1,4'-bipiperidinyl-1'-yl-4-[4-(2-oxo-1,2,4,5-tetrahydro-1,3-benzdiazepin-3-yl)-piperidin-1-yl]-butan-1,4-dion,
(26) (*S*)-2-(4-Amino-3-brom-5-trifluormethyl-benzyl)-1-[4-(1-methyl-piperidin-4-yl)-piperazin-1-yl]-4-[4-(2-oxo-1,2,4,5-tetrahydro-1,3-benzdiazepin-3-yl)-piperidin-1-yl]-butan-1,4-dion,
(27) (*S*)-2-(4-Amino-3-brom-5-trifluormethyl-benzyl)-1-(1'-methyl-4,4'-bipiperidinyl-1-yl)-4-[4-(2-oxo-1,2,4,5-tetrahydro-1,3-benzdiazepin-3-yl)-piperidin-1-yl]-butan-1,4-dion,
(28) (*S*)-2-(4-Amino-3-trifluormethyl-benzyl)-1-[4-(4-methy)-piperazin-1-yl)-piperidin-1-yl]-4-[4-(2-oxo-1,2,4,5-tetrahydro-1,3-benzdiazepin-3-yl)-piperidin-1-yl]-butan-1,4-dion,
(29) (*S*)-2-(4-Amino-3-chlor-5-trifluormethyl-benzyl)-1-(4-dimethylamino-piperidin-1-yl)-4-[4-(2-oxo-1,2,4,5-tetrahydro-1,3-benzdiazepin-3-yl)-piperidin-1-yl]-butan-1,4-dion,
(30) (*S*)-2-(4-Amino-3-chlor-5-trifluormethyl-benzyl)-1-(3-dimethylamino-piperidin-1-yl)-4-[4-(2-oxo-1,2,4,5-tetrahydro-1,3-benzdiazepin-3-yl)-piperidin-1-yl]-butan-1,4-dion,
(31) (*S*)-2-(4-Amino-3-chlor-5-trifluormethyl-benzyl)-4-[4-(2-oxo-1,2,4,5-tetrahydro-1,3-benzdiazepin-3-yl)-piperidin-1-yl]-1-(4-pyrrolidin-1-yl-piperidin-1-yl)-butan-1,4-dion,
(32) (*S*)-2-(4-Amino-3-chlor-5-trifluormethyl-benzyl)-1-(1'-methyl-4,4'-bipiperidinyl-1-yl)-4-[4-(2-oxo-1,2,4,5-tetrahydro-1,3-benzdiazepin-3-yl)-piperidin-1-yl]-butan-1,4-dion,
(33) (*S*)-2-(4-Amino-3-chlor-5-trifluormethyl-benzyl)-1-[4-(4-cyclopropylmethyl-piperazin-1-yl)-piperidin-1-yl]-4-[4-(2-oxo-1,2,4,5-tetrahydro-1,3-benzdiazepin-3-yl)-piperidin-1-yl]-butan-1,4-dion,
(34) (*S*)-2-(4-Amino-3-chlor-5-trifluormethyl-benzyl)-1-(4-morpholin-4-yl-piperidin-1-yl)-4-[4-(2-oxo-1,2,4,5-tetrahydro-1,3-benzdiazepin-3-yl)-piperidin-1-yl]-butan-1,4-dion,
(35) (*S*)-2-(4-Amino-3-chlor-5-trifluormethyl-benzyl)-4-[4-(2-oxo-1,2,4,5-tetrahydro-1,3-benzdiazepin-3-yl)-piperidin-1-yl]-1-(4-perhydro-azepin-1-yl-piperidin-1-yl)-butan-1,4-dion,
(36) (*S*)-2-(4-Amino-3-chlor-5-trifluormethyl-benzyl)-1-[4-(4-methyl-perhydro-1,4-diazepin-1-yl)-piperidin-1-yl]-4-[4-(2-oxo-1,2,4,5-tetrahydro-1,3-benzdiazepin-3-yl)-piperidin-1-yl]-butan-1,4-dion,
(37) (*S*)-2-(4-Amino-3-chlor-5-trifluormethyl-benzyl)-1-[4-(4-isopropyl-piperazin-1-yl)-piperidin-1-yl]-4-[4-(2-oxo-1,2,4,5-tetrahydro-1,3-benzdiazepin-3-yl)-piperidin-1-yl]-butan-1,4-dion,
(38) (*S*)-2-(4-Amino-3-chlor-5-trifluormethyl-benzyl)-1-1,4'-bipiperidinyl-1'-yl-4-[4-(2-oxo-1,2,4,5-tetrahydro-1,3-benzdiazepin-3-yl)-piperidin-1-yl]-butan-1,4-dion,
(39) (*S*)-2-(4-Amino-3-chlor-5-trifluormethyl-benzyl)-1-[4-(1-methyl-piperidin-4-yl)-perhydro-1,4-diazepin-1-yl]-4-[4-(2-oxo-1,2,4,5-tetrahydro-1,3-benzdiazepin-3-yl)-piperidin-1-yl]-butan-1,4-dion,
(40) (*S*)-2-(4-Amino-3-chlor-5-trifluormethyl-benzyl)-1-[3-(4-methyl-piperazin-1-yl)-azetidin-1-yl]-4-[4-(2-oxo-1,2,4,5-tetrahydro-1,3-benzdiazepin-3-yl)-piperidin-1-yl]-butan-1,4-dion,
(41) (*S*)-2-(4-Amino-3-chlor-5-trifluormethyl-benzyl)-4-[4-(2-oxo-1,2,4,5-tetrahydro-1,3-benzdiazepin-3-yl)-piperidin-1-yl]-1-(3-pyrrolidin-1-yl-azetidin-1-yl)-butan-1,4-dion,
(42) (*S*)-2-(4-Amino-3-chlor-5-trifluormethyl-benzyl)-4-[4-(2-oxo-1,2,4,5-tetrahydro-1,3-benzdiazepin-3-yl)-piperidin-1-yl]-1-(3-piperidin-1-yl-azetidin-1-yl)-butan-1,4-dion,
(43) (*S*)-2-(4-Amino-3-chlor-5-trifluormethyl-benzyl)-1-(3-diethylamino-azetidin-1-yl)-4-[4-(2-oxo-1,2,4,5-tetrahydro-1,3-benzdiazepin-3-yl)-piperidin-1-yl]-butan-1,4-dion,
(44) (*S*)-2-(4-Amino-3-chlor-5-trifluormethyl-benzyl)-1-(4-azetidin-1-yl-piperidin-1-yl)-4-[4-(2-oxo-1,2,4,5-tetrahydro-1,3-benzdiazepin-3-yl)-piperidin-1-yl]-butan-1,4-dion,
(45) (*S*)-1-[4-(4-Acetyl-piperazin-1-yl)-piperidin-1-yl]-2-(4-amino-3-chlor-5-trifluormethyl-benzyl)-4-[4-(2-oxo-1,2,4,5-tetrahydro-1,3-benzdiazepin-3-yl)-piperidin-1-yl]-butan-1,4-dion,
(46) (*S*)-2-(4-Amino-3-chlor-5-trifluormethyl-benzyl)-1-(4-diethylaminomethyl-piperidin-1-yl)-4-[4-(2-oxo-1,2,4,5-tetrahydro-1,3-benzdiazepin-3-yl)-piperidin-1-yl]-butan-1,4-dion,
(47) (*S*)-2-(4-Amino-3-chlor-5-trifluormethyl-benzyl)-1-[4-(4-ethyl-piperazin-1-yl)-piperidin-1-yl]-4-[4-(2-oxo-1,2,4,5-tetrahydro-1,3-benzdiazepin-3-yl)-piperidin-1-yl]-butan-1,4-dion,
(48) (*S*)-2-(4-Amino-3-chlor-5-trifluormethyl-benzyl)-1-[4-(1-ethyl-piperidin-4-yl)-piperazin-1-yl]-4-[4-(2-oxo-1,2,4,5-tetrahydro-1,3-benzdiazepin-3-yl)-piperidin-1-yl]-butan-1,4-dion,
(49) (*S*)-2-(4-Amino-3-chlor-5-trifluormethyl-benzyl)-4-[4-(2-oxo-1,2,4,5-tetrahydro-1,3-benzdiazepin-3-yl)-piperidin-1-yl]-1-(3,4,5,6-tetrahydro-2*H*-4,4'-bipyridinyl-1-yl)-butan-1,4-dion,
(50) (*S*)-2-(4-Amino-3-chlor-5-trifluormethyl-benzyl)-4-[4-(2-oxo-1,2,4,5-tetrahydro-1,3-benzdiazepin-3-yl)-piperidin-1-yl]-1-(4-pyridin-4-yl-piperazin-1-yl)-butan-1,4-dion,
(51) (*S*)-2-(4-Amino-3-chlor-5-trifluormethyl-benzyl)-4-[4-(2-oxo-1,2,4,5-tetrahydro-1,3-benzdiazepin-3-yl)-piperidin-1-yl]-1-(3-perhydro-azepin-1-yl-azetidin-1-yl)-butan-1,4-dion,
(52) (*S*)-2-(4-Amino-3-chlor-5-trifluormethyl-benzyl)-1-[4-(1-benzyl-piperidin-4-yl)-piperazin-1-yl]-4-[4-(2-oxo-1,2,4,5-tetrahydro-1,3-benzdiazepin-3-yl)-piperidin-1-yl]-butan-1,4-dion,
(53) (*S*)-2-(4-Amino-3-chlor-5-trifluormethyl-benzyl)-1-[4-(4-benzyl-piperazin-1-yl)-piperidin-1-yl]-4-[4-(2-oxo-1,2,4,5-tetrahydro-1,3-benzdiazepin-3-yl)-piperidin-1-yl]-butan-1,4-dion,
(54) (*S*)-2-(4-Amino-3-chlor-5-trifluormethyl-benzyl)-1-(7-dimethylaminomethyl-1,2,4,5-tetrahydro-3-benzazepin-3-yl)-4-[4-(2-oxo-1,2,4,5-tetrahydro-1,3-benzdiazepin-3-yl)-piperidin-1-yl]-butan-1,4-dion,
(55) (*S*)-2-(4-Amino-3-chlor-5-trifluormethyl-benzyl)-1-[4-(4-dimethylaminomethyl-phenyl)-piperidin-1-yl]-4-[4-(2-oxo-1,2,4,5-tetrahydro-1,3-benzdiazepin-3-yl)-piperidin-1-yl]-butan-1,4-dion,
(56) (*S*)-2-(4-Amino-3-chlor-5-trifluormethyl-benzyl)-4-[4-(2-oxo-1,2,4,5-tetrahydro-1,3-benzdiazepin-3-yl)-piperidin-1-yl]-1-{4-[4-(2,2,2-trifluor-ethyl)-piperazin-1-yl]-piperidin-1-yl}-butan-1,4-dion,
(57) (*S*)-2-(4-Amino-3-chlor-5-trifluormethyl-benzyl)-1-(1'-methansulfonyl-4,4'-bipipendinyl-1-yl)-4-[4-(2-oxo-1,2,4,5-tetrahydro-1,3-benzdiazepin-3-yl)-piperidin-1-yl]-butan-1,4-dion,
(58) (*S*)-2-(4-Amino-3-chlor-5-trifluormethyl-benzyl)-1-(9-methyl-3,9-diaza-spiro[5.5]undec-3-yl)-4-[4-(2-oxo-1,2,4,5-tetrahydro-1,3-benzdiazepin-3-yl)-piperidin-1-yl]-butan-1,4-dion,
(59) (*S*)-2-(4-Amino-3-chlor-5-trifluormethyl-benzyl)-4-[4-(2-oxo-1,2,4,5-tetrahydro-1,3-benzdiazepin-3-yl)-piperidin-1-yl]-1-(4-piperidin-1-yl-methyl-piperidin-1-yl)-butan-1,4-dion,
(60) (*S*)-2-(4-Amino-3-chlor-5-trifluormethyl-benzyl)-1-[4-(2-dimethylamino-ethyl)-piperidin-1-yl]-4-[4-(2-oxo-1,2,4,5-tetrahydro-1,3-benzdiazepin-3-yl)-piperidin-1-yl]-butan-1,4-dion,
(61) (*S*)-2-(4-Amino-3-chlor-5-trifluormethyl-benzyl)-*N*-methyl-*N*-[2-(1-methylpiperidin-4-yl)-ethyl]-4-oxo-4-[4-(2-oxo-1,2,4,5-tetrahydro-1,3-benzdiazepin-3-yl)-piperidin-1-yl]-butyramid,
(62) (*S*)-2-(4-Amino-3-chlor-5-trifluormethyl-benzyl)-*N*-methyl-*N*-(1-methyl-piperidin-4-ylmethyl)-4-oxo-4-[4-(2-oxo-1,2,4,5-tetrahydro-1,3-benzdiazepin-3-yl)-piperidin-1-yl]-butyramid,
(63) (*S*)-2-(4-Amino-3-chlor-5-trifluormethyl-benzyl)-4-[4-(2-oxo-1,2,4,5-tetrahydro-1,3-benzdiazepin-3-yl)-piperidin-1-yl]-1-piperidin-1-yl-butan-1,4-dion,
(64) (*S*)-2-(4-Amino-3-chlor-5-trifluormethyl-benzyl)-4-[4-(2-oxo-1,2,4,5-tetrahydro-1,3-benzdiazepin-3-yl)-piperidin-1-yl]-1-(4-propyl-piperidin-1-yl)-butan-1,4-dion,
(65) (*S*)-2-(4-Amino-3-chlor-5-trifluormethyl-benzyl)-1-(4-benzyl-piperidin-1-yl)-4-[4-(2-oxo-1,2,4,5-tetrahydro-1,3-benzdiazepin-3-yl)-piperidin-1-yl]-butan-1,4-dion,
(66) (*S*)-2-(4-Amino-3-chlor-5-trifluormethyl-benzyl)-1-[4-(2-diethylamino-ethyl)-piperidin-1-yl]-4-[4-(2-oxo-1,2,4,5-tetrahydro-1,3-benzdiazepin-3-yl)-piperidin-1-yl]-butan-1,4-dion,
(67) (*S*)-2-(4-Amino-3-chlor-5-trifluormethyl-benzyl)-1-(3-aza-spiro[5.5]undec-3-yl)-4-[4-(2-oxo-1,2,4.5-tetrahydro-1,3-benzdiazepin-3-yl)-piperidin-1-yl]-butan-1,4-dion,
(68) *N*-(1-{(S)-2-(4-Amino-3-chlor-5-trifluormethyl-benzyl)-4-oxo-4-[4-(2-oxo-1,2,4,5-tetrahydro-1.3-benzdiazep)n-3-yl)-piperidin-1-yl]-butyryl}-piperidin-4-yl)-N-methyl-methansulfonamid,
(69) *N*-(1-{(*S*)-2-(4-Amino-3-chlor-5-trifluormethyt-benzyl)-4-oxo-4-[4-(2-oxo-1,2,4,5-tetrahydro-1,3-benzdiazepin-3-yl)-piperidin-1-yl]-butyryl}-piperidin-4-yl)-methansulfonamid,
(70) (*S*)-2-(4-Amino-3-chlor-5-trifluormethyl-benzyl)-1-[4-(cyclopentyl-methylamino)-piperidin-1-yl]-4-[4-(2-oxo-1,2,4,5-tetrahydro-1,3-benzdiazepin-3-yl)-piperidin-1-yl]-butan-1,4-dion,
(71) (*S*)-2-(4-Amino-3-chlor-5-trifluormethyl-benzyl)-1-(4-methyl-piperidin-1-yl)-4-[4-(2-oxo-1,2,4,5-tetrahydro-1,3-benzdiazepin-3-yl)-piperidin-1-yl]-butan-1,4-dion,
(72) (1-{(*S*)-2-(4-Amino-3-chlor-5-trifluormethyl-benzyl)-4-oxo-4-[4-(2-oxo-1,2,4,5-tetrahydro-1,3-benzdiazepin-3-yl)-piperidin-1-yl]-butyryl}-piperidin-4-yl)-essigsäuremethylester,
(73) (*S*)-2-(4-Amino-3-chlor-5-triftuormethyl-benzyl)-1-(4-hydroxy-piperidin-1-yl)-4-[4-(2-oxo-1,2,4,5-tetrahydro-1,3-benzdiazepin-3-yl)-piperidin-1-yl]-butan-1,4-dion,
(74) (*S*)-2-(4-Amino-3-chlor-5-trifluormethyl-benzyl)-4-[4-(2-oxo-1,2,4,5-tetrahydro-1,3-benzdiazepin-3-yl)-piperidin-1-yl]-1-(4-trifluormethyl-piperidin-1-yl)-butan-1,4-dion,
(75) (*S*)-2-(4-Amino-3-chlor-5-trifluormethyl-benzyl)-1-[4-(1,1-dioxo-1,6-isothiazolidin-2-yl)-piperidin-1-yl]-4-[4-(2-oxo-1,2,4,5-tetrahydro-1,3-benzdiazepin-3-yl)-piperidin-1-yl]-butan-1,4-dion,
(76) (*S*)-2-(4-Amino-3-chlor-5-trifluormethyl-benzyl)-1-[4-(2-oxo-perhydro-1,3-oxazin-3-yl)-piperidin-1-yl]-4-[4-(2-oxo-1.2,4.5-tetrahydro-1,3-benzdiazepin-3-yl)-piperidin-1-yl]-butan-1,4-dion,
(77) 1-{(*S*)-2-(4-Amino-3-chlor-5-trifluormethyl-benzyl)-4-oxo-4-[4-(2-oxo-1,2,4,5-tetrahydro-1,3-benzdiazepin-3-yl)-piperidin-1-yl]-butyryl}-piperidin-4-carbonsäuremethylester,
(78) (*S*)-2-(4-Amino-3-chlor-5-trifluormethyl-benzyl)-1-(4-cyclohexyl-piperidin-1-yl)-4-[4-(2-oxo-1,2,4,5-tetrahydro-1,3-benzdiazepin-3-yl)-piperidin-1-yl]-butan-1,4-dion,
(79) (*S*)-2-(4-Amino-3-chlor-5-trifluormethyl-benzyl)-1-(4-*tert*-butylamino-piperidin-1-yl)-4-{4-(2-oxo-1,2,4,5-tetrahydro-1,3-benzdiazepin-3-yl)-piperidin-1-yl]-butan-1,4-dion,
(80) (*S*)-2-(4-Amino-3-chlor-5-trifluormethyl-benzyl)-4-[4-(2-oxo-1,2,4,5-tetrahydro-1,3-benzdiazepin-3-yl)-piperidin-1-yl]-1-(4-phenyl-piperidin-1-yl)-butan-1,4-dion,
(81) (*S*)-2-(4-Amino-3-chlor-5-trifluormethyl-benzyl)-4-[4-(2-oxo-1,2,4,5-tetrahydro-1,3-benzdiazepin-3-yl)-piperidin-1-yl]-1-(4-p-tolyl-piperidin-1-yl)-butan-1,4-dion,
(82) 8-{(*S*)-2-(4-Amino-3-chlor-5-trifluormethyl-benzyl)-4-oxo-4-[4-(2-oxo-1,2,4,5-tetrahydro-1,3-benzdiazepin-3-yl)-piperidin-1-yl]-butyryl}-1,3,8-triaza-spiro[4.5]decan-2,4-dion,
(83) (*S*)-2-(4-Amino-3-chlor-5-trifluormethyl-benzyl)-1-[4-(2-oxo-imidazolidin-1-yl)-piperidin-1-yl]-4-[4-(2-oxo-1,2,4,5-tetrahydro-1,3-benzdiazepin-3-yl)-piperidin-1-yl]-butan-1,4-dion,
(84) (*S*)-2-(4-Amino-3-chlor-5-trifluormethyl-benzyl)-1-(4-amino-4-methyl-piperidin-1-yl)-4-[4-(2-oxo-1,2,4,5-tetrahydro-1,3-benzdiazepin-3-yl)-piperidin-1-yl]-butan-1,4-dion,
(85) (*S*)-2-(4-Amino-3-chlor-5-trifluormethyl-benzyl)-1-[4-(1-methyl-piperidin-4-yl)-piperazin-1-yl]-4-[4-(2-oxo-1,2,4,5-tetrahydro-1,3-benzdiazepin-3-yl)-piperidin-1-yl]-butan-1,4-dion,
(86) (*S*)-2-(4-Amino-3-chlor-5-trifluormethyl-benzyl)-1-(2-amino-4,5,7,8-tetrahydro-thiazolo[4,5-d]azepin-6-yl)-4-[4-(2-oxo-1,2,4,5-tetrahydro-1,3-benzdiazepin-3-yl)-piperidin-1 -y)]-butan-1,4-dion,
(87) (*S*)-2-(4-Amino-3-chlor-5-trifluormethyl-benzyl)-1-[4-(2-methyl-imidazol-1-yl)-piperidin-1-yl]-4-[4-(2-oxo-1,2,4,5-tetrahydro-1,3-benzdiazepin-3-yl)-piperidin-1-yl]-butan-1,4-dion,
(88) (*S*)-2-(4-Amino-3-chlor-5-trifluormethyl-benzyl)-1-[4-(4-methyl-imidazol-1-yl)-piperidin-1-yl]-4-[4-(2-oxo-1,2.4,5-tetrahydro-1,3-benzdiazepin-3-yl)-piperudin-1-yl]-butan-1,4-dion,
(89) (*S*)-2-(4-Amino-3-chlor-5-trifluormethyl-benzyl)-4-[4-(2-oxo-1,2,4,5-tetrahydro-1,3-benzdiazepin-3-yl)-piperidin-1-yl]-1-(4-thiazol-2-yl-piperazin-1-yl)-butan-1,4-dion,
(90) (*S*)-2-(4-Amino-3-chlor-5-trifluormethyl-benzyl)-1-[4-(2,4-dimethyl-imidazol-1-yl)-piperidin-1-yl]-4-[4-(2-oxo-1,2,4,5-tetrahydro-1,3-benzdiazepin-3-yl)-piperidin-1-yl]-butan-1,4-dion,
(91) (*S*)-2-(4-Amino-3-chlor-5-trifluormethyl-benzyl)-1-(4-imidazol-1-yl-piperidin-1-yl)-4-[4-(2-oxo-1,2,4,5-tetrahydro-1,3-benzdiazepin-3-yl)-piperidin-1-yl]-butan-1,4-dion,
(92) (*S*)-2-(4-Amino-3-chlor-5-trifluormethyl-benzyl)-4-[4-(2-oxo-1,2,4,5-tetrahydro-1,3-benzdiazepin-3-yl)-piperidin-1-yl]-1-(4-1,2,4-triazol-1-yl-piperidin-1-yl)-butan-1,4-dion,
(93) (*S*)-2-(4-Amino-3-chlor-5-trifluormethyl-benzyl)-1-[4-(1-aza-bicyclo[2.2.2]oct-3-yl)-piperazin-1-yl]-4-[4-(2-oxo-1,2,4,5-tetrahydro-1,3-benzdiazepin-3-yl)-piperidin-1-yl]-butan-1,4-dion,
(94) (*S*)-2-(4-Amino-3-chlor-5-trifluormethyl-benzyl)-4-[4-(2-oxo-1,2,4,5-tetrahydro-1,3-benzdiazepin-3-yl)-piperidin-1-yl]-1-piperazin-1-yl-butan-1,4-dion,
(95) 4-{(*S*)-2-(4-Amino-3-chlor-5-trifluormethyl-benzyl)-4-oxo-4-[4-(2-oxo-1,2,4,5-tetrahydro-1,3-benzdiazepin-3-yl)-piperidin-1-yl]-butyryl}-piperazin-1-sulfonsäure (1-methyl-piperidin-4-yl)-amid,
(96) (*S*)-2-(4-Amino-3-chlor-5-trifluormethyl-benzyl)-N-(5-amino-pentyl)-4-oxo-4-[4-(2-oxo-1,2,4,5-tetrahydro-1,3-benzdiazepin-3-yl)-piperidin-1-yl]-butyramid,
(97) (*S*)-2-(4-Amino-3-chlor-5-trifluormethyl-benzyl)-*N*-(3-aminomethyl-benzyl)-4-oxo-4-[4-(2-oxo-1,2,4,5-tetrahydro-1,3-benzdiazepin-3-yl)-piperidin-1-yl]-butyramid,
(98) 1-{(*S*)-2-(4-Amino-3-chlor-5-trifluormethyl-benzyl)-4-oxo-4-[4-(2-oxo-1,2,4,5-tetrahydro-1,3-benzdiazepin-3-yl)-piperidin-1-yl]-butyryl}-piperidin-4-carbonsäure,
(99) (1-{(*S*)-2-(4-Amino-3-chlor-5-trifluormethyl-benzyl)-4-oxo-4-[4-(2-oxo-1,2,4,5-tetrahydro-1,3-benzdiazepin-3-yl)-piperidin-1-yl]-butyryl}-piperidin-4-yl)-essigsäure,
(100) (*S*)-2-(4-Amino-3-chlor-5-trifluormethyl-benzyl)-1-[4-(8-methyl-8-aza-bicyclo[3.2.1]oct-3-yl)-piperazin-1-yl]-4-[4-(2-oxo-1,2,4,5-tetrahydro-1,3-benzdiazepin-3-yl)-piperidin-1-yl]-butan-1,4-dion,
(101) (*S*)-2-(4-Amino-3-chlor-5-trifluormethyl-benzyl)-4-[4-(2-oxo-1,2,4,5-tetrahydro-1,3-benzdiazepin-3-yl)-piperidin-1-yl]-1-(4-piperazin-1-yl-piperidin-1-yl)-butan-1,4-dion,
(102) (*S*)-2-(4-Amino-3-chlor-5-trifluormethyl-benzyl)-1-[4-(1H-imidazol-4-yl)-piperidin-1-yl]-4-[4-(2-oxo-1,2,4,5-tetrahydro-1,3-benzdiazepin-3-yl)-piperidin-1-yl]-butan-1,4-dion,
(103) (*S*)-2-(4-Amino-3-chlor-5-trifluormethyl-benzyl)-4-[4-(2-oxo-1,2,4,5-tetrahydro-1,3-benzdiazepin-3-yl)-piperidin-1-yl]-1-{4-[4-(2,2,2-trifluor-acetyl)-phenyl]-piperazin-1-yl}-butan-1,4-dion,
(104) (*S*)-2-(4-Amino-3-chlor-5-trifluormethyl-benzyl)-1-[4-(4-methyl-piperazin-1-yl)-piperidin-1-yl]-4-[4-(2-oxo-1,4-dihydro-2*H*-chinazolin-3-yl)-piperidin-1-yl]-butan-1,4-dion,
(105) (*S*)-2-(4-Amino-3-chlor-5-trifluormethyl-benzyl)-1-[4-(4-methyl-piperazin-1-yl)-piperidin-1-yl]-4-[4-(5-oxo-3-phenyl-4,5-dihydro-1,2,4-triazol-1-yl)-piperidin-1-yl]-butan-1,4-dion,
(106) (*S*)-2-(4-Amino-3-chlor-5-trifluormethyl-benzyl)-1-[4-(4-methyt-piperazin-1-yl)-piperidin-1-yl]-4-[4-(2-oxo-1,2-dihydro-imidazo[4,5-c]chinolin-3-yl)-piperidin-1-yl]-butan-1,4-dion,
(107) (*S*)-2-(4-Amino-3-chlor-5-trifluormethyl-benzyl)-1-[4-(4-methyl-piperazin-1-yl)-piperidin-1-yl]-4-[4-(2-oxo-1,2-dihydro-4*H*-thieno[3,4-d]pyrimidin-3-yl)-piperidin-1-yl]-butan-1,4-dion,
(108) (*S*)-2-(4-Amino-3-chlor-5-trifluormethyl-benzyl)-1-[4-(5-methyl-2,5-diaza-bicyclo[2.2.1]hept-2-yl)-piperidin-1-yl]-4-[4-(2-oxo-1,2,4,5-tetrahydro-1,3-benzdiazepin-3-yl)-piperidin-1-yl]-butan-1,4-dion,
(109) (*S*)-2-(4-Amino-3,5-bis-trifluormethyl-benzyl)-1-[4-(4-methyl-perhydro-1,4-diazepin-1-yl)-piperidin-1-yl]-4-[4-(2-oxo-1,2,4,5-tetrahydro-1,3-benzdiazepin-3-yl)-piperidin-1-yl]-butan-1,4-dion,
(110) (*S*)-2-(4-Amino-3,5-bis-trifluormethyl-benzyl)-1-[4-(1-methyl-piperidin-4-yl)-perhydro-1,4-diazepin-1-yl]-4-[4-(2-oxo-1,2,4,5-tetrahydro-1,3-benzdiazepin-3-yl)-piperidin-1-yl]-butan-1,4-dion,
(111) (*S*)-2-(4-Amino-3,5-bis-trifluormethyl-benzyl)-1-[4-(1-methyl-piperidin-4-yl)-piperazin-1-yl]-4-[4-(2-oxo-1,2,4,5-tetrahydro-1,3-benzdiazepin-3-yl)-piperidin-1-yl]-butan-1,4-dion,
(112) (*S*)-2-(4-Amino-3,5-bis-trifluormethyl-benzyl)-1-[4-(1-aza-bicyclo[2.2.2]oct-3-yl)-piperazin-1-yl]-4-[4-(2-oxo-1,2,4,5-tetrahydro-1,3-benzdiazepin-3-yl)-piperidin-1-yl]-butan-1,4-dion,
(113) (*S*)-2-(4-Amino-3,5-bis-trifluormethyl-benzyl)-1-[4-(4-methyl-piperazin-1-yl)-piperidin-1-yl]-4-[4-(2-oxo-1,2,4,5-tetrahydro-1,3-benzdiazepin-3-yl)-piperidin-1-yl]-butan-1,4-dion,
(114) (*S*)-2-(4-Amino-3,5-bis-trifluormethyl-benzyl)-4-[4-(2-oxo-1,2,4,5-tetrahydro-1,3-benzdiazepin-3-yl)-piperidin-1-yl]-1-(4-perhydro-azepin-1-yl-piperidin-1-yl)-butan-1,4-dion,
(115) (*S*)-2-(4-Amino-3,5-bis-trifluormethyl-benzyl)-1-1,4'-bipiperidinyl-1'-yl-4-[4-(2-oxo-1,2,4,5-tetrahydro-1,3-benzdiazepin-3-yl)-piperidin-1-yl]-butan-1,4-dion,
(116) (*S*)-2-(4-Amino-3,5-bis-trifluormethyl-benzyl)-4-[4-(2-oxo-1,2,4,5-tetrahydro-1,3-benzdiazepin-3-yl)-piperidin-1-yl]-1-(4-piperazin-1-yl-piperidin-1-yl)-butan-1,4-dion,
(117) 4-(2-Oxo-1,2,4,5-tetrahydro-1,3-benzdiazepin-3-yl)-piperidin-1-carbonsäure-{(*R*)-1-(4-amino-3,5-bis-trifluormethyl-benzyl)-2-[4-(4-methyl-piperazin-1-yl)-piperidin-1-yl]-2-oxo-ethyl}-amid,
(118) 4-(2-Oxo-1,2,4,5-tetrahydro-1,3-benzdiazepin-3-yl)-piperidin-1-carbonsäure-{(*R*)-1-(4-amino-3,5-bis-trifluormethyl-benzyl)-2-[4-(1-methyl-piperidin-4-yl)-piperazin-1-yl]-2-oxo-ethyl}-amid,
(119) 4-(2-Oxo-1,2,4,5-tetrahydro-1,3-benzdiazepin-3-yl)-piperidin-1-carbonsäure-[(*R*)-1-(4-amino-3,5-bis-trifluormethyl-benzyl)-2-1,4'-bipiperidinyl-1'-yl-2-oxo-ethyl]-amid,
(120) 4-(2-Oxo-1,2,4,5-tetrahydro-1,3-benzdiazepin-3-yl)-piperidin-1-carbonsäure-[(*R*)-1-(4-amino-3,5-bis-trifluormethyl-benzyl)-2-oxo-2-(4-piperazin-1-yl-piperidin-1-yl)-ethyl]-amid,
(121) 4-(2-Oxo-1,2,4,5-tetrahydro-1,3-benzdiazepin-3-yl)-piperidin-1-carbonsäure-(R)-1-(4-amino-3-chlor-5-trifluormethyl-benzyl)-2-[4-(4-methyl-piperazin-1-yl)-piperidin-1-yl]-2-oxo-ethylester,
(122) 4-(2-Oxo-1,4-dihydro-2*H*-chinazolin-3-yl)-piperidin-1-carbonsäure [2-[1,4']bipiperidinyl-1'-yl-1-(4-brom-3-methyl-benzyl)-2-oxo-ethyl]-amid,
(123) 4-(2-Oxo-1,4-dihydro-2*H*-chinazolin-3-yl)-piperidin-1-carbonsäure-[1-(4-brom-3-methyl-benzyl)-2-(1'-methyl-[4,4']bipiperidinyl-1-yl)-2-oxo-ethyl]-amid,
(124) 4-(2-Oxo-1,4-dihydro-2*H*-chinazolin-3-yl)-piperidin-1-carbonsäure-{1-(4-brom-3-methyl-benzyl)-2-[4-(4-methyl-piperazin-1-yl)-piperidin-1-yl]-2-oxo-ethyl}-amid,
(125) 4-(2-Oxo-1,4-dihydro-2*H*-chinazolin-3-yl)-piperidin-1-carbonsäure-{1-(4-brom-3-methyl-benzyl)-2-[4-(1-methyl-piperidin-4-yl)-piperazin-1-yl]-2-oxo-ethyl}-amid,
(126) {4-[1-(3-(4-Brom-3-methyl-phenyl)-2-{[4-(2-oxo-1,4-dihydro-2*H*-chinazolin-3-yl)-piperidin-1-carbonyl]-amino}-propionyl)-piperidin-4-yl]-piperazin-1-yl}-essigsäureethylester,
(127) [1'-(3-(4-Brom-3-methyl-phenyl)-2-{[4-(2-oxo-1,4-dihydro-2*H*-chinazolin-3-yl)-piperidin-1-carbonyl]-amino}-propionyl)-[4,4']bipiperidinyl-1-yl]-essigsäureethylester,
(128) {4-[4-(3-(4-Brom-3-methyl-phenyl)-2-{[4-(2-oxo-1,4-dihydro-2*H*-chinazolin-3-yl)-piperidin-1-carbonyl]-amino}-propionyl)-piperazin-1-yl]-piperidin-1-yl}-essigsäureethylester,
(129) {4-[1-(3-(4-Brom-3-methyl-phenyl)-2-{[4-(2-oxo-1,4-dihydro-2*H*-chinazolin-3-yl)-piperidin-1-carbonyl]-amino}-propionyl)-piperidin-4-yl]-piperazin-1-yl}-essigsäure,
(130) [1'-(3-(4-Brom-3-methyl-phenyl)-2-{[4-(2-oxo-1,4-dihydro-2*H*-chinazolin-3-yl)-piperidin-1-carbonyl]-amino}-propionyl)-[4,4']bipiperidinyl-1-yl]-essigsäure,
(131) {4-[4-(3-(4-Brom-3-methyl-phenyl)-2-{[4-(2-oxo-1,4-dihydro-2*H*-chinazolin-3-yl)-piperidin-1-carbonyl]-amino}-propionyl)-piperazin-1-yl]-piperidin-1-yl}-essigsäure,
(132) 2-(4-Brom-3-methyl-benzyl)-1-[4-(4-methyl-piperazin-1-yl)-piperidin-1-yl]-4-[4-(2-oxo-1,4-dihydro-2*H*-chinazolin-3-yl)-piperidin-1-yl]-butan-1,4-dion,
(133) 2-(4-Brom-3-methyl-benzyl)-1-(1'-methyl-[4,4']bipiperidinyl-1-yl)-4-[4-(2-oxo-1,4-dihydro-2*H*-chinazolin-3-yl)-piperidin-1-yl]-butan-1,4-dion,
(134) 1-[1,4']Bipiperidinyl-1'-yl-2-(4-brom-3-methyl-benzyl)-4-[4-(2-oxo-1,4-dihydro-2*H*-chinazolin-3-yl)-piperidin-1-yl]-butan-1,4-dion,
(135) 2-(4-Brom-3-methyl-benzyl)-1-{4-[4-(3-dimethylamino-propyl)-phenyl]-piperazin-1-yl}-4-[4-(2-oxo-1,4-dihydro-2*H*-chinazolin-3-yl)-piperidin-1-yl]-butan-1,4-dion,
(136) [4-(1-{2-(4-Brom-3-methyl-benzyl)-4-oxo-4-[4-(2-oxo-1,4-dihydro-2*H-*chinazolin-3-yl)-piperidin-1-yl]-butyryl}-piperidin-4-yl)-piperazin-1-yl]-essigsäure,
(137) (1'-{2-(4-Brom-3-methyl-benzyl)-4-oxo-4-[4-(2-oxo-1,4-dihydro-2*H*-chinazolin-3-yl)-piperidin-1-yl]-butyryl}-[4,4']bipiperidinyl-1-yl)-essigsäuremethylester,
(138) (1'-{2-(4-Brom-3-methyl-benzyl)-4-oxo-4-[4-(2-oxo-1,4-dihydro-2*H*-chinazolin-3-yl)-piperidin-1-yl]-butyryl}-[4,4']bipiperidinyl-1-yl)-essigsäure,
(139) 4-(2-Oxo-1,4-dihydro-2H-chinazolin-3-yl)-piperidin-1-carbonsäure{1-(4-chlor-3-methyl-benzyl)-2-[4-(4-methyl-piperazin-1-yl)-piperidin-1-yl]-2-oxo-ethyl}-amid,
(140) 4-(2-Oxo-1,4-dihydro-2*H*-chinazolin-3-yl)-piperidin-1-carbonsäure-[2-[1,4']bipiperidinyl-1'-yl-1-(4-chlor-3-methyl-benzyl)-2-oxo-ethyl]-amid,
(141) 4-(2-Oxo-1,4-dihydro-2*H*-chinazolin-3-yl)-piperidin-1-carbonsäure-{1-(4-chlor-3-methyl-benzyl)-2-[4-(1-methyl-piperidin-4-yl)-piperazin-1-yl]-2-oxo-ethyl}-amid,
(142) 4-(2-Oxo-1,4-dihydro-2*H*-chinazolin-3-yl)-piperidin-1-carbonsäure-[1-(4-chlor-3-methyl-benzyl)-2-(1'-methyl-[4,4']bipiperidinyl-1-yl)-2-oxo-ethyl]-amid,
(143) [1'-(3-(4-Chlor-3-methyl-phenyl)-2-{[4-(2-oxo-1,4-dihydro-2*H*-chinazolin-3-yl)-piperid in-1-carbonyl]-amino}-propionyl)-[4,4']bipiperidinyl-1-yl]-essigsäureethylester,
(144) {4-[1-(3-(4-Chlor-3-methyl-phenyl)-2-{[4-(2-oxo-1,4-dihydro-2H-chinazolin-3-yl)-piperidin-1-carbonyl]-amino}-propionyl)-piperidin-4-yl]-piperazin-1-yl}-essigsäure-*ter*t-butylester,
(145) 4-(2-Oxo-1,4-dihydro-2*H*-chinazolin-3-yl)-piperidin-1-carbonsäure-{1-(4-chlor-3-methyl-benzyl)-2-oxo-2-[1'-(2,2,2-trifluor-ethyl)-[4,4']bipiperidinyl-1-yl]-ethyl}-amid,
(146) 4-(2-Oxo-1,4-dihydro-2*H*-chinazolin-3-yl)-piperidin-1-carbonsäure-(1-(4-chlor-3-methyl-benzyl)-2-oxo-2-{4-[4-(2,2,2-trifluor-ethyl)-piperazin-1-yl]-piperidin-1-yl}-ethyl)-amid,
(147) [1'-(3-(4-Chlor-3-methyl-phenyl)-2-{[4-(2-oxo-1,4-dihydro-2*H*-chinazolin-3-yl)-piperidin-1-carbonyl]-amino}-propionyl)-[4,4']bipiperidinyl-1-yl]-essigsäure,
(148) 2-(4-Chlor-3-methyl-benzyl)-1-[4-(4-ethyl-piperazin-1-yl)-piperidin-1-yl]-4-[4-(2-oxo-1,4-dihydro-2*H*-chinazolin-3-yl)-piperidin-1-yl]-butan-1,4-dion,
(149) 2-(4-Chlor-3-methyl-benzyl)-1-(1'-methyl-[4,4']bipiperidinyl-1-yl)-4-[4-(2-oxo-1,4-dihydro-2*H*-chinazolin-3-yl)-piperidin-1-yl]-butan-1,4-dion,
(150) 1-[1,4']Bipiperidinyl-1'-yl-2-(4-chlor-3-methyl-benzyl)-4-[4-(2-oxo-1,4-dihydro-2*H*-chinazolin-3-yl)-piperidin-1-yl]-butan-1,4-dion,
(151) 2-(4-Chlor-3-methyl-benzyl)-1-[4-(4-methyl-piperazin-1-yl)-piperidin-1-yl]-4-[4-(2-oxo-1,4-dihydro-2*H*-chinazolin-3-yl)-piperidin-1-yl]-butan-1,4-dion,
(152) 2-(4-Chlor-3-methyl-benzyl)-1-[4-(1-methyl-piperidin-4-yl)-piperazin-1-yl]-4-[4-(2-oxo-1,4-dihydro-2*H*-chinazolin-3-yl)-piperidin-1-yl]-butan-1,4-dion,
(153) 2-(4-Chlor-3-methyl-benzyl)-1-[4-(4-methansulfonyl-piperazin-1-yl)-piperidin-1-yl]-4-[4-(2-oxo-1,4-dihydro-2*H*-chinazolin-3-yl)-piperidin-1-yl]-butan-1,4-dion,
(154) 2-(4-Chlor-3-methyl-benzyl)-1-[4-(4-isopropyl-piperazin-1-yl)-piperidin-1-yl]-4-[4-(2-oxo-1,4-dihydro-2*H*-chinazolin-3-yl)-piperidin-1-yl]-butan-1,4-dion,
(155) 1-{2-(4-Chlor-3-methyl-benzyl)-4-oxo-4-[4-(2-oxo-1,4-dihydro-2*H*-chinazolin-3-yl)-piperidin-1-yl]-butyryl}-4-(1-methyl-piperidin-4-yl)-piperazin-2-carbonsäureethylester,
(156) 1-(1-{2-(4-Chlor-3-methyl-benzyl)-4-oxo-4-[4-(2-oxo-1,4-dihydro-2*H*-chinazolin-3-yl)-piperidin-1-yl]-butyryl}-piperidin-4-yl)-4-methyl-piperazin-2-carbonsäureethylester,
(157) 4-(1-{2-(4-Chlor-3-methyl-benzyl)-4-oxo-4-[4-(2-oxo-1,4-dihydro-2*H*-chinazolin-3-yl)-piperidin-1-yl]-butyryl}-piperidin-4-yl)-1-methyl-piperazin-2-carbonsäureethylester,
(158) 4-{2-(4-Chlor-3-methyl-benzyl)-4-oxo-4-[4-(2-oxo-1,4-dihydro-2*H*-chinazolin-3-yl)-piperidin-1-yl]-butyryl}-1-(1-methyl-piperidin-4-yl)-piperazin-2-carbonsäureethylester,
(159) 2-(4-Chlor-3-methyl-benzyl)-4-[4-(2-oxo-1,4-dihydro-2*H*-chinazolin-3-yl)-piperidin-1-yl]-1-[1'-(2,2,2-trifluor-ethyl)-[4,4']bipiperidinyl-1-yl]-butan-1,4-dion,
(160) 2-(4-Chlor-3-methyl-benzyl)-4-[4-(2-oxo-1,4-dihydro-2*H*-chinazolin-3-yl)-piperidin-1-yl]-1-{4-[4-(2,2,2-trifluor-ethyl)-piperazin-1-yl]-piperidin-1-yl}-butan-1,4-dion,
(161) [4-(1-{2-(4-Chlor-3-methyl-benzyl)-4-oxo-4-[4-(2-oxo-1,4-dihydro-2*H-*chinazolin-3-yl)-piperidin-1-yl]-butyryl}-piperidin-4-yl)-piperazin-1-yl]-essigsäure,
(162) (1'-{2-(4-Chlor-3-methyl-benryl)-4-oxo-4-[4-(2-oxo-1,4-dihydro-2*H*-chinazolin-3-yl)-piperidin-1-yl]-butyryl}-[4,4']bipiperidinyl-1-yl)-essigsäuremethylester,
(163) (1'-{2-(4-Chlor-3-methyl-benzyl)-4-oxo-4-[4-(2-oxo-1,4-dihydro-2H chinazolin-3-yl)-piperidin-1-yl]-butyryl}-[4,4']bipiperidinyl-1-yl)-essigsäure,
(164) 1-{2-(4-Chlor-3-methyl-benzyl)-4-oxo-4-[4-(2-oxo-1,4-dihydro-2*H*-chinazolin-3-yl)-piperidin-1-yl]-butyryl}-4-(1-methyl-piperidin-4-yl)-piperazin-2-carbonsäure,
(165) 1-(1-{2-(4-Chlor-3-methyl-benzyl)-4-oxo-4-[4-(2-oxo-1,4-dihydro-2*H*-chinazolin-3-yl)-piperidin-1-yl]-butyryl}-piperidin-4-yl)-4-methyl-piperazin-2-carbonsäure,
(166) 4-(1-{2-(4-Chlor-3-methyl-benzyl)-4-oxo-4-[4-(2-oxo-1,4-dihydro-2*H*-chinazolin-3-yl)-piperidin-1-yl]-butyryl}-piperidin-4-yl)-1-methyl-piperazin-2-carbonsäure,
(167) 2-(4-Chlor-3-methyl-benzyl)-1-[4-(1-methyl-piperidin-4-yl)-piperazin-1-yl]-4-[4-(2-oxo-1,2,4,5-tetrahydro-1,3-benzdiazepin-3-yl)-piperidin-1-yl]-butan-1,4-dion,
(168) 2-(4-Chlor-3-methyl-benzyl)-1-[4-(4-methyl-piperazin-1-yl)-piperidin-1-yl]-4-[4-(2-oxo-1,2,4,5-tetrahydro-1,3-benzdiazepin-3-yl)-piperidin-1-yl]-butan-1,4-dion,
(169) [4-(1-{2-(4-Chlor-3-methyl-benzyl)-4-oxo-4-[4-(5-oxo-3-phenyl-4,5-dihydro-1,2,4-triazol-1-yl)-piperidin-1-yl]-butyryl}-piperidin-4-yl)-piperazin-1-yl]-essigsäure,
(170) (1 '-{2-(4-Chlor-3-methyl-benzyl)-4-oxo-4-[4-(5-oxo-3-phenyl-4,5-dihydro-1,2,4-triazol-1-yl)-piperidin-1-yl]-butyryl}-4,4'-bipiperidinyl-1-yl)-essigsäuremethylester,
(171) (1'-{2-(4-Chlor-3-methyl-benzyl)-4-oxo-4-[4-(5-oxo-3-phenyl-4,5-dihydro-1,2,4-triazol-1-yl)-piperidin-1-yl]-butyryl}-4,4'-bipiperidinyl-1-yl)-essigsäure,
(172) 2-(3-Brom-4-chlor-5-methyl-benzyl)-1-[4-(4-methyl-piperazin-1-yl)-piperidin-1-yl]-4-[4-(2-oxo-1,4-dihydro-2*H*-chinazolin-3-yl)-piperidin-1-yl]-butan-1,4-dion,
(173) 2-(3-Brom-4-chlor-5-methyl-benzyl)-1-(1'-methyl-[4,4']bipiperidinyl-1-yl)-4-[4-(2-oxo-1,4-dihydro-2*H*-chinazolin-3-yl)-piperidin-1-yl]-butan-1,4-dion,
(174) 2-(3-Brom-4-chlor-5-methyl-benzyl)-4-[4-(2-oxo-1,4-dihydro-2*H*-chinazolin-3-yl)-piperidin-1-yl]-1-(4-pyridin-4-yl-piperazin-1-yl)-butan-1,4-dion,
(175) 2-(3-Brom-4-chlor-5-methyl-benzyl)-1-[4-(1-methyl-piperidin-4-yl)-piperazin-1-y)]-4-[4-(2-oxo-1,4-dihydro-2*H*-chinazolin-3-yl)-piperidin-1-yl]-butan-1,4-dion,
(176) [4-(1-{2-(3-Brom-4-chlor-5-methyl-benzyl)-4-oxo-4-[4-(2-oxo-1,4-dihydro-2*H-*chinazolin-3-yl)-piperidin-1-yl]-butyryl}-piperidin-4-yl)-piperazin-1-yl]-essigsäure,
(177) (1'-{2-(3-Brom-4-Chlor-5-methyl-benzyl)-4-oxo-4-[4-(2-oxo-1,4-dihydro-2*H-*chinazolin-3-yl)-piperidin-1-yl]-butyryl}-[4,4']bipiperidinyl-1-yl)-essigsäuremethylester,
(178) (1'-{2-(3-Brom-4-Chlor-5-methyl-benzyl)-4-oxo-4-[4-(2-oxo-1,4-dihydro-2*H-*chinazolin-3-yl)-piperidin-1-yl]-butyryl}-[4,4']bipiperidinyl-1-yl)-essigsäure,
(179) 4-(2-Oxo-1,4-dihydro-2*H*-chinazolin-3-yl)-piperidin-1-carbonsäure{1-(3-brom-4-chlor-5-methyl-benzyl)-2-[4-(4-methyl-piperazin-1-yl)-piperid in-1-yl]-2-oxo-ethyl}-amid,
(180) 4-(2-Oxo-1,4-dihydro-2*H*-chinazolin-3-yl)-piperidin-1-carbonsäure-[2-[1,4']bipiperidinyl-1'-yl-1-(3-brom-4-chlor-5-methyl-benzyl)-2-oxo-ethyl]-amid,
(181) 4-(2-Oxo-1,4-dihydro-2*H*-chinazolin-3-yl)-piperidin-1-carbonsäure-{1-(3-brom-4-chlor-5-methyl-benzyl)-2-[4-(1-methyl-piperidin-4-yl)-piperazin-1-yl]-2-oxo-ethyl}-amid,
(182) 4-(2-Oxo-1,4-dihydro-2*H*-chinazolin-3-yl)-piperidin-1-carbonsäure-[1-(3-brom-4-chlor-5-methyl-benzyl)-2-(1'-methyl-[4,4']bipiperidinyl-1-yl)-2-oxo-ethyl]-amid,
(183) 4-(2-Oxo-1,4-dihydro-2*H*-chinazolin-3-yl)-piperidin-1-carbonsäure-[1-(3-brom-4-chlor-5-methyl-benzyl)-2-oxo-2-(4-pyridin-4-yl-piperazin-1-yl)-ethyl]-amid,
(184) 2-(4-Chlor-3-trifluormethyl-benzyl)-1-(1'-methyl-[4,4']bipiperidinyl-1-yl)-4-[4-(2-oxo-1,4-dihydro-2*H*-chinazofin-3-yl)-piperidin-1-yl]-butan-1,4-dion,
(185) 2-(4-Chlor-3-trifluormethyl-benzyl)-1-[4-(4-methyl-piperazin-1-yl)-piperidin-1-yl]-4-[4-(2-oxo-1,4-dihydro-2*H*-chinazolin-3-yl)-piperidin-1-yl]-butan-1,4-dion,
(186) 1-[1,4']Bipiperidinyl-1'-yl-2-(4-chlor-3-trifluormethyl-benzyl)-4-[4-(2-oxo-1,4-dihydro-2*H*-chinazolin-3-yl)-piperidin-1-yl]-butan-1,4-dion,
(187) [4-(1-{2-(4-Chlor-3-trifluormethyl-benzyl)-4-oxo-4-[4-(2-oxo-1,4-dihydro-2*H-*chinazolin-3-yl)-piperidin-1-yl]-butyryl}-piperidin-4-yl)-piperazin-1-yl]-essigsäure,
(188) (1'-{2-(4-Chlor-3-trifluormethyl-benzyl)-4-oxo-4-[4-(2-oxo-1,4-dihydro-2*H-*chinazolin-3-yl)-piperidin-1-yl]-butyryl}-[4,4']bipiperidinyl-1-yl)-essigsäuremethylester,
(189) (1'-{2-(4-Chlor-3-trifluormethyl-benzyl)-4-oxo-4-[4-(2-oxo-1,4-dihydro-2*H-*chinazolin-3-yl)-piperidin-1-yl]-butyryl}-[4,4']bipiperidinyl-1-yl)-essigsäure,
(190) 2-(4-Chlor-3-trifluormethyl-benzyl)-1-(1'-methyl-[4,4']bipiperidinyl-1-yl)-4-[4-(2-oxo-1,4-dihydro-2*H*-chinazolin-3-yl)-piperidin-1-yl]-butan-1,4-dion,
deren Enantiomere, deren Diastereomere und deren Salze.

10. Physiologisch verträgliche Salze der Verbindungen nach einem der Ansprüche 1 bis 9 mit anorganischen oder organischen Säuren oder Basen.

11. Arzneimittel, enthaltend eine Verbindung nach mindestens einem der Ansprüche 1 bis 9 oder ein physiologisch verträgliches Salz gemäß Anspruch 10 neben gegebenenfalls einem oder mehreren inerten Trägerstoffen und/oder Verdünnungsmitteln.

12. Verwendung einer Verbindung nach mindestens einem der Ansprüche 1 bis 10 zur Herstellung eines Arzneimittels zur akuten und prophylaktischen Behandlung von Kopfschmerzen, insbesondere Migräne- bzw. Cluster-Kopfschmerz, Spannungskopfschmerz und chronischem Kopfschmerz.

13. Verwendung einer Verbindung nach mindestens einem der Ansprüche 1 bis 10 zur Herstellung eines Arzneimittels zur prophylaktischen Behandlung von Migräne-Kopfschmerz während der Prodromerscheinung oder zur akuten und prophylaktischen Behandlung von Migräne-Kopfschmerz, der vor oder während der Menstruation auftritt.

14. Verwendung einer Verbindung nach mindestens einem der Ansprüche 1 bis 10 zur Herstellung eines Arzneimittels zur Bekämpfung von nicht-insulinabhängigem Diabetes-mellitus (NIDDM).

15. Verwendung einer Verbindung nach mindestens einem der Ansprüche 1 bis 10 zur Herstellung eines Arzneimittels zur Behandlung von cardiovaskulären Erkrankungen, von Morphintoleranz, von Chlostritiumtoxin-bedingten Durchfallerkrankungen, von Erkrankungen der Haut, insbesondere von thermischen und strahlungsbedingten Schäden inklusive Sonnenbrand, von entzündlichen Erkrankungen wie insbesondere entzündlicher Gelenkerkrankungen wie Arthritis, von neurogenen Entzündungen der oralen Mucosa, von entzündlichen Lungenerkrankungen, von allergischer Rhinitis, von Asthma, von Erkrankungen, die mit einer überschießenden Gefäßerweiterung und dadurch bedingter verringerter Gefäßdurchblutung, wie insbesondere Schock oder Sepsis oder Hautrötungen, einhergeht, zur Linderung von Schmerzzuständen im allgemeinen, insbesondere bei neuropathischen Schmerzen, bei neuropathischen Schmerzzuständen im Rahmen systemischer neurotoxischer Erkrankungen sowie bei Schmerzzuständen, die auf entzündliche Prozesse zurückzuführen sind, oder zur präventiven oder akut therapeutischen Beeinflussung der durch Gefäßerweiterung und erhöhten Blutfluss verursachten Symptomatik von Hitzewallungen menopausaler, östrogendefizienter Frauen sowie hormonbehandelter Prostatakarzinompatienten.

16. Verfahren zur Herstellung eines Arzneimittels gemäß Anspruch 11, **dadurch gekennzeichnet, dass** auf nichtchemischem Weg eine Verbindung nach mindestens einem der Ansprüche 1 bis 10 in einen oder mehrere inerte Trägerstoffe und/oder Verdünnungsmittel eingearbeitet wird.

17. Verfahren zur Herstellung der Verbindungen der allgemeinen Formel (1) nach mindestens einem der Ansprüche 1 bis 9, **dadurch gekennzeichnet, dass**
(a) zur Herstellung von Verbindungen der allgemeinen Formel (I), in der X das Sauerstoffatom oder die NH-Gruppe bedeutet und R¹ bis R³ wie in Anspruch 1 definiert sind, mit der Maßgabe, dass diese Gruppen keine freie Carbonsäurefunktion enthalten:
Umsetzung von Piperidinen der allgemeinen Formel 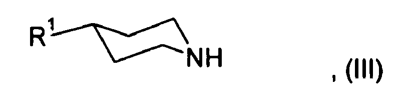 in der R¹ wie in Anspruch 1 definiert ist,
(i) mit Kohlensäurederivaten der allgemeinen Formel 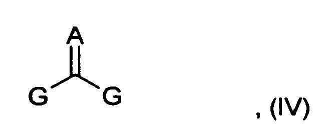 in der A wie in Anspruch 1 definiert ist und G eine nucleofuge Gruppe bedeutet, mit der Maßgabe, dass X die NH-Gruppe darstellt, oder
(ii) mit Kohlensäurederivaten der allgemeinen Formel 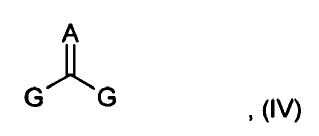 in der A das Sauerstoffatom darstellt und G eine nucleofuge Gruppe, die gleich oder verschieden sein kann, bedeutet mit der Maßgabe, dass X das Sauerstoffatom bedeutet,
und mit Verbindungen der allgemeinen Formel 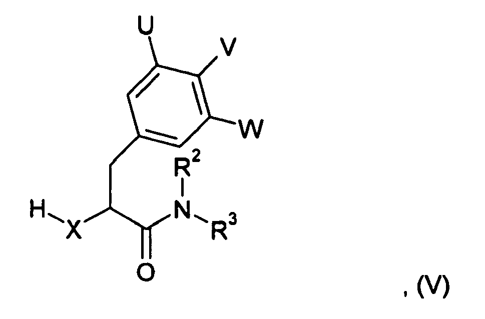 in der X ein Sauerstoffatom oder eine -NH-Gruppe bedeutet und U, V, W, R² und R³ wie in Anspruch 1 definiert sind, mit der Maßgabe, dass R² und R³ keine freie Carbonsäure- und/oder keine weitere freie primäre oder sekundäre aliphatische Aminofunktion oder weitere freie Hydroxyfunktion enthalten, oder
(b) zur Herstellung von Verbindungen der allgemeinen Formel (I), in der X die Methylengruppe bedeutet und R¹ bis R³ wie in Anspruch 1 definiert sind, mit der Maßgabe, dass diese Gruppen keine freie Carbonsäure- und/oder keine weitere freie primäre oder sekundäre aliphatische Aminofunktion enthalten:
Kupplung einer Carbonsäure der allgemeinen Formel 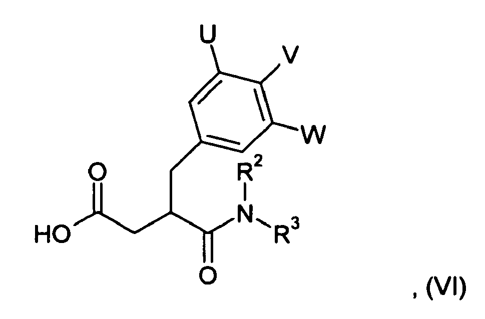 in der U, V, W, R² und R³ wie in Anspruch 1 definiert sind, mit einem Piperidin der allgemeinen Formel 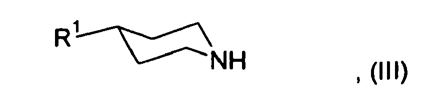 in der R¹ wie in Anspruch 1 definiert ist, oder
(c) zur Herstellung von Verbindungen der allgemeinen Formel (I), in der X die Methylengruppe bedeutet und R² und R³ wie in Anspruch 1 definiert sind, mit der Maßgabe, dass diese Gruppen kein freies primäres oder sekundäres Amin enthalten:
Kupplung einer Verbindung der allgemeinen Formel 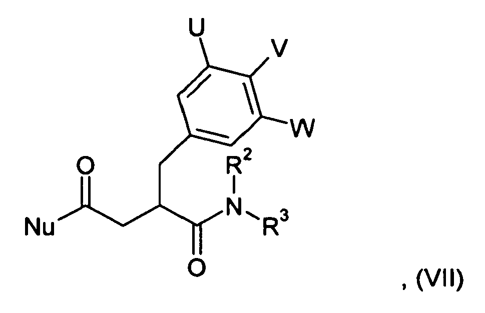 in der U, V, W, R² und R³ wie in Anspruch 1 definiert sind, mit der Maßgabe, dass R² und R³ kein freies primäres oder sekundäres Amin enthalten, und Nu eine Austrittsgruppe bedeutet,
mit einem Piperidin der allgemeinen Formel 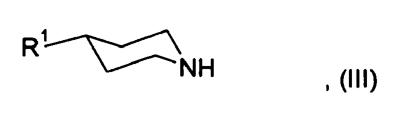 in der R¹ wie in Anspruch 1 definiert ist, oder
(d) zur Herstellung von Verbindungen der allgemeinen Formel (I), in der alle Reste wie in Anspruch 1 definiert sind:
Kupplung einer Carbonsäure der allgemeinen Formel 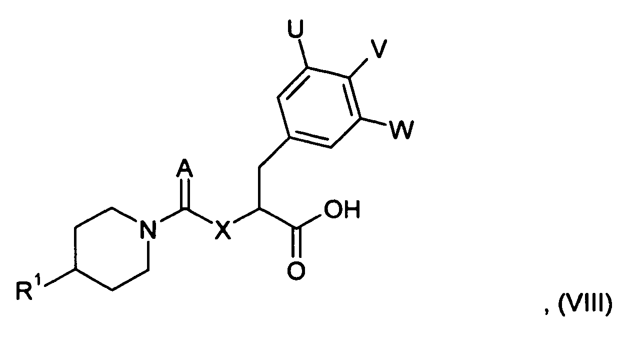 in der alle Reste wie in Anspruch 1 definiert sind, mit einem Amin der allgemeinen Formel HNR²R³, in der R² und R³ wie in Anspruch 1 definiert sind, mit der Maßgabe, dass diese Gruppen keine freie Carbonsäure- und/oder keine weitere freie primäre oder sekundäre aliphatische Aminofunktion enthalten, oder
(e) zur Herstellung von Verbindungen der allgemeinen Formel (1), in der R¹ wie in Anspruch 1 definiert ist, mit der Maßgabe, dass kein freies primäres oder sekundäres Amin enthalten ist:
Kupplung einer Verbindung der allgemeinen Formel 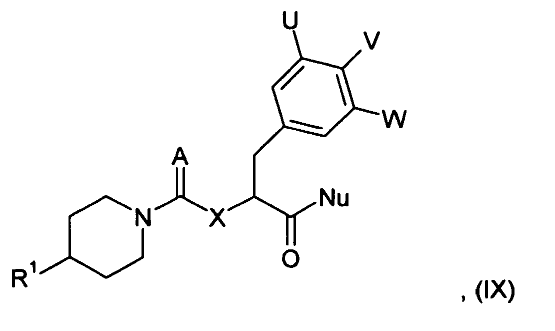 in der alle Reste wie in Anspruch 1 definiert sind und Nu eine Austrittsgruppe bedeutet,
mit einem Amin der allgemeinen Formel HNR²R³, in dem R² und R³ wie in Anspruch 1 sind, mit der Maßgabe, dass diese Gruppen keine freie Carbonsäure- und/oder keine weitere freie primäre oder sekundäre aliphatische Aminofunktion enthalten, und
erforderlichenfalls ein bei den vorstehend beschriebenen Umsetzungen verwendeter Schutzrest wieder abgespalten wird und/oder
gegebenenfalls verwendete Präcurserfunktionen in einer so erhaltenen Verbindung abgewandelt werden und/oder
gewünschtenfalls eine so erhaltene Verbindung der allgemeinen Formel (I) in ihre Stereoisomere aufgetrennt wird und/oder
eine so erhaltene Verbindung der allgemeinen Formel (I) in ihre Salze, insbesondere für die pharmazeutische Anwendung in ihre physiologisch veträglichen Salze übergeführt wird.

## Claims

1. CGRP antagonists of general formula 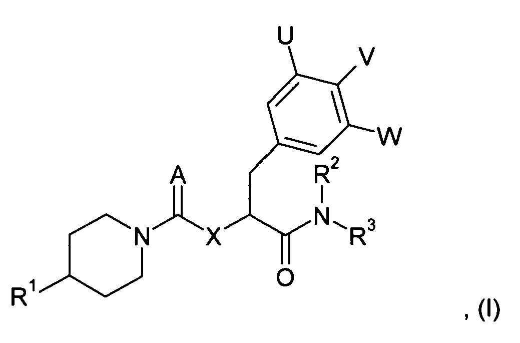 wherein
A denotes an oxygen or sulphur atom, a phenylsulphonylimino or cyanimino group,
X denotes an oxygen or sulphur atom, an imino group optionally substituted by a C₁₋₆-alkyl group or a methylene group optionally substituted by a C₁₋₆-alkyl group,
U denotes a C₁₋₆-alkyl, C₂₋₆-alkenyl or C₂₋₆-alkynyl group wherein each methylene group may be substituted by up to 2 fluorine atoms and each methyl group may be substituted by up to 3 fluorine atoms,
V denotes a chlorine or bromine atom, an amino, methylamino or hydroxy group,
W denotes a hydrogen, fluorine, chlorine, bromine or iodine atom, a difluoro- or trifluoromethyl group,
R¹ denotes a saturated, mono- or diunsaturated 5- to 7-membered aza, diaza, triaza, oxaza, thiaza, thiadiaza or *S,S*-dioxido-thiadiaza heterocyclic group,
in which the abovementioned heterocycles are linked via a carbon or nitrogen atom,
contain one or two carbonyl or thiocarbonyl groups adjacent to a nitrogen atom,
may be substituted at one of the nitrogen atoms by an alkyl group,
may be substituted at one or at two carbon atoms by an alkyl group, by a phenyl, phenylmethyl, naphthyl, biphenylyl, pyridinyl, diazinyl, furyl, thienyl, pyrrolyl, 1,3-oxazolyl, 1,3-thiazolyl, isoxazolyl, pyrazolyl, 1-methylpyrazolyl, imidazolyl or 1-methylimidazolyl group, while the substituents may be identical or different, and
wherein an olefinic double bond of one of the abovementioned unsaturated heterocycles may be fused to a phenyl, naphthyl, pyridine, diazine, 1,3-oxazole, thienyl, furan, thiazole, pyrrole, *N*-methylpyrrole or quinoline ring, to a 1*H*-quinolin-2-one ring optionally substituted at the nitrogen atom by an alkyl group or to an imidazole or *N*-methylimidazole ring or also two olefinic double bonds of one of the abovementioned unsaturated heterocycles may each be fused to a phenyl ring,
wherein the phenyl, pyridinyl, diazinyl, furyl, thienyl, pyrrolyl, 1,3-oxazolyl, 1,3-thiazolyl, isoxazolyl, pyrazolyl, 1-methylpyrazolyl, imidazolyl or 1-methylimidazolyl groups contained in R¹ as well as benzo-, thieno-, pyrido- and diazino-fused heterocycles in the carbon skeleton may additionally be mono-, di- or trisubstituted by fluorine, chlorine, bromine or iodine atoms, by alkyl, alkoxy, nitro, alkylthio, alkylsulphinyl, alkylsulphonyl, alkylsulphonylamino, phenyl, difluoromethyl, trifluoromethyl, alkoxycarbonyl, carboxy, hydroxy, amino, alkylamino, dialkylamino, acetyl, acetylamino, propionylamino, aminocarbonyl, alkylaminocarbonyl, dialkylaminocarbonyl, (4-morpholinyl)carbonyl, (1-pyrrolidinyl)carbonyl, (1-piperidinyl)carbonyl, (hexahydro-1-azepinyl)carbonyl, (4-methyl-1-piperazinyl)carbonyl, methylenedioxy, aminocarbonylamino, alkanoyl, cyano, difluoromethoxy, trifluoromethoxy, trifluoromethylthio, trifluoromethylsulphinyl or trifluoromethylsulphonyl groups, while the substituents may be identical or different,
R² denotes the hydrogen atom,
a phenylmethyl group or a C₂₋₇-alkyl group which may be substituted in the ω position by a cyclohexyl, phenyl, pyridinyl, diazinyl, hydroxy, amino, alkylamino, dialkylamino, carboxy, alkoxycarbonyl, aminocarbonyl, aminocarbonylamino, acetylamino, 1-pyrrolidinyl, 1-piperidinyl, 4-(1-piperidinyl)-1-piperidinyl, 4-morpholinyl, hexahydro-1*H*-1-azepinyl, [bis-(2-hydroxyethyl)]amino, 4-alkyl-1-piperazinyl or 4-(ω-hydroxy-C₂₋₇-alkyl)-1-piperazinyl group,
a phenyl or pyridinyl group,
while the abovementioned heterocyclic groups and phenyl groups may additionally be mono- di- or trisubstituted in the carbon skeleton by fluorine, chlorine, bromine or iodine atoms, by methyl, alkoxy, difluoromethyl, trifluoromethyl, hydroxy, amino, C₁₋₃-alkylamino, di-(C₁₋₃-alkyl)-amino, acetylamino, aminocarbonyl, cyano, methylsulphonyloxy, difluoromethoxy, trifluoromethoxy, trifluoromethylthio, trifluoromethylsulphinyl, trifluoromethylsulphonyl, amino-C₁₋₃-alkyl, C₁₋₃-alkylamino-C₁₋₃-alkyl or di-(C₁₋₃-alkyl)-amino-C₁₋₃-alkyl groups and the substituents may be identical or different,
R³ denotes the hydrogen atom or a C₁₋₃-alkyl group optionally substituted by a phenyl or pyridinyl group,
while the C₁₋₃-alkyl group may be linked to an alkyl group present in R² or a phenyl or pyridyl ring present in R² and the nitrogen atom to which they are bound, forming a ring, or
R² and R³ together with the enclosed nitrogen atom denote a group of general formula 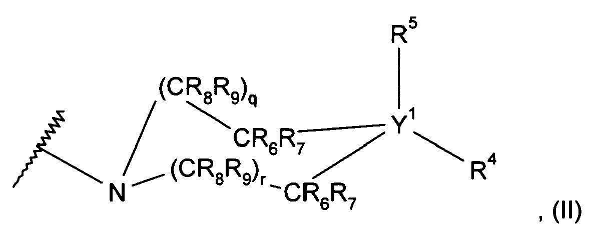 wherein
Y¹ denotes the carbon atom or, if R⁵ is a pair of free electrons, it may also denote the nitrogen atom,
q and r, if Y¹ denotes the carbon atom, represent the numbers 0, 1 or 2, or
q and r, if Y¹ denotes the nitrogen atom, represent the numbers 1 or 2,
R⁴ denotes the hydrogen atom, an amino, alkylamino, cycloalkylamino, dialkylamino, *N*-(cycloalkyl)-alkylamino, dicycloalkylamino, hydroxy, alkyl, cycloalkyl, amino-C₂₋₇-alkyl, alkylamino-C₂₋₇-alkyl, dialkylamino-C₂₋₇-alkyl, aminoiminomethyl, alkylcarbonyl, alkylsulphonyl, alkylcarbonylamino, alkylsulphonylamino, *N*-alkylcarbonyl-*N*-alkylamino, *N*-alkylsulphonyl-*N*-alkylamino, aminocarbonylamino, alkylaminocarbonylamino, dialkylaminocarbonylamino, cycloalkylaminocarbonylamino, dicycloalkylaminocarbonylamino, phenylaminocarbonylamino, aminocarbonylalkyl, alkylaminocarbonylalkyl, dialkylaminocarbonylalkyl, aminocarbonylaminoalkyl, alkoxycarbonyl, alkoxycarbonylalkyl or carboxyalkyl group,
or, if Y¹ does not denote the nitrogen atom, the carboxy, aminomethyl, alkylaminomethyl or dialkylaminomethyl group,
a phenyl, phenyl-C₁₋₃-alkyl, pyridinyl, diazinyl, 1-naphthyl, 2-naphthyl, pyridinylcarbonyl or phenylcarbonyl group which may each be mono-, di- or trisubstituted in the carbon skeleton by fluorine, chlorine, bromine or iodine atoms, by alkyl, alkoxy, methylsulphonyloxy, difluoromethyl, trifluoromethyl, hydroxy, amino, acetylamino, aminocarbonyl, aminocarbonylamino, aminocarbonylaminomethyl, cyano, carboxy, alkoxycarbonyl, carboxyalkyl, alkoxycarbonylalkyl, alkanoyl, ω-(dialkylamino)alkanoyl, ω-(dialkylamino)alkyl, ω-(dialkylamino)hydroxyalkyl, ω-(carboxy)alkanoyl, difluoromethoxy, trifluoromethoxy, trifluoromethylthio, trifluoromethylsulphinyl or trifluoromethylsulphonyl groups, while the substituents may be identical or different,
a saturated or mono- or polyunsaturated 4- to 10-membered azacycloalkyl group, a 5- to 10-membered oxaza-, thiaza, diaza- or triazacycloalkyl group, a 6- to 10-membered azabicyclo- or diazabicycloalkyl group, a 1-alkyl-4-piperidinylcarbonyl or 4-alkyl-1-piperazinylcarbonyl, a 1-alkyl-4-piperidinylamino, 1-alkyl-4-piperidinylaminocarbonyl or 1-alkyl-4-piperidinylaminosulphonyl group,
while the abovementioned mono- and bicyclic heterocycles are bound via a nitrogen or carbon atom,
a methylene group in the abovementioned mono- and bicyclic heterocycles may be replaced by a carbonyl or sulphonyl group,
in the abovementioned mono- and bicyclic heterocycles any methylene group not directly bound to a nitrogen, oxygen or sulphur atom may be substituted by one or two fluorine atoms,
the abovementioned mono- and bicyclic heterocycles as well as the 1-alkyl-4-piperidinylcarbonyl- and 4-alkyl-1-piperazinylcarbonyl group in the ring may be mono- or polysubstituted by a C₁₋₇-alkyl group and/or
monosubstituted by a benzyl, alkanoyl, dialkylamino, phenylcarbonyl, pyridinylcarbonyl, carboxy, carboxyalkanoyl, carboxyalkyl, alkoxycarbonylalkyl, alkoxycarbonyl, aminocarbonyl, alkylaminocarbonyl, alkylsulphonyl, cycloalkyl or cycloalkylalkyl group, or substituted by a cycloalkylcarbonyl, azacycloalkylcarbonyl, diazacycloalkylcarbonyl or oxazacycloalkylcarbonyl group optionally alkyl-substituted in the ring,
while the alicyclic moieties contained in these substituents each comprise 3 to 10 ring members and the heteroalicyclic moieties each comprise 4 to 10 ring members and
the phenyl and pyridinyl groups contained in the abovementioned groups may in turn be mono-, di- or trisubstituted by fluorine, chlorine, bromine or iodine atoms, by alkyl, alkoxy, methylsulphonyloxy, difluoromethyl, trifluoromethyl, hydroxy, amino, acetylamino, aminocarbonyl, aminocarbonylamino, aminocarbonylaminomethyl, cyano, carboxy, alkoxycarbonyl, carboxyalkyl, alkoxycarbonylalkyl, alkanoyl, ω-(dialkylamino)alkanoyl, ω-(carboxy)alkanoyl, difluoromethoxy, trifluoromethoxy, trifluoromethylthio, trifluoromethylsulphinyl or trifluoromethylsulphonyl groups, while the substituents may be identical or different,
R⁵ denotes a hydrogen atom,
a C₁₋₄-alkyl group , while an unbranched alkyl group may be substituted in the ω position by a phenyl, pyridinyl, diazinyl, amino, alkylamino, dialkylamino, 1-pyrrolidinyl, 1-piperidinyl, 4-methyl-1-piperazinyl, 4-morpholinyl or hexahydro-1*H*-1-azepinyl group,
an alkoxycarbonyl, the cyano or aminocarbonyl group or also, if Y¹ denotes a nitrogen atom, a pair of free electrons,
or, if Y¹ does not denote a nitrogen atom, also the fluorine atom, or
R⁴ and R⁵ together, if Y¹ denotes the carbon atom, denote a 4- to 7-membered cycloaliphatic ring in which one or two methylene groups may be replaced by an -NH- or -N(alkyl)- group and one or two additional methylene groups may be replaced by carbonyl groups,
while a hydrogen atom bound to a nitrogen atom within the abovementioned group R⁴ may be replaced by a protecting group,
R⁶ and R⁷, which may be identical or different, in each case denote a hydrogen atom, a C₁₋₃-alkyl or dialkylamino group or also, if Y¹ does not denote a nitrogen atom, the fluorine atom and
R⁸ and R⁹, which may be identical or different, each denote a hydrogen atom or a C₁₋₃-alkyl, carboxy or C₁₋₃-alkoxycarbonyl group,
while, unless otherwise stated, all the abovementioned alkyl and alkoxy groups as well as the alkyl groups present within the other groups specified comprise 1 to 7 carbon atoms and may be straight-chain or branched, while each methylene group may be substituted by up to 2 fluorine atoms and each methyl group may be substituted by up to 3 fluorine atoms,
all the abovementioned cycloalkyl groups as well as the cycloalkyl groups present within the other groups specified, unless otherwise stated, may comprise 3 to 10 carbon atoms, while each methylene group may be substituted by up to 2 fluorine atoms,
all the abovementioned aromatic and heteroaromatic groups may additionally be mono- di- or trisubstituted by fluorine, chlorine or bromine atoms, by cyano or hydroxy groups and the substituents may be identical or different,
the tautomers, diastereomers, enantiomers, hydrates, mixtures thereof and the salts thereof as well as the hydrates of the salts.

2. Compounds of general formula (I) according to claim 1, wherein
A, U, V, W, X, R² and R³ are defined as in claim 1 and
R¹ denotes a mono- or diunsaturated 5- to 7-membered aza, diaza, triaza or thiaza heterocyclic group,
in which the abovementioned heterocycles are linked via a carbon or nitrogen atom,
contain one or two carbonyl groups adjacent to a nitrogen atom, may be substituted at a carbon atom by a phenyl, pyridinyl, diazinyl, thienyl, pyrrolyl, 1,3-thiazolyl, isoxazolyl, pyrazolyl or 1-methylpyrazolyl group and
an olefinic double bond of one of the abovementioned unsaturated heterocycles may be fused to a phenyl, naphthyl, pyridine, diazine, thienyl or quinoline ring or to a 1*H*-quinolin-2-one ring optionally substituted at the nitrogen atom by a methyl group,
while the phenyl, pyridinyl, diazinyl, thienyl, pyrrolyl, 1,3-thiazolyl, isoxazolyl, pyrazolyl or 1-methylpyrazolyl groups contained in R¹ as well as the benzo-, pyrido- and diazino-fused heterocycles in the carbon skeleton may additionally be mono-, di- or trisubstituted by fluorine, chlorine, bromine or iodine atoms, by alkyl, alkoxy, nitro, difluoromethyl, trifluoromethyl, hydroxy, amino, alkylamino, dialkylamino, acetylamino, acetyl, cyano, difluoromethoxy or trifluoromethoxy groups, while the substituents may be identical or different,
while the abovementioned alkyl groups or the alkyl groups contained in the abovementioned groups, unless otherwise stated, contain 1 to 7 carbon atoms and may be branched or unbranched, while each methylene group may be substituted by up to 2 fluorine atoms and each methyl group may be substituted by up to 3 fluorine atoms, and
the abovementioned aromatic and heteroaromatic groups may additionally be mono- di- or trisubstituted by fluorine, chlorine or bromine atoms or by cyano or hydroxy groups and the substituents may be identical or different,
the tautomers, diastereomers, enantiomers, mixtures thereof and the salts thereof.

3. Compounds of general formula (I) according to claim 1, wherein
A, U, V, W, X, R² and R³ are defined as in claim 1 and
R¹ denotes a monounsaturated 5- to 7-membered diaza or triaza heterocyclic group,
while the abovementioned heterocycles are linked via a nitrogen atom,
contain a carbonyl group adjacent to a nitrogen atom and
may additionally be substituted at a carbon atom by a phenyl group,
and while an olefinic double bond of one of the abovementioned unsaturated heterocycles may be fused to a phenyl, thienyl or quinoline ring,
while the phenyl groups contained in R¹ as well as benzo-fused heterocycles in the carbon skeleton may additionally be mono-, di- or trisubstituted by fluorine, chlorine, bromine or iodine atoms, by methyl, methoxy, nitro, difluoromethyl, trifluoromethyl, hydroxy, amino, alkylamino, dialkylamino, acetylamino, acetyl, cyano, difluoromethoxy or trifluoromethoxy groups, while the substituents may be identical or different, but are preferably unsubstituted, or monosubstituted by a fluorine, chlorine or bromine atom or by a methyl or methoxy group,
while, unless otherwise stated, all the abovementioned alkyl groups as well as the alkyl groups present within the other groups comprise 1 to 7 carbon atoms and may be straight-chain or branched and the abovementioned aromatic and heteroaromatic groups may additionally be mono- di- or trisubstituted by fluorine, chlorine or bromine atoms or by cyano or hydroxy groups and the substituents may be identical or different,
the tautomers, diastereomers, enantiomers, mixtures thereof and the salts thereof.

4. Compounds of general formula (I) according to claim 1, wherein
A, U, V, W, X, R² and R³ are defined as in claim 1 and
R¹ denotes a 4-(2-oxo-1,2,4,5-tetrahydro-1,3-benzodiazepin-3-yl)-, 4-(2-oxo-1,4-dihydro-2*H*-quinazolin-3-yl)-, 4-(5-oxo-3-phenyl-4,5-dihydro-1,2,4-triazol-1-yl)-, 4-(2-oxo-1,2-dihydro-imidazo[4,5-c]quinolin-3-yl)-, 4-(2-oxo-1,2-dihydro-4*H*-thieno[3,4-d]pyrimidin-3-yl)-, 4-(2-oxo-1,4-dihydro-2*H*-thieno[3,2-d]pyrimidin-3-yl)-, 4-(5-oxo-4,5,7,8-tetrahydro-2-thia-4,6-diaza-azulen-6-yl)-, 4-(2-oxo-1,2,4,5-tetrahydro-thieno[3,2-d]-1,3-diazepin-3-yl)-, 4-(2-oxo-1,2,4,5-tetrahydro-thieno[2,3-d]-1,3-diazepin-3-yl)- or 4-(2-oxo-1,4-dihydro-2*H-*thieno[2,3-d]pyrimidin-3-yl)- group,
while the abovementioned mono- and bicyclic heterocycles in the carbon skeleton may additionally be monosubstituted by a methoxy group,
while the abovementioned aromatic and heteroaromatic groups by fluorine, chlorine or bromine atoms, by cyano or hydroxy groups may additionally be mono- di- or trisubstituted and the substituents may be identical or different,
the tautomers, diastereomers, enantiomers, mixtures thereof and the salts thereof.

5. Compounds of general formula (I) according to claim 1, wherein
A, U, V, W, X and R¹ are defined as in claim 1 and
R² denotes the hydrogen atom or
a phenylmethyl group or a C₂₋₇-alkyl group which may be substituted in the ω position by a phenyl, pyridinyl, hydroxy, amino, alkylamino, dialkylamino, carboxy, alkoxycarbonyl, aminocarbonyl, aminocarbonylamino, acetylamino, 1-pyrrolidinyl, 1-piperidinyl, 4-morpholinyl, [bis-(2-hydroxyethyl)]amino group
while the abovementioned heterocyclic groups and phenyl groups may additionally be mono-, di- or trisubstituted in the carbon skeleton by fluorine, chlorine, bromine or iodine atoms, by methyl, alkoxy, difluoromethyl, trifluoromethyl, hydroxy, amino, C₁₋₃-alkylamino, di-(C₁₋₃-alkyl)-amino, acetylamino, aminocarbonyl, cyano, difluoromethoxy, trifluoromethoxy, amino-C₁₋₃-alkyl, C₁₋₃-alkylamino-C₁₋₃-alkyl or di-(C₁₋₃-alkyl)-amino-C₁₋₃-alkyl groups and the substituents may be identical or different,
R³ denotes the hydrogen atom or a C₁₋₃-alkyl group,
while the C₁₋₃-alkyl group may be linked to an alkyl group present in R² or a phenyl or pyridyl ring present in R² and the nitrogen atom to which they are bound, forming a 5- to 7-membered ring, or
R² and R³ together with the enclosed nitrogen atom denote a group of general formula 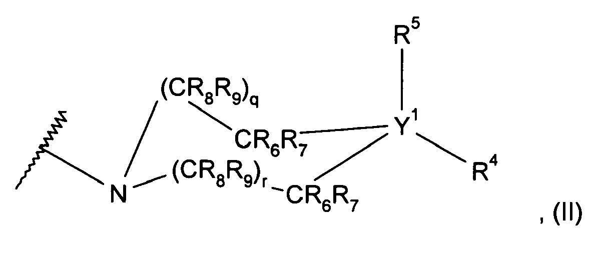 wherein
Y¹ denotes the carbon atom or, if R⁵ denotes a pair of free electrons, it may also denote the nitrogen atom,
q and r, if Y¹ denotes the carbon atom, represent the numbers 0 or 1 or
q and r, if Y¹ denotes the nitrogen atom, represent the numbers 1 or 2,
R⁴ denotes the hydrogen atom, a hydroxy, amino, alkylamino, C₃₋₆-cycloalkylamino, *N*-(C₃₋₆-cycloalkyl)-alkylamino or dialkylamino, an alkyl, trifluoromethyl, C₃₋₆-cycloalkyl, dialkylamino-C₂₋₇-alkyl, carboxyalkyl, alkoxycarbonylalkyl, alkylsulphonyl, alkylsulphonylamino or *N*-(alkylsulphonyl)-alkylamino group,
or, if Y¹ does not denote the nitrogen atom, it denotes the carboxy or dialkylaminomethyl group,
a phenyl, phenyl-C₁₋₃-alkyl, pyridinyl or diazinyl group each of which may be substituted by a fluorine, chlorine or bromine atom or by a trifluoromethylcarbonyl, methyl or methoxy group,
a saturated or mono- or polyunsaturated 4- to 7-membered azacycloalkyl group, a 5- to 7-membered oxaza-, diaza or triazacycloalkyl group, a 7- to 9-membered azabicyclo or diazabicycloalkyl group, a 1-alkyl-4-piperidinylamino or 1-alkyl-4-piperidinylaminosulphonyl group,
while the abovementioned mono- and bicyclic heterocycles are bound via a nitrogen or carbon atom,
a methylene group of the abovementioned mono- and bicyclic heterocycles may be replaced by a carbonyl or sulphonyl group, in the abovementioned mono- and bicyclic heterocycles any methylene group not directly bound to a nitrogen, oxygen or sulphur atom may be substituted by one or two fluorine atoms,
the abovementioned mono- and bicyclic heterocycles may be substituted by one or two C₁₋₃-alkyl groups wherein each methylene group may be substituted by up to 2 fluorine atoms and each methyl group may be substituted by up to 3 fluorine atoms, and/or
by a C₃₋₆-cycloalkyl-C₁₋₃-alkyl, benzyl, C₁₋₄-alkanoyl, di-(C₁₋₃-alkyl)-amino or C₁₋₃-alkylsulphonyl, by an alkoxycarbonyl, benzyloxycarbonyl, alkoxycarbonylalkyl, carboxy or carboxyalkyl group,
R⁵ denotes a hydrogen atom, a C₁₋₃-alkyl or alkoxycarbonyl group or,
if Y¹ denotes a nitrogen atom, it may also denote a pair of free electrons, or
R⁴ and R⁵ together, if Y¹ denotes the carbon atom, represent a 5- to 6-membered cycloaliphatic ring in which one or two methylene groups may be replaced by a ―NH- or ―N(methyl)- group and one or two further methylene groups may be replaced by carbonyl groups,
R⁶ and R⁷, which may be identical or different, in each case denote a hydrogen atom or a C₁₋₃-alkyl or di-(C₁₋₃-alkyl)-amino group and
R⁸ and R⁹, which may be identical or different, in each case denote a hydrogen atom or a C₁₋₃-alkyl, carboxy or C₁₋₃-alkoxycarbonyl group,
while, unless otherwise stated, all the abovementioned alkyl groups as well as the alkyl groups present within the other groups comprise 1 to 7 carbon atoms and may be straight-chain or branched and the abovementioned aromatic and heteroaromatic groups may additionally be mono-, di- or trisubstituted by fluorine, chlorine or bromine atoms or by cyano or hydroxy groups and the substituents may be identical or different,
the tautomers, diastereomers, enantiomers, mixtures thereof and the salts thereof.

6. Compounds of general formula (I) according to claim 1, wherein
A, U, V, W, X and R¹ are defined as in claim 1 and
R² denotes a phenylmethyl group or a C₂₋₇-alkyl group which may be substituted in the ω position by a phenyl, amino, alkylamino or dialkylamino group,
while the abovementioned phenyl group may be substituted by an amino-C₁₋₃-alkyl, C₁₋₃-alkylamino-C₁₋₃alkyl or di-(C₁₋₃-alkyl)-amino-C₁₋₃-alkyl group, or
R³ denotes the hydrogen atom or a C₁₋₃-alkyl group,
R² and R³ together with the nitrogen atom to which they are bound denote a 7-dimethylaminomethyl-1,2,4,5-tetrahydro-3-benzazepin-3-yl or 2-amino-4,5,7,8-tetrahydro-thiazolo[4,5-d]azepin-6-yl- group or
R² and R³ together with the enclosed nitrogen atom denote a group of general formula 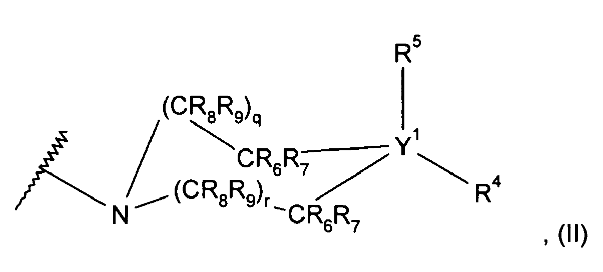 wherein
Y¹ denotes the carbon atom or, if R⁵ denotes a pair of free electrons, it may also denote the nitrogen atom,
q and r, if Y¹ denotes the carbon atom, represent the numbers 0 or 1 or
q and r, if Y¹ denotes the nitrogen atom, represent the numbers 1 or 2,
R⁴ denotes the hydrogen atom,
a phenyl, benzyl or pyridinyl group which may be substituted in each case by a fluorine, chlorine or bromine atom, by a trifluoromethylcarbonyl, methyl or methoxy group,
a hydroxy, carboxy, methyl, trifluoromethyl, *n*-propyl, phenyl, *p*-tolyl, *p*-trifluoromethylcarbonyl-phenyl, *p*-(3-dimethylaminopropyl)-phenyl, amino, benzyl, *tert*-butylamino, dimethylamino, diethylamino, diethylaminomethyl, 2-dimethylaminoethyl, 2-diethylaminoethyl, 5-aminopentyl, methoxycarbonyl, methoxycarbonylmethyl, perhydro-azepin-1-yl, 4-methyl-perhydro-1,4-diazepin-1-yl, 1-methyl-1-piperidinyl-4-yl, 4-piperazin-1-yl, 4-acetyl-piperazin-1-yl, 4-cyclopropylmethyl-piperazin-1-yl, pyrrolidin-1-yl, 4-ethyl-piperazin-1-yl, 4-isopropyl-piperazin-1-yl, piperidin-1-yl, piperidin-4-yl, morpholin-4-yl, 4,4-difluoro-1-piperidin-1-yl, 1-methyl-1-aza-bicyclo[3.2.1]oct-4-yl, 4-methyl-piperazin-1-yl, 4-ethylpiperazin-1-yl, 1-methyl-piperidin-1-yl, 4-carboxymethyl-piperazin-1-yl, 1-carboxymethyl-piperidin-4-yl, 4-benzyloxycarbonyl-piperazin-1-yl, 1-ethoxycarbonylmethyl-piperidin-4-yl, azetidin-1-yl, 5-methyl-2,5-diaza-bicyclo[2.2.1]hept-2-yl, 1-benzyl-piperidin-4-yl, 4-benzyl-piperazin-1-yl, 4-dimethylaminomethyl-1-phenyl, 2,2,2-trifluoroethyl-piperazin-1-yl, 1-methylsulphonyl-piperidin-4-yl, piperidin-1-yl-methyl, 1-methyl-piperidin-4-yl-amino, methylsulphonylamino, *N*-methylsulphonyl-*N*-methylamino, *N*-(cyclopentyl)-methylamino, 1,1-dioxo-λ⁶-isothiazolidin-2-yl, 2-oxo-perhydro-1,3-oxazin-3-yl, cyclohexyl, 2-oxo-imidazolidin-1-yl, 2-methyl-imidazol-1-yl, 4-methyl-imidazol-1-yl, 4-thiazol-2-yl, 2,4-dimethyl-imidazol-1-yl, 4-imidazol-1-yl, 1,2,4-triazol-1-yl, 1-aza-bicyclo[2.2.2]oct-3-yl, 1-methyl-piperidin-4-yl-methylsulphonyl, 1*H*-imidazol-4-yl, 4-ethoxycarbonylmethyl-piperazin-1-yl, 1-ethoxycarbonyl-piperidin-4-yl, 4-*tert-*butoxycarbonylmethyl-piperazin-1-yl, 1-(2,2,2-trifluoroethyl)-piperidin-4-yl, 4-methylsulphonyl-piperazin-1-yl, 2-carboxy-4-methyl-piperazin-1-yl, 3-carboxy-4-methyl-piperazin-1-yl, 2-ethoxycarbonyl-4-methyl-piperazin-1-yl, 3-ethoxycarbonyl-4-methyl-piperazin-1-yl or 4-(2,2,2-trifluoroethyl)-piperazin-1-yl- group,
R⁵ denotes a hydrogen atom, a methyl group or, if Y¹ denotes a nitrogen atom, it may also denote a pair of free electrons, or
R⁴ and R⁵ together, if Y¹ denotes the carbon atom, denote a 1-methyl-piperidin-4-ylidene, cyclohexylidene or imidazolidin-2,4-dion-5-ylidene group,
R⁶ and R⁷ in each case denote a hydrogen atom or a dimethylamino group and
R⁸ and R⁹ in each case denote the hydrogen atom, a carboxy or ethoxycarbonyl group,
while, unless otherwise stated, all the abovementioned alkyl groups as well as the alkyl groups present within the other groups comprise 1 to 7 carbon atoms and may be straight-chain or branched and the abovementioned aromatic and heteroaromatic groups may additionally be mono-, di- or trisubstituted by fluorine, chlorine or bromine atoms, by cyano or hydroxy groups and the substituents may be identical or different,
the tautomers, diastereomers, enantiomers, mixtures thereof and the salts thereof.

7. Compounds of general formula (I) according to claim 1, wherein
A denotes an oxygen atom, a cyanoimino or phenylsulphonylimino group,
X denotes an oxygen atom, an imino or methylene group,
U denotes an unbranched C₁₋₆-alkyl, C₂₋₆-alkenyl or C₂₋₆-alkynyl group wherein each methylene group may be substituted by up to 2 fluorine atoms and the methyl group may be substituted by up to 3 fluorine atoms,
V denotes an amino or hydroxy group and
W denotes a hydrogen, chlorine or bromine atom or a trifluoromethyl group,
R¹ denotes a monounsaturated 5- to 7-membered diaza- or triaza-heterocyclic group,
while the abovementioned heterocycles are linked via a nitrogen atom,
contain a carbonyl group adjacent to a nitrogen atom,
may additionally be substituted at a carbon atom by a phenyl group and
an olefinic double bond of one of the abovementioned unsaturated heterocycles may be fused to a phenyl, thienyl or quinoline ring,
while the phenyl groups contained in R¹ as well as benzo-fused heterocycles in the carbon skeleton may additionally be mono-, di- or trisubstituted by fluorine, chlorine, bromine or iodine atoms, by methyl, methoxy, nitro, difluoromethyl, trifluoromethyl, hydroxy, amino, alkylamino, dialkylamino, acetylamino, acetyl, cyano, difluoromethoxy or trifluoromethoxy groups, while the substituents may be identical or different, but are preferably unsubstituted or are monosubstituted by a fluorine, chlorine or bromine atom or by a methyl or methoxy group,
R² denotes the hydrogen atom or
a phenylmethyl group or a C₂₋₇-alkyl group which may be substituted in the ω position by a phenyl, pyridinyl, hydroxy, amino, alkylamino, dialkylamino, alkoxycarbonyl, carboxy, aminocarbonyl, aminocarbonylamino, acetylamino, 1-pyrrolidinyl, 1-piperidinyl, 4-morpholinyl or [bis-(2-hydroxyethyl)]amino group,
while the abovementioned heterocyclic groups and phenyl groups may additionally be mono-, di- or trisubstituted in the carbon skeleton by fluorine, chlorine, bromine or iodine atoms, by methyl, alkoxy, difluoromethyl, trifluoromethyl, hydroxy, amino, C₁₋₃-alkylamino, di-(C₁₋₃-alkyl)-amino, acetylamino, aminocarbonyl, cyano, difluoromethoxy, trifluoromethoxy, amino-C₁₋₃-alkyl, C₁₋₃-alkylamino-C₁₋₃-alkyl or di-(C₁₋₃-alkyl)-amino-C₁₋₃-alkyl groups and the substituents may be identical or different,
R³ denotes the hydrogen atom or a C₁₋₃-alkyl group,
while the C₁₋₃-alkyl group may be linked to an alkyl group present in R² or a phenyl or pyridyl ring present in R² and the nitrogen atom to which they are bound, forming a 5- to 7-membered ring, or
R² and R³ together with the enclosed nitrogen atom denote a group of general formula 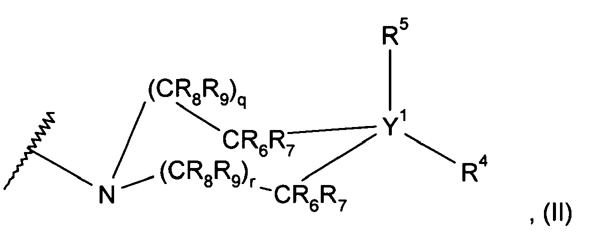 wherein
Y¹ denotes the carbon atom or, if R⁵ denotes a pair of free electrons, it may also denote the nitrogen atom,
q and r, if Y¹ denotes the carbon atom, represent the numbers 0 or 1 or
q and r, if Y¹ denotes the nitrogen atom, represent the numbers 1 or 2,
R⁴ denotes the hydrogen atom, a hydroxy, amino, alkylamino, C₃₋₆-cycloalkylamino, *N-*(C₃₋₆-cycloalkyl)-alkylamino or dialkylamino, an alkyl, trifluoromethyl, C₃₋₆-cycloalkyl, dialkylamino-C₂₋₇-alkyl, carboxyalkyl, alkoxycarbonylalkyl, alkylsulphonyl, alkylsulphonylamino or N-(alkylsulphonyl)-alkylamino group,
or, if Y¹ does not denote the nitrogen atom, it denotes the carboxy or dialkylaminomethyl group,
a phenyl, phenyl-C₁₋₃-alkyl, pyridinyl or diazinyl group which may be substituted in each case by a fluorine, chlorine or bromine atom, by a trifluoromethylcarbonyl, methyl or methoxy group,
a saturated or mono- or polyunsaturated 4- to 7-membered azacycloalkyl group, a 5- to 7-membered oxaza-, diaza- or triazacycloalkyl group, a 7-to 9-membered azabicyclo- or diazabicycloalkyl group, a 1-alkyl-4-piperidinylamino or 1-alkyl-4-piperidinylaminosulphonyl group,
while the abovementioned mono- and bicyclic heterocycles are bound via a nitrogen or carbon atom,
a methylene group of the abovementioned mono- and bicyclic heterocycles may be replaced by a carbonyl or sulphonyl group,
in the abovementioned mono- and bicyclic heterocycles any methylene group not directly bound to a nitrogen, oxygen or sulphur atom may be substituted by one or two fluorine atoms,
the abovementioned mono- and bicyclic heterocycles may be substituted by one or two C₁₋₃-alkyl groups, wherein each methylene group may be substituted by up to 2 fluorine atoms and each methyl group may be substituted by up to 3 fluorine atoms, and/or
may be substituted by a C₃₋₆-cycloalkyl-C₁₋₃-alkyl, benzyl, C₁₋₄alkanoyl, di-(C₁₋₃-alkyl)-amino or C₁₋₃-alkylsulphonyl, by an alkoxycarbonyl, benzyloxycarbonyl, alkoxycarbonylalkyl, carboxy or carboxyalkyl group,
R⁵ denotes a hydrogen atom, a C₁₋₃-alkyl or alkoxycarbonyl group or, if Y¹ denotes a nitrogen atom, it may also denote a pair of free electrons, or
R⁴ and R⁵ together, if Y¹ denotes the carbon atom, denote a 5- to 6-membered cycloaliphatic ring wherein one or two methylene groups may be replaced by a ―NH- or ―N(methyl)- group and one or two further methylene groups may be replaced by one or two carbonyl groups,
R⁶ and R⁷, which may be identical or different, in each case denote the hydrogen atom or a C₁₋₃-alkyl or di-(C₁₋₃-alkyl)-amino group and
R⁸ and R⁹, which may be identical or different, in each case denote the hydrogen atom or a C₁₋₃-alkyl, carboxy or C₁₋₃-alkoxycarbonyl group,
while, unless otherwise stated, the abovementioned alkyl groups or the alkyl groups contained in the abovementioned groups contain 1 to 7 carbon atoms and may be branched or unbranched and the abovementioned aromatic and heteroaromatic groups may additionally be mono-, di- or trisubstituted by fluorine, chlorine or bromine atoms, by cyano or hydroxy groups and the substituents may be identical or different
the tautomers, enantiomers, diastereomers, mixtures thereof and the salts thereof.

8. Compounds of general formula (I) according to claim 1, wherein
A denotes an oxygen atom,
X denotes an oxygen atom, an imino or methylene group,
U denotes a methyl, ethyl, C₂₋₄-alkenyl or C₂₋₄-alkynyl group wherein the methylene group may be substituted by up to 2 fluorine atoms and the methyl group may be substituted by up to 3 fluorine atoms,
V denotes an amino or hydroxy group,
W denotes a hydrogen, chlorine or bromine atom or a trifluoromethyl group,
R¹ denotes a 4-(2-oxo-1,2,4,5-tetrahydro-1,3-benzodiazepin-3-yl)-, 4-(2-oxo-1,4-dihydro-2H-quinazolin-3-yl)-, 4-(5-oxo-3-phenyl-4,5-dihydro-1,2,4-triazol-1-yl)-, 4-(2-oxo-1,2-dihydro-imidazo[4,5-c]quinolin-3-yl)-, 4-(2-oxo-1,2-dihydro-4*H*-thieno[3,4-d]pyrimidin-3-yl)-, 4-(2-oxo-1,4-dihydro-2*H*-thieno[3,2-d]pyrimidin-3-yl)-, 4-(5-oxo-4,5,7,8-tetrahydro-2-thia-4,6-diaza-azulen-6-yl)-, 4-(2-oxo-1,2,4,5-tetrahydro-thieno[3,2-d]-1,3-diazepin-3-yl)-, 4-(2-oxo-1,2,4,5-tetrahydro-thieno[2,3-d]-1,3-diazepin-3-yl)- or 4-(2-oxo-1,4-dihydro-2*H-*thieno[2,3-d]pyrimidin-3-yl)- group,
while the abovementioned mono- and bicyclic heterocycles in the carbon skeleton may additionally be monosubstituted by a methoxy group,
R² denotes a phenylmethyl group or a C₂₋₇-alkyl group which may be substituted in the ω position by a phenyl, amino, alkylamino or dialkylamino group,
while the abovementioned phenyl group may be substituted by an amino-C₁₋₃-alkyl, C₁₋₃-alkylamino-C₁₋₃-alkyl or di-(C₁₋₃-alkyl)-amino-C₁₋₃-alkyl group, or
R³ denotes the hydrogen atom or a C₁₋₃-alkyl group,
R² and R³ together with the nitrogen atom to which they are bound denote a 7-dimethylaminomethyl-1,2,4,5-tetrahydro-3-benzazepin-3-yl or 2-amino-4,5,7,8-tetrahydro-thiazolo[4,5-d]azepin-6-yl- group or
R² and R³ together with the enclosed nitrogen atom denote a group of general formula 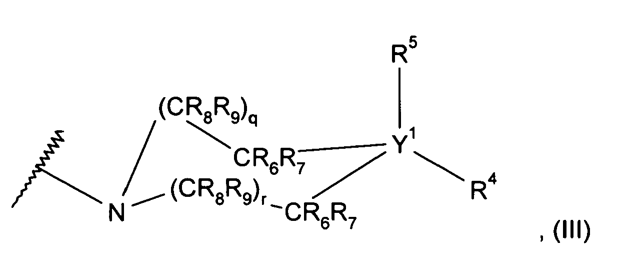 wherein
Y¹ represents the carbon atom or, if R⁵ denotes a pair of free electrons, it may also denote the nitrogen atom,
q and r, if Y¹ denotes the carbon atom, represent the numbers 0 or 1 or
q and r, if Y¹ denotes the nitrogen atom, represent the numbers 1 or 2,
R⁴ denotes the hydrogen atom,
a phenyl, benzyl or pyridinyl group which may be substituted in each case by a fluorine, chlorine or bromine atom, by a trifluoromethylcarbonyl, methyl or methoxy group,
a hydroxy, carboxy, methyl, trifluoromethyl, *n*-propyl, phenyl, p-tolyl, *p*-trifluoromethylcarbonyl-phenyl, *p*-(3-dimethylaminopropyl)-phenyl, amino, benzyl, *tert*-butylamino, dimethylamino, diethylamino, diethylaminomethyl, 2-dimethylaminoethyl, 2-diethylaminoethyl, 5-aminopentyl, methoxycarbonyl, methoxycarbonylmethyl, perhydro-azepin-1-yl, 4-methyl-perhydro-1,4-diazepin-1-yl, 1-methyl-1-piperidinyl-4-yl, 4-piperazin-1-yl, 4-acetyl-piperazin-1-yl, 4-cyclopropylmethyl-piperazin-1-yl, pyrrolidin-1-yl, 4-ethyl-piperazin-1-yl, 4-isopropyl-piperazin-1-yl, piperidin-1-yl, piperidin-4-yl, morpholin-4-yl, 4,4-difluoro-1-piperidin-1-yl, 1-methyl-1-aza-bicycto[3.2.1]oct-4-yl, 4-methyl-piperazin-1-yl, 4-ethylpiperazin-1-yl, 1-methyl-piperidin-1-yl, 4-carboxymethyl-piperazin-1-yl, 1-carboxymethyl-piperidin-4-yl, 4-benzyloxycarbonyl-piperazin-1-yl, 1-ethoxycarbonylmethyl-piperidin-4-yl, azetidin-1-yl, 5-methyl-2,5-diaza-bicyclo[2.2.1]hept-2-yl, 1-benzyl-piperidin-4-yl, 4-benzyl-piperazin-1-yl, 4-dimethylaminomethyl-1-phenyl, 2,2,2-trifluoroethyl-piperazin-1-yl, 1-methylsulphonyl-piperidin-4-yl, piperidin-1-yl-methyl, 1-methyl-piperidin-4-yl-amino, methylsulphonylamino, *N*-methylsulphonyl-*N*-methylamino, *N*-(cyclopentyl)-methylamino, 1,1-dioxo-λ⁶-isothiazolidin-2-yl, 2-oxo-perhydro-1,3-oxazin-3-yl, cyclohexyl, 2-oxo-imidazolidin-1-yl, 2-methyl-imidazol-1-yl, 4-methyl-imidazol-1-yl, 4-thiazol-2-yl, 2,4-dimethyl-imidazol-1-yl, 4-imidazol-1-yl, 1,2,4-triazol-1-yl, 1-aza-bicyclo[2.2.2]oct-3-yl, 1-methyl-piperidin-4-yl-methylsulphonyl, 1*H*-imidazol-4-yl, 4-ethoxycarbonylmethyl-piperazin-1-yl, 1-ethoxycarbonyl-piperidin-4-yl, 4*-tert*-butoxycarbonylmethyl-piperazin-1-yl, 1-(2,2,2-trifluoroethyl)-piperidin-4-yl, 4-methylsulphonyl-piperazin-1-yl, 2-carboxy-4-methyl-piperazin-1-yl, 3-carboxy-4-methyl-piperazin-1-yl, 2-ethoxycarbonyl-4-methyl-piperazin-1-yl, 3-ethoxycarbonyl-4-methyl-piperazin-1-yl or 4-(2,2,2-trifluoroethyl)-piperazin-1-yl group,
R⁵ denotes a hydrogen atom, a methyl group or, if Y¹ denotes a nitrogen atom, it may also denote a pair of free electrons, or
R⁴ and R⁵ together, if Y¹ denotes the carbon atom, denote a 1-methyl-piperidin-4-ylidene, cyclohexylidene or imidazolidin-2,4-dion-5-ylidene group,
R⁶ and R⁷ in each case denote a hydrogen atom or a dimethylamino group and
R⁸ and R⁹ in each case denote the hydrogen atom, a carboxy or ethoxycarbonyl group,
while, unless otherwise stated, all the abovementioned alkyl groups as well as the alkyl groups present within the other groups comprise 1 to 7 carbon atoms and may be straight-chain or branched and the abovementioned aromatic and heteroaromatic groups may additionally be mono-, di- or trisubstituted by fluorine, chlorine or bromine atoms or by cyano or hydroxy groups and the substituents may be identical or different,
the enantiomers, the diastereomers and the salts thereof.

9. The following compounds of general formula (I) according to claim 1:
(1) (*S*)-2-(4-amino-3-chloro-5-trifluoromethyl-benzyl)-1-[4-(4-methyl-piperazin-1-yl)-piperidin-1-yl]-4-[4-(2-oxo-1,2,4,5-tetrahydro-1,3-benzodiazepin-3-yl)-piperidin-1-yl]-butan-1,4-dione,
(2) 2-(4-amino-3-chloro-5-trifluoromethyl-benzyl)-1-(1'-methyl-[4,4']bipiperidinyl-1-yl)-4-[4-(2-oxo-1,4-dihydro-2*H*-quinazolin-3-yl)-piperidin-1-yl]-butan-1,4-dione,
(3) 2-(4-amino-3-chloro-5-trifluoromethyl-benzyl)-1-[4-(4-methyl-piperazin-1-yl)-piperid in-1-yl]-4-[4-(2-oxo-1,4-dihydro-2*H*-quinazolin-3-yl)-piperidin-1-yl]-butan-1,4-dione,
(4) 2-(4-amino-3-chloro-5-trifluoromethyl-benzyl)-1-[1,4']bipiperidinyl-1'-yl-4-[4-(2-oxo-1,4-dihydro-2*H*-quinazolin-3-yl)-piperidin-1-yl]-butan-1,4-dione,
(5) 2-(4-amino-3-chloro-5-trifluoromethyl-benzyl)-4-[4-(2-oxo-1,4-dihydro-2*H*-quinazolin-3-yl)-piperidin-1-yl]-1-(4-pyridin-4-yl-piperazin-1-yl)-butan-1,4-dione,
(6) 2-(4-amino-3-chloro-5-trifluoromethyl-benzyl)-1-[4-(1-methyl-piperidin-4-ylamino)-piperidin-1-yl]-4-[4-(2-oxo-1,4-dihydro-2*H*-quinazolin-3-yl)-piperidin-1-yl]-butan-1,4-dione,
(7) [4-(1-{2-(4-amino-3-chloro-5-trifluoromethyl-benzyl)-4-oxo-4-[4-(2-oxo-1,4-dihydro-2*H*-quinazolin-3-yl)-piperidin-1-yl]-butyryl}-piperidin-4-yl)-piperazin-1-yl]-acetic acid,
(8) methyl (1'-{2-(4-amino-3-chloro-5-trifluoromethyl-benzyl)-4-oxo-4-[4-(2-oxo-1,4-dihydro-2*H*-quinazolin-3-yl)-piperidin-1-yl]-butyryl}-[4,4']bipiperidinyl-1-yl)-acetate,
(9) (1'-{2-(4-amino-3-chloro-5-trifluoromethyl-benzyl)-4-oxo-4-[4-(2-oxo-1,4-dihydro-2*H*-quinazolin-3-yl)-piperidin-1-yl]-butyryl}-[4,4']bipiperidinyl-1-yl)-acetic acid,
(10) 4-(2-oxo-1,2,4,5-tetrahydro-1,3-benzodiazepin-3-yl)-piperidin-1-carboxylic acid-[(*R*)-1-(4-amino-3-chloro-5-trifluoromethyl-benzyl)-2-1,4'-bipiperidinyl-1'-yl-2-oxo-ethyl]-amide,
(11) 4-(2-oxo-1,2,4,5-tetrahydro-1,3-benzodiazepin-3-yl)-piperidin-1-carboxylic acid-[(*R*)-1-(4-amino-3-chloro-5-trifluoromethyl-benzyl)-2-(4-dimethylamino-piperidin-1-yl)-2-oxo-ethyl]-amide,
(12) 4-(2-oxo-1,2,4,5-tetrahydro-1,3-benzodiazepin-3-yl)-piperidin-1-carboxylic acid-[(*R*)-1-(4-amino-3-chloro-5-trifluoromethyl-benzyl)-2-oxo-2-(4-pyridin-4-yl-piperazin-1-yl)-ethyl]-amide,
(13) 4-(2-oxo-1,2,4,5-tetrahydro-1,3-benzodiazepin-3-yl)-piperidin-1-carboxylic acid-[(*R*)-1-(4-amino-3-chloro-5-trifluoromethyl-benzyl)-2-(1'-methyl-4,4'-bipiperidinyl-1-yl)-2-oxo-ethyl]-amide,
(14) benzyl 4-[1-((*R*)-3-(4-amino-3-chloro-5-trifluoromethyl-phenyl)-2-{[4-(2-oxo-1,2,4,5-tetrahydro-1,3-benzodiazepin-3-yl)-piperidin-1-carbonyl]-amino}-propionyl)-piperidin-4-yl]-piperazin-1-carboxylate,
(15) ethyl [1'-((*R*)-3-(4-amino-3-chloro-5-trifluoromethyl-phenyl)-2-{[4-(2-oxo-1,2,4,5-tetrahydro-1,3-benzodiazepin-3-yl)-piperidin-1-carbonyl]-amino}-propionyl)-4,4'-bipiperidinyl-1-yl]-acetate,
(16) 4-(2-oxo-1,2,4,5-tetrahydro-1,3-benzodiazepin-3-yl)-piperidin-1-carboxylic acid-{(*R*)-1-(4-amino-3-chloro-5-trifluoromethyl-benzyl)-2-[4-(4-methyl-piperazin-1-yl)-piperidin-1-yl]-2-oxo-ethyl}-amide,
(17) 4-(2-oxo-1,2,4,5-tetrahydro-1,3-benzodiazepin-3-yl)-piperidin-1-carboxylic acid-{(*R*)-1-(4-amino-3-chloro-5-trifluoromethyl-benzyl)-2-[4-(1-methyl-piperidin-4-yl)-piperazin-1-yl]-2-oxo-ethyl}-amide,
(18) 4-(2-oxo-1,2,4,5-tetrahydro-1,3-benzodiazepin-3-yl)-piperidin-1-carboxylic acid-[(*R*)-1-(4-amino-3-chloro-5-trifluoromethyl-benzyl)-2-(4-azetidin-1-yl-piperidin-1-yl)-2-oxo-ethyl]-amide,
(19) 4-(2-oxo-1,2,4,5-tetrahydro-1 ,3-benzodiazepin-3-yl)-piperidin-1-carboxylic acid-{(*R*)-1-(4-amino-3-chloro-5-trifluoromethyl-benzyl)-2-[4-(5-methyl-2,5-diaza-bicyclo[2.2.1]hept-2-yl)-piperidin-1-yl]-2-oxo-ethyl}-amide,
(20) 4-(2-oxo-1,2,4,5-tetrahydro-1,3-benzodiazepin-3-yl)-piperidin-1-carboxylic acid-[(*R*)-1-(4-amino-3-chloro-5-trifluoromethyl-benzyl)-2-oxo-2-(4-piperazin-1-yl-piperidin-1-yl)-ethyl]-amide,
(21) [1'-((*R*)-3-(4-amino-3-chloro-5-trifluoromethyl-phenyl)-2-{[4-(2-oxo-1,2,4,5-tetrahydro-1,3-benzodiazepin-3-yl)-piperidin-1-carbonyl]-amino}-propionyl)-4,4'-bipiperidinyl-1-yl]-acetic acid,
(22) 4-(2-oxo-1,2,4,5-tetrahydro-1,3-benzodiazepin-3-yl)-piperidin-1-carboxylic acid-[1-(4-amino-3-chloro-5-trifluoromethyl-benzyl)-2-1,4'-bipiperidinyl-1'-yl-2-oxo-ethyl]-amide,
(23) (*S*)-2-(4-amino-3-bromo-5-trifluoromethyl-benzyl)-1-[4-(4-methyl-piperazin-1-yl)-piperidin-1-yl]-4-[4-(2-oxo-1,2,4,5-tetrahydro-1,3-benzodiazepin-3-yl)-piperidin-1-yl]-butan-1,4-dione,
(24) (*S*)-2-(4-amino-3-bromo-5-trifluoromethyl-benzyl)-4-[4-(2-oxo-1,2,4,5-tetrahydro-1 ,3-benzodiazepin-3-yl)-piperidin-1-yl]-1-(4-pyridin-4-yl-piperazin-1-yl)-butan-1,4-dione,
(25) (*S*)-2-(4-amino-3-bromo-5-trifluoromethyl-benzyl)-1-1,4'-bipiperidinyl-1'-yl-4-[4-(2-oxo-1,2,4,5-tetrahydro-1,3-benzodiazepin-3-yl)-piperidin-1-yl]-butan-1,4-dione,
(26) (*S*)-2-(4-amino-3-bromo-5-trifluoromethyl-benzyl)-1-[4-(1-methyl-piperidin-4-yl)-piperazin-1-yl]-4-[4-(2-oxo-1,2,4,5-tetrahydro-1,3-benzodiazepin-3-yl)-piperidin-1-yl]-butan-1,4-dione,
(27) (*S*)-2-(4-amino-3-bromo-5-trifluoromethyl-benzyl)-1-(1'-methyl-4,4'-bipiperidinyl-1-yl)-4-[4-(2-oxo-1,2,4,5-tetrahydro-1,3-benzodiazepin-3-yl)-piperidin-1-yl]-butan-1,4-dione,
(28) (*S*)-2-(4-amino-3-trifluoromethyl-benzyl)-1-[4-(4-methyl-piperazin-1-yl)-piperidin-1-yl]-4-[4-(2-oxo-1,2,4,5-tetrahydro-1,3-benzodiazepin-3-yl)-piperidin-1-yl]-butan-1,4-dione,
(29) (*S*)-2-(4-amino-3-chloro-5-trifluoromethyl-benzyl)-1-(4-dimethylamino-piperidin-1-yl)-4-[4-(2-oxo-1,2,4,5-tetrahydro-1,3-benzodiazepin-3-yl)-piperidin-1-yl]-butan-1,4-dione,
(30) (*S*)-2-(4-amino-3-chloro-5-trifluoromethyl-benzyl)-1-(3-dimethylamino-piperidin-1-yl)-4-[4-(2-oxo-1,2,4,5-tetrahydro-1,3-benzodiazepin-3-yl)-piperidin-1-yl]-butan-1,4-dione,
(31) (*S*)-2-(4-amino-3-chloro-5-trifluoromethyl-benzyl)-4-[4-(2-oxo-1,2,4,5-tetrahydro-l ,3-benzodiazepin-3-yl)-piperidin-1-yl]-1-(4-pyrrolidin-1-yl-piperidin-1-yl)-butan-1,4-dione,
(32) (*S*)-2-(4-amino-3-chloro-5-trifluoromethyl-benzyl)-1-(1'-methyl-4,4'-bipiperidinyl-1-yl)-4-[4-(2-oxo-1,2,4,5-tetrahydro-1,3-benzodiazepin-3-yl)-piperidin-1-yl]-butan-1,4-dione,
(33) (*S*)-2-(4-amino-3-chloro-5-trifluoromethyl-benzyl)-1-[4-(4-cyclopropylmethyl-piperazin-1-yl)-piperidin-1-yl]-4-[4-(2-oxo-1,2,4,5-tetrahydro-1,3-benzodiazepin-3-yl)-piperidin-1-yl]-butan-1,4-dione
(34) (*S*)-2-(4-amino-3-chloro-5-trifluoromethyl-benzyl)-1-(4-morpholine-4-yl-piperidin-1-yl)-4-[4-(2-oxo-1,2,4,5-tetrahydro-1,3-benzodiazepin-3-yl)-piperidin-1-yl]-butan-1,4-dione,
(35) (*S*)-2-(4-amino-3-chloro-5-trifluoromethyl-benzyl)-4-[4-(2-oxo-1,2,4,5-tetrahydro-1,3-benzodiazepin-3-yl)-piperidin-1-yl]-1-(4-perhydro-azepin-1-yl-piperidin-1-yl)-butan-1,4-dione,
(36) (*S*)-2-(4-amino-3-chloro-5-trifluoromethyl-benzyl)-1-[4-(4-methyl-perhydro-1,4-diazepin-1-yl)-piperidin-1-yl]-4-[4-(2-oxo-1,2,4,5-tetrahydro-1,3-benzodiazepin-3-yl)-piperidin-1-yl]-butan-1,4-dione,
(37) (*S*)-2-(4-amino-3-chloro-5-trifluoromethyl-benzyl)-1-[4-(4-isopropyl-piperazin-1-yl)-piperidin-1-yl]-4-[4-(2-oxo-1,2,4,5-tetrahydro-1,3-benzodiazepin-3-yl)-piperidin-1-yl]-butan-1,4-dione,
(38) (*S*)-2-(4-amino-3-chloro-5-trifluoromethyl-benzyl)-1-1,4'-bipiperidinyl-1'-yl-4-[4-(2-oxo-1,2,4,5-tetrahydro-1 ,3-benzodiazepin-3-yl)-piperidin-1-yl]-butan-1,4-dione,
(39) (*S*)-2-(4-amino-3-chloro-5-trifluoromethyl-benzyl)-1-[4-(1-methyl-piperidin-4-yl)-perhydro-1,4-diazepin-1-yl]-4-[4-(2-oxo-1,2,4,5-tetrahydro-1,3-benzodiazepin-3-yl)-piperidin-1-yl]-butan-1,4-dione,
(40) (*S*)-2-(4-amino-3-chloro-5-trifluoromethyl-benzyl)-1-[3-(4-methyl-piperazin-1-yl)-azetidin-1-yl]-4-[4-(2-oxo-1,2,4,5-tetrahydro-1,3-benzodiazepin-3-yl)-piperidin-1-yl]-butan-1,4-dione,
(41) (*S*)-2-(4-amino-3-chloro-5-trifluoromethyl-benzyl)-4-[4-(2-oxo-1,2,4,5-tetrahydro-1,3-benzodiazepin-3-yl)-piperidin-1-yl]-1-(3-pyrrolidin-1-yl-azetidin-1-yl)-butan-1,4-dione,
(42) (*S*)-2-(4-amino-3-chloro-5-trifluoromethyl-benzyl)-4-[4-(2-oxo-1,2,4,5-tetrahydro-1,3-benzodiazepin-3-yl)-piperidin-1-yl]-1-(3-piperidin-1-yl-azetidin-1-yl)-butan-1,4-dione,
(43) (*S*)-2-(4-amino-3-chloro-5-trifluoromethyl-benzyl)-1-(3-diethylamino-azetidin-1-yl)-4-[4-(2-oxo-1,2,4,5-tetrahydro-1,3-benzodiazepin-3-yl)-piperidin-1-yl]-butan-1,4-dione,
(44) (*S*)-2-(4-amino-3-chloro-5-trifluoromethyl-benzyl)-1-(4-azetidin-1-yl-piperidin-1-yl)-4-[4-(2-oxo-1,2,4,5-tetrahydro-1,3-benzodiazepin-3-yl)-piperidin-1-yl]-butan-1,4-dione,
(45) (*S*)-1-[4-(4-acetyl-piperazin-1-yl)-piperidin-1-yl]-2-(4-amino-3-chloro-5-trifluoromethyl-benzyl)-4-[4-(2-oxo-1,2,4,5-tetrahydro-1,3-benzodiazepin-3-yl)-piperidin-1-yl]-butan-1,4-dione,
(46) (*S*)-2-(4-amino-3-chloro-5-trifluoromethyl-benzyl)-1-(4-diethylaminomethyl-piperidin-1-yl)-4-[4-(2-oxo-1,2,4,5-tetrahydro-1,3-benzodiazepin-3-yl)-piperidin-1-yl]-butan-1,4-dione,
(47) (*S*)-2-(4-amino-3-chloro-5-trifluoromethyl-benzyl)-1-[4-(4-ethyl-piperazin-1-yl)-piperidin-1-yl]-4-[4-(2-oxo-1,2,4,5-tetrahydro-1,3-benzodiazepin-3-yl)-piperidin-1-yl]-butan-1,4-dione,
(48) (*S*)-2-(4-amino-3-chloro-5-trifluoromethyl-benzyl)-1-[4-(1-ethyl-piperidin-4-yl)-piperazin-1-yl]-4-[4-(2-oxo-1,2,4,5-tetrahydro-1,3-benzodiazepin-3-yl)-piperidin-1-yl]-butan-1,4-dione,
(49) (*S*)-2-(4-amino-3-chloro-5-trifluoromethyl-benzyl)-4-[4-(2-oxo-1,2,4,5-tetrahydro-1,3-benzodiazepin-3-yl)-piperidin-1-yl]-1-(3,4,5,6-tetrahydro-2*H*-4,4'-bipyridinyl-1-yl)-butan-1,4-dione,
(50) (*S*)-2-(4-amino-3-chloro-5-trifluoromethyl-benzyl)-4-[4-(2-oxo-1,2,4,5-tetrahydro-1,3-benzodiazepin-3-yl)-piperidin-1-yl]-1-(4-pyridin-4-yl-piperazin-1-yl)-butan-1,4-dione,
(51) (*S*)-2-(4-amino-3-chloro-5-trifluoromethyl-benzyl)-4-[4-(2-oxo-1,2,4,5-tetrahydro-1 ,3-benzodiazepin-3-yl)-piperidin-1-yl]-1-(3-perhydro-azepin-1-yl-azetidin-1-yl)-butan-1,4-dione,
(52) (*S*)-2-(4-amino-3-chloro-5-trifluoromethyl-benzyl)-1-[4-(1-benzyl-piperidin-4-yl)-piperazin-1-yl]-4-[4-(2-oxo-1,2,4,5-tetrahydro-1,3-benzodiazepin-3-yl)-piperidin-1-yl]-butan-1,4-dione,
(53) (*S*)-2-(4-amino-3-chloro-5-trifluoromethyl-benzyl)-1-[4-(4-benzyl-piperazin-1-yl)-piperidin-1-yl]-4-[4-(2-oxo-1,2,4,5-tetrahydro-1,3-benzodiazepin-3-yl)-piperidin-1-yl]-butan-1,4-dione,
(54) (*S*)-2-(4-amino-3-chloro-5-trifluoromethyl-benzyl)-1-(7-dimethylaminomethyl-1,2,4,5-tetrahydro-3-benzazepin-3-yl)-4-[4-(2-oxo-1,2,4,5-tetrahydro-1,3-benzodiazepin-3-yl)-piperidin-1-yl]-butan-1,4-dione,
(55) (*S*)-2-(4-amino-3-chloro-5-trifluoromethyl-benzyl)-1-[4-(4-dimethylaminomethyl-phenyl)-piperidin-1-yl]-4-[4-(2-oxo-1,2,4,5-tetrahydro-1,3-benzodiazepin-3-yl)-piperidin-1-yl]-butan-1,4-dione,
(56) (*S*)-2-(4-amino-3-chloro-5-trifluoromethyl-benzyl)-4-[4-(2-oxo-1,2,4,5-tetrahydro-1,3-benzodiazepin-3-yl)-piperidin-1-yl]-1-{4-[4-(2,2,2-trifluoro-ethyl)-piperazin-1-yl]-piperid in-1-yl}-butan-1,4-dione,
(57) (*S*)-2-(4-amino-3-chloro-5-trifluoromethyl-benzyl)-1-(1'-methanesulphonyl-4,4'-bipiperidinyl-1-yl)-4-[4-(2-oxo-1,2,4,5-tetrahydro-1,3-benzodiazepin-3-yl)-piperidin-1-yl]-butan-1,4-dione,
(58) (*S*)-2-(4-amino-3-chloro-5-trifluoromethyl-benzyl)-1-(9-methyl-3,9-diaza-spiro[5.5]undec-3-yl)-4-[4-(2-oxo-1,2,4,5-tetrahydro-1,3-benzodiazepin-3-yl)-piperidin-1-yl]-butan-1,4-dione,
(59) (*S*)-2-(4-amino-3-chloro-5-trifluoromethyl-benzyl)-4-[4-(2-oxo-1,2,4,5-tetrahydro-1,3-benzodiazepin-3-yl)-piperidin-1-yl]-1-(4-piperidin-1-yl-methyl-piperidin-1-yl)-butan-1,4-dione,
(60) (*S*)-2-(4-amino-3-chloro-5-trifluoromethyl-benzyl)-1-[4-(2-dimethylamino-ethyl)-piperidin-1-yl]-4-[4-(2-oxo-1,2,4,5-tetrahydro-1,3-benzodiazepin-3-yl)-piperidin-1-yl]-butan-1,4-dione,
(61) (*S*)-2-(4-amino-3-chloro-5-trifluoromethyl-benzyl)-N-methyl-N-[2-(1-methyl-piperidin-4-yl)-ethyl]-4-oxo-4-[4-(2-oxo-1,2,4,5-tetrahydro-1,3-benzodiazepin-3-yl)-piperidin-1-yl]-butyramide,
(62) (*S*)-2-(4-amino-3-chloro-5-trifluoromethyl-benzyl)-N-methyl-N-(1-methyl-piperidin-4-ylmethyl)-4-oxo-4-[4-(2-oxo-1 ,2,4,5-tetrahydro-1 ,3-benzodiazepin-3-yl)-piperidin-1-yl]-butyramide,
(63) (*S*)-2-(4-amino-3-chloro-5-trifluoromethyl-benzyl)-4-[4-(2-oxo-1,2,4,5-tetrahydro-1,3-benzodiazepin-3-yl)-piperidin-1-yl]-1-piperidin-1-yl-butan-1,4-dione,
(64) (*S*)-2-(4-amino-3-chloro-5-trifluoromethyl-benzyl)-4-[4-(2-oxo-1,2,4,5-tetrahydro-1,3-benzodiazepin-3-yl)-piperidin-1-yl]-1-(4-propyl-piperidin-1-yl)-butan-1,4-dione,
(65) (*S*)-2-(4-amino-3-chloro-5-trifluoromethyl-benzyl)-1-(4-benzyl-piperidin-1-yl)-4-[4-(2-oxo-1,2,4,5-tetrahydro-1,3-benzodiazepin-3-yl)-piperidin-1-yl]-butan-1,4-dione,
(66) (*S*)-2-(4-amino-3-chloro-5-trifluoromethyl-benzyl)-1-[4-(2-diethylaminoethyl)-piperidin-1-yl]-4-[4-(2-oxo-1,2,4,5-tetrahydro-1,3-benzodiazepin-3-yl)-piperidin-1-yl]-butan-1,4-dione,
(67) (*S*)-2-(4-amino-3-chloro-5-trifluoromethyl-benzyl)-1-(3-aza-spiro[5.5]-undec-3-yl)-4-[4-(2-oxo-1,2,4,5-tetrahydro-1,3-benzodiazepin-3-yl)-piperidin-1-yl]-butan-1,4-dione,
(68) *N*-(1-{(*S*)-2-(4-amino-3-chloro-5-trifluoromethyl-benzyl)-4-oxo-4-[4-(2-oxo-1,2,4,5-tetrahydro-1,3-benzodiazepin-3-yl)-piperidin-1-yl]-butyryl}-piperidin-4-yl)-N-methyl-methanesulphonamide,
(69) *N*-(1-{(*S*)-2-(4-amino-3-chloro-5-trifluoromethyl-benzyl)-4-oxo-4-[4-(2-oxo-1,2,4,5-tetrahydro-1,3-benzodiazepin-3-yl)-piperidin-1-yl]-butyryl}-piperidin-4-yl)-methanesulphonamide,
(70) (*S*)-2-(4-amino-3-chloro-5-trifluoromethyl-benzyl)-1-[4-(cyclopentylmethyl-amino)-piperidin-1-yl]-4-[4-(2-oxo-1,2,4,5-tetrahydro-1,3-benzodiazepin-3-yl)-piperidin-1-yl]-butan-1,4-dione,
(71) (*S*)-2-(4-amino-3-chloro-5-trifluoromethyl-benzyl)-1-(4-methyl-piperidin-1-yl)-4-[4-(2-oxo-1,2,4,5-tetrahydro-1,3-benzodiazepin-3-yl)-piperidin-1-yl]-butan-1,4-dione,
(72) methyl (1-{(*S*)-2-(4-amino-3-chloro-5-trifluoromethyl-benzyl)-4-oxo-4-[4-(2-oxo-1,2,4,5-tetrahydro-1,3-benzodiazepin-3-yl)-piperidin-1-yl]-butyryl}-piperidin-4-yl)-acetate,
(73) (*S*)-2-(4-amino-3-chloro-5-trifluoromethyl-benzyl)-1-(4-hydroxy-piperidin-1-yl)-4-[4-(2-oxo-1,2,4,5-tetrahydro-1,3-benzodiazepin-3-yl)-piperidin-1-yl]-butan-1,4-dione,
(74) (*S*)-2-(4-amino-3-chloro-5-trifluoromethyl-benzyl)-4-[4-(2-oxo-1,2,4,5-tetrahydro-1,3-benzodiazepin-3-yl)-piperidin-1-yl]-1-(4-trifluoromethyl-piperidin-1-yl)-butan-1,4-dione,
(75) (*S*)-2-(4-amino-3-chloro-5-trifluoromethyl-benzyl)-1-[4-(1,1-dioxo-1,6-isothiazolidin-2-yl)-piperidin-1-yl]-4-[4-(2-oxo-1,2,4,5-tetrahydro-1,3-benzodiazepin-3-yl)-piperidin-1-yl]-butan-1,4-dione,
(76) (*S*)-2-(4-amino-3-chloro-5-trifluoromethyl-benzyl)-1-[4-(2-oxo-perhydro-1,3-oxazin-3-yl)-piperidin-1-yl]-4-[4-(2-oxo-1,2,4,5-tetrahydro-1,3-benzodiazepin-3-yl)-piperidin-1-yl]-butan-1,4-dione,
(77) methyl 1-{(S)-2-(4-amino-3-chloro-5-trifluoromethyl-benzyl)-4-oxo-4-[4-(2-oxo-1,2,4,5-tetrahydro-1,3-benzodiazepin-3-yl)-piperidin-1-yl]-butyryl}-piperidine-4-carboxylate,
(78) (*S*)-2-(4-amino-3-chloro-5-trifluoromethyl-benzyl)-1-(4-cyclohexyl-piperidin-1-yl)-4-[4-(2-oxo-1,2,4,5-tetrahydro-1,3-benzodiazepin-3-yl)-piperidin-1-yl]-butan-1,4-dione,
(79) (*S*)-2-(4-amino-3-chloro-5-trifluoromethyl-benzyl)-1-(4-tert-butylamino-piperidin-1-yl)-4-[4-(2-oxo-1,2,4,5-tetrahydro-1,3-benzodiazepin-3-yl)-piperidin-1-yl]-butan-1,4-dione,
(80) (*S*)-2-(4-amino-3-chloro-5-trifluoromethyl-benzyl)-4-[4-(2-oxo-1,2,4,5-tetrahydro-1,3-benzodiazepin-3-yl)-piperidin-1-yl]-1-(4-phenyl-piperidin-1-yl)-butan-1,4-dione,
(81) (*S*)-2-(4-amino-3-chloro-5-trifluoromethyl-benzyl)-4-[4-(2-oxo-1,2,4,5-tetrahydro-1,3-benzodiazepin-3-yl)-piperidin-1-yl]-1-(4-p-tolyl-piperidin-1-yl)-butan-1,4-dione,
(82) 8-{(*S*)-2-(4-amino-3-chloro-5-trifluoromethyl-benzyl)-4-oxo-4-[4-(2-oxo-1,2,4,5-tetrahydro-1,3-benzodiazepin-3-yl)-piperidin-1-yl]-butyryl}-1,3,8-triaza-spiro[4.5]decan-2,4-dione,
(83) (*S*)-2-(4-amino-3-chloro-5-trifluoromethyl-benzyl)-1-[4-(2-oxo-imidazolidin-1-yl)-piperidin-1-yl]-4-[4-(2-oxo-1,2,4,5-tetrahydro-1,3-benzodiazepin-3-yl)-piperidin-1-yl]-butan-1,4-dione,
(84) (*S*)-2-(4-amino-3-chloro-5-trifluoromethyl-benzyl)-1-(4-amino-4-methyl-piperidin-1-yl)-4-[4-(2-oxo-1,2,4,5-tetrahydro-1,3-benzodiazepin-3-yl)-piperidin-1-yl]-butan-1,4-dione,
(85) (*S*)-2-(4-amino-3-chloro-5-trifluoromethyl-benzyl)-1-[4-(1-methyl-piperidin-4-yl)-piperazin-1-yl]-4-[4-(2-oxo-1,2,4,5-tetrahydro-1,3-benzodiazepin-3-yl)-piperidin-1-yl]-butan-1,4-dione,
(86) (*S*)-2-(4-amino-3-chloro-5-trifluoromethyl-benzyl)-1-(2-amino-4,5,7,8-tetrahydro-thiazolo[4,5-d]azepin-6-yl)-4-[4-(2-oxo-1,2,4,5-tetrahydro-1,3-benzodiazepin-3-yl)-piperidin-1-yl]-butan-1,4-dione,
(87) (*S*)-2-(4-amino-3-chloro-5-trifluoromethyl-benzyl)-1-[4-(2-methyl-imidazol-1-yl)-piperidin-1-yl]-4-[4-(2-oxo-1,2,4,5-tetrahydro-1,3-benzodiazepin-3-yl)-piperidin-1-yl]-butan-1,4-dione,
(88) (*S*)-2-(4-amino-3-chloro-5-trifluoromethyl-benzyl)-1-[4-(4-methyl-imidazol-1-yl)-piperidin-1-yl]-4-[4-(2-oxo-1,2,4,5-tetrahydro-1,3-benzodiazepin-3-yl)-piperidin-1-yl]-butan-1,4-dione,
(89) (*S*)-2-(4-amino-3-chloro-5-trifluoromethyl-benzyl)-4-[4-(2-oxo-1,2,4,5-tetrahydro-1,3-benzodiazepin-3-yl)-piperidin-1-yl]-1-(4-thiazol-2-yl-piperazin-1-yl)-butan-1,4-dione,
(90) (*S*)-2-(4-amino-3-chloro-5-trifluoromethyl-benzyl)-1-[4-(2,4-dimethyl-imidazol-1-yl)-piperidin-1-yl]-4-[4-(2-oxo-1,2,4,5-tetrahydro-1,3-benzodiazepin-3-yl)-piperidin-1-yl]-butan-1,4-dione,
(91) (*S*)-2-(4-amino-3-chloro-5-trifluoromethyl-benzyl)-1-(4-imidazol-1-yl-piperidin-1-yl)-4-[4-(2-oxo-1,2,4,5-tetrahydro-1,3-benzodiazepin-3-yl)-piperidin-1-yl]-butan-1,4-dione,
(92) (*S*)-2-(4-amino-3-chloro-5-trifluoromethyl-benzyl)-4-[4-(2-oxo-1,2,4,5-tetrahydro-1, 3-benzodiazepin-3-yl)-piperidin-1-yl]-1-(4-1,2,4-triazol-1-yl-piperidin-1-yl)-butan-1,4-dione,
(93) (*S*)-2-(4-amino-3-chloro-5-trifluoromethyl-benzyl)-1-[4-(1-aza-bicyclo[2.2.2]oct-3-yl)-piperazin-1-yl]-4-[4-(2-oxo-1,2,4,5-tetrahydro-1,3-benzodiazepin-3-yl)-piperidin-1-yl]-butan-1,4-dione,
(94) (*S*)-2-(4-amino-3-chloro-5-trifluoromethyl-benzyl)-4-[4-(2-oxo-1,2,4,5-tetrahydro-1,3-benzodiazepin-3-yl)-piperidin-1-yl]-1-piperazin-1-yl-butan-1,4-dione,
(95) 4-{(*S*)-2-(4-amino-3-chloro-5-trifluoromethyl-benzyl)-4-oxo-4-[4-(2-oxo-1,2,4,5-tetrahydro-1,3-benzodiazepin-3-yl)-piperidin-1-yl]-butyryl}-piperazin-1-sulphonic acid (1-methyl-piperidin-4-yl)-amide,
(96) (*S*)-2-(4-amino-3-chloro-5-trifluoromethyl-benzyl)-N-(5-amino-pentyl)-4-oxo-4-[4-(2-oxo-1,2,4,5-tetrahydro-1,3-benzodiazepin-3-yl)-piperidin-1-yl]-butyramide,
(97) (*S*)-2-(4-amino-3-chloro-5-trifluoromethyl-benzyl)-N-(3-aminomethyl-benzyl)-4-oxo-4-[4-(2-oxo-1,2,4,5-tetrahydro-1,3-benzodiazepin-3-yl)-piperidin-1-yl]-butyramide,
(98) 1-{(*S*)-2-(4-amino-3-chloro-5-trifluoromethyl-benzyl)-4-oxo-4-[4-(2-oxo-1,2,4,5-tetrahydro-1,3-benzodiazepin-3-yl)-piperidin-1-yl]-butyryl}-piperidin-4-carboxylic acid,
(99) (1-{(*S*)-2-(4-amino-3-chloro-5-trifluoromethyl-benzyl)-4-oxo-4-[4-(2-oxo-1,2,4,5-tetrahydro-1,3-benzodiazepin-3-yl)-piperidin-1-yl]-butyryl}-piperidin-4-yl)-acetic acid,
(100) (*S*)-2-(4-amino-3-chloro-5-trifluoromethyl-benzyl)-1-[4-(8-methyl-8-aza-bicyclo[3.2.1]oct-3-yl)-piperazin-1-yl]-4-[4-(2-oxo-1,2,4,5-tetrahydro-1,3-benzodiazepin-3-yl)-piperidin-1-yl]-butan-1,4-dione,
(101) (*S*)-2-(4-amino-3-chloro-5-trifluoromethyl-benzyl)-4-[4-(2-oxo-1,2,4,5-tetrahydro-1,3-benzodiazepin-3-yl)-piperidin-1-yl]-1-(4-piperazin-1-yl-piperidin-1-yl)-butan-1,4-dione,
(102) (*S*)-2-(4-amino-3-chloro-5-trifluoromethyl-benzyl)-1-[4-(1*H*-imidazol-4-yl)-piperidin-1-yl]-4-[4-(2-oxo-1,2,4,5-tetrahydro-1,3-benzodiazepin-3-yl)-piperidin-1-yl]-butan-1,4-dione,
(103) (*S*)-2-(4-amino-3-chloro-5-trifluoromethyl-benzyl)-4-[4-(2-oxo-1,2,4,5-tetrahydro-1,3-benzodiazepin-3-yl)-piperidin-1-yl]-1-{4-[4-(2,2,2-trifluoro-acetyl)-phenyl]-piperazin-1-yl}-butan-1,4-dione,
(104) (*S*)-2-(4-amino-3-chloro-5-trifluoromethyl-benzyl)-1-[4-(4-methyl-piperazin-1-yl)-piperidin-1-yl]-4-[4-(2-oxo-1,4-dihydro-2*H*-quinazolin-3-yl)-piperidin-1-yl]-butan-1,4-dione,
(105) (*S*)-2-(4-amino-3-chloro-5-trifluoromethyl-benzyl)-1-[4-(4-methyl-piperazin-1-yl)-piperidin-1-yl]-4-[4-(5-oxo-3-phenyl-4,5-dihydro-1,2,4-triazol-1-yl)-piperidin-1-yl]-butan-1,4-dione,
(106) (*S*)-2-(4-amino-3-chloro-5-trifluoromethyl-benzyl)-1-[4-(4-methyl-piperazin-1-yl)-piperidin-1-yl]-4-[4-(2-oxo-1,2-dihydro-imidazo[4,5-c]quinoline-3-yl)-piperidin-1-yl]-butan-1,4-dione,
(107) (*S*)-2-(4-amino-3-chloro-5-trifluoromethyl-benzyl)-1-[4-(4-methyl-piperazin-1-yl)-piperidin-1-yl]-4-[4-(2-oxo-1,2-dihydro-4*H*-thieno[3,4-d]pyrimidin-3-yl)-piperidin-1-yl]-butan-1,4-dione,
(108) (*S*)-2-(4-amino-3-chloro-5-trifluoromethyl-benzyl)-1-[4-(5-methyl-2,5-diaza-bicyclo[2.2.1]hept-2-yl)-piperidin-1-yl]-4-[4-(2-oxo-1,2,4,5-tetrahydro-1,3-benzodiazepin-3-yl)-piperidin-1-yl]-butan-1,4-dione,
(109) (*S*)-2-(4-amino-3,5-bis-trifluoromethyl-benzyl)-1-[4-(4-methyl-perhydro-1,4-diazepin-1-yl)-piperidin-1-yl]-4-[4-(2-oxo-1,2,4,5-tetrahydro-1,3-benzodiazepin-3-yl)-piperidin-1-yl]-butan-1,4-dione,
(110) (*S*)-2-(4-amino-3,5-bis-trifluoromethyl-benzyl)-1-[4-(1-methyl-piperidin-4-yl)-perhydro-1,4-diazepin-1-yl]-4-[4-(2-oxo-1,2,4,5-tetrahydro-1,3-benzodiazepin-3-yl)-piperidin-1-yl]-butan-1,4-dione,
(111) (*S*)-2-(4-amino-3,5-bis-trifluoromethyl-benzyl)-1-[4-(1-methyl-piperidin-4-yl)-piperazin-1-yl]-4-[4-(2-oxo-1,2,4,5-tetrahydro-1,3-benzodiazepin-3-yl)-piperidin-1-yl]-butan-1,4-dione,
(112) (*S*)-2-(4-amino-3,5-bis-trifluoromethyl-benzyl)-1-[4-(1-aza-bicyclo[2.2.2]oct-3-yl)-piperazin-1-yl]-4-[4-(2-oxo-1,2,4,5-tetrahydro-1,3-benzodiazepin-3-yl)-piperidin-1-yl]-butan-1,4-dione,
(113) (*S*)-2-(4-amino-3,5-bis-trifluoromethyl-benzyl)-1-[4-(4-methyl-piperazin-1-yl)-piperidin-1-yl]-4-[4-(2-oxo-1,2,4,5-tetrahydro-1,3-benzodiazepin-3-yl)-piperidin-1-yl]-butan-1,4-dione,
(114) (*S*)-2-(4-amino-3,5-bis-trifluoromethyl-benzyl)-4-[4-(2-oxo-1,2,4,5-tetrahydro-1,3-benzodiazepin-3-yl)-piperidin-1-yl]-1-(4-perhydro-azepin-1-yl-piperidin-1-yl)-butan-1,4-dione,
(115) (*S*)-2-(4-amino-3,5-bis-trifluoromethyl-benzyl)-1-1,4'-bipiperidinyl-1'-yl-4-[4-(2-oxo-1,2,4,5-tetrahydro-1,3-benzodiazepin-3-yl)-piperidin-1-yl]-butan-1,4-dione,
(116) (*S*)-2-(4-amino-3,5-bis-trifluoromethyl-benzyl)-4-[4-(2-oxo-1,2,4,5-tetrahydro-1,3-benzodiazepin-3-yl)-piperidin-1-yl]-1-(4-piperazin-1-yl-piperidin-1-yl)-butan-1,4-dione,
(117) 4-(2-oxo-1,2,4,5-tetrahydro-1,3-benzodiazepin-3-yl)-piperidin-1-carboxylic acid-{(*R*)-1-(4-amino-3,5-bis-trifluoromethyl-benzyl)-2-[4-(4-methyl-piperazin-1-yl)-piperidin-1-yl]-2-oxo-ethyl}-amide,
(118) 4-(2-oxo-1,2,4,5-tetrahydro-1,3-benzodiazepin-3-yl)-piperidin-1-carboxylic acid-{(*R*)-1-(4-amino-3,5-bis-trifluoromethyl-benzyl)-2-[4-(1-methyl-piperidin-4-yl)-piperazin-1-yl]-2-oxo-ethyl}-amide,
(119) 4-(2-oxo-1,2,4,5-tetrahydro-1,3-benzodiazepin-3-yl)-piperidin-1-carboxylic acid-[(*R*)-1-(4-amino-3,5-bis-trifluoromethyl-benzyl)-2-1,4'-bipiperidinyl-1 '-yl-2-oxo-ethyl]-amide,
(120) 4-(2-oxo-1,2,4,5-tetrahydro-1,3-benzodiazepin-3-yl)-pipehdin-1-carboxylic acid-[(*R*)-1-(4-amino-3,5-bis-trifluoromethyl-benzyl)-2-oxo-2-(4-piperazin-1-yl-piperidin-1-yl)-ethyl]-amide,
(121) (R)-1-(4-amino-3-chloro-5-trifluoromethyl-benzyl)-2-[4-(4-methyl-piperazin-1-yl)-piperidin-1-yl]-2-oxo-ethyl 4-(2-oxo-1,2,4,5-tetrahydro-1,3-benzodiazepin-3-yl)-piperidin-1-carboxylate,
(122) 4-(2-oxo-1,4-dihydro-2*H*-quinazolin-3-yl)-piperidin-1-carboxylic acid [2-[1,4']bipiperidinyl-1'-yl-1-(4-bromo-3-methyl-benzyl)-2-oxo-ethyl]-amide,
(123) 4-(2-oxo-1,4-dihydro-2*H*-quinazolin-3-yl)-piperidin-1-carboxylic acid-[1-(4-bromo-3-methyl-benzyl)-2-(1'-methyl-[4,4']bipiperidinyl-1-yl)-2-oxoethyl]-amide,
(124) 4-(2-oxo-1,4-dihydro-2*H*-quinazolin-3-yl)-piperidin-1-carboxylic acid-{1-(4-bromo-3-methyl-benzyl)-2-[4-(4-methyl-piperazin-1-yl)-piperidin-1-yl]-2-oxo-ethyl}-amide,
(125) 4-(2-oxo-1,4-dihydro-2*H*-quinazolin-3-yl)-piperidin-1-carboxylic acid-{1-(4-bromo-3-methyl-benzyl)-2-[4-(1-methyl-piperidin-4-yl)-piperazin-1-yl]-2-oxo-ethyl}-amide,
(126) ethyl {4-[1-(3-(4-bromo-3-methyl-phenyl)-2-{[4-(2-oxo-1,4-dihydro-2*H-*quinazolin-3-yl)-piperidin-1-carbonyl]-amino}-propionyl)-piperidin-4-yl]-piperazin-1-yl}-acetate,
(127) ethyl [1'-(3-(4-bromo-3-methyl-phenyl)-2-{[4-(2-oxo-1,4-dihydro-2*H-*quinazolin-3-yl)-piperidin-1-carbonyl]-amino}-propionyl)-[4,4']bipiperidinyl-1-yl]-acetate,
(128) ethyl {4-[4-(3-(4-bromo-3-methyl-phenyl)-2-{[4-(2-oxo-1,4-dihydro-2*H-*quinazolin-3-yl)-piperidin-1-carbonyl]-amino}-propionyl)-piperazin-1-yl]-piperidin-1-yl}-acetate,
(129) {4-[1-(3-(4-bromo-3-methyl-phenyl)-2-{[4-(2-oxo-1,4-dihydro-2*H-*quinazolin-3-yl)-piperidin-1-carbonyl]-amino}-propionyl)-piperidin-4-yl]-piperazin-1-yl}-acetic acid,
(130) [1'-(3-(4-bromo-3-methyl-phenyl)-2-{[4-(2-oxo-1,4-dihydro-2*H-*quinazolin-3-yl)-piperidin-1-carbonyl]-amino}-propionyl)-[4,4']bipiperidinyl-1-yl]-acetic acid,
(131) {4-[4-(3-(4-bromo-3-methyl-phenyl)-2-{[4-(2-oxo-1,4-dihydro-2*H-*quinazolin-3-yl)-piperidin-1-carbonyl]-amino}-propionyl)-piperazin-1-yl]-piperidin-1-yl}-acetic acid,
(132) 2-(4-bromo-3-methyl-benzyl)-1-[4-(4-methyl-piperazin-1-yl)-piperidin-1-yl]-4-[4-(2-oxo-1,4-dihydro-2*H*-quinazolin-3-yl)-piperidin-1-yl]-butan-1,4-dione,
(133) 2-(4-bromo-3-methyl-benzyl)-1-(1'-methyl-[4,4']bipiperidinyl-1-yl)-4-[4-(2-oxo-1,4-dihydro-2*H*-quinazolin-3-yl)-piperidin-1-yl]-butan-1,4-dione,
(134) 1-[1,4']Bipiperidinyt-1'-yl-2-(4-bromo-3-methyl-benzyl)-4-[4-(2-oxo-1,4-dihydro-2*H*-quinazolin-3-yl)-piperidin-1-yl]-butan-1,4-dione,
(135) 2-(4-bromo-3-methyl-benzyl)-1-{4-[4-(3-dimethylamino-propyl)-phenyl]-piperazin-1-yl}-4-[4-(2-oxo-1,4-dihydro-2*H*-quinazolin-3-yl)-piperidin-1-yl]-butan-1,4-dione,
(136) [4-(1-{2-(4-bromo-3-methyl-benzyl)-4-oxo-4-[4-(2-oxo-1,4-dihydro-2*H-*quinazolin-3-yl)-piperidin-1-yl]-butyryl}-piperidin-4-yl)-piperazin-1-yl]-acetic acid,
(137) methyl (1'-{2-(4-bromo-3-methyl-benzyl)-4-oxo-4-[4-(2-oxo-1,4-dihydro-2*H*-quinazolin-3-yl)-piperidin-1-yl]-butyryl}-[4,4']bipiperidinyl-1-yl)-acetate,
(138) (1'-{2-(4-bromo-3-methyl-benzyl)-4-oxo-4-[4-(2-oxo-1,4-dihydro-2*H-*quinazolin-3-yl)-piperidin-1-yl]-butyryl}-[4,4']bipiperidinyl-1-yl)-acetic acid,
(139) 4-(2-oxo-1,4-dihydro-2H-quinazolin-3-yl)-piperidin-1-carboxylic acid{1-(4-chloro-3-methyl-benzyl)-2-[4-(4-methyl-piperazin-1-yl)-piperidin-1-yl]-2-oxo-ethyl}-amide,
(140) 4-(2-oxo-1,4-dihydro-2*H*-quinazolin-3-yl)-piperidin-1-carboxylic acid-[2-[1,4']bipiperidinyl-1'-yl-1-(4-chloro-3-methyl-benzyl)-2-oxo-ethyl]-amide,
(141) 4-(2-oxo-1,4-dihydro-2*H*-quinazolin-3-yl)-piperidin-1-carboxylic acid-{1-(4-chloro-3-methyl-benzyl)-2-[4-(1-methyl-piperidin-4-yl)-piperazin-1-yl]-2-oxo-ethyl}-amide,
(142) 4-(2-oxo-1,4-dihydro-2*H*-quinazolin-3-yl)-piperidin-1-carboxylic acid-[1-(4-chloro-3-methyl-benzyl)-2-(1'-methyl-[4,4']bipiperidinyl-1-yl)-2-oxoethyl]-amide,
(143) ethyl [1'-(3-(4-chloro-3-methyl-phenyl)-2-{[4-(2-oxo-1,4-dihydro-2*H-*quinazolin-3-yl)-piperidin-1-carbonyl]-amino}-propionyl)-[4,4']bipiperidinyl-1-yl]-acetate,
(144) *tert*-butyl{4-[1-(3-(4-chloro-3-methyl-phenyl)-2-{[4-(2-oxo-1,4-dihydro-2H-quinazolin-3-yl)-piperidin-1-carbonyl]-amino}-propionyl)-piperidin-4-yl]-piperazin-1-yl}-acetate,
(145) 4-(2-oxo-1,4-dihydro-2*H*-quinazolin-3-yl)-piperidin-1-carboxylic acid-{1-(4-chloro-3-methyl-benzyl)-2-oxo-2-[1'-(2,2,2-trifluoro-ethyl)-[4,4']bipiperidinyl-1-yl]-ethyl}-amide,
(146) 4-(2-oxo-1,4-dihydro-2*H*-quinazolin-3-yl)-piperidin-1-carboxylic acid-(1-(4-chloro-3-methyl-benzyl)-2-oxo-2-{4-[4-(2,2,2-trifluoro-ethyl)-piperazin-1-yl]-piperidin-1-yl}-ethyl)-amide,
(147) [1'-(3-(4-chloro-3-methyl-phenyl)-2-{[4-(2-oxo-1,4-dihydro-2*H-*quinazolin-3-yl)-piperidin-1-carbonyl]-amino}-propionyl)-[4,4']bipiperidinyl-1-yl]-acetic acid,
(148) 2-(4-chloro-3-methyl-benzyl)-1-[4-(4-ethyl-piperazin-1-yl)-piperidin-1-yl]-4-[4-(2-oxo-1,4-dihydro-2*H*-quinazolin-3-yl)-piperidin-1-yl]-butan-1,4-dione,
(149) 2-(4-chloro-3-methyl-benzyl)-1-(1'-methyl-[4,4']bipiperidinyl-1-yl)-4-[4-(2-oxo-1,4-dihydro-2*H*-quinazolin-3-yl)-piperidin-1-yl]-butan-1,4-dione,
(150) 1-[1,4']Bipiperidinyl-1'-yl-2-(4-chloro-3-methyl-benzyl)-4-[4-(2-oxo-1,4-dihydro-2*H*-quinazolin-3-yl)-piperidin-1-yl]-butan-1,4-dione,
(151) 2-(4-chloro-3-methyl-benzyl)-1-[4-(4-methyl-piperazin-1-yl)-piperidin-1-yl]-4-[4-(2-oxo-1,4-dihydro-2*H*-quinazolin-3-yl)-piperidin-1-yl]-butan-1,4-dione,
(152) 2-(4-chloro-3-methyl-benzyl)-1-[4-(1-methyl-piperidin-4-yl)-piperazin-1-yl]-4-[4-(2-oxo-1,4-dihydro-2*H*-quinazolin-3-yl)-piperidin-1-yl]-butan-1,4-dione,
(153) 2-(4-chloro-3-methyl-benzyl)-1-[4-(4-methanesulphonyl-piperazin-1-yl)-piperidin-1-yl]-4-[4-(2-oxo-1,4-dihydro-2*H*-quinazolin-3-yl)-piperidin-1-yl]-butan-1,4-dione,
(154) 2-(4-chloro-3-methyl-benzyl)-1-[4-(4-isopropyl-piperazin-1-yl)-piperidin-1-yl]-4-[4-(2-oxo-1,4-dihydro-2*H*-quinazolin-3-yl)-piperidin-1-yl]-butan-1,4-dione,
(155) ethyl 1-{2-(4-chloro-3-methyl-benzyl)-4-oxo-4-[4-(2-oxo-1,4-dihydro-2*H*-quinazolin-3-yl)-piperidin-1-yl]-butyryl}-4-(1-methyl-piperidin-4-yl)-piperazin-2-carboxylate,
(156) ethyl 1-(1-{2-(4-chloro-3-methyl-benzyl)-4-oxo-4-[4-(2-oxo-1,4-dihydro-2*H*-quinazolin-3-yl)-piperidin-1-yl]-butyryl}-piperidin-4-yl)-4-methyl-piperazin-2-carboxylate,
(157) ethyl 4-(1-{2-(4-chloro-3-methyl-benzyl)-4-oxo-4-[4-(2-oxo-1,4-dihydro-2*H*-quinazolin-3-yl)-piperidin-1-yl]-butyryl}-piperidin-4-yl)-1-methyl-piperazin-2-carboxylate,
(158) ethyl 4-{2-(4-chloro-3-methyl-benzyl)-4-oxo-4-[4-(2-oxo-1,4-dihydro-2*H*-quinazolin-3-yl)-piperidin-1-yl]-butyryl}-1-(1-methyl-piperidin-4-yl)-piperazin-2-carboxylate,
(159) 2-(4-chloro-3-methyl-benzyl)-4-[4-(2-oxo-1,4-dihydro-2*H*-quinazolin-3-yl)-piperidin-1-yl]-1-[1'-(2,2,2-triftuoro-ethyl)-[4,4']bipiperidinyl-1-yl]-butan-1,4-dione,
(160) 2-(4-chloro-3-methyl-benzyl)-4-[4-(2-oxo-1,4-dihydro-2*H*-quinazolin-3-yl)-piperidin-1-yl]-1-{4-[4-(2,2,2-trifluoro-ethyl)-piperazin-1-yl]-piperid in-1-yl}-butan-1,4-dione,
(161) [4-(1-{2-(4-chloro-3-methyl-benzyl)-4-oxo-4-[4-(2-oxo-1,4-dihydro-2H-quinazolin-3-yl)-piperidin-1-yl]-butyryl}-piperidin-4-yl)-piperazin-1-yl]-acetic acid,
(162) methyl (1'-{2-(4-chloro-3-methyl-benzyl)-4-oxo-4-[4-(2-oxo-1,4-dihydro-2*H*-quinazolin-3-yl)-piperidin-1-yl]-butyryl}-[4,4']bipiperidinyl-1-yl)-acetate,
(163) (1'-{2-(4-chloro-3-methyl-benzyl)-4-oxo-4-[4-(2-oxo-1,4-dihydro-2*H-*quinazolin-3-yl)-piperidin-1-yl]-butyryl}-[4,4']bipiperid inyl-1-yl)-acetic acid,
(164) 1-{2-(4-chloro-3-methyl-benzyl)-4-oxo-4-[4-(2-oxo-1,4-dihydro-2*H-*quinazolin-3-yl)-piperidin-1-yl]-butyryl}-4-(1-methyl-piperidin-4-yl)-piperazine-2-carboxylic acid,
(165) 1-(1-{2-(4-chloro-3-methyl-benzyl)-4-oxo-4-[4-(2-oxo-1,4-dihydro-2*H-*quinazolin-3-yl)-piperidin-1-yl]-butyryl}-piperidin-4-yl)-4-methylpiperazine-2-carboxylic acid,
(166) 4-(1-{2-(4-chloro-3-methyl-benzyl)-4-oxo-4-[4-(2-oxo-1,4-dihydro-2*H-*quinazolin-3-yl)-piperidin-1-yl]-butyryl}-piperidin-4-yl)-1-methylpiperazine-2-carboxylic acid,
(167) 2-(4-chloro-3-methyl-benzyl)-1-[4-(1-methyl-piperidin-4-yl)-piperazin-1-yl]-4-[4-(2-oxo-1,2,4,5-tetrahydro-1,3-benzodiazepin-3-yl)-piperidin-1-yl]-butan-1,4-dione,
(168) 2-(4-chloro-3-methyl-benzyl)-1-[4-(4-methyl-piperazin-1-yl)-piperidin-1-yl]-4-[4-(2-oxo-1,2,4,5-tetrahydro-1,3-benzodiazepin-3-yl)-piperidin-1-yl]-butan-1,4-dione,
(169) [4-(1-{2-(4-chloro-3-methyl-benzyl)-4-oxo-4-[4-(5-oxo-3-phenyl-4,5-dihydro-1,2,4-triazol-1-yl)-piperidin-1-yl]-butyryl}-piperidin-4-yl)-piperazin-1-yl]-acetic acid,
(170) methyl (1'-{2-(4-chloro-3-methyl-benzyl)-4-oxo-4-[4-(5-oxo-3-phenyl-4,5-dihydro-1,2,4-triazol-1-yl)-piperidin-1-yl]-butyryl}-4,4'-bipiperidinyl-1-yl)-acetate,
(171) (1'-{2-(4-chloro-3-methyl-benzyl)-4-oxo-4-[4-(5-oxo-3-phenyl-4,5-dihydro-1,2,4-triazol-1-yl)-piperidin-1-yl]-butyryl}-4,4'-bipiperidinyl-1-yl)-acetic acid,
(172) 2-(3-bromo-4-chloro-5-methyl-benzyl)-1-[4-(4-methyl-piperazin-1-yl)-piperidin-1-yl]-4-[4-(2-oxo-1,4-dihydro-2*H*-quinazolin-3-yl)-piperidin-1-yl]-butan-1,4-dione,
(173) 2-(3-bromo-4-chloro-5-methyl-benzyl)-1-(1'-methyl-[4,4']bipiperidinyl-1-yl)-4-[4-(2-oxo-1,4-dihydro-2*H*-quinazolin-3-yl)-piperidin-1-yl]-butan-1,4-dione,
(174) 2-(3-bromo-4-chloro-5-methyl-benzyl)-4-[4-(2-oxo-1,4-dihydro-2*H-*quinazolin-3-yl)-piperid in-1-yl]-1-(4-pyridin-4-yl-piperazin-1-yl)-butan-1,4-dione,
(175) 2-(3-bromo-4-chloro-5-methyl-benzyl)-1-[4-(1-methyl-piperidin-4-yl)-piperazin-1-yl]-4-[4-(2-oxo-1,4-dihydro-2*H*-quinazolin-3-yl)-piperidin-1-yl]-butan-1,4-dione,
(176) [4-(1-{2-(3-bromo-4-chloro-5-methyl-benzyl)-4-oxo-4-[4-(2-oxo-1,4-dihydro-2*H*-quinazolin-3-yl)-piperidin-1-yl]-butyryl}-piperidin-4-yl)-piperazin-1-yl]-acetic acid,
(177) methyl (1'-{2-(3-bromo-4-chloro-5-methyl-benzyl)-4-oxo-4-[4-(2-oxo-1,4-dihydro-2*H*-quinazolin-3-yl)-piperidin-1-yl]-butyryl}-[4,4']bipiperidinyl-1-yl)-acetate,
(178) (1'-{2-(3-bromo-4-chloro-5-methyl-benzyl)-4-oxo-4-[4-(2-oxo-1,4-dihydro-2*H*-quinazolin-3-yl)-piperidin-1-yl]-butyryl}-[4,4']bipiperidinyl-1-yl)-acetic acid,
(179) 4-(2-oxo-1,4-dihydro-2*H*-quinazolin-3-yl)-piperidin-1-carboxylic acid{1-(3-bromo-4-chloro-5-methyl-benzyl)-2-[4-(4-methyl-piperazin-1-yl)-piperidin-1-yl]-2-oxo-ethyl}-amide,
(180) 4-(2-oxo-1,4-dihydro-2*H*-quinazotin-3-yl)-piperidin-1-carboxylic acid-[2-[1,4']bipiperidinyl-1'-yl-1-(3-bromo-4-chloro-5-methyl-benzyl)-2-oxoethyl]-amide,
(181) 4-(2-oxo-1,4-dihydro-2*H*-quinazolin-3-yl)-piperidin-1-carboxylic acid-{1-(3-bromo-4-chloro-5-methyl-benzyl)-2-[4-(1-methyl-piperidin-4-yl)-piperazin-1-yl]-2-oxo-ethyl}-amide,
(182) 4-(2-oxo-1,4-dihydro-2*H*-quinazolin-3-yl)-piperidin-1-carboxylic acid-[1-(3-bromo-4-chloro-5-methyl-benzyl)-2-(1'-methyl-[4,4']bipiperidinyl-1-yl)-2-oxo-ethyl]-amide,
(183) 4-(2-oxo-1,4-dihydro-2*H*-quinazolin-3-yl)-piperidin-1-carboxylic acid-[1-(3-bromo-4-chloro-5-methyl-benzyl)-2-oxo-2-(4-pyridin-4-yl-piperazin-1-yl)-ethyl]-amide,
(184) 2-(4-chloro-3-trifluoromethyl-benzyl)-1-(1'-methyl-[4,4']bipiperidinyl-1-yl)-4-[4-(2-oxo-1,4-dihydro-2*H*-quinazolin-3-yl)-piperidin-1-yl]-butan-1,4-dione,
(185) 2-(4-chloro-3-trifluoromethyl-benzyl)-1-[4-(4-methyl-piperazin-1-yl)-piperidin-1-yl]-4-[4-(2-oxo-1,4-dihydro-2*H*-quinazolin-3-yl)-piperidin-1-yl]-butan-1,4-dione,
(186) 1-[1,4']Bipiperidinyl-1'-yl-2-(4-chloro-3-trifluoromethyl-benzyl)-4-[4-(2-oxo-1,4-dihydro-2*H*-quinazolin-3-yl)-piperidin-1-yl]-butan-1,4-dione,
(187) [4-(1-{2-(4-chloro-3-trifluoromethyl-benzyl)-4-oxo-4-[4-(2-oxo-1,4-dihydro-2*H*-quinazolin-3-yl)-piperidin-1-yl]-butyryl}-piperidin-4-yl)-piperazin-1-yl]-acetic acid,
(188) methyl (1'-{2-(4-chloro-3-trifluoromethyl-benzyl)-4-oxo-4-[4-(2-oxo-1,4-dihydro-2*H*-quinazolin-3-yl)-piperidin-1-yl]-butyryl}-[4,4']bipiperidinyl-1-yl)-acetate,
(189) (1'-{2-(4-chloro-3-trifluoromethyl-benzyl)-4-oxo-4-[4-(2-oxo-1,4-dihydro-2*H*-quinazolin-3-yl)-piperidin-1-yl]-butyryl}-[4,4']bipiperidinyl-1-yl)-acetic acid,
(190) 2-(4-chloro-3-trifluoromethyl-benzyl)-1-(1'-methyl-[4,4']bipiperidinyl-1-yl)-4-[4-(2-oxo-1,4-dihydro-2*H*-quinazolin-3-yl)-piperidin-1-yl]-butan-1,4-dione,
the enantiomers, the diastereomers and the salts thereof.

10. Physiologically acceptable salts of the compounds according to at least one of claims 1 to 9 with inorganic or organic acids or bases inorganic or organic acids or bases.

11. Pharmaceutical compositions containing a compound according to at least one of claims 1 to 9 or a physiologically acceptable salt according to claim 10 optionally together with one or more inert carriers and/or diluents.

12. Use of a compound according to at least one of claims 1 to 10 for preparing a pharmaceutical composition for the acute and prophylactic treatment of headaches, particularly migraine or cluster headaches, tension headache and chronic headache.

13. Use of a compound according to at least one of claims 1 to 10 for preparing a pharmaceutical composition for the prophylactic treatment of migraine headache during the prodrome period or for the acute and prophylactic treatment of migraine headache which occurs prior to or during menstruation.

14. Use of a compound according to at least one of claims 1 to 10 for preparing a pharmaceutical composition for combating non-insulin-dependent diabetes mellitus (NIDDM).

15. Use of a compound according to at least one of claims 1 to 10 for preparing a pharmaceutical composition for the treatment of cardiovascular diseases, morphine tolerance, diarrhoea caused by clostridium toxin, skin diseases, particularly thermal and radiation-induced skin damage including sunburn, inflammatory diseases, e.g. particularly inflammatory diseases of the joints such as arthritis, neurogenic inflammation of the oral mucosa, inflammatory lung diseases, allergic rhinitis, asthma, diseases accompanied by excessive vasodilatation and resultant reduced circulation of the blood, e.g. particularly shock and sepsis or erythema, for alleviating pain in general, particularly in cases of neuropathic pain, in neuropathic pain within the framework of systemic neurotoxic diseases and in pain caused by inflammatory processes, or for preventive or acute therapeutic treatment of the symptoms of hot flushes caused by vasodilatation and increased blood flow in menopausal oestrogen-deficient women and hormone-treated patients with prostate carcinoma.

16. Process for preparing a pharmaceutical composition according to claim 11, **characterised in that** a compound according to at least one of claims 1 to 10 is incorporated in one or more inert carriers and/or diluents by a non-chemical method.

17. Process for preparing the compounds of general formula (I) according to at least one of claims 1 to 9, **characterised in that**
(a) in order to prepare compounds of general formula (I) wherein X denotes the oxygen atom or the NH group and R¹ to R³ are defined as in claim 1, with the proviso that these groups do not contain any free carboxylic acid function:
reacting piperidines of general formula 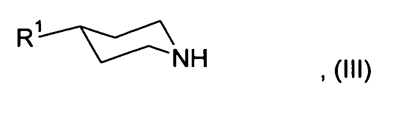 wherein R¹ is defined as in claim 1,
(i) with carbonic acid derivatives of general formula 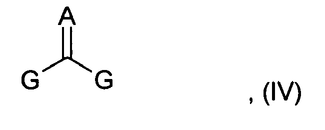 wherein A is defined as in claim 1 and G denotes a nucleofugic group, with the proviso that X denotes the NH- group, or
(ii) with carbonic acid derivatives of general formula 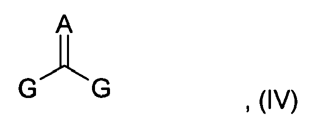 wherein A denotes the oxygen atom and G denotes a nucleofugic group, which may be identical or different, with the proviso that X denotes the oxygen atom,
and with compounds of general formula 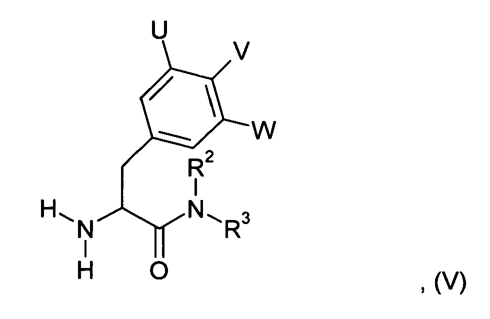 wherein X denotes an oxygen atom or an -NH group and U, V, W, R² and R³ are defined as in claim 1, with the proviso that R² and R³ do not contain any free carboxylic acid and/or any other free primary or secondary aliphatic amino function or other free hydroxy function, or
(b) In order to prepare compounds of general formula (I) wherein X denotes the methylene group and R¹ to R³ are defined as in claim 1, with the proviso that these groups do not contain any free carboxylic acid and/or other free primary or secondary aliphatic amino function:
coupling a carboxylic acid of general formula 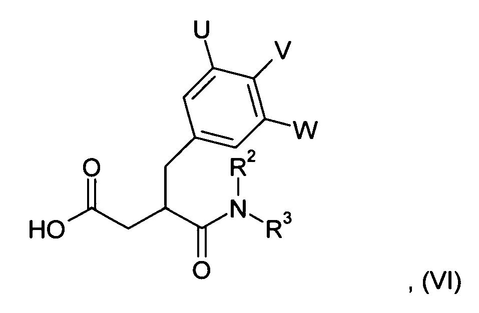
wherein U, V, W, R² and R³ are defined as in claim 1, to a piperidine of general formula 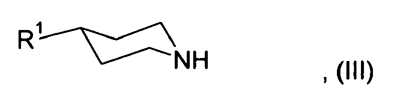 wherein R¹ is defined as in claim 1, or
(c) in order to prepare compounds of general formula (I) wherein X denotes the methylene group and R² and R³ are defined as in claim 1, with the proviso that these groups do not contain any free primary or secondary amine:
coupling a compound of general formula 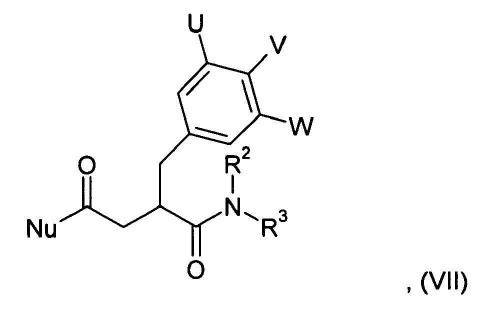
wherein U, V, W, R² and R³ are defined as in claim 1, with the proviso that R² and R³ do not contain any free primary or secondary amine, and Nu denotes a leaving group,
with a piperidine of general formula 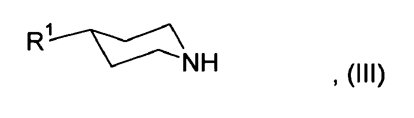 wherein R¹ is defined as in claim 1, or
(d) in order to prepare compounds of general formula (I) wherein all the groups are defined as in claim 1:
coupling a carboxylic acid of general formula 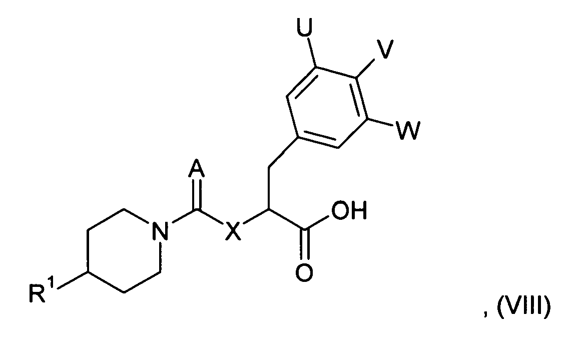
wherein all the groups are defined as in claim 1, with an amine of general formula HNR²R³, wherein R² and R³ are defined as in claim 1, with the proviso that these groups do not contain any free carboxylic acid and/or other free primary or secondary aliphatic amino function, or
(e) in order to prepare compounds of general formula (I) wherein R¹ is defined as in claim 1, with the proviso that no free primary or secondary amine is present:
coupling a compound of general formula 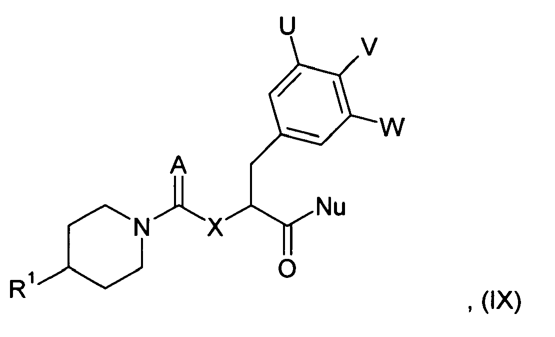 wherein all the groups are defined as in claim 1 and Nu denotes a leaving group,
with an amine of general formula HNR²R³, wherein R² and R³ are defined as in claim 1, with the proviso that these groups do not contain any free carboxylic acid and/or other free primary or secondary aliphatic amino function, and
if necessary any protecting group used in the reactions described above is cleaved again and/or
any precursor functions used in a compound thus obtained are converted and/or
if desired a compound of general formula (I) thus obtained is resolved into the stereoisomers thereof and / or
a compound of general formula (I) thus obtained is converted into the salts thereof, particularly for pharmaceutical use into the physiologically acceptable salts thereof.

## Revendications

1. Agents antagonistes CGRP de formule générale : 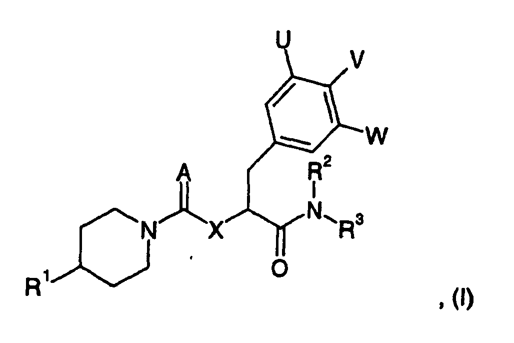 dans laquelle
A désigne un atome d'oxygène ou de soufre, un groupe phénylsulfonylimino- ou cyanimino,
X désigne un atome d'oxygène ou de soufre, un groupe imino substitué, le cas échéant, par un groupe alkyle en C₁₋₆ ou un groupe méthylène substitué, le cas échéant, par un groupe alkyle en C₁₋₆,
U désigne un groupe alkyle en C₁₋₆, alcényle en C₂₋₆ ou alcynyle en C₂₋₆, dans lequel chaque groupe méthylène peut être substitué par jusqu'à 2 atomes de fluor et chaque groupe méthyle peut être substitué par jusqu'à 3 atomes de fluor,
V désigne un atome de chlore ou de brome, un groupe amino, méthylamino ou hydroxy,
W désigne un atome d'hydrogène, de fluor, de chlore, de brome ou d'iode, un groupe difluoro- ou trifluorométhyle,
R¹ désigne un hétérocycle aza-, diaza-, triaza-, oxaza-, thiaza-, thiadiaza- ou S,S-dioxydo- thiadiaza saturé, simplement ou doublement insaturé ayant de 5 à 7 chaînons,
les hétérocycles susmentionnés étant reliés par l'intermédiaire d'un atome de carbone ou d'un atome d'azote,
contenant un ou deux groupes carbonyle ou thiocarbonyle voisins d'un atome d'azote,
pouvant être substitués, sur un des atome d'azote, par un groupe alkyle,
pouvant être substitués sur un ou deux atomes de carbone par un groupe alkyle, par un groupe phényle, phénylméthyle, naphtyle, biphénylyle, pyridinyle, diazinyle, furyle, thiényle, pyrrolyle, 1,3-oxazolyle, 1,3-thiazolyle, isoxazolyle, pyrazolyle, 1-méthylpyrazolyle, imidazolyl- ou 1-méthylimidazolyle, les substituants pouvant être identiques ou différents, et
une liaison double oléfinique d'un des hétérocycles insaturés susmentionnés pouvant être condensée avec un cycle phényle, naphtyle, pyridine, diazine, 1,3-oxazol-, thiényle, furan-, thiazol-, pyrrol-, N-méthylpyrrol- ou quinoléine, avec un cycle 1H-quinoléin-2-one substitué, le cas échéant, à l'atome d'azote par un groupe alkyle, ou avec un cycle imidazol, ou N-méthyl-imidazol, ou bien chacune de deux liaisons doubles oléfiniques d'un des hétérocycles susmentionnés pouvant être condensés avec un cycle phényle,
les groupes phényle, pyridinyle, diazinyle, furyle, thiényle, pyrrolyle, 1,3-oxazolyle, 1,3-thiazolyle, isoxazolyle, pyrazolyle, 1-méthylpyrazolyle, imidazolyl- ou 1-méthylimidazolyle contenus dans R¹ ainsi que les hétérocyles benzo, thiéno-, pyrido- et diazino-condensés dans le squelette de carbone pouvant être mono-, di- ou trisubstitués en sus par des atomes de fluor, de chlore, de brome ou d'iode, par des groupes alkyle, alkoxy, nitro-, alkylthio-, alkylsulfinyle, alkylsulfonyle, alkylsulfonylamino, phényle, difluorométhyle, trifluorométhyle, alkoxycarbonyle, carboxy, hydroxy, amino, alkylamino, dialkylamino, acétyle, acétylamino, propionylamino, aminocarbonyle, alkylaminocarbonyle, dialkylaminocarbonyle, (4-morpholinyl)-carbonyle, (1-pyrrolidinyl)carbonyle, (1-pipéridinyl)carbonyle, (hexahydro-1-azépinyl)carbonyle, (4-méthyl-1-pipérazinyl)carbonyle, méthylènedioxy, aminocarbonylamino, alkanoyle, cyano, difluorométhoxy, trifluorométhoxy, trifluorométhylthio-, trifluorométhylsulfinyl- ou trifluorométhylsulfonyle, les substituants pouvant être identiques ou différents,
R² désigne un atome d'hydrogène,
un groupe phénylméthyle ou un groupe alkyle en C₂₋₇, qui peut être substitué en position ω par un groupe cyclohexyle, phényle, pyridinyle, diazinyle, hydroxy, amino, alkylamino, dialkylamino, carboxy, alkoxycarbonyle, aminocarbonyle, aminocarbonylamino, acétylamino, 1-pyrrolidinyle, 1-pipéridinyle, 4-(1-pipéndinyl)-1-pipéridinyle, 4-morpholinyle, hexahydro-1H-1-azépinyle, [bis-(2-hydroxyéthyl)amino, 4-alkyl-1-pipérazinyl- ou 4-(ω)-hydroxy-alkyl-C₂₋₇)-1-pipérazinyl-,
un groupe phényle ou pyridinyle,
les radicaux hétérocycliques et les groupes phényle susmentionnés pouvant être, en sus sur le squelette de carbone, mono-, di- ou trisubstitués par de atomes de fluor, de chlore, de brome ou d'iode, par des groupes méthyle, alkoxy, difluorométhyle, trifluorométhyle, hydroxy, amino, alkylamino en C₁₋₃, di-(alkyle-C₁₋₃)amino, acétylamino, aminocarbonyle, cyano, méthylsulfonyloxy, difluorométhoxy, trifluorométhoxy, trifluorométhylthio-, trifluorométhylsulfinyle, trifluorométhylsulfonyle, amino- alkyle en C₁₋₃, alkylamino en C₁₋₃-alkyle en C₁₋₃ ou di-(alkyle-C₁₋₃)-amino-(alkyle-C₁₋₃) et les substituants pouvant être identiques ou différents,
R³ désigne un atome d'hydrogène ou un groupe alkyle en C₁₋₃ éventuellement substitué par un groupe phényle ou pyridinyle,
le groupe alkyle en C₁₋₃ pouvant être lié à un groupe alkyle présent dans R² ou à un cycle phényle ou pyridyline présent dans R² et à l'atome d'azote, auquel il sont liés, avec formation d'un cycle, ou
R² et R³ désignent, conjointement à l'atome d'azote inclus, un radical de la formule générale : 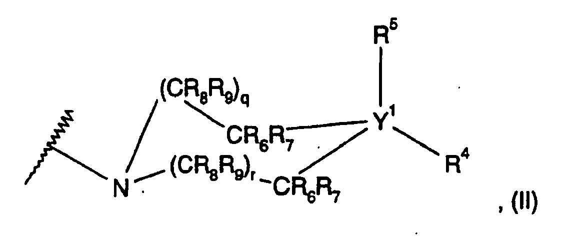 dans laquelle
Y¹ désigne un atome de carbone ou, lorsque R⁵ représente un paire d'électrons libre, également un atome d'azote,
q et r, lorsque Y¹ représente un atome de carbone, désignent les chiffres 0,1 1 ou 2 ou
q et r, lorsque Y¹ représente un atome d'azote, désignent les chiffres 1 ou 2,
R⁴ désigne un atome d'hydrogène, un groupe amino, alkylamino, cycloalkylamino, dialkylamino, N-(cycloalkyl)-alkylamino, dicycloalkylamino, hydroxy, alkyle, cycloalkyle, amino-alkyle-C₂₋₇, alkylamino-alkyle-C₂₋₇, dialkylamino-alkyle-C₂₋₇, aminoiminométhyle, alkylcarbonyle, alkylsulfonyle, alkylcarbonylamino, alkylsulfonylamino, N-alkylcarbonyl-N-alkylamino, N-alkylsulfonyl-N-alkylamino, aminocarbonylamino, alkylaminocarbonylamino, dialkylaminocarbonylamino, cycloalkylaminocarbonylamino, dicycloalkylaminocarbonylamino, phénylaminocarbonylamino, aminocarbonylalkyle, alkylaminocarbonylalkyle, dialkylaminocarbonylalkyle, aminocarbonylaminoalkyle, alkoxycarbonyle, alkoxycarbonylalkyle ou carboxyalkyle,
ou également, lorsque Y¹ ne représente pas un atome d'azote, le groupe carboxy, aminométhyle, alkylaminométhyl- ou dialkylaminométhyl-,
un groupe phényle, phényl-alkyle-C₁₋₃, pyridinyle, diazinyle, 1-naphtyle, 2-naphtyle, pyridinylcarbonyle ou phénylcarbonyle, chacun pouvant être mono-, di- ou trisubstitué, dans le squelette de carbone, par des atomes de fluor, de chlore, de brome ou d'iode, par un groupe alkyle, alkoxy, diéthylsulfonyloxy, difluorométhyle, trifluorométhyle, hydroxy, amino, acétylamino, aminocarbonyle, aminocarbonylamino, aminocarbonylaminométhyle, cyano, carboxy, alkoxycarbonyle, carboxyalkyle, alkoxycarbonylalkyle, alcanoyle, (ω-(dialkylamino)alcanoyle, ω-(dialkylamino)alkyle, ω-(dialkylamino)hydroxyalkyle, ω-(carboxy)alkanoyle, difluorométhoxy, trifluorométhoxy, trifluorométhylthio, trifluorométhylsulfinyle ou trifluorométhylsulfonyle, les substituants pouvant être identiques ou différents,
un groupe azacycloalkyle saturé ou mono- ou polyinsaturé, ayant de 4 à 10 chaînons, un groupe oxaza-, thiaza-, diaza- ou triazacycloalkyle ayant de 5 à 10 chaînons, un groupe azabicyclo- ou diazabicycloalkyle ayant de 6 à 10 chaînons, un groupe 1-alkyl-4-pipéridinylcarbonyle ou 4-alkyl-1-pipérazinylcarbonyle, un groupe 1-alkyl-4-pipéridinylamino, 1-alkyl-4-pipéridinylaminocarbonyle ou 1-alkyl-4-pipéridinylaminosulfonyle,
les hétérocycles mono- et bicycliques susmentionnés étant reliés par l'intermédiaire d'un atome d'azote ou d'un atome de carbone,
un groupe méthylène, dans lequel les hétérocycles mono- et bicycliques susmentionnés peuvent être remplacés par un groupe carbonyle ou sulfonyle,
dans les hétérocycles mono- et bicycliques susmentionnés, un groupe méthylène, non directement lié à un atome d'azote, à un atome d'oxygène ou à un atome de soufre, pouvant être substitué par un ou deux atomes de fluor,
les hétérocycles mono- et bicycliques susmentionnés ainsi que les groupes 1-alkyl-4-pipéridinylcarbonyle et 4-alkyl-1-pipérazinylcarbonyle dans le cycle pouvant être substitués une ou plusieurs fois par un groupe alkyle-C₁₋₇ et/ou
être simplement substitués par un groupe benzyle, alcanoyle, dialkylamino, phénylcarbonyle, pyridinylcarbonyle, carboxy, carboxyalcanoyle, carboxyalkyle, alkoxycarbonylalkyle, alkoxycarbonyle, aminocarbonyle, alkylaminocarbonyle, alkylsulfonyle, cycloalkyle ou cycloalkylalkyle, par un groupe cycloalkylcarbonyle éventuellement alkylé sur le cycle azacycloalkylcarbonyle, diazacycloalkylcarbonyl- ou oxazacycloalkylcarbonyl-,
les fractions alicycliques contenues dans ces substituants comprenant de 3 à 10 chaînons de cycle et les fractions hétérocycliques comprenant chacun de 4 à 10 chaînons et
les radicaux phényle et pyridinyle contenus dans les radicaux susmentionnés pouvant être, à leur tour, mono-, di- ou trisubstitués par des atomes de fluor, de chlore, de brome ou d'iode, par les groupes alkyle, alkoxy, méthylsulfonyloxy, difluorométhyle, trifluorométhyle, hydroxy, amino, acétylamino, aminocarbonyle, aminocarbonylamino, aminocarbonylaminométhyle, cyano, carboxy, alkoxycarbonyle, carboxyalkyle, alkoxycarbonylalkyle, alcanoyle, ω-(dialkylamino)alcanoyle, ω-(carboxy)alcanoyle, difluorométhoxy, trifluorométhoxy, trifluorométhylthio-, trifluorométhylsulfinyl- ou trifluorométhylsulfonyle, les substituants pouvant être identiques ou différents,
R⁵ désigne un atome d'hydrogène,
un radical alkyle-C₁₋₄, un radical alkyle non ramifié pouvant être substitué en position ω par un groupment phényle, pyridinyle, diazinyle, amino, alkylamino, dialkylamino, 1-pyrrolidinyle, 1-pipéridinyle, 4-méthyl-1-pipérazinyle, 4-morpholinyle ou hexahydro-1H-1-azépinyle,
un groupment alkoxycarbonyle, cyano- ou aminocarbonyle ou également, lorsque Y¹ représente un atome d'azote, une paire d'électrons libre,
ou, lorsque Y¹ ne représente aucun atome d'azote, également un atome de fluor ou R⁴ et R⁵ conjointement, lorsque Y¹ représente un atome de carbone, désignent un cycle cycloaliphatique ayant de 4 à 7 chaînons, dans lequel un ou deux groupes méthylène peuvent être remplacés par un groupe -NH- ou -N(alkyle)- ou un ou deux groupe méthylène supplémentaires peuvent l'être par des groupes carbonyle,
un atome d'hydrogène lié à un atome d'azote au sein du groupe R⁴ suscité pouvant être remplacé par un radical de protection,
R⁶ et R⁷, qui peuvent être identiques ou différents, désignent chacun un atome d'hydrogène, un groupe alkyle-C₁₋₃ ou dialkylamino ou également, lorsque Y¹ ne représente pas un atome d'azote, un atome de fluor et
R⁸ et R⁹, qui peuvent être identiques ou différents, désignent, chacun, un atome d'hydrogène ou un groupe alkyle-C₁₋₃, carboxy ou alkoxycarbonyl-C₁₋₃,
tous les groupes alkyle et alkoxy susmentionnés ainsi que les groupes alkyles présents au sein des autres radicaux cités, sauf indication expresse du contraire, comprenant de 1 à 7 atomes de carbone et pouvant être linéaires ou ramifiés, chaque groupe méthylène pouvant être substitué par jusqu'à 2 atomes de fluor et chaque groupe méthyle pouvant être substitué par jusqu'à 3 atomes de fluor ,
tous les groupes cycloalkyle susmentionnés ainsi que les groupes cycloalkyle présents au sein des autres radicaux cités, sauf indication expresse du contraire, comprenant de 3 à 10 atomes de carbone, chaque groupe méthylène pouvant être substitué par jusqu'à 2 atomes de fluor, et
tous les radicaux aromatiques et hétéroatomiques susmentionnés pouvant en outre être mono-, di- ou trisubstitués en sus par des atomes de fluor, de chlore ou de brome, par des groupes cyano- ou hydroxy et les substituants pouvant être identiques ou différents,
leurs tautomères, leurs diastéréoisomères, leurs énantiomères, leurs hydrates, leurs mélanges et leurs sels ainsi que les hydrates des sels.

2. Composés de la formule générale (I) selon la revendication 1, dans laquelle
A, U, V, W, X, R² et R³ sont définis comme dans la revendication 1 et
R¹ désigne un hétérocycle aza-, diaza-, triaza- ou thiaza simplement ou doublement insaturé ayant de 5 à 7 chaînons,
les hétérocycles susmentionnés étant reliés par l'intermédiaire d'un atome de carbone ou d'un atome d'azote,
contenant un ou deux groupes carbonyle voisins d'un atome d'azote,
pouvant être substitués, sur un atome de carbone, par un groupe phényle, pyridinyle, diazinyle, thiényle, pyrrolyle, 1,3-thiazolyle, isoxazolyle, pyrazolyle ou 1-méthylpyrazolyle et
une liaison double oléfinique d'un des hétérocycles insaturés susmentionnés pouvant être condensé avec un cycle phényle, naphtyle, pyridine, diazine, thiényle ou quinoléine ou avec un cycle 1H-quinoléine-2-one substitué avec un groupe méthyle, le cas échéant, sur l'atome d'azote,
les groupes phényle, pyridinyle, diazinyle, thiényle, pyrrolyle, 1,3-thiazolyle, isoxazolyle, pyrazolyle ou 1-méthylpyrazolyle contenus dans R¹ ainsi que les hétérocycles benzo-, pyrido- et diazinocondensés dans le squelette de carbone, pouvant être en sus mono-, di ou trisubstitués par des atomes de fluor, de chlore, de brome ou d'iode, par les groupes alkyle, alkoxy, nitro, difluorométhyle, trifluorométhyle, hydroxy, amino, alkylamino, dialkylamino, acétylamino, acétyle, cyano, difluorométhoxy ou trifluorométhoxy, les substituants pouvant être identiques ou différents,
les groupes alkyle susmentionnés ou les groupes alkyles contenus dans les autres radicaux susmentionnés, sauf indication expresse du contraire, contenant de 1 à 7 atomes de carbone et pouvant être linéaires ou ramifiés, chaque groupe méthylène pouvant être substitué par jusqu'à 2 atomes de fluor et chaque groupe méthyle pouvant être substitué par jusqu'à 3 atomes de fluor,
les radicaux aromatiques et hétéroatomiques susmentionnés pouvant en outre être mono-, di- ou trisubstitués par des atomes de fluor, de chlore ou de brome, par des groupes cyano ou hydroxy et les substituants pouvant être identiques ou différents,
leurs tautomères, leurs diastéréoisomères, leurs énantiomères, leurs mélanges et leurs sels.

3. Composés de la formule générale (I) selon la revendication 1, dans laquelle
A, U, V, W, X, R² et R³ sont définis comme dans la revendication 1 et
R¹ désigne un hétérocycle diaza ou triaza monoinsaturé ayant de 5 à 7 chaînons, les hétérocycles susmentionnés étant reliés par l'intermédiaire d'un atome d'azote, contenant un groupe carbonyle voisin d'un atome d'azote et
pouvant être substitués sur un atome de carbone en sus par un groupe phényle,
et une liaison double oléfinique d'un des hétérocycles insaturés susmentionnés pouvant être condensée avec un cycle phényle, thiényle ou quinoléine,
les groupes phényle contenus dans R¹ ainsi que les hétérocycles benzo-condensés dans le squelette de carbone pouvant être en outre mono-, di- ou trisubstitués par des atomes de fluor, de chlore, de brome ou d'iode, par les groupes méthyle, méthoxy, nitro, difluorométhyle, trifluorométhyle, hydroxy, amino, alkylamino, dialkylamino, acétylamino, acétyle, cyano, difluorométhoxy ou trifluorométhoxy, les substituants pouvant être identiques ou différents, pouvant toutefois, de préférence, être non substitués ou mono-substitués par un atome de fluor, de chlore ou de brome, par un groupe méthyle ou méthoxy,
tous les groupes alkyle susmentionnés ainsi que les groupes alkyle présents au sein des autres radicaux, sauf indication expresse du contraire, comprenant de 1 à 7 atomes de carbone et pouvant être linéaires ou ramifiés, et les radicaux aromatiques et hétéroaromatiques susmentionnés pouvant être en outre mono-, di- ou trisubstitués par des atomes de fluor, de chlore ou de brome, par des groupes cyano ou hydroxy et les substituants pouvant être identiques ou différents,
leurs tautomères, leurs diastéréoisomères, leurs énantiomères, leurs mélanges et leurs sels.

4. Composés de la formule générale (I) selon la revendication 1, dans laquelle
A, U, V, W, X, R² et R³ sont définis comme dans la revendication 1 et
R¹ désigne un groupe 4-(2-oxo-1,2,4,5-tétrahydro-1,3-benzodiazépin-3-yl)-, 4-(2-oxo-1,4-dihydro-2H-quinazolin-3-yl)-, 4-(5-oxo-3-phényl-4,5-dihydro-1,2,4-triazol-1-yl)-, 4-(2-oxo-1,2-dihydro-imidazo[4,5-c]quinoléin-3-yl)-, 4-(2-oxo-1,2-dihydro-4H-thiéno[3,4-d]pyrimidin-3-yl)-, 4-(2-oxo-1,4-dihydro-2H-thiéno[3,2-d]pyrimidin-3-yl)-, 4-(5-oxo-4,5,7,8-tétrahydro-2-thia-4,6-diaza-azulèn-6-yl)-, 4-(2-oxo-1,2,4,5-tétrahydro-thiéno[3,2-d]-1,3-diazépin-3-yl)-, 4-(2-oxo-1,2,4,5-tétrahydro-thiéno-[2,3-d]-1,3-diazépin-3-yl)- ou 4-(2-oxo-1,4-dihydro-2H-thiéno[2,3-d]-pyrimidin-3-yl)-,
les hétérocycles mono- ou bicycliques susmentionnés dans le squelette de carbone pouvant en outre être mono-substitués par un groupe méthoxy,
tous les radicaux aromatiques et hétéroatomiques susmentionnés pouvant en outre être mono-, di- ou trisubstitués en sus par des atomes de fluor, de chlore ou de brome, par des groupes cyano ou hydroxy et les substituants pouvant être identiques ou différents,
leurs tautomères, leurs diastéréoisomères, leurs énantiomères, leurs mélanges et leurs sels.

5. Composés de la formule générale (I) selon la revendication 1, dans laquelle
A, U, V, W, X, et R¹ sont définis comme dans la revendication 1 et
R² désigne un atome d'hydrogène ou
un groupe phénylméthyle ou un groupe alkyle-C₂₋₇, qui peut être substitué en position ω par un groupe phényle, pyridinyle, hydroxy, amino, alkylamino, dialkylamino, carboxy, alkoxy-carbonyle, aminocarbonyle, aminocarbonylamino, acétylamino, 1-pyrrolidinyle, 1-pipéridinyle, 4-morpholinyle, [bis-(2-hydroxyéthyl)amino,
les radicaux hétérocycliques et les groupes phényl susmentionnés pouvant être, en sus sur le squelette de carbone, mono-, di- ou trisubstitués par de atomes de fluor, de chlore, de brome ou d'iode, par des groupes méthyle, alkoxy, difluorométhyle, trifluorométhyle, hydroxy, amino, alkylamino-C₁-₃-, di-(alkyl-C₁-₃-)amino, acétylamino, aminocarbonyle, cyano, difluorométhoxy, trifluorométhoxy, amino-alkyl-C₁-₃, alkylamino-C₁-₃-alkyle-C₁-₃ ou di-(alkyl-C₁-₃)-amino-alkyl-C₁-₃ et les substituants pouvant être identiques ou différents,
R³ désigne un atome d'hydrogène ou un groupe alkyle-C₁₋₃,
le groupe alkyle-C₁₋₃ pouvant être lié à un groupe alkyle présent dans R² ou à un cycle phényle ou pyridyle présent dans R² et à l'atome d'azote, auquel il sont liés, avec formation d'un cycle ayant de 5 à 7 chaînons, ou
R² et R³ désignent, conjointement avec l'atome d'azote inclus, un radical de la formule générale : 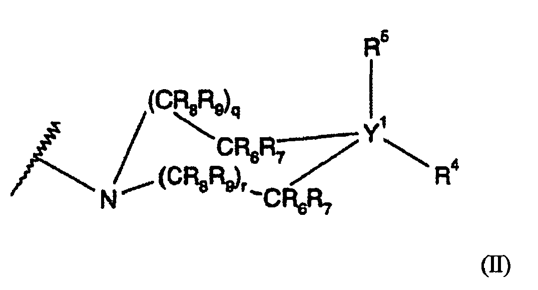 danslaquelle
Y¹ désigne un atome de carbone ou, lorsque R⁵ représente une paire d'électrons libre, également un atome d'azote,
q et r, lorsque Y¹ représente un atome de carbone, désignent les chiflrres 0 ou 1, ou
q et r, lorsque Y¹ représente un atome d'azote, désignent les chiffres 1 ou 2,
R⁴ désigne un atome d'hydrogène, un groupe hydroxy, amino, alkylamino, cycloalkylamino-C₃₋₆, N-(cycloalkyl-C₃₋₆)-alkylamino ou dialkylamino, un groupe alkyle, trifluorométhyle, cycloalkyle-C₃₋₆, dialkylamino-alkyle-C₂₋₇, carboxyalkyle, alkoxy-carbonylalkyle, alkylsulfonyle, alkylsulfonylamino- ou N-(alkylsulfonyl)-alkyl-amino,
ou également, lorsque Y¹ ne représente pas un atome d'azote, le groupe carboxy ou dialkylaminométhyle,
un groupe phényle, phényl-alkyl-C₁₋₃, pyridinyle ou diazinyle, qui peut être substitué à chaque fois par un atome de fluor, de chlore ou de brome, par un groupe trifluorométhylcarbonyle, méthyle ou méthoxy,
un groupe azacycloalkyle, saturé ou mono- ou polyinsaturé, ayant de 4 à 7 chaînons, un groupe oxaza-, diaza- ou triazacycloalkyle ayant de 5 à 7 chaînons, un groupe azabicyclo- ou diazabicycloalkyle, ayant de 7 à 9 chaînons, un groupe 1-alkyl-4-pipéridinylamino ou un groupe 1-alkyl-4-pipéridinylaminosulfonyle,
les hétérocycles mono- et bicycliques susmentionnés étant reliés par l'intermédiaire d'un atome d'azote ou d'un atome de carbone,
un groupe méthylène des hétérocycles mono- et bicycliques susmentionnés pouvant être remplacé par un groupe carbonyle ou sulfonyle,
dans les hétérocycles mono- et bicycliques susmentionnés un groupe méthylène, non directement lié à un atome d'azote, à un atome d'oxygène ou à un atome de soufre, pouvant être substitué par un ou deux atomes de fluor,
les hétérocycles mono- et bicycliques susmentionnés pouvant être substitués par un ou deux groupes alkyle-C₁₋₃, dans lesquels chaque groupe méthylène peut être substitué par jusqu'à 2 et chaque groupe méthyle par jusqu'à 3 atomes de fluor, et/ou par un groupe cycloalkyel-C₃-₆-alkyle-C₁₋₃, benzyle, alcanoyle-C₁₋₄, di-(alkyl-C₁₋₄)-amino ou alkylsulfonyl-C₁₋₃, par un groupe alkoxycarbonyle, benzyloxycarbonyle, alkoxycarbonylalkyle, carboxy ou carboxyalkyle,
R⁵ désigne un atome d'hydrogène, un groupe C₁₋₃-alkyle ou alkoxycarbonyle ou,
lorsque Y¹ représente un atome d'azote, également une paire d'électrons libre ou
R⁴ et R⁵ conjointement, lorsque Y¹ représente un atome de carbone, désignent un cycle cycloaliphatique ayant de 5 à 6 chaînons, dans lequel un ou deux groupes méthylène peuvent être remplacés par un groupe -NH- ou -N(méthyl)- ou un ou deux groupes méthylène supplémentaires peuvent être remplacés par des groupes carbonyle,
R⁶ et R⁷, qui peuvent être identiques ou différents, désignent chacun un atome d'hydrogène ou un groupe allcyle-C₁₋₃ ou di-(alkyl-C₁-₃)-amino et
R⁸ et R⁹, qui peuvent être identiques ou différents, représentent chacun un atome d'hydrogène ou un groupe alkyl-C₁₋₃, carboxy ou alkoxycarbonyl-C₁₋₃,
tous les groupes alkyles susmentionnés ainsi que les groupes alkyle présents au sein des autres radicaux, sauf indication expresse du contraire, comprenant de 1 à 7 atomes de carbone et pouvant être linéaires ou ramifiés et les radicaux aromatiques et hétéroaromatiques pouvant être en outre mono-, di- ou trisubstitués par des atomes de fluor, de chlore ou de brome, par des groupes cyano ou hydroxy et les substituants pouvant être identiques ou différents,
leurs tautomères, leurs diastéréoisomères, leurs énantiomères, leurs mélanges et leurs sels.

6. Composés de la formule générale (I) selon la revendication 1, dans laquelle
A, U, V, W, X, et R¹ sont définis comme dans la revendication 1 et
R² désigne un groupe phényle ou un groupe alkyle-C₂₋₇, qui peut être substitué en position ω par un groupe phényle, amino, alkylamino ou dialkylamino,
le groupe phényle suscité pouvant être substitué par un groupe amino-alkyle-C₁₋₃, alkylamino-C₁₋₃-alkyle-C₁₋₃ ou di-(alkyle-C₁-₃)-amino-alkyle-C₁₋₃,
R³ désigne un atome d'hydrogène ou un groupe alkyle-C₁₋₃,
R² et R³ conjointement avec l'atome d'azote auquel ils sont reliés, désignent un groupe 7-diméthylaminométhyl-1,2,4,5-tétrahydro-3-benzazépin-3-yle ou 2-amino-4,5,7,8-tétrahydro-thiazolo[4,5-d]azépin-6-yle ou
R² et R³ désignent, conjointement avec l'atome d'azote inclus, un radical de la formule générale 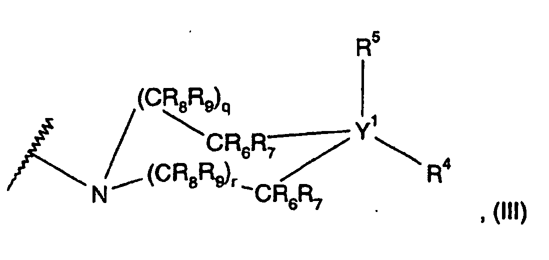 dans laquelle
Y¹ désigne un atome de carbone ou, lorsque R⁵ représente une paire d'électrons libre, également un atome d'azote,
q et r, lorsque Y¹ représente un atome de carbone, désignent les chiffres 0 ou 1 ou
q et r, lorsque Y¹ représente un atome d'azote, désignent les chiffres 1 ou 2,
R⁴ désigne un atome d'hydrogène,
un groupe phényle, benzyle ou pyridinyle, qui peut être substitué à chaque fois par un atome de fluor, de chlore ou de brome, par un groupe trifluorométhylcarbonyle, méthyle ou méthoxy,
un groupe hydroxy, carboxy, méthyle, trifluorométhyle, n-propyle, phényle, p-tolyle, p-trifluorométhylcarbonyl-phényle, p-(3-diméthylaminopropyl)-phényle, amino, benzyle, tert-butylamino, diméthylamino, diéthylamino, diéthylaminométhyle, 2-diméthylaminoéthyle, 2-diéthylaminoéthyle, 5-aminopentyle, méthoxycarbonyle, méthoxycarbonylméthyle, perhydro-azépin-1-yle, 4-méthyl-perhydro-1,4-diazépin-1-yle, 1-méthyl-1-pipéridinyl-4-yle, 4-pipérazin-1-yle, 4-acétyl-pipérazin-1-yle, 4-cyclopropylméthyl-pipérazin-1-yle, pyrrolidin-1-yle, 4-éthylpipérazinyle, 4-isopropyl-pipérazin-1-yle, pipéridin-1-yle, pipéridin-4-yle, morpholin-4-yle, 4,4-difluor-1-pipéridin-1-yle, 1-méthyl-1-aza-bicyclo[3.2.1]oct-4-yle, 4-méthyl-pipérazin-1-yle, 4-éthylpipérazin-1-yle, 1-méthyl-pipéridin-1-yle, 4-carboxyméthyl-pipérazin-1-yle, 1-carboxyméthyl-pipéridin-4-yle, 4-benzyloxycarbonyl-pipérazin-1-yle, 1-éthoxycarbonylméthyl-pipéridin-4-yle, azétidin-1-yle, 5-méthyl-2,5-diazabicyclo[2.2.1]hept-2-yle, 1-benzyl-pipéridin-4-yle, 4-benzyl-pipérazin-1-yle, 4-diméthylaminométhyl-1-phényle, 2,2,2-trifluoroéthyl-pipérazin-1-yle, 1-méthyl-sulfonyl-pipéridin-4-yle, pipéridin-1-yl-méthyle, 1-méthyl-pipéridin-4-yl-amino, méthylsulfonylamino, N-méthylsulfonyl-N-méthylamino, N-(cyclopentyl)-méthyl-amino, 1,1-dioxo-λ⁶-isothiazolidin-2-yle, 2-oxo-perhydro-1,3-oxazin-3-yle, cyclohexyle, 2-oxo-imidazolidin-1-yle, 2-méthyl-imidazol-1-yle, 4-méthyl-imidazol-1-yle, 4-thiazol-2-yle, 2,4-diméthyl-imidazol-1-yle, 4-imidazol-1-yle, 1,2,4-triazol-1-yle, 1-aza-bicyclo[2.2.2]oct-3-yle, 1-méthyl-pipéridin-4-yl-méthylsulfonyle, 1 H-imidazol-4-yle, 4-éthoxycarbonylméthyl-pipérazin-1-yle, 1-éthoxycarbonyl-pipéridin-4-yle, 4-tert-butoxycarbonylméthyl-pipérazin-1-yle, 1-(2,2,2-trifluoroéthyl)-pipéridin-4-yle, 4-méthylsulfonyl-pipérazin-1-yle, 2-carboxy-4-méthyl-pipérazin-1-yle, 3-carboxy-4-méthyl-pipérazin-1-yle, 2-éthoxycarbonyl-4-méthyl-pipérazin-1-yle, 3-éthoxycarbonyl-4-méthyl-pipérazin-1-yle ou 4-(2,2,2-trifluoroéthyl)-pipérazin-1-yle,
R⁵ désigne un atome d'hydrogène, un groupe méthyle ou, lorsque Y¹ représente un atome d'azote, également une paire d'électrons libre ou
R⁴ et R⁵ conjointement, lorsque Y¹ représente un atome de carbone, signifient un groupe 1-méthyl-pipéridin-4-ylidène, cyclohexylidène ou imidazolidin-2,4-dion-5-ylidène,
R⁶ et R⁷ désignent chacun un atome d'hydrogène ou un groupe diméthylamino et
R⁸ et R⁹ désignent chacun un atome d'hydrogène, un groupe carboxy ou éthoxycarbonyle,
tous les groupes alkyle susmentionnés ainsi que les groupes alkyle présents au sein des autres radicaux, sauf indication expresse du contraire, comprenant de 1 à 7 atomes de carbone et pouvant être linéaires ou ramifiés et les radicaux aromatiques et hétéroaromatiques pouvant être en outre mono-, di- ou trisubstitués par des atomes de fluor, de chlore ou de brome, par des groupes cyano ou hydroxy et les substituants pouvant être identiques ou différents,
leurs tautomères, leurs diastéréoisomères, leurs énantiomères, leurs mélanges et leurs sels.

7. Composés de la formule générale (I) selon la revendication 1, dans lesquels
A désigne un atome d'oxygène, un groupe cyanimino ou phénylsulfonylimino,
X désigne un atome d'oxygène, un groupe imino ou méthylène,
U désigne un groupe alkyle-C₁₋₆, alcényle-C₂₋₆ ou alcynyle-C₂₋₆, dans lequel chaque groupe méthylène peut être substitué par jusqu'à 2 atomes de fluor et chaque groupe méthyle peut être substitué par jusqu'à 3 atomes de fluor,
V désigne un groupe amino ou hydroxy,
W désigne un atome d'hydrogène, un atome de chlore ou de brome, ou un groupe trifluorométhyle,
R¹ désigne un hétérocycle diaza ou triaza, mono- ou polyinsaturé allant de 5 à 7 chaînons,
les hétérocycles susmentionnés étant reliés par l'intermédiaire d'un atome d'azote, contenant un groupe carbonyle voisin d'un atome d'azote et
pouvant être substitué en outre sur un atome de carbone en sus par un groupe phényle,
une liaison double oléfinique d'un des hétérocycles insaturés susmentionnés pouvant être condensée avec un cycle phényle, thiényle ou quinoléine,
les groupes phényle contenus dans R¹ ainsi que les hétérocycles benzocondensés sur le squelette de carbone pouvant être en outre mono-, di- ou trisubstitués par des atomes de fluor, de chlore, de brome ou d'iode, par les groupes méthyle, méthoxy, nitro-, difluorométhyle, trifluorométhyle, hydroxy, amino, alkylamino, dialkylamino, acétylamino, acétyle, cyano, difluorométhoxy ou trifluorométhoxy, les substituants pouvant être identiques ou différents, pouvant toutefois, de préférence, être non substitués ou mono-substitués par un atome de fluor, de chlore ou de brome, par un groupe méthyle ou méthoxy,
R² désigne un atome d'hydrogène ou
un groupe phényle ou un groupe alkyl-C₂₋₇, qui peut être substitué en position ω par un groupe phényle, pyridinyle, hydroxy, amino, alkylamino, dialkylamino, alkoxy-carbonyle, carboxy, aminocarbonyle, aminocarbonylamino, acétylamino, 1-pyrrolidinyle, 1-pipéridinyle, 4-morpholinyle, [bis-(2-hydroxyéthyl)amino,
les radicaux hétérocycliques et les groupes phényle susmentionnés pouvant être, en outre mono-, di- ou trisubstitués sur le squelette de carbone par des atomes de fluor, de chlore, de brome ou d'iode, par des groupes méthyle, alkoxy, difluorométhyle, trifluorométhyle, hydroxy, amino, alkylamino-C₁-₃, di-(alkyl-C₁-₃-)amino, acétylamino, aminocarbonyle, cyano, difluorométhoxy, trifluorométhoxy, amino-alkyle-C₁₋₃, alkylamino-C₁₋₃-alkyle-C₁₋₃ ou di-(alkyle-C₁-₃)-amino-alkyle-C₁₋₃ et les substituants pouvant être identiques ou différents,
R³ désigne un atome d'hydrogène ou un groupe alkyle-C₁₋₃,
le groupe alkyle-C₁₋₃ pouvant être lié, avec formation d'un cycle ayant de 5 à 7 chaînons, à un groupe alkyle présent dans R² ou à un cycle phényle ou pyridyle présent dans R² et à l'atome d'azote, auquel ils sont liés ou
R² et R³ désignent, conjointement à l'atome d'azote inclus, un radical de la formule générale 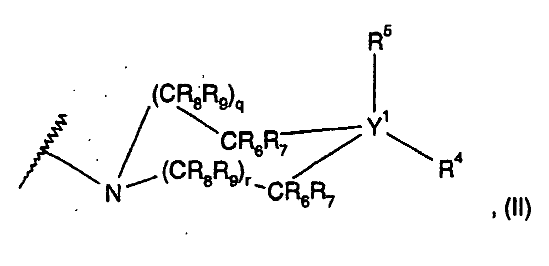 dans laquelle
Y¹ désigne un atome de carbone ou, lorsque R⁵ représente une paire d'électrons libre, également un atome d'azote,
q et r, lorsque Y¹ représente un atome de carbone, les chiffres 0 ou 1 ou
q et r, lorsque Y¹ représente un atome d'azote, les chiffres 1 ou 2 ou
R⁴ désigne un atome d'hydrogène, un groupe hydroxy, amino, alkylamino, cycloalkylamino-C₃₋₆, N-(cycloalkyl-C₃₋₆)-alkylamino ou dialkylamino, un groupe alkyle, trifluorométhyle, cycloalkyl-C₃₋₆, dialkylamino-alkyl-C₂₋₇, carboxyalkyle, alkoxy-carbonylalkyle, alkylsulfonyle, alkylsulfonylamino ou N-(alkylsulfonyl)-alkyl-amino,
ou également, lorsque Y¹ ne représente pas un atome d'azote, le groupe carboxy ou dialkylaminométhyle,
un groupe phényle, phényl-alkyl-C₁₋₃, pyridinyle ou diazinyle, chacun éventuellement substitué par un atome de fluor, de chlore ou de brome, par un groupe trifluorométhylcarbonyle, méthyle ou méthoxy,
un groupe azacycloalkyle, saturé ou mono- ou polyinsaturé, ayant de 4 à 7 chaînons, un groupe oxaza-, diaza- ou triazacycloalkyle ayant de 5 à 7 chaînons, un groupe azabicyclo- ou diazabicycloalkyle, ayant de 7 à 9 chaînons, un groupe 1-alkyl-4-pipéridinylamino- ou un groupe 1-alkyl-4-pipéridinylaminosulfonyl-,
les hétérocycles mono- et bicycliques susmentionnés étant reliés par l'intermédiaire d'un atome d'azote ou d'un atome de carbone,
un groupe méthylène des hétérocycles mono- et bicycliques susmentionnés pouvant être remplacé par un groupe carbonyle ou sulfonyle,
dans les hétérocycles mono- et bicycliques susmentionnés un groupe méthylène, non directement lié à un atome d'azote, à un atome d'oxygène ou à un atome de soufre, pouvant être substitué par un ou deux atomes de fluor,
les hétérocycles mono- et bicycliques susmentionnés pouvant être substitués par un ou deux groupes alkyl-C₁₋₃, dans lesquels chaque groupe méthylène peut être substitué par jusqu'à 2 et chaque groupe méthyle par jusqu'à 3 atomes de fluor, et/ou
par un groupe cycloalkyl-C₃-₆-alkyl-C₁₋₃, benzyle, alcanoyl-C₁₋₄, di-(alkyl-C₁₋₃)-amino- ou alkylsulfonyl-C₁₋₃, par un groupe alkoxycarbonyle, benzyloxycarbonyle, alkoxycarbonylalkyle, carboxy- ou carboxyalkyle,
R⁵ désigne un atome d'hydrogène, un groupe alkyle-C₁₋₃ ou alkoxycarbonyle ou,
lorsque Y¹ représente un atome d'azote, également une paire d'électrons libre ou
R⁴ et R⁵ conjointement, lorsque Y¹ représente un atome de carbone, désignent un cycle cycloaliphatique ayant de 5 à 6 chaînons, dans lequel un ou deux groupes méthylène peuvent être remplacés par un groupe -NH- ou -N(méthyl)- ou un ou deux groupes méthylène supplémentaires peuvent être remplacés par des groupes carbonyle,
R⁶ et R⁷, qui peuvent être identiques ou différents, désignent chacun un atome d'hydrogène ou un groupe alkyl-C₁₋₃- ou di-(alkyl-C₁₋₃)-amino et
R⁸ et R⁹, qui peuvent être identiques ou différents, désignent chacun un atome d'hydrogène ou un groupe alkyle-C₁₋₃, carboxy ou alkoxycarbonyle-C₁₋₃,
les groupes alkyle susmentionnés ou les groupes alkyle présents au sein des autres radicaux susmentionnés, sauf indication expresse du contraire, comprenant de 1 à 7 atomes de carbone et pouvant être linéaires ou ramifiés et les radicaux aromatiques et hétéroaromatiques pouvant être en outre mono-, di- ou trisubstitués par des atomes de fluor, de chlore ou de brome, par des groupes cyano ou hydroxy et les substituants pouvant être identiques ou différents,
leurs tautomères, leurs énantiomères, leurs diastéréoisomères, leurs mélanges et leurs sels.

8. Composés de la formule générale (I) selon la revendication 1, dans lesquels
A désigne un atome d'oxygène,
X désigne un atome d'oxygène, un groupe imino ou méthylène,
U désigne un groupe méthyle, éthyle alcényle-C₂₋₄ ou alcynyle-C₂₋₄, dans lequel chaque groupe méthylène peut être substitué par jusqu'à 2 atomes de fluor et chaque groupe méthyle peut être substitué par jusqu'à 3 atomes de fluor,
V désigne un groupe amino- ou hydroxy,
W désigne un atome d'hydrogène, un atome de chlore ou de brome ou un groupe trifluorométhyle,
R¹ désigne un groupe 4-(2-oxo-1,2,4,5-tétrahydro-1,3-benzodiazépin-3-yl)-, 4-(2-oxo-1,4-dihydro-2H-quinazolin-3-yl)-, 4-(5-oxo-3-phényl-4,5-dihydro-1,2,4- triazol-1-yl)-, 4-(2-oxo-1,2-dihydro-imidazo[4,5-c]quinoléine-3-yl)-, 4-(2-oxo-1,2-dihydro-4H-thiéno[3,4-d]pyrimidin-3-yl)-e, 4-(2-oxo-1,4-dihydro-2H-thiéno[3,2-d]pyrimidin-3-yl)-, 4-(5-oxo-4,5,7,8-tétrahydro-2-thia-4,6-diaza-azulèn-6-yl)-, 4-(2-oxo-1,2,4,5-tétrahydro-thiéno[3,2-d]-1,3-diazépin-3-yl)-, 4-(2-oxo-1,2,4,5-tétrahydro-thiéno-[2,3-d]-1,3-diazépin-3-yl)- ou 4-(2-oxo-1,4-dihydro-2H-thiéno[2,3-d]-pyrimidin-3-yl)-,
les hétérocycles mon- ou bicycliques susmentionnés dans le squelette de carbone pouvant en outre être monosubstitués par un groupe méthoxy,
R² désigne un groupe phényle ou un groupe alkyle-C₂-₇, qui peut être substitué en position ω par un groupe phényle, amino, alkylamino ou dialkylamino,
le groupe phényle suscité pouvant être substitué par un groupe amino-alkyl-C₁-₃, alkylamino- C₁₋₃-alkyle-C₁₋₃ ou di-(alkyl-C₁₋₃-)-amino-alkyle-C₁₋₃,
R³ désigne un atome d'hydrogène ou un groupe alkyle-C₁₋₃,
R² et R³ conjointement avec l'atome d'azote, auquel ils sont reliés, désignent un groupe 7-diméthylaminométhyl-1,2,4,5-tétrahydro-3-benzazépin-3-yle ou 2-amino-4,5,7,8-tétrahydro-thiazolo[4,5-d]azépin-6-yle ou
R² et R³ désignent, conjointement avec l'atome d'azote inclus, un radical de la formule générale : 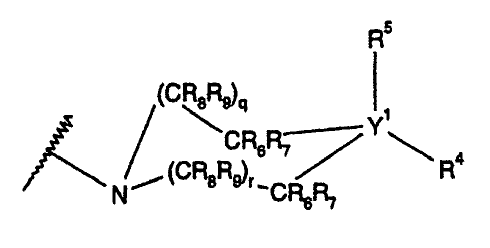 dans laquelle
Y¹ désigne un atome de carbone ou, lorsque R⁵ représente une paire d'électrons libre, également un atome d'azote,
q et r, lorsque Y¹ représente un atome de carbone, désignent les chiffres 0 ou 1 ou
q et r, lorsque Y¹ représente un atome d'azote, désignent les chiffres 1 ou 2,
R⁴ désigne un atome d'hydrogène,
un groupe phényle, benzyle ou pyridinyle, éventuellement substitués par un atome de fluor, de chlore ou de brome, par un groupe trifluorométhylcarbonyle, méthyle ou méthoxy,
un groupe hydroxy, carboxy, méthyle, trifluorométhyle, n-propyle, phényle, p-tolyle, p-trifluorométhylcarbonyl-phényle, p-(3-diméthylaminopropyi)-phényle, amino, benzyle, tert-butylamino, diméthylamino, diéthylamino, diéthylaminométhyle, 2-diméthylaminoéthyle, 2-diéthylaminoéthyle, 5-aminopentyle, méthoxycarbonyle, méthoxycarbonylméthyle, perhydro-azépin-1-yle, 4-méthyl-perhydro-1,4-diazépin-1-yle, 1-méthyl-1-pipéridinyl-4-yle, 4-pipérazin-1-yle, 4-acétyl-pipérazin-1-yle, 4-cyclopropylméthyl-pipérazin-1-yle, pyrrolidin-1-yle, 4-éthyl-pipérazin-1-yle, 4-isopropyl-pipérazin-1-yle, pipéridin-1-yle, pipéridin-4-yle, morpholin-4-yle, 4,4-difluoro-1-pipéridin-1-yle, 1-méthyl-1-aza-bicyclo[3.2.1]oct-4-yle, 4-méthyl-pipérazin-1-yle, 4-éthylpipérazin-1-yle, 1-méthyl-pipéridin-1-yle, 4-carboxyméthyl-pipérazin-1-yle, 1-carboxyméthyl-pipéridin-4-yle, 4-benzyloxycarbonyl-pipérazin-1-yle, 1-éthoxycarbonylméthyl-pipéridin-4-yle, azétidin-1-yle, 5-méthyl-2,5-diaza-bicyclo [2.2.1]hept-2-yle , 1-benzyl-pipéridin-4-yle, 4-benzyl-pipérazin-1-yle, 4-diméthylaminométhyl-1-phenyle, 2,2,2-trifluoroéthyl-pipérazin-1-yle, 1-méthyl-sulfonyl-pipéridin-4-yle, pipéridin-1-yl-méthyle, 1-méthyl-pipéridin-4-yl-amino, méthylsulfonylamino, N-1-éthylsulfonyl-N-méthylamino, N-(cyclopentyl)-méthylamino, 1, 1-dioxo-λ6-isothiazolidin-2-yle, 2-oxo-perhydro-1,3-oxazin-3-yle, cyclohexyle, 2-oxo-imidazolidin-1-yle, 2-méthyl-imidazol-1-yle, 4-méthyl-imidazol-1-yle, 4-thiazol-2-yle, 2,4-diméthyl-imidazol-1-yle, 4-imidazol-1-yle, 1,2,4-triazol-1-yle, 1-aza-bicyclo(2.2.2)oct-3-yle, 1-méthyl-pipéridin-4-yl-méthylsulfonyle, 1H-imidazol-4-yle, 4-éthoxycarbonylméthyl-pipérazin-1-yle, 1-éthoxycarbonyl-pipéridin-4-yl- 4-tert-butoxycarbonylméthyl-pipérazin-1-yle, 1-(2,2,2-trifluoroéthyl)-pipéridin-4-yle, 4-méthylsulfonyl-pipérazin-1-yle, 2-carboxy-4-méthyl-pipérazin-1-yle, 3-carboxy-4-méthyl-pipérazin-1-yle, 2-éthoxycarbonyl-4-méthyl-pipérazin-1-yle, 3-éthoxycarbonyl-4-méthyl-pipérazin-1-yl- ou 4-(2,2,2-trifluoroéthyl)-pipérazin-1-yl-,
R⁵ désigne un atome d'hydrogène, un groupe méthyle ou, lorsque Y¹ représente un atome d'azote, également une paire d'électrons libre ou
R⁴ et R⁵ conjointement, lorsque Y¹ représente un atome de carbone, signifient un groupe 1-méthyl-pipéridin-4-ylidène, cyclohexylidène ou imidazolidin-2,4-dion-5-ylidène,
R⁶ et R⁷, désignent chacun un atome d'hydrogène ou un groupe diméthylamino et
R⁸ et R⁹ représentent chacun un atome d'hydrogène, un groupe carboxy ou éthoxycarbonyle,
tous les groupes alkyle susmentionnés ainsi que les groupes alkyle présents au sein des autres radicaux, sauf indication expresse du contraire, comprenant de 1 à 7 atomes de carbone et pouvant être linéaires ou ramifiés et les radicaux aromatiques et hétéroaromatiques susmentionnés pouvant être en outre mono-, di- ou trisubstitués par des atomes de fluor, de chlore ou de brome, par des groupes cyano ou hydroxy et les substituants pouvant être identiques ou différents,
leurs énantiomères, leurs diastéréoisomères et leurs sels.

9. Composés de la formule générale (I) selon la revendication 1 choisis parmi :
(1) la (S)-2-(4-amino-3-chloro-5-trifluorométhyl-benzyl)-1-[4-(4-méthyl-pipérazin-1-yl)-pipéridin-1-yl]-4-[4-(2-oxo-1,2,4,5-tétrahydro-1,3-benzodiazépin-3-yl)-pipéridin-1-yl]-butane-1,4-dione, la 2-(4-amino-3-chloro-5-trifluorométhyl-benzyl)-1-(1'-méthyl-[4,4']bipipéridinyl-1-yl)-4-[4-(2-oxo-1,4-dihydro-2H-quinazolin-3-yl)-pipéridin-1-yl]-butane-1,4-dione,
(2) la 2-(4-amino-3-chloro-5-trifluorométhyl-benzyl)-1-[4-(4-méthyl-pipérazin-1-yl)-pipéridin-1-yl]-4-[4-(2-oxo-1,4-dihydro-2H-quinazolin-3-yl)-pipéridin-1-yl]-butane-1,4-dione,
(3) la 2-(4-amino-3-chloro-5-trifluorométhyl-benzyl)-1-[1,4']bipipéridinyl-1'-yl-4-[4-(2-oxo-1,4-dihydro-2H-quinazolin-3-yl)-pipéridin-1-yl]-butane-1,4-dione,
(4) la 2-(4-amino-3-chloro-5-trifluorométhyl-benzyl)-4-[4-(2-oxo-1,4-dihydro-2H-quinazolin-3-yl)-pipéridin-1-yl]-1-(4-pyridin-4-yl-pipérazin-1-yl)-butane-1,4-dione,
(5) la 2-(4-amino-3-chloro-5-trifluorométhyl-benzyl)-1-[4-(1-méthyl-pipéridin-4-ylamino)-pipéridin-1-yl]-4-[4-(2-oxo-1,4-dihydro-2H-quinazolin-3-yl)-pipéridin-1-yl]-butane-1,4-dione,
(7) l'acide [4-(1-{2-(4-amino-3-chloro-5-trifluorométhyl-benzyl)-4-oxo-4-[4-(2-oxo-1,4-dihydro-2H-quinazolin-3-pipéridin-1-yl]-butyrlyl}-pipéridin-4-yl)-pipérazin-1-yl)acétique,
(8) l'ester méthylique de l'acide (1'-{2-(4-amino-3-chloro-5-trifluorométhyl-benzyl)-4-oxo-4-[4-(2-oxo-1,4-dihydro-2H-quinazolin-3-yl)-pipéridin-1-yl]-butyryl}-[4,4']bipipéridinyl-1 -yl}-acétique,
(9) l'acide (1'-{2-(4-amino-3-chloro-5-trifluorométhyl-benzyl)-4-oxo-4-[4-(2-oxo-1,4-dihydro-2H-quinazolin-3-yl)-pipéridin-1-yl]-butyryl}-[4,4']bipipéridinyl-1-yl}-acétique,
(10) le [(R)-1-(4-amino-3-chloro-5-trifluorométhyl-benzyl)-2-1,4'-bipipéridinyl-1'-yl-2-oxo-éthyl]-amide de l'acide 4-(2-oxo-1,2,4,5-tétrahydro-1,3-benzodiazépin-3-yl)-pipéridin-1-carboxylique,
(11) le [(R)-1-(4-amino-3-chloro-5-trifluorométhyl-benzyl)-2-(4-diméthylamino-pipéridin-1-yl]-2-oxo-éthyl]-amide de l'acide 4-(2-oxo-1,2,4,5-tétrahydro-1,3-benzodiazépin-3-yl)-pipéridin-1-carboxylique,
(12) le [(R)-1-(4-amino-3-chloro-5-trifluorométhyl-benzyl)-2-oxo-2-(4-pyridin-4-yl-pipérazin-1-yl)-éthy]-amide de l'acide 4-(2-oxo-1,2,4,5-tétrahydro-1,3-benzodiazépin-3-yl)-pipéridin-1-carboxylique,
(13) le [(R)-1-(4-amino-3-chloro-5-trifluorométhyl-benzyl-2-(l'-méthyl-4,4'-bipipéridinyl-1-yl)-2-oxo-éthyl]-amide de l'acide 4-(2-oxo-1,2,4,5-tétrahydro-1,3-benzodiazépin-3-yl)-pipéridin-1-carboxylique,
(14) l'ester benzylique de l'acide 4-[1-((R)-3-(4-amino-3-chloro-5-trifluorométhyl-phényl)-2-{[4-(2-oxo-1,2,4,5-tétrahydro-1,3-benzodiazépin-3-yl)-pipéridin-1-carbonyl]-amino}-propionyl)-pipéridin-4-yl]-pipérazin-1-carboxylique,
(15) l'ester éthylique de l'acide [1'-((R)-3-(4-amino-3-chloro-5-trifluorométhyl-phényl)-2-{[4-(2-oxo-1,2,4,5-téohydro-1,3-benzobiazépin-3-yl)-pipéridin-1-carbonyl]-amino}-prppionyl)-4,4'-bipipéridinyl-1-yl]-acétique,
(16) le {(R)-1-(4-amino-3-chloro-5-trifluorométhyl-benzyl)-2-[4-(4-méthyl-pipérazin-1-yl)-pipéridin-1-yl]-2-oxo-éthyl)-amide de l'acide 4-(2-oxo-1,2,4,5-tétrahydro-1,3-benzodiazépin-3-yl)-pipéridin-1-carboxylique,
(17) le {(R)-1-(4-amino-3-chloro-5-trifluorométhyl-benzyl)-2-[4-(1-méthyl-pipéridin-4-yl)-pipérazin-1-yl]-2-oxo-éthyl)-amide de l'acide 4-(2-oxo-1,2,4,5-tétrahydro-1,3-benzodiazépin-3-yl)-pipéridin-1-carboxylique,
(18) le [(R)-1-(4-amino-3-chloro-5-trifluorométhyl-benzyl)-2-(4-azétidin-1-yl-pipéridin-1-yl)-2-oxo-éthyl]-amide de l'acide 4-(2-oxo-1,2,4,5-tétrahydro-1,3-benzodiazépin-3-yl)-pipéridin-1-carboxylique,
(19) le {(R)-1-(4-amino-3-chloro-5-trifluorométhyl-benzyl)-2-[4-(5-méthyl-2,5-diaza-bicyclo[2.2.1]hept-2-yl)-pipéridin-1-yl]-2-oxo-éthyl)-amide de l'acide 4-(2-oxo-1,2,4,5-terahydro-1,3-benzodiazépin-3-yl)-pipéridin-1-carboxylique,
(20) le [(R)-1-(4-amino-3-chloro-5-trifluorométhyl-benzyl)-2-oxo-2-(4-pipérazin-1-yl-pipéridin-1-yl)-éthyl)-amide de l'acide 4-(2-oxo-1,2,4,5-tétrahydro-1,3-benzodiazépin-3-yl)-pipéridin-1-carboxylique,
(21) l'acide [1'-((R)-3-(4-amino-3-chloro-5-trifluorométhyl-phényl)-2-{[4-(2-oxo-1,2,4,5-tétrahydro-1,3-benzodiazépin-3-yl)-pipéridin-1-carbonyl]-amino}-propionyl)-4,4'-bipipéridinyl-1-yl)-acétique,
(22) le [1-(4-amino-3-chloro-5-trifluorométhyl-benzyl)-2-1,4'-bipipéridinyl-1'-yl-2-oxo-éthyl]-amide de l'acide 4-(2-oxo-1,2,4,5-tétrahydro-1,3-benzodiazépin-3-yl)-pipéridin-1-carboxylique,
(23) la (S)-2-(4-amino-3-bromo-5-trifluorométhyl-benzyl)-1-[4-(4-méthyl-pipérazin-1-yl)-pipéridin-1-yl]-4-[4-(2-oxo-1,2,4,5-tétrahydro-1,3-benzodiazépin-3-yl)-pipéridin-1-yl]-butane-1,4-dione,
(24) la (S)-2-(4-amino-3-bromo-5-trifluorométhyl-benzyl)-4-[4-(2-oxo-1,2,4,5-tétrahydro-1,3-benzodiazépin-3-yl)-pipéridin-1-yl]-1-(4-pyridin-4-yl-pipérazin-1-yl)-butane-1,4-dione,
(25) la (S)-2-(4-amino-3-bromo-5-trifluorométhyl-benzyl)-1-1,4'-bipipéridinyl-1'-yl-4-[4-(2-oxo-1,2,4,5-tétrahydro-1,3-benzodiazépin-3-yl)-pipéridin-1-yl]-butane-1,4-dione,
(26) la (*S*)-2-(4-amino-3-bromo-5-trifuorométhyl-benzyl)-1-[4-(1-méthyl-pipéridin-4-yl)-pipérazin-1-yl]-4-[4-(2-oxo-1,2,4,5-tétrahydro-1,3-benzodiazépin-3-yl)-pipéridin-1-yl]-butane-1,4-dione,
(27) la (S)-2-(4-amino-3-bromo-5-trifluorométhyl-benzyl)-1-(1'-méthyl-4,4'-bipiperidinyl-1-yl)-4-[4-(2-oxo-1,2,4,5-tétrahydro-1,3-benzodiazépin-3-yl)-pipéridin-1-yl]-butane-1,4-dione,
(28) la (S)-2-(4-amino-3-trifluorométhyl-benzyl)-1-[4-(4-méthyl-pipérazin-1-yl)-pipéridin-1-yl]-4-[4-(2-oxo-1,2,4,5-tétrahydro-1,3-benzodiazépin-3-yl)-pipéridin-1-yl]-butane-1,4-dione,
(29) la (S)-2-(4-amino-3-chloro-5-trifluorométhyl-benzyl)-1-(4-diméthylamino-pipéridin-1-yl)-4-[4-(2-oxo-1,2,4,5-tétrahydro-1,3-benzodiazépin-3-yl)-pipéridin-1-yl]-butane-1,4-dione,
(30) la (S)-2-(4-amino-3-chloro-5-trifluorométhyl-benzyl)-1-(3-diméthylamino-pipéridin-1-yl)-4-[4-(2-oxo-1,2,4,5-tétrahydro-1,3-benzodiazépin-3-yl)-pipéridin-1-yl]-butane-1,4-dione,
(31) la (S)-2-(4-amino-3-chloro-5-trifluorométhyl-benzyl)-4-[4-(2-oxo-1,2,4,5-tétrahydro-1,3-benzodiazépin-3-yl)pipéridin-1-yl]-1-(4-pyrrolidin-1-yl-pipéridin-1-yl)butane-1,4-dione,
(32) la (S)-2-(4-amino-3-chloro-5-trifluorométhyl-benzyl)-1-(1-méthyl-4,4-bipipéridinyl-yl)-4-[4-(2-oxo-1,2,4,5-tétrahydro-1,3-benzodiazépin-3-yl)-pipéridin-1-yl]-butane-1,4-dione,
(33) la (S)-2-(4-amino-3-chloro-5-trifluorométhyl-benzyl)-1-[4-(4-cyclopropylméthyl-pipérazin-1-yl)-pipéridin-1-yl]-4-[4-(2-oxo-1,2,4,5-tétrahydro-1,3-benzodiazépin-3-yl)-pipéridin-1-yl]-butane-1,4-dione,
(34) la (S)-2-(4-amino-3-chloro-5-trifluorométhyl-benzyl)-1-(4-morpholin-4-yl-pipéridin-1-yl]-4-[4-(2-oxo-1,2,4,5-tétrahydro-1,3-benzodiazépin-3-yl)-pipéridin-1-yl]-butane-1,4-dione,
(35) la (S)-2-(4-amino-3-chloro-5-trifluorométhyl-benzyl)-4-[4-(2-oxo-1,2,4,5-tétrahydro-1,3-benzodiazépin-3-yl)-pipéridin-1-yl]-1-(4-perhydro-azépin-1-yl-pipéridin-1-yl)-butane-1,4-dione,
(36) la (S)-2-(4-amino-3-chloro-5-trifluorométhyl-benzyl)-1-[4-(4-méthyl-perhydro-1,4-diazépin-1-yl)-pipéridin-1-yl]-4-[4-(2-oxo-1,2,4,5-tétrahydro-1,3-benzodiazépin-3-yl)-pipéridin-1-yl]-butane-1,4-dione,
(37) la (S)-2-(4-amino-3-chloro-5-trifluorométhyl-benzyl)-1-[4-(4-isopropyl-pipérazin-1-yl)-pipéridin-1-yl)-4-[4-(2-oxo-1,2,4,5-tétrahydro-1,3-benzodiazépin-3-yl)-pipéridin-1-yl]-butane-1,4-dione,
(38) la (S)-2-(4-amino-3-chloro-5-trifluorométhyl-benzyl)-1-1,4'-bipipéridinyl-1'-yl-4[4-(2-oxo-1,2,4,5-tétrahydro-1,3-benzodiazépin-3-yl)-pipéridin-1-yl]-butane-1 4-dione,
(39) la (S)-2-(-amino-3-chloro-5-trifluorométhyl-benzyl)-1-[4-(1-méthyl-pipéridin-4-yl)-perhydro-1,4-diazépin-1-yl]-4-[4-(2-oxo- 1,2,4,5-tétrahydro-1,3-benzodiazépin-3-yl)-pipéridin-1-yl]-butane-1,4-dione,
(40) la (S)-2-(4-amino-3-chloro-5-trifluorométhyl-benzyl)-1-[3-(4-méthyl-pipérazin-1-yl)-azétidin-1-yl]-4-[4-(2-oxo-1,2,4,5-tétiahydro-1,3-benzodiazépin-3-yl)-pipéridin-1-yl)butane-1,4-dione,
(41) la (S)2-(4-amino-3-chloro-5-trifluomméthyl-benzyl)4-[4-(2-oxo-1,2,4,5-tétrahydro-1,3-benzodiazépin-3-yl)pipéridin-1-yl]-1-(3-pyrrolidin-1-yl-azétidin-1-yl)-butane-1,4-dione,
(42) la (S)-2-(4-amino-3-chloro-5-trifluorométhyl-benzyl)-4-[4-(2-oxo-1,2,4,5-tétrahydro-1,3-benzodiazépin-3-yl)-pipéridin-1-yl]-1-(3-pipérdin-1-yl-azétidin-1-yl)-butane-1,4-dione,
(43) la (S)-2-(4-amino-3-chloro-5-trifluorométhyl-benzyl)-1-(3-diéthylamino-azétidin-1-yl)-4-[4-(2-oxo-1,2,4,5-tétrahydro-1,3-benzodiazépin-3-yl)-pipéridin-1-yl]-butane-1,4-dione,
(44) la (S)-2-(4-amino-3-chloro-5-trifluorométhyl-benzyl)-1-(4-azétidin-1-yl-pipéridin-1-yl)-4-[4-(2-oxo-1,2,4,5-tétrahydro-1,3-benzodiazépin-3-yl)-pipéridin-1-yl]-butane-1,4-dione,
(45) la (S)-1-[4-(4-acétyl-pipérazin-1-yl)-pipéridin-1-yl]-2-(4-amino-3-chloro-5-trifluorométhyl-benzyl)-4-[4-(2-oxo-1,2,4,5-tétrahydro-1,3-benzodiazépin-3-yl)-pipéridin-1-yl]-butane-1,4-dione,
(46) la (S)-2-(4-amino-3-chloro-5-trifluorométhyl-benzyl)-1-(4-diéthylaminométhyl-pipéridin-1-yl)4-[4-(2-oxo-1,2,4,5-tétrahydro-1,3-benzodiazépin-3-yl)-pipéridin-1-yl]-butane-1,4-dione,
(47) la (S)-2-(4-amino-3-chloro-5-trifluorméthyl-benzyl)-1-[4-(4-éthyl-pipérazin-1-yl)-pipéridin-1-yl]-4-[4-(2-oxo-1,2,4,5-tétrahydro-1,3-benzodiazépin-3-yl)pipéridin-1-yl]-butane-1,4-dione,
(48) la (S)-2-(4-amino-3-chlon)-5-tniluorométhyl-benzyl)-1-[4-(1-éthyl-pipéridin-4-yl)-pipérazin-1-yl]-4-[4-(2-oxo-1,2,4,5-térahydro-1,3-benzodiazépin-3-yl)-pipéndin-1-yl]-butane-1,4-dione,
(49) la (S)-2-(4-amino-3-chloro-5-trifluorométhyl-benzyl)-4-[4-(2-oxo-1,2,4,5-tétrahydro-1,3-benzodiazépin-3-yl)-pipéridin-1-yl]-1-(3,4,5,6-tétrahydro-2H-4,4'-bipyridinyl-1-yl)-butane-1,4-dione,
(50) la (*S*)-2-[4-amino-3-chloro-5-trifluorométhyl-benzyl)-4-[4-(2-oxo-1,2,4,5-tétrahydro-1,3-bemodiazépin-3-yl)-pipéridin-1-yl]-1-(4-pyridin-4-yl-pipérazin-1-yl)-butane-1,4-dione,
(51) la (S)-2-{4-amino-3-chloro-5-trifluorométhyl-benzyl)-4-[4-(2-oxo-1,2,4,5-tétrahydro-1,3-benzodiazépin-3-yl)-pipéridin-1-yl]-1-(3-perhydro-azépin-1-yl-azétidin-1-yl)-butane-1,4-dione,
(52) la (S)-2-(4-amino-3-chloro-5-trifluorométhyl-benzyl)-1-[4-(1-benzyl-pipéridin-4-yl)-pipérazin-1-yl]-4-[4-(2-oxo-1,2,4,5-tétrahydro-1,3-benzodiazépin-3-yl)-pipéridin-1-yl]-butane-1,4-dione,
(53) la (S)-2-(4-amino-3-chloro-5-trifluorométhyl-benzyl)-1-[4-(4-benzyl-pipérazin-1-yl)-pipéridin-1-yl]-4-[4-(2-oxo-1,2,4,5-tétrahydro-1,3-benzodiazépin-3-yl)-pipéridin-1-yl]-butane-1,4-dione,
(54) la (S)-2-(4-amino-3-chloro-5-trifluorométhyl-benzyl)-1-(7-diméthylaminométhyl-1,2,4,5-tétrahydro-3-benzazépin-3-yl)-4-[4-(2-oxo-1,2,4,5-tétrahydro-1,3-benzodiazépin-3-yl)-pipéridin-1-yl)-butane-1,4-dione,
(55) la (S)-2-(4-amino-3-chloro-5-trifluorométhyl-benzyl)-1-[4-(4-diméthylaminométhyl-phényl)-pipéridin-1-yl]-4-[4-(2-oxo-1,2,4,5-tétrahydro-1,3-benzodiazépin-3-yl)-pipéridin-1-yl]-butane-1,4dione,
(56) la (S)-2-(4-amino-3-chloro-5-trifluorométhyl-benzyl)-4-[4-(2-oxo-1,2,4,5-tétrahydro-1,3-benzodiazépin-3-yl)-pipéridin-1-yl]-1-{4-[4-(2,2,2 trifluoro-éthyl)-pipéiazin-1-yl]-pipéridin-1-yl}-butane-1,4-dione,
(57) la (S)-2-(4-amino-3-chloro-5-trifluorométhyl-benzyl)-1-(1'-mémansulfonyl-4-4'-bipipéridinyl-1-yl)-4-[4-(2-xo-1,2,4,5-tétrahydro-1,3-benzodiazépin-3-yl)-pipéridin-1-yl]-butane-1,4-dione,
(58) la (S)-2-(4-amino-3-chloro-5-trifluorométhyl-benzyl)-1-(9-méthyl-3,9-diazaspiro[5.5]undéc-3-yl)-4-[4-(2-oxo-1,2,4,5-tétrahydro-1,3-benzodiazépin-3-yl)-pipéridin-1-yl]-butane-1,4-dione,
(59) la (S)-2-(4-amino-3-chloro-5-truluorométhyl-benzyl)-4-[4-(2-oxo-1,2,4,5-tetrahydro-1,3-benzodiazépin-3-yl)-pipéridin-1-yl]-1-(4-pipéridin-1-yl-méthyl-pipéridin-1-yl)-butane-1,4-dione,
(60) la (S)-2-(4-amino-3-chloro-5-trifluorométhyl-benzyl)-1-[4-(2-diméthylamino-éthyl)-pipéridin-1-yl]-4-[4-(2-oxo-1,2,4,5-tétrahydro-1,3-benzodiazépin-3-yl)-pipéridin-1-yl)-butane-1,4-dione,
(61) le (S)-2-(4-aqmino-3-chloro-5-trifluorométhyl-benzyl)-N-méthyl-N-[2-(1-méthyl-pipéridin-4-yl)-éthyl]-4-oxo-4-[4-(2-oxo-1,2,4,5-tétrahydro-1,3-benzodiazépin-3-yl)-pipéridin-1-yl]-butyramide,
(62) le (S)-2-(4-amino-3-chloro-5-trifluorométhyl-benzyl)-N-méthyl-N-(1-méthyl-pipéridin-4-ylméthyl)-4-oxo-4-[4-(2-oxo-1,2,4,5-tétrahydro-1,3-benzodiazépin-3-yl)-pipéridin-1-yl]-butyramide,
(63) la (S)-(4-amino-3-chloro-5-trifluorométhyl-benzyl)-4-[4-(2-oxo-1,2,4,5-tétrahydro-1,3-benzodiazépin-3-yl)-pipéridin-1-yl]-1-pipéridin-1-yl0-butane-1,4-dione
(64) la (S)-2-(4-amino-3-chloro-5-trifluorométhyl-benzyl)-4-[4-(2-oxo-1,2,4,5-tétrahydro-1,3-benzodiazépin-3-yl)-pipéridin-1-yl]-1-(4-propyl-pipéridin-1-yl)-butane-1,4-dione,
(65) la (S)-2-(4-amino-3-chloro-5-triffuorométhyl-benzyl)-1-(4-benzyl-pipéridin-1-yl)-4-[4-(2-oxo-1,2,4,5-tétrahydro-1,3-benzodiazépin-3-yl)-pipéridin-1-yl]-butane-1,4-dione,
(66) la (S)-2-(4-anùno-3-chloro-5-trifluorométhyl-benzyl)-1-[4-(2-diéthylamino-éthyl)-pipéànyl-1-yl]-4-[4-(2-oxo-1,2,4,5-tétrahydro-1,3-benzodiazépin-3-yl)-pipéridin-1-yl]-butane-1,4-dione,
(67) la (S)-2-(4-amino-3-chloro-5-trifluorométhyl-benzyl)-1-(3-aza-spiro[5.5]undéc-3-yl)-4-[4-(2-oxo-1,2,4,5-tétrahydro-1,3-benzodiazépin-3-yl)-pipéridin-1-yl]-butane-1,4-dione,
(68) le N-(1-{(S)-2-(4-amino-3-chloro-5-trifluorométhyl-benzyl)-4-oxo-4-[4-(2-oxo-1,2,4,5-tétrahydro-1,3-benzodiazépin-3-yl)-pipéridin-1-yl]-butyryl}-pipéridin-4-yl)-N-méthyl-méthanesulfonamide,
(69) le N-(1-{(S)-2-(4-amino-3-chloro-5-trifluorométhyl-benzyl)-4-oxo-4-[4-(2-oxo-1,2,4,5-tétrahydro-1,3-benzodiazépin-3-yl)pipéridin-1-yl]-butyryl}-pipéridin-4-yl)-méthanesulfonamide,
(70) le (S)-2-(4-amino-3-chloro-5-trifluorométhyl-benzyl)-1-[4-(cyclopentyl-méthylamino)-pipéridin-1-yl]-4-[4-(2-oxo-1,2,4,5-tétrahydro-1,3-benzodiazépin-3-yl)-pipéridin-1-yl]-butane-1,4-dione,
(71) la (S)-2-(4-amino-3-chloro-5-trifluorométhyl-benzyl)-1-(4-méthyl-piperidin-1-yl)-4-[4-(2-oxo-1,2,4,5-tétrahydro-1,3-benzodiazépin-3-yl)-pipéridin-1-yl]-butane-1,4-dione,
(72) l'ester méthylique de l'acide (1-{(S)-2-(4-amino-3-chloro-5-trifluorométhyl-benzyl)-4-oxo-4-[4-(2-oxo-1,2,4,5-tétrahydro-1,3-benzodiazépin-3-yl)-pipéridin-1-yl]-butyryl}-pipéridin-4-yl)-acétique,
(73) la (S)-2-(4-amino-3-chloro-5-trifluorométhyl-benzyl)-1-(4-hydroxy-pipéridin-l-yl)-4-[4-(2-oxo-1,2,4,5-tétrahydro-1,3-benzodiazépin-3-yl)-pipéridin-1-yl]-butane-1,4-dione,
(74) la (S)-2-(4-amino-3-chloro-5-trifluorométhyl-benzyl)-4-[4-(2-oxo-1,2,4,5-tétrahydro-1,3-benzodiazépin-3-yl)-pipéridin-1-yl]-1-(4-trifluorométhyl-pipéridin-1-yl)-butane-1,4-dione,
(75) la (S)-2-(4-amino-3-chloro-5-trifluorométhyl-benzyl)-1-[4-(1,1-dioxo-1,6-isothiazolidin-2-yl)-pipéridin-1-yl]-4-[4-(2-oxo-1,2,4,5-tétrahydro-1,3-benzodiazépin-3-yl)-pipéridin-1-yl]-butane-1,4-dione,
(76) la (S)-2-(4-amino-3-chloro-5-trifluorométhyl-benzyl)-1-[4-(2-oxo-perhydro-1,3-oxazin-3-yl)-pipéridin-1-yl]-4-[4-(2-oxo-1,2,4,5-tétrahydro-1,3-benzodiazépin-3-yl)-pipéridin-1-yl]-butane-1,4-dione,
(77) l'ester méthylique de l'acide 1-{(S)-2-(4-amino-3-chloro-5-üifluorométhyl-benzyl)-4-oxo-4-[4-(2-oxo-1,2,4,5-tétrahydro-1,3-benzodiazépin-3-yl)-pipéridin-1-yl]-butyryl}-pipéridin-4-carboxylique,
(78) la (S)-2-(4-amino-3-chloro-5-trifluorométhyl-benzyl)-1-(4-cyclohexyl-pipéridin-1-yl)-4-[4-(2-oxo-1,2,4,5-tétrahydro-1,3-benzodiazépin-3-yl)-pipéridin-1-yl]-butane-1,4-dione,
(79) la (S)-2-(4-ammo-3-chloro-5-trifluorométhyl-benzyl)-1-(4-tert-butylamino-pipéridin-1-yl)-4-[4-(2-oxo-1,2,4,5-tétrahydro-1,3-benzodiazépin-3-yl)-pipéridin-1-yl]-butane-1,4-dione,
(80) la (S)-2-(4-amino-3-chloro-5-trifluoromémthyl-benzyl)-4-[4-(2-oxo-1,2,4,5-tétrahydro-1,3-benzodiazépin-3-yl)-pipéridin-1-yl]-1-(4-phényl-pipéridin-1-yl)-butane-1,4-dione,
(81) la (S)-2-(4-amino-3-chloro-5-trifluorométhyl-benzyl)-4-[4-(2-oxo-1,2,4,5-tetrahydro-1,3-benzodiazépin-3-yl)-pipéridin-1-yl]-1-(4-p-tolyl-pipéridin-1-yl)-butane-1,4-dione,
(82) la {(S)-2-(4-amino-3-chloro-5-trifluorométhyl-benzyl)-4-oxo-4-[4-(2-oxo-1,2,4,5-tetrahydro-1,3-benzodiazépin-3-yl)-pipéridin-1-yl]-butyryl}-1,3,8-triazaspiro[4.5]décan-2,4-dione,
(83) la (S)-2-(4-amino-3-chloro-5-trifluorométhyl-benzyl)-1-[4-(2-oxo-imidazolidin-1-yl)-pipéridùi-1-yl]-4-[4-(2-oxo-1,2,4,5-téùahydro-1,3-benzodiazépin-3-yl)-pipéridin-1-yl]-butane-1,4-dione,
(84) la (S)-2-(4-amino-3-chloro-5-trifluorométhyl-benzyl)-1-(4-amino-4-méthyl-pipéridin-1-yl)-4-[4-(2-oxo-1,2,4,5-tétrahydro-1,3-benzodiazépin-3-yl)-pipéridin-1-yl]-butane-1,4-dione,
(85) la (S)-2-(4-amino-3-chloro-5-trifluorométhyl-benzyl)-1-[4-(1-méthyl-pipéridin-4-yl)-pipérazin-1-yl]-4-[4-(2-oxo-1,2,4,5-téahydro-1,3-benzodizépin-3-yl)-pipéridin-1-yl]-butane-1,4-dione,
(86) la (S)-2-(4-amino-3-chloro-5-trifluorométhyl-benzyl)-1-(2-amino-4,5,7,8-tétrahydro-thiazolo[4,5-d]azépin-6-yl)-4-[4-(2-oxo-1,2,4,5-tétrahydro-1,3-benzodiazépin-3-yl)-pipéridin-1-yl]-butane-1,4-dione,
(87) la (S)-2-(4-amino-3-chloro-5-trifluorométhyl-benzyl)-1-[4-(2-méthyl-imidazol-1-yl)-pipéridin-1-yl]-4-[4-(2-oxo-1,2,4,5-tétrahydro-1,3-benzodiazépin-3-yl)-pipéridin-1-yl]-butane-1,4-dione,
(88) la (S)-2-(4-amino-3-chloro-5-trifluorométhyl-benzyl)-1-[4-(4-méthyl-imidazol-1-yl)-pipéridin-1-yl)-4-(4-(2-oxo-1,2,4,5-tétrahydro-1,3-benzodiazépin-3-yl)-pipéridin-1-yl]-butane-1,4-dione,
(89) la (S)-2-(4-amino-3-chloro-5-trifluorométhyl-benzyl)-4-[4-(2-oxo-1,2,4,5-tétrahydro-1,3-benzodiazépin-3-yl)-pipéndin-1-yl]-1-(4-thiazol-2-yl-pipérazin-1-yl)-butane-1,4-dione,
(90) la (S)2-(4-amino-3-chloro-5-trifluorométhyl-benzyl)-1-[4-(2,4-diméthyl-imidazol-1-yl)-pipéridin-1-yl]-4-[4-(2-oxo-1, 2,4,5-tétrahydro-1,3-benzodiazépin-3-yl)-pipérdin-1-yl]-butane-1,4-dione,
(91) (la S)-2-(4-amho-3-chloro-5-trifluométhyl-benzyl)-1-(4-imidazol-1-yl]-pipéàdin-1-yl)-4-[4-(2-oxo-1,2,4,5-tétrahydro-1,3-benzodiazépin-3-yl)-pipéridin-1-yl]-butane-1,4-dione,
(92) la (S)-2-(4-ammo-3-chloro-5-trifluorométhyl-benzyl)-4-[4-(2-oxo-1,2,4,5-tetrahydro-1,3-benzodiazépin-3-yl)-pipéridin-1-yl]-1-(4-1,2,4-triazol-1-yl-pipéridin-1-yl)-butane-1,4-dione,
(93) la (S)-2-(4-amino-3-chloro-5-trifluorométhyl-benzyl)-1-[4-(1-aza-bicyclo[2.2.2]oct-3-yl)-pipérazin-1-yl]-4-[4-(2-oxo-1,2,4,5-tétrahydro-1,3-benzodiazépin-3-yl)-pipéridin-1-yl]-butane-1,4-dione,
(94) la (S)-2-(4-amino-3-chloro-5-trifluorométhyl-benzyl)-4-[4-(2-oxo-1,2,4,5-tétrahydro-1,3-benzodiazépin-3-yl)-pipéridin-1-yl]-1-pipérazin-1-yl-butane-1,4-dione,
(95) le (1-méthyl-pipéridin-4-yl)-amide de l'acide 4-{(S))-2-4-amino-3-chloro-5-trifluorométhyl-benzyl)-4-oxo-4-[4-(2-oxo-1,2,4,5-tétrahydro-1,3-benzodiazépin-3-yl)-pipéridin-1-yl]-butyryl}-pipérazin-1-sulfonique,
(96) le (S)-2-(4-amino-3-chloro-5-trifluorométhyl-benzyl)-N-(5-amino-pentyl)4-oxo-4-[4-(2-oxo-1,2,4,5-tétrahydro-1,3-benzodiazépin-3-yl)-pipéridin-1-yl]-butyramide,
(97) le (S)-2-(4-amino-3-chloro-5-trifluorométhyl-benzyl)-N-(3-aminométhyl-benzyl)-4-oxo-4-[4-(2-oxo-1,2,4,5-tétrahydro-1,3-benzodiazépin-3-yl)-pipéridin-1-yl]-buyramide,
(98) l'acide 1 {(S)-2-(4-amino-3-chloro-5-trifluorométhyl-benzyl)4-oxo-4-[4-(2-oxo-1,2,4,5-tétrahydro-1,3-benzodiazépin-3-yl)-pipéridin-1-yl]-butyryl}-pipéridin-4-carboxylique,
(99) l'acide (1-{(S)-2-(4-amino-3-chloro-5-trifluorométhyl-benzyl)-4-oxo-4-[4-(2-oxo-1,2,4,5-tétrahydro-1,3-benzodiazépin-3-yl)-pipéridin-1-yl]-butyryl}-pipéridin-4-yl)-acétique,
(100) la (S)-2-(4-amino-3-chloro-5-trifluorométhyl-benzyl)-1-[4-(8-méthyl-8-azabicyclo[3.2.1]oct-3-yl)-pipérazin-1-yl]4-[4-(2-oxo-1,2,4,5-tétrahydro-1,3-benzodiazépin-3-yl)-pipéridin-1-yl]-butane-1,4-dione,
(101) la (S)-2-(4-amino-3-chloro-5-trifluorométhyl-benzyl)-4-[4-(2-oxo-1,2,4,5-tétrahydro-1,3-benzodiazépin-3-yl)-pipéridin-1-yl]-1-(4-pipérazin-1-yl-pipéridin-1-yl)-butane-1,4-dione,
(102) la (S)-2-(4-amino-3-chloro-5-trifluorométhyl-benzyl)-1-[4-(1H-imidazol-4-yl)-pipéridin-1-yl]-4-[4-(2-oxo-1,2,4,5-tétrahydro-1,3-benzodiazépin-3-yl)-pipéridin-1-yl]-butane-1,4-dione,
(103) la (S)-2-(4-amino-3-chloro-5-trifluorométhyl-benzyl)-4-[4-(2-oxo-1,2,4,5-tétrahydro-1,3-benzodiazépin-3-yl)-pipéridin-1-yl]-1-{4-[4-(2,2,2-trifluoroacétyl)-phényl]-pipérazin-1-yl}-butane-1,4-dione,
(104) la (S)-2-(4-amino-3-chloro-5-trifluorométhyl-benzyl)-1-[4-(4-méthyl-pipérazin-1-yl)-pipéridin-1-yl]-4-[4-(2-oxo-1,4-dihydro-2H-quinazolin-3-yl)-pipéridin-1-yl]-butane-1,4-dione,
(105) la (S-2-(4-amino-3-chloro-5-trifluorométhyl-benzyl)-1-[4-(4-méthyl-pipérazin-1-yl)-pipéridin-1-yl]-4-[4-(5-oxo-3phényl-4,5-dihydro-1,2,4-triazol-1-yl)-pipéridin-1-yl]-butane-1,4-dione,
(106) la (S)-2-(4-amino-3-chloro-5-trifluorométhyl-benzyl)-1-[4-(4-méthyl-pipérazin-1-yl)-pipéridin-1-yl]-4-[4-(2-oxo-1,2-dihydm-imidazo[4,5-c)quinoléine-3-yl)-pipéàdin-1-yl]-butane-1,4-dione,
(107) la (S)-2-(4-amino-3-chloro-5-trifluorométhyl-benzyl)-1-[4-(4-méthyl-pipérazin-1-yl)-pipéridin-1-yl]-4-[4-(2-oxo-1,2-dihydro-4H-thiéno[3,4-dipyrimidin-3-yl)-pipéridin-1-yl]-butane-1,4-dione,
(108) la (S)-2-(4-amino-3-chloro-5-trifluorométhyl-benzyl)-1-[4-(5-méthyl-2,5-diazabicyclo[2,2,1]hept-2-yl)-pipéridin-1-yl]-[4-(2-oxo-1,2,4,5-tétrahydro-1,3-benzodiazépin-3-yl)-pipéridin-1-yl]-butane-1,4-dione,
(109) la (S)-2-(4-amino-3,5-bis-trifluorométhyl-benzyl)-1-[4-(4-méthyl-perhydro-1,4-diazépin-1-yl)-pipéridin-1-yl]-4-[4-(2-oxo-1,2,4,5-tétrahydro-1,3-benzodiazépin-3-yl)-pipéridin-1-yl]-butane-1,4,-dione,
(110) la (*S*)-2-(4-amino-3,5-bis-trifluorométhyl-benzyl)-1-[4-(1-méthyl-pipéridin-4-yl)-perhydro-1,4-diazépin-1-yl]-4-[4-(2-oxo-1,2,4,5-tétrahydro-1,3-benzodiazépin-3-yl)-pipéridin-1-yl]-butane-1,4-dione,
(111) la (S)-2-(4-amino-3,5-bis-trifluorométhyl-benzyl)-1-[4-(1-méthyl-pipéridin-4-yl)-pipérazin-1-yl]-4-[4-(2-oxo-1,2,4,5-tétrahydro-1,3-benzodiazépin-3-yl)-pipéridin-1-yl]-butane-1,4-dione,
(112) la (S)-2-(4-amino-3,5-bis-trifluorométhyl-benzyl)-1-[4-(1-aza-bicyclo[2.2.2]oct-3-yl)-pipérazin-1-yl]-4-[4-(2-oxo-1,2,4,5-tétrahydro-1,3-benzodiazépin-3-yl)-pipéridin-1 -yl]-butane-1,4-dione,
(113) la (S)-2-(4-amino-3,5-bis-trifluorométhyl-benzyl)-1-[4-(4-méthyl-pipérazin-1-yl)-pipéridin-1-yl]-4-[4-(2-oxo-1,2,4,5-tétrahydro-1,3-benzodiazépin-3-yl)-pipéridin-1-yl]-butane-1,4-dione,
(114) la (S)-2-(4-amino-3,5-bis-trifluorométhyl-benzyl)-4-[4-(2-oxo-1,2,4,5-tétrahydro-1,3-benzodiazépin-3-yl)-pipéndin-1-yl]-1-(4-perhydro-azépin-1-yl-pipéndm-1-yl)-butane-1,4-dione,
(115) la (S)-2-(4-amino-3,5-bis-trifluorométhyl-benzyl)-1-1,4'-bipipéridinyl-1'-yl-4-[4-(2-oxo-1,2,4,5-tétrahydro-1,3-benzodiazépin-3-yl)-pipéridin-1-yl]-butane-1,4-dione,
(116) la (S)-2-(4-amino-3,5-bis-trifluorométhyl-benzyl)-4-[4-(2-oxo-1,2,4,5-tétrahydro-1,3-benzodiazépin-3-yl)-pipéridin-1-yl]-1-(4-pipérazin-1-yl-pipéridin-1-yl)-butane-1,4-dione,
(117) le {(R)-1-(4-amino-3,5-bis-trifluorométhyl-benzyl)-2-[4-(4-méthyl-pipérazin-1-yl)-pipéridin-1-yl]-2-oxo-éthyl)-amide de l'acide 4-(2-oxo-1,2,4,5-tétrahydro-1,3-benzodiazépin-3-yl)-pipéridin-1-carboxylique,
(118) le {(R)-1-(4-amino-3,5-bis-trifluorométhyl-benzyl)-2-[4-(1-méthyl-pipéridin-4-yl)-pipérazin-1-yl]-2-oxo-éthyl)-amide de l'acide 4-(2-oxo-1,2,4,5-tetrahydro-1,3-benzodiazepin-3-yl)-pipéridin-1-carboxylique,
(119) le [(R)-1-(4-amino-3,5-bis-truluorométhyl-benzyl)-2-1,4-bipipéridinyl-1'-yl-2-oxo-éthyl]-amide de l'acide 4-(2-oxo-1,2,4,5-tétrahydro-1,3-benzodiazépin-3-yl)pipéridin-1 carboxylique,
(120) le [(R)-1-(4-amino-3,5-bis-trifluorométhyl-benzyl)-2-oxo-2-(4-pipérazin-1-yl]-pipéridin-1-yl)-éthyl]-amide de l'acide 4-(2-oxo-1,2,4,5-tétrahydro-1,3-benzodiazépin-3-yl)-pipéridin-1-carboxylique,
(121) l'ester (R)-1-(4-amino-3-chloro-5-trifluorométhyl-benzyl)-2-[4-(4-méthyl-pipérazin-l-yl)-pipéridin-1-yl]-2-oxo-éthylique de l'acide 4-(2-oxo-1,2,4,5-tétrahydro-1,3-benzodiazépin-3-yl)-pipéridin-1-carboxylique,
(122) le [2-[1,4]bipipéridinyl-1'-yl-1-(4-bromo-3-méthyl-benzyl)-2-oxo-éthyl]-amide de l'acide 4-(2-oxo-1,4-dihydro-2H-quinazolin-3-yl)-pipéndin-1-carboxylique,
(123) le [1-(4-bromo-3-méthyl-benzyl)-2-(1'-méthyl-[4,4]bipipéridinyl-1-yl)-2-oxo-éthyl]-amide de l'acide 4-(2-oxo-1,4-dihydro-2H-quinazolin-3-yl)-pipéridin-carboxlylique,
(124) le {1-(4-bromo-3-méthyl-benzyl)-2-[4-(4-méthyl-pipérazin-1-yl)-pipéridin-1-yl]-2-oxo-éthyl}-amide de l'acide 4-(2-oxo-1,4-dihydro-2H-quinazolin-3-yl)-pipéridin-1-carboxylique,
(125) le {1-(4-bromo-3-méthyl-benzyl)-2-[4-(1-méthyl-pipéridin4-yl)-pipérazin-1-yl]-2-oxo-éthyl}-amide de l'acide 4-(2-oxo-1,4-dihydro-2H-quinazolin-3-yl)-pipéridin-1-carboxylique,
(126) l'ester éthylique de l'acide {4-[1-(3-(4-bromo-3-méthyl-phényl)-2-{[4-(2-oxo-1,4-dihydro-2H-quinazolin-3-yl)-piperidin-1-carbonyl]-amino}-propionyl)-pipéridin-4-yl]-pipér a z i n-1-acétique,
(127) l'ester éthylique de l'acide [1-(3-(4-bromo-3-méthyl-phényl-2-{[4-(2-oxo-1,4-dihydro-2H-quinazolin-3-yl)-pipéridin-1-carbonyl]-amino}-propionyl)-[4,4'] 1bipipéridinyl-1-yl]-acétique,
(128) l'ester éthylique de l'acide {4-[4-(3-(4-bromo-3-méthyl-phényl-2-{[4-(2-oxo-1,4-dihydro-2H-quinazolin-3-y)-pipéridin-1-carbonyl]-amino}-propionyl)-pipérazin-1-yl]-pipéridin-1-yl}-acétique,
(129) l'acide {4-[1-(3-(4-bromo-3-méthyl-phényl)-2-{[4-(2-oxo-1,4-dihydro-2H-quinazolin-3-yl)-pipéridin-1-carbonyl]-amino}-propionyl)-pipéridin-4-yl]-pipérazin-1-yl}-acétique,
(130) l'acide [1'-(3-(4-bromo-3-méthyl-phényl-2-{[4-(2-oxo-1,4-dihydro-2H-quinazolin-3-yl)-pipéridin-1-carbonyl]-amino}-propionyl)-[4,4']bipipéridinyl-1-yl]-acétique,
(131) l'acide {4-[4-(3-(4-bromo-3-méthyl-phényl)-2-{[4-(2-oxo-1,4-dihydro-2H-quinazolin-3-yl)-pipéridin-1-carbonyl]-amino-propionyl)-pipérazin-1-yl]-pipéridin-1-yl}-acétique,
(132) la 2-(4-bromo-3-méthyl-benzyl)-1-[4-(4-méthyl-pipérazin-1-yl)-pipéridin-1-yl]-4-[4-(2-oxo-1,4-dihydro-2H-quinazolin-3-yl)-pipéridin-1-yl]-butane-1,4-dione,
(133) la 2-(4-bromo-3-méthyl-benzyl)-1-(1'-méthyl-[4,4']bipipéridinyl-1-yl)-4[4-(2-oxo-1,4-dihydro-2H-quinazolin-3-yl)-pipéridin-1-yl]-butane-1,4-dione,
(134) la 1-[1,4']bipipéridinyl-l'-yl-2-(4-bromo-3-méthyl-benzyl)-4-[4-(2-oxo-1,4-dihydro-2H-quinazolin-3-yl)-pipéridin-1-yl]-butane-1,4-dione,
(135) la 2-(4-bromo-3-méthyl-benzyl)-1-{4-[4-(3-diméthylamino-propyl)-phényl]-pipérnzin-1-yl}-4-[4-(2-oxo-1,4-dihydro-2H-quinazolin-3-yl)-pipéridin-1-yl]-butane-1,4-dione,
(136) l'acide [4-(1-{2-(4-bromo-3-méthyl-benzyl)-4-oxo-4-[4-(2-oxo-1,4-dihydro-2H-quinazolin-3-yl)-pipéridin-1-yl]-butyryl}-pipéridin-4-yl)-pipérazin-1-yl]-acétique,
(137) l'ester méthylique de l'acide (1'-{2-(4-bromo-3-méthyl-benzyl)-4-oxo-4-[4-(2-oxo-1,4-dihydro-2H-quinazolin-3-yl)-pipéridin-1-yl]-butyryl}-[4,4']bipipéridinyl-1-yl)-acétique,
(138) l'acide (1'-{2-(4-bromo-3-méthyl-benzyl)-4-oxo-4-[4-(2-oxo-1,4-dihydro-2H-quinazolin-3-yl)-pipéridin-1-yl]-butyryl}-[4,4']bipipéridinyl-1-yl)-acétique,
(139) le {1-(4-chloro-3-méthyl-benzyl)-2-[4-(4-méthyl-pipérazin-1-yl)-pipéridin-1-yl]-2-oxo-éthyl}-amide de l'acide 4-(2-oxo-1,4-dihydro-2H-quinazolin-3-yl)-pipéridin-1-carboxylique,
(140) le [2-[1,4']bipipéridinyl-1'-yl-1-(4-chloro-3-méthyl-benzyl)-2-oxo-éthyl]-amide de l'acide 4-(2-oxo-1,4-dihydro-2H-quinazolin-3-yl)-pipéridin-1-carboxylique,
(141) le {1-(4-chloro-3-méthyl-benzyl)-2-[4-(1-méthyl-pipéridin-4-yl)-pipérazin-1-yl]-2-oxo-éthyl}-amide de l'acide 4-(2-oxo-1,4-dihydro-2H-quinazolin-3-yl)-pipéridin-1-carboxylique,
(142) le [1-(4-chloro-3-méthyl-benzyl)-2-(1-méthyl-[4,4']bipipéridinyl-1-yl)-2-oxo-éthyl]-amide de l'acide 4-(2-oxo-1,4-dihydro-2H-quinazolin-3-yl)-pipéridin-1-carboxylique,
(143) l'ester éthylique de l'acide [1'-(3-(4-chloro-3-méthyl-phényl)-2-{[4-(2-oxo-1,4-dihydro-2H-quinazolin-3-yl)-pipéridin-1 -carbonyl]-amino}-propionyl)-[4,4']bipipéridinyl-1-yl}-acétique,
(144) l'ester tert-butylique de l'acide {4-[1-(3-(4-chloro-3-méthyl-phényl)-2-{[4-(2-oxo-1,4-dihydro-2H-quinazolin-3-yl)-pipéridin-1-carbonyl]-amino}-propionyl)-pipéridin-4-yl]-pipérazin-1-yl}-acétique,
(145) le {1-(4-chloro-3-méthyl-benzyl)-2-oxo-2-[1'-(2,2,2-trifluoro-éthyl)-[4,4']bipipéridinyl-1-yl]-éthyl}-amide de l'acide 4-(2-oxo-1,4-dihydro-2H-quinazolin-3-yl)-pipéridin-1-carboxylique,
(146) le (1-(4-chloro-3-méthyl-benzyl)-2-oxo-2-{4-[4-(2,2,2-trifluoro-éthyl)-pipérazin-1-yl]-pipéridin-1-yl}-éthyl)-amide de l'acide 4-(2-oxo-1,4-dihydro-2H-qumazolin-3-yl)-pipéridin-1-carboxylique,
(147) l'acide [1'-(3-(4-chloro-3-méthyl-phényl)-2-{[4-(2-oxo-1,4-dihydro-2H-quinazolin-3-yl)-pipéridin-1-carbonyl]-amino}-propionyl)-[4,4]bipipéridinyl-1-yl]-acétique,
(148) la 2-(4-chloro-3-méthyl-benzyl)-I-[4-(4-éthyl-pipérazin-l-yl)-pipéridin-l-yl]-4-[4-(2-oxo-1,4-dihydro-2H-quinazolin-3-yl)-pipéridin-1-yl]-butane-1,4-dione,
(149) la 2-(4-chloro-3-méthyl-benzyl)-1-(l'-méthyl-[4,4']bipipéridinyl-1-yl)-4-[4-(2-oxo-1,4-dihydro-2H-quinazolin-3-yl)-pipéridin-1-yl]-butane-1,4-dione,
(150) la 1-[1,4']bipipéridinyl-1'-yl-2-(4-chloro-3-méthyl-benzyl)-4-[4-(2-oxo-1,4-dihydro-2H-chinazoiin-3-yl)-pipéridin-l -yl]-butane-1,4-dione,
(151) la 2-(4-chloro-3-méthyl-benzyl)-1-[4-(4-méthyl-pipérazin-1-yl)-pipéridin-1-yl]-4-[4-(2-oxo-1,4-dihydro-2H-quinazolin-3-yl)-pipéridin-1-yl]-butane-1,4-dione,
(152) la 2-(4-cNoro-3-méthyl-benzyl)-1-[4-(1-méthyl-pipéridin-4-yl)pipérazin-1-yl]-4-[4-(2-oxo-1,4-dihydro-2H-quinazolin-3-yl)-pipéridin-1-yl]-butane-1,4-dione,
(153) la 2-(4-chloro-3-méthyl-benzyl)-1-[4-(4-méthanesulfonyl-pipérazin-1-yl)-pipéridin-1-yl]-4-[4-(2-oxo-1,4-dihydro-2H-quinazolin-3-yl)-pipéridin-1-yl]-butane-1,4-dione,
(154) la 2-(4-chloro-3-méthyl-benzyl)-1-[4-(4-isopropyl-pipérazin-1-yl)-pipéridin-1-yl]-4-[4-(2-oxo-1,4-dihydro-2H-quinazolin-3-yl)-pipéridin-1-yl]-butane-1,4-dione,
(155) l'ester éthylique de l'acide 1-{2-(4-chloro-3-méthyl-benzyl)-4-oxo-4-[4-(2-oxo-1,4-dihydro-2H-quinazolin-3-yl)-pipéridin-1-yl]-butytyl}-4-(1-méthyl-pipéridin-4-yl)-pipérazin-2-carboxylique,
(156) l'ester éthylique de l'acide 1-(1-{2-(4-chloro-3-méthyl-benzyl)-4-oxo-4-[4-(2-oxo-1,4-dihydro-2H-quinazolin-3-yl)-pipéridin-1-yl]-butyryl}-pipéridin-4-yl)-4-méthyl-pipérazin-2-carboxylique,
(157) l'ester éthylique de l'acide 4-(1-{2-(4-chloro-3-méthyl-benzyl)-4-oxo-4-[4-(2-oxo-1,4-dihydro-2H-quinazolin-3-yl)-pipéridin-1-yl]-butyryl}-pipéridin-4-yl)-1-méthyl-pipérazin-2-carboxylique,
(158) l'ester éthylique de l'acide 4-{2-(4-chloro-3-méthyl-benzyl)-4-oxo-4-[4-(2-oxo-1,4-dihydro-2H-quinazolin-3-yl)-pipéridin-1-yl]-butyryl}-1-(1-méthyl-pipéridin-4-yl)-pipérazin-2-carboxylique,
(159) la 2-(4-chloro-3-méthyl-benzyl)-4-[4-(2-oxo-1,4-dihydro-2H-quinazolin-3-yl)-pipéridin-1-yl]-1-[1'-(2,2,2-trifluoro-éthyl)-[4,4']bipipéridinyl-1-yl)-butane-1,4-dione,
(160) la 2-(4-chloro-3-méthyl-benzyl)-4-[4-(2-oxo-1,4-dihydro-2H-quinazolin-3-yl)-pipéridirrl-yl]-1-{4-[4-(2,2,2-triffuoro-éthyl)pipérazin-1-yl]pipéridin-1-yl}-butane-1,4-dione,
(161) l'acide [4-(1-{2-(4-chloro-3-méthyl-benzyl)-4-oxo-4-[4-(2-oxo-1,4-dihydro-2H-quinazolin-3-yl)-pipéridin-1-yl]-butyryl}-pipéndm-4-yl)-pipérazin-1-yl]-acétique,
(162) l'ester méthylique de l'acide (1'-{2-(4-chloro-3-méthyl-benzyl)-4-oxo-4-[4-(2-oxo-1,4-dihydro-2H-quinazolin-3-yl)-pipéridin-1-yl]-butyryl}-[4,4]bipipéridinyl-1-yl)-acétique,
(163) l'acide (1'-{2-(4-chloro-3-méthyl-benzyl)-4-oxo-4-[4-(2-oxo-1,4-dihydro-2H-quinazolin-3-yl)-pipéridin-1-yl]-butyryl}-[4,4']bipipéridinyl-1-yl)-acétique,
(164) l'acide 1-{2-(4-chloro-3-méthyl-benzyl)-4-oxo-4-[4-(2-oxo-1,4-dihydro-2H-quinazolin-3-yl)-péridin-1-yl]-butyryl}-4-(1-méthyl-pipéridin-4-yl)-pipérazin-2-carboxylique,
(165) l'acide 1-(1-{2-(4-chloro-3-méthyl-benzyl)-4-oxo-4-[4-(2-oxo-1,4-dihydro-2H-quinazolin-3-yl)-pipéridin-1-yl]-butyryl}-pipéridin-4-yl)-4-méthyl-pipérazin-2-carboxylique,
(166) l'acide 4-(1-{2-(4-chloro-3-méthyl-benzyl)-4-oxo-4-[4-(2-oxo-1,4-dihydro-2H-quinazolin-3-yl)-pipéridin-1-yl]-butyryl}-pipéridin-4-yl)-1-méthyl-pipérazin-2-carboxylique,
(167) la 2-(4-chloro-3-méthyl-benzyl)-1-[4-(1-méthyl-pipéridin-4-yl)-pipérazin-1-yl]-4-[4-(2-oxo-1,2,4,5-tétrahydro-1,3-benzodiazépin-3-yl)-pipéridin-1-yl]-butane-1,4-dione,
(168) la 2-(4-chloro-3-méthyl-benzyl)-1-[4-(4-méthyl-pipérazin-1-yl)-pipéridin-1-yl]-4-[4-(2-oxo-1,2,4,5-tétrahydro-1,3-benzodiazépin-3-yl)-pipéridin-1-yl]-butane-1,4-dione,
(169) l'acide [4-(1-{2-(4-chloro-3-méthyl-benzyl)-4-oxo-4-[4-(5-oxo-3-phényl-4,5-dihydro-1,2,4-triazol-1-yl)-pipéridin-1-yl]-butyryl}-pipéridin-4-yl)-pipérazin-1-yl]-acétique,
(170) l'ester méthylique de l'acide (1'-{2-(4-chloro-3-méthyl-benzyl)-4-oxo-4-[4-(5-oxo-3-phényl-4,5-dihydro-1,2,4-triazol-1-yl)-pipéridin-1-yl]-butyryl}-4,4-bipipéridinyl-1-yl)-acétique,
(171) l'acide (1'-{2-(4-chloro-3-méthyl-benzyl)-4-oxo-4-[4-(5-oxo-3-phényl-4,5-dihydro-1,2,4-triazol-1-yl)-pipéridin-1-yl]-butyryl}-4,4'-bipipéridinyl-1-yl)-acétique,
(172) la 2-(3-bromo-4-chloro-5-méthyl-benzyl)-1-[4-(4-méthyl-pipérazin-1-yl)-pipéridin-1-yl]-4-[4-(2-oxo-1,4-dihydro-2H-quinazolin-3-yl)-pipéridin-1-yl]-butane-1,4-dione,
(173) la 2-(3-bromo-4-chloro-5-méthyl-benzyl)-1-(1'-méthyl-[4,4']bipipéridinyl-1-yl)-4-[4-(2-oxo-1,4-dihydro-2H-quinazolin-3-yl)-pipéridin-1-yl]-butane-1,4-dione,
(174) la 2-(3-bromo-4-chloro-5-méthyl-benzyl)-4-[4-(2-oxo-1,4-dihydro-2H-quinazolin-3-yl)-pipéridin-1-yl]-1-(4-pyridin-4-yl-pipérazin-1-yl)-butane-1,4-dione,
(175) la 2-(3-bromo-4-chloro-5-méthyl-benzyl)-1-[4-(1-méthyl-pipéridin-4-yl)-pipérazin-1-yl]-4-[4-(2-oxo-1,4-dihydro-2H-quinazolin-3-yl)-pipéridin-1-yl]-butane-1,4-dione,
(176) l'acide [4-(1-{2-(3-bromo-4-chloro-5-méthyl-benzyl)-4-oxo-4-[4-(2-oxo-1,4-dihydro-2H-quinazolin-3-yl)-pipéridm-1-yl]-butyryl}-pipéridin-4-yl)-pipérazin-1-yl]-acétique,
(177) l'ester méthylique de l'acide (1'-(2-(3-bromo-4-chloro-5-méthyl-benzyl)-4-oxo-4-[4-(2-oxo-1,4-dihydro-2H-quinazolin-3-yl)-pipéridin-1-yl]-butyryl}-[4,4']bipipéridinyl-1-yl)-acétique,
(178) l'acide (1'-{2-(3-bromo-4-chloro-5-méthyl-benzyl)-4-oxo-4-[4-(2-oxo-1,4-dihydro-2H-quinazolin-3-yl)-pipéridin-1-yl]-butyryl}-[4,4']bipipéridinyl-1-yl)-acétique,
(179) le {1-(3-bromo-4-chloro-5-méthyl-benzyl)-2-[4-(4-méthyl-pipérazin-1-yl)-pipéridin-1-yl]-2-oxo-éthyl}-amide de l'acide 4-(2-oxo-1,4-dihydro-2H-quinazolin-3-yl)-pipéridin-1-carboxylique,
(180) le [2-[1,4']bipipéridinyl-1'-yl-1-(3-bromo-4-chloro-5-méthyl-benzyl)-2-oxo-éthyl]-amide de l'acide 4-(2-oxo-1,4-dihydro-2H-quinazolin-3-yl)-pipéridin-1-carboxylique,
(181) le {1-(3-bromo-4-chloro-5-méthyl-benzyl)-2-[4-(1-méthyl-pipéridin-4-yl)-pipérazin-1-yl]-2-oxo-éthyl}-amide de l'acide 4-(2-oxo-1,4-dihydro-2H-quinazolin-3-yl)-pipéridin-1-carboxylique,
(182) le [1-(3-bromo-4-chloro-5-méthyl-benzyl)-2-(1'-méthyl-[4,4']bipipéridin-1-yl)-2-oxo-éthyl)-amide de l'acide 4-(2-oxo-1,4-dihydro-2H-quinazolin-3-yl)-pipéridin-1-carboxylique,
(183) le [1-(3-bromo-4-chloro-5-méthyl-benzyl)-2-oxo-2-(4-pyridin-4-yl-pipérazin-1-yl)-éthyl]-amide de l'acide 4-(2-oxo-1,4-dihydro-2H-quinazolin-3-yl)-pipéridin-1-carboxylique,
(184) la 2-(4-chloro-3-trifluorométhyl-benzyl)-1-(l'-méthyl-[4,4']bipipéridin-1-yl)-4-[4-(2-oxo-1,4-dihydro-2H-quinazolin-3-yl)-pipéridin-1-yl]-butane-1,4-dione,
(185) la 2-(4-chloro-3-trifluorométhyl-benzyl)-1-[4-(4-méthyl-pipérazin-1-yl)-pipéridin-1-yl]-4-[4-(2-oxo-1,4-dihydro-2H-quinazolin-3-yl)-pipéridin-1-yl]-butane-1,4-dione,
(186) la 1-[1,4']bipipéridinyl-1'-yl-2-(4-chloro-3-trifluorométhyl-benzyl)-4-[4-(2-oxo-1,4-dihydro-2H-quinazolin-3-yl)-pipéridin-1-yl]-butane-1,4-dione,
(187) l'acide [4-(1-{2-(4-chloro-3-trifluorométhyl-benzyl)-4-oxo-4-[4-(2-oxo-1,4-dihydro-2H-quinazolin-3-yl)-pipéridin-1-yl)-butyryl}-pipéridin-4-yl)-pipérazin-1-yl]-acétique,
(188) l'ester méthylique de l'acide (1'-{2-(4-chloro-3-tiifluorométhyl-benzyl)-4-oxo-4-[4-(2-oxo-1,4-dihydro-2H-quinazolin-3-yl)-pipéridin-1-yl]-butyryl)-[4,4']bipipéridinyl-1-yl)-acétique,
(189) l'acide (1'-{2-(4-chloro-3-trifluorométhyl-benzyl)-4-oxo-4-[4-(2-oxo-1,4-dihydro-2H-qunazolin-3-yl)-pipéridin-1-yl]-butyryl}-[4,4]bipipéridinyl-1-yl)-acétique,
(190) la 2-(4-chloro-3-trifluorométhyl-benzyl)-1-(1'-méthyl-[4,4']bipipéridinyl-1-yl]-4[4-(2-oxo-1,4-dihydro-2H-quinazolin-3-yl)-pipéridin-1-yl]-butane-1,4-dione,
leurs énantiomères, leurs diastéréoisomères et leurs sels.

10. Sels physiologiquement acceptables des composés selon l'une quelconque des revendications 1 à 9 et d'acides ou de bases minéraux ou organiques.

11. Médicaments, contenant un composé selon au moins l'une quelconque des revendications 1 à 9 ou un sel physiologiquement acceptable selon la revendication 10, avec, en outre, le cas échéant, un ou plusieurs excipients et/ou diluants inertes.

12. Utilisation d'un composé selon au moins l'une quelconque des revendications 1 à 10 pour la fabrication d'un médicament pour le traitement aigu et prophylactique des maux de tête, en particulier des douleurs de migraine ou des maux de tête de type cluster, des maux de tête de tension et de maux de tête chroniques.

13. Utilisation d'un composé selon au moins l'une quelconque des revendications 1 à 10, pour la fabrication d'un médicament pour le traitement prophylactique des maux de tête de migraine pendant les signes précurseurs ou pour le traitement aigu et prophylactique des maux de tête de migraine, qui apparaissent avant ou pendant les menstruations.

14. Utilisation d'un composé selon au moins l'une quelconque des revendications 1 à 10 pour la fabrication d'un médicament pour la lutte contre le diabète sucré à non dépendance de l'insuline (NIDDM).

15. Utilisation d'un composé selon au moins l'une quelconque des revendications 1 à 10 pour la fabrication d'un médicament pour le traitement des maladies cardiovasculaires, de l'intolérance vis-à-vis de la morphine, des maladies diarrhéiques conditionnés par la toxine Chlostritium, des maladies de la peau, en particulier, des lésions thermiques et dues aux rayonnements, y compris les coups de soleil, des maladies inflammatoires comme, en particulier, les maladies articulaires inflammatoires comme l'arthrite, des inflammations neurogènes de la muqueuse orale, des maladies pulmonaires inflammatoires, de la rhinite allergique, de l'asthme, de maladies qui proviennent d'une vasodilatation excessive et de l'irrigation sanguine réduite des vaisseaux qui en résulte, comme, en particulier les chocs ou la sepsie ou les rougissements de la peau, pour soulager des états douloureux en général, en particulier, dans le cas de douleurs neuropathiques, dans le cas d'états douloureux neuropathiques dans le cadre de maladies neurotoxiques systémiques ainsi que dans le cas d'états douloureux qui remontent à des processus inflammatoires ou pour la prévention ou le traitement aigu de la symptomatique causée par la vasodilatation et le flux sanguin accru des bouffées de chaleur de femmes ménopausale, déficientes en oestrogène ainsi que de patients souffrant d'un carcinome de la prostate sous traitement d'hormones.

16. Procédé pour la fabrication d'un médicament selon la revendication 11, **caractérisé en ce que** l'on incorpore, par une voie non chimique, un composé selon au moins l'une quelconque des revendications 1 à 10 dans un ou plusieurs excipients et/ou diluants inertes.

17. Procédé pour la fabrication des composés de la formule générale (I) selon au moins l'une quelconque des revendications 1 à 9, **caractérisé en ce que**
(a) en vue de la fabrication de composés de la formule générale (I), dans laquelle X représente un atome d'oxygène ou le groupe NH et R¹ à R³ sont définis comme dans la revendication 1, sous réserve que ces groupes ne contiennent aucune fonction d'acide carboxylique libre:
réaction de pipéridines de la formule générale : 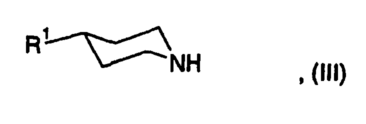 dans laquelle R¹ est défini comme dans la revendication 1,
(i) avec des dérivés de l'acide carbonique de la formule générale : 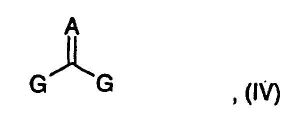 dans laquelle A est défini comme dans la revendication 1 et G signifie un groupe nucléofuge, sous réserve que X représente le groupe NH, ou
(ii) avec des dérivés de l'acide carbonique de la formule générale : 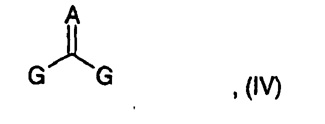 dans laquelle A représente un atome d'oxygène et chaque G indépendamment un groupe nucléofuge, sous réserve que X signifie un atome d'oxygène,
et avec les composés de la formule générale 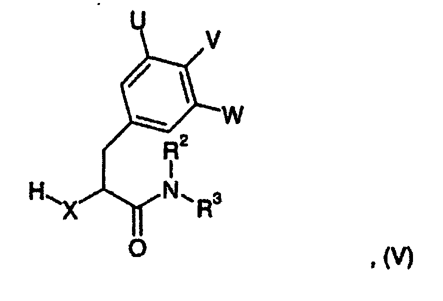 dans laquelle X signifie un atome d'oxygène ou un groupe -NH et U, V, W, R² et R³ sont définis comme dans la revendication 1, sous réserve que R² et R³ ne contiennent aucune fonction acide carboxylique libre et/ou aucune autre fonction amino aliphatique primaire ou secondaire libre ou aucune autre fonction hydroxy libre ou
(b) en vue de la fabrication de composés de la formule générale (I), dans laquelle X représente un groupe méthylène et R¹ à R³ sont définis comme dans la revendication 1, sous réserve que Ces groupes ne contiennent aucune fonction acide carboxylique libre et/ou aucune autre fonction amino aliphatique primaire ou secondaire libre:
Couplage d'un acide carboxylique de la formule générale : 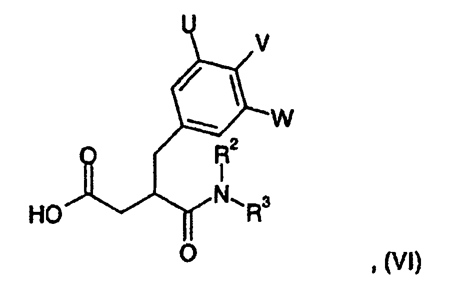
dans laquelle U, V, W, R² et R³ sont définis comme dans la revendication 1, avec une pipéridine de la formule générale : 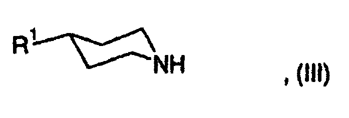 dans laquelle R¹ est défini comme dans la revendication 1, ou
(c) en vue de la fabrication de composés de la formule générale (I), dans laquelle X signifie le groupe méthylène et R² et R³ sont définis comme dans la revendication 1, sous réserve que ces groupes ne contiennent aucune fonction amine primaire ou secondaire libre:
Couplage d'un composé de la formule générale : 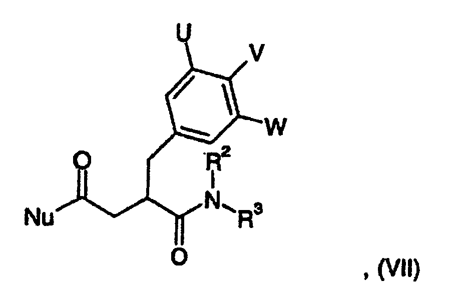
dans laquelle U, V, W, R² et R³ sont définis comme dans la revendication 1, sous réserve que R² et R³ ne contiennent aucune amine primaire ou secondaire libre et que Nu signifie un groupment sortant,
avec une pipéridine de la formule générale : 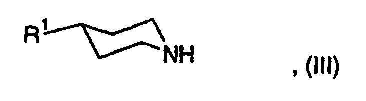 dans laquelle R¹ est défini comme dans la revendication 1, ou
(d) en vue de la fabrication de composés de la formule générale (I), dans laquelle tous les radicaux sont comme définis dans la revendication 1:
Couplage d'un acide carboxylique de la formule générale : 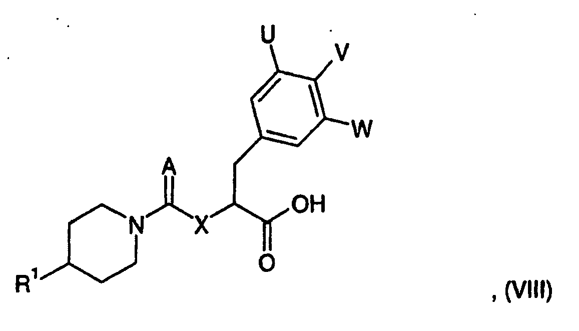
dans laquelle tous les radicaux sont définis comme dans la revendication 1, avec une amine de la formule générale HNR²R³, dans laquelle R² et R³ sont comme définis dans la revendication 1, sous réserve que ces groupes ne contiennent aucune fonction acide carboxylique libre et/ou aucune autre fonction amino aliphatique primaire ou secondaire libre ou
(e) en vue de la fabrication de composés de la formule générale (I), dans laquelle R¹ est défini comme dans la revendication 1, sous réserve qu'aucune amine primaire ou secondaire libre n'y soit contenue:
Couplage d'un composé de la formule générale : 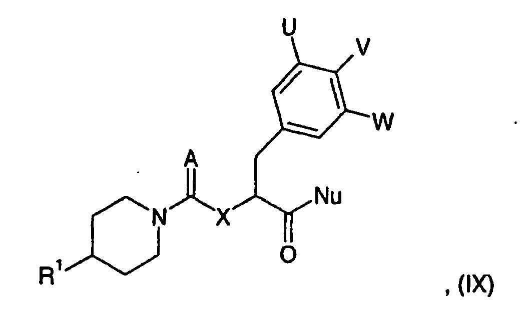
dans laquelle tous les radicaux sont comme définis dans la revendication 1 et Nu représente un groupe partant,
avec une amine de la formule générale HNR²R³, dans laquelle R² et R³ sont comme définis dans la revendication 1, sous réserve que ces groupes ne contiennent aucune fonction acide carboxylique libre et/ou aucune autre fonction amino aliphatique primaire ou secondaire libre,
et
si besoin est, coupure d'un radical de protection utilisé lors des réactions décrites ci-dessus et/ou
conversion d'éventuelles fonctions de précurseurs utilisées, présentes dans un composé ainsi obtenu et/ou
si on le souhaite, séparation des stéréoisomères d'un composé ainsi obtenu de la formule générale (I) et/ou
conversion d'un composé ainsi obtenu de la formule générale (I) en sel, en particulier pour l'application pharmaceutique, en sels compatibles du point de vue physiologique.
